# EUROPEAN PATENT SPECIFICATION

(11) **EP 3 119 423 B1**
(45) Date of publication and mention of the grant of the patent: **14.12.2022**
(21) Application number: 15712503.0
(22) Date of filing: 13.03.2015
(51) Int. Cl.: A61K 39/00, A61K 39/12, A61K 39/395, C07K 14/725, C07K 14/705, C07K 16/28, C07K 16/32, C12N 5/0783, A61P 35/00, C07K 16/30, A61K 39/39, A61P 35/02

(54) **TREATMENT OF CANCER USING CHIMERIC ANTIGEN RECEPTOR**
BEHANDELUNG VON KREBS MIT EINEM CHIMÄREN ANTIGENREZEPTOR
TRAITEMENT DU CANCER AVEC UN RÉCEPTEUR D'ANTIGÈNE CHIMÉRIQUE

(30) Priority: 15.03.2014 US 201461953783 P; 07.04.2014 US 201461976375 P; 21.07.2014 US 201462027154 P; 06.11.2014 US 201462076146 P; 29.12.2014 US 201462097286 P
(43) Date of publication of application: 25.01.2017
(60) Divisional application: 19169356.3
(73) Proprietor: Novartis AG, 4056 Basel (CH); The Trustees of The University of Pennsylvania, Philadelphia, PA 19104 (US)
(72) Inventor: LOEW, Andreas, Cambridge, MA 02139 (US); MILONE, Michael C., Cherry Hill, NJ 08002 (US); POWELL, Daniel J., Bala Cynwyd, PA 19004 (US); ZHAO, Yangbing, Lumberton, NJ 08048 (US)
(74) Representative: Wilding, James Roger
(86) International application number: PCT/US2015/020606
(87) International publication number: WO 2015/142675

(56) References cited:
- WO-A2-2014/145252
- WO-A2-2014/145252
- FATEMEH RAHIMI JAMNANI ET AL: "T cells expressing VHH-directed oligoclonal chimeric HER2 antigen receptors: Towards tumor-directed oligoclonal T cell therapy", BIOCHIMICA ET BIOPHYSICA ACTA (BBA) - GENERAL SUBJECTS, vol. 1840, no. 1, 1 January 2014 (2014-01-01), pages 378-386, XP055108962, ISSN: 0304-4165, DOI: 10.1016/j.bbagen.2013.09.029
- NAZANIN PIROOZNIA ET AL: "The Construction of Chimeric T-Cell Receptor with Spacer Base of Modeling Study of VHH and MUC1 Interaction", JOURNAL OF BIOMEDICINE AND BIOTECHNOLOGY, vol. 2011, 1 January 2011 (2011-01-01), pages 1-11, XP055369439, US ISSN: 1110-7243, DOI: 10.1155/2011/578128
- V. D. FEDOROV ET AL: "PD-1- and CTLA-4-Based Inhibitory Chimeric Antigen Receptors (iCARs) Divert Off-Target Immunotherapy Responses", SCIENCE TRANSLATIONAL MEDICINE, vol. 5, no. 215, 11 December 2013 (2013-12-11), pages 215ra172-215ra172, XP055210508, ISSN: 1946-6234, DOI: 10.1126/scitranslmed.3006597
- JULIE R PARK ET AL: "Adoptive Transfer of Chimeric Antigen Receptor Re-directed Cytolytic T Lymphocyte Clones in Patients with Neuroblastoma", MOLECULAR THERAPY, 1 January 2007 (2007-01-01), XP055021897, ISSN: 1525-0016, DOI: 10.1038/sj.mt.6300104
- M. J. RIESE ET AL: "Enhanced Effector Responses in Activated CD8+ T Cells Deficient in Diacylglycerol Kinases", CANCER RESEARCH, vol. 73, no. 12, 10 April 2013 (2013-04-10), pages 3566-3577, XP055142357, ISSN: 0008-5472, DOI: 10.1158/0008-5472.CAN-12-3874
- JOHN L B ET AL: "Anti-PD-1 antibody therapy potently enhances the eradication of established tumors by gene-modified T cells", CLINICAL CANCER RESEARCH, THE AMERICAN ASSOCIATION FOR CANCER RESEARCH, US, vol. 19, no. 20, 15 October 2013 (2013-10-15), pages 5636-5646, XP002737460, ISSN: 1078-0432, DOI: 10.1158/1078-0432.CCR-13-0458 [retrieved on 2013-07-19]
- I. M. STROMNES ET AL: "Abrogation of Src Homology Region 2 Domain-Containing Phosphatase 1 in Tumor-Specific T Cells Improves Efficacy of Adoptive Immunotherapy by Enhancing the Effector Function and Accumulation of Short-Lived Effector T Cells In Vivo", THE JOURNAL OF IMMUNOLOGY, vol. 189, no. 4, 15 August 2012 (2012-08-15), pages 1812-1825, XP055190965, ISSN: 0022-1767, DOI: 10.4049/jimmunol.1200552
- MICHAEL H. KERSHAW ET AL: "Gene-engineered T cells for cancer therapy", NATURE REVIEWS CANCER, vol. 13, no. 8, 24 July 2013 (2013-07-24), pages 525-541, XP055185130, ISSN: 1474-175X, DOI: 10.1038/nrc3565
- LIANG-CHUAN WANG ET AL: "Overcoming intrinsic inhibitory pathways to augment the antineoplastic activity of adoptively transferred T cells: Re-tuning your CAR before hitting a rocky road", ONCOIMMUNOLOGY, vol. 2, no. 11, 1 November 2013 (2013-11-01), page e26492, XP055191167, DOI: 10.4161/onci.26492
- FATEMEH RAHIMI JAMNANI ET AL: "T cells expressing VHH-directed oligoclonal chimeric HER2 antigen receptors: Towards tumor-directed oligoclonal T cell therapy", BIOCHIMICA ET BIOPHYSICA ACTA (BBA) - GENERAL SUBJECTS, vol. 1840, no. 1, 1 January 2014 (2014-01-01), pages 378-386, XP055108962, ISSN: 0304-4165, DOI: 10.1016/j.bbagen.2013.09.029
- NAZANIN PIROOZNIA ET AL: "The Construction of Chimeric T-Cell Receptor with Spacer Base of Modeling Study of VHH and MUC1 Interaction", JOURNAL OF BIOMEDICINE AND BIOTECHNOLOGY, vol. 2011, 1 January 2011 (2011-01-01), pages 1-11, XP055369439, US ISSN: 1110-7243, DOI: 10.1155/2011/578128
- V. D. FEDOROV ET AL: "PD-1- and CTLA-4-Based Inhibitory Chimeric Antigen Receptors (iCARs) Divert Off-Target Immunotherapy Responses", SCIENCE TRANSLATIONAL MEDICINE, vol. 5, no. 215, 11 December 2013 (2013-12-11), pages 215ra172-215ra172, XP055210508, ISSN: 1946-6234, DOI: 10.1126/scitranslmed.3006597

## Description

### RELATED APPLICATIONS

This application claims priority to U.S. Serial No. 61/953,783, filed March 15, 2014, U.S. Serial No. 61/976,375, filed April 7, 2014, U.S. Serial No. 62/027,154, filed July 21, 2014, U.S. Serial No. 62/076,146, filed November 6, 2014, and U.S. Serial No. 62/097,286, filed December 29, 2014.

### FIELD OF THE INVENTION

The present invention relates generally to the use of immune effector cells (e.g., T cells, NK cells) engineered to express a Chimeric Antigen Receptor (CAR) to treat a disease associated with expression of a tumor antigen.

### BACKGROUND OF THE INVENTION

Adoptive cell transfer (ACT) therapy with autologous T-cells, especially with T-cells transduced with Chimeric Antigen Receptors (CARs), has shown promise in hematologic cancer trials. Jamnani et al., Biochimica et Biophysica Acta 1840 (2014):378-386 describes an anti-HER2 VHH CAR. Pirooznia et al., J Biomed Biotech (2011):578128 relates to models of VHH CARs. Federov et al., Sci Transl Med (2013);5(215):215ra172 reports that PD-1- and CTLA-4-based inhibitory CARs divert off-target immunotherapy responses.

### SUMMARY OF THE INVENTION

The present invention provides an immune effector cell (e.g. a population of immune effector cells), comprising:
(a) a first chimeric antigen receptor (CAR) molecule or nucleic acid encoding the same, wherein said first CAR molecule is a CAR polypeptide comprising an antigen binding domain, a transmembrane domain, and an intracellular signaling domain comprising a costimulatory domain and/or a primary signaling domain, and wherein said antigen binding domain comprises a VHH domain and binds to a tumor antigen; and
(b) a second CAR molecule or nucleic acid encoding the same, wherein said second CAR molecule is a CAR polypeptide having an antigen binding domain comprising an scFv.

The present invention also provides said immune effector cell for use as a medicament.

The present invention also provides said immune effector cell for use in the treatment of a disease expressing a tumor antigen.

These and further embodiments are set out in the attached claims.

### DETAILED DESCRIPTION

The technical information set out below may in some respects go beyond the scope of the invention, which is defined by the attached claims. The additional technical information is provided to place the actual invention in a broader technical context and to illustrate possible related technical developments.

In addition, incidental references to methods for treatment of the human or animal body by surgery or therapy and diagnostic methods practised on the human or animal body are not to be construed as claiming protection for such method themselves, but are instead to be construed as referring to products, in particular substances or compositions, for use in any of these methods.

The present disclosure pertains, at least in part, to the use of immune effector cells (e.g., T cells, NK cells) engineered to express a CAR that binds to a tumor antigen as described herein to treat cancer associated with expression of said tumor antigen. The present invention provides an immune effector cell comprising a first CAR and a second CAR, or comprising nucleic acids encoding the same, and the therapeutic use of such a cell, as defined in the attached claims.

### CAR-encoding nucleic acids

Accordingly, in one aspect, the invention employs an isolated nucleic acid molecule encoding a chimeric antigen receptor (CAR), wherein the CAR comprises an antigen binding domain that binds to a tumor antigen as described herein, a transmembrane domain (e.g., a transmembrane domain described herein), and an intracellular signaling domain (e.g., an intracellular signaling domain described herein) (e.g., an intracellular signaling domain comprising a costimulatory domain (e.g., a costimulatory domain described herein) and/or a primary signaling domain (e.g., a primary signaling domain described herein). In some embodiments, the tumor antigen is chosen from one or more of: CD19; CD123; CD22; CD30; CD171; CS-1 (also referred to as CD2 subset 1, CRACC, SLAMF7, CD319, and 19A24); C-type lectin-like molecule-1 (CLL-1 or CLECL1); CD33; epidermal growth factor receptor variant III (EGFRvIII); ganglioside G2 (GD2); ganglioside GD3 (aNeu5Ac(2-8)aNeu5Ac(2-3)bDGalp(1-4)bDGlcp(1-1)Cer); TNF receptor family member B cell maturation (BCMA); Tn antigen ((Tn Ag) or (GalNAcα-Ser/Thr)); prostate-specific membrane antigen (PSMA); Receptor tyrosine kinase-like orphan receptor 1 (ROR1); Fms-Like Tyrosine Kinase 3 (FLT3); Tumor-associated glycoprotein 72 (TAG72); CD38; CD44v6; Carcinoembryonic antigen (CEA); Epithelial cell adhesion molecule (EPCAM); B7H3 (CD276); KIT (CD117); Interleukin-13 receptor subunit alpha-2 (IL-13Ra2 or CD213A2); Mesothelin; Interleukin 11 receptor alpha (IL-11Ra); prostate stem cell antigen (PSCA); Protease Serine 21 (Testisin or PRSS21); vascular endothelial growth factor receptor 2 (VEGFR2); Lewis(Y) antigen; CD24; Platelet-derived growth factor receptor beta (PDGFR-beta); Stage-specific embryonic antigen-4 (SSEA-4); CD20; Folate receptor alpha; Receptor tyrosine-protein kinase ERBB2 (Her2/neu); Mucin 1, cell surface associated (MUC1); epidermal growth factor receptor (EGFR); neural cell adhesion molecule (NCAM); Prostase; prostatic acid phosphatase (PAP); elongation factor 2 mutated (ELF2M); Ephrin B2; fibroblast activation protein alpha (FAP); insulin-like growth factor 1 receptor (IGF-I receptor), carbonic anhydrase IX (CAIX); Proteasome (Prosome, Macropain) Subunit, Beta Type, 9 (LMP2); glycoprotein 100 (gp100); oncogene fusion protein consisting of breakpoint cluster region (BCR) and Abelson murine leukemia viral oncogene homolog 1 (Abl) (bcr-abl); tyrosinase; ephrin type-A receptor 2 (EphA2); Fucosyl GM1; sialyl Lewis adhesion molecule (sLe); ganglioside GM3 (aNeu5Ac(2-3)bDGalp(1-4)bDGlcp(1-1)Cer); transglutaminase 5 (TGS5); high molecular weight-melanoma-associated antigen (HMWMAA); o-acetyl-GD2 ganglioside (OAcGD2); Folate receptor beta; tumor endothelial marker 1 (TEM1/CD248); tumor endothelial marker 7-related (TEM7R); claudin 6 (CLDN6); thyroid stimulating hormone receptor (TSHR); G protein-coupled receptor class C group 5, member D (GPRC5D); chromosome X open reading frame 61 (CXORF61); CD97; CD179a; anaplastic lymphoma kinase (ALK); Polysialic acid; placenta-specific 1 (PLAC1); hexasaccharide portion of globoH glycoceramide (GloboH); mammary gland differentiation antigen (NY-BR-1); uroplakin 2 (UPK2); Hepatitis A virus cellular receptor 1 (HAVCR1); adrenoceptor beta 3 (ADRB3); pannexin 3 (PANX3); G protein-coupled receptor 20 (GPR20); lymphocyte antigen 6 complex, locus K 9 (LY6K); Olfactory receptor 51E2 (OR51E2); TCR Gamma Alternate Reading Frame Protein (TARP); Wilms tumor protein (WT1); Cancer/testis antigen 1 (NY-ESO-1); Cancer/testis antigen 2 (LAGE-1a); Melanoma-associated antigen 1 (MAGE-A1); ETS translocation-variant gene 6, located on chromosome 12p (ETV6-AML); sperm protein 17 (SPA17); X Antigen Family, Member 1A (XAGE1); angiopoietin-binding cell surface receptor 2 (Tie 2); melanoma cancer testis antigen-1 (MAD-CT-1); melanoma cancer testis antigen-2 (MAD-CT-2); Fos-related antigen 1; tumor protein p53 (p53); p53 mutant; prostein; surviving; telomerase; prostate carcinoma tumor antigen-1 (PCTA-1 or Galectin 8), melanoma antigen recognized by T cells 1 (MelanA or MARTI); Rat sarcoma (Ras) mutant; human Telomerase reverse transcriptase (hTERT); sarcoma translocation breakpoints; melanoma inhibitor of apoptosis (ML-IAP); ERG (transmembrane protease, serine 2 (TMPRSS2) ETS fusion gene); N-Acetyl glucosaminyl-transferase V (NA17); paired box protein Pax-3 (PAX3); Androgen receptor; Cyclin B1; v-myc avian myelocytomatosis viral oncogene neuroblastoma derived homolog (MYCN); Ras Homolog Family Member C (RhoC); Tyrosinase-related protein 2 (TRP-2); Cytochrome P450 1B1 (CYP1B1); CCCTC-Binding Factor (Zinc Finger Protein)-Like (BORIS or Brother of the Regulator of Imprinted Sites), Squamous Cell Carcinoma Antigen Recognized By T Cells 3 (SART3); Paired box protein Pax-5 (PAX5); proacrosin binding protein sp32 (OY-TES1); lymphocyte-specific protein tyrosine kinase (LCK); A kinase anchor protein 4 (AKAP-4); synovial sarcoma, X breakpoint 2 (SSX2); Receptor for Advanced Glycation Endproducts (RAGE-1); renal ubiquitous 1 (RU1); renal ubiquitous 2 (RU2); legumain; human papilloma virus E6 (HPV E6); human papilloma virus E7 (HPV E7); intestinal carboxyl esterase; heat shock protein 70-2 mutated (mut hsp70-2); CD79a; CD79b; CD72; Leukocyte-associated immunoglobulin-like receptor 1 (LAIR1); Fc fragment of IgA receptor (FCAR or CD89); Leukocyte immunoglobulin-like receptor subfamily A member 2 (LILRA2); CD300 molecule-like family member f (CD300LF); C-type lectin domain family 12 member A (CLEC12A); bone marrow stromal cell antigen 2 (BST2); EGF-like module-containing mucin-like hormone receptor-like 2 (EMR2); lymphocyte antigen 75 (LY75); Glypican-3 (GPC3); Fc receptor-like 5 (FCRL5); and immunoglobulin lambda-like polypeptide 1 (IGLL1).

In some embodiments, tumor antigen bound by the encoded CAR molecule is chosen from one or more of: TSHR, CD171, CS-1, CLL-1, GD3, Tn Ag, FLT3, CD38, CD44v6, B7H3, KIT, IL-13Ra2, IL-11Ra, PSCA, PRSS21, VEGFR2, LewisY, CD24, PDGFR-beta, SSEA-4, MUC1, EGFR, NCAM, CAIX, LMP2, EphA2, Fucosyl GM1, sLe, GM3, TGS5, HMWMAA, o-acetyl-GD2, Folate receptor beta, TEM1/CD248, TEM7R, CLDN6, GPRC5D, CXORF61, CD97, CD179a, ALK, Polysialic acid, PLAC1, GloboH, NY-BR-1, UPK2, HAVCR1, ADRB3, PANX3, GPR20, LY6K, OR51E2, TARP, WT1, ETV6-AML, sperm protein 17, XAGE1, Tie 2, MAD-CT-1, MAD-CT-2, Fos-related antigen 1, p53 mutant, hTERT, sarcoma translocation breakpoints, ML-IAP, ERG (TMPRSS2 ETS fusion gene), NA17, PAX3, Androgen receptor, Cyclin B1, MYCN, RhoC, CYP1B1, BORIS, SART3, PAX5, OY-TES1, LCK, AKAP-4, SSX2, CD79a, CD79b, CD72, LAIR1, FCAR, LILRA2, CD300LF, CLEC12A, BST2, EMR2, LY75, GPC3, FCRL5, and IGLL1.

In certain embodiments, the tumor antigen bound by the encoded CAR molecule is chosen from one or more of: TSHR, CLDN6, GPRC5D, CXORF61, CD97, CD179a, ALK, Polysialic acid, PLAC1, GloboH, NY-BR-1, UPK2, HAVCR1, ADRB3, PANX3, GPR20, LY6K, and OR51E2.

In some instances, the antigen binding domain of the encoded CAR molecule comprises an antibody, an antibody fragment, an scFv, a Fv, a Fab, a (Fab')2, a single domain antibody (SDAB), a VH or VL domain, or a camelid VHH domain. In the present invention, the antigen binding domain of the first CAR comprises a VHH domain and the antigen binding domain of the second CAR comprises an scFv.

In some embodiments, the transmembrane domain of the encoded CAR molecule comprises a transmembrane domain chosen from the transmembrane domain of an alpha, beta or zeta chain of a T-cell receptor, CD28, CD3 epsilon, CD45, CD4, CD5, CD8, CD9, CD16, CD22, CD33, CD37, CD64, CD80, CD86, CD134, CD137, CD154, KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), CD160, CD19, IL2R beta, IL2R gamma, IL7R α, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO-3), BLAME (SLAMF8), SELPLG (CD162), LTBR, PAG/Cbp, NKp44, NKp30, NKp46, NKG2D, and/or NKG2C.

In certain embodiments, the encoded transmembrane domain comprises an amino acid sequence of a CD8 transmembrane domain having at least one, two or three modifications but not more than 20, 10 or 5 modifications of an amino acid sequence of SEQ ID NO: 12, or a sequence with 95-99% identity to an amino acid sequence of SEQ ID NO: 12. In one embodiment, the encoded transmembrane domain comprises the sequence of SEQ ID NO: 12.

In other embodiments, the nucleic acid molecule comprises a nucleotide sequence of a CD8 transmembrane domain, e.g., comprising the sequence of SEQ ID NO: 13, or a sequence with 95-99% identity thereof.

In certain embodiments, the encoded antigen binding domain is connected to the transmembrane domain by a hinge region. In one embodiment, the encoded hinge region comprises the amino acid sequence of a CD8 hinge, e.g., SEQ ID NO: 2; or the amino acid sequence of an IgG4 hinge, e.g., SEQ ID NO: 6, or a sequence with 95-99% identity to SEQ ID NO:2 or 6. In other embodiments, the nucleic acid sequence encoding the hinge region comprises a sequence of SEQ ID NO: 3 or SEQ ID NO: 7, corresponding to a CD8 hinge or an IgG4 hinge, respectively, or a sequence with 95-99% identity to SEQ ID NO:3 or 7.

In other embodiments, the nucleic acid molecule encodes an intracellular signaling domain comprising a sequence encoding a primary signaling domain and/or a sequence encoding a costimulatory signaling domain. In some embodiments, the intracellular signaling domain comprises a sequence encoding a primary signaling domain. In some embodiments, the intracellular signaling domain comprises a sequence encoding a costimulatory signaling domain. In some embodiments, the intracellular signaling domain comprises a sequence encoding a primary signaling domain and a sequence encoding a costimulatory signaling domain.

In certain embodiments, the encoded primary signaling domain comprises a functional signaling domain of a protein selected from the group consisting of CD3 zeta, CD3 gamma, CD3 delta, CD3 epsilon, common FcR gamma (FCER1G), FcR beta (Fc Epsilon R1b), CD79a, CD79b, Fcgamma RIIa, DAP10, and DAP12.

In one embodiment, the encoded primary signaling domain comprises a functional signaling domain of CD3 zeta. The encoded CD3 zeta primary signaling domain can comprise an amino acid sequence having at least one, two or three modifications but not more than 20, 10 or 5 modifications of an amino acid sequence of SEQ ID NO: 18 or SEQ ID NO: 20, or a sequence with 95-99% identity to an amino acid sequence of SEQ ID NO:18 or SEQ ID NO: 20. In some embodiments, the encoded primary signaling domain comprises a sequence of SEQ ID NO:18 or SEQ ID NO: 20. In other embodiments, the nucleic acid sequence encoding the primary signaling domain comprises a sequence of SEQ ID NO:19 or SEQ ID NO: 21, or a sequence with 95-99% identity thereof.

In some embodiments, the encoded intracellular signaling domain comprises a sequence encoding a costimulatory signaling domain. For example, the intracellular signaling domain can comprise a sequence encoding a primary signaling domain and a sequence encoding a costimulatory signaling domain. In some embodiments, the encoded costimulatory signaling domain comprises a functional signaling domain of a protein chosen from one or more of CD27, CD28, 4-1BB (CD137), OX40, CD30, CD40, PD-1, ICOS, lymphocyte function-associated antigen-1 (LFA-1), CD2, CD7, LIGHT, NKG2C, B7-H3, a ligand that specifically binds with CD83, CDS, ICAM-1, GITR, BAFFR, HVEM (LIGHTR), SLAMF7, NKp80 (KLRF1), CD160, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, NKp44, NKp30, NKp46, or NKG2D.

In certain embodiments, the encoded costimulatory signaling domain comprises an amino acid sequence having at least one, two or three modifications but not more than 20, 10 or 5 modifications of an amino acid sequence of SEQ ID NO: 14 or SEQ ID NO: 16, or a sequence with 95-99% identity to an amino acid sequence of SEQ ID NO:14 or SEQ ID NO: 16. In one embodiment, the encoded costimulatory signaling domain comprises a sequence of SEQ ID NO: 14 or SEQ ID NO: 16. In other embodiments, the nucleic acid sequence encoding the costimulatory signaling domain comprises a sequence of SEQ ID NO: 15 or SEQ ID NO: 17, or a sequence with 95-99% identity thereof.

In other embodiments, the encoded intracellular domain comprises the sequence of SEQ ID NO: 14 or SEQ ID NO: 16, and the sequence of SEQ ID NO: 18 or SEQ ID NO: 20, wherein the sequences comprising the intracellular signaling domain are expressed in the same frame and as a single polypeptide chain.

In certain embodiments, the nucleic acid sequence encoding the intracellular signaling domain comprises a sequence of SEQ ID NO: 15 or SEQ ID NO: 17, or a sequence with 95-99% identity thereof, and a sequence of SEQ ID NO:19 or SEQ ID NO:21, or a sequence with 95-99% identity thereof.

In some embodiments, the nucleic acid molecule further comprises a leader sequence. In one embodiment, the leader sequence comprises the sequence of SEQ ID NO: 2.

In certain embodiments, the encoded antigen binding domain has a binding affinity KD of 10⁻⁴ M to 10⁻⁸ M.

In one instance, the encoded antigen binding domain is an antigen binding domain described herein, e.g., an antigen binding domain described herein for a target provided above.

In one embodiment, the encoded CAR molecule comprises an antigen binding domain that has a binding affinity KD of 10⁻⁴ M to 10⁻⁸ M, e.g., 10⁻⁵ M to 10⁻⁷ M, e.g., 10⁻⁶ M or 10⁻⁷ M, for the target antigen. In one embodiment, the antigen binding domain has a binding affinity that is at least five-fold, 10-fold, 20-fold, 30-fold, 50-fold, 100-fold or 1,000-fold less than a reference antibody, e.g., an antibody described herein. In one embodiment, the encoded antigen binding domain has a binding affinity at least 5-fold less than a reference antibody (e.g., an antibody from which the antigen binding domain is derived).

In one aspect, the invention employs an isolated nucleic acid molecule encoding a chimeric antigen receptor (CAR), wherein the CAR comprises an antigen binding domain that binds to a tumor-supporting antigen (e.g., a tumor-supporting antigen as described herein), a transmembrane domain (e.g., a transmembrane domain described herein), and an intracellular signaling domain (e.g., an intracellular signaling domain described herein) (e.g., an intracellular signaling domain comprising a costimulatory domain (e.g., a costimulatory domain described herein) and/or a primary signaling domain (e.g., a primary signaling domain described herein). In some embodiments, the tumor-supporting antigen is an antigen present on a stromal cell or a myeloid-derived suppressor cell (MDSC).

### Vectors

In another aspect, the invention employs a vector comprising a nucleic acid sequence encoding a CAR in accordance with the claims. In one embodiment, the vector is chosen from a DNA vector, an RNA vector, a plasmid, a lentivirus vector, adenoviral vector, or a retrovirus vector. In one embodiment, the vector is a lentivirus vector.

In an embodiment, the vector comprises a nucleic acid sequence that encodes a CAR, and a nucleic acid sequence that encodes an inhibitory molecule comprising: an inhKIR cytoplasmic domain; a transmembrane domain, e.g., a KIR transmembrane domain; and an inhibitor cytoplasmic domain, e.g., an ITIM domain, e.g., an inhKIR ITIM domain. In an embodiment the inhibitory molecule is a naturally occurring inhKIR, or a sequence sharing at least 50, 60, 70, 80, 85, 90, 95, or 99% homology with, or that differs by no more than 1, 2, 3, 4, 5, 6, 7, 8, 9, 10, 15, or 20 residues from, a naturally occurring inhKIR.

In an embodiment, the nucleic acid sequence that encodes an inhibitory molecule comprises: a SLAM family cytoplasmic domain; a transmembrane domain, e.g., a SLAM family transmembrane domain; and an inhibitor cytoplasmic domain, e.g., a SLAM family domain, e.g., an SLAM family ITIM domain. In an embodiment the inhibitory molecule is a naturally occurring SLAM family member, or a sequence sharing at least 50, 60, 70, 80, 85, 90, 95, or 99% homology with, or that differs by no more than 1, 2, 3, 4, 5, 6, 7, 8, 9, 10, 15, or 20 residues from, a naturally occurring SLAM family member.

In one embodiment, the vector further comprises a promoter. In some embodiments, the promoter is chosen from an EF-1 promoter, a CMV IE gene promoter, an EF-1α promoter, an ubiquitin C promoter, or a phosphoglycerate kinase (PGK) promoter. In one embodiment, the promoter is an EF-1 promoter. In one embodiment, the EF-1 promoter comprises a sequence of SEQ ID NO: 1.

In one embodiment, the vector is an *in vitro* transcribed vector, e.g., a vector that transcribes RNA of a nucleic acid molecule in accordance with the claims. In one embodiment, the nucleic acid sequence in the vector further comprises a poly(A) tail, e.g., a poly A tail described herein, e.g., comprising about 150 adenosine bases (SEQ ID NO:33). In one embodiment, the nucleic acid sequence in the vector further comprises a 3'UTR, e.g., a 3' UTR described herein, e.g., comprising at least one repeat of a 3'UTR derived from human beta-globulin. In one embodiment, the nucleic acid sequence in the vector further comprises promoter, e.g., a T2A promoter.

### CAR polypeptides

In another aspect, the invention employs an isolated CAR polypeptide molecule comprising an antigen binding domain, a transmembrane domain, and an intracellular signaling domain, wherein said antigen binding domain binds to a tumor antigen chosen from one or more of: CD19, CD123, CD22, CD30, CD171, CS-1, CLL-1 (CLECL1), CD33, EGFRvIII, GD2, GD3, BCMA, Tn Ag, PSMA, ROR1, FLT3, TAG72, CD38, CD44v6, CEA, EPCAM, B7H3, KIT, IL-13Ra2, Mesothelin, IL-11Ra, PSCA, PRSS21, VEGFR2, LewisY, CD24, PDGFR-beta, SSEA-4, CD20, Folate receptor alpha, ERBB2 (Her2/neu), MUC1, EGFR, NCAM, Prostase, PAP, ELF2M, Ephrin B2, FAP, IGF-I receptor, CAIX, LMP2, gp100, bcr-abl, tyrosinase, EphA2, Fucosyl GM1, sLe, GM3, TGS5, HMWMAA, o-acetyl-GD2, Folate receptor beta, TEM1/CD248, TEM7R, CLDN6, TSHR, GPRC5D, CXORF61, CD97, CD179a, ALK, Polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, MAGE A1, ETV6-AML, sperm protein 17, XAGE1, Tie 2, MAD-CT-1, MAD-CT-2, Fos-related antigen 1, p53, p53 mutant, prostein, survivin and telomerase, PCTA-1/Galectin 8, MelanA/MART1, Ras mutant, hTERT, sarcoma translocation breakpoints, ML-IAP, ERG (TMPRSS2 ETS fusion gene), NA17, PAX3, Androgen receptor, Cyclin B1, MYCN, RhoC, TRP-2, CYP1B1, BORIS, SART3, PAX5, OY-TES1, LCK, AKAP-4, SSX2, RAGE-1, human telomerase reverse transcriptase, RU1, RU2, legumain, HPV E6,E7, intestinal carboxyl esterase, mut hsp70-2, CD79a, CD79b, CD72, LAIR1, FCAR, LILRA2, CD300LF, CLEC12A, BST2, EMR2, LY75, GPC3, FCRL5, and IGLL1.

In some embodiments, the antigen binding domain of the CAR polypeptide molecule binds to a tumor antigen chosen from one or more of: TSHR, CD171, CS-1, CLL-1, GD3, Tn Ag, FLT3, CD38, CD44v6, B7H3, KIT, IL-13Ra2, IL-11Ra, PSCA, PRSS21, VEGFR2, LewisY, CD24, PDGFR-beta, SSEA-4, MUC1, EGFR, NCAM, CAIX, LMP2, EphA2, Fucosyl GM1, sLe, GM3, TGS5, HMWMAA, o-acetyl-GD2, Folate receptor beta, TEM1/CD248, TEM7R, CLDN6, GPRC5D, CXORF61, CD97, CD179a, ALK, Polysialic acid, PLAC1, GloboH, NY-BR-1, UPK2, HAVCR1, ADRB3, PANX3, GPR20, LY6K, OR51E2, TARP, WT1, ETV6-AML, sperm protein 17, XAGE1, Tie 2, MAD-CT-1, MAD-CT-2, Fos-related antigen 1, p53 mutant, hTERT, sarcoma translocation breakpoints, ML-IAP, ERG (TMPRSS2 ETS fusion gene), NA17, PAX3, Androgen receptor, Cyclin B1, MYCN, RhoC, CYP1B1, BORIS, SART3, PAX5, OY-TES1, LCK, AKAP-4, SSX2, CD79a, CD79b, CD72, LAIR1, FCAR, LILRA2, CD300LF, CLEC12A, BST2, EMR2, LY75, GPC3, FCRL5, and IGLL1.

In some embodiments, the antigen binding domain of the CAR polypeptide molecule binds to a tumor antigen chosen from one or more of: TSHR, CLDN6, GPRC5D, CXORF61, CD97, CD179a, ALK, polysialic acid, PLAC1, GloboH, NY-BR-1, UPK2, HAVCR1, ADRB3, PANX3, GPR20, LY6K, and OR51E2.

In some instances, the antigen binding domain of the CAR polypeptide molecule comprises an antibody, an antibody fragment, an scFv, a Fv, a Fab, a (Fab')2, a single domain antibody (SDAB), a VH or VL domain, or a camelid VHH domain. In the present invention, the antigen binding domain of the first CAR comprises a VHH domain and the antigen binding domain of the second CAR comprises an scFv.

In some embodiments, the antigen binding domain of the CAR poypeptide molecule comprises a transmembrane domain of a protein chosen from an alpha, beta or zeta chain of a T-cell receptor, CD28, CD3 epsilon, CD45, CD4, CD5, CD8, CD9, CD16, CD22, CD33, CD37, CD64, CD80, CD86, CD134, CD137, CD154, KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), CD160, CD19, IL2R beta, IL2R gamma, IL7R α, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO-3), BLAME (SLAMF8), SELPLG (CD162), LTBR, PAG/Cbp, NKp44, NKp30, NKp46, NKG2D, and/or NKG2C.

In other embodiments, the transmembrane domain of the CAR polypeptide molecule comprises an amino acid sequence having at least one, two or three modifications but not more than 20, 10 or 5 modifications of an amino acid sequence of a CD8 transmembrane domain, e.g., SEQ ID NO: 12, or a sequence with 95-99% identity to an amino acid sequence of SEQ ID NO: 12. In one embodiment, the transmembrane domain comprises a sequence of SEQ ID NO: 12.

In other embodiments, the antigen binding domain of the CAR polypeptide molecule is connected to the transmembrane domain by a hinge region. In one embodiment, the encoded hinge region comprises the amino acid sequence of a CD8 hinge, e.g., SEQ ID NO: 2, or the amino acid sequence of an IgG4 hinge, e.g., SEQ ID NO: 6, or a sequence with 95-99% identity thereof.

In other embodiments, the intracellular signaling domain of the CAR polypeptide molecule comprises a primary signaling domain and/or a costimulatory signaling domain. In other embodiments, the intracellular signaling domain of the CAR polypeptide molecule comprises a primary signaling domain. In other embodiments, the intracellular signaling domain of the CAR polypeptide molecule comprises a costimulatory signaling domain. In yet other embodiments, the intracellular signaling domain of the CAR polypeptide molecule comprises a primary signaling domain and a costimulatory signaling domain.

In other embodiments, the primary signaling domain of the CAR polypeptide molecule comprises a functional signaling domain of a protein selected from the group consisting of CD3 zeta, CD3 gamma, CD3 delta, CD3 epsilon, common FcR gamma (FCER1G), FcR beta (Fc Epsilon R1b), CD79a, CD79b, Fcgamma RIIa, DAP10, and DAP12. In one embodiment, the primary signaling domain comprises a functional signaling domain of CD3 zeta. The CD3 zeta primary signaling domain can comprise an amino acid sequence having at least one, two or three modifications but not more than 20, 10 or 5 modifications of an amino acid sequence of SEQ ID NO: 18 or SEQ ID NO: 20, or a sequence with 95-99% identity to an amino acid sequence of SEQ ID NO: 18 or SEQ ID NO: 20. In some embodiments, the primary signaling domain of the CAR polypeptide molecule comprises a sequence of SEQ ID NO:18 or SEQ ID NO: 20.

In some embodiments, the intracellular signaling domain of the CAR polypeptide molecule comprises a sequence encoding a costimulatory signaling domain. For example, the intracellular signaling domain can comprise a sequence encoding a primary signaling domain and a sequence encoding a costimulatory signaling domain. In some embodiments, the encoded costimulatory signaling domain comprises a functional signaling domain of a protein chosen from one or more of CD27, CD28, 4-1BB (CD137), OX40, CD30, CD40, PD-1, ICOS, lymphocyte function-associated antigen-1 (LFA-1), CD2, CD7, LIGHT, NKG2C, B7-H3, a ligand that specifically binds with CD83, CDS, ICAM-1, GITR, BAFFR, HVEM (LIGHTR), SLAMF7, NKp80 (KLRF1), CD160, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, NKp44, NKp30, NKp46, or NKG2D.

In certain embodiments, the costimulatory signaling domain of the CAR polypeptide molecule comprises an amino acid sequence having at least one, two or three modifications but not more than 20, 10 or 5 modifications of an amino acid sequence of SEQ ID NO:14 or SEQ ID NO: 16, or a sequence with 95-99% identity to an amino acid sequence of SEQ ID NO:14 or SEQ ID NO: 16. In one embodiment, the encoded costimulatory signaling domain comprises a sequence of SEQ ID NO: 14 or SEQ ID NO: 16. In other embodiments, the intracellular domain of the CAR polypeptide molecule comprises the sequence of SEQ ID NO: 14 or SEQ ID NO: 16, and the sequence of SEQ ID NO: 18 or SEQ ID NO: 20, wherein the sequences comprising the intracellular signaling domain are expressed in the same frame and as a single polypeptide chain.

In some embodiments, the CAR polypeptide molecule further comprises a leader sequence. In one embodiment, the leader sequence comprises the sequence of SEQ ID NO: 2.

In certain embodiments, the antigen binding domain of the CAR polypeptide molecule has a binding affinity KD of 10⁻⁴ M to 10⁻⁸ M. In one embodiment, the antigen binding domain is an antigen binding domain described herein, e.g., an antigen binding domain described herein for a target provided above. In one embodiment, the CAR molecule comprises an antigen binding domain that has a binding affinity KD of 10⁻⁴ M to 10⁻⁸ M, e.g., 10⁻⁵ M to 10⁻⁷ M, e.g., 10⁻⁶ M or 10⁻⁷ M, for the target antigen. In one embodiment, the antigen binding domain has a binding affinity that is at least five-fold, 10-fold, 20-fold, 30-fold, 50-fold, 100-fold or 1,000-fold less than a reference antibody, e.g., an antibody described herein. In one embodiment, the encoded antigen binding domain has a binding affinity at least 5-fold less than a reference antibody (e.g., an antibody from which the antigen binding domain is derived).

In another aspect, the invention employs an isolated CAR polypeptide molecule comprising an antigen binding domain, a transmembrane domain, and an intracellular signaling domain, wherein said antigen binding domain binds to a tumor-supporting antigen (e.g., a tumor-supporting antigen as described herein). In some embodimentsinstances, the tumor-supporting antigen is an antigen present on a stromal cell or a myeloid-derived suppressor cell (MDSC).

### CAR-expressing cells

In another aspect, the disclosure pertains to a cell, e.g., an immune effector cell, (e.g., a population of cells, e.g., a population of immune effector cells) comprising a nucleic acid molecule, a CAR polypeptide molecule, or a vector as described herein. The present invention provides an immune effector cell in accordance with the appended claims.

In one embodiment, the cell is a human T cell. In one embodiment, the cell is a cell described herein, e.g., a human T cell, e.g., a human T cell described herein; or a human NK cell, e.g., a human NK cell described herein. In one embodiment, the human T cell is a CD8+ T cell. In one embodiment, the cell is a T cell and the T cell is diaglycerol kinase (DGK) deficient. In one embodiment, the cell is a T cell and the T cell is Ikaros deficient. In one embodiment, the cell is a T cell and the T cell is both DGK and Ikaros deficient.

In another embodiment, a CAR-expressing immune effector cell can further express another agent, e.g., an agent which enhances the activity of a CAR-expressing cell. For example, in one embodiment, the agent can be an agent which inhibits an inhibitory molecule. Examples of inhibitory molecules include PD-1, PD-L1, CTLA-4, TIM-3, CEACAM (e.g., CEACAM-1, CEACAM-3 and/or CEACAM-5), LAG-3, VISTA, BTLA, TIGIT, LAIR1, CD160, 2B4 and TGFR beta, e.g., as described herein. In one embodiment, the agent that inhibits an inhibitory molecule comprises a first polypeptide, e.g., an inhibitory molecule, associated with a second polypeptide that provides a positive signal to the cell, e.g., an intracellular signaling domain described herein. In one embodiment, the agent comprises a first polypeptide, e.g., of an inhibitory molecule such as PD-1, PD-L1, CTLA-4, TIM-3, CEACAM (e.g., CEACAM-1, CEACAM-3 and/or CEACAM-5), LAG-3, VISTA, BTLA, TIGIT, LAIR1, CD160, 2B4 or TGFR beta, or a fragment of any of these, and a second polypeptide which is an intracellular signaling domain described herein (e.g., comprising a costimulatory domain (e.g., 41BB, CD27 or CD28, e.g., as described herein) and/or a primary signaling domain (e.g., a CD3 zeta signaling domain described herein). In one embodiment, the agent comprises a first polypeptide of PD-1 or a fragment thereof, and a second polypeptide of an intracellular signaling domain described herein (e.g., a CD28, CD27, OX40 or 4-IBB signaling domain described herein and/or a CD3 zeta signaling domain described herein).

In the present invention, the CAR-expressing immune effector cell further comprises a second CAR, e.g., a second CAR that includes a different antigen binding domain, e.g., to the same target or a different target. In one embodiment, the second CAR includes an antigen binding domain to a target expressed on the same cancer cell type as the target of the first CAR. In one embodiment, the CAR-expressing immune effector cell comprises a first CAR that targets a first antigen and includes an intracellular signaling domain having a costimulatory signaling domain but not a primary signaling domain, and a second CAR that targets a second, different, antigen and includes an intracellular signaling domain having a primary signaling domain but not a costimulatory signaling domain.

While not wishing to be bound by theory, placement of a costimulatory signaling domain, e.g., 4-1BB, CD28, CD27 or OX-40, onto the first CAR, and the primary signaling domain, e.g., CD3 zeta, on the second CAR can limit the CAR activity to cells where both targets are expressed. In one embodiment, the CAR expressing immune effector cell comprises a first CAR that includes an antigen binding domain, a transmembrane domain and a costimulatory domain and a second CAR that targets an antigen other than antigen targeted by the first CAR (e.g., an antigen expressed on the same cancer cell type as the first target) and includes an antigen binding domain, a transmembrane domain and a primary signaling domain. In another embodiment, the CAR expressing immune effector cell comprises a first CAR that includes an antigen binding domain, a transmembrane domain and a primary signaling domain and a second CAR that targets an antigen other than antigen targeted by the first CAR (e.g., an antigen expressed on the same cancer cell type as the first target) and includes an antigen binding domain to the antigen, a transmembrane domain and a costimulatory signaling domain.

In one embodiment, the CAR-expressing immune effector cell comprises a CAR, e.g., a CAR to a target described above, and an inhibitory CAR. In one embodiment, the inhibitory CAR comprises an antigen binding domain that binds an antigen found on normal cells but not cancer cells, e.g., normal cells that also express the target. In one embodiment, the inhibitory CAR comprises the antigen binding domain, a transmembrane domain and an intracellular domain of an inhibitory molecule. For example, the intracellular domain of the inhibitory CAR can be an intracellular domain of PD1, PD-L1, CTLA-4, TIM-3, CEACAM (e.g., CEACAM-1, CEACAM-3 and/or CEACAM-5), LAG-3, VISTA, BTLA, TIGIT, LAIR1, CD160, 2B4 or TGFR beta.

In one embodiment, an immune effector cell (e.g., T cell, NK cell) comprises a first CAR comprising an antigen binding domain that binds to a tumor antigen as described herein, and a second CAR comprising a PD1 extracellular domain or a fragment thereof.

In one embodiment, the cell further comprises an inhibitory molecule comprising: an inhKIR cytoplasmic domain; a transmembrane domain, e.g., a KIR transmembrane domain; and an inhibitor cytoplasmic domain, e.g., an ITIM domain, e.g., an inhKIR ITIM domain. In an embodiment the inhibitory molecule is a naturally occurring inhKIR, or a sequence sharing at least 50, 60, 70, 80, 85, 90, 95, or 99% homology with, or that differs by no more than 1, 2, 3, 4, 5, 6, 7, 8, 9, 10, 15, or 20 residues from, a naturally occurring inhKIR.

In one embodiment, the cell further comprises an inhibitory molecule comprising: a SLAM family cytoplasmic domain; a transmembrane domain, e.g., a SLAM family transmembrane domain; and an inhibitor cytoplasmic domain, e.g., a SLAM family domain, e.g., an SLAM family ITIM domain. In an embodiment the inhibitory molecule is a naturally occurring SLAM family member, or a sequence sharing at least 50, 60, 70, 80, 85, 90, 95, or 99% homology with, or that differs by no more than 1, 2, 3, 4, 5, 6, 7, 8, 9, 10, 15, or 20 residues from, a naturally occurring SLAM family member.

In one embodiment, the second CAR in the cell is an inhibitory CAR, wherein the inhibitory CAR comprises an antigen binding domain, a transmembrane domain, and an intracellular domain of an inhibitory molecule. The inhibitory molecule can be chosen from one or more of: PD1, PD-L1, CTLA-4, TIM-3, LAG-3, VISTA, BTLA, TIGIT, LAIR1, CD160, 2B4, TGFR beta, CEACAM-1, CEACAM-3, and CEACAM-5. In one embodiment, the second CAR molecule comprises the extracellular domain of PD1 or a fragment thereof.

In embodiments, the second CAR molecule in the cell further comprises an intracellular signaling domain comprising a primary signaling domain and/or an intracellular signaling domain.

In other embodiments, the intracellular signaling domain in the cell comprises a primary signaling domain comprising the functional domain of CD3 zeta and a costimulatory signaling domain comprising the functional domain of 4-1BB.

In one embodiment, the second CAR molecule in the cell comprises the amino acid sequence of SEQ ID NO: 26.

In certain embodiments, the antigen binding domain of the second CAR molecule comprises a scFv and the antigen binding domain of the first CAR molecule comprises a camelid VHH domain.

### Methods of treatment/Combination therapies

In another aspect, the present disclosure provides a method comprising administering a CAR molecule, e.g., a CAR molecule described herein, or a cell comprising a nucleic acid encoding a CAR molecule, e.g., a CAR molecule described herein. In one instance, the subject has a disorder described herein, e.g., the subject has cancer, e.g., the subject has a cancer which expresses a target antigen described herein. In one instance, the subject is a human.

In another aspect, the disclosure pertains to a method of treating a subject having a disease associated with expression of a cancer associated antigen as described herein comprising administering to the subject an effective amount of a cell comprising a CAR molecule, e.g., a CAR molecule described herein.

In yet another aspect, the disclosure features a method of treating a subject having a disease associated with expression of a tumor antigen, comprising administering to the subject an effective amount of a cell, e.g., an immune effector cell *(e.g.,* a population of immune effector cells) comprising a CAR molecule, wherein the CAR molecule comprises an antigen binding domain, a transmembrane domain, and an intracellular domain, said intracellular domain comprises a costimulatory domain and/or a primary signaling domain, wherein said antigen binding domain binds to the tumor antigen associated with the disease, e.g. a tumor antigen as described herein.

In a related aspect, the disclosure features a method of treating a subject having a disease associated with expression of a tumor antigen. The method comprises administering to the subject an effective amount of a cell, e.g., an immune effector cell *(e.g.,* a population of immune effector cells) comprising a CAR molecule, in combination with an agent that increases the efficacy of the immune cell, wherein:
(i) the CAR molecule comprises an antigen binding domain, a transmembrane domain, and an intracellular domain comprising a costimulatory domain and/or a primary signaling domain, wherein said antigen binding domain binds to the tumor antigen associated with the disease, e.g. a tumor antigen as discosed herein; and
(ii) the agent that increases the efficacy of the immune cell is chosen from one or more of:
   (i) a protein phosphatase inhibitor;
   (ii) a kinase inhibitor;
   (iii) a cytokine;
   (iv) an inhibitor of an immune inhibitory molecule; or
   (v) an agent that decreases the level or activity of a T_{REG} cell.

In a related aspect, the disclosure features a method of treating a subject having a disease associated with expression of a tumor antigen, comprising administering to the subject an effective amount of a cell, e.g., an immune effector cell *(e.g.,* a population of immune effector cells) comprising a CAR molecule, wherein:
(i) the CAR molecule comprises an antigen binding domain, a transmembrane domain, and an intracellular domain comprising a costimulatory domain and/or a primary signaling domain, wherein said antigen binding domain binds to the tumor antigen associated with the disease, e.g., a tumor antigen as disclosed herein; and
(ii) the antigen binding domain of the CAR molecule has a binding affinity at least 5-fold less than an antibody from which the antigen binding domain is derived.

In another aspect, the disclosure features a composition comprising an immune effector cell *(e.g.,* a population of immune effector cells) comprising a CAR molecule (e.g., a CAR molecule as described herein) for use in the treatment of a subject having a disease associated with expression of a tumor antigen, e.g., a disorder as described herein. The present invention provides an immune effector cell as defined in the claims for use as a medicament. The present invention also provides an immune effector cell as defined in the claims for use in the treatment of a disease expressing a tumor antigen.

In certain embodiments of the aforesaid uses, the disease associated with a tumor antigen, e.g., a tumor antigen described herein, is selected from a proliferative disease such as a cancer or malignancy or a precancerous condition such as a myelodysplasia, a myelodysplastic syndrome or a preleukemia, or is a non-cancer related indication associated with expression of a tumor antigen described herein. In one embodiment, the disease is a cancer described herein, e.g., a cancer described herein as being associated with a target described herein. In one embodiment, the disease is a hematologic cancer. In one embodiment, the hematologic cancer is leukemia. In one embodiment, the cancer is selected from the group consisting of one or more acute leukemias including but not limited to B-cell acute lymphoid leukemia ("BALL"), T-cell acute lymphoid leukemia ("TALL"), acute lymphoid leukemia (ALL); one or more chronic leukemias including but not limited to chronic myelogenous leukemia (CML), chronic lymphocytic leukemia (CLL); additional hematologic cancers or hematologic conditions including, but not limited to B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative conditions, MALT lymphoma, mantle cell lymphoma, Marginal zone lymphoma, multiple myeloma, myelodysplasia and myelodysplastic syndrome, non-Hodgkin lymphoma, Hodgkin lymphoma, plasmablastic lymphoma, plasmacytoid dendritic cell neoplasm, Waldenstrom macroglobulinemia, and "preleukemia" which are a diverse collection of hematological conditions united by ineffective production (or dysplasia) of myeloid blood cells, and to disease associated with expression of a tumor antigen described herein include, but not limited to, atypical and/or non-classical cancers, malignancies, precancerous conditions or proliferative diseases expressing a tumor antigen as described herein; and any combination thereof. In another embodiment, the disease associated with a tumor antigen described herein is a solid tumor.

In certain embodiments of the aforesaid uses, the tumor antigen associated with the disease is chosen from one or more of: CD19, CD123, CD22, CD30, CD171, CS-1, CLL-1 (CLECL1), CD33, EGFRvIII, GD2, GD3, BCMA, Tn Ag, PSMA, ROR1, FLT3, TAG72, CD38, CD44v6, CEA, EPCAM, B7H3, KIT, IL-13Ra2, Mesothelin, IL-11Ra, PSCA, PRSS21, VEGFR2, LewisY, CD24, PDGFR-beta, SSEA-4, CD20, Folate receptor alpha, ERBB2 (Her2/neu), MUC1, EGFR, NCAM, Prostase, PAP, ELF2M, Ephrin B2, FAP, IGF-I receptor, CAIX, LMP2, gp100, bcr-abl, tyrosinase, EphA2, Fucosyl GM1, sLe, GM3, TGS5, HMWMAA, o-acetyl-GD2, Folate receptor beta, TEM1/CD248, TEM7R, CLDN6, TSHR, GPRC5D, CXORF61, CD97, CD179a, ALK, Polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, MAGE A1, ETV6-AML, sperm protein 17, XAGE1, Tie 2, MAD-CT-1, MAD-CT-2, Fos-related antigen 1, p53, p53 mutant, prostein, survivin and telomerase, PCTA-1/Galectin 8, MelanA/MART1, Ras mutant, hTERT, sarcoma translocation breakpoints, ML-IAP, ERG (TMPRSS2 ETS fusion gene), NA17, PAX3, Androgen receptor, Cyclin B1, MYCN, RhoC, TRP-2, CYP1B1, BORIS, SART3, PAX5, OY-TES1, LCK, AKAP-4, SSX2, RAGE-1, human telomerase reverse transcriptase, RU1, RU2, legumain, HPV E6, E7, intestinal carboxyl esterase, mut hsp70-2, CD79a, CD79b, CD72, LAIR1, FCAR, LILRA2, CD300LF, CLEC12A, BST2, EMR2, LY75, GPC3, FCRL5, and IGLL1.

In other embodiments of the aforesaid uses, the tumor antigen associated with the disease is chosen from one or more of: TSHR, TSHR, CD171, CS-1, CLL-1, GD3, Tn Ag, FLT3, CD38, CD44v6, B7H3, KIT, IL-13Ra2, IL-11Ra, PSCA, PRSS21, VEGFR2, LewisY, CD24, PDGFR-beta, SSEA-4, MUC1, EGFR, NCAM, CAIX, LMP2, EphA2, Fucosyl GM1, sLe, GM3, TGS5, HMWMAA, o-acetyl-GD2, Folate receptor beta, TEM1/CD248, TEM7R, CLDN6, GPRC5D, CXORF61, CD97, CD179a, ALK, Polysialic acid, PLAC1, GloboH, NY-BR-1, UPK2, HAVCR1, ADRB3, PANX3, GPR20, LY6K, OR51E2, TARP, WT1, ETV6-AML, sperm protein 17, XAGE1, Tie 2, MAD-CT-1, MAD-CT-2, Fos-related antigen 1, p53 mutant, hTERT, sarcoma translocation breakpoints, ML-IAP, ERG (TMPRSS2 ETS fusion gene), NA17, PAX3, Androgen receptor, Cyclin B1, MYCN, RhoC, CYP1B1, BORIS, SART3, PAX5, OY-TES1, LCK, AKAP-4, SSX2, CD79a, CD79b, CD72, LAIR1, FCAR, LILRA2, CD300LF, CLEC12A, BST2, EMR2, LY75, GPC3, FCRL5, and IGLL1.

In other embodiments of the aforesaid uses, the tumor antigen associated with the disease is chosen from one or more of: TSHR, CLDN6, GPRC5D, CXORF61, CD97, CD179a, ALK, Polysialic acid, PLAC1, GloboH, NY-BR-1, UPK2, HAVCR1, ADRB3, PANX3, GPR20, LY6K, and OR51E2.

In certain embodiments, the uses are carried out in combination with an agent that increases the efficacy of the immune effector cell, e.g., an agent as described herein.

In any of the aforesaid uses, the disease associated with expression of the tumor antigen is selected from the group consisting of a proliferative disease, a precancerous condition, a cancer, and a non-cancer related indication associated with expression of the tumor antigen.

The cancer can be a hematologic cancer, e.g., a cancer chosen from one or more of chronic lymphocytic leukemia (CLL), acute leukemias, acute lymphoid leukemia (ALL), B-cell acute lymphoid leukemia (B-ALL), T-cell acute lymphoid leukemia (T-ALL), chronic myelogenous leukemia (CML), B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative conditions, MALT lymphoma, mantle cell lymphoma, marginal zone lymphoma, multiple myeloma, myelodysplasia and myelodysplastic syndrome, non-Hodgkin's lymphoma, Hodgkin's lymphoma, plasmablastic lymphoma, plasmacytoid dendritic cell neoplasm, Waldenstrom macroglobulinemia, or pre-leukemia.

The cancer can also be chosen from colon cancer, rectal cancer, renal-cell carcinoma, liver cancer, non-small cell carcinoma of the lung, cancer of the small intestine, cancer of the esophagus, melanoma, bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, Hodgkin's Disease, non-Hodgkin's lymphoma, cancer of the endocrine system, cancer of the thyroid gland, cancer of the parathyroid gland, cancer of the adrenal gland, sarcoma of soft tissue, cancer of the urethra, cancer of the penis, solid tumors of childhood, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally induced cancers, combinations of said cancers, and metastatic lesions of said cancers.

In certain embodiments of the uses, the CAR molecule is administered in combination with an agent that increases the efficacy of the immune effector cell, e.g., one or more of a protein phosphatase inhibitor, a kinase inhibitor, a cytokine, an inhibitor of an immune inhibitory molecule; or an agent that decreases the level or activity of a T_{REG} cell.

In certain embodiments, the protein phosphatase inhibitor is a SHP-1 inhibitor and/or an SHP-2 inhibitor.

In other embodiments, kinase inhibitor is chosen from one or more of a CDK4 inhibitor, a CDK4/6 inhibitor (e.g., palbociclib), a BTK inhibitor (e.g., ibrutinib or RN-486), an mTOR inhibitor (e.g., rapamycin or everolimus (RAD001)), an MNK inhibitor, or a dual P13K/mTOR inhibitor. In one embodiment, the BTK inhibitor does not reduce or inhibit the kinase activity of interleukin-2-inducible kinase (ITK).

In other embodiments, the agent that inhibits the immune inhibitory molecule comprises an antibody or antibody fragment, an inhibitory nucleic acid, a clustered regularly interspaced short palindromic repeats (CRISPR), a transcription-activator like effector nuclease (TALEN), or a zinc finger endonuclease (ZFN) that inhibits the expression of the inhibitory molecule.

In other embodiments, the agent that decreases the level or activity of the T_{REG} cells is chosen from cyclophosphamide, anti-GITR antibody, CD25-depletion, or a combination thereof.

In certain embodiments, the immune inhibitory molecule is selected from the group consisting of PD1, PD-L1, CTLA-4, TIM-3, LAG-3, VISTA, BTLA, TIGIT, LAIR1, CD160, 2B4, TGFR beta, CEACAM-1, CEACAM-3, and CEACAM-5.

In other embodiments, the agent that inhibits the inhibitory molecule comprises a first polypeptide comprising an inhibitory molecule or a fragment thereof and a second polypeptide that provides a positive signal to the cell, and wherein the first and second polypeptides are expressed on the CAR-containing immune cells, wherein (i) the first polypeptide comprises PD1, PD-L1, CTLA-4, TIM-3, LAG3, VISTA, BTLA, TIGIT, LAIR1, CD160, 2B4, TGFR beta, CEACAM-1, CEACAM-3, and CEACAM-5 or a fragment thereof; and/or (ii) the second polypeptide comprises an intracellular signaling domain comprising a primary signaling domain and/or a costimulatory signaling domain. In one embodiment, the primary signaling domain comprises a functional domain of CD3 zeta; and/or the costimulatory signaling domain comprises a functional domain of a protein selected from 41BB, CD27 and CD28.

In other embodiments, cytokine is chosen from IL-7, IL-15 or IL-21, or both.

In other embodiments, the immune effector cell comprising the CAR molecule and a second, e.g., any of the combination therapies disclosed herein (e.g., the agent that that increases the efficacy of the immune effector cell) are administered substantially simultaneously or sequentially.

In other embodiments, the immune cell comprising the CAR molecule is administered in combination with a molecule that targets GITR and/or modulates GITR function. In certain embodiments, the molecule targeting GITR and/or modulating GITR function is administered prior to the CAR-expressing cell or population of cells, or prior to apheresis.

In one embodiment, lymphocyte infusion, for example allogeneic lymphocyte infusion, is used in the treatment of the cancer, wherein the lymphocyte infusion comprises at least one CAR-expressing cell of the present invention. In one embodiment, autologous lymphocyte infusion is used in the treatment of the cancer, wherein the autologous lymphocyte infusion comprises at least one CAR-expressing cell described herein.

In one embodiment, the cell is a T cell and the T cell is diaglycerol kinase (DGK) deficient. In one embodiment, the cell is a T cell and the T cell is Ikaros deficient. In one embodiment, the cell is a T cell and the T cell is both DGK and Ikaros deficient.

In one embodiment, a cell expressing the CAR molecule is administered in combination with an agent which enhances the activity of a CAR-expressing cell, wherein the agent is a cytokine, e.g., IL-7, IL-15, IL-21, or a combination thereof. The cytokine can be delivered in combination with, e.g., simultaneously or shortly after, administration of the CAR-expressing cell. Alternatively, the cytokine can be delivered after a prolonged period of time after administration of the CAR-expressing cell, e.g., after assessment of the subject's response to the CAR-expressing cell. In one embodiment the cytokine is administered to the subject simultaneously (e.g., administered on the same day) with or shortly after administration (e.g., administered 1 day, 2 days, 3 days, 4 days, 5 days, 6 days, or 7 days after administration) of the cell or population of cells of any of claims 61-80. In other embodiments, the cytokine is administered to the subject after a prolonged period of time (e.g., e.g., at least 2 weeks, 3 weeks, 4 weeks, 6 weeks, 8 weeks, 10 weeks, or more) after administration of the cell or population of cells of any of claims 61-80, or after assessment of the subject's response to the cell.

In other embodiments, the cells expressing a CAR molecule are administered in combination with an agent that ameliorates one or more side effects associated with administration of a cell expressing a CAR molecule. Side effects associated with the CAR-expressing cell can be chosen from cytokine release syndrome (CRS) or hemophagocytic lymphohistiocytosis (HLH).

In embodiments of any of the aforeseaid uses, the cells expressing the CAR molecule are administered in combination with an agent that treats the disease associated with expression of the tumor antigen, e.g., any of the second or third therapies disclosed herein. Additional exemplary combinations include one or more of the following.

In another embodiment, the cell expressing the CAR molecule can be administered in combination with another agent, e.g., a kinase inhibitor and/or checkpoint inhibitor described herein. In an embodiment, a cell expressing the CAR molecule can further express another agent, e.g., an agent which enhances the activity of a CAR-expressing cell.

For example, in one embodiment, the agent that enhances the activity of a CAR-expressing cell can be an agent which inhibits an inhibitory molecule (e.g., an immune inhibitor molecule). Examples of inhibitory molecules include PD1, PD-L1, CTLA-4, TIM-3, CEACAM (e.g., CEACAM-1, CEACAM-3 and/or CEACAM-5), LAG-3, VISTA, BTLA, TIGIT, LAIR1, CD160, 2B4 and TGFR beta.

In one embodiment, the agent that inhibits the inhibitory molecule is an inhibitory nucleic acid is a dsRNA, a siRNA, or a shRNA. In embodiments, the inhibitory nucleic acid is linked to the nucleic acid that encodes a component of the CAR molecule. For example, the inhibitory molecule can be expressed on the CAR-expressing cell.

In another embodiment, the agent which inhibits an inhibitory molecule, e.g., is a molecule described herein, e.g., an agent that comprises a first polypeptide, e.g., an inhibitory molecule, associated with a second polypeptide that provides a positive signal to the cell, e.g., an intracellular signaling domain described herein. In one embodiment, the agent comprises a first polypeptide, e.g., of an inhibitory molecule such as PD-1, PD-L1, CTLA-4, TIM-3, CEACAM (e.g., CEACAM-1, CEACAM-3 and/or CEACAM-5), LAG-3, VISTA, BTLA, TIGIT, LAIR1, CD160, 2B4 or TGFR beta, or a fragment of any of these (e.g., at least a portion of the extracellular domain of any of these), and a second polypeptide which is an intracellular signaling domain described herein (e.g., comprising a costimulatory domain (e.g., 41BB, CD27 or CD28, e.g., as described herein) and/or a primary signaling domain (e.g., a CD3 zeta signaling domain described herein). In one embodiment, the agent comprises a first polypeptide of PD1 or a fragment thereof (e.g., at least a portion of the extracellular domain of PD1), and a second polypeptide of an intracellular signaling domain described herein (e.g., a CD28 signaling domain described herein and/or a CD3 zeta signaling domain described herein).

In one embodiment, the CAR-expressing immune effector cell of the present invention, e.g., T cell or NK cell, is administered to a subject that has received a previous stem cell transplantation, e.g., autologous stem cell transplantation.

In one embodiment, the CAR-expressing immune effector cell of the present invention, e.g., T cell or NK cells, is administered to a subject that has received a previous dose of melphalan.

In one embodiment, the cell expressing a CAR molecule is administered in combination with an agent that increases the efficacy of a cell expressing a CAR molecule, e.g., an agent described herein.

In one embodiment, the cells expressing a CAR molecule are administered in combination with a low, immune enhancing dose of an mTOR inhibitor. While not wishing to be bound by theory, it is believed that treatment with a low, immune enhancing, dose (e.g., a dose that is insufficient to completely suppress the immune system but sufficient to improve immune function) is accompanied by a decrease in PD-1 positive T cells or an increase in PD-1 negative cells. PD-1 positive T cells, but not PD-1 negative T cells, can be exhausted by engagement with cells which express a PD-1 ligand, e.g., PD-L1 or PD-L2.

In an embodiment this approach can be used to optimize the performance of CAR cells in the subject. While not wishing to be bound by theory, it is believed that, in an embodiment, the performance of endogenous, non-modified immune effector cells, e.g., T cells or NK cells, is improved. While not wishing to be bound by theory, it is believed that, in an embodiment, the performance of a target antigen CAR- expressing cell is improved. In other embodiments, cells, e.g., T cells or NK cells, which have, or will be engineered to express a CAR, can be treated ex vivo by contact with an amount of an mTOR inhibitor that increases the number of PD1 negative immune effector cells, e.g., T cells or increases the ratio of PD1 negative immune effector cells, e.g., T cells/ PD1 positive immune effector cells, e.g., T cells.

In an embodiment, administration of a low, immune enhancing, dose of an mTOR inhibitor, e.g., an allosteric inhibitor, e.g., RAD001, or a catalytic inhibitor, is initiated prior to administration of an CAR expressing cell described herein, e.g., T cells or NK cells. In an embodiment, the CAR cells are administered after a sufficient time, or sufficient dosing, of an mTOR inhibitor, such that the level of PD1 negative immune effector cells, e.g., T cells or NK cells, or the ratio ofPD1 negative immune effector cells, e.g., T cells/ PD1 positive immune effector cells, e.g., T cells, has been, at least transiently, increased.

The cell, e.g., T cell or NK cell, to be engineered to express a CAR, can be harvested after a sufficient time, or after sufficient dosing of the low, immune enhancing, dose of an mTOR inhibitor, such that the level of PD1 negative immune effector cells, e.g., T cells, or the ratio of PD1 negative immune effector cells, e.g., T cells/ PD1 positive immune effector cells, e.g., T cells, in the subject or harvested from the subject has been, at least transiently, increased.

In one embodiment, the cell expressing a CAR molecule is administered in combination with an agent that ameliorates one or more side effect associated with administration of a cell expressing a CAR molecule, e.g., an agent described herein.

In one embodiment, the cell expressing a CAR molecule is administered in combination with an agent that treats the disease associated with a cancer associated antigen as described herein, e.g., an agent described herein.

In one embodiment, a cell expressing two or more CAR molecules is administered to a subject in need thereof to treat cancer. In one embodiment, a population of cells including a CAR expressing cell is administered to a subject in need thereof to treat cancer.

In one embodiment, the cell expressing a CAR molecule is administered at a dose and/or dosing schedule described herein.

In one embodiment, the CAR molecule is introduced into immune effector cells (e.g., T cells, NK cells), e.g., using *in vitro* transcription, and the subject (e.g., human) receives an initial administration of cells comprising a CAR molecule, and one or more subsequent administrations of cells comprising a CAR molecule, wherein the one or more subsequent administrations are administered less than 15 days, e.g., 14, 13, 12, 11, 10, 9, 8, 7, 6, 5, 4, 3, or 2 days after the previous administration. In one embodiment, more than one administration of cells comprising a CAR molecule are administered to the subject (e.g., human) per week, e.g., 2, 3, or 4 administrations of cells comprising a CAR molecule are administered per week. In one embodiment, the subject (e.g., human subject) receives more than one administration of cells comprising a CAR molecule per week (e.g., 2, 3 or 4 administrations per week) (also referred to herein as a cycle), followed by a week of no administration of cells comprising a CAR molecule, and then one or more additional administration of cells comprising a CAR molecule (e.g., more than one administration of the cells comprising a CAR molecule per week) is administered to the subject. In another embodiment, the subject (e.g., human subject) receives more than one cycle of cells comprising a CAR molecule, and the time between each cycle is less than 10, 9, 8, 7, 6, 5, 4, or 3 days. In one embodiment, the cells comprising a CAR molecule are administered every other day for 3 administrations per week. In one embodiment, the cells comprising a CAR molecule are administered for at least two, three, four, five, six, seven, eight or more weeks.

In one embodiment, the cells expressing a CAR molecule are administered as a first line treatment for the disease, e.g., the cancer, e.g., the cancer described herein. In another embodiment, the cells expressing a CAR molecule are administered as a second, third, fourth line treatment for the disease, e.g., the cancer, e.g., the cancer described herein.

In one embodiment, a population of cells of the invention is administered.

In another aspect, the disclosure pertains to the isolated nucleic acid molecule encoding a CAR of the disclosure, the isolated polypeptide molecule of a CAR of the disclosure, the vector comprising a CAR of the disclosure, and the cell comprising a CAR of the disclosure for use as a medicament.

In another aspect, the disclosure pertains to a the isolated nucleic acid molecule encoding a CAR of the disclosure, the isolated polypeptide molecule of a CAR of the disclosure, the vector comprising a CAR of the disclosure, and the cell comprising a CAR of the disclosure for use in the treatment of a disease expressing a cancer associated antigen as described herein.

In another aspect, the invention pertains to a cell expressing a CAR molecule as defined in the claims for use as a medicament in combination with a cytokine, e.g., IL-7, IL-15 and/or IL-21 as described herein. In another aspect, the disclosure pertains to a cytokine described herein for use as a medicament in combination with a cell expressing a CAR molecule described herein.

In another aspect, the invention pertains to a cell expressing a CAR molecule as defined in the claims for use as a medicament in combination with a kinase inhibitor and/or a checkpoint inhibitor as described herein. In another aspect, the disclosure pertains to a kinase inhibitor and/or a checkpoint inhibitor described herein for use as a medicament in combination with a cell expressing a CAR molecule described herein.

In another aspect, the invention pertains to a cell expressing a CAR molecule as defined in the claims for use in combination with a cytokine, e.g., IL-7, IL-15 and/or IL-21 as described herein, in the treatment of a disease expressing a tumor antigen targeted by the CAR. In another aspect, the disclosure pertains to a cytokine described herein for use in combination with a cell expressing a CAR molecule described herein, in the treatment of a disease expressing a tumor antigen targeted by the CAR

In another aspect, the invention pertains to a cell expressing a CAR molecule as defined in the claims for use in combination with a kinase inhibitor and/or a checkpoint inhibitor as described herein, in the treatment of a disease expressing a tumor antigen targeted by the CAR. In another aspect, the disclosure pertains to a kinase inhibitor and/or a checkpoint inhibitor described herein for use in combination with a cell expressing a CAR molecule described herein, in the treatment of a disease expressing a tumor antigen targeted by the CAR

In another aspect, the present disclosure provides a method comprising administering a CAR molecule, e.g., a CAR molecule described herein, or a cell comprising a nucleic acid encoding a CAR molecule, e.g., a CAR molecule described herein. In one instance, the subject has a disorder described herein, e.g., the subject has cancer, e.g., the subject has a cancer and has tumor-supporting cells which express a tumor-supporting antigen described herein. In one instance, the subject is a human.

In another aspect, the disclosure pertains to a method of treating a subject having a disease associated with expression of a tumor-supporting antigen as described herein comprising administering to the subject an effective amount of a cell comprising a CAR molecule, e.g., a CAR molecule described herein.

In yet another aspect, the disclosure features a method of treating a subject having a disease associated with expression of a tumor-supporting antigen, comprising administering to the subject an effective amount of a cell, e.g., an immune effector cell (*e.g.*, a population of immune effector cells) comprising a CAR molecule, wherein the CAR molecule comprises an antigen binding domain, a transmembrane domain, and an intracellular domain, said intracellular domain comprises a costimulatory domain and/or a primary signaling domain, wherein said antigen binding domain binds to the tumor-supporting antigen associated with the disease, e.g. a tumor-supporting antigen as described herein.

In a related aspect, the disclosure features a method of treating a subject having a disease associated with expression of a tumor-supporting antigen. The method comprises administering to the subject an effective amount of a cell, e.g., an immune effector cell (*e.g.*, a population of immune effector cells) comprising a CAR molecule, in combination with an agent that increases the efficacy of the immune cell, wherein:
(i) the CAR molecule comprises an antigen binding domain, a transmembrane domain, and an intracellular domain comprising a costimulatory domain and/or a primary signaling domain, wherein said antigen binding domain binds to the tumor-supporting antigen associated with the disease, e.g. a tumor-supporting antigen as disclosed herein; and
(ii) the agent that increases the efficacy of the immune cell is chosen from one or more of:
   (i) a protein phosphatase inhibitor;
   (ii) a kinase inhibitor;
   (iii) a cytokine;
   (iv) an inhibitor of an immune inhibitory molecule; or
   (v) an agent that decreases the level or activity of a T_{REG} cell.

In a related aspect, the disclosure features a method of treating a subject having a disease associated with expression of a tumor-supporting antigen, comprising administering to the subject an effective amount of a cell, e.g., an immune effector cell *(e.g.,* a population of immune effector cells) comprising a CAR molecule, wherein:
(i) the CAR molecule comprises an antigen binding domain, a transmembrane domain, and an intracellular domain comprising a costimulatory domain and/or a primary signaling domain, wherein said antigen binding domain binds to the tumor-supporting antigen associated with the disease, e.g., a tumor-supporting antigen as disclosed herein; and
(ii) the antigen binding domain of the CAR molecule has a binding affinity at least 5-fold less than an antibody from which the antigen binding domain is derived.

In another aspect, the invention features a composition comprising an immune effector cell (e.g., a population of immune effector cells) as defined in the claims for use in the treatment of a subject having a disease associated with expression of a tumor-supporting antigen, e.g., a disorder as described herein.

In any of the uses of the invention, the disease associated with expression of the tumor-supporting antigen is selected from the group consisting of a proliferative disease, a precancerous condition, a cancer, and a non-cancer related indication associated with expression of the tumor-supporting antigen. In an embodiment, the disease associated with a tumor-supporting antigen described herein is a solid tumor.

In one embodiment of the uses, the CAR molecule is administered in combination with another agent. In one embodiment, the agent can be a kinase inhibitor, e.g., a CDK4/6 inhibitor, a BTK inhibitor, an mTOR inhibitor, a MNK inhibitor, or a dual PI3K/mTOR inhibitor, and combinations thereof. In one embodiment, the kinase inhibitor is a CDK4 inhibitor, e.g., a CDK4 inhibitor described herein, e.g., a CD4/6 inhibitor, such as, e.g., 6-Acetyl-8-cyclopentyl-5-methyl-2-(5-piperazin-1-yl-pyridin-2-ylamino)-8H-pyrido[2,3-d]pyrimidin-7-one, hydrochloride (also referred to as palbociclib or PD0332991). In one embodiment, the kinase inhibitor is a BTK inhibitor, e.g., a BTK inhibitor described herein, such as, e.g., ibrutinib. In one embodiment, the kinase inhibitor is an mTOR inhibitor, e.g., an mTOR inhibitor described herein, such as, e.g., rapamycin, a rapamycin analog, OSI-027. The mTOR inhibitor can be, e.g., an mTORC1 inhibitor and/or an mTORC2 inhibitor, e.g., an mTORC1 inhibitor and/or mTORC2 inhibitor described herein. In one embodiment, the kinase inhibitor is a MNK inhibitor, e.g., a MNK inhibitor described herein, such as, e.g., 4-amino-5-(4-fluoroanilino)-pyrazolo [3,4-d] pyrimidine. The MNK inhibitor can be, e.g., a MNK1a, MNK1b, MNK2a and/or MNK2b inhibitor. The dual PI3K/mTOR inhibitor can be, e.g., PF-04695102.

In one embodiment of the uses, the kinase inhibitor is a CDK4 inhibitor selected from aloisine A; flavopiridol or HMR-1275, 2-(2-chlorophenyl)-5,7-dihydroxy-8-[(3S,4R)-3-hydroxy-1-methyl-4-piperidinyl]-4-chromenone; crizotinib (PF-02341066; 2-(2-Chlorophenyl)-5,7-dihydroxy-8-[(2R,3S)-2-(hydroxymethyl)-1-methyl-3-pyrrolidinyl]- 4H-1-benzopyran-4-one, hydrochloride (P276-00); 1-methyl-5-[[2-[5-(trifluoromethyl)-1H-imidazol-2-yl]-4-pyridinyl]oxy]-N-[4-(trifluoromethyl)phenyl]-1H-benzimidazol-2-amine (RAF265); indisulam (E7070); roscovitine (CYC202); palbociclib (PD0332991); dinaciclib (SCH727965); N-[5-[[(5-tert-butyloxazol-2-yl)methyl]thio]thiazol-2-yl]piperidine-4-carboxamide (BMS 387032); 4-[[9-chloro-7-(2,6-difluorophenyl)-5H-pyrimido[5,4-d][2]benzazepin-2-yl]amino]-benzoic acid (MLN8054); 5-[3-(4,6-difluoro-1H-benzimidazol-2-yl)-1H-indazol-5-yl]-N-ethyl-4-methyl-3-pyridinemethanamine (AG-024322); 4-(2,6-dichlorobenzoylamino)-1H-pyrazole-3-carboxylic acid N-(piperidin-4-yl)amide (AT7519); 4-[2-methyl-1-(1-methylethyl)-1H-imidazol-5-yl]-N-[4-(methylsulfonyl)phenyl]- 2-pyrimidinamine (AZD5438); and XL281 (BMS908662).

In one embodiment of the uses, the kinase inhibitor is a CDK4 inhibitor, e.g., palbociclib (PD0332991), and the palbociclib is administered at a dose of about 50 mg, 60 mg, 70 mg, 75 mg, 80 mg, 90 mg, 100 mg, 105 mg, 110 mg, 115 mg, 120 mg, 125 mg, 130 mg, 135 mg (e.g., 75 mg, 100 mg or 125 mg) daily for a period of time, e.g., daily for 14-21 days of a 28 day cycle, or daily for 7-12 days of a 21 day cycle. In one embodiment, 1, 2, 3, 4, 5, 6, 7, 8, 9, 10, 11, 12 or more cycles of palbociclib are administered.

In one embodiment of the uses, the kinase inhibitor is a BTK inhibitor selected from ibrutinib (PCI-32765); GDC-0834; RN-486; CGI-560; CGI-1764; HM-71224; CC-292; ONO-4059; CNX-774; and LFM-A13. In one embodiment, the BTK inhibitor does not reduce or inhibit the kinase activity of interleukin-2-inducible kinase (ITK), and is selected from GDC-0834; RN-486; CGI-560; CGI-1764; HM-71224; CC-292; ONO-4059; CNX-774; and LFM-A13.

In one embodiment of the uses, the kinase inhibitor is a BTK inhibitor, e.g., ibrutinib (PCI-32765), and the ibrutinib is administered at a dose of about 250 mg, 300 mg, 350 mg, 400 mg, 420 mg, 440 mg, 460 mg, 480 mg, 500 mg, 520 mg, 540 mg, 560 mg, 580 mg, 600 mg (e.g., 250 mg, 420 mg or 560 mg) daily for a period of time, e.g., daily for 21 day cycle, or daily for 28 day cycle. In one embodiment, 1, 2, 3, 4, 5, 6, 7, 8, 9, 10, 11, 12 or more cycles of ibrutinib are administered.

In one embodiment of the uses, the kinase inhibitor is a BTK inhibitor that does not inhibit the kinase activity of ITK, e.g., RN-486, and RN-486 is administered at a dose of about 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg (e.g., 150 mg, 200 mg or 250 mg) daily for a period of time, e.g., daily a 28 day cycle. In one embodiment, 1, 2, 3, 4, 5, 6, 7, or more cycles of RN-486 are administered.

In one embodiment of the uses, the kinase inhibitor is an mTOR inhibitor selected from temsirolimus; ridaforolimus (1*R*,2*R*,4*S*)-4-[(2*R*)-2 [(1*R*,9*S*,12*S*,15*R*,16*E*,18*R*,19*R*,21*R*, 23*S*,24*E*,26*E*,28*Z*,30*S*,32*S*,35*R*)-1,18-dihydroxy-19,30-dimethoxy-15,17,21,23, 29,35-hexamethyl-2,3,10,14,20-pentaoxo-11,36-dioxa-4-azatricyclo[30.3.1.0^{4,9}] hexatriaconta-16,24,26,28-tetraen-12-yl]propyl]-2-methoxycyclohexyl dimethylphosphinate, also known as AP23573 and MK8669; everolimus (RAD001); rapamycin (AY22989); simapimod; (5-{2,4-bis[(3*S*)-3-methylmorpholin-4-yl]pyrido[2,3-*d*]pyrimidin-7-yl}-2-methoxyphenyl)methanol (AZD8055); 2-amino-8-[*trans*-4-(2-hydroxyethoxy)cyclohexyl]-6-(6-methoxy-3-pyridinyl)-4-methyl-pyrido[2,3-*d*]pyrimidin-7(8H)-one (PF04691502); and *N*²-[1,4-dioxo-4-[[4-(4-oxo-8-phenyl-4*H*-1-benzopyran-2-yl)morpholinium-4-yl]methoxy]butyl]-L-arginylglycyl-L-α-aspartylL-serine-, inner salt (SF1126); and XL765.

In one embodiment of the uses, the kinase inhibitor is an mTOR inhibitor, e.g., rapamycin, and the rapamycin is administered at a dose of about 3 mg, 4 mg, 5 mg, 6 mg, 7 mg, 8 mg, 9 mg, 10 mg (e.g., 6 mg) daily for a period of time, e.g., daily for 21 day cycle, or daily for 28 day cycle. In one embodiment, 1, 2, 3, 4, 5, 6, 7, 8, 9, 10, 11, 12 or more cycles of rapamycin are administered. In one embodiment, the kinase inhibitor is an mTOR inhibitor, e.g., everolimus and the everolimus is administered at a dose of about 2 mg, 2.5 mg, 3 mg, 4 mg, 5 mg, 6 mg, 7 mg, 8 mg, 9 mg, 10 mg, 11 mg, 12 mg, 13 mg, 14 mg, 15 mg (e.g., 10 mg) daily for a period of time, e.g., daily for 28 day cycle. In one embodiment, 1, 2, 3, 4, 5, 6, 7, 8, 9, 10, 11, 12 or more cycles of everolimus are administered.

In one embodiment of the uses, the kinase inhibitor is an MNK inhibitor selected from CGP052088; 4-amino-3-(p-fluorophenylamino)-pyrazolo [3,4-*d*] pyrimidine (CGP57380); cercosporamide; ETC-1780445-2; and 4-amino-5-(4-fluoroanilino)-pyrazolo [3,4-*d*] pyrimidine.

In one embodiment of the uses, the kinase inhibitor is a dual phosphatidylinositol 3-kinase (PI3K) and mTOR inhibitor selected from 2-Amino-8-[*trans-*4-(2-hydroxyethoxy)cyclohexyl]-6-(6-methoxy-3-pyridinyl)-4-methyl-pyrido[2,3-*d*]pyrimidin-7(8*H*)-one (PF-04691502); N-[4-[[4-(Dimethylamino)-1-piperidinyl]carbonyl]phenyl]-*N*'-[4-(4,6-di-4-morpholinyl-1,3,5-triazin-2-yl)phenyl]urea (PF-05212384, PKI-587); 2-Methyl-2-{4-[3-methyl-2-oxo-8-(quinolin-3-yl)-2,3-dihydro-1*H*-imidazo[4,5-c]quinolin-1-yl]phenyl}propanenitrile (BEZ-235); apitolisib (GDC-0980, RG7422); 2,4-Difluoro-N-{2-(methyloxy)-5-[4-(4-pyridazinyl)-6-quinolinyl]-3-pyridinyl}benzenesulfonamide (GSK2126458); 8-(6-methoxypyridin-3-yl)-3-methyl-1-(4-(piperazin-1-yl)-3-(trifluoromethyl)phenyl)-1H-imidazo[4,5-c]quinolin-2(3H)-one Maleic acid (NVP-BGT226); 3-[4-(4-Morpholinylpyrido[3',2':4,5]furo[3,2-d]pyrimidin-2-yl]phenol (PI-103); 5-(9-isopropyl-8-methyl-2-morpholino-9H-purin-6-yl)pyrimidin-2-amine (VS-5584, SB2343); and N-[2-[(3,5-Dimethoxyphenyl)amino]quinoxalin-3-yl]-4-[(4-methyl-3-methoxyphenyl)carbonyl]aminophenylsulfonamide (XL765).

In one embodiment of the uses, a CAR expressing immune effector cell described herein is administered to a subject in combination with a protein tyrosine phosphatase inhibitor, e.g., a protein tyrosine phosphatase inhibitor described herein. In one embodiment, the protein tyrosine phosphatase inhibitor is an SHP-1 inhibitor, e.g., an SHP-1 inhibitor described herein, such as, e.g., sodium stibogluconate. In one embodiment, the protein tyrosine phosphatase inhibitor is an SHP-2 inhibitor.

In one embodiment of the uses, the CAR molecule is administered in combination with another agent, and the agent is a cytokine. The cytokine can be, e.g., IL-7, IL-15, IL-21, or a combination thereof. In another embodiment, the CAR molecule is administered in combination with a checkpoint inhibitor, e.g., a checkpoint inhibitor described herein. For example, in one embodiment, the check point inhibitor inhibits an inhibitory molecule selected from PD-1, PD-L1, CTLA-4, TIM-3, CEACAM (e.g., CEACAM-1, CEACAM-3 and/or CEACAM-5), LAG-3, VISTA, BTLA, TIGIT, LAIR1, CD160, 2B4 and TGFR beta.

### Methods of making CAR-expressing cells

In another aspect, the disclosure pertains to a method of making a cell (e.g., an immune effector cell or population thereof) comprising introducing into (e.g., transducing) a cell, e.g., a T cell or a NK cell described herein, with a vector of comprising a nucleic acid encoding a CAR, e.g., a CAR described herein; or a nucleic acid encoding a CAR molecule e.g., a CAR described herein. Methods of making CAR-expressing cells are not claimed as such.

The cell in the methods is an immune effector cell (e.g., aT cell or a NK cell, or a combination thereof). In some instances, the cell in the methods is diaglycerol kinase (DGK) and/or Ikaros deficient.

In some instances, the introducing the nucleic acid molecule encoding a CAR comprises transducing a vector comprising the nucleic acid molecule encoding a CAR, or transfecting the nucleic acid molecule encoding a CAR, wherein the nucleic acid molecule is an in vitro transcribed RNA.

In some instances, the method further comprises:
a. providing a population of immune effector cells (e.g., T cells or NK cells); and
b. removing T regulatory cells from the population, thereby providing a population of T regulatory-depleted cells;
wherein steps a) and b) are performed prior to introducing the nucleic acid encoding the CAR to the population.

In instances of the methods, the T regulatory cells comprise CD25+ T cells, and are removed from the cell population using an anti-CD25 antibody, or fragment thereof. The anti-CD25 antibody, or fragment thereof, can be conjugated to a substrate, e.g., a bead.

In other instances, the population of T regulatory-depleted cells provided from step (b) contains less than 30%, 25%, 20%, 15%, 10%, 5%, 4%, 3%, 2%, 1% of CD25+ cells.

In yet other instances, the method further comprises:
removing cells from the population which express a tumor antigen that does not comprise CD25 to provide a population of T regulatory-depleted and tumor antigen depleted cells prior to introducing the nucleic acid encoding a CAR to the population. The tumor antigen can be selected from CD19, CD30, CD38, CD123, CD20, CD14 or CD11b, or a combination thereof.

In other instances, the method further comprises
removing cells from the population which express a checkpoint inhibitor, to provide a population of T regulatory-depleted and inhibitory molecule depleted cells prior to introducing the nucleic acid encoding a CAR to the population. The checkpoint inhibitor can be chosen from PD-1, LAG-3, TIM3, B7-H1, CD160, P1H, 2B4, CEACAM (e.g., CEACAM-1, CEACAM-3, and/or CEACAM-5), TIGIT, CTLA-4, BTLA, and LAIR1.

Further instances disclosed herein encompass providing a population of immune effector cells. The population of immune effector cells provided can be selected based upon the expression of one or more of CD3, CD28, CD4, CD8, CD45RA, and/or CD45RO. In certain instances, the population of immune effector cells provided are CD3+ and/or CD28+.

In certain instances of the method, the method further comprises expanding the population of cells after the nucleic acid molecule encoding a CAR has been introduced.

In instances, the population of cells is expanded for a period of 8 days or less.

In certain instances, the population of cells is expanded in culture for 5 days, and the resulting cells are more potent than the same cells expanded in culture for 9 days under the same culture conditions.

In other instances, the population of cells is expanded in culture for 5 days show at least a one, two, three or four fold increase in cell doublings upon antigen stimulation as compared to the same cells expanded in culture for 9 days under the same culture conditions.

In yet other instances, the population of cells is expanded in culture for 5 days, and the resulting cells exhibit higher proinflammatory IFN-γ and/or GM-CSF levels, as compared to the same cells expanded in culture for 9 days under the same culture conditions.

In other instances, the population of cells is expanded by culturing the cells in the presence of an agent that stimulates a CD3/TCR complex associated signal and/or a ligand that stimulates a costimulatory molecule on the surface of the cells. The agent can be a bead conjugated with anti-CD3 antibody, or a fragment thereof, and/or anti-CD28 antibody, or a fragment thereof.

In other instances, the population of cells is expanded in an appropriate media that includes one or more interleukin that result in at least a 200-fold, 250-fold, 300-fold, or 350-fold increase in cells over a 14 day expansion period, as measured by flow cytometry.

In other instances, the population of cells is expanded in the presence IL-15 and/or IL-7.

In certain instances, the method further includes cryopresercing he population of the cells after the appropriate expansion period.

In yet other instances, the method of making discosed herein further comprises contacting the population of immune effector cells with a nucleic acid encoding a telomerase subunit, e.g., hTERT. The the nucleic acid encoding the telomerase subunit can be DNA.

The present disclosure also provides a method of generating a population of RNA-engineered cells, e.g., cells described herein, e.g., immune effector cells (e.g., T cells, NK cells), transiently expressing exogenous RNA. The method comprises introducing an *in vitro* transcribed RNA or synthetic RNA into a cell, where the RNA comprises a nucleic acid encoding a CAR molecule described herein.

In another aspect, the disclosure pertains to a method of providing an anti-tumor immunity in a subject comprising administering to the subject an effective amount of a cell comprising a CAR molecule, e.g., a cell expressing a CAR molecule described herein. In one instance, the cell is an autologous T cell or NK cell. In one instance, the cell is an allogeneic T cell or NK cell. In one instance, the subject is a human.

In one aspect, the invention includes a population of autologous cells that are transfected or transduced with a vector comprising a nucleic acid molecule encoding a CAR molecule, said cells being in accordance with the claims. In one embodiment, the vector is a retroviral vector. In one embodiment, the vector is a self-inactivating lentiviral vector as described elsewhere herein. In one embodiment, the vector is delivered (e.g., by transfecting or electroporating) to a cell, e.g., a T cell or a NK cell, wherein the vector comprises a nucleic acid molecule encoding a CAR in accordance with the claims, which is transcribed as an mRNA molecule, and the CARs is translated from the RNA molecule and expressed on the surface of the cell.

In another aspect, the present invention provides a population of CAR-expressing immune effector cells (e.g., T cells or NK cells) in accordance with the claims. In some embodiments, the population of CAR-expressing cells comprises a mixture of cells expressing different CARs. For example, in one instance, the population of CAR-expressing immune effector cells (e.g., T cells or NK cells) can include a first cell expressing a CAR having an antigen binding domain that binds to a first tumor antigen as described herein, and a second cell expressing a CAR having a different antigen binding domain that binds to a second tumor antigen as described herein. As another example, the population of CAR-expressing cells can include a first cell expressing a CAR that includes an antigen binding domain that binds to a tumor antigen as described herein, and a second cell expressing a CAR that includes an antigen binding domain to a target other than a tumor antigen as described herein. In one instance, the population of CAR-expressing cells includes, e.g., a first cell expressing a CAR that includes a primary intracellular signaling domain, and a second cell expressing a CAR that includes a secondary signaling domain, e.g., a costimulatory signaling domain.

In another aspect, the present invention provides a population of cells in accordance with the claims wherein at least one cell in the population expresses a CAR having an antigen binding domain that binds to a tumor antigen as described herein, and a second cell expressing another agent, e.g., an agent which enhances the activity of a CAR-expressing cell. For example, in one embodiment, the agent can be an agent which inhibits an inhibitory molecule. Examples of inhibitory molecules include PD-1, PD-L1, CTLA-4, TIM-3, CEACAM (e.g., CEACAM-1, CEACAM-3 and/or CEACAM-5), LAG-3, VISTA, BTLA, TIGIT, LAIR1, CD160, 2B4 and TGFR beta. In one embodiment, the agent which inhibits an inhibitory molecule, e.g., is a molecule described herein, e.g., an agent that comprises a first polypeptide, e.g., an inhibitory molecule, associated with a second polypeptide that provides a positive signal to the cell, e.g., an intracellular signaling domain described herein. In one embodiment, the agent comprises a first polypeptide, e.g., of an inhibitory molecule such as PD-1, LAG-3, CTLA-4, CD160, BTLA, LAIR1, TIM-3, CEACAM (e.g., CEACAM-1, CEACAM-3 and/or CEACAM-5), 2B4 and TIGIT, or a fragment of any of these, and a second polypeptide which is an intracellular signaling domain described herein (e.g., comprising a costimulatory domain (e.g., 41BB, CD27 or CD28, e.g., as described herein) and/or a primary signaling domain (e.g., a CD3 zeta signaling domain described herein). In one embodiment, the agent comprises a first polypeptide of PD-1 or a fragment thereof, and a second polypeptide of an intracellular signaling domain described herein (e.g., a CD28, CD27, OX40 or 4-IBB signaling domain described herein and/or a CD3 zeta signaling domain described herein).

In one embodiment, the nucleic acid molecule encoding a CAR molecule is expressed as an mRNA molecule. In one embodiment, the genetically modified CAR - expressing immune effector cells (e.g., T cells, NK cells) can be generated by transfecting or electroporating an RNA molecule encoding the desired CARs (e.g., without a vector sequence) into the cell. In one embodiment, a CAR molecule is translated from the RNA molecule once it is incorporated and expressed on the surface of the recombinant cell.

### BRIEF DESCRIPTION OF THE DRAWINGS

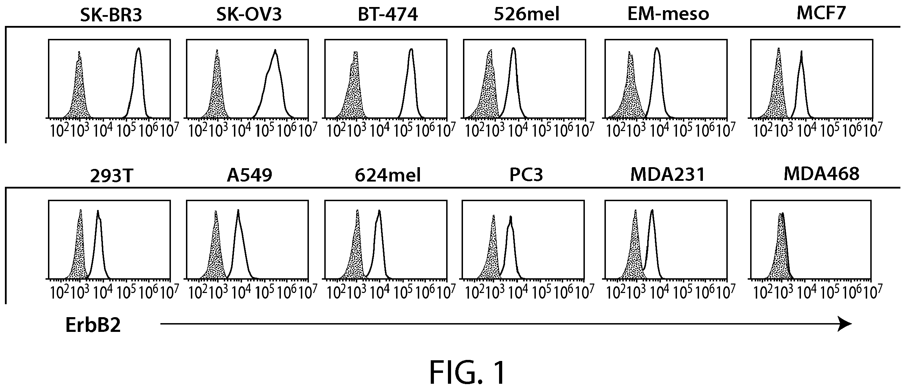

**Fig.1**: A panel of images showing flow cytometry detection of ErbB2 surface expression on tumors and cell lines. Cells were stained with anti-ErbB2 Affibody-biotin and detected with streptavidin-allophycocyanin (APC) (open histograms); cells incubated with APC alone indicate background (grey histograms).

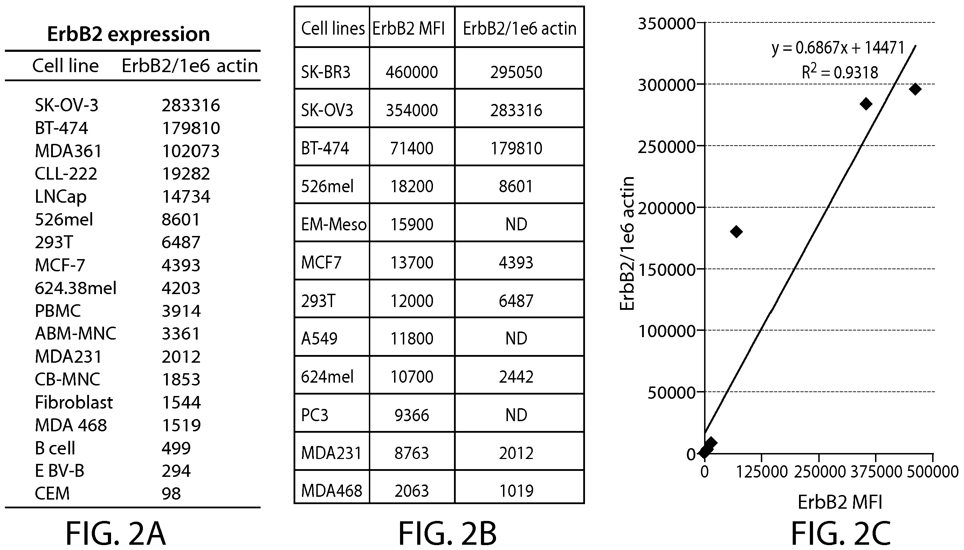

**Fig. 2A, 2B, and 2C****:** Correlation of ErbB2 detection by flow cytometry and quantitative PCR. Copy numbers of ErbB2 detected by quantitative PCR (ErbB2/1E6 actin) (Fig. 2A). ErbB2 mean fluorescence intensity (ErbB2 MFI) as shown in the histograms in Fig. 1 (Fig. 2B). The correlation is plotted between the ErbB2 expression detected by flow cytometry (MFI, x-axis) and quantitative PCR (y-axis). The forward and reverse primers and probe used for ErbB2 quantitative PCR are as follows: ErbB2-1F, GCCTCCACTTCAACCACAGT (SEQ ID NO: 50); ErbB2-1R, TCAAACGTGTCTGTGTTGTAGGT; ErbB2-1M2, FAM-CAGTGCAGCTCACAGATG (SEQ ID NO: 51).

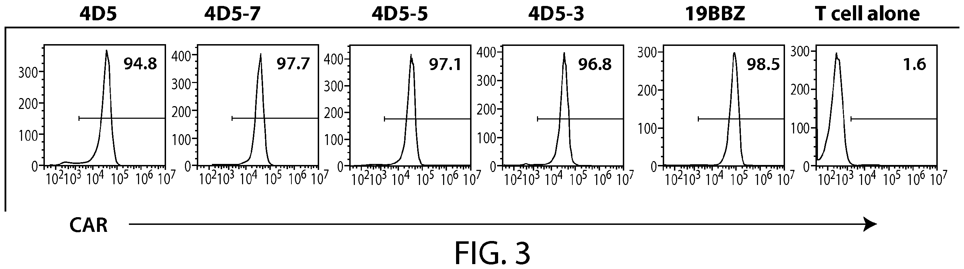

**Fig. 3****.** A panel of images showing FACS analysis of affinity-tuned CAR expression in mRNA electroporated T cells. T cells were electroporated with indicated CAR mRNA and one day after the electroporation, the CAR expression was detected using an anti-mouse IgG Fab antibody (for CD19-BBZ) or ErbB2-Fc (for ErbB2-BBZ CARs). T cells without electroporation were used as a negative control.

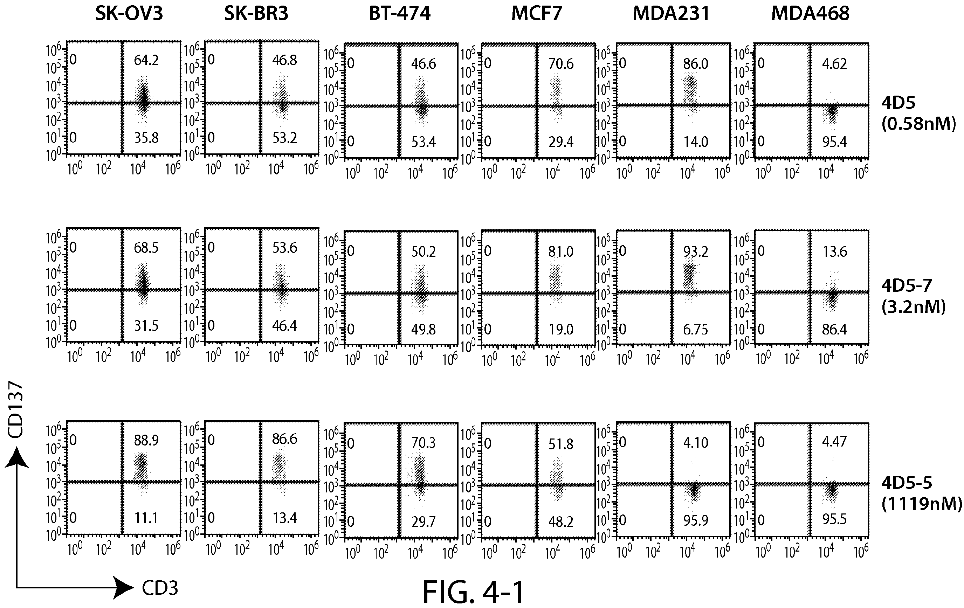

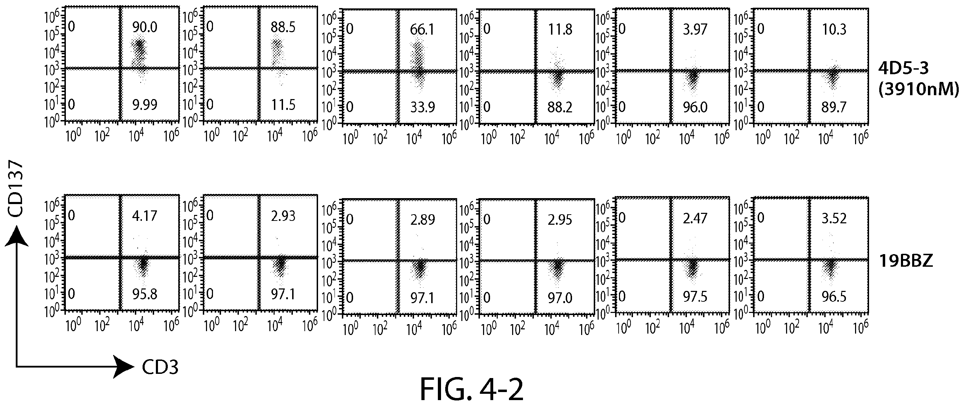

**Fig. 4****.** A panel of images showing the induction of CD137 (4-1BB) expression on CAR T cells after stimulation by tumor cells was measured. One day after electroporation the various CAR T cells (K_{D}, nM) were co-cultured with the indicated tumor cell lines and CD137 expression was measured after 24 hr.

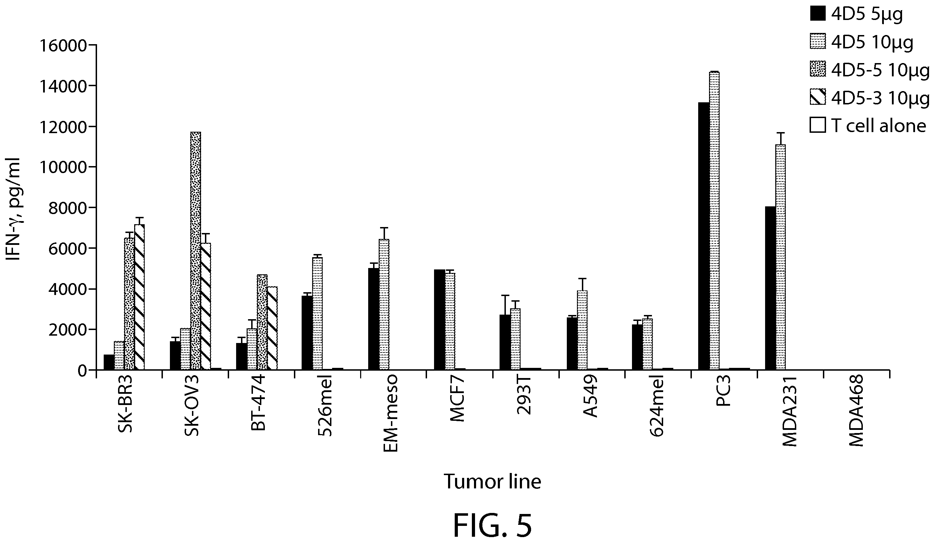

**Fig. 5****.** Cytokine secretion was measured (ELISA) in culture supernatants. T cells were electroporated with 5ug or 10ug affinity-tuned ErbB2 CAR mRNA as indicated. One day after the electroporation, the CAR T cells were co-cultured with indicated tumor cell lines for 24h. Bar chart shows results from a representative experiment (values represent the average ± SD of duplicates) for IFN-gamma.

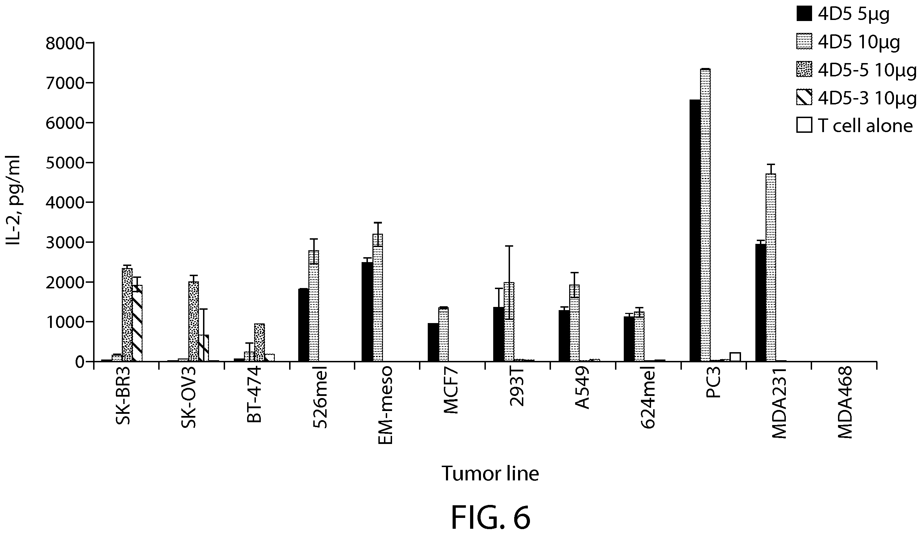

**Fig. 6****.** Cytokine secretion was measured (ELISA) in culture supernatants. T cells were electroporated with 5ug or 10ug affinity-tuned ErbB2 CAR mRNA as indicated. One day after the electroporation, the CAR T cells were co-cultured with indicated tumor cell lines for 24h. Bar chart shows results from a representative experiment (values represent the average ± SD of duplicates) for IL-2.

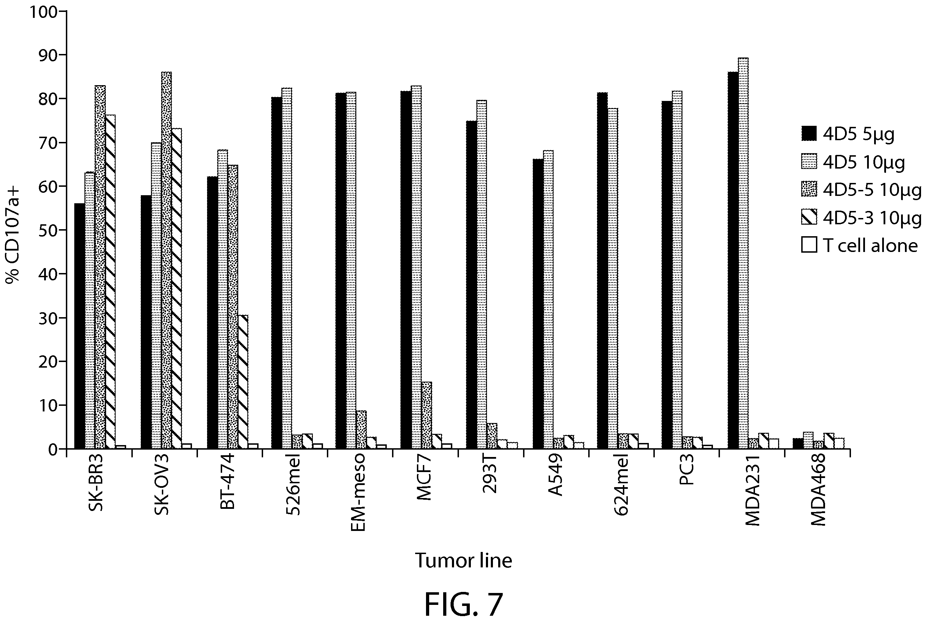

**Fig. 7****.** CD107a up-regulation on CAR T cells stimulated by tumors. T cells were electroporated with 5ug or 10ug ErbB2 CAR mRNAs encoding the indicated scFv and one day later the CAR T cells were co-cultured with the indicated cell line for 4 hr CD107a expression was measured by gating on CD3+CD8+ cells.

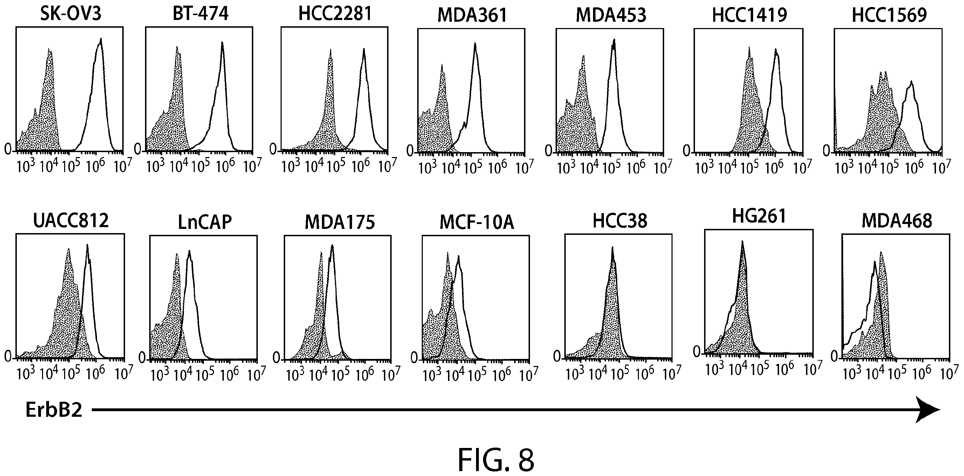

**Fig. 8**. A panel of images showing that additional tumor cell lines were examined for ErbB2 expression by flow cytometry using Biotin-ErbB2 Affibody (streptavidin-PE) staining (open histograms). The same cells stained only with Streptavidin-PE were used as negative control (grey histograms).

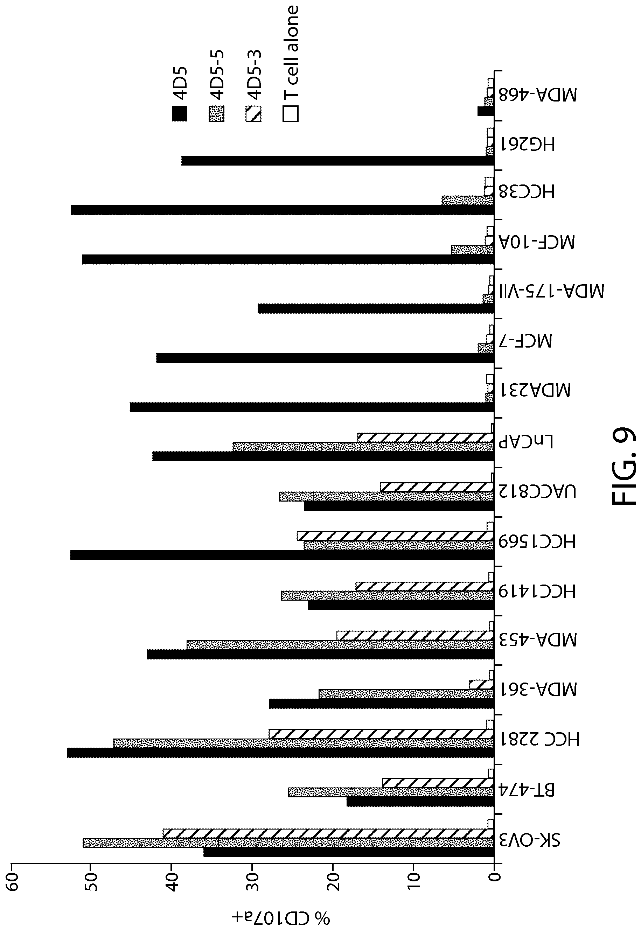

**Fig.9**. T cells electroporated with ErbB2 CAR mRNA were stimulated with tumor lines tested in C. SK-OK3, BT-474, HCC2281, MDA-361, MDA-453, HCC-1419, HCC-1569, UACC-812 and LnCap were reported to be ErbB2 amplified tumors, while MDA-175, MCF-10A, HCC38, HG261 were reported to be ErbB2 low or negative cell lines. After 4h stimulation, CD107a on the T cells were monitored by flow cytometry staining and the % cells expressing CD107a plotted.

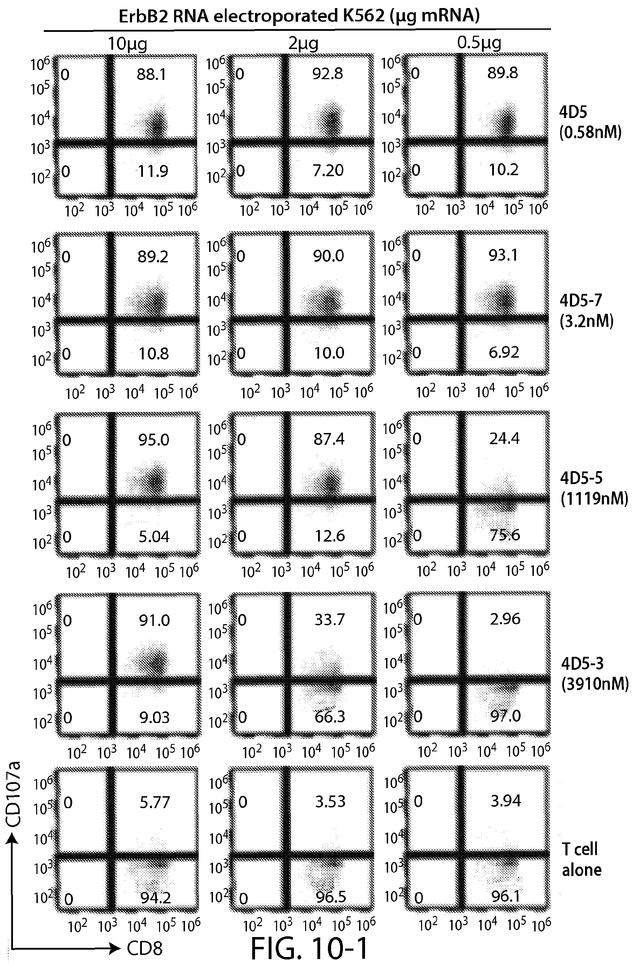

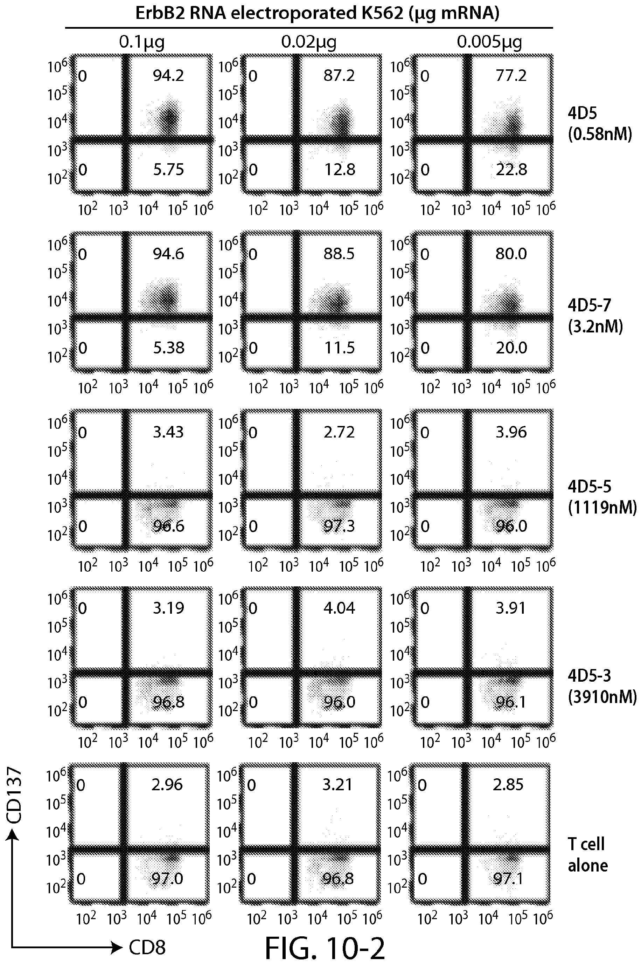

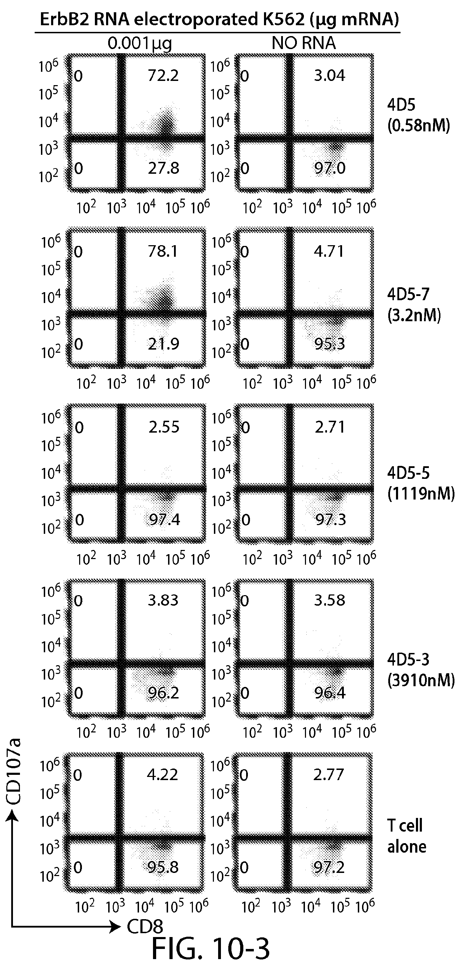

**Fig. 10**. A panel of images showing that recognition of K562 cells were electroporated with indicated amounts of ErbB2 mRNA and CAR T cells expressing the indicated scFv (K_{D}, nM) were co-cultured with target for 4h and the % CD107a expression was quantified on CD3+CD8+ cells.

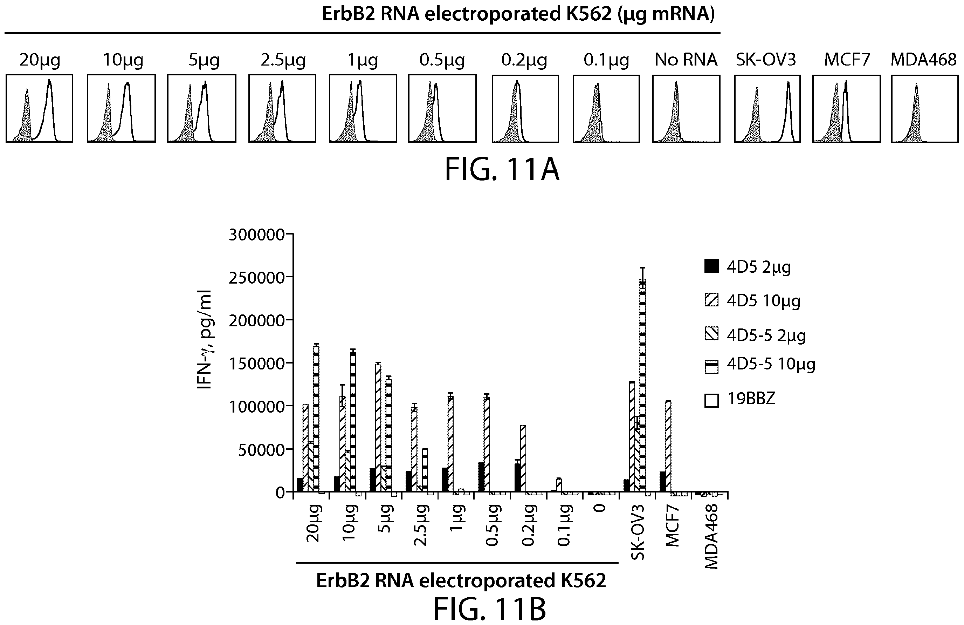

**Fig.11A**. A panel of images showing ErbB2 expression in K562 cells after electroporation. K562 cells were electroporated with the indicated amount of ErbB2 mRNA and staining indicates cells with ErbB2 expression (open histogram); cells incubated with secondary antibody alone indicates background (grey histogram).
**Fig. 11B****.** IFN-gamma secretion by the panel of ErbB2 CART cells stimulated by ErbB2 mRNA electroporated K562 cells. K562 cells were electroporated with 2ug or 10ug ErbB2 CAR mRNA as indicated. CAR T cells were co-cultured with indicted K562 targets and IFN-gamma secretion was measured by ELISA after 24hrs.

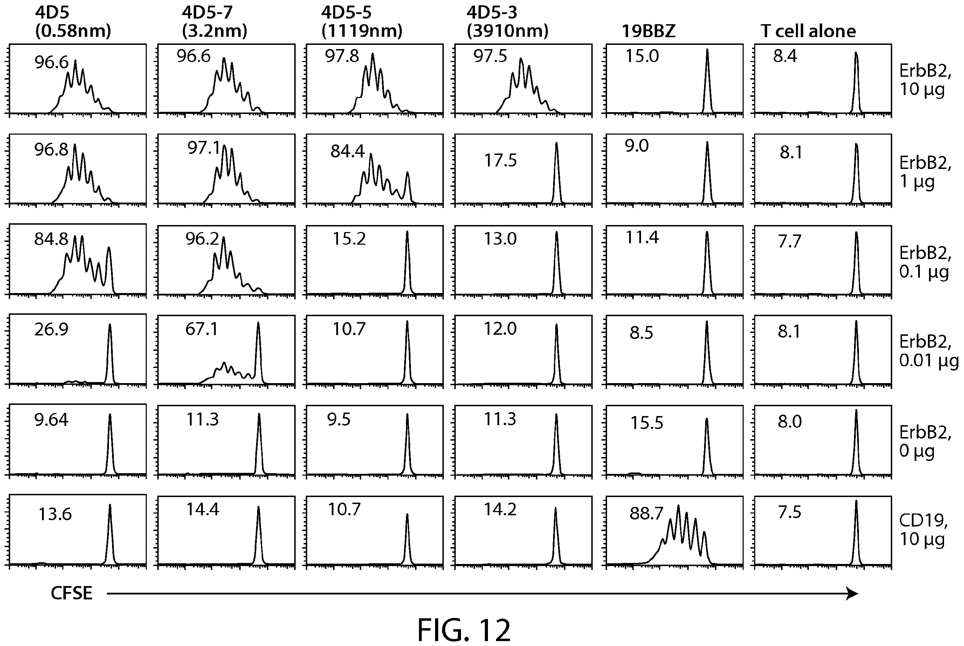

**Fig. 12**. A panel of images showing proliferation of the panel of affinity-tuned CAR T cells after stimulation by ErbB2 mRNA electroporated K562 cells. Resting T cells were labeled with CFSE and electroporated with 10ug CAR mRNA. K562 cells were electroporated with the indicated 9amount of ErbB2 mRNA or control CD19 mRNA (19BBBZ). The T cells and irradiated targets were cultured (1:1 ratio) for 7 days and CFSE dilution measured by flow cytometry (CD3 gated); the % divided T cells is shown.

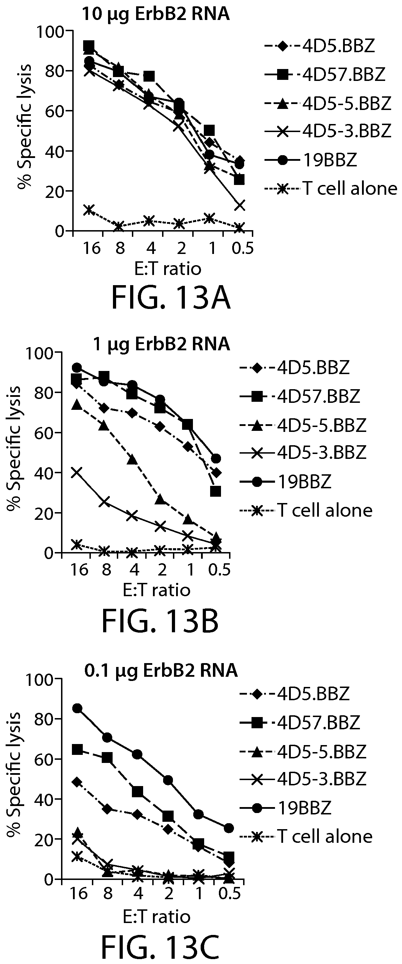

**Fig. 13A, 13B, and 13C**. The cytotoxicity of the panel of CAR T cells against ErbB2 mRNA electroporated Nalm6-CBG target cells was measured. T cells were electroporated with ErbB2 or CD19 CAR mRNA as indicated. CD19+ve Nalm6-CBG (click beetle green) target cells were electroporated with ErbB2 mRNA at the indicated dose: 10 µg ErbB2 RNA (Fig. 13A); 1 µg ErbB2 RNA (Fig. 13B); and 0.1 µg ErbB2 RNA (Fig. 13C). One day after the electroporation, the CAR T cells were co-cultured with Nalm6-CBG cells at indicated E:T ratio and % specific lysis calculated after 8hr.

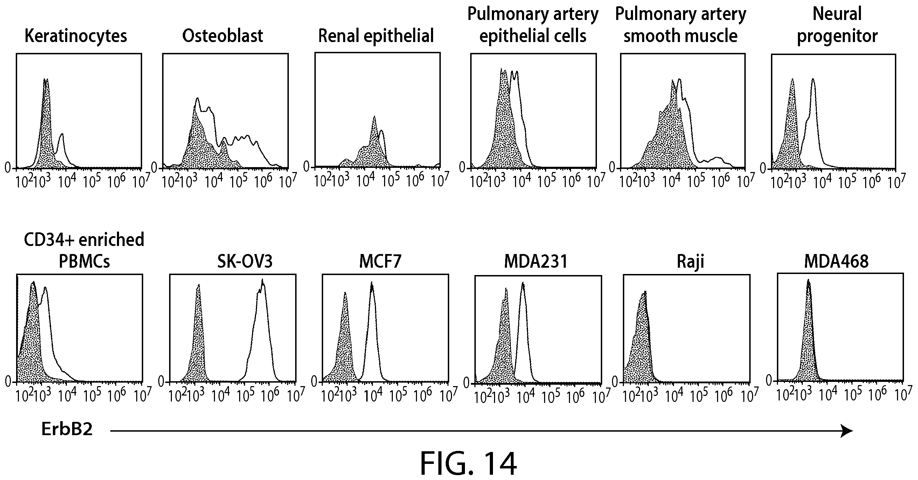

**Fig. 14**. A panel of images showing ErbB2 expression in the indicated primary cell lines. The primary cell lines were stained using anti-ErBb2 Affibody-biotin and detected using streptavidin-allophycocyanin (APC) (open histograms); cells stained with APC only were used as control (grey histograms).

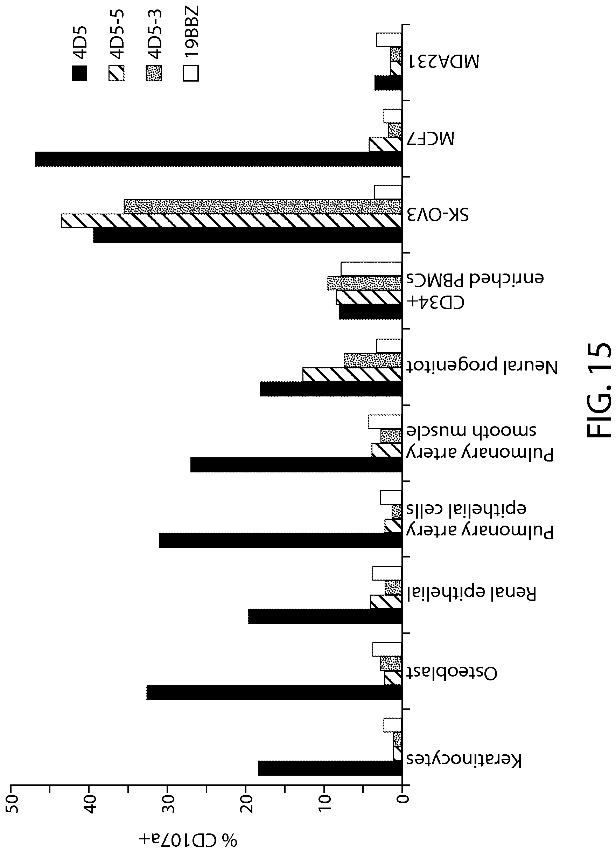

**Fig. 15**. Selective targeting of ErbB2 on primary cell lines. The panel of CAR T cells was stimulated with the indicted primary cell lines for 4h and the % of CAR T cells expressing CD107a was measured by gating on CD3+CD8+ cells.

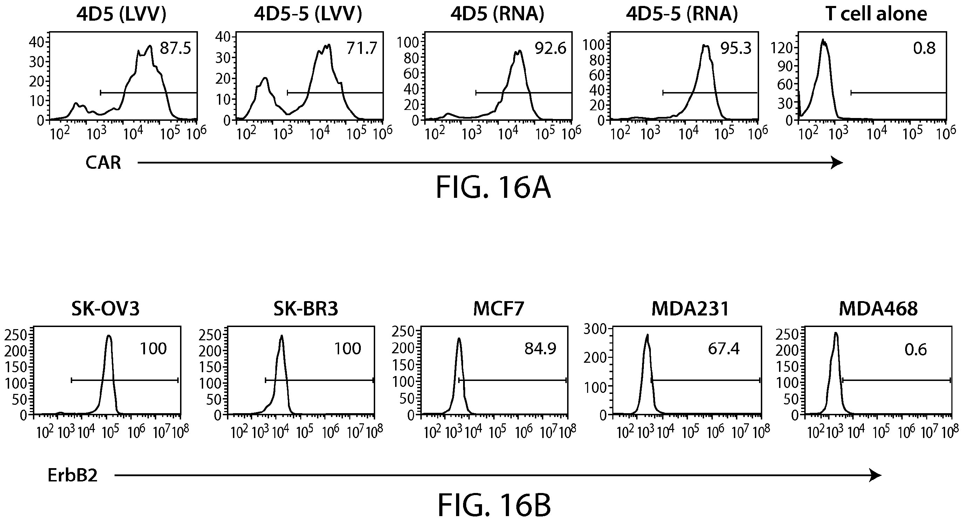

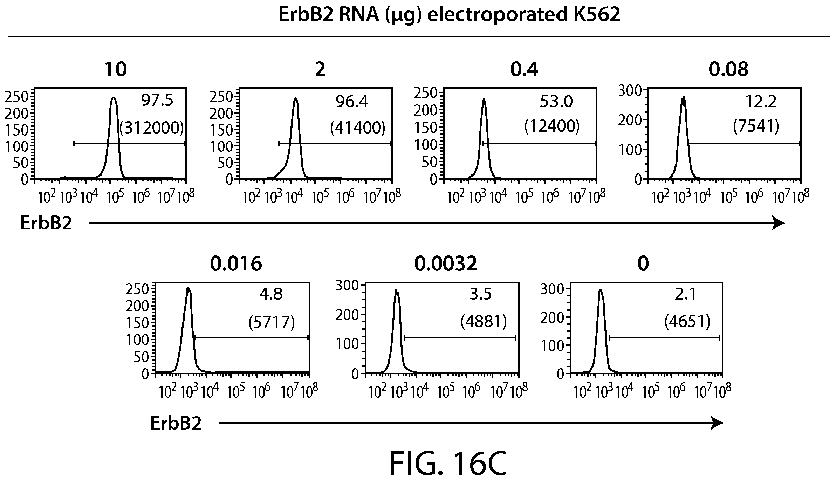

**Fig. 16A, 16B****, and** **16C**. Panels of images showing that T cells were modified with high (4D5) or low (4D5-5) affinity ErbB2 CAR using lentiviral transduction (LVV) or mRNA electroporation (RNA) as indicated. The % CAR expression and brightness was measured using ErbB2-Fc (Fig. 16A). ErbB2 expression on a panel of tumor lines and K562 cells electroporated with ErbB2 mRNA was detected by flow cytometry (Fig. 16B and 16C); percentage cells +cells and (MFI) shown for K562 cells.

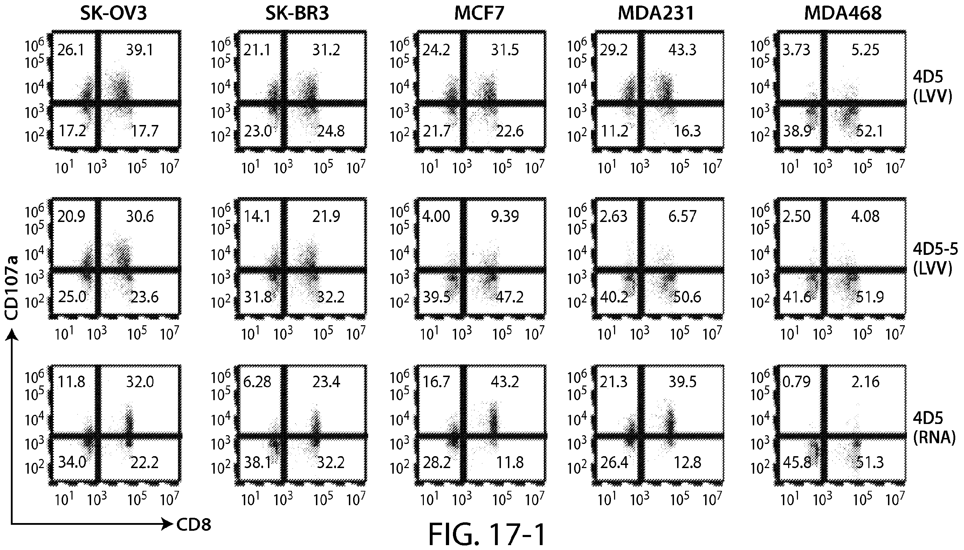

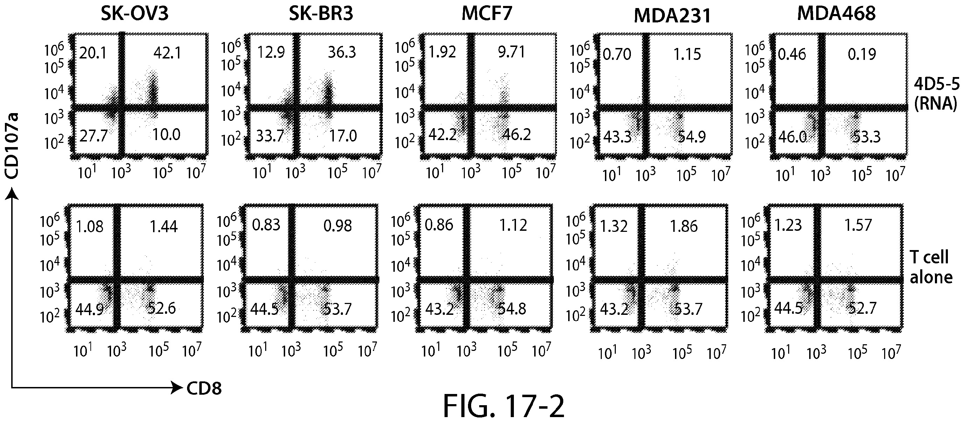

**Fig.17**. A panel of images showing CAR T cell recognition of the indicated tumor lines. CD107a up-regulation was measured on lentiviral transduced or mRNA electroporated CAR T cells after 4hr stimulation with indicated tumor lines (gated on CD3+ cells).

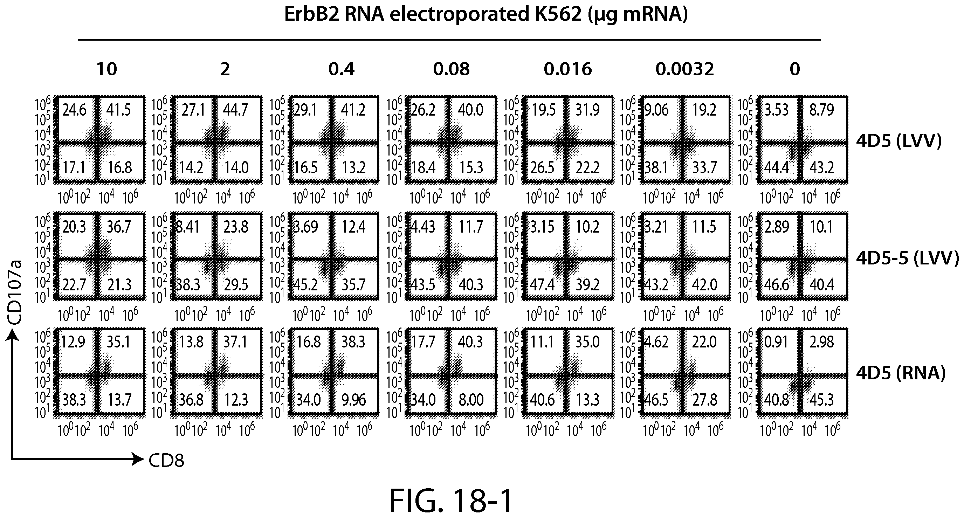

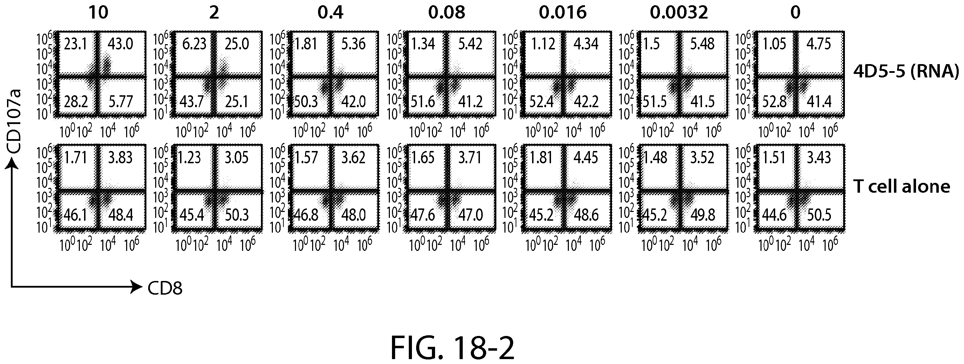

**Fig.18**. Panel of images showing CAR T cell recognition of the K562 cells electroporated with the indicated amounts of ErbB2 mRNA. Induction of CD107a expression was measured on lentiviral transduced or mRNA electroporated CAR T cells after 4hr stimulation with ErbB2 electroporated K562 cells by gating on CD3+ cells.

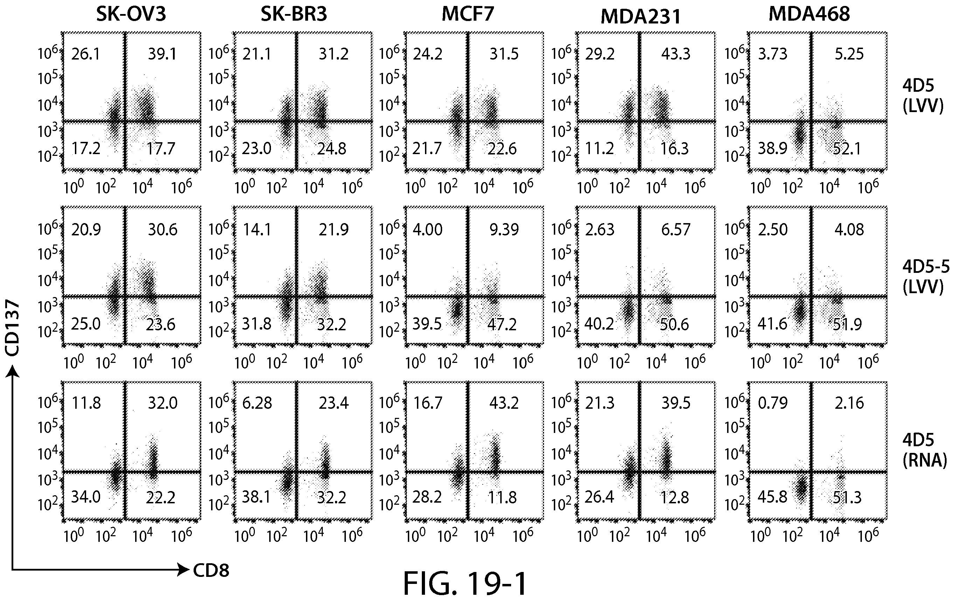

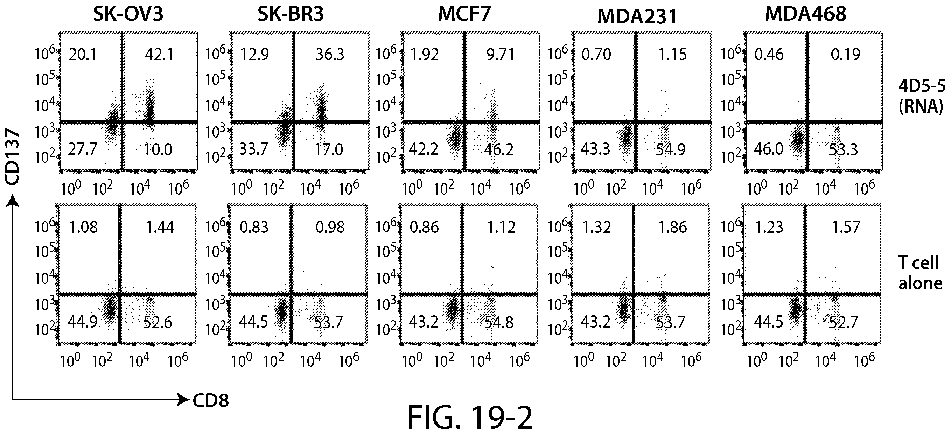

**Fig. 19**. Panel of images showing ErbB2 target dependent upregulation of CD107a on lentiviral transduced or mRNA electroporated T cells. T cells as shown in main text fig. 4A were stimulated 4hr with tumor cell lines expressing ErbB2 at levels varying from over-expressed to low levels. CD107a up-regulation was detected by flow cytometry (CD3+ gated).

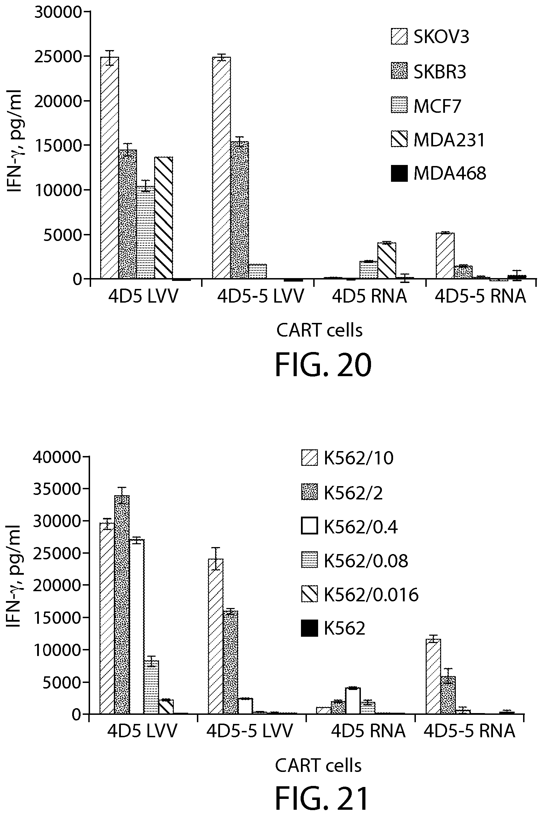

**Fig.20**. IFN-gamma secretion by lentiviral transduced or RNA electroporated CAR T cells was measured by ELISA after 18hr.
**Fig. 21**. IFN-gamma production by CAR T cells measured 18hr after stimulation with K562 cells electroporated with indicated amount of ErbB2 mRNA.

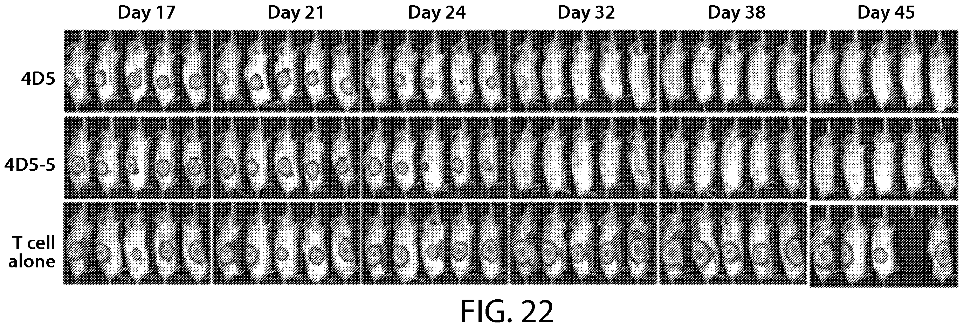

**Fig. 22**. Set of images showing regression of advanced vascularized tumors in mice treated by affinity tuned ErbB2 CAR T cells. Flank tumors were established by injection of 5×10⁶ SK-OV3-CBG (s.c.) in NOD-SCID-γ-/- (NSG) mice (n= 5). Eighteen days after tumor inoculation, mice were randomized to equalize tumor burden and treated with 1×10⁷ lentivirally transduced T cells expressing either higher affinity (4D5.BBZ) or lower affinity (4D5-5.BBZ) CAR . Mice treated with non-transduced T cells ( T Cell Alone) served as controls. Animals were imaged at the indicated time points post tumor inoculation.

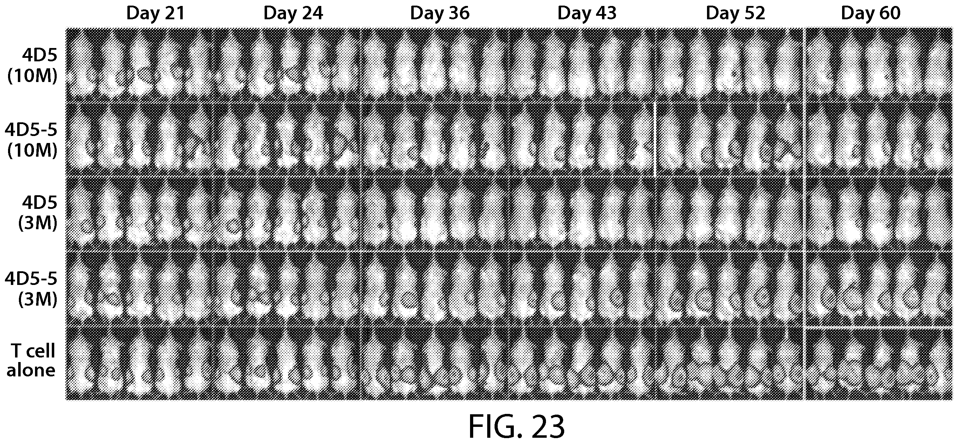

**Fig. 23****.** Set of images showing In vivo discrimination of high ErbB2 (SK-OV3) and low ErbB2 (PC3) expressing tumors by affinity tuned CARs. T cells modified with different affinity ErbB2 CARs by lentiviral transduction were tested in dual-tumor engrafted NSG mice. Mice were implanted with PC3-CBG tumor cells (1e6 cells/mouse, s.c.) on the right flank on day 0. On day 5 the same mice were given SK-OV3-CBG tumor cells (5e6 cells/mouse, s.c.) on the left flank. The mice were treated with T cells (i.v.) on at day 23 after PC3 tumor inoculation. CAR T cells were given as a single injection of 10e6/mouse (10M), or 3e6/mouse (3M) as indicted. Mice treated with non-transduced T cells served as control. Animals were imaged at the indicated time post PC3 tumor inoculation.

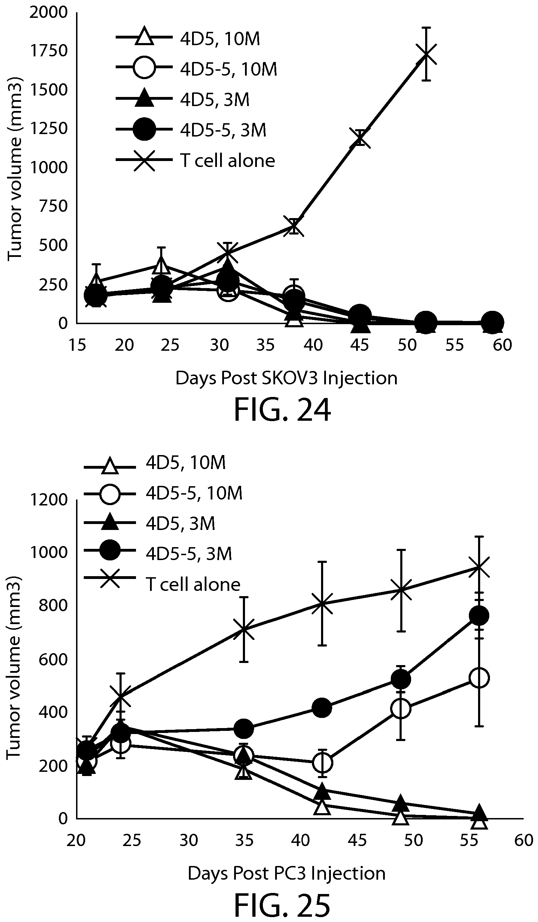

**Fig. 24**. SK-OV3 tumor size in dual-tumor grafted NSG mice treated with the indicated affinity tuned ErbB2 CARs. SK-OV3 tumor sizes were measured over time (days, x-axis), and the tumor volume was calculated and plotted (mm³, y-axis).
**Fig. 25**. PC3 tumor sizes in the dual-tumor grafted NSG mice treated with the indicated affinity tuned ErbB2 CARs. PC3 tumor sizes were measured over time (days, x-axis), and the tumor volume was calculated and plotted (mm³, y-axis).

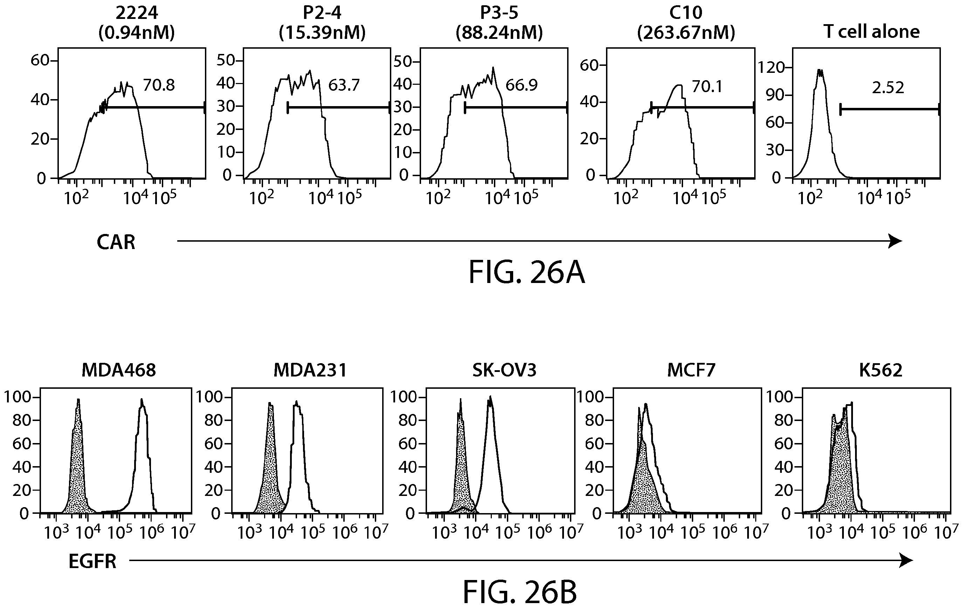

**Fig. 26A and 26B****.** Sets of images showing CAR expression on T cells electroporated with EGFR CAR mRNA were stained by an anti-human IgG Fab and detected by flow cytometry staining (Fig. 26A); the affinity of the scFv is indicated (nM). Tumor lines (Fig. 26B) were stained with anti-EGFR Affibody-FITC (open histograms), the same cells were stained with mouse IgG1-FITC as isotype control (grey histograms).

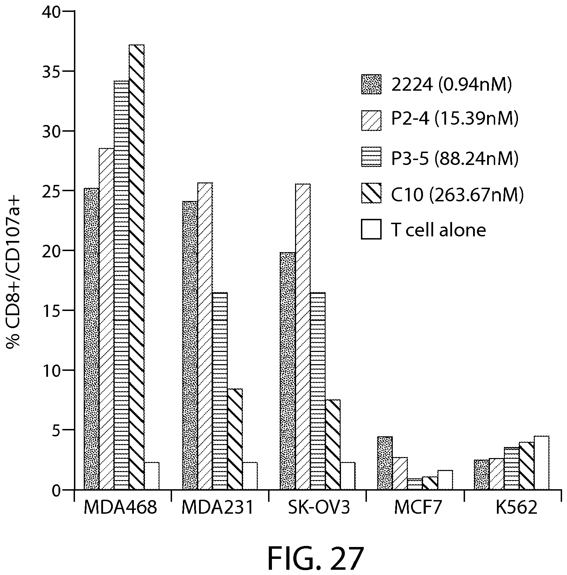

**Fig. 27****.** EGFR CAR recognition sensitivity is correlated with affinity. A panel of EGFR CAR T cells with the indicated affinity of the scFv (KD, nM) was stimulated with the panel of tumors expressing EGFR at the density shown in Fig. 26B. After 4h stimulation, CD107a up-regulation on the CAR T cells was detected by gating on CD3+ cells.

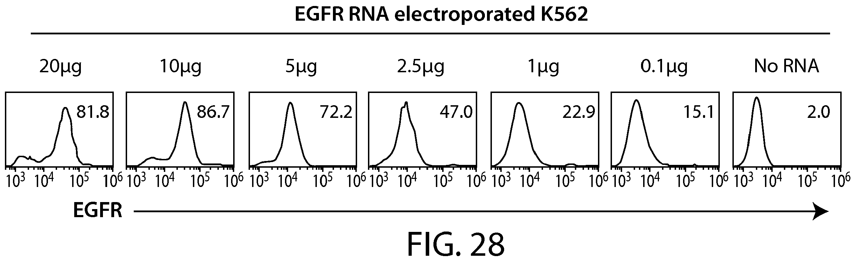

**Fig. 28****.** A set of images showing ErbB2 expression in K562 cells electroporated with the indicated amount of EGFR mRNA. EGFR expression was detected using anti-EGFR Affibody-FITC staining 14h post electroporation.

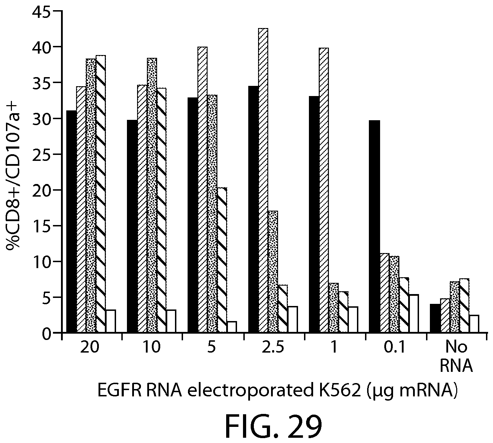

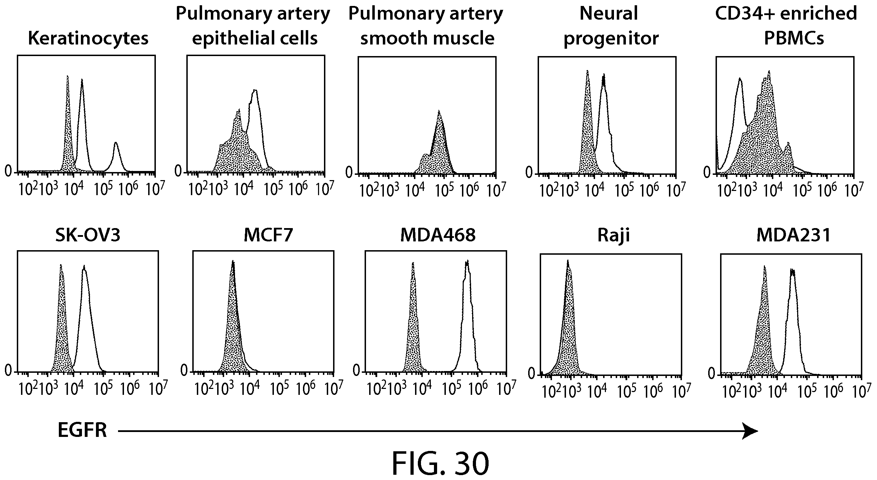

**Fig. 29****.** EGFR CAR recognition sensitivity is correlated with affinity. T cells were electroporated with the panel of EGFR CARs with different affinities as indicated and stimulated with K562 electroporated with EGFR mRNA at different levels as shown in Fig. 30. After 4hr stimulation, CD107a expression on CAR T cells was measured by gating on CD3+ cells.
**Fig. 30****.** Affinity dependent recognition of primary cell lines and tumor cells using affinity-tuned EGFR CARs. T cells were electroporated with the indicated EGFR CAR mRNA. One day after electroporation, the CAR T cells were stimulated with the panel of cells for 4 hr and the induction CD107a expression on the CAR T cells was quantified (CD3+ gated).

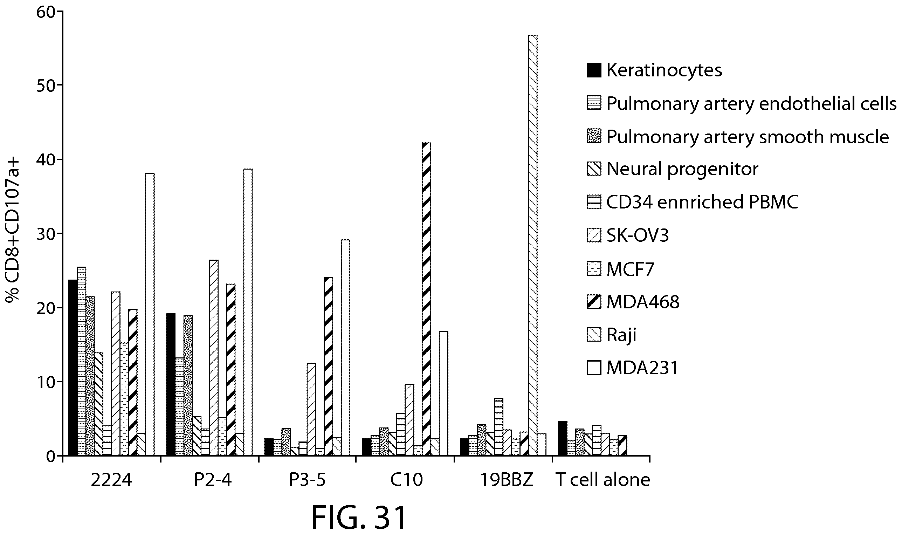

**Fig. 31**. Differential recognition of primary cell lines by T cells modified with affinity-tuned EGFR CARs. The percentage of CD8+CD107a+ double positive cells was plotted.

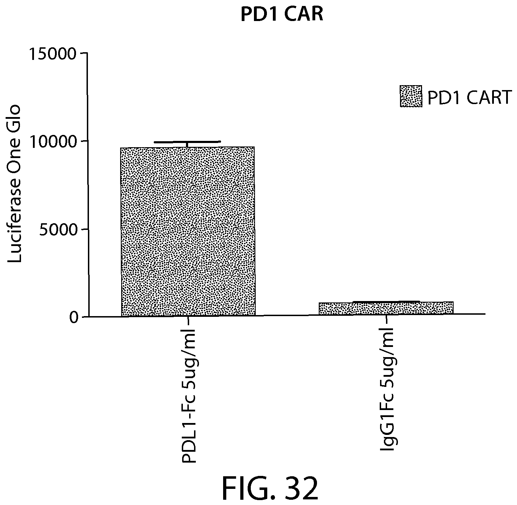

**Fig. 32****.** depicts NFAT inducible promoter driven luciferase activity of a PD1 CAR as compared to the control treatment by IgG1-Fc. FIG22B depicts NFAT inducible promoter driven luciferase activity of a PD1 RCAR which include PD1-ECD -TM-FRB and FKBP-4 1BB-CD3 zeta as compared to the control treatment by IgG1-Fc.

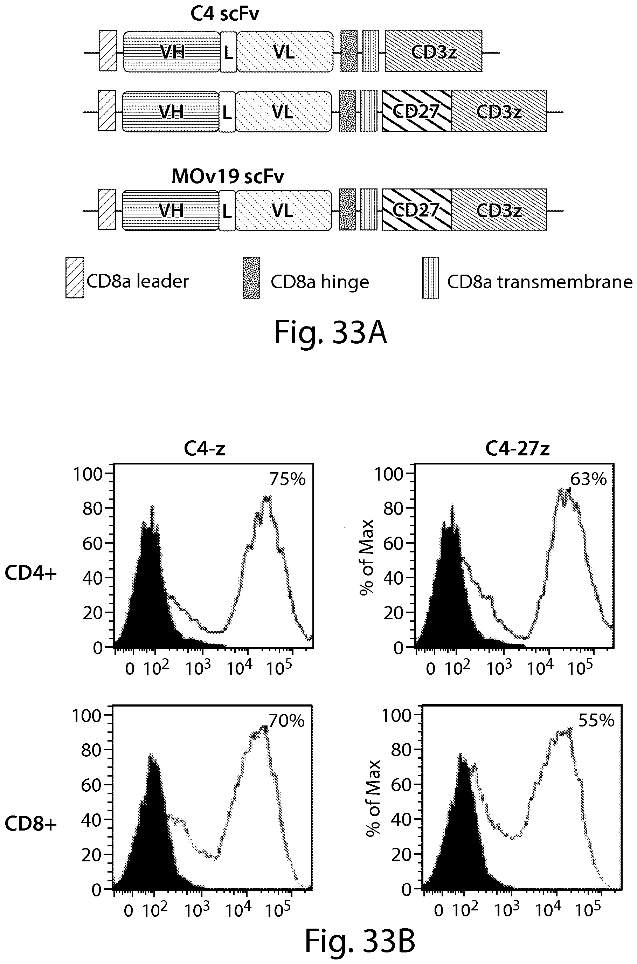

**Fig. 33A and 33B****.** Generation of folate receptor alpha (FRA)-specific fully human chimeric antigen receptor (CAR) T cells. (Fig. 33A) Schematic representation of C4 based CAR constructs containing the CD3ζ cytosolic domain alone (C4-z) or in combination with the CD27 costimulatory module (C4-27z). The murine anti-human FRA MOv19-27z CAR is also shown. (Fig. 33B) A set of images showing transduced T cells consisted of CD4- and CD8-positive cells with both subsets expressing C4 CARs.C4 CAR expression (open histograms) was detected via biotin-labeled rabbit anti-human IgG (H+L) staining followed by streptavidin-phycoerythrin after transduction with lentivirus compared to untransduced (UNT) T cells (filled gray histograms). Transduction efficiencies are indicated with the percentage of CAR expression in parentheses. ScFv, single-chain antibody variable fragment L, linker; C4, anti-FRA scFv; VH, variable H chain; VL, variable L chain; TM, transmembrane region.

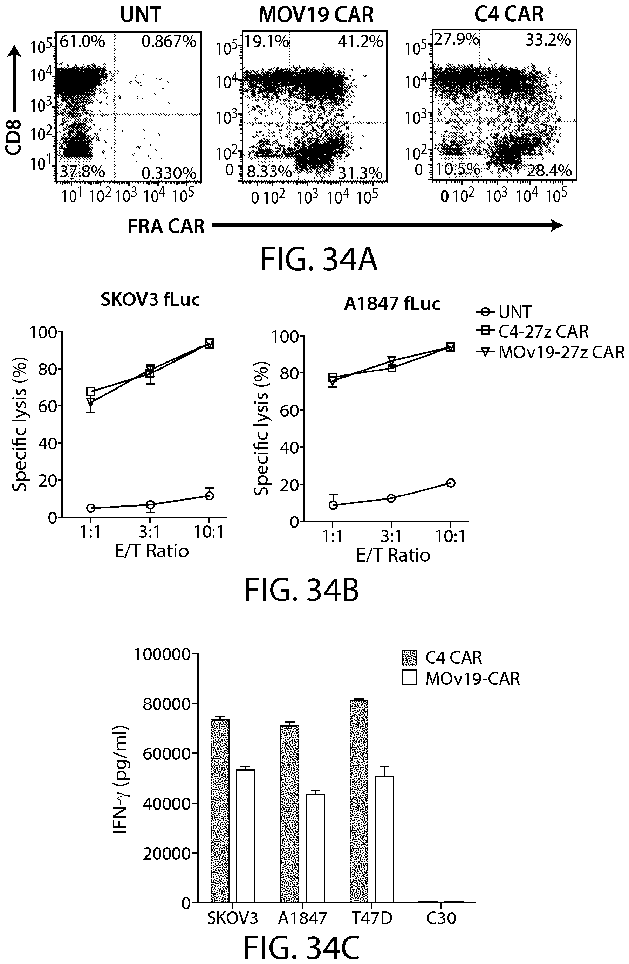

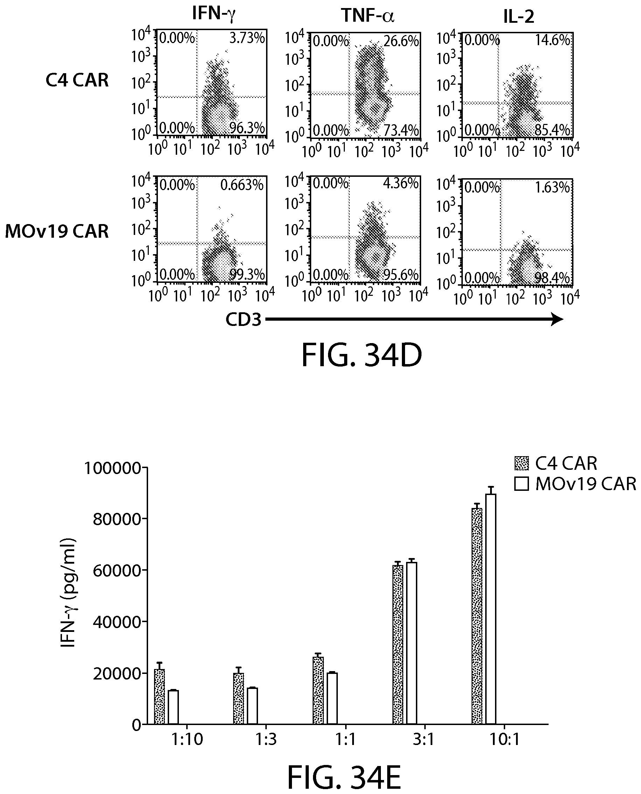

**Fig. 34A, 34B, 34C****,** **34D, and 34E****.** Comparison of anti-tumor activity of FR-specific C4 and MOv19 CARs with CD27costimulatory endodomain in vitro. (Fig. 34A) Set of images showing C4 and MOv19 CARs expression on primary human T cells can be detected via biotin-labeled recombinant FRA protein followed by SA-PE. As shown, both CD8+ T and CD8- (CD4+) cells can efficiently express CARs as measured by flow cytometry. (Fig. 34B) Set of graphs showing C4 and MOv19 CARs-transduced T cells showed lytic function in a bioluminescent killing assay. CAR-T cells killed FR+ SKOV3 and A1847 at the indicated E/T ratio more than 20 hours. Untransduced T cells served as negative controls. Mean and SD of triplicate wells from 1 of at least 3 independent experiments is shown. (Fig. 34C) C4 or MOv19 CAR T cells were co-cultured with FRA+ target cells (SKOV3, A1847 and T47D) and FRA- (C30) at a 1:1 E:T ratio. (Fig. 34D) Set of images showing C4 or MOv19 CAR T cells were stimulated with SKOV3 cells for 5-hour in the presence of Golgi inhibitor and analyzed by flow cytometry for intracellular IFN-g, TNF-a and IL-2. (Fig. 34E) IFN-g release assay of C4 and MOv19 CAR T cells after overnight co-culture with FRA+ tumor cells (at 1:10, 1:3, 1:1, 3:1 and 10:1 ratios).

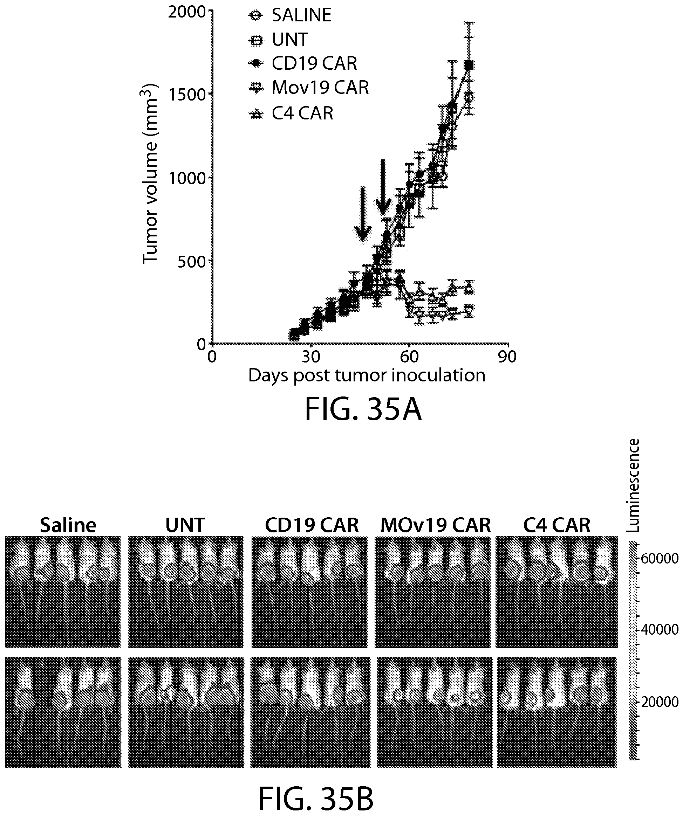

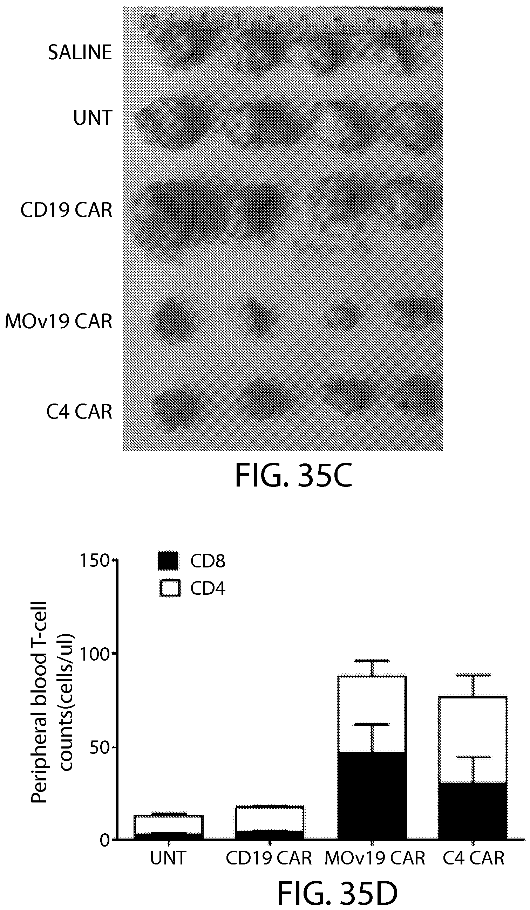

**Fig. 35A, 35B****,** **35C, and 35D****.** Antitumor activity of C4-CAR T cells is comparable to MOv19 CAR T cells. (Fig. 35A) Tumor regression mediated by C4-27z and MOv19-27z CAR T cells. NSG mice bearing established subcutaneous tumor were treated with i.v. injections of 1×10⁷ C4-27z and MOv19-27z CAR+ T cells or control CD19-27z and UNT T cells or saline on day 40 and 45. Tumor growth was assessed by caliper measurement. Tumors treated with C4-27z CAR or MOv19 CAR T cells (∼60% CAR expression) regressed (arrows indicate days of T cell infusion); tumors treated with saline, UNT or CD19-27z CAR T cells did not regress 3 weeks post-first T cell dose. (Fig. 35B) Set of images showing SKOV3 fLuc+ bioluminescence signal was decreased in C4-27z and MOv19-27z CAR T cells treated mice compared with the CD19-27z and the control treatment groups 3 weeks after the first T cell dose. (Fig. 35C) Macroscopic evaluation of resected tumor specimens following T cell therapy. Tumors were harvested from mice at the time of euthanasia, nearly 45 days after first T cell injection. (Fig. 35D) Stable persistence of C4 CAR and MOv19 CAR T cells *in vivo.* Peripheral blood was collected 3 weeks after the first T cell infusion and quantified for the absolute number of human CD4+ and CD8+ T cells/µl of blood. Mean cell count ± SEM is shown with *n* = 5 for all groups.

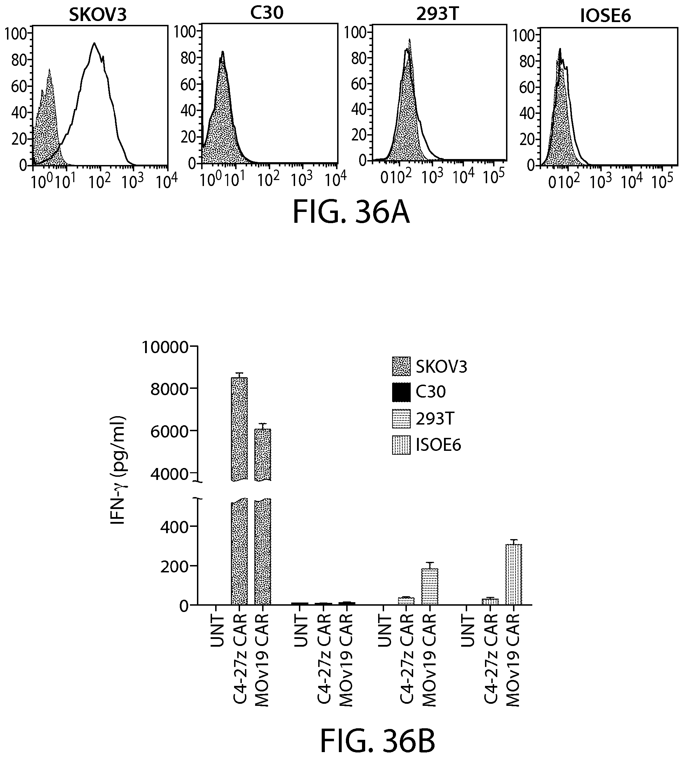

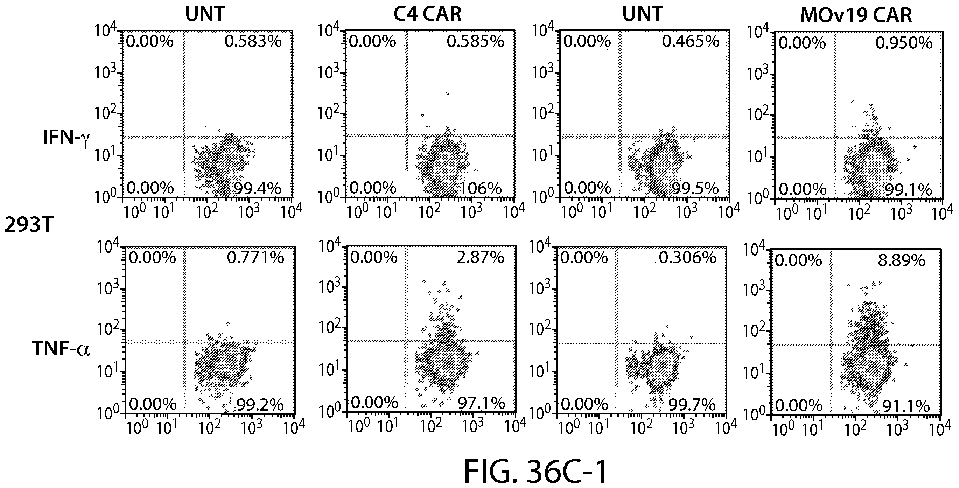

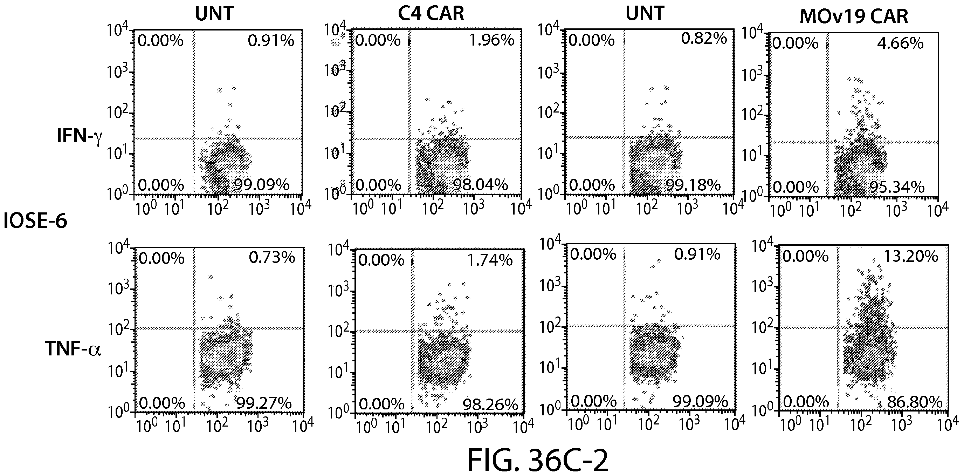

**Fig. 36A, 36B****, and 36C.** C4 CAR T cells showed minimal cytotoxic activity in vitro. (Fig.36A) Set of images showing human embryonic kidney 293T cells and normal epithelial ovarian cell line IOSE6 express very low level of FRA.SKOV3 and C30 served as positive and negative controls, respectively. (Fig. 36B) C4-27z CAR T cells secret minimal amount of IFN-γ following overnight incubation with normal 293T cells and IOSE 6 cell lines expressing low levels of surface FRA compared to MOvl9-27z CAR T cells. (Fig. 36C) Set of images showing C4 and MOv19 CAR T cells were stimulated with 293T or IOSE6 cells for 5-hour in the presence of Golgi inhibitor and analyzed by flow cytometry for intracellular IFN-g and TNF-a.

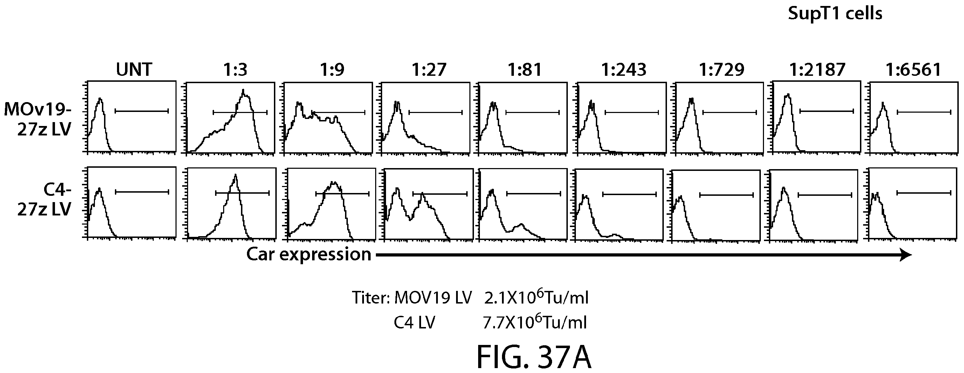

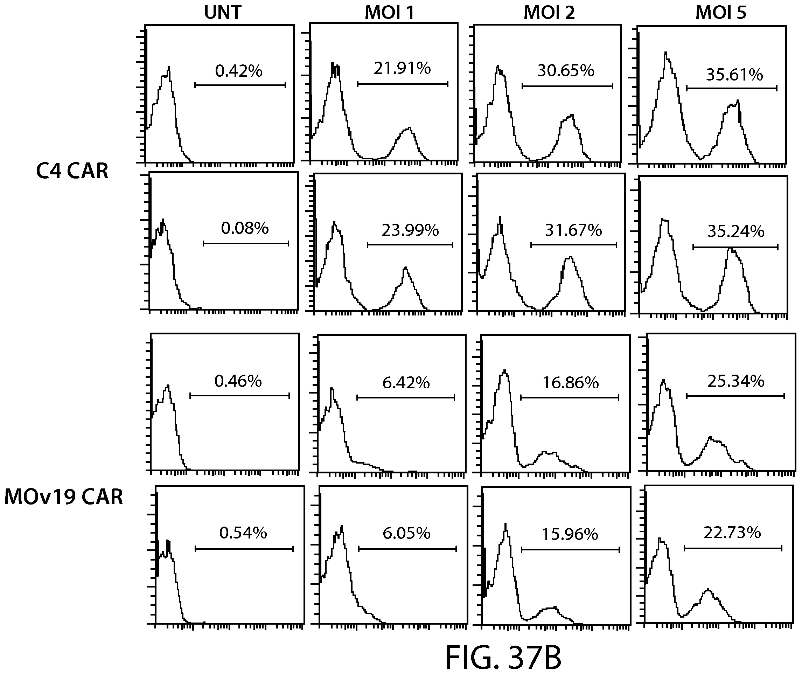

**Fig. 37A** **and** **37B****.** Fully human C4 CAR is expressed and detected on T cell surface. (Fig. 37A) Set of images showing lentiviral titers (transduction units, TU) were determined using SupT1 cells based on 3-fold serial dilution of concentrated virus from 1:3 to a final dilution of 1:6,561.C4 CAR-encoding lentivirus has a higher titer when Compared to the titer of MOv19 CAR encoding lentivirus, following the same production and concentration protocols in parallel. (Fig. 37B) Set of images showing primary human T cells were infected with C4 CAR or MOv19 CAR encoding lentivirus at a multiplicity of infection (MOI) of 1, 2 or 5. These data represent one of at least three independent experiments.

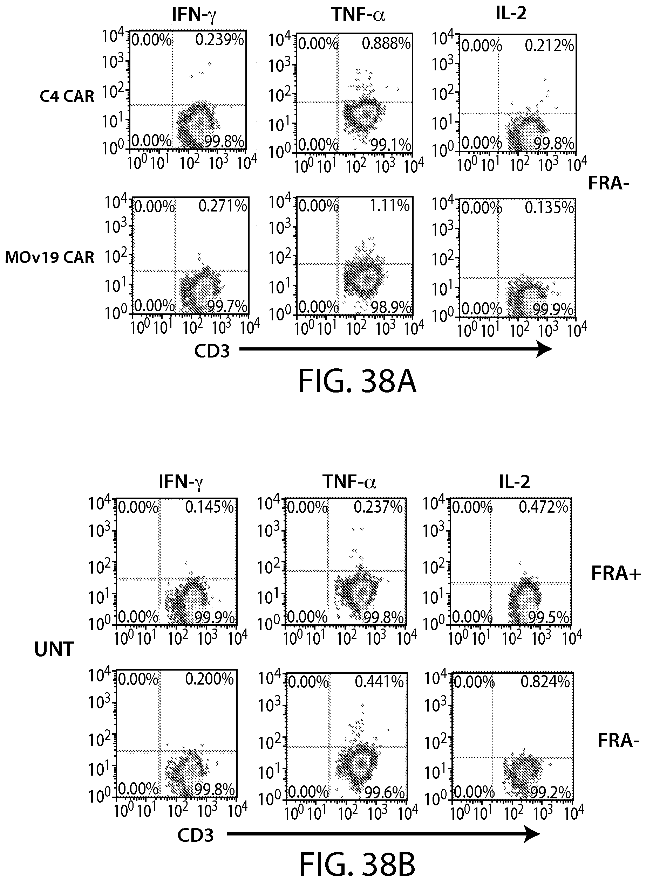

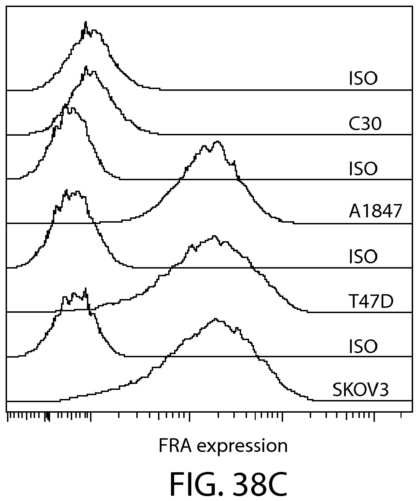

**Fig. 38A, 38B****,** **38C****.** Figs. 38A and 38B are sets of images showing untransduced T cells that were stimulated with FRA+ SKOV3 cells and C4 or MOv19 CAR T cells that were stimulated with FRA- C30 cells for 5-hour in the presence of Golgi inhibitor and analyzed by flow cytometry for intracellular IFN-g, TNF-a and IL-2. Fig. 38C shows αFR expression on SKOV3, A1847 and T47D tumor cell lines; C30 cell line was used as a negative control.

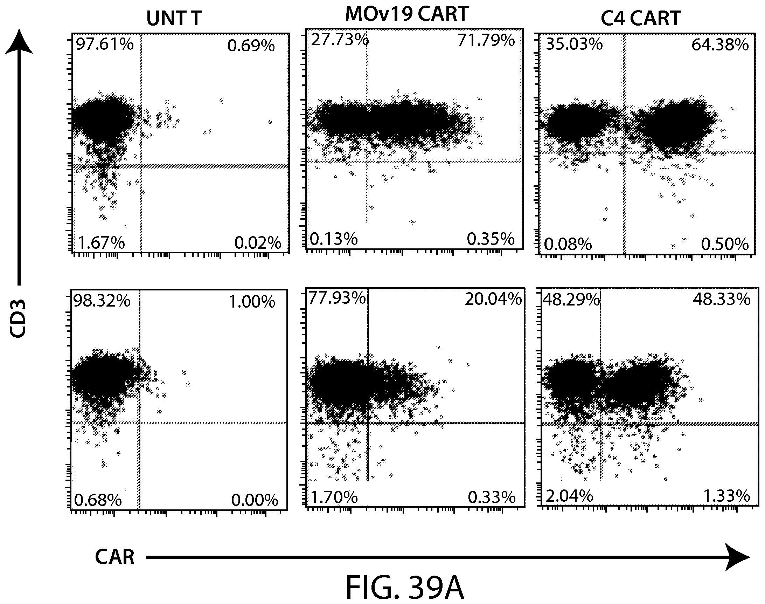

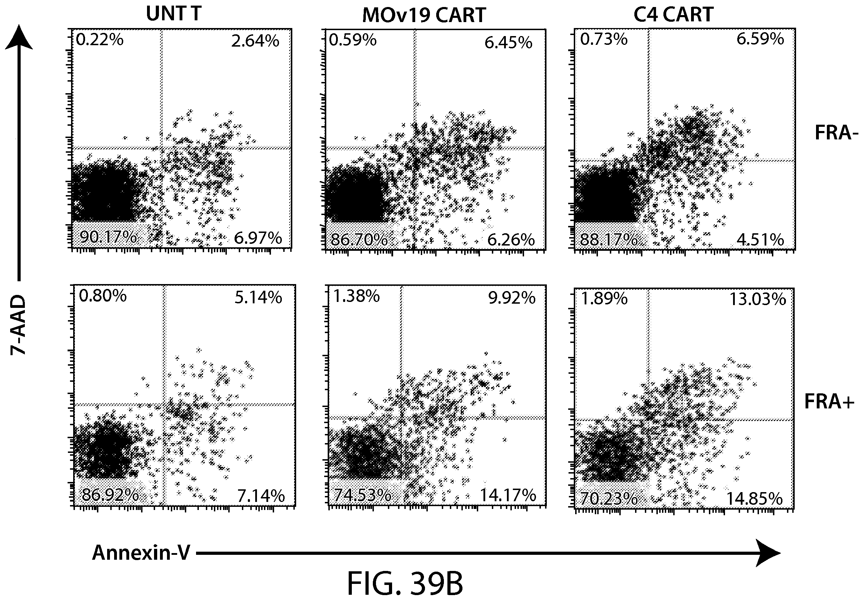

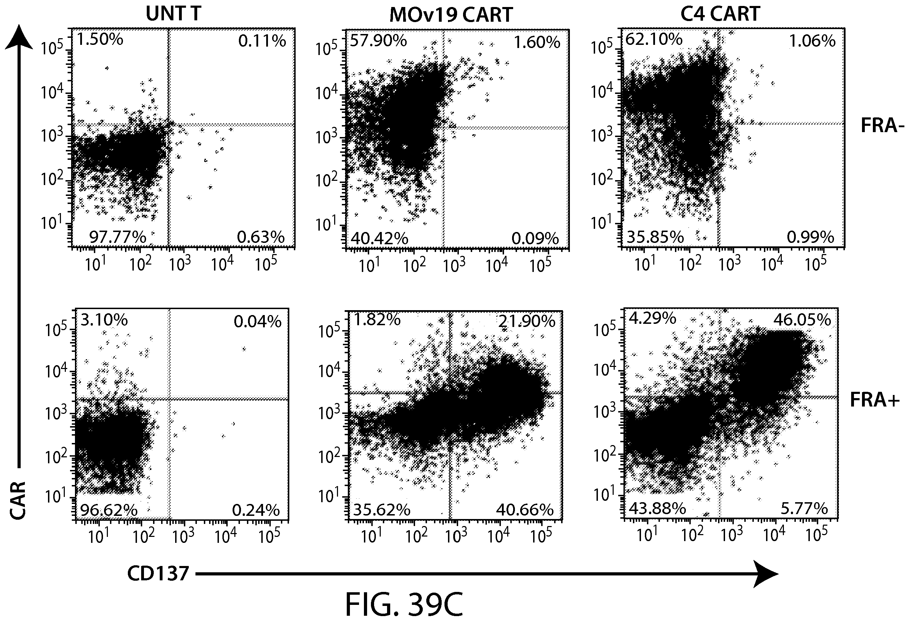

**Fig. 39A****,** **39B****,** **39C**. Sets of images showing CAR down-modulation may impair the antitumor activity of MOv19 CAR but not C4 CAR. C4 and MOv19 CAR T cells were stimulated with SKOV3 or C30 cells for 5-hour in the presence of Golgi inhibitor and analyzed by flow cytometry for T cell surface of CAR expression and intracellular IFN-g, TNF-α and IL-2. (Fig. 39A) Flow cytometry analysis of CAR expression changes after 4 h coculture with αFR+ or αFR- tumor cells. (Fig. 39B) MOv19 and C4 CAR T cells cocultured with αFR+ or αFR- tumor cells and then stained with annexin V and 7-AAD. Apoptotic cells are indicated as the percentage of gated cells. In Fig. 39C, cells were stained for CD137.

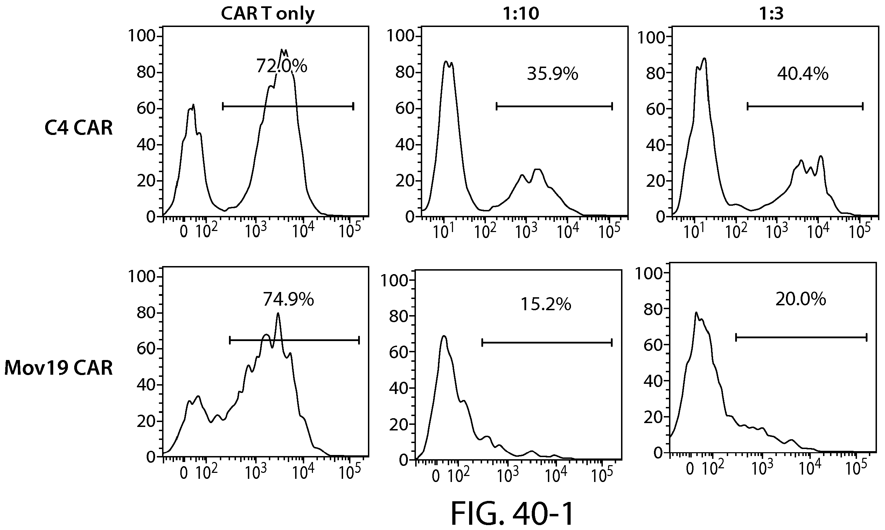

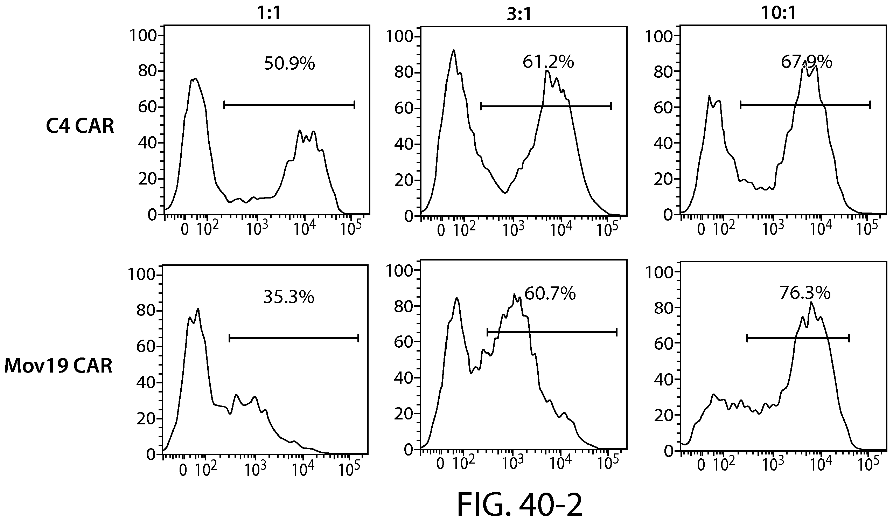

**Fig. 40****.** Set of images showing flow cytometry analysis of CAR expression changes after overnight co-culture with FRA+ tumor cells (at 1:10, 1:3, 1:1, 3:1 and 10:1 ratios).

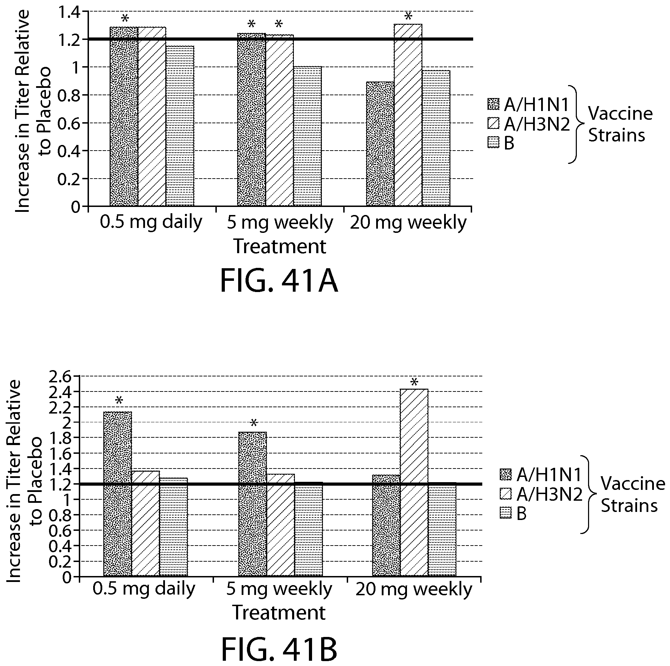

**Fig. 41** comprising **Figs. 41A** and **41B**, are graphs showing an increase in titers to influenza vaccine strains as compared to placebo. In FIG. 41A, the increase above baseline in influenza geometric mean titers to each of the 3 influenza vaccine strains (H1N1 A/California/ 07/2009, H3N2 A/Victoria/210/2009, B/Brisbane/60/ 2008) relative to the increase in the placebo cohort 4 weeks after vaccination is shown for each of the RAD001 dosing cohorts in the intention to treat population. The bold black line indicates the 1.2 fold increase in titers relative to placebo that is required to be met for 2 out of 3 influenza vaccine strains to meet the primary endpoint of the study. The star "^{∗}" indicates that the increase in GMT titer relative to placebo exceeds 1 with posterior probability of at least 80%. FIG 41B is a graph of the same data as in FIG. 41A for the subset of subjects with baseline influenza titers <= 1:40.

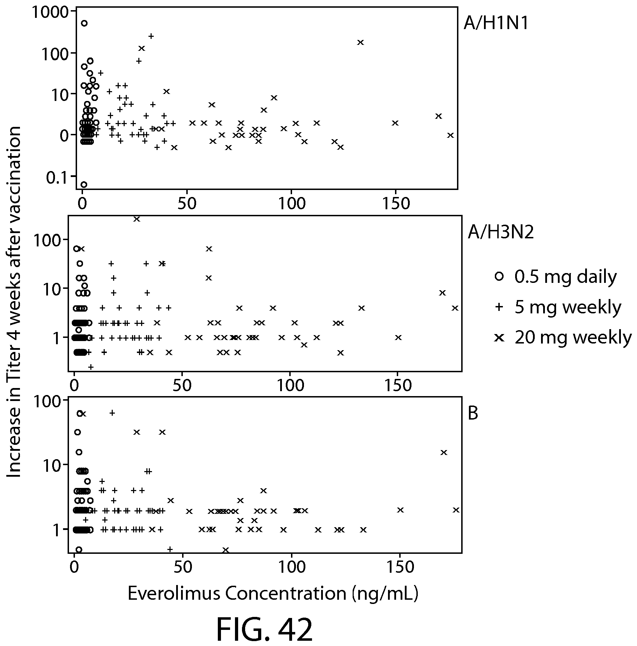

**Fig. 42** shows a set of scatter plots of RAD001 concentration versus fold increase in geometric mean titer to each influenza vaccine strain 4 weeks after vaccination. RAD001 concentrations (1 hour post dose) were measured after subjects had been dosed for 4 weeks. All subjects who had pharmacokinetic measurements were included in the analysis set. The fold increase in geometric mean titers at 4 weeks post vaccination relative to baseline is shown on the y axis.

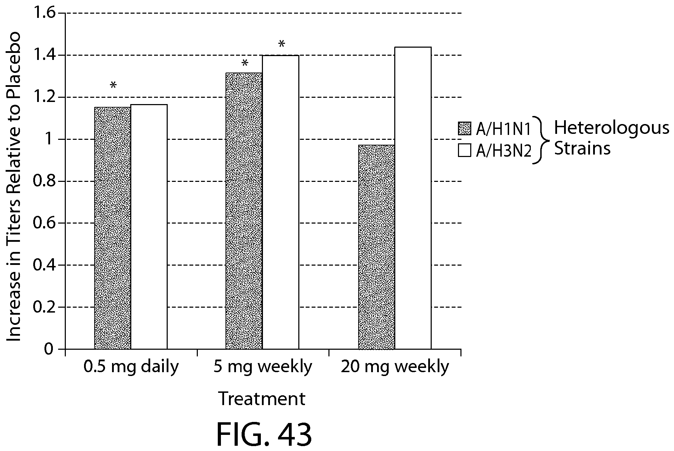

**Fig. 43** is a graphic representation showing increase in titers to heterologous influenza strains as compared to placebo. The increase above baseline in influenza geometric mean titers to 2 heterologous influenza strains (A/H1N1 strain A/New Jersey/8/76 and A/H3N2 strain A/Victoria/361/11) not contained in the influenza vaccine relative to the increase in the placebo cohort 4 weeks after vaccination is shown for each of the RAD001 dosing cohorts in the intention to treat population. ^{∗} indicates increase in titer relative to placebo exceeds 1 with a posterior probability of at least 80%.

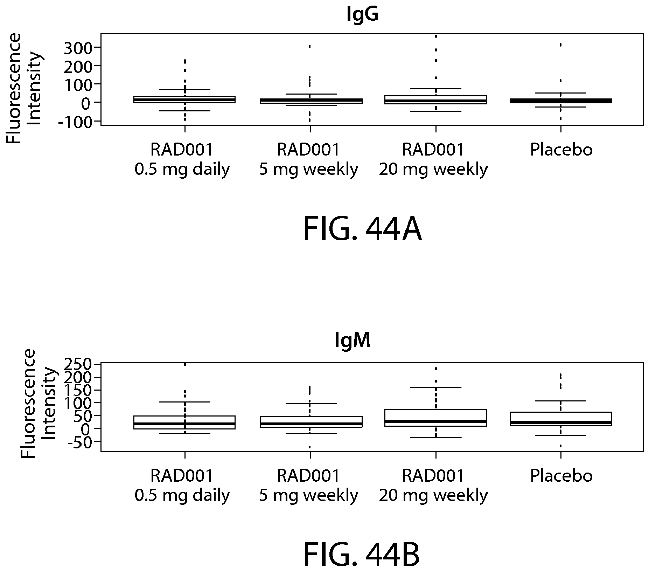

**Fig. 44****,** comprising **Figs. 44A** and **44B**, are graphic representations of IgG and IgM levels before and after influenza vaccination. Levels of anti-A/H1N1/California/07/2009 influenza IgG and IgM were measured in serum obtained from subjects before and 4 weeks post influenza vaccination. No significant difference in the change from baseline to 4 weeks post vaccination in anti-H1N1 influenza IgG and IgM levels were detected between the RAD001 and placebo cohorts (all p values > 0.05 by Kruskal-Wallis rank sum test).

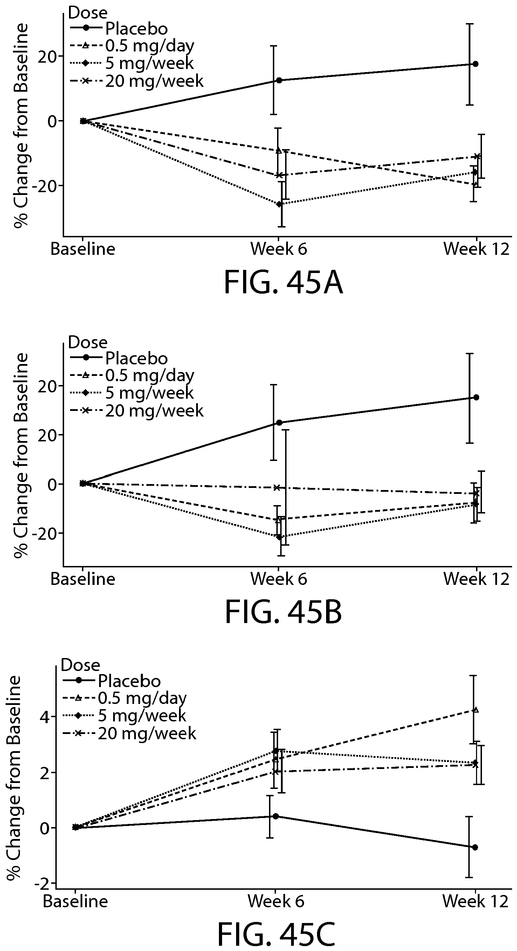

**Fig. 45**, comprising **Figs. 45A**, **45B**, and **45C** are graphic representations of the decrease in percent of PD-1-positive CD4 and CD8 and increase in PD-1-negative CD4 T cells after RAD001 treatment. The percent of PD-1-positive CD4, CD8 and PD-1-negative CD4 T cells was determined by FACS analysis of PBMC samples at baseline, after 6 weeks of study drug treatment (Week 6) and 6 weeks after study drug discontinuation and 4 weeks after influenza vaccination (Week 12). FIG. 45A shows there was a significant decrease (-37.1 - -28.5%) in PD-1-positive CD4 T cells at week 12 in cohorts receiving RAD001 at dose levels 0.5mg/Day (n=25), 5mg/Week (n=29) and 20 mg/Week (n=30) as compared to the placebo cohort (n=25) with p=0.002 (0.02), p=0.003 (q=0.03), and p= 0.01 (q=0.05) respectively. FIG. 45B shows there was a significant decrease (-43.3 - -38.5%) in PD-1-positive CD8 T cells at week 12 in cohorts receiving RAD001 (n=109) at dose levels 0.5mg/Day (n=25), 5mg/Week (n=29) and 20 mg/Week (n=30) as compared to the placebo cohort (n=25) with p=0.01 (0.05), p=0.007 (q=0.04), and p= 0.01 (q=0.05) respectively. FIG. 45C shows was a significant increase (3.0 - 4.9%) in PD-1-negative CD4 T cells at week 12 in cohorts receiving RAD001 (n=109) at dose levels 0.5mg/Day (n=25), 5mg/Week (n=29) and 20 mg/Week (n=30) as compared to the placebo cohort (n=25) with p=0.0007 (0.02), p=0.03 (q=0.07), and p= 0.03 (q=0.08) respectively.

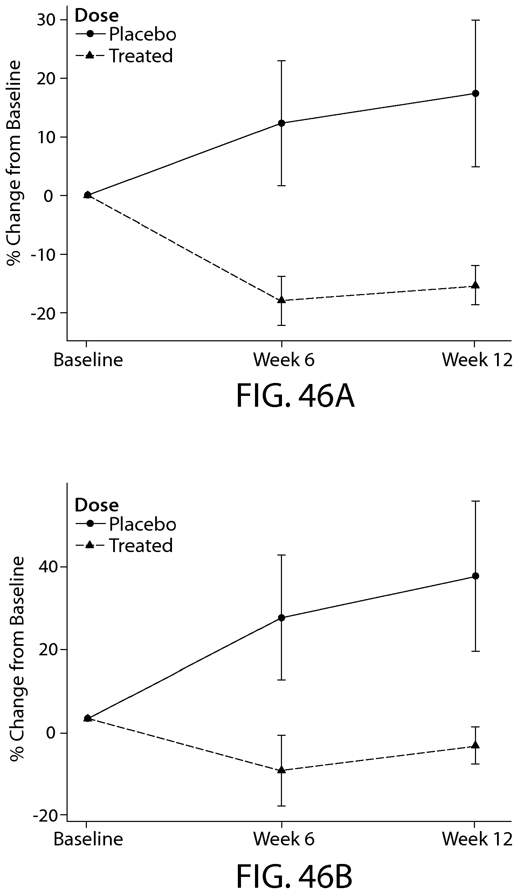

**Fig. 46****,** comprising **Figs. 46A** and **46B****,** are graphic representations of the decrease in percent of PD-1-positive CD4 and CD8 and increase in PD-1-negative CD4 T cells after RAD001 treatment. The percent of PD-1-positive CD4, CD8 and PD-1-negative CD4 T cells was determined by FACS analysis of PBMC samples at baseline, after 6 weeks of study drug treatment (Week 6) and 6 weeks after study drug discontinuation and 4 weeks after influenza vaccination (Week 12). FIG. 46A shows there was a significant decrease (-37.1 - -28.5%) in PD-1-positive CD4 T cells at week 12 in cohorts receiving RAD001 at dose levels 0.5mg/Day (n=25), 5mg/Week (n=29) and 20 mg/Week (n=30) as compared to the placebo cohort (n=25) with p=0.002 (0.02), p=0.003 (q=0.03), and p= 0.01 (q=0.05) respectively. FIG. 46B shows there was a significant decrease (-43.3 - -38.5%) in PD-1-positive CD8 T cells at week 12 in cohorts receiving RAD001 (n=109) at dose levels 0.5mg/Day (n=25), 5mg/Week (n=29) and 20 mg/Week (n=30) as compared to the placebo cohort (n=25) with p=0.01 (0.05), p=0.007 (q=0.04), and p= 0.01 (q=0.05) respectively.

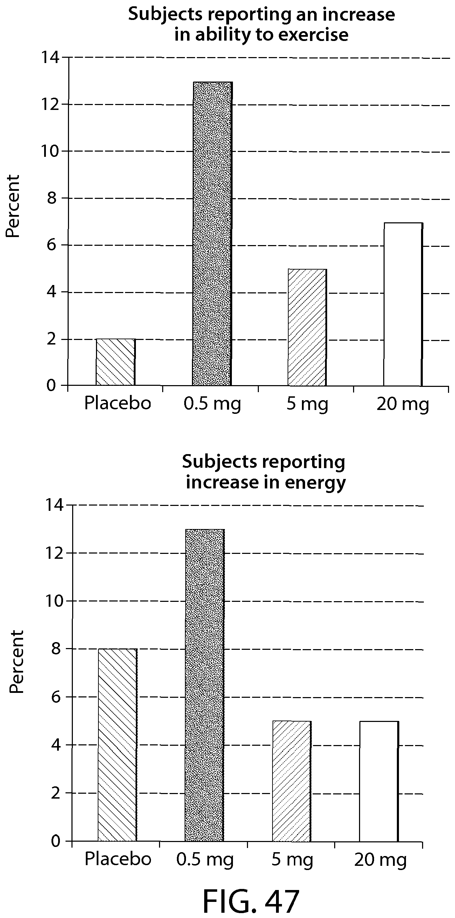

**Fig. 47** is a set of graphs depicting increases in exercise and energy in elderly subjects in response to RAD001.

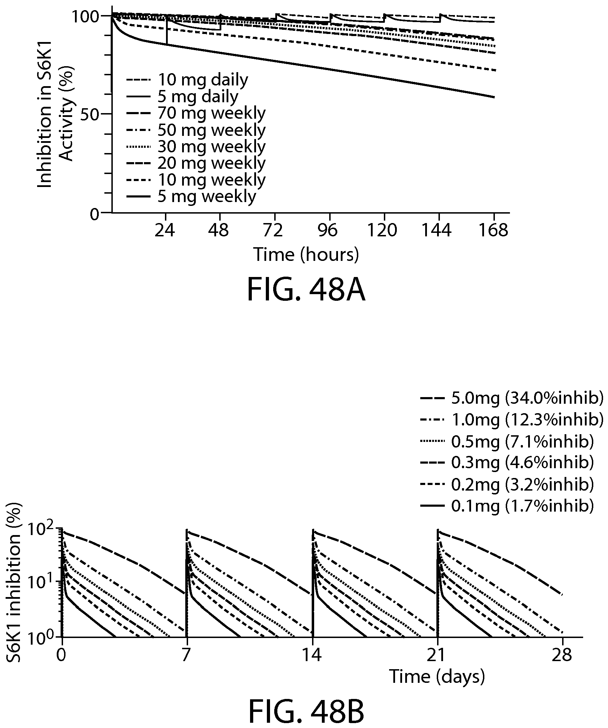

**Fig. 48**, comprising **Figs. 48A** and **48B**, depict the effect of RAD001 on P70 S6K activity in cell lines. FIG. 48A depicts P70 S6 kinase inhibition with higher doses of weekly and daily RAD001; FIG. 48B depicts P70 S6 kinase inhibition with lower doses of weekly RAD001.

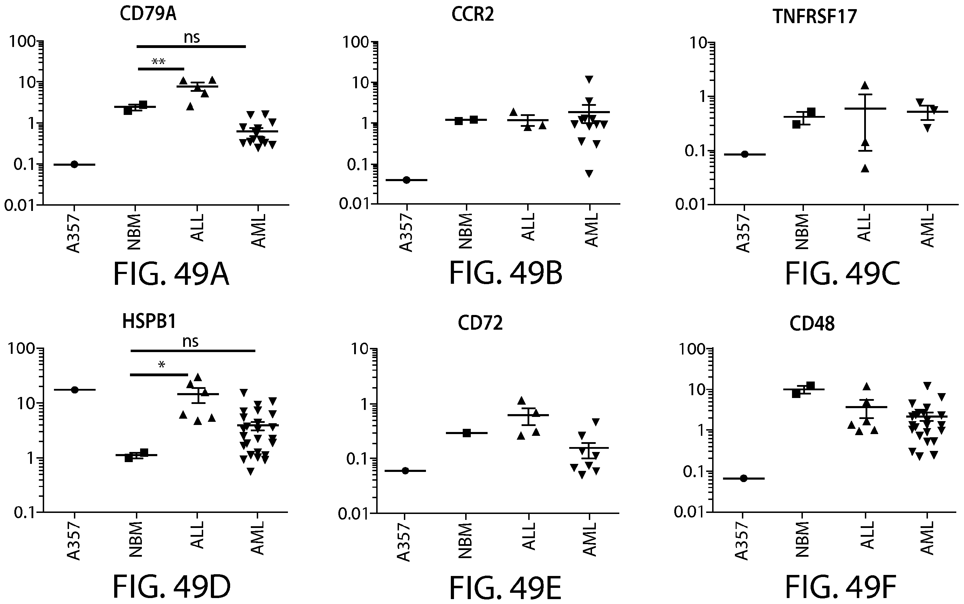

**Fig. 49A, 49B, 49C, 49D, 49E, and 49F****.** Graphs represent normalized MFI values for the following genes in individual samples: CD79A (Fig. 49A), CCR2 (Fig. 49B), TNFRSF17 (Fig. 49C), HSPB1 (Fig. 49D), CD72 (Fig. 49E), and CD48 (Fig. 49F). Y axis represents normalized MFI; X axis represents the target gene.

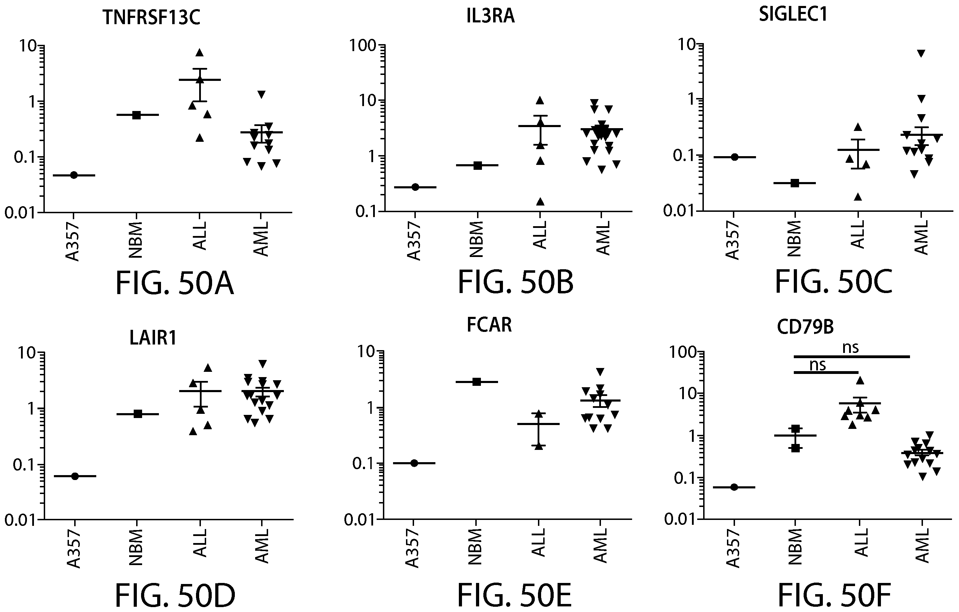

**Fig. 50A, 50B, 50C, 50D, 50E, and 50F****.** Graphs represent normalized MFI values for the following genes in individual samples: TNFRSF13C (Fig. 50A), IL3RA (Fig. 50B), SIGLEC1 (Fig. 50C), LAIR1 (Fig. 50D), FCAR (Fig. 50E), and CD79B (Fig. 50F). Y axis represents normalized MFI; X axis represents the target gene.

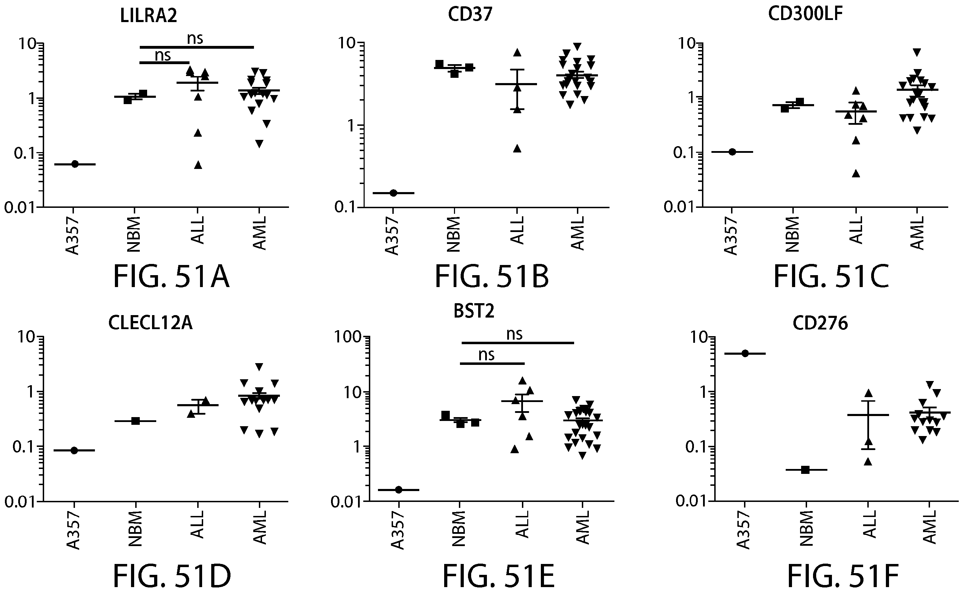

**Fig. 51A, 51B, 51C, 51D, 51E, and 51F****.** Graphs represent normalized MFI values for the following genes in individual samples: LILRA2 (Fig. 51A), CD37 (Fig. 51B), CD300LF (Fig. 51C), CLEC12A (Fig. 51D), BST2 (Fig. 51E), and CD276 (Fig. 51F). Y axis represents normalized MFI; X axis represents the target gene.

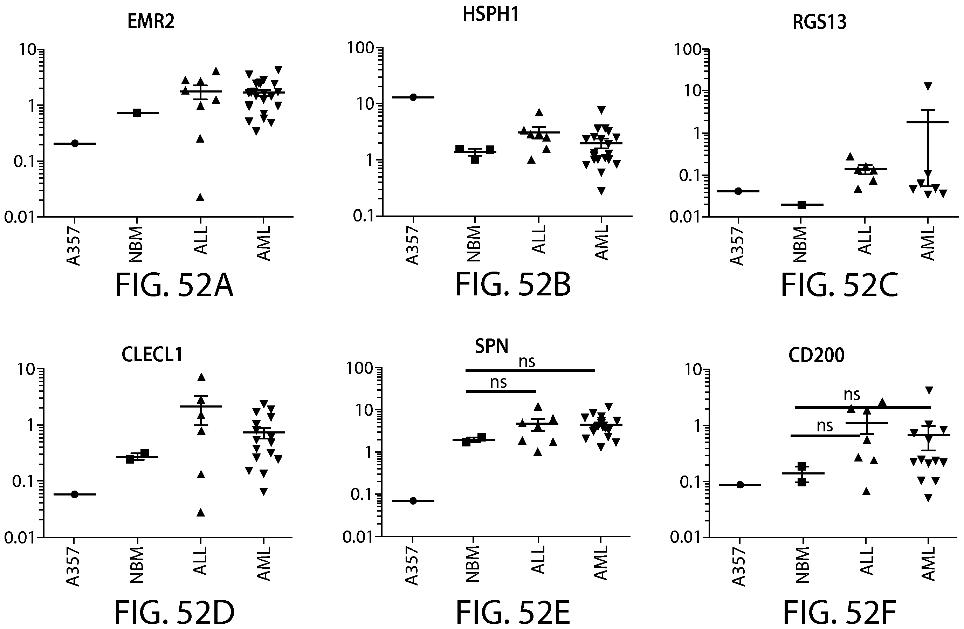

**Fig. 52A, 52B, 52C, 52D, 52E, and 52F****.** Graphs represent normalized MFI values for the following genes in individual samples: EMR2 (Fig. 52A), HSPH1 (Fig. 52B), RGS13 (Fig. 52C), CLECL1 (Fig. 52D), SPN (Fig. 52E), and CD200 (Fig. 52F). Y axis represents normalized MFI; X axis represents the target gene.

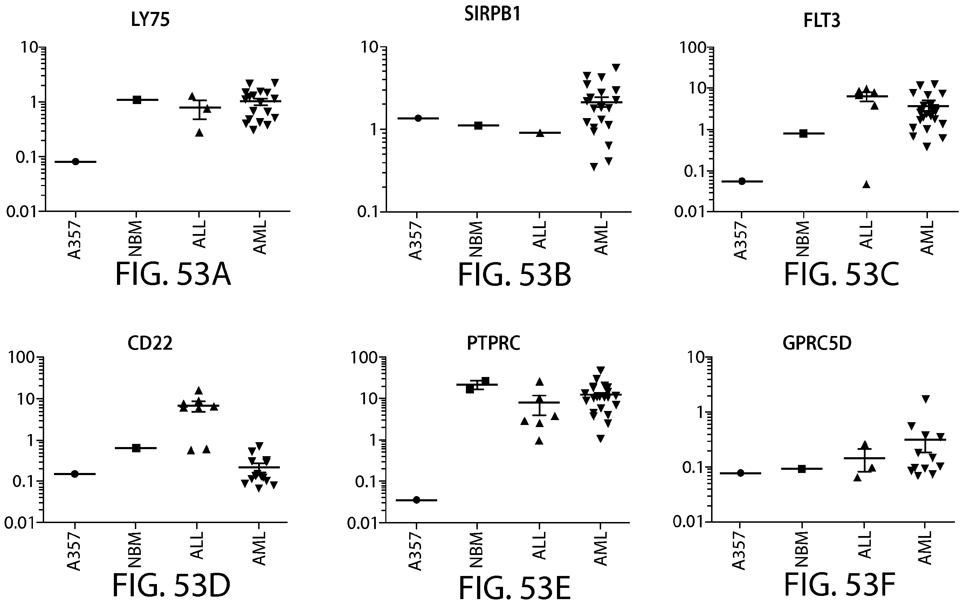

**Fig. 53A, 53B, 53C, 53D, 53E, and 53F****.** Graphs represent normalized MFI values for the following genes in individual samples: LY75 (Fig. 53A), SIRPB1 (Fig. 53B), FLT3 (Fig. 53C), CD22 (Fig. 53D), PTPRC (Fig. 53E), and GPRC5D (Fig. 53F). Y axis represents normalized MFI; X axis represents the target gene.

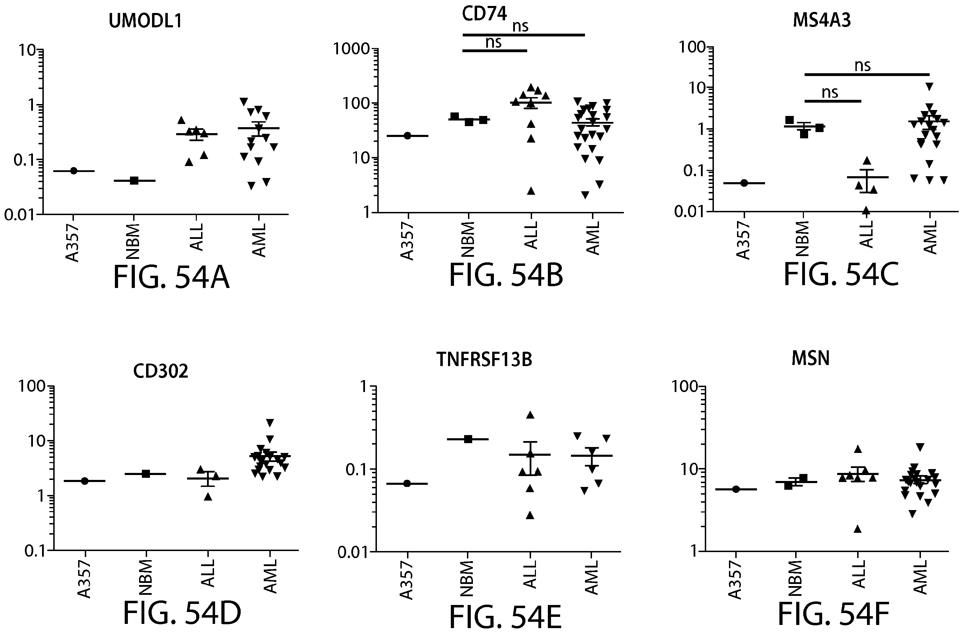

**Fig. 54A, 54B, 54C, 54D, 54E, and 54F****.** Graphs represent normalized MFI values for the following genes in individual samples: UMODL1 (Fig. 54A), CD74 (Fig. 54B), MS4A3 (Fig. 54C), CD302 (Fig. 54D), TNFRFSF13B (Fig. 54E), and MSN (Fig. 54F). Y axis represents normalized MFI; X axis represents the target gene.

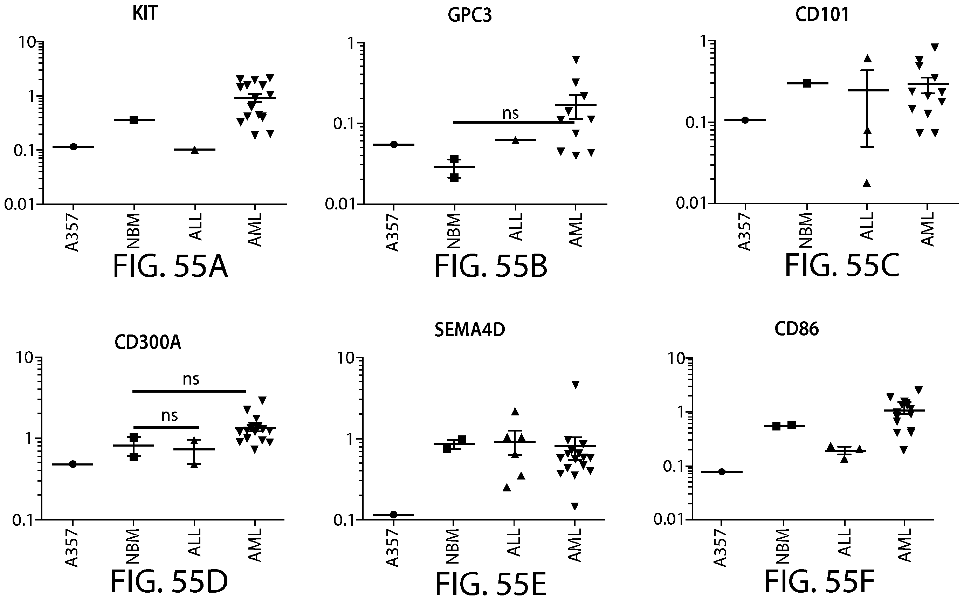

**Fig. 55A, 55B, 55C, 55D, 55E, and 55F****.** Graphs represent normalized MFI values for the following genes in individual samples: KIT (Fig. 55A), GPC3 (Fig. 55B), CD101 (Fig. 55C), CD300A (Fig. 55D), SEMA4D (Fig. 55E), and CD86 (Fig. 55F). Y axis represents normalized MFI; X axis represents the target gene.

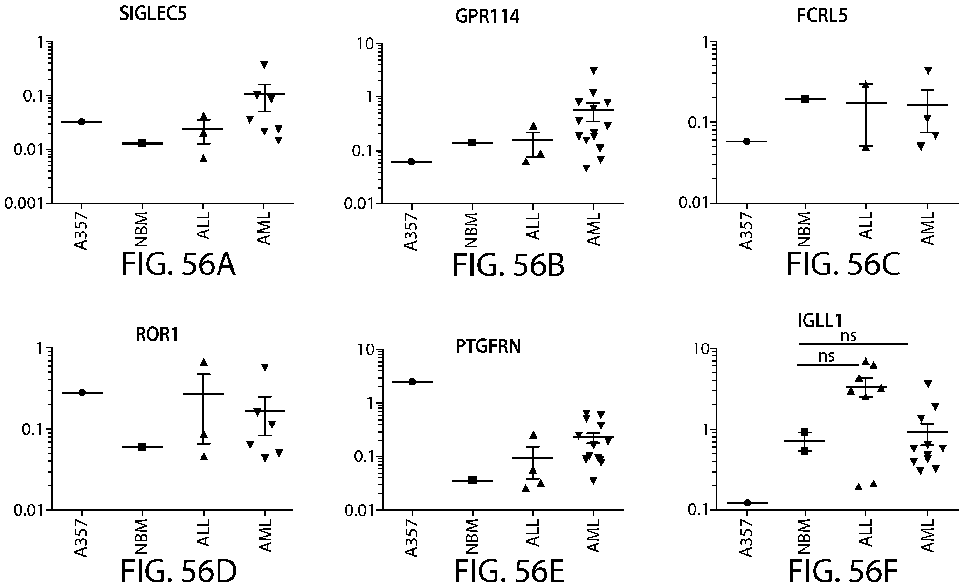

**Fig. 56A, 56B, 56C, 56D, 56E, and 56F****.** Graphs represent normalized MFI values for the following genes in individual samples: SIGLEC5 (Fig. 56A), GPR114 (Fig. 56B), FCRL5 (Fig. 56C), ROR1 (Fig. 56D), PTGFRN (Fig. 56E), and IGLL1 (Fig. 56F). Y axis represents normalized MFI; X axis represents the target gene.

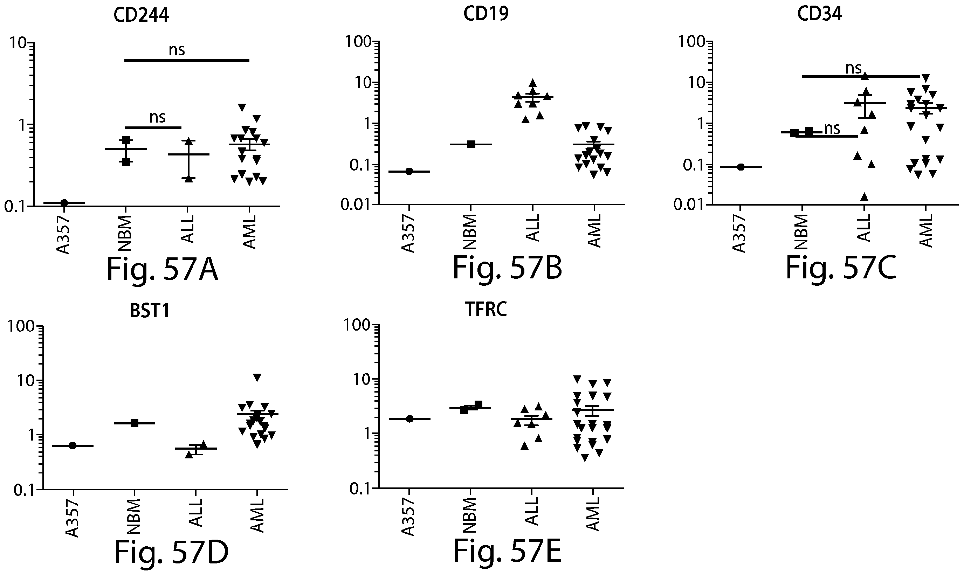

**Fig. 57A, 57B, 57C, 57D, and 57E****.** Graphs represent normalized MFI values for the following genes in individual samples: CD244 (Fig. 57A), CD19 (Fig. 57B), CD34 (Fig. 57C), BST1 (Fig. 57D), and TFRC (Fig. 57E). Y axis represents normalized MFI; X axis represents the target gene.

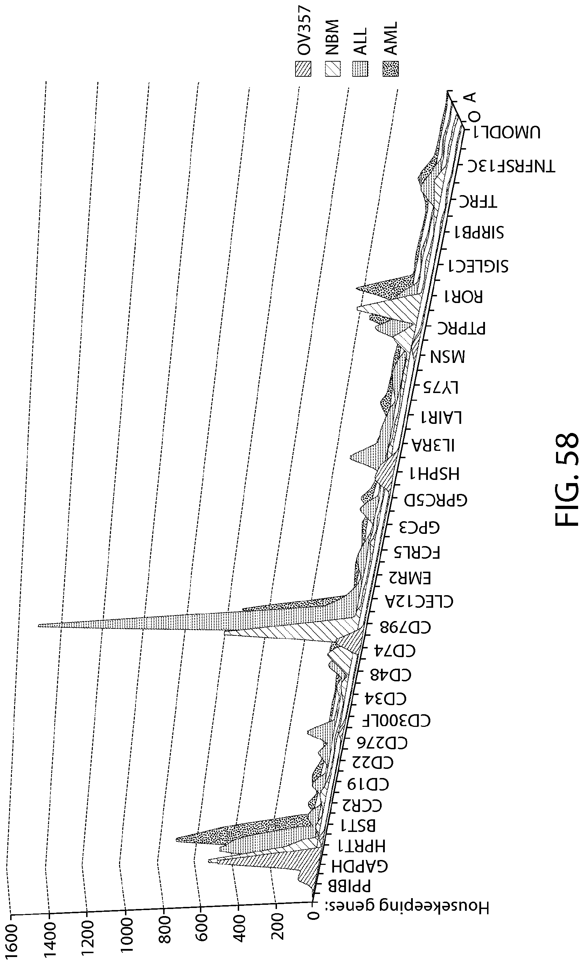

**Fig. 58**. Cumulative representation of the average normalized MFI values relative to PP1B housekeeping gene from AML, ALL, normal bone marrow (NBM), or OV357 cell line (OV357). Y axis represents normalized MFI; X axis represents the target genes.

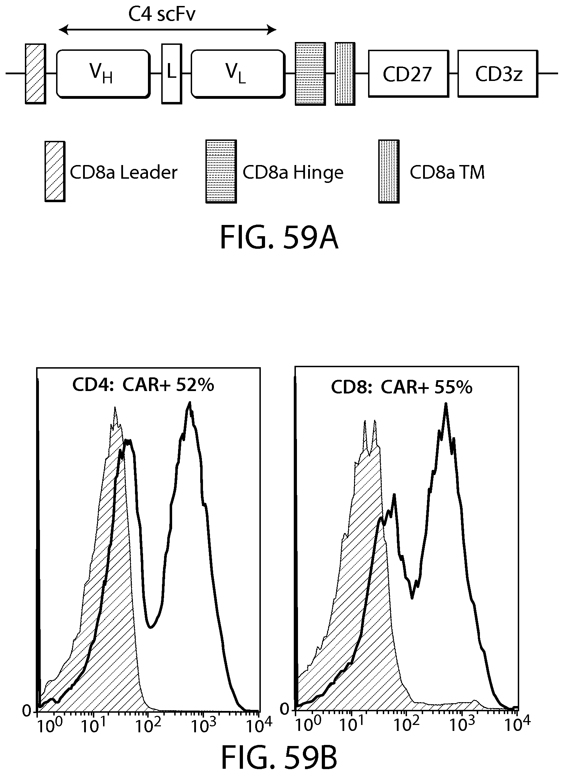

**Fig. 59A and 59B****.** Generation of T cells expressing CAR that targets Folate Receptor α. Fig. 59A is a schematic diagram of C4-27z CAR vector. Fig. 59B is a set of representative FACS histogram plots of CAR expression on CD4+ and CD8+ T cells 48 hours after lentiviral transduction.

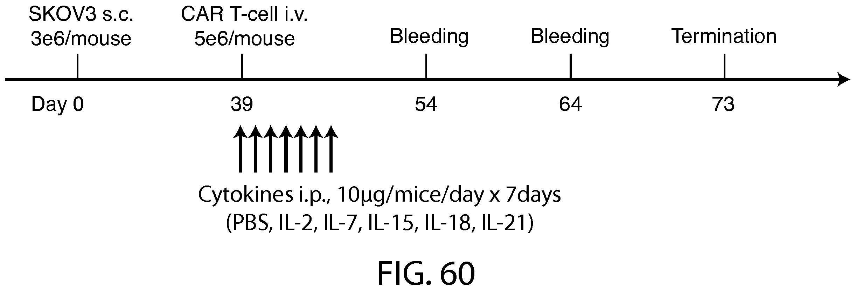

**Fig. 60****.** Schematic diagram of the in vivo experiment for testing different cytokines in combination with CAR-T cells in an ovarian tumor mouse model.

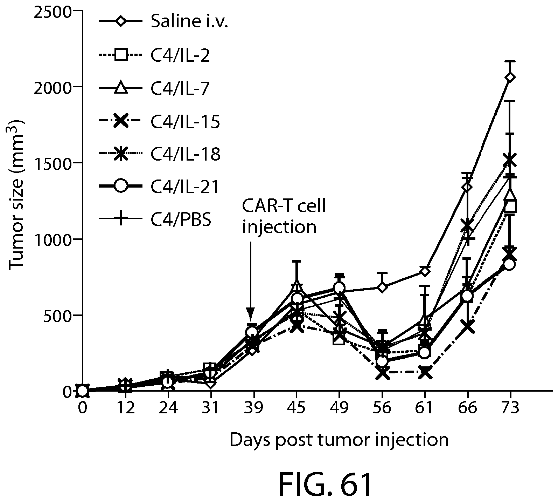

**Fig. 61****.** Tumor growth curve of mice treated with various cytokine exposed C4-27z CAR-T cells, anti-CD19-27z CAR-T cells and untransduced T cells. The data are presented as mean value ± SEM. The arrow indicates the time of T cell infusion.

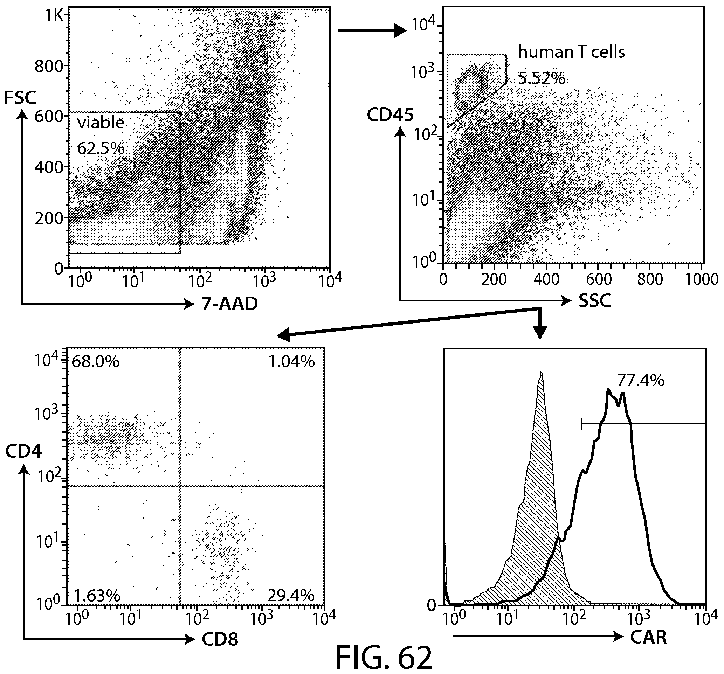

**Fig. 62****.** Bioluminescence images show fLuc+ SKOV3 tumors in NSG mice immediately before (day 38), two weeks (day53) and five weeks (day 74) after first intravenous injection of CAR-T cells.

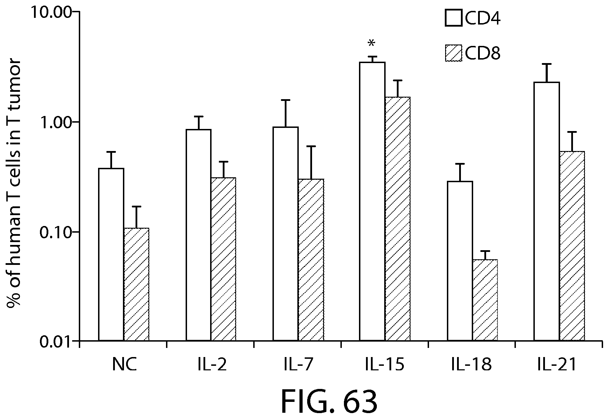

**Fig. 63****.** Quantitation of circulating human CD4+ and CD8+ T cell counts in mice peripheral blood 15 days after the first dose of CAR-T cell infusion.

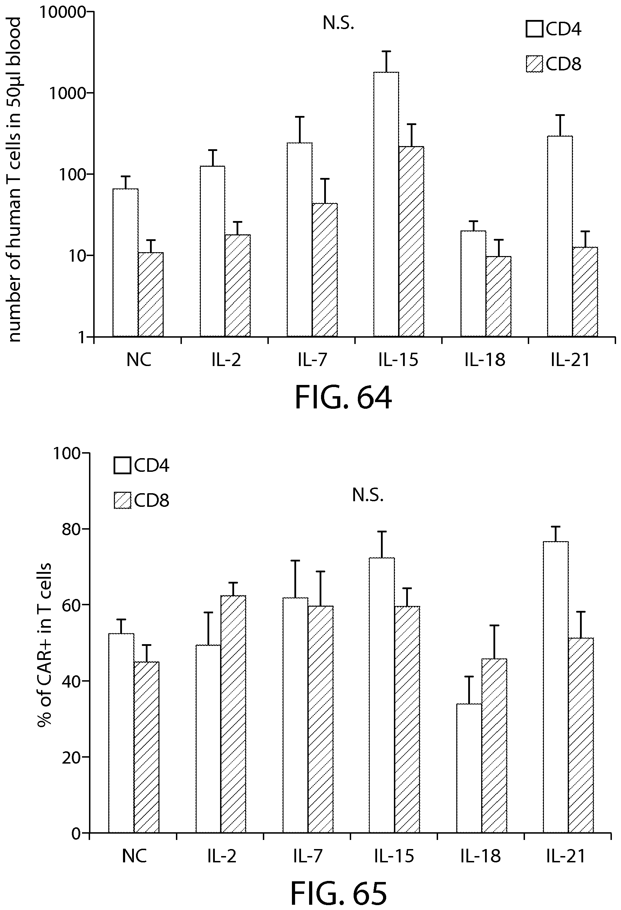

**Fig. 64****.** Quantitation of CAR expression on circulating human CD4+ and CD8+ T cells in mice blood.
**Fig. 65****.** Distribution of T-cell subsets of circulating human T cells in mice blood based on CD45RA and CD62L staining.

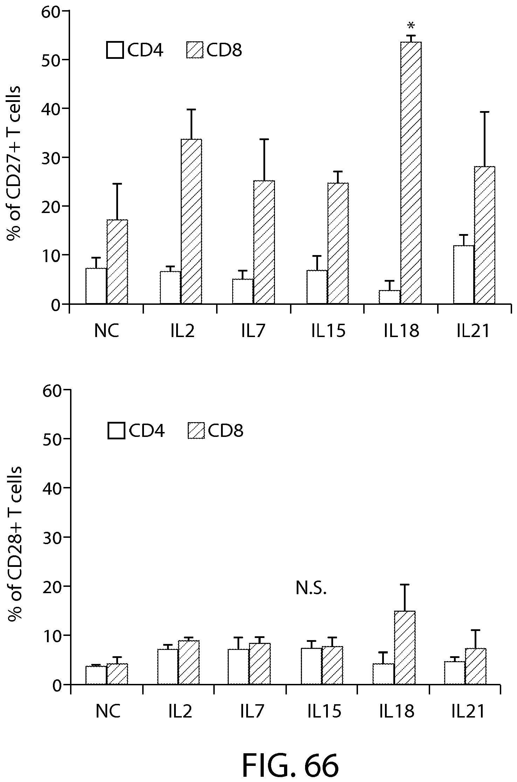

**Fig. 66****.** Set of graphs showing quantitation of CD27 and CD28 expression on circulating human CD4+ and CD8+ T cells in mice blood.

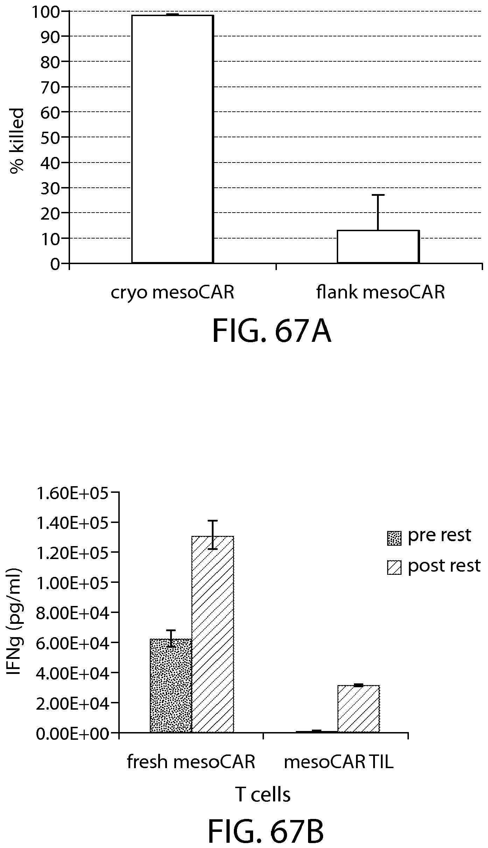

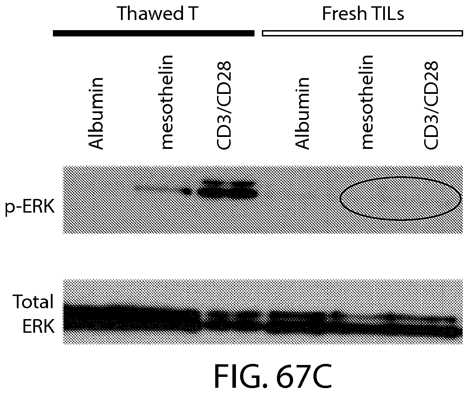

**Fig. 67A, 67B****, and** **67C**. Loss of fucntionality of mesoCAR T cells in the tumor microenvironment (TILs) over time compared to fresh or thawed mesoCAR T cells. A) Cytotoxicity assay (EMMESO TIL ex vivo killing assay (immediate harvest)); B) IFNγ release assay (IFNgamma level after 20 hr 50:1 coculture with 5K EMMESO/ffluc cells pre/post 30IU/ml IL2 overnight rest); and C) western blot analysis of ERK signaling (via phosphorylation).

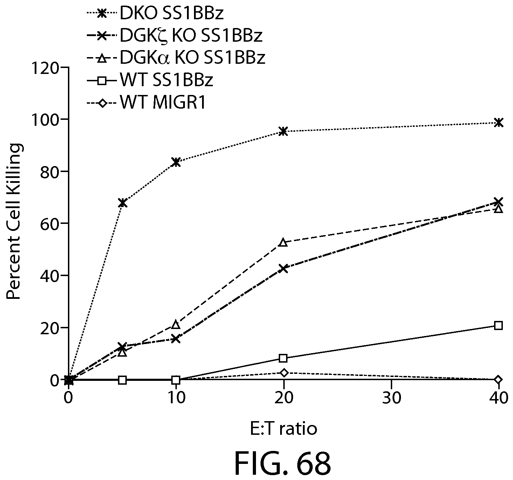

**Fig. 68**. The effect of deletion of DGK on cytotoxicity of mesoCAR T cells. Percent target cell killing is assessed at different effector:target ratios.

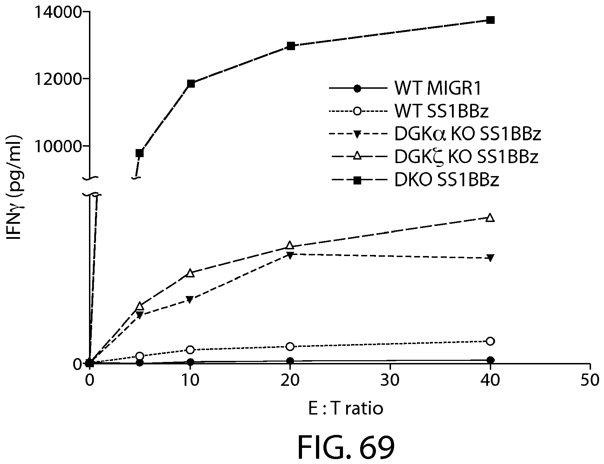

**Fig. 69**. The effect of deletion of DGK on IFNγ production and release from mesoCAR T cells. Concentration of IFNγ is assessed at different effector:target ratios.

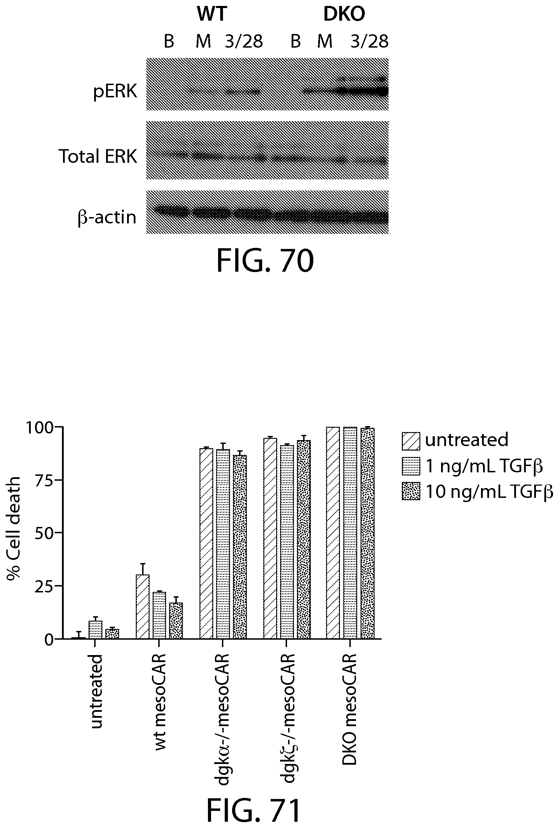

**Fig. 70****.** The effect of deletion of DGK on ERK signaling, or T cell activation, mesoCAR T cells. B: albumin, M: mesothelin, 3/28: CD3/CD28 stimulated cells.
**Fig. 71****.** The effect of deletion of DGK on TGFβ sensitivity of mesoCAR T cells with regard to cytotoxic activity.

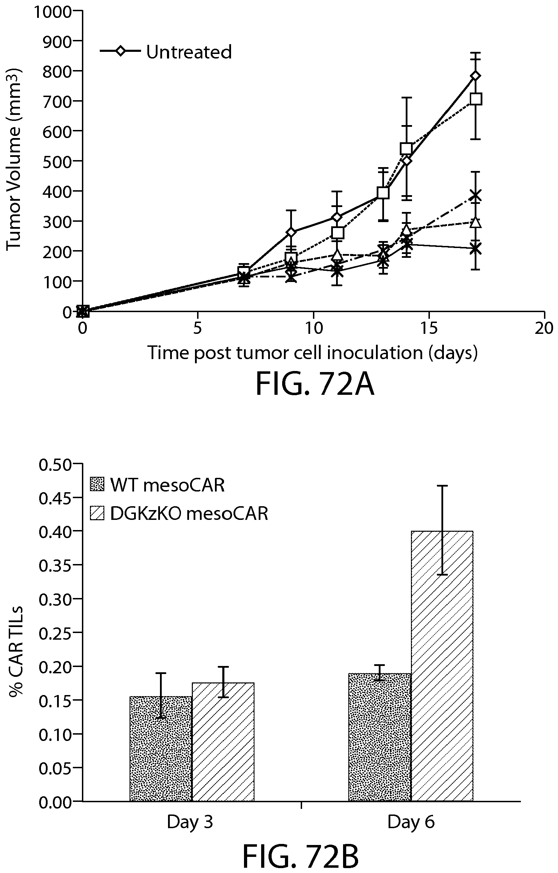

**Fig. 72A and 72B****.** The effect of deletion of DGK on therapeutic efficacy of mesoCAR T cells in a tumor mouse model. A) Effect on anti-tumor activity is shown by tumor volume over time. B) Persistance and proliferation of tumor infiltrating cells.

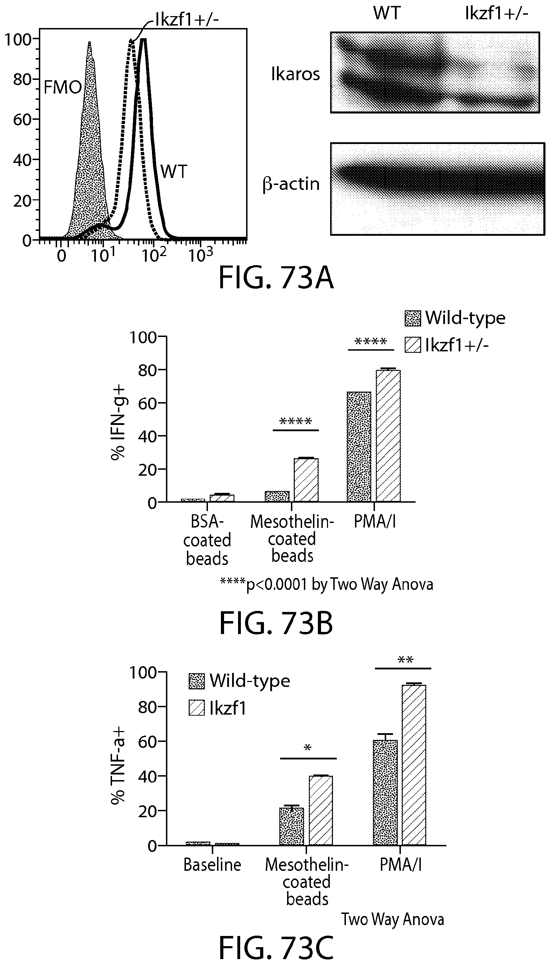

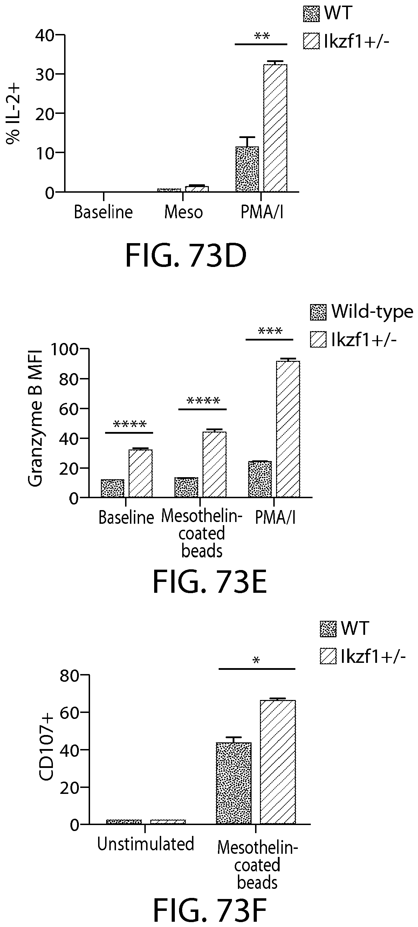

**Fig. 73A, 73B, 73C****,** **73D, 73E, and 73F** shows the cytokine production and cytotoxic mediator release in CAR-expressing T cells with reduced levels of Ikaros. Fig. 73A shows Ikaros expression in wild-type and *Ikzf1*+/- CAR T cells as measured by flow cytometry (left panel) and western blot (right panel). Following stimulation with mesothelin-coated beads, PMA/Ionomycin (PMA/I), or BSA-coated beads (control), the percentage of cells producing IFN-γ (FIG. 73B), TNF-α (FIG. 73C), and IL-2 (FIG. 73D), the cytotoxic mediator granzyme B (FIG. 73E), and CD107a expression (FIG. 73F) was determined.

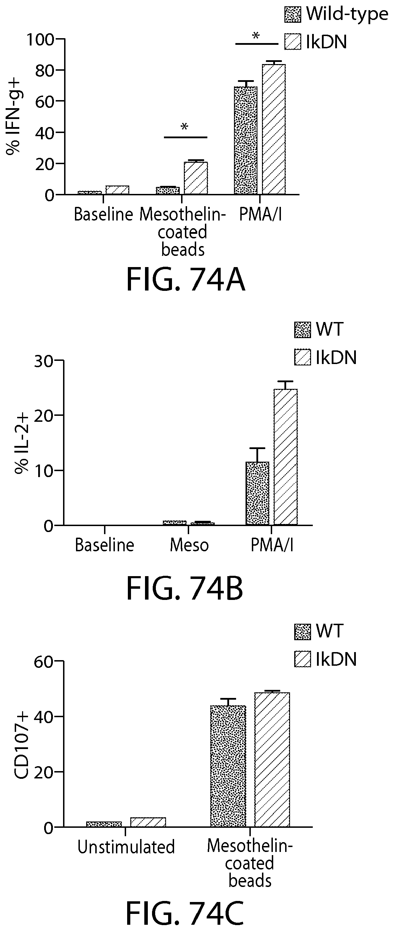

**Fig. 74A, 74B, and 74C****.** Cytokine production and cytotoxic mediator release in CAR-expressing T cells with a dominant negative allele of Ikaros (IkDN). Following stimulation with mesothelin-coated beads, PMA/Ionomycin (PMA/I), or BSA-coated beads (control), the percentage of cells producing IFN-γ (FIG. 74A), IL-2 (FIG. 74B), and CD107a expression (FIG. 74C) was determined.

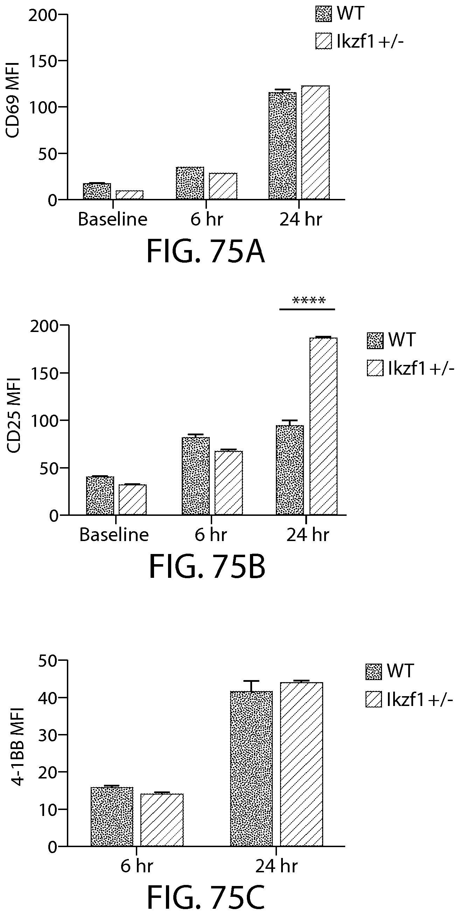

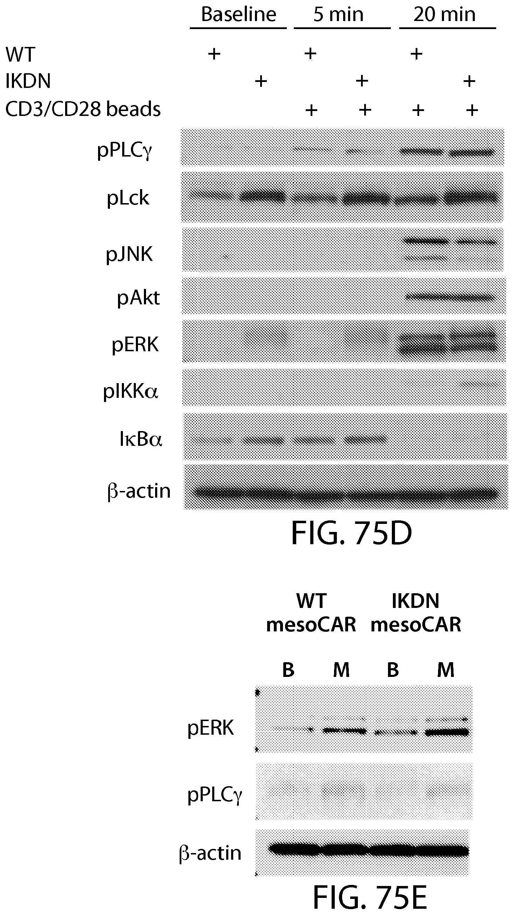

**Fig. 75A, 75B, 75C****,** **75D, and 75E****.** The depletion of Ikaros did not augment activation and signaling of CAR T cells following antigen stimulation. The levels of CD69 (FIG. 75A), CD25 (FIG. 75B), and 4-1BB (FIG. 75C) was determined by flow cytometry at the indicated time points in *Ikzf1+*/*-* CAR T cells. In FIG. 75D, the RAS/ERK signaling pathways were examined in wild-type (WT) and Ikaros dominant negative cells (IkDN) after TCR stimulation with CD3/CD28 antibodies. The levels of phosphorylated TCR signaling proteins such as phosphorylated PLCγ, phosphorylated Lck, phosphorylated JNK, phosphorylated Akt, phosphorylated ERK, phosphorylated IKKα, and IκBα were assessed by western blot. In FIG. 75E, WT and IkDN cells transduced with mesoCAR were stimulated with BSA or mesothelin-coated beads, and downstream signaling pathways were examined by western blot by assessing the levels of phosphorylated ERK and phosphorylated PLCγ.

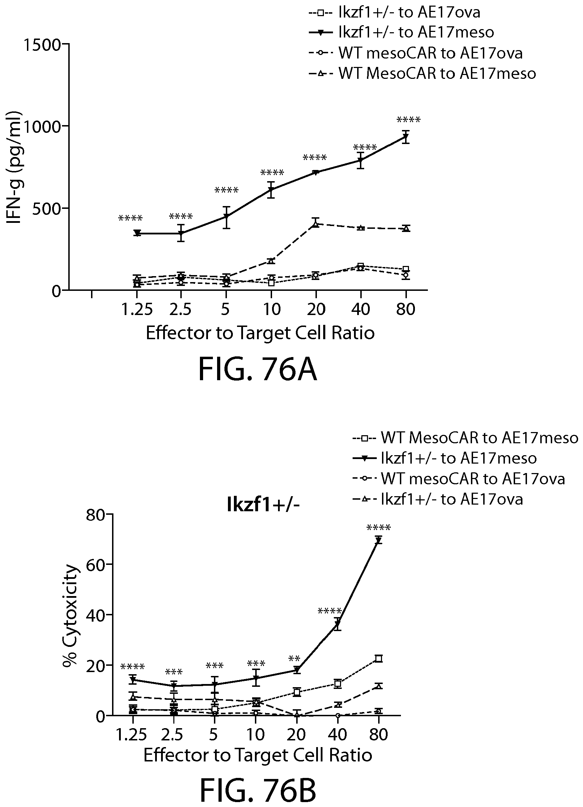

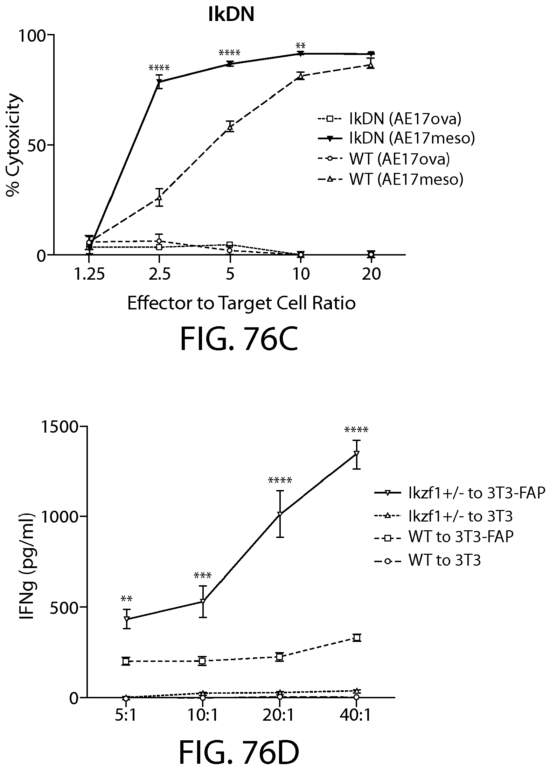

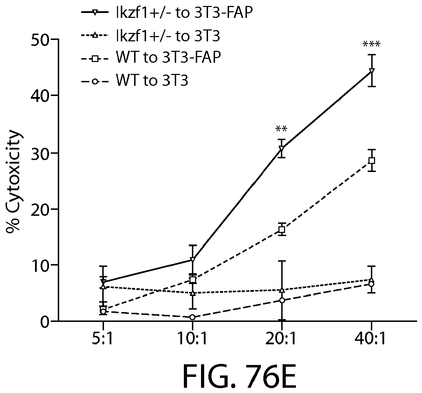

**Fig. 76A, 76B****,** **76C, 76D****, and** **76E****.** The reduction of Ikaros in CAR T cells augments the response against target cells AE17 or mesothelin-expressing AE17 (AE17 meso)*in vitro.* FIG. 76A depicts IFNγ production in WT and *Ikzf1+*/*-* meso CART cells at the indicated effector:target cell ratios. Cytolysis of meso CAR-expressing WT and *Ikzf1*+/-(FIG. 76B) and IkDN (FIG. 76C) was measured at the indicated effector:target cell ratios. IFNγ production (FIG. 76D) and cytolysis (FIG. 76E) of WT and *Ikzf1+*/*-* transduced with FAP-CAR was measured at the indicated effector:target cell ratios, where the target cells were FAP-expressing 3T3 cells.

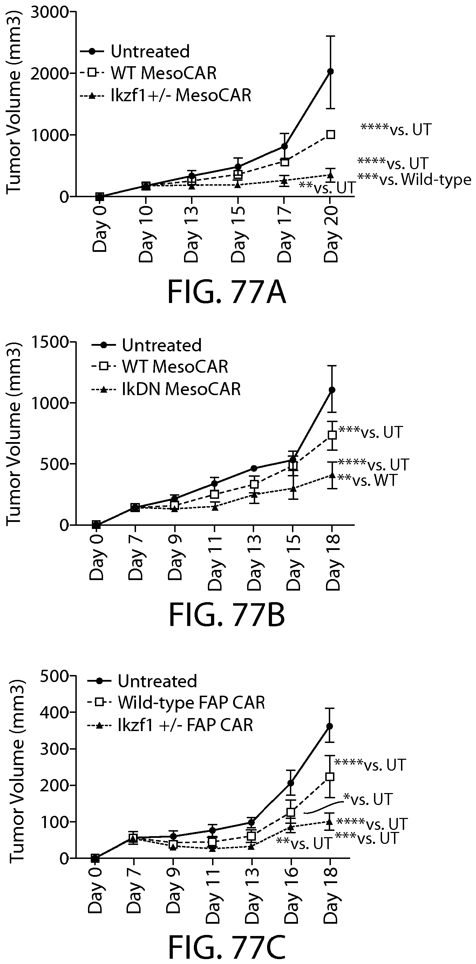

**Fig. 77A, 77B, and 77C****.** Efficacy of CAR T cells with depletion of Ikaros against established tumors *in vivo.* CAR T cells were administered to mice bearing established mesothelin-expressing AE17 tumors. Tumor volume was measured after administration with mesoCAR-expressing WT and *Ikzf1+*/*-* (FIG. 77A) or IkDN (FIG. 77B). Tumor volume was measured after administration of FAP-CAR-expressing WT and *Ikzf1*+/-(FIG. 75C).

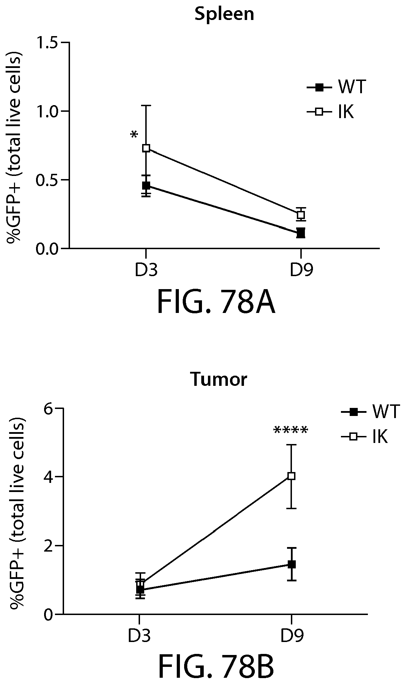

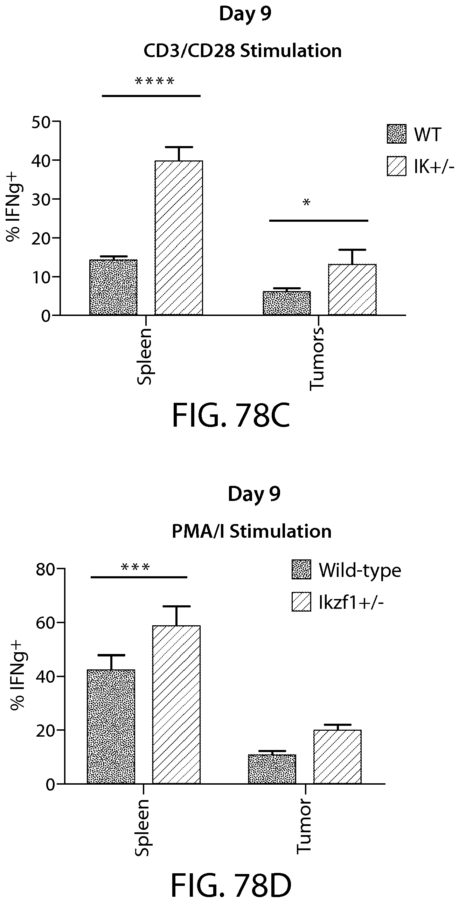

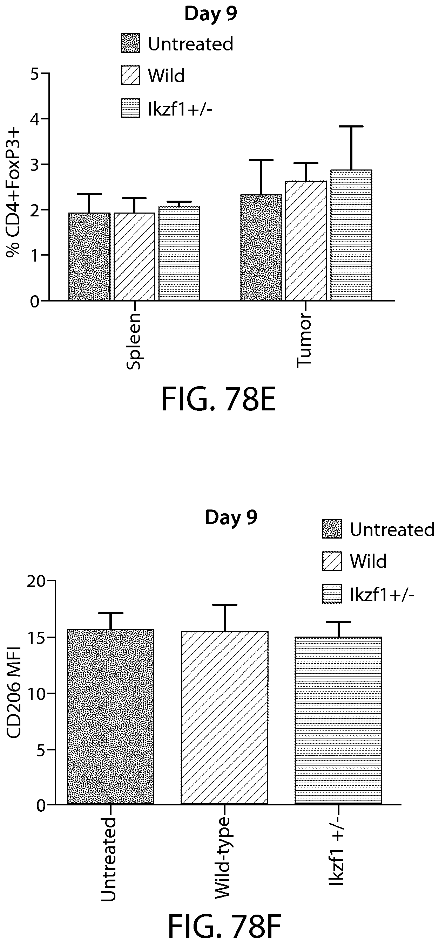

**Fig. 78A, 78B****,** **78C, 78D****,** **78E, and 78F****.** Increased persistence and resistance of *Ikzf1*+/- CAR T cells in the immunosuppressive tumor microenvironment compared to WT CAR T cells. The percentage of CAR-expressing WT or *Ikzf1+*/*-* cells (GFP positive) were detected by flow cytometry from harvested from the spleen (FIG. 78A) and the tumors (FIG. 78B). The functional capacity of the CAR T cells harvested 3 days after infusion from the spleen or tumors was assessed by measuring IFNγ production after stimulation with CD3/CD28 antibodies (FIG. 78C) or PMA/Ionomycin (PMA/I) (FIG. 78D). Regulatory T cells (CD4+FoxP3+ expression) and macrophages (CD206 expression) were assessed by measuring the expression of Treg or macrophage markers on CAR T cells harvested 9 days after infusion from the spleen or tumors.

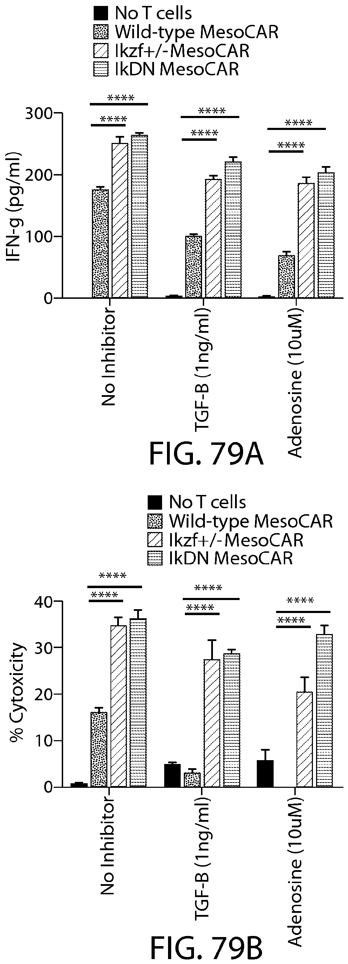

**Fig. 79A and 79B****.** T cells with reduced Ikaros levels are less sensitive to soluble inhibitory factors TGFβ and adenosine. MesoCAR-expressing WT, *Ikzf1*+/-, and IkDN cells were tested for their ability to produce IFNγ (FIG. 79A) and cytotoxicity (FIG. 79B) in response to TGF-β or adenosine.

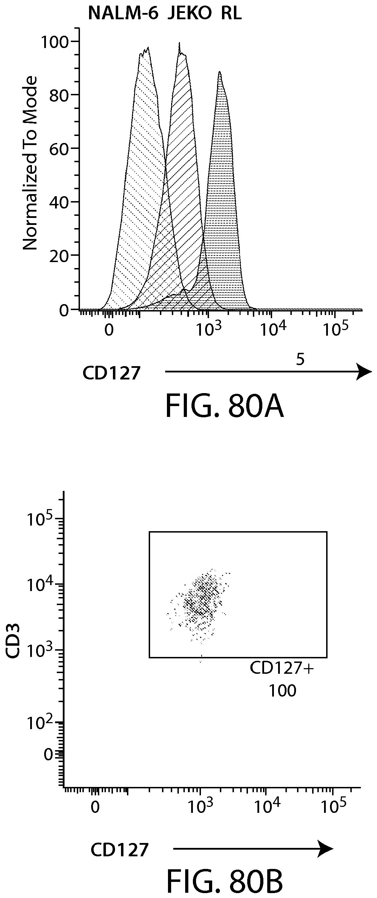

**Fig. 80A** and **80B** show IL-7 receptor (CD127) expression on cancer cell lines and CART cells. Expression of CD127 was measured by flow cytometry analysis in three cancer cell lines: RL (mantle cell lymphoma), JEKO (also known as Jeko-1, mantle cell lymphoma), and Nalm-6 (B-ALL) (Fig. 80A). CD127 expression was measured by flow cytometry analysis on CD3 positive (CART) cells that had been infused and circulating in NSG mice (Fig. 80B).

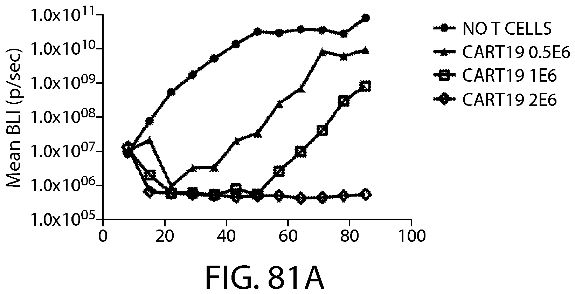

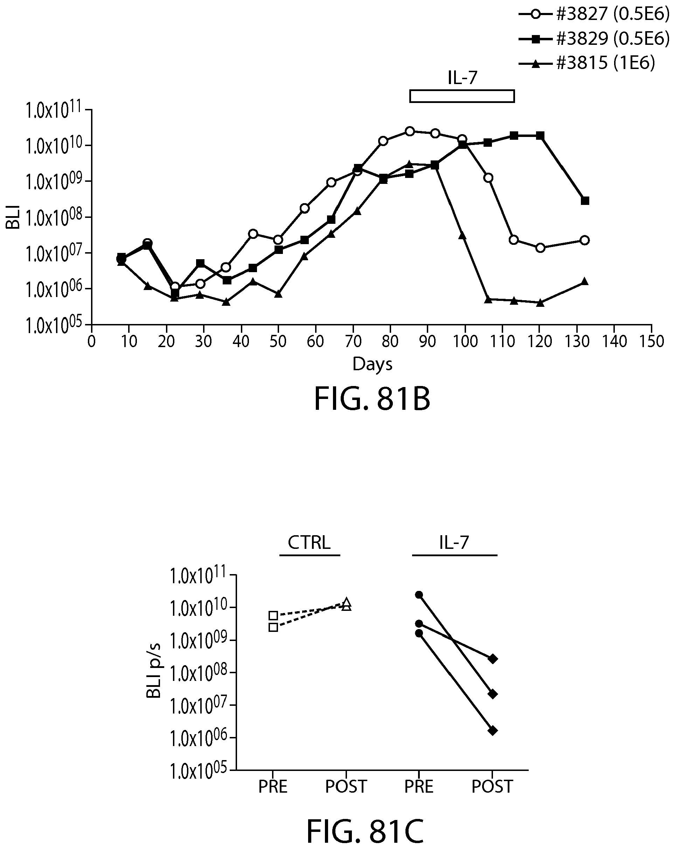

**Fig. 81A****,** **81B, and 81C** show the anti-tumor response after CART19 treatment and subsequent IL-7 treatment. NSG mice engrafted with a luciferase-expressing mantle lymphoma cell line (RL-luc) at Day 0 were treated with varying dosages of CART19 cells at Day 6, and tumor burden was monitored. Mice were divided into 4 groups and received no CART19 cells, 0.5×10⁶ CART19 cells (CART19 0.5E6), 1×10⁶ CART19 cells (CART19 1E6), or 2×10⁶ CART19 cells (CART19 2E6). Tumor burden after CART treatment was measured by detection of bioluminescence (mean BLI) (Fig. 81A). Mice receiving 0.5×10⁶ CART19 cells (CART19 0.5E6) or 1×10⁶ CART19 cells (CART19 1E6) were randomized to receive recominbant human IL-7 (rhIL-7) or not. Tumor burden, represented here by mean bioluminescence (BLI), was monitored for the three mice (#3827, #3829, and #3815, receiving the indicated initial CART19 dose) from Figure 81A that were treated with IL-7 starting at Day 85 (Fig. 81B). IL-7 was administered through IP injection 3 times weekly. Tumor burden, represented here by mean bioluminescence (BLI) before Day 85 (PRE) and after Day 115 (POST) was compared between mice that did not receive IL-7 (CTRL) and mice that received IL-7 treatment (IL-7) (Fig. 81C).

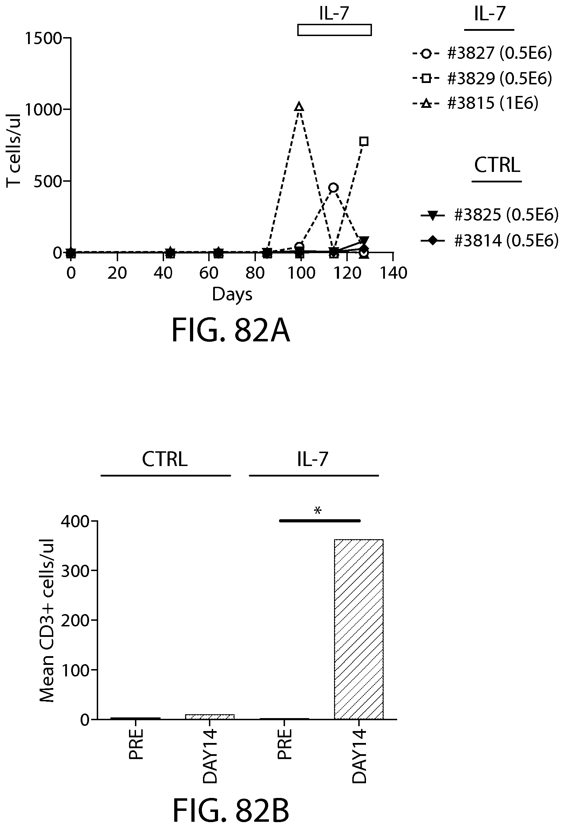

**Fig. 82A and 82B** show the T cell dynamics after IL-7 treatment. The level of human T cells detected in the blood was monitored for each of the mice receiving IL-7 or control mice (Fig. 82A). The level of CART19 cells (CD3+ cells) detected in the blood was measured before (PRE) and 14 days after (Day 14) initiation of IL-7 treatment (Fig. 82B).

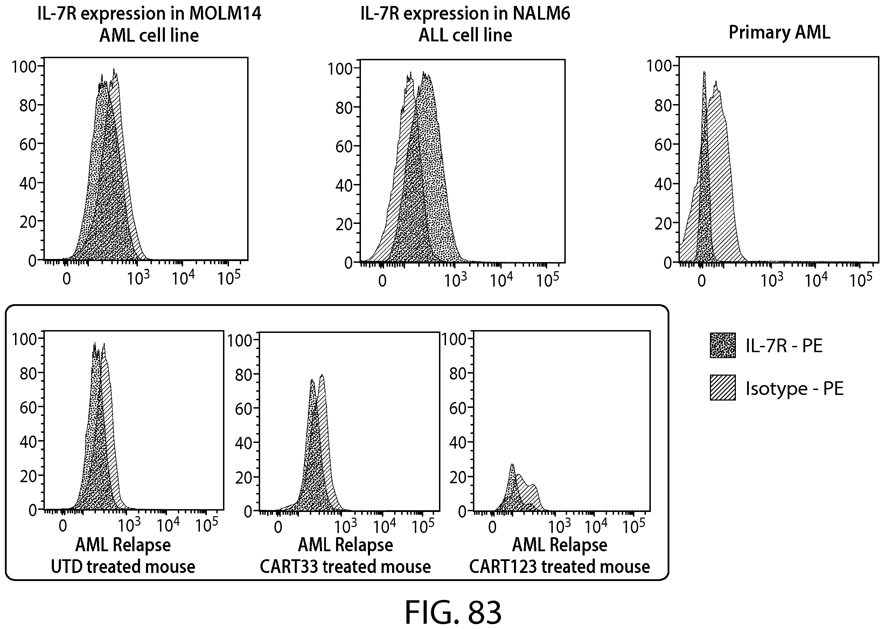

**Fig. 83** is a set of images that shows the IL-7 receptor (IL-7R) expression as detected by flow cytometry. Top panels show IL-7R in leukemia cells: AML cell line MOLM14 (top left), B-ALL cell line NALM6 (top middle), and primary AML (top right). Bottom panels show IL-7R in AML cells after relapse in mouse models of AML that have received CART treatment: untransduced T cells (UTD) (bottom left), CART33 treated mouse (bottom middle), and a CART123 treated mouse (bottom right).

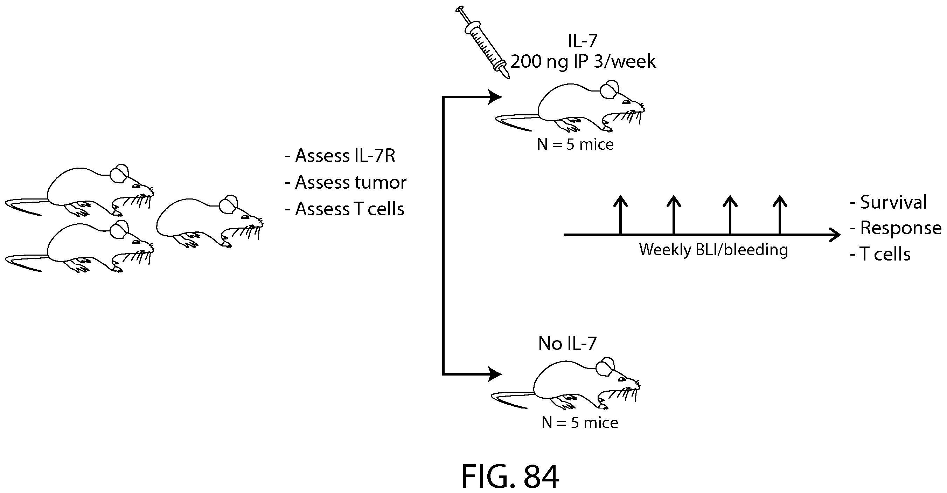

**Fig. 84** is a diagram showing the experimental schema for NSG mice were engrafted with luciferase+ MOLM14, and treated with untransduced cells (UTD), CART33 or CART123, followed by serial BLI to assess tumor burden. Mice that experienced a relapse after initial disease response were randomized to receive no treatment (no IL-7) or treatment with IL-7 200ng IP three times per week. Serial BLI was performed to assess tumor burden, survival, and T cell expansion.

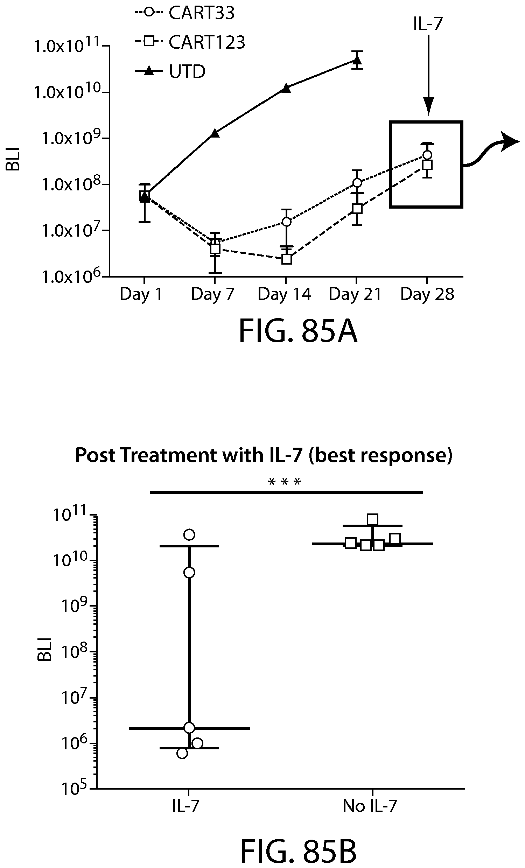

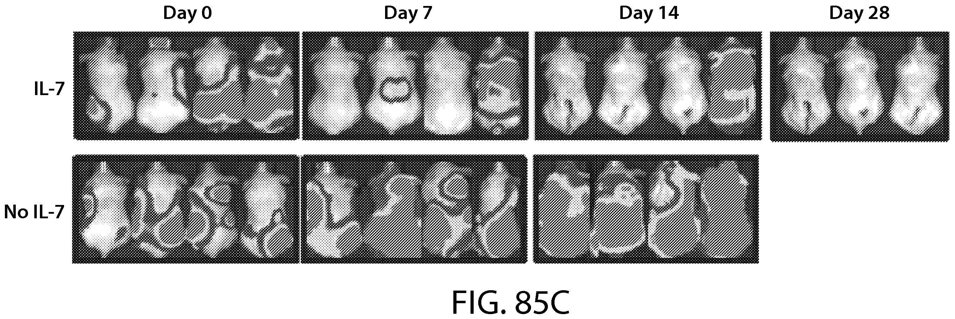

**Fig 85A, 85B****, and** **85C** show the anti-tumor response after CART treatment and subsequent IL-7 treatment. NSG mice engrafted with luciferase-expressing MOLM14 cells were treated with untransduced T cells (UTD), CART33 cells, or CART123 cells. Tumor burden after CART treatment was measured by bioluminescence imaging (BLI) (Fig. 85A). Mice that received CART123 or CART33 initially responded to T cell treatment but relapsed by 14 days after T cells. These mice were then assigned to treatment with IL-7 or not on day 28. Tumor burden after IL-7 (IL-7) or control treatment (no IL-7) was measured by bioluminescence imaging (BLI) (Fig. 85B). Representative images of bioluminescence imaging during IL-7 treatment are shown in Figure 85C.

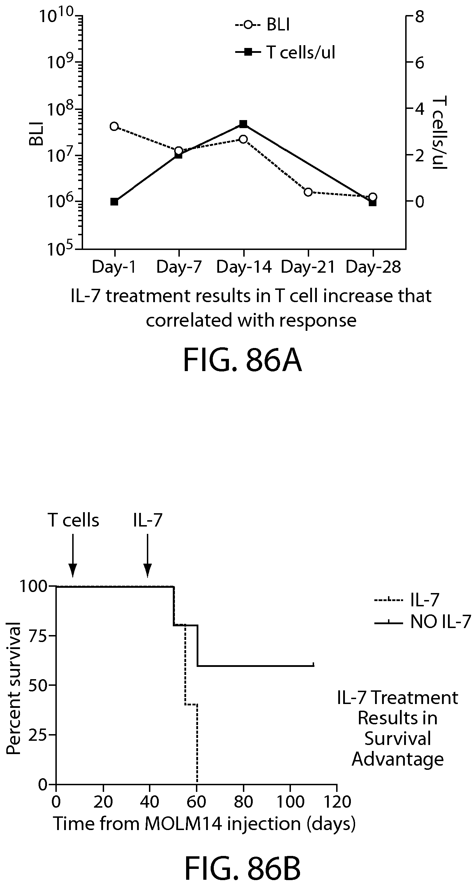

**Fig. 86A and 86B** show T cell expansion and survival after IL-7 treatment in the relapsed AML model. IL-7 treatment resulted in T cell expansion that correlated with reduction in tumor burden (Fig. 86A): T cell expansion was assessed by measuring T cells in the blood (right Y-axis) and compared with bioluminescence imaging (BLI, left Y-axis). Survival of mice comparing mice having received IL-7 treatment or control was monitored from the time of MOLM14 injection (Fig. 86B).

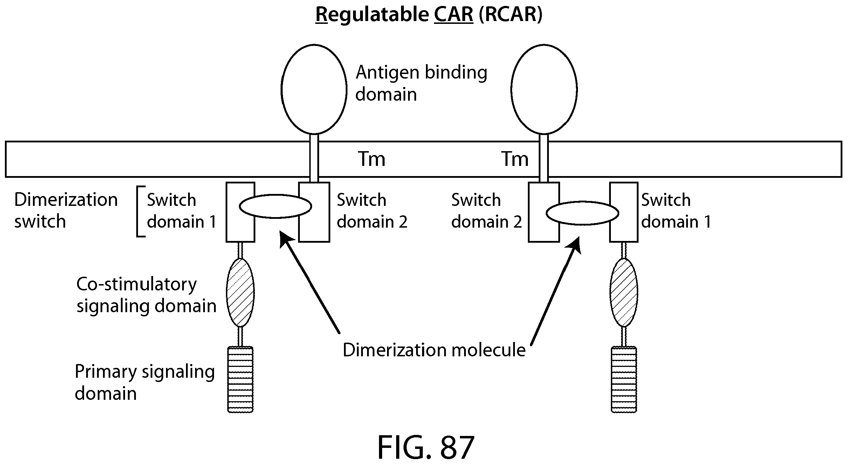

**Fig. 87****.** This schematic diagram depicts the structures of two exemplary RCAR configurations. The antigen binding members comprise an antigen binding domain, a transmembrane domain, and a switch domain. The intracellular binding members comprise a switch domain, a co-stimulatory signaling domain and a primary signaling domain. The two configurations demonstrate that the first and second switch domains described herein can be in different orientations with respect to the antigen binding member and the intracellular binding member. Other RCAR configurations are further described herein.

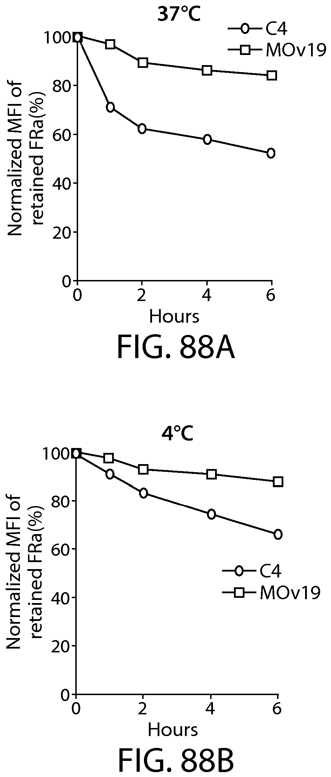

**Fig. 88A, 88B** are a set of graphs showing results of an αFR dissociation assay. C4-27z or MOv19-27z CAR T cells were labeled with recombinant biotinylated αFR protein and then incubated at 37 °C (Fig. 88A) or 4 °C (Fig. 88B) in a time course assay in the presence of excess nonbiotinylated αFR. Percent retained αFR (y-axis) was normalized and scored as mean fluorescence intensity (MFI) postincubation ÷ preincubation MFI × 100.

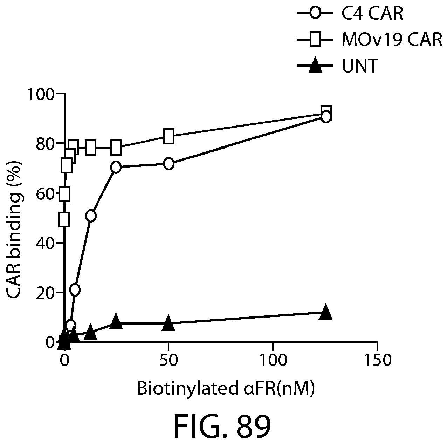

**Fig. 89** is a graph showing a titration analysis on the binding of biotinylated αFR protein to αFR CAR T cells. Result of a representative experiment from three independent experiments is presented.

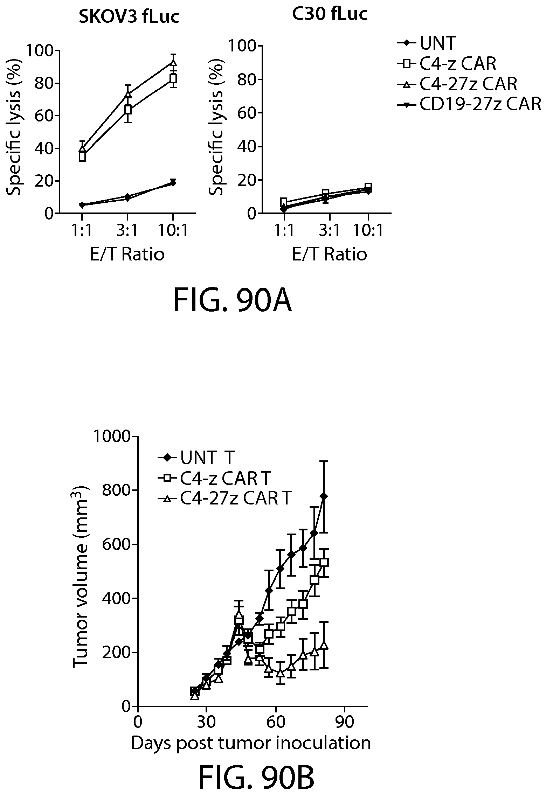

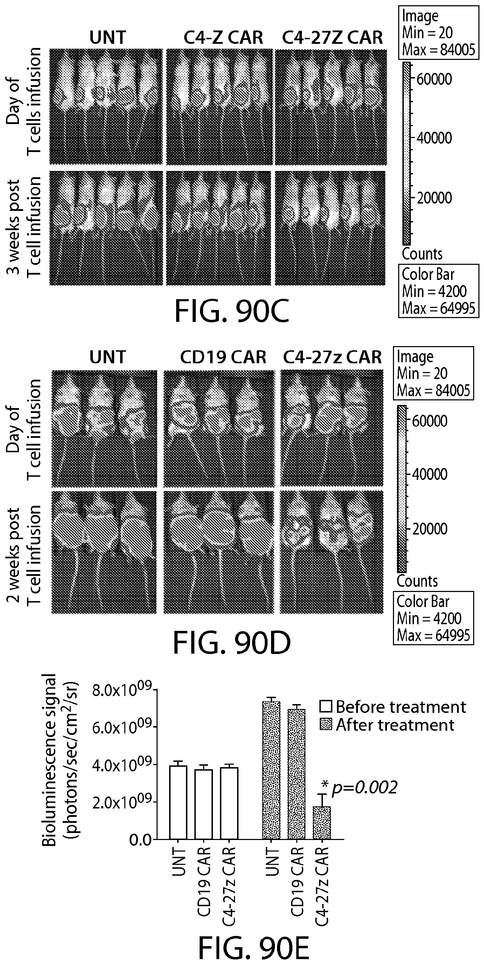

**Fig. 90A, 90B****,** **90C, 90D, 90E****.** Antitumor activity of human T cells expressing C4 CAR *in vitro* and *in vivo.* **(A)** Cytotoxicity of αFR-expressing tumor cells SKOV3 by CAR T cells in 18-hour bioluminescence assay at the indicated E/T ratio. Untransduced (UNT) T cells and CD19 CAR T cells served as negative effector controls. C30 cells served as negative target cell control. Percent tumor cell viability was calculated as the mean luminescence of the experimental sample minus background divided by the mean luminescence of the input number of target cells used in the assay minus background times 100. All data are represented as a mean of triplicate wells. **(B)** NSG mice bearing established s.c. tumor were treated with intravenous (i.v.) injections of 1×10⁷ CAR+ T cells on day 40 post tumor inoculation. Tumor growth was assessed by caliper measurement [V=1/2(length × width²)]. **(C)** Tumor progression was followed by *in vivo* bioluminescence imaging. Incorporation of CD27 signals enhanced antitumor activity in vivo; C4-27z T cell therapy was superior to therapy with the C4-z CAR T cell which lacks a CD27 costimulation domain. **(D)** NSG mice received i.p. injection of 3 x 10 SKOV3 fLuc tumor cells and were randomized into 3 groups before beginning therapy with UNT T cells or T cells expressing C4- 27z or CD19-27z CAR via i.v. infusion on day 21 and 25 after tumor inoculation. Bioluminescence images show fLuc+ SKOV3 tumors in NSG mice immediately prior to and two weeks after second i.v. injection. **(E)** Photon emission from fLuc tumor cells was quantified and the mean ± SD bioluminescence signal determined prior to and two weeks after second i.v. injection of 1 × 10⁷ CAR-T cells on days 21 and 25 after tumor inoculation. There was a significant difference in tumor burden between C4-27z CAR T cells and control T-cell groups 14 days after second T-cell dose injection (p=0.002). These results suggest that the antitumor activity mediated by C4-27z CAR T cells was antigen-specific in vivo.

### FURTHER DETAILED DESCRIPTION

### Definitions

Unless defined otherwise, all technical and scientific terms used herein have the same meaning as commonly understood by one of ordinary skill in the art to which the invention pertains.

The term "a" and "an" refers to one or to more than one (i.e., to at least one) of the grammatical object of the article. By way of example, "an element" means one element or more than one element.

The term "about" when referring to a measurable value such as an amount, a temporal duration, and the like, is meant to encompass variations of ±20% or in some instances ±10%, or in some instances ±5%, or in some instances ±1%, or in some instances ±0.1% from the specified value, as such variations are appropriate to perform the disclosed methods.

The term "Chimeric Antigen Receptor" or alternatively a "CAR" refers to a set of polypeptides, typically two in the simplest embodiments, which when in an immune effector cell, provides the cell with specificity for a target cell, typically a cancer cell, and with intracellular signal generation. In some embodiments, a CAR comprises at least an extracellular antigen binding domain, a transmembrane domain and a cytoplasmic signaling domain (also referred to herein as "an intracellular signaling domain") comprising a functional signaling domain derived from a stimulatory molecule and/or costimulatory molecule as defined below. In some aspects, the set of polypeptides are contiguous with eachother. In some embodiments, the set of polypeptides include a dimerization switch that, upon the presence of a dimerization molecule, can couple the polypeptides to one another, e.g., can couple an antigen binding domain to an intracellular signaling domain. In one aspect, the stimulatory molecule is the zeta chain associated with the T cell receptor complex. In one aspect, the cytoplasmic signaling domain further comprises one or more functional signaling domains derived from at least one costimulatory molecule as defined below. In one aspect, the costimulatory molecule is chosen from the costimulatory molecules described herein, e.g., 4-1BB (i.e., CD137), CD27 and/or CD28. In one aspect, the CAR comprises a chimeric fusion protein comprising an extracellular antigen binding domain, a transmembrane domain and an intracellular signaling domain comprising a functional signaling domain derived from a stimulatory molecule. In one aspect, the CAR comprises a chimeric fusion protein comprising an extracellular antigen binding domain, a transmembrane domain and an intracellular signaling domain comprising a functional signaling domain derived from a costimulatory molecule and a functional signaling domain derived from a stimulatory molecule. In one aspect, the CAR comprises a chimeric fusion protein comprising an extracellular antigen binding domain, a transmembrane domain and an intracellular signaling domain comprising two functional signaling domains derived from one or more costimulatory molecule(s) and a functional signaling domain derived from a stimulatory molecule. In one aspect, the CAR comprises a chimeric fusion protein comprising an extracellular antigen binding domain, a transmembrane domain and an intracellular signaling domain comprising at least two functional signaling domains derived from one or more costimulatory molecule(s) and a functional signaling domain derived from a stimulatory molecule. In one aspect the CAR comprises an optional leader sequence at the amino-terminus (N-ter) of the CAR fusion protein. In one aspect, the CAR further comprises a leader sequence at the N-terminus of the extracellular antigen binding domain, wherein the leader sequence is optionally cleaved from the antigen binding domain (e.g., a scFv) during cellular processing and localization of the CAR to the cellular membrane.

A CAR that comprises an antigen binding domain (e.g., a scFv, or TCR) that targets a specific tumor maker X, such as those described herein, is also referred to as XCAR. For example, a CAR that comprises an antigen binding domain that targets CD19 is referred to as CD19CAR.

The term "signaling domain" refers to the functional portion of a protein which acts by transmitting information within the cell to regulate cellular activity via defined signaling pathways by generating second messengers or functioning as effectors by responding to such messengers.

The term "antibody," as used herein, refers to a protein, or polypeptide sequence derived from an immunoglobulin molecule which specifically binds with an antigen. Antibodies can be polyclonal or monoclonal, multiple or single chain, or intact immunoglobulins, and may be derived from natural sources or from recombinant sources. Antibodies can be tetramers of immunoglobulin molecules.

The term "antibody fragment" refers to at least one portion of an antibody, that retains the ability to specifically interact with (e.g., by binding, steric hinderance, stabilizing/destabilizing, spatial distribution) an epitope of an antigen. Examples of antibody fragments include, but are not limited to, Fab, Fab', F(ab')₂, Fv fragments, scFv antibody fragments, disulfide-linked Fvs (sdFv), a Fd fragment consisting of the VH and CH1 domains, linear antibodies, single domain antibodies such as sdAb (either VL or VH), camelid VHH domains, multi-specific antibodies formed from antibody fragments such as a bivalent fragment comprising two Fab fragments linked by a disulfide brudge at the hinge region, and an isolated CDR or other epitope binding fragments of an antibody. An antigen binding fragment can also be incorporated into single domain antibodies, maxibodies, minibodies, nanobodies, intrabodies, diabodies, triabodies, tetrabodies, v-NAR and bis-scFv (see, e.g., Hollinger and Hudson, Nature Biotechnology 23:1126-1136, 2005). Antigen binding fragments can also be grafted into scaffolds based on polypeptides such as a fibronectin type III (Fn3)(see U.S. Patent No.: 6,703,199, which describes fibronectin polypeptide minibodies).

The term "scFv" refers to a fusion protein comprising at least one antibody fragment comprising a variable region of a light chain and at least one antibody fragment comprising a variable region of a heavy chain, wherein the light and heavy chain variable regions are contiguously linked, e.g., via a synthetic linker, e.g., a short flexible polypeptide linker, and capable of being expressed as a single chain polypeptide, and wherein the scFv retains the specificity of the intact antibody from which it is derived. Unless specified, as used herein an scFv may have the VL and VH variable regions in either order, e.g., with respect to the N-terminal and C-terminal ends of the polypeptide, the scFv may comprise VL-linker-VH or may comprise VH-linker-VL.

The portion of the CAR of the disclosure comprising an antibody or antibody fragment thereof may exist in a variety of forms where the antigen binding domain is expressed as part of a contiguous polypeptide chain including, for example, a single domain antibody fragment (sdAb), a single chain antibody (scFv), a humanized antibody or bispecific antibody (Harlow et al., 1999, In: Using Antibodies: A Laboratory Manual, Cold Spring Harbor Laboratory Press, NY; Harlow et al., 1989, In: Antibodies: A Laboratory Manual, Cold Spring Harbor, New York; Houston et al., 1988, Proc. Natl. Acad. Sci. USA 85:5879-5883; Bird et al., 1988, Science 242:423-426). In one aspect, the antigen binding domain of a CAR composition of the disclosure comprises an antibody fragment. In a further aspect, the CAR comprises an antibody fragment that comprises a scFv. The precise amino acid sequence boundaries of a given CDR can be determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme), Al-Lazikani et al., (1997) JMB 273,927-948 ("Chothia" numbering scheme), or a combination thereof.

As used herein, the term "binding domain" or "antibody molecule" refers to a protein, e.g., an immunoglobulin chain or fragment thereof, comprising at least one immunoglobulin variable domain sequence. The term "binding domain" or "antibody molecule" encompasses antibodies and antibody fragments. In an instance, an antibody molecule is a multispecific antibody molecule, e.g., it comprises a plurality of immunoglobulin variable domain sequences, wherein a first immunoglobulin variable domain sequence of the plurality has binding specificity for a first epitope and a second immunoglobulin variable domain sequence of the plurality has binding specificity for a second epitope. In an instance, a multispecific antibody molecule is a bispecific antibody molecule. A bispecific antibody has specificity for no more than two antigens. A bispecific antibody molecule is characterized by a first immunoglobulin variable domain sequence which has binding specificity for a first epitope and a second immunoglobulin variable domain sequence that has binding specificity for a second epitope.

The portion of the CAR of the disclosure comprising an antibody or antibody fragment thereof may exist in a variety of forms where the antigen binding domain is expressed as part of a contiguous polypeptide chain including, for example, a single domain antibody fragment (sdAb), a single chain antibody (scFv), a humanized antibody, or bispecific antibody (Harlow et al., 1999, In: Using Antibodies: A Laboratory Manual, Cold Spring Harbor Laboratory Press, NY; Harlow et al., 1989, In: Antibodies: A Laboratory Manual, Cold Spring Harbor, New York; Houston et al., 1988, Proc. Natl. Acad. Sci. USA 85:5879-5883; Bird et al., 1988, Science 242:423-426). In one aspect, the antigen binding domain of a CAR composition of the disclosure comprises an antibody fragment. In a further aspect, the CAR comprises an antibody fragment that comprises a scFv.

The term "antibody heavy chain," refers to the larger of the two types of polypeptide chains present in antibody molecules in their naturally occurring conformations, and which normally determines the class to which the antibody belongs.

The term "antibody light chain," refers to the smaller of the two types of polypeptide chains present in antibody molecules in their naturally occurring conformations. Kappa (κ) and lambda (λ) light chains refer to the two major antibody light chain isotypes.

The term "recombinant antibody" refers to an antibody which is generated using recombinant DNA technology, such as, for example, an antibody expressed by a bacteriophage or yeast expression system. The term should also be construed to mean an antibody which has been generated by the synthesis of a DNA molecule encoding the antibody and which DNA molecule expresses an antibody protein, or an amino acid sequence specifying the antibody, wherein the DNA or amino acid sequence has been obtained using recombinant DNA or amino acid sequence technology which is available and well known in the art.

The term "antigen" or "Ag" refers to a molecule that provokes an immune response. This immune response may involve either antibody production, or the activation of specific immunologically-competent cells, or both. The skilled artisan will understand that any macromolecule, including virtually all proteins or peptides, can serve as an antigen. Furthermore, antigens can be derived from recombinant or genomic DNA. A skilled artisan will understand that any DNA, which comprises a nucleotide sequences or a partial nucleotide sequence encoding a protein that elicits an immune response therefore encodes an "antigen" as that term is used herein. Furthermore, one skilled in the art will understand that an antigen need not be encoded solely by a full length nucleotide sequence of a gene. It is readily apparent that the present invention includes, but is not limited to, the use of partial nucleotide sequences of more than one gene and that these nucleotide sequences are arranged in various combinations to encode polypeptides that elicit the desired immune response. Moreover, a skilled artisan will understand that an antigen need not be encoded by a "gene" at all. It is readily apparent that an antigen can be generated synthesized or can be derived from a biological sample, or might be macromolecule besides a polypeptide. Such a biological sample can include, but is not limited to a tissue sample, a tumor sample, a cell or a fluid with other biological components.

The term "anti-cancer effect" refers to a biological effect which can be manifested by various means, including but not limited to, e.g., a decrease in tumor volume, a decrease in the number of cancer cells, a decrease in the number of metastases, an increase in life expectancy, decrease in cancer cell proliferation, decrease in cancer cell survival, or amelioration of various physiological symptoms associated with the cancerous condition. An "anti-cancer effect" can also be manifested by the ability of the peptides, polynucleotides, cells and antibodies in prevention of the occurrence of cancer in the first place. The term "anti-tumor effect" refers to a biological effect which can be manifested by various means, including but not limited to, e.g., a decrease in tumor volume, a decrease in the number of tumor cells, a decrease in tumor cell proliferation, or a decrease in tumor cell survival.

The term "autologous" refers to any material derived from the same individual to whom it is later to be re-introduced into the individual.

The term "allogeneic" refers to any material derived from a different animal of the same species as the individual to whom the material is introduced. Two or more individuals are said to be allogeneic to one another when the genes at one or more loci are not identical. In some aspects, allogeneic material from individuals of the same species may be sufficiently unlike genetically to interact antigenically

The term "xenogeneic" refers to a graft derived from an animal of a different species.

The term "cancer" refers to a disease characterized by the uncontrolled growth of aberrant cells. Cancer cells can spread locally or through the bloodstream and lymphatic system to other parts of the body. Examples of various cancers are described herein and include but are not limited to, breast cancer, prostate cancer, ovarian cancer, cervical cancer, skin cancer, pancreatic cancer, colorectal cancer, renal cancer, liver cancer, brain cancer, lymphoma, leukemia, lung cancer and the like. The terms "tumor" and "cancer" are used interchangeably herein, e.g., both terms encompass solid and liquid, e.g., diffuse or circulating, tumors. As used herein, the term "cancer" or "tumor" includes premalignant, as well as malignant cancers and tumors.

"Derived from" as that term is used herein, indicates a relationship between a first and a second molecule. It generally refers to structural similarity between the first molecule and a second molecule and does not connotate or include a process or source limitation on a first molecule that is derived from a second molecule. For example, in the case of an intracellular signaling domain that is derived from a CD3zeta molecule, the intracellular signaling domain retains sufficient CD3zeta structure such that is has the required function, namely, the ability to generate a signal under the appropriate conditions. It does not connotate or include a limitation to a particular process of producing the intracellular signaling domain, e.g., it does not mean that, to provide the intracellular signaling domain, one must start with a CD3zeta sequence and delete unwanted sequence, or impose mutations, to arrive at the intracellular signaling domain.

The phrase "disease associated with expression of a tumor antigen as described herein" includes, but is not limited to, a disease associated with expression of a tumor antigen as described herein or condition associated with cells which express a tumor antigen as described herein including, e.g., proliferative diseases such as a cancer or malignancy or a precancerous condition such as a myelodysplasia, a myelodysplastic syndrome or a preleukemia; or a noncancer related indication associated with cells which express a tumor antigen as described herein. In one aspect, a cancer associated with expression of a tumor antigen as described herein is a hematological cancer. In one aspect, a cancer associated with expression of a tumor antigen as described herein is a solid cancer. Further diseases associated with expression of a tumor antigen described herein include, but not limited to, e.g., atypical and/or non-classical cancers, malignancies, precancerous conditions or proliferative diseases associated with expression of a tumor antigen as described herein. Non-cancer related indications associated with expression of a tumor antigen as described herein include, but are not limited to, e.g., autoimmune disease, (e.g., lupus), inflammatory disorders (allergy and asthma) and transplantation. In some embodiments, the tumor antigen-expressing cells express, or at any time expressed, mRNA encoding the tumor antigen. In an embodiment, the tumor antigen-expressing cells produce the tumor antigen protein (e.g., wild-type or mutant), and the tumor antigen protein may be present at normal levels or reduced levels. In an embodiment, the tumor antigen-expressing cells produced detectable levels of a tumor antigen protein at one point, and subsequently produced substantially no detectable tumor antigen protein.

The term "conservative sequence modifications" refers to amino acid modifications that do not significantly affect or alter the binding characteristics of the antibody or antibody fragment containing the amino acid sequence. Such conservative modifications include amino acid substitutions, additions and deletions. Modifications can be introduced into an antibody or antibody fragment of the disclosure by standard techniques known in the art, such as site-directed mutagenesis and PCR-mediated mutagenesis. Conservative amino acid substitutions are ones in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), betabranched side chains (e.g., threonine, valine, isoleucine) and aromatic side chains (e.g., tyrosine, phenylalanine, tryptophan, histidine). Thus, one or more amino acid residues within a CAR used in the invention can be replaced with other amino acid residues from the same side chain family and the altered CAR can be tested using the functional assays described herein.

The term "stimulation," refers to a primary response induced by binding of a stimulatory molecule (e.g., a TCR/CD3 complex or CAR) with its cognate ligand (or tumor antigen in the case of a CAR) thereby mediating a signal transduction event, such as, but not limited to, signal transduction via the TCR/CD3 complex or signal transduction via the appropriate NK receptor or signaling domains of the CAR. Stimulation can mediate altered expression of certain molecules.

The term "stimulatory molecule," refers to a molecule expressed by aan immune cell (e.g., T cell, NK cell, B cell) that provides the cytoplasmic signaling sequence(s) that regulate activation of the immune cell in a stimulatory way for at least some aspect of the immune cell signaling pathway. In one aspect, the signal is a primary signal that is initiated by, for instance, binding of a TCR/CD3 complex with an MHC molecule loaded with peptide, and which leads to mediation of a T cell response, including, but not limited to, proliferation, activation, differentiation, and the like. A primary cytoplasmic signaling sequence (also referred to as a "primary signaling domain") that acts in a stimulatory manner may contain a signaling motif which is known as immunoreceptor tyrosine-based activation motif or ITAM. Examples of an ITAM containing cytoplasmic signaling sequence that is of particular use in the invention includes, but is not limited to, those derived from CD3 zeta, common FcR gamma (FCER1G), Fc gamma RIIa, FcR beta (Fc Epsilon R1b), CD3 gamma, CD3 delta, CD3 epsilon, CD79a, CD79b, DAP10, and DAP12. In a specific CAR used in the invention, the intracellular signaling domain in any one or more CARS used in the invention comprises an intracellular signaling sequence, e.g., a primary signaling sequence of CD3-zeta. In a specific CAR used in the invention, the primary signaling sequence of CD3-zeta is the sequence provided as SEQ ID NO: 18, or the equivalent residues from a non-human species, e.g., mouse, rodent, monkey, ape and the like. In a specific CAR used in the invention, the primary signaling sequence of CD3-zeta is the sequence as provided in SEQ ID NO:20, or the equivalent residues from a non-human species, e.g., mouse, rodent, monkey, ape and the like.

The term "antigen presenting cell" or "APC" refers to an immune system cell such as an accessory cell (e.g., a B-cell, a dendritic cell, and the like) that displays a foreign antigen complexed with major histocompatibility complexes (MHC's) on its surface. T-cells may recognize these complexes using their T-cell receptors (TCRs). APCs process antigens and present them to T-cells.

An "intracellular signaling domain," as the term is used herein, refers to an intracellular portion of a molecule. The intracellular signaling domain generates a signal that promotes an immune effector function of the CAR containing cell, e.g., a CART cell. Examples of immune effector function, e.g., in a CART cell, include cytolytic activity and helper activity, including the secretion of cytokines.

In an embodiment, the intracellular signaling domain can comprise a primary intracellular signaling domain. Exemplary primary intracellular signaling domains include those derived from the molecules responsible for primary stimulation, or antigen dependent simulation. In an embodiment, the intracellular signaling domain can comprise a costimulatory intracellular domain. Exemplary costimulatory intracellular signaling domains include those derived from molecules responsible for costimulatory signals, or antigen independent stimulation. For example, in the case of a CART, a primary intracellular signaling domain can comprise a cytoplasmic sequence of a T cell receptor, and a costimulatory intracellular signaling domain can comprise cytoplasmic sequence from co-receptor or costimulatory molecule.

A primary intracellular signaling domain can comprise a signaling motif which is known as an immunoreceptor tyrosine-based activation motif or ITAM. Examples of ITAM containing primary cytoplasmic signaling sequences include, but are not limited to, those derived from CD3 zeta, common FcR gamma (FCER1G), Fc gamma RIIa, FcR beta (Fc Epsilon R1b), CD3 gamma, CD3 delta, CD3 epsilon, CD79a, CD79b, DAP10, and DAP12.

The term "zeta" or alternatively "zeta chain", "CD3-zeta" or "TCR-zeta" is defined as the protein provided as GenBan Acc. No. BAG36664.1, or the equivalent residues from a non-human species, e.g., mouse, rodent, monkey, ape and the like, and a "zeta stimulatory domain" or alternatively a "CD3-zeta stimulatory domain" or a "TCR-zeta stimulatory domain" is defined as the amino acid residues from the cytoplasmic domain of the zeta chain, or functional derivatives thereof, that are sufficient to functionally transmit an initial signal necessary for T cell activation. In one aspect the cytoplasmic domain of zeta comprises residues 52 through 164 of GenBank Acc. No. BAG36664.1 or the equivalent residues from a non-human species, e.g., mouse, rodent, monkey, ape and the like, that are functional orthologs thereof. In one aspect, the "zeta stimulatory domain" or a "CD3-zeta stimulatory domain" is the sequence provided as SEQ ID NO: 18. In one aspect, the "zeta stimulatory domain" or a "CD3-zeta stimulatory domain" is the sequence provided as SEQ ID NO:20.

The term a "costimulatory molecule" refers to a cognate binding partner on a T cell that specifically binds with a costimulatory ligand, thereby mediating a costimulatory response by the T cell, such as, but not limited to, proliferation. Costimulatory molecules are cell surface molecules other than antigen receptors or their ligands that are contribute to an efficient immune response. Costimulatory molecules include, but are not limited to an MHC class I molecule, BTLA and a Toll ligand receptor, as well as OX40, CD27, CD28, CDS, ICAM-1, LFA-1 (CD11a/CD18), ICOS (CD278), and 4-1BB (CD137). Further examples of such costimulatory molecules include CDS, ICAM-1, GITR, BAFFR, HVEM (LIGHTR), SLAMF7, NKp80 (KLRF1), NKp44, NKp30, NKp46, CD160, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, CD19a, and a ligand that specifically binds with CD83.

A costimulatory intracellular signaling domain can be the intracellular portion of a costimulatory molecule. A costimulatory molecule can be represented in the following protein families: TNF receptor proteins, Immunoglobulin-like proteins, cytokine receptors, integrins, signaling lymphocytic activation molecules (SLAM proteins), and activating NK cell receptors. Examples of such molecules include CD27, CD28, 4-1BB (CD137), OX40, GITR, CD30, CD40, ICOS, BAFFR, HVEM, ICAM-1, lymphocyte function-associated antigen-1 (LFA-1), CD2, CDS, CD7, CD287, LIGHT, NKG2C, NKG2D, SLAMF7, NKp80, NKp30, NKp44, NKp46, CD160, B7-H3, and a ligand that specifically binds with CD83, and the like.

The intracellular signaling domain can comprise the entire intracellular portion, or the entire native intracellular signaling domain, of the molecule from which it is derived, or a functional fragment or derivative thereof.

The term "4-1BB" refers to a member of the TNFR superfamily with an amino acid sequence provided as GenBank Acc. No. AAA62478.2, or the equivalent residues from a non-human species, e.g., mouse, rodent, monkey, ape and the like; and a "4-1BB costimulatory domain" is defined as amino acid residues 214-255 of GenBank Acc. No. AAA62478.2, or the equivalent residues from a non-human species, e.g., mouse, rodent, monkey, ape and the like. In one aspect, the "4-1BB costimulatory domain" is the sequence provided as SEQ ID NO: 14 or the equivalent residues from a non-human species, e.g., mouse, rodent, monkey, ape and the like.

"Immune effector cell," as that term is used herein, refers to a cell that is involved in an immune response, e.g., in the promotion of an immune effector response. Examples of immune effector cells include T cells, e.g., alpha/beta T cells and gamma/delta T cells, B cells, natural killer (NK) cells, natural killer T (NKT) cells, mast cells, and myeloic-derived phagocytes.

"Immune effector function or immune effector response," as that term is used herein, refers to function or response, e.g., of an immune effector cell, that enhances or promotes an immune attack of a target cell. E.g., an immune effector function or response refers a property of a T or NK cell that promotes killing or the inhibition of growth or proliferation, of a target cell. In the case of a T cell, primary stimulation and co-stimulation are examples of immune effector function or response.

The term "encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (e.g., rRNA, tRNA and mRNA) or a defined sequence of amino acids and the biological properties resulting therefrom. Thus, a gene, cDNA, or RNA, encodes a protein if transcription and translation of mRNA corresponding to that gene produces the protein in a cell or other biological system. Both the coding strand, the nucleotide sequence of which is identical to the mRNA sequence and is usually provided in sequence listings, and the non-coding strand, used as the template for transcription of a gene or cDNA, can be referred to as encoding the protein or other product of that gene or cDNA.

Unless otherwise specified, a "nucleotide sequence encoding an amino acid sequence" includes all nucleotide sequences that are degenerate versions of each other and that encode the same amino acid sequence. The phrase nucleotide sequence that encodes a protein or a RNA may also include introns to the extent that the nucleotide sequence encoding the protein may in some version contain an intron(s).

The term "effective amount" or "therapeutically effective amount" are used interchangeably herein, and refer to an amount of a compound, formulation, material, or composition, as described herein effective to achieve a particular biological result.

The term "endogenous" refers to any material from or produced inside an organism, cell, tissue or system.

The term "exogenous" refers to any material introduced from or produced outside an organism, cell, tissue or system.

The term "expression" refers to the transcription and/or translation of a particular nucleotide sequence driven by a promoter.

The term "transfer vector" refers to a composition of matter which comprises an isolated nucleic acid and which can be used to deliver the isolated nucleic acid to the interior of a cell. Numerous vectors are known in the art including, but not limited to, linear polynucleotides, polynucleotides associated with ionic or amphiphilic compounds, plasmids, and viruses. Thus, the term "transfer vector" includes an autonomously replicating plasmid or a virus. The term should also be construed to further include nonplasmid and non-viral compounds which facilitate transfer of nucleic acid into cells, such as, for example, a polylysine compound, liposome, and the like. Examples of viral transfer vectors include, but are not limited to, adenoviral vectors, adeno-associated virus vectors, retroviral vectors, lentiviral vectors, and the like.

The term "expression vector" refers to a vector comprising a recombinant polynucleotide comprising expression control sequences operatively linked to a nucleotide sequence to be expressed. An expression vector comprises sufficient cis-acting elements for expression; other elements for expression can be supplied by the host cell or in an in vitro expression system. Expression vectors include all those known in the art, including cosmids, plasmids (e.g., naked or contained in liposomes) and viruses (e.g., lentiviruses, retroviruses, adenoviruses, and adeno-associated viruses) that incorporate the recombinant polynucleotide.

The term "lentivirus" refers to a genus of the Retroviridae family. Lentiviruses are unique among the retroviruses in being able to infect non-dividing cells; they can deliver a significant amount of genetic information into the DNA of the host cell, so they are one of the most efficient methods of a gene delivery vector. HIV, SIV, and FIV are all examples of lentiviruses.

The term "lentiviral vector" refers to a vector derived from at least a portion of a lentivirus genome, including especially a self-inactivating lentiviral vector as provided in Milone et al., Mol. Ther. 17(8): 1453-1464 (2009). Other examples of lentivirus vectors that may be used in the clinic, include but are not limited to, e.g., the LENTIVECTOR^{®} gene delivery technology from Oxford BioMedica, the LENTIMAX^{™} vector system from Lentigen and the like. Nonclinical types of lentiviral vectors are also available and would be known to one skilled in the art.

The term "homologous" or "identity" refers to the subunit sequence identity between two polymeric molecules, e.g., between two nucleic acid molecules, such as, two DNA molecules or two RNA molecules, or between two polypeptide molecules. When a subunit position in both of the two molecules is occupied by the same monomeric subunit; e.g., if a position in each of two DNA molecules is occupied by adenine, then they are homologous or identical at that position. The homology between two sequences is a direct function of the number of matching or homologous positions; e.g., if half (e.g., five positions in a polymer ten subunits in length) of the positions in two sequences are homologous, the two sequences are 50% homologous; if 90% of the positions (e.g., 9 of 10), are matched or homologous, the two sequences are 90% homologous.

"Humanized" forms of non-human (e.g., murine) antibodies are chimeric immunoglobulins, immunoglobulin chains or fragments thereof (such as Fv, Fab, Fab', F(ab')2 or other antigen-binding subsequences of antibodies) which contain minimal sequence derived from non-human immunoglobulin. For the most part, humanized antibodies and antibody fragments thereof are human immunoglobulins (recipient antibody or antibody fragment) in which residues from a complementary-determining region (CDR) of the recipient are replaced by residues from a CDR of a non-human species (donor antibody) such as mouse, rat or rabbit having the desired specificity, affinity, and capacity. In some instances, Fv framework region (FR) residues of the human immunoglobulin are replaced by corresponding non-human residues. Furthermore, a humanized antibody/antibody fragment can comprise residues which are found neither in the recipient antibody nor in the imported CDR or framework sequences. These modifications can further refine and optimize antibody or antibody fragment performance. In general, the humanized antibody or antibody fragment thereof will comprise substantially all of at least one, and typically two, variable domains, in which all or substantially all of the CDR regions correspond to those of a non-human immunoglobulin and all or a significant portion of the FR regions are those of a human immunoglobulin sequence. The humanized antibody or antibody fragment can also comprise at least a portion of an immunoglobulin constant region (Fc), typically that of a human immunoglobulin. For further details, see Jones et al., Nature, 321: 522-525, 1986; Reichmann et al., Nature, 332: 323-329, 1988; Presta, Curr. Op. Struct. Biol., 2: 593-596, 1992.

"Fully human" refers to an immunoglobulin, such as an antibody or antibody fragment, where the whole molecule is of human origin or consists of an amino acid sequence identical to a human form of the antibody or immunoglobulin.

The term "isolated" means altered or removed from the natural state. For example, a nucleic acid or a peptide naturally present in a living animal is not "isolated," but the same nucleic acid or peptide partially or completely separated from the coexisting materials of its natural state is "isolated." An isolated nucleic acid or protein can exist in substantially purified form, or can exist in a non-native environment such as, for example, a host cell.

In the context of the present invention, the following abbreviations for the commonly occurring nucleic acid bases are used. "A" refers to adenosine, "C" refers to cytosine, "G" refers to guanosine, "T" refers to thymidine, and "U" refers to uridine.

The term "operably linked" or "transcriptional control" refers to functional linkage between a regulatory sequence and a heterologous nucleic acid sequence resulting in expression of the latter. For example, a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Operably linked DNA sequences can be contiguous with each other and, e.g., where necessary to join two protein coding regions, are in the same reading frame.

The term "parenteral" administration of an immunogenic composition includes, e.g., subcutaneous (s.c.), intravenous (i.v.), intramuscular (i.m.), or intrasternal injection, intratumoral, or infusion techniques.

The term "nucleic acid" or "polynucleotide" refers to deoxyribonucleic acids (DNA) or ribonucleic acids (RNA) and polymers thereof in either single- or doublestranded form. Unless specifically limited, the term encompasses nucleic acids containing known analogues of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions), alleles, orthologs, SNPs, and complementary sequences as well as the sequence explicitly indicated. Specifically, degenerate codon substitutions may be achieved by generating sequences in which the third position of one or more selected (or all) codons is substituted with mixedbase and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)).

The terms "peptide," "polypeptide," and "protein" are used interchangeably, and refer to a compound comprised of amino acid residues covalently linked by peptide bonds. A protein or peptide must contain at least two amino acids, and no limitation is placed on the maximum number of amino acids that can comprise a protein's or peptide's sequence. Polypeptides include any peptide or protein comprising two or more amino acids joined to each other by peptide bonds. As used herein, the term refers to both short chains, which also commonly are referred to in the art as peptides, oligopeptides and oligomers, for example, and to longer chains, which generally are referred to in the art as proteins, of which there are many types. "Polypeptides" include, for example, biologically active fragments, substantially homologous polypeptides, oligopeptides, homodimers, heterodimers, variants of polypeptides, modified polypeptides, derivatives, analogs, fusion proteins, among others. A polypeptide includes a natural peptide, a recombinant peptide, or a combination thereof.

The term "promoter" refers to a DNA sequence recognized by the synthetic machinery of the cell, or introduced synthetic machinery, required to initiate the specific transcription of a polynucleotide sequence.

The term "promoter/regulatory sequence" refers to a nucleic acid sequence which is required for expression of a gene product operably linked to the promoter/regulatory sequence. In some instances, this sequence may be the core promoter sequence and in other instances, this sequence may also include an enhancer sequence and other regulatory elements which are required for expression of the gene product. The promoter/regulatory sequence may, for example, be one which expresses the gene product in a tissue specific manner.

The term "constitutive" promoter refers to a nucleotide sequence which, when operably linked with a polynucleotide which encodes or specifies a gene product, causes the gene product to be produced in a cell under most or all physiological conditions of the cell.

The term "inducible" promoter refers to a nucleotide sequence which, when operably linked with a polynucleotide which encodes or specifies a gene product, causes the gene product to be produced in a cell substantially only when an inducer which corresponds to the promoter is present in the cell.

The term "tissue-specific" promoter refers to a nucleotide sequence which, when operably linked with a polynucleotide encodes or specified by a gene, causes the gene product to be produced in a cell substantially only if the cell is a cell of the tissue type corresponding to the promoter.

The terms "cancer associated antigen" or "tumor antigen" interchangeably refers to a molecule (typically a protein, carbohydrate or lipid) that is expressed on the surface of a cancer cell, either entirely or as a fragment (e.g., MHC/peptide), and which is useful for the preferential targeting of a pharmacological agent to the cancer cell. In some embodiments, a tumor antigen is a marker expressed by both normal cells and cancer cells, e.g., a lineage marker, e.g., CD19 on B cells. In some embodiments, a tumor antigen is a cell surface molecule that is overexpressed in a cancer cell in comparison to a normal cell, for instance, 1-fold over expression, 2-fold overexpression, 3-fold overexpression or more in comparison to a normal cell. In some enbodiments, a tumor antigen is a cell surface molecule that is inappropriately synthesized in the cancer cell, for instance, a molecule that contains deletions, additions or mutations in comparison to the molecule expressed on a normal cell. In some embodiments, a tumor antigen will be expressed exclusively on the cell surface of a cancer cell, entirely or as a fragment (e.g., MHC/peptide), and not synthesized or expressed on the surface of a normal cell. In some embodiments, the CARs of the present disclosure includes CARs comprising an antigen binding domain (e.g., antibody or antibody fragment) that binds to a MHC presented peptide. Normally, peptides derived from endogenous proteins fill the pockets of Major histocompatibility complex (MHC) class I molecules, and are recognized by T cell receptors (TCRs) on CD8 + T lymphocytes. The MHC class I complexes are constitutively expressed by all nucleated cells. In cancer, virus-specific and/or tumor-specific peptide/MHC complexes represent a unique class of cell surface targets for immunotherapy. TCR-like antibodies targeting peptides derived from viral or tumor antigens in the context of human leukocyte antigen (HLA)-A1 or HLA-A2 have been described (see, e.g., Sastry et al., J Virol. 2011 85(5): 1935-1942; Sergeeva et al., Blood, 2011 117(16):4262-4272; Verma et al., J Immunol 2010 184(4):2156-2165; Willemsen et al., Gene Ther 2001 8(21) : 1601-1608; Dao et al., Sci Transl Med 2013 5(176) :176ra33 ; Tassev et al., Cancer Gene Ther 2012 19(2):84-100). For example, TCR-like antibody can be identified from screening a library, such as a human scFv phage displayed library.

The term "tumor-supporting antigen" or "cancer-supporting antigen" interchangeably refer to a molecule (typically a protein, carbohydrate or lipid) that is expressed on the surface of a cell that is, itself, not cancerous, but supports the cancer cells, e.g., by promoting their growth or survival e.g., resistance to immune cells. Exemplary cells of this type include stromal cells and myeloid-derived suppressor cells (MDSCs). The tumor-supporting antigen itself need not play a role in supporting the tumor cells so long as the antigen is present on a cell that supports cancer cells.

The term "flexible polypeptide linker" or "linker" as used in the context of a scFv refers to a peptide linker that consists of amino acids such as glycine and/or serine residues used alone or in combination, to link variable heavy and variable light chain regions together. In one embodiment, the flexible polypeptide linker is a Gly/Ser linker and comprises the amino acid sequence (Gly-Gly-Gly-Ser)n, where n is a positive integer equal to or greater than 1. For example, n=1, n=2, n=3. n=4, n=5 and n=6, n=7, n=8, n=9 and n=10 (SEQ ID NO:28). In one embodiment, the flexible polypeptide linkers include, but are not limited to, (Gly₄ Ser)₄ (SEQ ID NO:29) or (Gly₄ Ser)₃ (SEQ ID NO:30). In another embodiment, the linkers include multiple repeats of (Gly₂Ser), (GlySer) or (Gly₃Ser) (SEQ ID NO:31). Also included within the scope of the invention are linkers described in WO2012/138475.

As used herein, a 5' cap (also termed an RNA cap, an RNA 7-methylguanosine cap or an RNA m⁷G cap) is a modified guanine nucleotide that has been added to the "front" or 5' end of a eukaryotic messenger RNA shortly after the start of transcription. The 5' cap consists of a terminal group which is linked to the first transcribed nucleotide. Its presence is critical for recognition by the ribosome and protection from RNases. Cap addition is coupled to transcription, and occurs co-transcriptionally, such that each influences the other. Shortly after the start of transcription, the 5' end of the mRNA being synthesized is bound by a cap-synthesizing complex associated with RNA polymerase. This enzymatic complex catalyzes the chemical reactions that are required for mRNA capping. Synthesis proceeds as a multi-step biochemical reaction. The capping moiety can be modified to modulate functionality of mRNA such as its stability or efficiency of translation.

As used herein, "in vitro transcribed RNA" refers to RNA, preferably mRNA, that has been synthesized in vitro. Generally, the in vitro transcribed RNA is generated from an in vitro transcription vector. The in vitro transcription vector comprises a template that is used to generate the in vitro transcribed RNA.

As used herein, a "poly(A)" is a series of adenosines attached by polyadenylation to the mRNA. In the preferred embodiment of a construct for transient expression, the polyA is between 50 and 5000 (SEQ ID NO: 34), preferably greater than 64, more preferably greater than 100, most preferably greater than 300 or 400. poly(A) sequences can be modified chemically or enzymatically to modulate mRNA functionality such as localization, stability or efficiency of translation.

As used herein, "polyadenylation" refers to the covalent linkage of a polyadenylyl moiety, or its modified variant, to a messenger RNA molecule. In eukaryotic organisms, most messenger RNA (mRNA) molecules are polyadenylated at the 3' end. The 3' poly(A) tail is a long sequence of adenine nucleotides (often several hundred) added to the pre-mRNA through the action of an enzyme, polyadenylate polymerase. In higher eukaryotes, the poly(A) tail is added onto transcripts that contain a specific sequence, the polyadenylation signal. The poly(A) tail and the protein bound to it aid in protecting mRNA from degradation by exonucleases. Polyadenylation is also important for transcription termination, export of the mRNA from the nucleus, and translation. Polyadenylation occurs in the nucleus immediately after transcription of DNA into RNA, but additionally can also occur later in the cytoplasm. After transcription has been terminated, the mRNA chain is cleaved through the action of an endonuclease complex associated with RNA polymerase. The cleavage site is usually characterized by the presence of the base sequence AAUAAA near the cleavage site. After the mRNA has been cleaved, adenosine residues are added to the free 3' end at the cleavage site.

As used herein, "transient" refers to expression of a non-integrated transgene for a period of hours, days or weeks, wherein the period of time of expression is less than the period of time for expression of the gene if integrated into the genome or contained within a stable plasmid replicon in the host cell.

As used herein, the terms "treat", "treatment" and "treating" refer to the reduction or amelioration of the progression, severity and/or duration of a proliferative disorder, or the amelioration of one or more symptoms (preferably, one or more discernible symptoms) of a proliferative disorder resulting from the administration of one or more therapies (e.g., one or more therapeutic agents such as a CAR of the disclosure). In specific embodiments, the terms "treat", "treatment" and "treating" refer to the amelioration of at least one measurable physical parameter of a proliferative disorder, such as growth of a tumor, not necessarily discernible by the patient. In other embodiments the terms "treat", "treatment" and "treating" -refer to the inhibition of the progression of a proliferative disorder, either physically by, e.g., stabilization of a discernible symptom, physiologically by, e.g., stabilization of a physical parameter, or both. In other embodiments the terms "treat", "treatment" and "treating" refer to the reduction or stabilization of tumor size or cancerous cell count.

The term "signal transduction pathway" refers to the biochemical relationship between a variety of signal transduction molecules that play a role in the transmission of a signal from one portion of a cell to another portion of a cell. The phrase "cell surface receptor" includes molecules and complexes of molecules capable of receiving a signal and transmitting signal across the membrane of a cell.

The term "subject" is intended to include living organisms in which an immune response can be elicited (e.g., mammals, human).

The term, a "substantially purified" cell refers to a cell that is essentially free of other cell types. A substantially purified cell also refers to a cell which has been separated from other cell types with which it is normally associated in its naturally occurring state. In some instances, a population of substantially purified cells refers to a homogenous population of cells. In other instances, this term refers simply to cell that have been separated from the cells with which they are naturally associated in their natural state. In some aspects, the cells are cultured in vitro. In other aspects, the cells are not cultured in vitro.

The term "therapeutic" as used herein means a treatment. A therapeutic effect is obtained by reduction, suppression, remission, or eradication of a disease state.

The term "prophylaxis" as used herein means the prevention of or protective treatment for a disease or disease state.

In the context of the present invention, "tumor antigen" or "hyperproliferative disorder antigen" or "antigen associated with a hyperproliferative disorder" refers to antigens that are common to specific hyperproliferative disorders. In certain aspects, the hyperproliferative disorder antigens of the present disclosure are derived from, cancers including but not limited to primary or metastatic melanoma, thymoma, lymphoma, sarcoma, lung cancer, liver cancer, non-Hodgkin lymphoma, Hodgkin lymphoma, leukemias, uterine cancer, cervical cancer, bladder cancer, kidney cancer and adenocarcinomas such as breast cancer, prostate cancer, ovarian cancer, pancreatic cancer, and the like.

The term "transfected" or "transformed" or "transduced" refers to a process by which exogenous nucleic acid is transferred or introduced into the host cell. A "transfected" or "transformed" or "transduced" cell is one which has been transfected, transformed or transduced with exogenous nucleic acid. The cell includes the primary subject cell and its progeny.

The term "specifically binds," refers to an antibody, or a ligand, which recognizes and binds with a binding partner (e.g., a tumor antigen) protein present in a sample, but which antibody or ligand does not substantially recognize or bind other molecules in the sample.

"Regulatable chimeric antigen receptor (RCAR),"as that term is used herein, refers to a set of polypeptides, typically two in the simplest embodiments, which when in a RCARX cell, provides the RCARX cell with specificity for a target cell, typically a cancer cell, and with regulatable intracellular signal generation or proliferation, which can optimize an immune effector property of the RCARX cell. An RCARX cell relies at least in part, on an antigen binding domain to provide specificity to a target cell that comprises the antigen bound by the antigen binding domain. In an embodiment, an RCAR includes a dimerization switch that, upon the presence of a dimerization molecule, can couple an intracellular signaling domain to the antigen binding domain.

"Membrane anchor" or "membrane tethering domain", as that term is used herein, refers to a polypeptide or moiety, e.g., a myristoyl group, sufficient to anchor an extracellular or intracellular domain to the plasma membrane.

"Switch domain," as that term is used herein, e.g., when referring to an RCAR, refers to an entity, typically a polypeptide-based entity, that, in the presence of a dimerization molecule, associates with another switch domain. The association results in a functional coupling of a first entity linked to, e.g., fused to, a first switch domain, and a second entity linked to, e.g., fused to, a second switch domain. A first and second switch domain are collectively referred to as a dimerization switch. In embodiments, the first and second switch domains are the same as one another, e.g., they are polypeptides having the same primary amino acid sequence, and are referred to collectively as a homodimerization switch. In embodiments, the first and second switch domains are different from one another, e.g., they are polypeptides having different primary amino acid sequences, and are referred to collectively as a heterodimerization switch. In embodiments, the switch is intracellular. In embodiments, the switch is extracellular. In embodiments, the switch domain is a polypeptide-based entity, e.g., FKBP or FRB-based, and the dimerization molecule is small molecule, e.g., a rapalogue. In embodiments, the switch domain is a polypeptide-based entity, e.g., an scFv that binds a myc peptide, and the dimerization molecule is a polypeptide, a fragment thereof, or a multimer of a polypeptide, e.g., a myc ligand or multimers of a myc ligand that bind to one or more myc scFvs. In embodiments, the switch domain is a polypeptide-based entity, e.g., myc receptor, and the dimerization molecule is an antibody or fragments thereof, e.g., myc antibody.

"Dimerization molecule," as that term is used herein, e.g., when referring to an RCAR, refers to a molecule that promotes the association of a first switch domain with a second switch domain. In embodiments, the dimerization molecule does not naturally occur in the subject, or does not occur in concentrations that would result in significant dimerization. In embodiments, the dimerization molecule is a small molecule, e.g., rapamycin or a rapalogue, e.g, RAD001.

The term "bioequivalent" refers to an amount of an agent other than the reference compound (e.g., RAD001), required to produce an effect equivalent to the effect produced by the reference dose or reference amount of the reference compound (e.g., RAD001). In an embodiment the effect is the level of mTOR inhibition, e.g., as measured by P70 S6 kinase inhibition, e.g., as evaluated in an in vivo or in vitro assay, e.g., as measured by an assay described herein, e.g., the Boulay assay. In an embodiment, the effect is alteration of the ratio of PD-1 positive/PD-1 negative T cells, as measured by cell sorting. In an embodiment a bioequivalent amount or dose of an mTOR inhibitor is the amount or dose that achieves the same level of P70 S6 kinase inhibition as does the reference dose or reference amount of a reference compound. In an embodiment, a bioequivalent amount or dose of an mTOR inhibitor is the amount or dose that achieves the same level of alteration in the ratio of PD-1 positive/PD-1 negative T cells as does the reference dose or reference amount of a reference compound.

The term "low, immune enhancing, dose" when used in conjunction with an mTOR inhibitor, e.g., an allosteric mTOR inhibitor, e.g., RAD001 or rapamycin, or a catalytic mTOR inhibitor, refers to a dose of mTOR inhibitor that partially, but not fully, inhibits mTOR activity, e.g., as measured by the inhibition of P70 S6 kinase activity. Methods for evaluating mTOR activity, e.g., by inhibition of P70 S6 kinase, are discussed herein. The dose is insufficient to result in complete immune suppression but is sufficient to enhance the immune response. In an embodiment, the low, immune enhancing, dose of mTOR inhibitor results in a decrease in the number of PD-1 positive T cells and/or an increase in the number of PD-1 negative T cells, or an increase in the ratio of PD-1 negative T cells/PD-1 positive T cells. In an embodiment, the low, immune enhancing, dose of mTOR inhibitor results in an increase in the number of naive T cells. In an embodiment, the low, immune enhancing, dose of mTOR inhibitor results in one or more of the following:
an increase in the expression of one or more of the following markers: CD62L^{high}, CD127^{high}, CD27⁺, and BCL2, e.g., on memory T cells, e.g., memory T cell precursors;
a decrease in the expression of KLRG1, e.g., on memory T cells, e.g., memory T cell precursors; and
an increase in the number of memory T cell precursors, e.g., cells with any one or combination of the following characteristics: increased CD62L^{high}, increased CD127^{high}, increased CD27⁺, decreased KLRG1, and increased BCL2;
wherein any of the changes described above occurs, e.g., at least transiently, e.g., as compared to a non-treated subject.

"Refractory" as used herein refers to a disease, e.g., cancer, that does not respond to a treatment. In embodiments, a refractory cancer can be resistant to a treatment before or at the beginning of the treatment. In other embodiments, the refractory cancer can become resistant during a treatment. A refractory cancer is also called a resistant cancer.

"Relapsed" as used herein refers to the return of a disease (e.g., cancer) or the signs and symptoms of a disease such as cancer after a period of improvement, e.g., after prior treatment of a therapy, e.g., cancer therapy

Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. As another example, a range such as 95-99% identity, includes something with 95%, 96%, 97%, 98% or 99% identity, and includes subranges such as 96-99%, 96-98%, 96-97%, 97-99%, 97-98% and 98-99% identity. This applies regardless of the breadth of the range.

### Description

Disclosed herein are compositions of matter and methods of use for the treatment of a disease such as cancer using immune effector cells (e.g., T cells, NK cells) engineered with CARs of the disclosure.

In one aspect, the disclosure provides a number of chimeric antigen receptors (CAR) comprising an antigen binding domain (e.g., antibody or antibody fragment, TCR or TCR fragment) engineered for specific binding to a tumor antigen, e.g., a tumor antigen described herein. In one aspect, the disclosure provides an immune effector cell (e.g., T cell, NK cell) engineered to express a CAR, wherein the engineered immune effector cell exhibits an anticancer property. The present invention provides an immune effector cell comprising a first CAR and a second CAR, or comprising nucleic acids encoding the same, and the therapeutic use of such a cell, as defined in the attached claims. In one aspect, a cell is transformed with the CAR and the CAR is expressed on the cell surface. In some embodiments, the cell (e.g., T cell, NK cell) is transduced with a viral vector encoding a CAR. In some embodiments, the viral vector is a retroviral vector. In some embodiments, the viral vector is a lentiviral vector. In some such embodiments, the cell may stably express the CAR. In another embodiment, the cell (e.g., T cell, NK cell) is transfected with a nucleic acid, e.g., mRNA, cDNA, DNA, encoding a CAR. In some such embodiments, the cell may transiently express the CAR.

In one aspect, the antigen binding domain of a CAR described herein is a scFv antibody fragment. In one aspect, such antibody fragments are functional in that they retain the equivalent binding affinity, e.g., they bind the same antigen with comparable affinity, as the IgG antibody from which it is derived. In other embodiments, the antibody fragment has a lower binding affinity, e.g., it binds the same antigen with a lower binding affinity than the antibody from which it is derived, but is functional in that it provides a biological response described herein. In one embodiment, the CAR molecule comprises an antibody fragment that has a binding affinity KD of 10⁻⁴ M to 10⁻⁸ M, e.g., 10⁻⁵ M to 10⁻⁷ M, e.g., 10⁻⁶ M or 10⁻⁷ M, for the target antigen. In one embodiment, the antibody fragment has a binding affinity that is at least five-fold, 10-fold, 20-fold, 30-fold, 50-fold, 100-fold or 1,000-fold less than a reference antibody, e.g., an antibody described herein.

In one aspect such antibody fragments are functional in that they provide a biological response that can include, but is not limited to, activation of an immune response, inhibition of signal-transduction origination from its target antigen, inhibition of kinase activity, and the like, as will be understood by a skilled artisan.

In one aspect, the antigen binding domain of the CAR is a scFv antibody fragment that is humanized compared to the murine sequence of the scFv from which it is derived.

In one aspect, the antigen binding domain of a CAR used in the invention (e.g., a scFv) is encoded by a nucleic acid molecule whose sequence has been codon optimized for expression in a mammalian cell. In one aspect, entire CAR construct used in the invention is encoded by a nucleic acid molecule whose entire sequence has been codon optimized for expression in a mammalian cell. Codon optimization refers to the discovery that the frequency of occurrence of synonymous codons (i.e., codons that code for the same amino acid) in coding DNA is biased in different species. Such codon degeneracy allows an identical polypeptide to be encoded by a variety of nucleotide sequences. A variety of codon optimization methods is known in the art, and include, e.g., methods disclosed in at least US Patent Numbers 5,786,464 and 6,114,148.

In one aspect, the CARs used in the invention combine an antigen binding domain of a specific antibody with an intracellular signaling molecule. For example, in some aspects, the intracellular signaling molecule includes, but is not limited to, CD3-zeta chain, 4-1BB and CD28 signaling modules and combinations thereof. In one aspect, the antigen binding domain binds to a tumor antigen as described herein.

Furthermore, the present invention provides CAR-expressing cells and their use in medicaments or methods for treating, among other diseases, cancer or any malignancy or autoimmune diseases involving cells or tissues which express a tumor antigen as described herein.

In one aspect, the CAR of the disclosure can be used to eradicate a normal cell that express a tumor antigen as described herein, thereby applicable for use as a cellular conditioning therapy prior to cell transplantation. In one aspect, the normal cell that expresses a tumor antigen as described herein is a normal stem cell and the cell transplantation is a stem cell transplantation.

In one aspect, the disclosure provides an immune effector cell (e.g., T cell, NK cell) engineered to express a chimeric antigen receptor (CAR), wherein the engineered immune effector cell exhibits an antitumor property. A preferred antigen is a cancer associated antigen (i.e., tumor antigen) described herein. In one aspect, the antigen binding domain of the CAR comprises a partially humanized antibody fragment. In one aspect, the antigen binding domain of the CAR comprises a partially humanized scFv. Accordingly, the disclosure provides CARs that comprises a humanized antigen binding domain and is engineered into a cell, e.g., a T cell or a NK cell, and methods of their use for adoptive therapy.

In one aspect, the CARs used in the invention comprise at least one intracellular domain selected from the group of a CD137 (4-1BB) signaling domain, a CD28 signaling domain, a CD27 signal domain, a CD3zeta signal domain, and any combination thereof. In one aspect, the CARs used in the invention comprise at least one intracellular signaling domain is from one or more costimulatory molecule(s) other than a CD137 (4-1BB) or CD28.

Sequences of some examples of various components of CARs used in the instant invention are listed in Table 1, where aa stands for amino acids, and na stands for nucleic acids that encode the corresponding peptide.

**Table 1. Sequences of various components of CAR (aa - amino acids, na - nucleic acids that encodes the corresponding protein)**

| SEQ ID NO | description | Sequence | Corresp. To huCD19 |
|---|---|---|---|
| 1 | EF-1 promoter | CGTGAGGCTCCGGTGCCCGTCAGTGGGCAGAGCGCACATCGCCCAC AGTCCCCGAGAAGTTGGGGGGAGGGGTCGGCAATTGAACCGGTGC CTAGAGAAGGTGGCGCGGGGTAAACTGGGAAAGTGATGTCGTGTA CTGGCTCCGCCTTTTTCCCGAGGGTGGGGGAGAACCGTATATAAGT GCAGTAGTCGCCGTGAACGTTCTTTTTCGCAACGGGTTTGCCGCCAG AACACAGGTAAGTGCCGTGTGTGGTTCCCGCGGGCCTGGCCTCTTTA CGGGTTATGGCCCTTGCGTGCCTTGAATTACTTCCACCTGGCTGCAG TACGTGATTCTTGATCCCGAGCTTCGGGTTGGAAGTGGGTGGGAGA GTTCGAGGCCTTGCGCTTAAGGAGCCCCTTCGCCTCGTGCTTGAGTT GAGGCCTGGCCTGGGCGCTGGGGCCGCCGCGTGCGAATCTGGTGG CACCTTCGCGCCTGTCTCGCTGCTTTCGATAAGTCTCTAGCCATTTAA AATTTTTGATGACCTGCTGCGACGCTTTTTTTCTGGCAAGATAGTCTT GTAAATGCGGGCCAAGATCTGCACACTGGTATTTCGGTTTTTGGGGC CGCGGGCGGCGACGGGGCCCGTGCGTCCCAGCGCACATGTTCGGC GAGGCGGGGCCTGCGAGCGCGGCCACCGAGAATCGGACGGGGGTA GTCTCAAGCTGGCCGGCCTGCTCTGGTGCCTGGCCTCGCGCCGCCGT GTATCGCCCCGCCCTGGGCGGCAAGGCTGGCCCGGTCGGCACCAGT TGCGTGAGCGGAAAGATGGCCGCTTCCCGGCCCTGCTGCAGGGAGC TCAAAATGGAGGACGCGGCGCTCGGGAGAGCGGGCGGGTGAGTCA CCCACACAAAGGAAAAGGGCCTTTCCGTCCTCAGCCGTCGCTTCATG TGACTCCACGGAGTACCGGGCGCCGTCCAGGCACCTCGATTAGTTCT CGAGCTTTTGGAGTACGTCGTCTTTAGGTTGGGGGGAGGGGTTTTAT GCGATGGAGTTTCCCCACACTGAGTGGGTGGAGACTGAAGTTAGGC CAGCTTGGCACTTGATGTAATTCTCCTTGGAATTTGCCCTTTTTGAGT TTGGATCTTGGTTCATTCTCAAGCCTCAGACAGTGGTTCAAAGTTTTT TTCTTCCATTTCAGGTGTCGTGA | 100 |
| 2 | Leader (aa) | MALPVTALLLPLALLLHAARP | 13 |
| 3 | Leader (na) | ATGGCCCTGCCTGTGACAGCCCTGCTGCTGCCTCTGGCTCTGCTGCT GCATGCCGCTAGACCC | 54 |
| 4 | CD 8 hinge (aa) | TTTPAPRPPTPAPTIASQPLSLRPEACRPAAGGAVHTRGLDFACD | 14 |
| 5 | CD8 hinge (na) | ACCACGACGCCAGCGCCGCGACCACCAACACCGGCGCCCACCATCG CGTCGCAGCCCCTGTCCCTGCGCCCAGAGGCGTGCCGGCCAGCGGC GGGGGGCGCAGTGCACACGAGGGGGCTGGACTTCGCCTGTGAT | 55 |
| 6 | Ig4 hinge (aa) | ESKYGPPCPPCPAPEFLGGPSVFLFPPKPKDTLMISRTPEVTCVVVDVSQ EDPEVQFNWYVDGVEVHNAKTKPREEQFNSTYRVVSVLTVLHQDWLN GKEYKCKVSNKGLPSSIEKTISKAKGQPREPQVYTLPPSQEEMTKNQVSL TCLVKGFYPSDIAVEWESNGQPENNYKTTPPVLDSDGSFFLYSRLTVDKS RWQEGNVFSCSVMHEALHNHYTQKSLSLSLGKM | 102 |
| 7 | Ig4 hinge (na) | GAGAGCAAGTACGGCCCTCCCTGCCCCCCTTGCCCTGCCCCCGAGTT CCTGGGCGGACCCAGCGTGTTCCTGTTCCCCCCCAAGCCCAAGGACA CCCTGATGATCAGCCGGACCCCCGAGGTGACCTGTGTGGTGGTGGA CGTGTCCCAGGAGGACCCCGAGGTCCAGTTCAACTGGTACGTGGAC | 103 |
| | | GGCGTGGAGGTGCACAACGCCAAGACCAAGCCCCGGGAGGAGCAG TTCAATAGCACCTACCGGGTGGTGTCCGTGCTGACCGTGCTGCACCA GGACTGGCTGAACGGCAAGGAATACAAGTGTAAGGTGTCCAACAAG GGCCTGCCCAGCAGCATCGAGAAAACCATCAGCAAGGCCAAGGGCC AGCCTCGGGAGCCCCAGGTGTACACCCTGCCCCCTAGCCAAGAGGA GATGACCAAGAACCAGGTGTCCCTGACCTGCCTGGTGAAGGGCTTCT ACCCCAGCGACATCGCCGTGGAGTGGGAGAGCAACGGCCAGCCCG AGAACAACTACAAGACCACCCCCCCTGTGCTGGACAGCGACGGCAG CTTCTTCCTGTACAGCCGGCTGACCGTGGACAAGAGCCGGTGGCAG GAGGGCAACGTCTTTAGCTGCTCCGTGATGCACGAGGCCCTGCACA ACCACTACACCCAGAAGAGCCTGAGCCTGTCCCTGGGCAAGATG | |
| 8 | IgD hinge (aa) | RWPESPKAQASSVPTAQPQAEGSLAKATTAPATTRNTGRGGEEKKKEK EKEEQEERETKTPECPSHTQPLGVYLLTPAVQDLWLRDKATFTCFVVGS DLKDAHLTWEVAGKVPTGGVEEGLLERHSNGSQSQHSRLTLPRSLWN AGTSVTCTLNHPSLPPQRLMALREPAAQAPVKLSLNLLASSDPPEAASW LLCEVSGFSPPNILLMWLEDQREVNTSGFAPARPPPQPGSTTFWAWSV LRVPAPPSPQPATYTCVVSHEDSRTLLNASRSLEVSYVTDH | 47 |
| 9 | IgD hinge (na) | AGGTGGCCCGAAAGTCCCAAGGCCCAGGCATCTAGTGTTCCTACTGC ACAGCCCCAGGCAGAAGGCAGCCTAGCCAAAGCTACTACTGCACCT GCCACTACGCGCAATACTGGCCGTGGCGGGAGGAGAAGAAAAAG GAGAAAGAGAAAGAAGAACAGGAAGAGAGGGAGACCAAGACCCCT GAATGTCCATCCCATACCCAGCCGCTGGGCGTCTATCTCTTGACTCCC GCAGTACAGGACTTGTGGCTTAGAGATAAGGCCACCTTTACATGTTT CGTCGTGGGCTCTGACCTGAAGGATGCCCATTTGACTTGGGAGGTT GCCGGAAAGGTACCCACAGGGGGGGTTGAGGAAGGGTTGCTGGAG CGCCATTCCAATGGCTCTCAGAGCCAGCACTCAAGACTCACCCTTCC GAGATCCCTGTGGAACGCCGGGACCTCTGTCACATGTACTCTAAATC ATCCTAGCCTGCCCCCACAGCGTCTGATGGCCCTTAGAGAGCCAGCC GCCCAGGCACCAGTTAAGCTTAGCCTGAATCTGCTCGCCAGTAGTGA TCCCCCAGAGGCCGCCAGCTGGCTCTTATGCGAAGTGTCCGGCTTTA GCCCGCCCAACATCTTGCTCATGTGGCTGGAGGACCAGCGAGAAGT GAACACCAGCGGCTTCGCTCCAGCCCGGCCCCCACCCCAGCCGGGTT CTACCACATTCTGGGCCTGGAGTGTCTTAAGGGTCCCAGCACCACCT AGCCCCCAGCCAGCCACATACACCTGTGTTGTGTCCCATGAAGATAG CAGGACCCTGCTAAATGCTTCTAGGAGTCTGGAGGTTTCCTACGTGA CTGACCATT | 48 |
| 10 | GS hinge/linker (aa) | GGGGSGGGGS | 49 |
| 11 | GS hinge/linker (na) | GGTGGCGGAGGTTCTGGAGGTGGAGGTTCC | 50 |
| 12 | CD8TM (aa) | IYIWAPLAGTCGVLLLSLVITLYC | 15 |
| 13 | CD8 TM (na) | ATCTACATCTGGGCGCCCTTGGCCGGGACTTGTGGGGTCCTTCTCCT GTCACTGGTTATCACCCTTTACTGC | 56 |
| 14 | 4-1BB intracellular domain (aa) | KRGRKKLLYIFKQPFMRPVQTTQEEDGCSCRFPEEEEGGCEL | 16 |
| 15 | 4-1BB intracellular domain (na) | AAACGGGGCAGAAAGAAACTCCTGTATATATTCAAACAACCATTTAT GAGACCAGTACAAACTACTCAAGAGGAAGATGGCTGTAGCTGCCGA TTTCCAGAAGAAGAAGAAGGAGGATGTGAACTG | 60 |
| 16 | CD27 (aa) | QRRKYRSNKGESPVEPAEPCRYSCPREEEGSTIPIQEDYRKPEPACSP | 51 |
| 17 | CD27 (na) | AGGAGTAAGAGGAGCAGGCTCCTGCACAGTGACTACATGAACATGA CTCCCCGCCGCCCCGGGCCCACCCGCAAGCATTACCAGCCCTATGCC CCACCACGCGACTTCGCAGCCTATCGCTCC | 52 |
| 18 | CD3-zeta (aa) | RVKFSRSADAPAYKQGQNQLYNELNLGRREEYDVLDKRRGRDPEMGG KPRRKNPQEGLYNELQKDKMAEAYSEIGMKGERRRGKGHDGLYQGLS TATKDTYDALHMQALPPR | 17 |
| 19 | CD3-zeta (na) | AGAGTGAAGTTCAGCAGGAGCGCAGACGCCCCCGCGTACAAGCAG GGCCAGAACCAGCTCTATAACGAGCTCAATCTAGGACGAAGAGAGG AGTACGATGTTTTGGACAAGAGACGTGGCCGGGACCCTGAGATGGG GGGAAAGCCGAGAAGGAAGAACCCTCAGGAAGGCCTGTACAATGA ACTGCAGAAAGATAAGATGGCGGAGGCCTACAGTGAGATTGGGAT GAAAGGCGAGCGCCGGAGGGGCAAGGGGCACGATGGCCTTTACCA GGGTCTCAGTACAGCCACCAAGGACACCTACGACGCCCTTCACATGC AGGCCCTGCCCCCTCGC | 101 |
| 20 | CD3-zeta (aa) | RVKFSRSADAPAYQQGQNQLYNELNLGRREEYDVLDKRRGRDPEMGG KPRRKNPQEGLYNELQKDKMAEAYSEIGMKGERRRGKGHDGLYQGLS TATKDTYDALHMQALPPR | 43 |
| 21 | CD3-zeta (na) | AGAGTGAAGTTCAGCAGGAGCGCAGACGCCCCCGCGTACCAGCAG GGCCAG AACCAGCTCTATAACGAGCTCAATCTAGGACGAAGAGAGGAGTACG ATGTTT TGGACAAGAGACGTGGCCGGGACCCTGAGATGGGGGGAAAGCCGA GAAGGA AGAACCCTCAGGAAGGCCTGTACAATGAACTGCAGAAAGATAAGAT GGCGG AGGCCTACAGTGAGATTGGGATGAAAGGCGAGCGCCGGAGGGGCA AGGGGC ACGATGGCCTTTACCAGGGTCTCAGTACAGCCACCAAGGACACCTAC GACGC CCTTCACATGCAGGCCCTGCCCCCTCGC | 44 |
| 22 | linker | GGGGS | 18 |
| 23 | linker | GGTGGCGGAGGTTCTGGAGGTGGAGGTTCC | 50 |
| 24 | PD-1 extracellular domain (aa) | | |
| 25 | PD-1 extracellular domain (na) | Cccggatggtttctggactctccggatcgcccgtggaatcccccaaccttctcaccggcact cttggttgtgactgagggcgataatgcgaccttcacgtgctcgttctccaacacctccgaat cattcgtgctgaactggtaccgcatgagcccgtcaaaccagaccgacaagctcgccgcgtt tccggaagatcggtcgcaaccgggacaggattgtcggttccgcgtgactcaactgccgaat ggcagagacttccacatgagcgtggtccgcgctaggcgaaacgactccgggacctacctg tgcggagccatctcgctggcgcctaaggcccaaatcaaagagagcttgagggccgaactg agagtgaccgagcgcagagctgaggtgccaactgcacatccatccccatcgcctcggcct gcggggcagtttcagaccctggtc | |
| 26 | PD-1 CAR (aa) with signal | Malpvtalllplalllhaarppgwfldspdrpwnpptfspallvvtegdnatftcsfsntse sfvlnwyrmspsnqtdklaafpedrsqpgqdcrfrvtqlpngrdfhmsvvrarrndsgt ylcgaislapkaqikeslraelrvterraevptahpspsprpagqfqtlvtttpaprpptpa ptiasqplslrpeacrpaaggavhtrgldfacdiyiwaplagtcgvlllslvitlyckrgrkklly ifkqpfmrpvqttqeedgcscrfpeeeeggcelrvkfsrsadapaykqgqnqlynelnl grreeydvldkrrgrdpemggkprrknpqeglynelqkdkmaeayseigmkgerrrg kghdglyqglstatkdtydalhmqalppr | |
| 27 | PD-1 CAR (na) | Atggccctccctgtcactgccctgcttctcccccctcgcactcctgctccacgccgctagacca cccggatggtttctggactctccggatcgcccgtggaatcccccaaccttctcaccggcact cttggttgtgactgagggcgataatgcgaccttcacgtgctcgttctccaacacctccgaat cattcgtgctgaactggtaccgcatgagcccgtcaaaccagaccgacaagctcgccgcgtt tccggaagatcggtcgcaaccgggacaggattgtcggttccgcgtgactcaactgccgaat ggcagagacttccacatgagcgtggtccgcgctaggcgaaacgactccgggacctacctg tgcggagccatctcgctggcgcctaaggcccaaatcaaagagagcttgagggccgaactg agagtgaccgagcgcagagctgaggtgccaactgcacatccatccccatcgcctcggcct gcggggcagtttcagaccctggtcacgaccactccggcgccgcgcccaccgactccggcc ccaactatcgcgagccagcccctgtcgctgaggccggaagcatgccgccctgccgccgga ggtgctgtgcatacccggggattggacttcgcatgcgacatctacatttgggctcctctcgc cggaacttgtggcgtgctccttctgtccctggtcatcaccctgtactgcaagcggggtcgga aaaagcttctgtacattttcaagcagcccttcatgaggcccgtgcaaaccacccaggagga ggacggttgctcctgccggttccccgaagaggaagaaggaggttgcgagctgcgcgtgaa gttctcccggagcgccgacgcccccgcctataagcagggccagaaccagctgtacaacga actgaacctgggacggcgggaagagtacgatgtgctggacaagcggcgcggccgggacc ccgaaatgggcgggaagcctagaagaaagaaccctcaggaaggcctgtataacgagctg cagaaggacaagatggccgaggcctactccgaaattgggatgaagggagagcggcgga ggggaaaggggcacgacggcctgtaccaaggactgtccaccgccaccaaggacacatac gatgccctgcacatgcaggcccttccccctcgc | |
| 28 | linker | (Gly-Gly-Gly-Ser)ₙ, where n = 1-10 | 105 |
| 29 | linker | (Gly4 Ser)4 | 106 |
| 30 | linker | (Gly4 Ser)3 | 107 |
| 31 | linker | (Gly3Ser) | 108 |
| 32 | polyA | aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa | 118 |
| | | aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa | |
| 33 | polyA | aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa | 104 |
| 34 | polyA | aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa | 109 |
| | | aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa | |
| | | aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa | |
| 35 | polyA | tttttttttt tttttttttt tttttttttt tttttttttt tttttttttt tttttttttt tttttttttt tttttttttt tttttttttt tttttttttt | 110 |
| 36 | polyA | tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt | 111 |
| | | tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt tttttttttt | |
| 37 | polyA | aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa | 112 |
| | | aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa | |
| | | aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa | |
| 38 | polyA | aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa  aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa aaaaaaaaaa  aaaaaaaaaa aaaaaaaaaa aaaaaaaaaa aaaaaaaaaa | 113 |
| 39 | PD1 CAR (aa) | Pgwfldspdrpwnpptfspallvvtegdnatftcsfsntsesfvlnwyrmspsnqtdkl aafpedrsqpgqdcrfrvtqlpngrdfhmsvvrarrndsgtylcgaislapkaqikeslra elrvterraevptahpspsprpagqfqtlvtttpaprpptpaptiasqplslrpeacrpaa ggavhtrgldfacdiyiwaplagtcgvlllslvitlyckrgrkkllyifkqpfmrpvqttqeed gcscrfpeeeeggcelrvkfsrsadapaykqgqnqlynelnlgrreeydvldkrrgrdpe mggkprrknpqeglynelqkdkmaeayseigmkgerrrgkghdglyqglstatkdty dalhmqalppr | |

### Cancer Associated Antigens

The present disclosure provides immune effector cells (e.g., T cells, NK cells) that are engineered to contain one or more CARs that direct the immune effector cells to cancer. This is achieved through an antigen binding domain on the CAR that is specific for a cancer associated antigen. There are two classes of cancer associated antigens (tumor antigens) that can be targeted by the CARs of the instant disclosure: (1) cancer associated antigens that are expressed on the surface of cancer cells; and (2) cancer associated antigens that itself is intracellar, however, a fragment of such antigen (peptide) is presented on the surface of the cancer cells by MHC (major histocompatibility complex). The present invention provides immune effector cells in accordance with the claims.

Accordingly, the present invention employs CARs that target the following cancer associated antigens (tumor antigens): CD19, CD123, CD22, CD30, CD171, CS-1, CLL-1 (CLECL1), CD33, EGFRvIII, GD2, GD3, BCMA, Tn Ag, PSMA, ROR1, FLT3, FAP, TAG72, CD38, CD44v6, CEA, EPCAM, B7H3, KIT, IL-13Ra2, Mesothelin, IL-11Ra, PSCA, VEGFR2, LewisY, CD24, PDGFR-beta, PRSS21, SSEA-4, CD20, Folate receptor alpha, ERBB2 (Her2/neu), MUC1, EGFR, NCAM, Prostase, PAP, ELF2M, Ephrin B2, IGF-I receptor, CAIX, LMP2, gp100, bcr-abl, tyrosinase, EphA2, Fucosyl GM1, sLe, GM3, TGS5, HMWMAA, o-acetyl-GD2, Folate receptor beta, TEM1/CD248, TEM7R, CLDN6, TSHR, GPRC5D, CXORF61, CD97, CD179a, ALK, Polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, legumain, HPV E6,E7, MAGE-A1, MAGE A1, ETV6-AML, sperm protein 17, XAGE1, Tie 2, MAD-CT-1, MAD-CT-2, Fos-related antigen 1, p53, p53 mutant, prostein, survivin and telomerase, PCTA-1/Galectin 8, MelanA/MART1, Ras mutant, hTERT, sarcoma translocation breakpoints, ML-IAP, ERG (TMPRSS2 ETS fusion gene), NA17, PAX3, Androgen receptor, Cyclin B1, MYCN, RhoC, TRP-2, CYP1B1, BORIS, SART3, PAX5, OY-TES1, LCK, AKAP-4, SSX2, RAGE-1, human telomerase reverse transcriptase, RU1, RU2, intestinal carboxyl esterase, mut hsp70-2, CD79a, CD79b, CD72, LAIR1, FCAR, LILRA2, CD300LF, CLEC12A, BST2, EMR2, LY75, GPC3, FCRL5, and IGLL1.

### Tumor-supporting antigens

A CAR described herein can comprise an antigen binding domain (e.g., antibody or antibody fragment, TCR or TCR fragment) that binds to a tumor-supporting antigen (e.g., a tumor-supporting antigen as described herein). In some embodiments, the tumor-supporting antigen is an antigen present on a stromal cell or a myeloid-derived suppressor cell (MDSC). Stromal cells can secrete growth factors to promote cell division in the microenvironment. MDSC cells can inhibit T cell proliferation and activation. Without wishing to be bound by theory, in some embodiments, the CAR-expressing cells destroy the tumor-supporting cells, thereby indirectly inhibiting tumor growth or survival.

In embodiments, the stromal cell antigen is chosen from one or more of: bone marrow stromal cell antigen 2 (BST2), fibroblast activation protein (FAP) and tenascin. In an embodiment, the FAP-specific antibody is, competes for binding with, or has the same CDRs as, sibrotuzumab. In embodiments, the MDSC antigen is chosen from one or more of: CD33, CD11b, C14, CD15, and CD66b. Accordingly, in some embodiments, the tumor-supporting antigen is chosen from one or more of: bone marrow stromal cell antigen 2 (BST2), fibroblast activation protein (FAP) or tenascin, CD33, CD11b, C14, CD15, and CD66b.

### Chimeric Antigen Receptor (CAR)

The present invention may employ a recombinant DNA construct comprising sequences encoding a CAR, wherein the CAR comprises an antigen binding domain that binds specifically to a cancer associated antigen described herein, wherein the sequence of the antigen binding domain is contiguous with and in the same reading frame as a nucleic acid sequence encoding an intracellular signaling domain. The intracellular signaling domain can comprise a costimulatory signaling domain and/or a primary signaling domain, e.g., a zeta chain. The costimulatory signaling domain refers to a portion of the CAR comprising at least a portion of the intracellular domain of a costimulatory molecule.

In specific aspects, a CAR construct used in the invention comprises a scFv domain, wherein the scFv may be preceded by an optional leader sequence such as provided in SEQ ID NO: 2, and followed by an optional hinge sequence such as provided in SEQ ID NO:4 or SEQ ID NO:6 or SEQ ID NO:8 or SEQ ID NO:10, a transmembrane region such as provided in SEQ ID NO: 12, an intracellular signalling domain that includes SEQ ID NO:14 or SEQ ID NO:16 and a CD3 zeta sequence that includes SEQ ID NO:18 or SEQ ID NO:20, e.g., wherein the domains are contiguous with and in the same reading frame to form a single fusion protein.

In one aspect, exemplary CAR constructs comprise an optional leader sequence (e.g., a leader sequence described herein), an extracellular antigen binding domain (e.g., an antigen binding domain described herein), a hinge (e.g., a hinge region described herein), a transmembrane domain (e.g., a transmembrane domain described herein), and an intracellular stimulatory domain (e.g., an intracellular stimulatory domain decribed herein). In one aspect, an exemplary CAR construct comprises an optional leader sequence (e.g., a leader sequence described herein), an extracellular antigen binding domain (e.g., an antigen binding domain described herein), a hinge (e.g., a hinge region described herein), a transmembrane domain (e.g., a transmembrane domain described herein), an intracellular costimulatory signaling domain (e.g., a costimulatory signaling domain described herein) and/or an intracellular primary signaling domain (e.g., a primary signaling domain described herein).

An exemplary leader sequence is provided as SEQ ID NO: 2. An exemplary hinge/spacer sequence is provided as SEQ ID NO: 4 or SEQ ID NO:6 or SEQ ID NO:8 or SEQ ID NO: 10. An exemplary transmembrane domain sequence is provided as SEQ ID NO: 12. An exemplary sequence of the intracellular signaling domain of the 4-1BB protein is provided as SEQ ID NO: 14. An exemplary sequence of the intracellular signaling domain of CD27 is provided as SEQ ID NO:16. An exemplary CD3zeta domain sequence is provided as SEQ ID NO: 18 or SEQ ID NO:20.

In one aspect, the present invention employs a recombinant nucleic acid construct comprising a nucleic acid molecule encoding a CAR, wherein the nucleic acid molecule comprises the nucleic acid sequence encoding an antigen binding domain that is contiguous with and in the same reading frame as a nucleic acid sequence encoding an intracellular signaling domain.

In one aspect, the present invention employs a recombinant nucleic acid construct comprising a nucleic acid molecule encoding a CAR, wherein the nucleic acid molecule comprises a nucleic acid sequence encoding an antigen binding domain, wherein the sequence is contiguous with and in the same reading frame as the nucleic acid sequence encoding an intracellular signaling domain. An exemplary intracellular signaling domain that can be used in the CAR includes, but is not limited to, one or more intracellular signaling domains of, e.g., CD3-zeta, CD28, CD27, 4-1BB, and the like. In some instances, the CAR can comprise any combination of CD3-zeta, CD28, 4-1BB, and the like.

The nucleic acid sequences coding for the desired molecules can be obtained using recombinant methods known in the art, such as, for example by screening libraries from cells expressing the nucleic acid molecule, by deriving the nucleic acid molecule from a vector known to include the same, or by isolating directly from cells and tissues containing the same, using standard techniques. Alternatively, the nucleic acid of interest can be produced synthetically, rather than cloned.

The present invention may employ retroviral and lentiviral vector constructs expressing a CAR that can be directly transduced into a cell.

The present invention may employ an RNA construct that can be directly transfected into a cell. A method for generating mRNA for use in transfection involves *in vitro* transcription (IVT) of a template with specially designed primers, followed by polyA addition, to produce a construct containing 3' and 5' untranslated sequence ("UTR") (e.g., a 3' and/or 5' UTR described herein), a 5' cap (e.g., a 5' cap described herein) and/or Internal Ribosome Entry Site (IRES) (e.g., an IRES described herein), the nucleic acid to be expressed, and a polyA tail, typically 50-2000 bases in length (SEQ ID NO:32). RNA so produced can efficiently transfect different kinds of cells. In one instance, the template includes sequences for the CAR. In an instance, an RNA CAR vector is transduced into a cell, e.g., a T cell or a NK cell, by electroporation.

### Antigen binding domain

The CAR employed in the invention comprises a target-specific binding element otherwise referred to as an antigen binding domain. The choice of moiety depends upon the type and number of ligands that define the surface of a target cell. For example, the antigen binding domain may be chosen to recognize a ligand that acts as a cell surface marker on target cells associated with a particular disease state. Thus, examples of cell surface markers that may act as ligands for the antigen binding domain in a CAR of the disclosure include those associated with viral, bacterial and parasitic infections, autoimmune disease and cancer cells.

In one aspect, the CAR-mediated T-cell response can be directed to an antigen of interest by way of engineering an antigen binding domain that specifically binds a desired antigen into the CAR

In one aspect, the portion of the CAR comprising the antigen binding domain comprises an antigen binding domain that targets a tumor antigen, e.g., a tumor antigen described herein.

The antigen binding domain of a CAR of the disclosure can be any domain that binds to the antigen including but not limited to a monoclonal antibody, a polyclonal antibody, a recombinant antibody, a human antibody, a humanized antibody, and a functional fragment thereof, including but not limited to a single-domain antibody such as a heavy chain variable domain (VH), a light chain variable domain (VL) and a variable domain (VHH) of camelid derived nanobody, and to an alternative scaffold known in the art to function as antigen binding domain, such as a recombinant fibronectin domain, a T cell receptor (TCR), or a fragment there of, e.g., single chain TCR, and the like. In the present invention, the antigen binding domain of the first CAR comprises a VHH domain and the antigen binding domain of the second CAR comprises an scFv. In some instances, it is beneficial for the antigen binding domain to be derived from the same species in which the CAR will ultimately be used in. For example, for use in humans, it may be beneficial for the antigen binding domain of the CAR to comprise human or humanized residues for the antigen binding domain of an antibody or antibody fragment.

In one embodiment, an antigen binding domain against CD22 is an antigen binding portion, e.g., CDRs, of an antibody described in, e.g., Haso et al., Blood, 121(7): 1165-1174 (2013); Wayne et al., Clin Cancer Res 16(6): 1894-1903 (2010); Kato et al., Leuk Res 37(1):83-88 (2013); Creative BioMart (creativebiomart.net): MOM-18047-S(P).

In one embodiment, an antigen binding domain against CS-1 is an antigen binding portion, e.g., CDRs, of Elotuzumab (BMS), see e.g., Tai et al., 2008, Blood 112(4):1329-37; Tai et al., 2007, Blood. 110(5):1656-63.

In one embodiment, an antigen binding domain against CLL-1 is an antigen binding portion, e.g., CDRs, of an antibody available from R&D, ebiosciences, Abcam, for example, PE-CLL1-hu Cat# 353604 (BioLegend); and PE-CLL1 (CLEC12A) Cat# 562566 (BD).

In one embodiment, an antigen binding domain against CD33 is an antigen binding portion, e.g., CDRs, of an antibody described in, e.g., Bross et al., Clin Cancer Res 7(6):1490-1496 (2001) (Gemtuzumab Ozogamicin, hP67.6),Caron et al., Cancer Res 52(24):6761-6767 (1992) (Lintuzumab, HuM195), Lapusan et al., Invest New Drugs 30(3):1121-1131 (2012) (AVE9633), Aigner et al., Leukemia 27(5): 1107-1115 (2013) (AMG330, CD33 BiTE), Dutour et al., Adv hematol 2012:683065 (2012), and Pizzitola et al., Leukemia doi:10.1038/Lue.2014.62 (2014).

In one embodiment, an antigen binding domain against GD2 is an antigen binding portion, e.g., CDRs, of an antibody described in, e.g., Mujoo et al., Cancer Res. 47(4):1098-1104 (1987); Cheung et al., Cancer Res 45(6):2642-2649 (1985), Cheung et al., J Clin Oncol 5(9):1430-1440 (1987), Cheung et al., J Clin Oncol 16(9):3053-3060 (1998), Handgretinger et al., Cancer Immunol Immunother 35(3):199-204 (1992). In some embodiments, an antigen binding domain against GD2 is an antigen binding portion of an antibody selected from mAb 14.18, 14G2a, ch14.18, hu14.18, 3F8, hu3F8, 3G6, 8B6, 60C3, 10B8, ME36.1, and 8H9, see e.g., WO2012033885, WO2013040371, WO2013192294, WO2013061273, WO2013123061, WO2013074916, and WO201385552. In some embodiments, an antigen binding domain against GD2 is an antigen binding portion of an antibody described in US Publication No.: 20100150910 or PCT Publication No.: WO 2011160119.

In one embodiment, an antigen binding domain against BCMA is an antigen binding portion, e.g., CDRs, of an antibody described in, e.g., WO2012163805, WO200112812, and WO2003062401.

In one embodiment, an antigen binding domain against Tn antigen is an antigen binding portion, e.g., CDRs, of an antibody described in, e.g., US8,440,798, Brooks et al., PNAS 107(22):10056-10061 (2010), and Stone et al., OncoImmunology 1(6):863-873(2012).

In one embodiment, an antigen binding domain against PSMA is an antigen binding portion, e.g., CDRs, of an antibody described in, e.g., Parker et al., Protein Expr Purif 89(2):136-145 (2013), US 20110268656 (J591 ScFv); Frigerio et al, European J Cancer 49(9):2223-2232 (2013) (scFvD2B); WO 2006125481 (mAbs 3/A12, 3/E7 and 3/F11) and single chain antibody fragments (scFv A5 and D7).

In one embodiment, an antigen binding domain against ROR1 is an antigen binding portion, e.g., CDRs, of an antibody described in, e.g., Hudecek et al., Clin Cancer Res 19(12):3153-3164 (2013); WO 2011159847; and US20130101607.

In one embodiment, an antigen binding domain against FLT3 is an antigen binding portion, e.g., CDRs, of an antibody described in, e.g., WO2011076922, US5777084, EP0754230, US20090297529, and several commercial catalog antibodies (R&D, ebiosciences, Abcam).

In one embodiment, an antigen binding domain against TAG72 is an antigen binding portion, e.g., CDRs, of an antibody described in, e.g., Hombach et al., Gastroenterology 113(4):1163-1170 (1997); and Abcam ab691.

In one embodiment, an antigen binding domain against FAP is an antigen binding portion, e.g., CDRs, of an antibody described in, e.g., Ostermann et al., Clinical Cancer Research 14:4584-4592 (2008) (FAP5), US Pat. Publication No. 2009/0304718; sibrotuzumab (see e.g., Hofheinz et al., Oncology Research and Treatment 26(1), 2003); and Tran et al., J Exp Med 210(6):1125-1135 (2013).

In one embodiment, an antigen binding domain against CD38 is an antigen binding portion, e.g., CDRs, of daratumumab (see, e.g., Groen et al., Blood 116(21):1261-1262 (2010); MOR202 (see, e.g., US8,263,746); or antibodies described in US8,362,211.

In one embodiment, an antigen binding domain against CD44v6 is an antigen binding portion, e.g., CDRs, of an antibody described in, e.g., Casucci et al., Blood 122(20):3461-3472 (2013).

In one embodiment, an antigen binding domain against CEA is an antigen binding portion, e.g., CDRs, of an antibody described in, e.g., Chmielewski et al., Gastoenterology 143(4):1095-1107 (2012).

In one embodiment, an antigen binding domain against EPCAM is an antigen binding portion, e.g., CDRS, of an antibody selected from MT110, EpCAM-CD3 bispecific Ab (see, e.g., clinicaltrials.gov/ct2/show/NCT00635596); Edrecolomab; 3622W94; ING-1; and adecatumumab (MT201).

In one embodiment, an antigen binding domain against PRSS21 is an antigen binding portion, e.g., CDRs, of an antibody described in US Patent No.: 8,080,650.

In one embodiment, an antigen binding domain against B7H3 is an antigen binding portion, e.g., CDRs, of an antibody MGA271 (Macrogenics).

In one embodiment, an antigen binding domain against KIT is an antigen binding portion, e.g., CDRs, of an antibody described in, e.g., US7915391, US20120288506 , and several commercial catalog antibodies.

In one embodiment, an antigen binding domain against IL-13Ra2 is an antigen binding portion, e.g., CDRs, of an antibody described in, e.g., WO2008/146911, WO2004087758, several commercial catalog antibodies, and WO2004087758.

In one embodiment, an antigen binding domain against CD30 is an antigen binding portion, e.g., CDRs, of an antibody described in, e.g., US7090843 B1, and EP0805871.

In one embodiment, an antigen binding domain against GD3 is an antigen binding portion, e.g., CDRs, of an antibody described in, e.g., US7253263; US 8,207,308; US 20120276046; EP1013761; WO2005035577; and US6437098.

In one embodiment, an antigen binding domain against CD171 is an antigen binding portion, e.g., CDRs, of an antibody described in, e.g., Hong et al., J Immunother 37(2):93-104 (2014).

In one embodiment, an antigen binding domain against IL-11Ra is an antigen binding portion, e.g., CDRs, of an antibody available from Abcam (cat# ab55262) or Novus Biologicals (cat# EPR5446). In another embodiment, an antigen binding domain again IL-11Ra is a peptide, see, e.g., Huang et al., Cancer Res 72(1):271-281 (2012).

In one embodiment, an antigen binding domain against PSCA is an antigen binding portion, e.g., CDRs, of an antibody described in, e.g., Morgenroth et al., Prostate 67(10):1121-1131 (2007) (scFv 7F5); Nejatollahi et al., J of Oncology 2013(2013), article ID 839831 (scFv C5-II); and US Pat Publication No. 20090311181.

In one embodiment, an antigen binding domain against VEGFR2 is an antigen binding portion, e.g., CDRs, of an antibody described in, e.g., Chinnasamy et al., J Clin Invest 120(11):3953-3968 (2010).

In one embodiment, an antigen binding domain against LewisY is an antigen binding portion, e.g., CDRs, of an antibody described in, e.g., Kelly et al., Cancer Biother Radiopharm 23(4):411-423 (2008) (hu3S193 Ab (scFvs)); Dolezal et al., Protein Engineering 16(1):47-56 (2003) (NC10 scFv).

In one embodiment, an antigen binding domain against CD24 is an antigen binding portion, e.g., CDRs, of an antibody described in, e.g., Maliar et al., Gastroenterology 143(5):1375-1384 (2012).

In one embodiment, an antigen binding domain against PDGFR-beta is an antigen binding portion, e.g., CDRs, of an antibody Abcam ab32570.

In one embodiment, an antigen binding domain against SSEA-4 is an antigen binding portion, e.g., CDRs, of antibody MC813 (Cell Signaling), or other commercially available antibodies.

In one embodiment, an antigen binding domain against CD20 is an antigen binding portion, e.g., CDRs, of the antibody Rituximab, Ofatumumab, Ocrelizumab, Veltuzumab, or GA101.

In one embodiment, an antigen binding domain against Folate receptor alpha is an antigen binding portion, e.g., CDRs, of the antibody IMGN853, or an antibody described in US20120009181; US4851332, LK26: US5952484.

In one embodiment, an antigen binding domain against ERBB2 (Her2/neu) is an antigen binding portion, e.g., CDRs, of the antibody trastuzumab, or pertuzumab.

In one embodiment, an antigen binding domain against MUC1 is an antigen binding portion, e.g., CDRs, of the antibody SAR566658.

In one embodiment, the antigen binding domain against EGFR is antigen binding portion, e.g., CDRs, of the antibody cetuximab, panitumumab, zalutumumab, nimotuzumab, or matuzumab.

In one embodiment, an antigen binding domain against NCAM is an antigen binding portion, e.g., CDRs, of the antibody clone 2-2B: MAB5324 (EMD Millipore)

In one embodiment, an antigen binding domain against Ephrin B2 is an antigen binding portion, e.g., CDRs, of an antibody described in, e.g., Abengozar et al., Blood 119(19):4565-4576 (2012).

In one embodiment, an antigen binding domain against IGF-I receptor is an antigen binding portion, e.g., CDRs, of an antibody described in, e.g., US8344112 B2; EP2322550 A1; WO 2006/138315, or PCT/US2006/022995.

In one embodiment, an antigen binding domain against CAIX is an antigen binding portion, e.g., CDRs, of the antibody clone 303123 (R&D Systems).

In one embodiment, an antigen binding domain against LMP2 is an antigen binding portion, e.g., CDRs, of an antibody described in, e.g., US7,410,640, or US20050129701.

In one embodiment, an antigen binding domain against gp100 is an antigen binding portion, e.g., CDRs, of the antibody HMB45, NKIbetaB, or an antibody described in WO2013165940, or US20130295007

In one embodiment, an antigen binding domain against tyrosinase is an antigen binding portion, e.g., CDRs, of an antibody described in, e.g., US5843674; or US19950504048.

In one embodiment, an antigen binding domain against EphA2 is an antigen binding portion, e.g., CDRs, of an antibody described in, e.g., Yu et al., Mol Ther 22(1):102-111 (2014).

In one embodiment, an antigen binding domain against GD3 is an antigen binding portion, e.g., CDRs, of an antibody described in, e.g., US7253263; US 8,207,308; US 20120276046; EP1013761 A3; 20120276046; WO2005035577; or US6437098.

In one embodiment, an antigen binding domain against fucosyl GM1 is an antigen binding portion, e.g., CDRs, of an antibody described in, e.g., US20100297138; or WO2007/067992.

In one embodiment, an antigen binding domain against sLe is an antigen binding portion, e.g., CDRs, of the antibody G193 (for lewis Y), see Scott AM et al, Cancer Res 60: 3254-61 (2000), also as described in Neeson et al, J Immunol May 2013 190 (Meeting Abstract Supplement) 177.10.

In one embodiment, an antigen binding domain against GM3 is an antigen binding portion, e.g., CDRs, of the antibody CA 2523449 (mAb 14F7).

In one embodiment, an antigen binding domain against HMWMAA is an antigen binding portion, e.g., CDRs, of an antibody described in, e.g., Kmiecik et al., Oncoimmunology 3(1):e27185 (2014) (PMID: 24575382) (mAb9.2.27); US6528481; WO2010033866; or US 20140004124.

In one embodiment, an antigen binding domain against o-acetyl-GD2 is an antigen binding portion, e.g., CDRs, of the antibody 8B6.

In one embodiment, an antigen binding domain against TEM1/CD248 is an antigen binding portion, e.g., CDRs, of an antibody described in, e.g., Marty et al., Cancer Lett 235(2):298-308 (2006); Zhao et al., J Immunol Methods 363(2):221-232 (2011).

In one embodiment, an antigen binding domain against CLDN6 is an antigen binding portion, e.g., CDRs, of the antibody IMAB027 (Ganymed Pharmaceuticals), see e.g., clinicaltrial.gov/show/NCT02054351.

In one embodiment, an antigen binding domain against TSHR is an antigen binding portion, e.g., CDRs, of an antibody described in, e.g., US8,603,466; US8,501,415; or US8,309,693.

In one embodiment, an antigen binding domain against GPRC5D is an antigen binding portion, e.g., CDRs, of the antibody FAB6300A (R&D Systems); or LS-A4180 (Lifespan Biosciences).

In one embodiment, an antigen binding domain against CD97 is an antigen binding portion, e.g., CDRs, of an antibody described in, e.g., US6,846,911;de Groot et al., J Immunol 183(6):4127-4134 (2009); or an antibody from R&D:MAB3734.

In one embodiment, an antigen binding domain against ALK is an antigen binding portion, e.g., CDRs, of an antibody described in, e.g., Mino-Kenudson et al., Clin Cancer Res 16(5):1561-1571 (2010).

In one embodiment, an antigen binding domain against polysialic acid is an antigen binding portion, e.g., CDRs, of an antibody described in, e.g., Nagae et al., J Biol Chem 288(47):33784-33796 (2013).

In one embodiment, an antigen binding domain against PLAC1 is an antigen binding portion, e.g., CDRs, of an antibody described in, e.g., Ghods et al., Biotechnol Appl Biochem 2013 doi:10.1002/bab.1177.

In one embodiment, an antigen binding domain against GloboH is an antigen binding portion of the antibody VK9; or an antibody described in, e.g., Kudryashov V et al, Glycoconj J.15(3):243-9 ( 1998), Lou et al., Proc Natl Acad Sci USA 111(7):2482-2487 (2014) ; MBr1: Bremer E-G et al. J Biol Chem 259:14773-14777 (1984).

In one embodiment, an antigen binding domain against NY-BR-1 is an antigen binding portion, e.g., CDRs of an antibody described in, e.g., Jager et al., Appl Immunohistochem Mol Morphol 15(1):77-83 (2007).

In one embodiment, an antigen binding domain against WT-1 is an antigen binding portion, e.g., CDRs, of an antibody described in, e.g., Dao et al., Sci Transl Med 5(176):176ra33 (2013); or WO2012/135854.

In one embodiment, an antigen binding domain against MAGE-A1 is an antigen binding portion, e.g., CDRs, of an antibody described in, e.g., Willemsen et al., J Immunol 174(12):7853-7858 (2005) (TCR-like scFv).

In one embodiment, an antigen binding domain against sperm protein 17 is an antigen binding portion, e.g., CDRs, of an antibody described in, e.g., Song et al., Target Oncol 2013 Aug 14 (PMID: 23943313); Song et al., Med Oncol 29(4):2923-2931 (2012).

In one embodiment, an antigen binding domain against Tie 2 is an antigen binding portion, e.g., CDRs, of the antibody AB33 (Cell Signaling Technology).

In one embodiment, an antigen binding domain against MAD-CT-2 is an antigen binding portion, e.g., CDRs, of an antibody described in, e.g., PMID: 2450952; US7635753.

In one embodiment, an antigen binding domain against Fos-related antigen 1 is an antigen binding portion, e.g., CDRs, of the antibody 12F9 (Novus Biologicals).

In one embodiment, an antigen binding domain against MelanA/MART1 is an antigen binding portion, e.g., CDRs, of an antibody described in, EP2514766 A2; or US 7,749,719.

In one embodiment, an antigen binding domain against sarcoma translocation breakpoints is an antigen binding portion, e.g., CDRs, of an antibody described in, e.g., Luo et al, EMBO Mol. Med. 4(6):453-461 (2012).

In one embodiment, an antigen binding domain against TRP-2 is an antigen binding portion, e.g., CDRs, of an antibody described in, e.g., Wang et al, J Exp Med. 184(6):2207-16 (1996).

In one embodiment, an antigen binding domain against CYP1B1 is an antigen binding portion, e.g., CDRs, of an antibody described in, e.g., Maecker et al, Blood 102 (9): 3287-3294 (2003).

In one embodiment, an antigen binding domain against RAGE-1 is an antigen binding portion, e.g., CDRs, of the antibody MAB5328 (EMD Millipore).

In one embodiment, an antigen binding domain against human telomerase reverse transcriptase is an antigen binding portion, e.g., CDRs, of the antibody cat no: LS-B95-100 (Lifespan Biosciences)

In one embodiment, an antigen binding domain against intestinal carboxyl esterase is an antigen binding portion, e.g., CDRs, of the antibody 4F12: cat no: LS-B6190-50 (Lifespan Biosciences).

In one embodiment, an antigen binding domain against mut hsp70-2 is an antigen binding portion, e.g., CDRs, of the antibody Lifespan Biosciences: monoclonal: cat no: LS-C133261-100 (Lifespan Biosciences).

In one embodiment, an antigen binding domain against CD79a is an antigen binding portion, e.g., CDRs, of the antibody Anti-CD79a antibody [HM47/A9] (ab3121), available from Abcam; antibody CD79A Antibody #3351 available from Cell Signalling Technology; or antibody HPA017748 - Anti-CD79A antibody produced in rabbit, available from Sigma Aldrich.

In one embodiment, an antigen binding domain against CD79b is an antigen binding portion, e.g., CDRs, of the antibody polatuzumab vedotin, anti-CD79b described in Dornan et al., "Therapeutic potential of an anti-CD79b antibody-drug conjugate, anti-CD79b-vc-MMAE, for the treatment of non-Hodgkin lymphoma" Blood. 2009 Sep 24;114(13):2721-9. doi: 10.1182/blood-2009-02-205500. Epub 2009 Jul 24, or the bispecific antibody Anti-CD79b/CD3 described in "4507 Pre-Clinical Characterization of T Cell-Dependent Bispecific Antibody Anti-CD79b/CD3 As a Potential Therapy for B Cell Malignancies" Abstracts of 56th ASH Annual Meeting and Exposition, San Francisco, CA December 6-9 2014.

In one embodiment, an antigen binding domain against CD72 is an antigen binding portion, e.g., CDRs, of the antibody J3-109 described in Myers, and Uckun, "An anti-CD72 immunotoxin against therapy-refractory B-lineage acute lymphoblastic leukemia." Leuk Lymphoma. 1995 Jun;18(1-2):119-22, or anti-CD72 (10D6.8.1, mIgG1) described in Polson et al., "Antibody-Drug Conjugates for the Treatment of Non-Hodgkin's Lymphoma: Target and Linker-Drug Selection" Cancer Res March 15, 2009 69; 2358.

In one embodiment, an antigen binding domain against LAIR1 is an antigen binding portion, e.g., CDRs, of the antibody ANT-301 LAIR1 antibody, available from ProSpec; or anti-human CD305 (LAIR1) Antibody, available from BioLegend.

In one embodiment, an antigen binding domain against FCAR is an antigen binding portion, e.g., CDRs, of the antibody CD89/FCARAntibody (Catalog#10414-H08H), available from Sino Biological Inc.

In one embodiment, an antigen binding domain against LILRA2 is an antigen binding portion, e.g., CDRs, of the antibody LILRA2 monoclonal antibody (M17), clone 3C7, available from Abnova, or Mouse Anti-LILRA2 antibody, Monoclonal (2D7), available from Lifespan Biosciences..

In one embodiment, an antigen binding domain against CD300LF is an antigen binding portion, e.g., CDRs, of the antibody Mouse Anti-CMRF35-like molecule 1 antibody, Monoclonal[UP-D2], available from BioLegend, or Rat Anti-CMRF35-like molecule 1 antibody, Monoclonal[234903], available from R&D Systems..

In one embodiment, an antigen binding domain against CLEC12A is an antigen binding portion, e.g., CDRs, of the antibody Bispecific T cell Engager (BiTE) scFvantibody and ADC described in Noordhuis et al., "Targeting of CLEC12A In Acute Myeloid Leukemia by Antibody-Drug-Conjugates and Bispecific CLL-1xCD3 BiTE Antibody" 53rd ASH Annual Meeting and Exposition, December 10-13, 2011, and MCLA-117 (Merus).

In one embodiment, an antigen binding domain against BST2 (also called CD317) is an antigen binding portion, e.g., CDRs, of the antibody Mouse Anti-CD317 antibody, Monoclonal[3H4], available from Antibodies-Online or Mouse Anti-CD317 antibody, Monoclonal[696739], available from R&D Systems.

In one embodiment, an antigen binding domain against EMR2 (also called CD312) is an antigen binding portion, e.g., CDRs, of the antibody Mouse Anti-CD312 antibody, Monoclonal[LS-B8033] available from Lifespan Biosciences, or Mouse Anti-CD312 antibody, Monoclonal[494025] available from R&D Systems.

In one embodiment, an antigen binding domain against LY75 is an antigen binding portion, e.g., CDRs, of the antibody Mouse Anti-Lymphocyte antigen 75 antibody, Monoclonal [HD30] available from EMD Millipore or Mouse Anti-Lymphocyte antigen 75 antibody, Monoclonal[A15797] available from Life Technologies.

In one embodiment, an antigen binding domain against GPC3 is an antigen binding portion, e.g., CDRs, of the antibody hGC33 described in Nakano K, Ishiguro T, Konishi H, et al. Generation of a humanized anti-glypican 3 antibody by CDR grafting and stability optimization. Anticancer Drugs. 2010 Nov;21(10):907-916, or MDX-1414, HN3, or YP7, all three of which are described in Feng et al., "Glypican-3 antibodies: a new therapeutic target for liver cancer." FEBS Lett. 2014 Jan 21;588(2):377-82.

In one embodiment, an antigen binding domain against FCRL5 is an antigen binding portion, e.g., CDRs, of the anti-FcRL5 antibody described in Elkins et al., "FcRL5 as a target of antibody-drug conjugates for the treatment of multiple myeloma" Mol Cancer Ther. 2012 Oct;11(10):2222-32..

In one embodiment, an antigen binding domain against IGLL1 is an antigen binding portion, e.g., CDRs, of the antibody Mouse Anti-Immunoglobulin lambda-like polypeptide 1 antibody, Monoclonal[AT1G4] available from Lifespan Biosciences, Mouse Anti-Immunoglobulin lambda-like polypeptide 1 antibody, Monoclonal[HSL11] available from BioLegend.

In one embodiment, the antigen binding domain comprises one, two three (e.g., all three) heavy chain CDRs, HC CDR1, HC CDR2 and HC CDR3, from an antibody listed above, and/or one, two, three (e.g., all three) light chain CDRs, LC CDR1, LC CDR2 and LC CDR3, from an antibody listed above. In one embodiment, the antigen binding domain comprises a heavy chain variable region and/or a variable light chain region of an antibody listed above.

In another aspect, the antigen binding domain comprises a humanized antibody or an antibody fragment. In some aspects, a non-human antibody is humanized, where specific sequences or regions of the antibody are modified to increase similarity to an antibody naturally produced in a human or fragment thereof. In one aspect, the antigen binding domain is humanized.

A humanized antibody can be produced using a variety of techniques known in the art, including but not limited to, CDR-grafting (see, e.g., European Patent No. EP 239,400; International Publication No. WO 91/09967; and U.S. Pat. Nos. 5,225,539, 5,530,101, and 5,585,089), veneering or resurfacing (see, e.g., European Patent Nos. EP 592,106 and EP 519,596; Padlan, 1991, Molecular Immunology, 28(4/5):489-498; Studnicka et al., 1994, Protein Engineering, 7(6):805-814; and Roguska et al., 1994, PNAS, 91:969-973), chain shuffling (see, e.g., U.S. Pat. No. 5,565,332), and techniques disclosed in, e.g., U.S. Patent Application Publication No. US2005/0042664, U.S. Patent Application Publication No. US2005/0048617, U.S. Pat. No. 6,407,213, U.S. Pat. No. 5,766,886, International Publication No. WO 9317105, Tan et al., J. Immunol., 169:1119-25 (2002), Caldas et al., Protein Eng., 13(5):353-60 (2000), Morea et al., Methods, 20(3):267-79 (2000), Baca et al., J. Biol. Chem., 272(16):10678-84 (1997), Roguska et al., Protein Eng., 9(10):895-904 (1996), Couto et al., Cancer Res., 55 (23 Supp):5973s-5977s (1995), Couto et al., Cancer Res., 55(8):1717-22 (1995), Sandhu J S, Gene, 150(2):409-10 (1994), and Pedersen et al., J. Mol. Biol., 235(3):959-73 (1994). Often, framework residues in the framework regions will be substituted with the corresponding residue from the CDR donor antibody to alter, for example improve, antigen binding. These framework substitutions are identified by methods well-known in the art, e.g., by modeling of the interactions of the CDR and framework residues to identify framework residues important for antigen binding and sequence comparison to identify unusual framework residues at particular positions. (See, e.g., Queen et al., U.S. Pat. No. 5,585,089; and Riechmann et al., 1988, Nature, 332:323.)

A humanized antibody or antibody fragment has one or more amino acid residues remaining in it from a source which is nonhuman. These nonhuman amino acid residues are often referred to as "import" residues, which are typically taken from an "import" variable domain. As provided herein, humanized antibodies or antibody fragments comprise one or more CDRs from nonhuman immunoglobulin molecules and framework regions wherein the amino acid residues comprising the framework are derived completely or mostly from human germline. Multiple techniques for humanization of antibodies or antibody fragments are well-known in the art and can essentially be performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting rodent CDRs or CDR sequences for the corresponding sequences of a human antibody, i.e., CDR-grafting (EP 239,400; PCT Publication No. WO 91/09967; and U.S. Pat. Nos. 4,816,567; 6,331,415; 5,225,539; 5,530,101; 5,585,089; 6,548,640). In such humanized antibodies and antibody fragments, substantially less than an intact human variable domain has been substituted by the corresponding sequence from a nonhuman species. Humanized antibodies are often human antibodies in which some CDR residues and possibly some framework (FR) residues are substituted by residues from analogous sites in rodent antibodies. Humanization of antibodies and antibody fragments can also be achieved by veneering or resurfacing (EP 592,106; EP 519,596; Padlan, 1991, Molecular Immunology, 28(4/5):489-498; Studnicka et al., Protein Engineering, 7(6):805-814 (1994); and Roguska et al., PNAS, 91:969-973 (1994)) or chain shuffling (U.S. Pat. No. 5,565,332).

The choice of human variable domains, both light and heavy, to be used in making the humanized antibodies is to reduce antigenicity. According to the so-called "bestfit" method, the sequence of the variable domain of a rodent antibody is screened against the entire library of known human variable-domain sequences. The human sequence which is closest to that of the rodent is then accepted as the human framework (FR) for the humanized antibody (Sims et al., J. Immunol., 151:2296 (1993); Chothia et al., J. Mol. Biol., 196:901 (1987)). Another method uses a particular framework derived from the consensus sequence of all human antibodies of a particular subgroup of light or heavy chains. The same framework may be used for several different humanized antibodies (see, e.g., Nicholson et al. Mol. Immun. 34 (16-17): 1157-1165 (1997); Carter et al., Proc. Natl. Acad. Sci. USA, 89:4285 (1992); Presta et al., J. Immunol., 151:2623 (1993)). In some embodiments, the framework region, e.g., all four framework regions, of the heavy chain variable region are derived from a VH4)_4-59 germline sequence. In one embodiment, the framework region can comprise, one, two, three, four or five modifications, e.g., substitutions, e.g., from the amino acid at the corresponding murine sequence. In one embodiment, the framework region, e.g., all four framework regions of the light chain variable region are derived from a VK3_1.25 germline sequence. In one embodiment, the framework region can comprise, one, two, three, four or five modifications, e.g., substitutions, e.g., from the amino acid at the corresponding murine sequence.

In some aspects, the portion of a CAR composition of the invention that comprises an antibody fragment is humanized with retention of high affinity for the target antigen and other favorable biological properties. According to one aspect of the disclosure, humanized antibodies and antibody fragments are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, e.g., the analysis of residues that influence the ability of the candidate immunoglobulin to bind the target antigen. In this way, FR residues can be selected and combined from the recipient and import sequences so that the desired antibody or antibody fragment characteristic, such as increased affinity for the target antigen, is achieved. In general, the CDR residues are directly and most substantially involved in influencing antigen binding.

A humanized antibody or antibody fragment may retain a similar antigenic specificity as the original antibody, e.g., in the present invention, the ability to bind human a cancer associated antigen as described herein. In some embodiments, a humanized antibody or antibody fragment may have improved affinity and/or specificity of binding to human a cancer associated antigen as described herein.

In one aspect, the antigen binding domain employed in the invention is characterized by particular functional features or properties of an antibody or antibody fragment. For example, in one aspect, the portion of a CAR composition of the invention that comprises an antigen binding domain specifically binds a tumor antigen as described herein.

In one aspect, the anti-cancer associated antigen as described herein binding domain is a fragment, e.g., a single chain variable fragment (scFv). In one aspect, the anti-cancer associated antigen as described herein binding domain is a Fv, a Fab, a (Fab')2, or a bi-functional (e.g. bi-specific) hybrid antibody (e.g., Lanzavecchia et al., Eur. J. Immunol. 17, 105 (1987)). In the present invention, the antigen binding domain of the first CAR comprises a VHH domain and the antigen binding domain of the second CAR comprises an scFv. In one aspect, the antibodies and fragments thereof employed in the invention binds a cancer associated antigen as described herein protein with wild-type or enhanced affinity.

In some instances, scFvs can be prepared according to method known in the art (see, for example, Bird et al., (1988) Science 242:423-426 and Huston et al., (1988) Proc. Natl. Acad. Sci. USA 85:5879-5883). ScFv molecules can be produced by linking VH and VL regions together using flexible polypeptide linkers. The scFv molecules comprise a linker (e.g., a Ser-Gly linker) with an optimized length and/or amino acid composition. The linker length can greatly affect how the variable regions of a scFv fold and interact. In fact, if a short polypeptide linker is employed (e.g., between 5-10 amino acids) intrachain folding is prevented. Interchain folding is also required to bring the two variable regions together to form a functional epitope binding site. For examples of linker orientation and size see, e.g., Hollinger et al. 1993 Proc Natl Acad. Sci. U.S.A. 90:6444-6448, U.S. Patent Application Publication Nos. 2005/0100543, 2005/0175606, 2007/0014794, and PCT publication Nos. WO2006/020258 and WO2007/024715.

An scFv can comprise a linker of at least 1, 2, 3, 4, 5, 6, 7, 8, 9, 10, 11, 12, 13, 14, 15, 16, 17, 18, 19, 20, 25, 30, 35, 40, 45, 50, or more amino acid residues between its VL and VH regions. The linker sequence may comprise any naturally occurring amino acid. In some embodiments, the linker sequence comprises amino acids glycine and serine. In another embodiment, the linker sequence comprises sets of glycine and serine repeats such as (Gly₄Ser)n, where n is a positive integer equal to or greater than 1 (SEQ ID NO:22). In one embodiment, the linker can be (Gly₄Ser)₄ (SEQ ID NO:29) or (Gly₄Ser)₃(SEQ ID NO:30). Variation in the linker length may retain or enhance activity, giving rise to superior efficacy in activity studies.

In another instance, the antigen binding domain is a T cell receptor ("TCR"), or a fragment thereof, for example, a single chain TCR (scTCR). Methods to make such TCRs are known in the art. See, e.g., Willemsen RA et al, Gene Therapy 7: 1369-1377 (2000); Zhang T et al, Cancer Gene Ther 11: 487-496 (2004); Aggen et al, Gene Ther. 19(4):365-74 (2012). For example, scTCR can be engineered that contains the Vα and Vβ genes from a T cell clone linked by a linker (e.g., a flexible peptide). This approach is very useful to cancer associated target that itself is intracellar, however, a fragment of such antigen (peptide) is presented on the surface of the cancer cells by MHC.

### Bispecific CARs

In an embodiment a multispecific antibody molecule is a bispecific antibody molecule. A bispecific antibody has specificity for no more than two antigens. A bispecific antibody molecule is characterized by a first immunoglobulin variable domain sequence which has binding specificity for a first epitope and a second immunoglobulin variable domain sequence that has binding specificity for a second epitope. In an embodiment the first and second epitopes are on the same antigen, e.g., the same protein (or subunit of a multimeric protein). In an embodiment the first and second epitopes overlap. In an embodiment the first and second epitopes do not overlap. In an embodiment the first and second epitopes are on different antigens, e.g., different proteins (or different subunits of a multimeric protein). In an embodiment a bispecific antibody molecule comprises a heavy chain variable domain sequence and a light chain variable domain sequence which have binding specificity for a first epitope and a heavy chain variable domain sequence and a light chain variable domain sequence which have binding specificity for a second epitope. In an embodiment a bispecific antibody molecule comprises a half antibody having binding specificity for a first epitope and a half antibody having binding specificity for a second epitope. In an embodiment a bispecific antibody molecule comprises a half antibody, or fragment thereof, having binding specificity for a first epitope and a half antibody, or fragment thereof, having binding specificity for a second epitope. In an embodiment a bispecific antibody molecule comprises a scFv, or fragment thereof, have binding specificity for a first epitope and a scFv, or fragment thereof, have binding specificity for a second epitope.

In certain embodiments, the antibody molecule is a multi-specific (e.g., a bispecific or a trispecific) antibody molecule. Protocols for generating bispecific or heterodimeric antibody molecules are known in the art; including but not limited to, for example, the "knob in a hole" approach described in, *e.g.,* US 5731168; the electrostatic steering Fc pairing as described in, *e.g.,* WO 09/089004, WO 06/106905 and WO 2010/129304; Strand Exchange Engineered Domains (SEED) heterodimer formation as described in, *e.g.,* WO 07/110205; Fab arm exchange as described in, *e.g.,* WO 08/119353, WO 2011/131746, and WO 2013/060867; double antibody conjugate, *e.g.,* by antibody cross-linking to generate a bi-specific structure using a heterobifunctional reagent having an amine-reactive group and a sulfhydryl reactive group as described in, *e.g.,* US 4433059; bispecific antibody determinants generated by recombining half antibodies (heavy-light chain pairs or Fabs) from different antibodies through cycle of reduction and oxidation of disulfide bonds between the two heavy chains, as described in, *e.g.,* US 4444878; trifunctional antibodies, *e.g.,* three Fab' fragments cross-linked through sulfhdryl reactive groups, as described in, *e.g.,* US5273743; biosynthetic binding proteins, *e.g.,* pair of scFvs cross-linked through C-terminal tails preferably through disulfide or amine-reactive chemical cross-linking, as described in, *e.g.,* US5534254; bifunctional antibodies, *e.g.,* Fab fragments with different binding specificities dimerized through leucine zippers *(e.g.,* c-fos and c-jun) that have replaced the constant domain, as described in, *e.g.,* US5582996; bispecific and oligospecific mono-and oligovalent receptors, *e.g.,* VH-CH1 regions of two antibodies (two Fab fragments) linked through a polypeptide spacer between the CH1 region of one antibody and the VH region of the other antibody typically with associated light chains, as described in, *e.g.,* US5591828; bispecific DNA-antibody conjugates, *e.g.,* crosslinking of antibodies or Fab fragments through a double stranded piece of DNA, as described in, *e.g.,* US5635602; bispecific fusion proteins, *e.g.,* an expression construct containing two scFvs with a hydrophilic helical peptide linker between them and a full constant region, as described in, *e.g.,* US5637481; multivalent and multispecific binding proteins, *e.g.,* dimer of polypeptides having first domain with binding region of Ig heavy chain variable region, and second domain with binding region of Ig light chain variable region, generally termed diabodies (higher order structures are also encompassed creating for bispecifc, trispecific, or tetraspecific molecules, as described in, *e.g.,* US5837242; minibody constructs with linked VL and VH chains further connected with peptide spacers to an antibody hinge region and CH3 region, which can be dimerized to form bispecific/multivalent molecules, as described in, *e.g.,* US5837821; VH and VL domains linked with a short peptide linker (*e.g.,* 5 or 10 amino acids) or no linker at all in either orientation, which can form dimers to form bispecific diabodies; trimers and tetramers, as described in, *e.g.,* US5844094; String of VH domains (or VL domains in family members) connected by peptide linkages with crosslinkable groups at the C-terminus futher associated with VL domains to form a series of FVs (or scFvs), as described in, *e.g.,* US5864019; and single chain binding polypeptides with both a VH and a VL domain linked through a peptide linker are combined into multivalent structures through non-covalent or chemical crosslinking to form, *e.g.,* homobivalent, heterobivalent, trivalent, and tetravalent structures using both scFV or diabody type format, as described in, *e.g.,* US5869620. Additional exemplary multispecific and bispecific molecules and methods of making the same are found, for example, in US5910573, US5932448, US5959083, US5989830, US6005079, US6239259, US6294353, US6333396, US6476198, US6511663, US6670453, US6743896, US6809185, US6833441, US7129330, US7183076, US7521056, US7527787, US7534866, US7612181, US2002004587A1, US2002076406A1, US2002103345A1, US2003207346A1, US2003211078A1, US2004219643A1, US2004220388A1, US2004242847A1, US2005003403A1, US2005004352A1, US2005069552A1, US2005079170A1, US2005100543A1, US2005136049A1, US2005136051A1, US2005163782A1, US2005266425A1, US2006083747A1, US2006120960A1, US2006204493A1, US2006263367A1, US2007004909A1, US2007087381A1, US2007128150A1, US2007141049A1, US2007154901A1, US2007274985A1, US2008050370A1, US2008069820A1, US2008152645A1, US2008171855A1, US2008241884A1, US2008254512A1, US2008260738A1, US2009130106A1, US2009148905A1, US2009155275A1, US2009162359A1, US2009162360A1, US2009175851A1, US2009175867A1, US2009232811A1, US2009234105A1, US2009263392A1, US2009274649A1, EP346087A2, WO0006605A2, WO02072635A2, WO04081051A1, WO06020258A2, WO2007044887A2, WO2007095338A2, WO2007137760A2, WO2008119353A1, WO2009021754A2, WO2009068630A1, WO9103493A1, WO9323537A1, WO9409131A1, WO9412625A2, WO9509917A1, WO9637621A2, WO9964460A1.

Within each antibody or antibody fragment (e.g., scFvof a bispecific antibody molecule, the VH can be upstream or downstream of the VL. In some embodiments, the upstream antibody or antibody fragment (e.g., scFv) is arranged with its VH (VHi) upstream of its VL (VLi) and the downstream antibody or antibody fragment (e.g., scFv) is arranged with its VL (VL₂) upstream of its VH (VH₂), such that the overall bispecific antibody molecule has the arrangement VH₁-VL₁-VL₂-VH₂. In other embodiments, the upstream antibody or antibody fragment (e.g., scFv) is arranged with its VL (VLi) upstream of its VH (VHi) and the downstream antibody or antibody fragment (e.g., scFv) is arranged with its VH (VH₂) upstream of its VL (VL₂), such that the overall bispecific antibody molecule has the arrangement VL₁-VH₁-VH₂-VL₂. Optionally, a linker is disposed between the two antibodies or antibody fragments (e.g., scFvs), e.g., between VLi and VL₂ if the construct is arranged as VH₁-VL₁-VL₂-VH₂, or between VHi and VH₂ if the construct is arranged as VL₁-VH₁-VH₂-VL₂. The linker may be a linker as described herein, e.g., a (Gly₄-Ser)n linker, wherein n is 1, 2, 3, 4, 5, or 6, preferably 4 (SEQ ID NO: 64). In general, the linker between the two scFvs should be long enough to avoid mispairing between the domains of the two scFvs. Optionally, a linker is disposed between the VL and VH of the first scFv. Optionally, a linker is disposed between the VL and VH of the second scFv. In constructs that have multiple linkers, any two or more of the linkers can be the same or different. Accordingly, in some embodiments, a bispecific CAR comprises VLs, VHs, and optionally one or more linkers in an arrangement as described herein.

### Stability and Mutations

The stability of an antigen binding domain to a cancer associated antigen as described herein, e.g., scFv molecules (e.g., soluble scFv), can be evaluated in reference to the biophysical properties (e.g., thermal stability) of a conventional control scFv molecule or a full length antibody. In one embodiment, the humanized scFv has a thermal stability that is greater than about 0.1, about 0.25, about 0.5, about 0.75, about 1, about 1.25, about 1.5, about 1.75, about 2, about 2.5, about 3, about 3.5, about 4, about 4.5, about 5, about 5.5, about 6, about 6.5, about 7, about 7.5, about 8, about 8.5, about 9, about 9.5, about 10 degrees, about 11 degrees, about 12 degrees, about 13 degrees, about 14 degrees, or about 15 degrees Celsius than a control binding molecule (e.g. a conventional scFv molecule) in the described assays.

The improved thermal stability of the antigen binding domain to a cancer associated antigen described herein, e.g., scFv is subsequently conferred to the entire CAR construct, leading to improved therapeutic properties of the CAR construct. The thermal stability of the antigen binding domain of a cancer associated antigen described herein, e.g., scFv, can be improved by at least about 2°C or 3°C as compared to a conventional antibody. In one embodiment, the antigen binding domain of a cancer associated antigen described herein, e.g., scFv, has a 1°C improved thermal stability as compared to a conventional antibody. In another embodiment, the antigen binding domain of a cancer associated antigen described herein, e.g., scFv, has a 2°C improved thermal stability as compared to a conventional antibody. In another embodiment, the scFv has a 4, 5, 6, 7, 8, 9, 10, 11, 12, 13, 14, 15°C improved thermal stability as compared to a conventional antibody. Comparisons can be made, for example, between the scFv molecules disclosed herein and scFv molecules or Fab fragments of an antibody from which the scFv VH and VL were derived. Thermal stability can be measured using methods known in the art. For example, in one instance, Tm can be measured. Methods for measuring Tm and other methods of determining protein stability are described in more detail below.

Mutations in scFv (arising through humanization or direct mutagenesis of the soluble scFv) can alter the stability of the scFv and improve the overall stability of the scFv and the CAR construct. Stability of the humanized scFv is compared against the murine scFv using measurements such as Tm, temperature denaturation and temperature aggregation.

The binding capacity of the mutant scFvs can be determined using assays known in the art and described herein.

In one embodiment, the antigen binding domain of a cancer associated antigen described herein, e.g., scFv, comprises at least one mutation arising from the humanization process such that the mutated scFv confers improved stability to the CAR construct. In another embodiment, the antigen binding domain of a cancer associated antigen described herein, e.g., scFv, comprises at least 1, 2, 3, 4, 5, 6, 7, 8, 9, 10 mutations arising from the humanization process such that the mutated scFv confers improved stability to the CAR construct.

### Methods of Evaluating Protein Stability

The stability of an antigen binding domain may be assessed using, e.g., the methods described below. Such methods allow for the determination of multiple thermal unfolding transitions where the least stable domain either unfolds first or limits the overall stability threshold of a multidomain unit that unfolds cooperatively (e.g., a multidomain protein which exhibits a single unfolding transition). The least stable domain can be identified in a number of additional ways. Mutagenesis can be performed to probe which domain limits the overall stability. Additionally, protease resistance of a multidomain protein can be performed under conditions where the least stable domain is known to be intrinsically unfolded via DSC or other spectroscopic methods (Fontana, et al., (1997) Fold. Des., 2: R17-26; Dimasi et al. (2009) J. Mol. Biol. 393: 672-692). Once the least stable domain is identified, the sequence encoding this domain (or a portion thereof) may be employed as a test sequence in the methods.

### a) Thermal Stability

The thermal stability of the compositions may be analyzed using a number of non-limiting biophysical or biochemical techniques known in the art. In certain instances, thermal stability is evaluated by analytical spectroscopy.

An exemplary analytical spectroscopy method is Differential Scanning Calorimetry (DSC). DSC employs a calorimeter which is sensitive to the heat absorbances that accompany the unfolding of most proteins or protein domains (see, e.g. Sanchez-Ruiz, et al., Biochemistry, 27: 1648-52, 1988). To determine the thermal stability of a protein, a sample of the protein is inserted into the calorimeter and the temperature is raised until the Fab or scFv unfolds. The temperature at which the protein unfolds is indicative of overall protein stability.

Another exemplary analytical spectroscopy method is Circular Dichroism (CD) spectroscopy. CD spectrometry measures the optical activity of a composition as a function of increasing temperature. Circular dichroism (CD) spectroscopy measures differences in the absorption of left-handed polarized light versus right-handed polarized light which arise due to structural asymmetry. A disordered or unfolded structure results in a CD spectrum very different from that of an ordered or folded structure. The CD spectrum reflects the sensitivity of the proteins to the denaturing effects of increasing temperature and is therefore indicative of a protein's thermal stability (see van Mierlo and Steemsma, J. Biotechnol., 79(3):281-98, 2000).

Another exemplary analytical spectroscopy method for measuring thermal stability is Fluorescence Emission Spectroscopy (see van Mierlo and Steemsma, supra). Yet another exemplary analytical spectroscopy method for measuring thermal stability is Nuclear Magnetic Resonance (NMR) spectroscopy (see, e.g. van Mierlo and Steemsma, supra).

The thermal stability of a composition can be measured biochemically. An exemplary biochemical method for assessing thermal stability is a thermal challenge assay. In a "thermal challenge assay", a composition is subjected to a range of elevated temperatures for a set period of time. For example, in one instance, test scFv molecules or molecules comprising scFv molecules are subject to a range of increasing temperatures, e.g., for 1-1.5 hours. The activity of the protein is then assayed by a relevant biochemical assay. For example, if the protein is a binding protein (e.g. an scFv or scFv-containing polypeptide) the binding activity of the binding protein may be determined by a functional or quantitative ELISA.

Such an assay may be done in a high-throughput format and those disclosed in the Examples using *E. coli* and high throughput screening. A library of antigen binding domains, e.g., that includes an antigen binding domain to -a cancer associated antigen described herein, e.g., scFv variants, may be created using methods known in the art. Antigen binding domain, e.g., to -a cancer associated antigen described herein, e.g., scFv, expression may be induced and the antigen binding domain, e.g., to -a cancer associated antigen described herein, e.g., scFv, may be subjected to thermal challenge. The challenged test samples may be assayed for binding and those antigen binding domains to -a cancer associated antigen described herein, e.g., scFvs, which are stable may be scaled up and further characterized.

Thermal stability is evaluated by measuring the melting temperature (Tm) of a composition using any of the above techniques (e.g. analytical spectroscopy techniques). The melting temperature is the temperature at the midpoint of a thermal transition curve wherein 50% of molecules of a composition are in a folded state (See e.g., Dimasi et al. (2009) J. Mol Biol. 393: 672-692). In one instance, Tm values for an antigen binding domain to a cancer associated antigen described herein, e.g., scFv, are about 40°C, 41°C, 42°C, 43°C, 44°C, 45°C, 46°C, 47°C, 48°C, 49°C, 50°C, 51°C, 52°C, 53°C, 54°C, 55°C, 56°C, 57°C, 58°C, 59°C, 60°C, 61°C, 62°C, 63°C, 64°C, 65°C, 66°C, 67°C, 68°C, 69°C, 70°C, 71°C, 72°C, 73°C, 74°C, 75°C, 76°C, 77°C, 78°C, 79°C, 80°C, 81°C, 82°C, 83°C, 84°C, 85°C, 86°C, 87°C, 88°C, 89°C, 90°C, 91°C, 92°C, 93°C, 94°C, 95°C, 96°C, 97°C, 98°C, 99°C, 100°C. In one instance, Tm values for an IgG is about 40°C, 41°C, 42°C, 43°C, 44°C, 45°C, 46°C, 47°C, 48°C, 49°C, 50°C, 51°C, 52°C, 53°C, 54°C, 55°C, 56°C, 57°C, 58°C, 59°C, 60°C, 61°C, 62°C, 63°C, 64°C, 65°C, 66°C, 67°C, 68°C, 69°C, 70°C, 71°C, 72°C, 73°C, 74°C, 75°C, 76°C, 77°C, 78°C, 79°C, 80°C, 81°C, 82°C, 83°C, 84°C, 85°C, 86°C, 87°C, 88°C, 89°C, 90°C, 91°C, 92°C, 93°C, 94°C, 95°C, 96°C, 97°C, 98°C, 99°C, 100°C. In one instance, Tm values for an multivalent antibody is about 40°C, 41°C, 42°C, 43°C, 44°C, 45°C, 46°C, 47°C, 48°C, 49°C, 50°C, 51°C, 52°C, 53°C, 54°C, 55°C, 56°C, 57°C, 58°C, 59°C, 60°C, 61°C, 62°C, 63°C, 64°C, 65°C, 66°C, 67°C, 68°C, 69°C, 70°C, 71°C, 72°C, 73°C, 74°C, 75°C, 76°C, 77°C, 78°C, 79°C, 80°C, 81°C, 82°C, 83°C, 84°C, 85°C, 86°C, 87°C, 88°C, 89°C, 90°C, 91°C, 92°C, 93°C, 94°C, 95°C, 96°C, 97°C, 98°C, 99°C, 100°C.

Thermal stability is also evaluated by measuring the specific heat or heat capacity (Cp) of a composition using an analytical calorimetric technique (e.g. DSC). The specific heat of a composition is the energy (e.g. in kcal/mol) is required to rise by 1°C, the temperature of 1 mol of water. As large Cp is a hallmark of a denatured or inactive protein composition. The change in heat capacity (ΔCp) of a composition is measured by determining the specific heat of a composition before and after its thermal transition. Thermal stability may also be evaluated by measuring or determining other parameters of thermodynamic stability including Gibbs free energy of unfolding (ΔG), enthalpy of unfolding (ΔH), or entropy of unfolding (ΔS). One or more of the above biochemical assays (e.g. a thermal challenge assay) are used to determine the temperature (i.e. the Tc value) at which 50% of the composition retains its activity (e.g. binding activity).

In addition, mutations to the antigen binding domain of a cancer associated antigen described herein, e.g., scFv, can be made to alter the thermal stability of the antigen binding domain of a cancer associated antigen described herein, e.g., scFv, as compared with the unmutated antigen binding domain of a cancer associated antigen described herein, e.g., scFv. When the humanized antigen binding domain of a cancer associated antigen described herein, e.g., scFv, is incorporated into a CAR construct, the antigen binding domain of the cancer associated antigen described herein, e.g., humanized scFv, confers thermal stability to the overall CARs used in the present invention. In one embodiment, the antigen binding domain to a cancer associated antigen described herein, e.g., scFv, comprises a single mutation that confers thermal stability to the antigen binding domain of the cancer associated antigen described herein, e.g., scFv. In another embodiment, the antigen binding domain to a cancer associated antigen described herein, e.g., scFv, comprises multiple mutations that confer thermal stability to the antigen binding domain to the cancer associated antigen described herein, e.g., scFv. In one embodiment, the multiple mutations in the antigen binding domain to a cancer associated antigen described herein, e.g., scFv, have an additive effect on thermal stability of the antigen binding domain to the cancer associated antigen described herein binding domain, e.g., scFv.

### b) % Aggregation

The stability of a composition can be determined by measuring its propensity to aggregate. Aggregation can be measured by a number of non-limiting biochemical or biophysical techniques. For example, the aggregation of a composition may be evaluated using chromatography, e.g. Size-Exclusion Chromatography (SEC). SEC separates molecules on the basis of size. A column is filled with semi-solid beads of a polymeric gel that will admit ions and small molecules into their interior but not large ones. When a protein composition is applied to the top of the column, the compact folded proteins (i.e. non-aggregated proteins) are distributed through a larger volume of solvent than is available to the large protein aggregates. Consequently, the large aggregates move more rapidly through the column, and in this way the mixture can be separated or fractionated into its components. Each fraction can be separately quantified (e.g. by light scattering) as it elutes from the gel. Accordingly, the % aggregation of a composition can be determined by comparing the concentration of a fraction with the total concentration of protein applied to the gel. Stable compositions elute from the column as essentially a single fraction and appear as essentially a single peak in the elution profile or chromatogram.

### c) Binding Affinity

The stability of a composition can be assessed by determining its target binding affinity. A wide variety of methods for determining binding affinity are known in the art. An exemplary method for determining binding affinity employs surface plasmon resonance. Surface plasmon resonance is an optical phenomenon that allows for the analysis of realtime biospecific interactions by detection of alterations in protein concentrations within a biosensor matrix, for example using the BIAcore system (Pharmacia Biosensor AB, Uppsala, Sweden and Piscataway, N.J.). For further descriptions, see Jonsson, U., et al. (1993) Ann. Biol. Clin. 51:19-26; Jonsson, U., i (1991) Biotechniques 11:620-627; Johnsson, B., et al. (1995) J. Mol. Recognit. 8:125-131; and Johnnson, B., et al. (1991) Anal. Biochem. 198:268-277.

In one aspect, the antigen binding domain of the CAR comprises an amino acid sequence that is homologous to an antigen binding domain amino acid sequence described herein, and the antigen binding domain retains the desired functional properties of the antigen binding domain described herein.

In one specific aspect, the CAR composition of the disclosure comprises an antibody fragment. In a further aspect, the antibody fragment comprises an scFv.

In various aspects, the antigen binding domain of the CAR is engineered by modifying one or more amino acids within one or both variable regions (e.g., VH and/or VL), for example within one or more CDR regions and/or within one or more framework regions. In one specific aspect, the CAR composition of the disclosure comprises an antibody fragment. In a further aspect, the antibody fragment comprises an scFv.

It will be understood by one of ordinary skill in the art that the antibody or antibody fragment used in the invention may further be modified such that they vary in amino acid sequence (e.g., from wild-type), but not in desired activity. For example, additional nucleotide substitutions leading to amino acid substitutions at "non-essential" amino acid residues may be made to the protein For example, a nonessential amino acid residue in a molecule may be replaced with another amino acid residue from the same side chain family. In another embodiment, a string of amino acids can be replaced with a structurally similar string that differs in order and/or composition of side chain family members, e.g., a conservative substitution, in which an amino acid residue is replaced with an amino acid residue having a similar side chain, may be made.

Families of amino acid residues having similar side chains have been defined in the art, including basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

Percent identity in the context of two or more nucleic acids or polypeptide sequences, refers to two or more sequences that are the same. Two sequences are "substantially identical" if two sequences have a specified percentage of amino acid residues or nucleotides that are the same (e.g., 60% identity, optionally 70%, 71%. 72%. 73%, 74%, 75%, 76%, 77%, 78%, 79%, 80%,81%, 82%, 83%, 84%, 85%, 86%, 87%, 88%, 89%, 90%, 91%, 92%, 93%, 94%, 95%, 96%, 97%, 98%, 99% identity over a specified region, or, when not specified, over the entire sequence), when compared and aligned for maximum correspondence over a comparison window, or designated region as measured using one of the following sequence comparison algorithms or by manual alignment and visual inspection. Optionally, the identity exists over a region that is at least about 50 nucleotides (or 10 amino acids) in length, or more preferably over a region that is 100 to 500 or 1000 or more nucleotides (or 20, 50, 200 or more amino acids) in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters. Methods of alignment of sequences for comparison are well known in the art. Optimal alignment of sequences for comparison can be conducted, e.g., by the local homology algorithm of Smith and Waterman, (1970) Adv. Appl. Math. 2:482c, by the homology alignment algorithm of Needleman and Wunsch, (1970) J. Mol. Biol. 48:443, by the search for similarity method of Pearson and Lipman, (1988) Proc. Nat'l. Acad. Sci. USA 85:2444, by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (see, e.g., Brent et al., (2003) Current Protocols in Molecular Biology).

Two examples of algorithms that are suitable for determining percent sequence identity and sequence similarity are the BLAST and BLAST 2.0 algorithms, which are described in Altschul et al., (1977) Nuc. Acids Res. 25:3389-3402; and Altschul et al., (1990) J. Mol. Biol. 215:403-410, respectively. Software for performing BLAST analyses is publicly available through the National Center for Biotechnology Information.

The percent identity between two amino acid sequences can also be determined using the algorithm of E. Meyers and W. Miller, (1988) Comput. Appl. Biosci. 4:11-17) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (1970) J. Mol. Biol. 48:444-453) algorithm which has been incorporated into the GAP program in the GCG software package (available at www.gcg.com), using either a Blossom 62 matrix or a PAM250 matrix, and a gap weight of 16, 14, 12, 10, 8, 6, or 4 and a length weight of 1, 2, 3, 4, 5, or 6.

In one aspect, the present invention contemplates modifications of the starting antibody or fragment (e.g., scFv) amino acid sequence that generate functionally equivalent molecules. For example, the VH or VL of an antigen binding domain to a cancer associated antigen described herein, e.g., scFv, comprised in the CAR can be modified to retain at least about 70%, 71%. 72%. 73%, 74%, 75%, 76%, 77%, 78%, 79%, 80%,81%, 82%, 83%, 84%, 85%, 86%, 87%, 88%, 89%, 90%, 91%, 92%, 93%, 94%, 95%, 96%, 97%, 98%, 99% identity of the starting VH or VL framework region of the antigen binding domain to the cancer associated antigen described herein, e.g., scFv. The present invention contemplates modifications of the entire CAR construct, e.g., modifications in one or more amino acid sequences of the various domains of the CAR construct in order to generate functionally equivalent molecules. The CAR construct can be modified to retain at least about 70%, 71%. 72%. 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% identity of the starting CAR construct.

### Transmembrane domain

With respect to the transmembrane domain, in various embodiments, a CAR can be designed to comprise a transmembrane domain that is attached to the extracellular domain of the CAR. A transmembrane domain can include one or more additional amino acids adjacent to the transmembrane region, e.g., one or more amino acid associated with the extracellular region of the protein from which the transmembrane was derived (e.g., 1, 2, 3, 4, 5, 6, 7, 8, 9, 10 up to 15 amino acids of the extracellular region) and/or one or more additional amino acids associated with the intracellular region of the protein from which the transmembrane protein is derived (e.g., 1, 2, 3, 4, 5, 6, 7, 8, 9, 10 up to 15 amino acids of the intracellular region). In one aspect, the transmembrane domain is one that is associated with one of the other domains of the CAR e.g., in one embodiment, the transmembrane domain may be from the same protein that the signaling domain, costimulatory domain or the hinge domain is derived from. In another aspect, the transmembrane domain is not derived from the same protein that any other domain of the CAR is derived from. In some instances, the transmembrane domain can be selected or modified by amino acid substitution to avoid binding of such domains to the transmembrane domains of the same or different surface membrane proteins, e.g., to minimize interactions with other members of the receptor complex. In one aspect, the transmembrane domain is capable of homodimerization with another CAR on the cell surface of a CAR-expressing cell. In a different aspect, the amino acid sequence of the transmembrane domain may be modified or substituted so as to minimize interactions with the binding domains of the native binding partner present in the same CAR-expressing cell.

The transmembrane domain may be derived either from a natural or from a recombinant source. Where the source is natural, the domain may be derived from any membrane-bound or transmembrane protein. In one aspect the transmembrane domain is capable of signaling to the intracellular domain(s) whenever the CAR has bound to a target. A transmembrane domain of particular use in this invention may include at least the transmembrane region(s) of e.g., the alpha, beta or zeta chain of the T-cell receptor, CD28, CD27, CD3 epsilon, CD45, CD4, CD5, CD8, CD9, CD16, CD22, CD33, CD37, CD64, CD80, CD86, CD134, CD137, CD154. In some embodiments, a transmembrane domain may include at least the transmembrane region(s) of, e.g., KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), NKp44, NKp30, NKp46, CD160, CD19, IL2R beta, IL2R gamma, IL7R α, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO-3), BLAME (SLAMF8), SELPLG (CD162), LTBR, PAG/Cbp, NKG2D, NKG2C.

In some instances, the transmembrane domain can be attached to the extracellular region of the CAR, e.g., the antigen binding domain of the CAR, via a hinge, e.g., a hinge from a human protein. For example, in one embodiment, the hinge can be a human Ig (immunoglobulin) hinge (e.g., an IgG4 hinge, an IgD hinge), a GS linker (e.g., a GS linker described herein), a KIR2DS2 hinge or a CD8a hinge. In one embodiment, the hinge or spacer comprises (e.g., consists of) the amino acid sequence of SEQ ID NO:4. In one aspect, the transmembrane domain comprises (e.g., consists of) a transmembrane domain of SEQ ID NO: 12.

In one aspect, the hinge or spacer comprises an IgG4 hinge. For example, in one embodiment, the hinge or spacer comprises a hinge of the amino acid sequence ESKYGPPCPPCPAPEFLGGPSVFLFPPKPKDTLMISRTPEVTCVVVDVSQEDPEVQFN WYVDGVEVHNAKTKPREEQFNSTYRVVSVLTVLHQDWLNGKEYKCKVSNKGLPS SIEKTISKAKGQPREPQVYTLPPSQEEMTKNQVSLTCLVKGFYPSDIAVEWESNGQP ENNYKTTPPVLDSDGSFFLYSRLTVDKSRWQEGNVFSCSVMHEALHNHYTQKSLS LSLGKM (SEQ ID NO:6). In some embodiments, the hinge or spacer comprises a hinge encoded by a nucleotide sequence of

GAGAGCAAGTACGGCCCTCCCTGCCCCCCTTGCCCTGCCCCCGAGTTCCTGGGC
GGACCCAGCGTGTTCCTGTTCCCCCCCAAGCCCAAGGACACCCTGATGATCAGC
CGGACCCCCGAGGTGACCTGTGTGGTGGTGGACGTGTCCCAGGAGGACCCCGA
GGTCCAGTTCAACTGGTACGTGGACGGCGTGGAGGTGCACAACGCCAAGACCA
AGCCCCGGGAGGAGCAGTTCAATAGCACCTACCGGGTGGTGTCCGTGCTGACC
GTGCTGCACCAGGACTGGCTGAACGGCAAGGAATACAAGTGTAAGGTGTCCAA
CAAGGGCCTGCCCAGCAGCATCGAGAAAACCATCAGCAAGGCCAAGGGCCAGC
CTCGGGAGCCCCAGGTGTACACCCTGCCCCCTAGCCAAGAGGAGATGACCAAG
AACCAGGTGTCCCTGACCTGCCTGGTGAAGGGCTTCTACCCCAGCGACATCGCC
GTGGAGTGGGAGAGCAACGGCCAGCCCGAGAACAACTACAAGACCACCCCCCC
TGTGCTGGACAGCGACGGCAGCTTCTTCCTGTACAGCCGGCTGACCGTGGACAA
GAGCCGGTGGCAGGAGGGCAACGTCTTTAGCTGCTCCGTGATGCACGAGGCCC
TGCACAACCACTACACCCAGAAGAGCCTGAGCCTGTCCCTGGGCAAGATG (SEQ
ID NO:7).

In one aspect, the hinge or spacer comprises an IgD hinge. For example, in one embodiment, the hinge or spacer comprises a hinge of the amino acid sequence RWPESPKAQASSVPTAQPQAEGSLAKATTAPATTRNTGRGGEEKKKEKEKEEQEE RETKTPECPSHTQPLGVYLLTPAVQDLWLRDKATFTCFVVGSDLKDAHLTWEVAG KVPTGGVEEGLLERHSNGSQSQHSRLTLPRSLWNAGTSVTCTLNHPSLPPQRLMAL REPAAQAPVKLSLNLLASSDPPEAASWLLCEVSGFSPPNILLMWLEDQREVNTSGF APARPPPQPGSTTFWAWSVLRVPAPPSPQPATYTCVVSHEDSRTLLNASRSLEVSYV TDH (SEQ ID NO:8). In some embodiments, the hinge or spacer comprises a hinge encoded by a nucleotide sequence of

AGGTGGCCCGAAAGTCCCAAGGCCCAGGCATCTAGTGTTCCTACTGCACAGCCC
CAGGCAGAAGGCAGCCTAGCCAAAGCTACTACTGCACCTGCCACTACGCGCAA
TACTGGCCGTGGCGGGGAGGAGAAGAAAAAGGAGAAAGAGAAAGAAGAACAG
GAAGAGAGGGAGACCAAGACCCCTGAATGTCCATCCCATACCCAGCCGCTGGG
CGTCTATCTCTTGACTCCCGCAGTACAGGACTTGTGGCTTAGAGATAAGGCCAC
CTTTACATGTTTCGTCGTGGGCTCTGACCTGAAGGATGCCCATTTGACTTGGGA

GGTTGCCGGAAAGGTACCCACAGGGGGGTTGAGGAAGGGTTGCTGGAGCGCC
ATTCCAATGGCTCTCAGAGCCAGCACTCAAGACTCACCCTTCCGAGATCCCTGT
GGAACGCCGGGACCTCTGTCACATGTACTCTAAATCATCCTAGCCTGCCCCCAC
AGCGTCTGATGGCCCTTAGAGAGCCAGCCGCCCAGGCACCAGTTAAGCTTAGCC
TGAATCTGCTCGCCAGTAGTGATCCCCCAGAGGCCGCCAGCTGGCTCTTATGCG
AAGTGTCCGGCTTTAGCCCGCCCAACATCTTGCTCATGTGGCTGGAGGACCAGC
GAGAAGTGAACACCAGCGGCTTCGCTCCAGCCCGGCCCCCACCCCAGCCGGGT
TCTACCACATTCTGGGCCTGGAGTGTCTTAAGGGTCCCAGCACCACCTAGCCCC
CAGCCAGCCACATACACCTGTGTTGTGTCCCATGAAGATAGCAGGACCCTGCTA
AATGCTTCTAGGAGTCTGGAGGTTTCCTACGTGACTGACCATT (SEQ ID NO:9).

In one aspect, the transmembrane domain may be recombinant, in which case it will comprise predominantly hydrophobic residues such as leucine and valine. In one aspect a triplet of phenylalanine, tryptophan and valine can be found at each end of a recombinant transmembrane domain.

Optionally, a short oligo- or polypeptide linker, between 2 and 10 amino acids in length may form the linkage between the transmembrane domain and the cytoplasmic region of the CAR. A glycine-serine doublet provides a particularly suitable linker. For example, in one aspect, the linker comprises the amino acid sequence of GGGGSGGGGS (SEQ ID NO: 10). In some embodiments, the linker is encoded by a nucleotide sequence of GGTGGCGGAGGTTCTGGAGGTGGAGGTTCC (SEQ ID NO:11).

In one aspect, the hinge or spacer comprises a KIR2DS2 hinge.

### Cytoplasmic domain

The cytoplasmic domain or region of the CAR includes an intracellular signaling domain. An intracellular signaling domain is generally responsible for activation of at least one of the normal effector functions of the immune cell in which the CAR has been introduced. The term "effector function" refers to a specialized function of a cell. Effector function of a T cell, for example, may be cytolytic activity or helper activity including the secretion of cytokines. Thus the term "intracellular signaling domain" refers to the portion of a protein which transduces the effector function signal and directs the cell to perform a specialized function. While usually the entire intracellular signaling domain can be employed, in many cases it is not necessary to use the entire chain. To the extent that a truncated portion of the intracellular signaling domain is used, such truncated portion may be used in place of the intact chain as long as it transduces the effector function signal. The term intracellular signaling domain is thus meant to include any truncated portion of the intracellular signaling domain sufficient to transduce the effector function signal.

Examples of intracellular signaling domains for use in the CAR employed in the invention include the cytoplasmic sequences of the T cell receptor (TCR) and co-receptors that act in concert to initiate signal transduction following antigen receptor engagement, as well as any derivative or variant of these sequences and any recombinant sequence that has the same functional capability.

It is known that signals generated through the TCR alone are insufficient for full activation of the T cell and that a secondary and/or costimulatory signal is also required. Thus, T cell activation can be said to be mediated by two distinct classes of cytoplasmic signaling sequences: those that initiate antigen-dependent primary activation through the TCR (primary intracellular signaling domains) and those that act in an antigen-independent manner to provide a secondary or costimulatory signal (secondary cytoplasmic domain, e.g., a costimulatory domain).

A primary signaling domain regulates primary activation of the TCR complex either in a stimulatory way, or in an inhibitory way. Primary intracellular signaling domains that act in a stimulatory manner may contain signaling motifs which are known as immunoreceptor tyrosine-based activation motifs or ITAMs.

Examples of ITAM containing primary intracellular signaling domains that are of particular use in the invention include those of CD3 zeta, common FcR gamma (FCER1G), Fc gamma RIIa, FcR beta (Fc Epsilon R1b), CD3 gamma, CD3 delta, CD3 epsilon, CD79a, CD79b, DAP10, and DAP12. In one embodiment, a CAR employed in the invention comprises an intracellular signaling domain, e.g., a primary signaling domain of CD3 -zeta.

In one embodiment, a primary signaling domain comprises a modified ITAM domain, e.g., a mutated ITAM domain which has altered (e.g., increased or decreased) activity as compared to the native ITAM domain. In one embodiment, a primary signaling domain comprises a modified ITAM-containing primary intracellular signaling domain, e.g., an optimized and/or truncated ITAM-containing primary intracellular signaling domain. In an embodiment, a primary signaling domain comprises one, two, three, four or more ITAM motifs.

The intracellular signalling domain of the CAR can comprise the CD3-zeta signaling domain by itself or it can be combined with any other desired intracellular signaling domain(s) useful in the context of a CAR employed in the invention. For example, the intracellular signaling domain of the CAR can comprise a CD3 zeta chain portion and a costimulatory signaling domain. The costimulatory signaling domain refers to a portion of the CAR comprising the intracellular domain of a costimulatory molecule. A costimulatory molecule is a cell surface molecule other than an antigen receptor or its ligands that is required for an efficient response of lymphocytes to an antigen. Examples of such molecules include CD27, CD28, 4-1BB (CD137), OX40, CD30, CD40, PD-1, ICOS, lymphocyte function-associated antigen-1 (LFA-1), CD2, CD7, LIGHT, NKG2C, B7-H3, and a ligand that specifically binds with CD83, and the like. For example, CD27 costimulation has been demonstrated to enhance expansion, effector function, and survival of human CART cells in vitro and augments human T cell persistence and antitumor activity in vivo (Song et al. Blood. 2012; 119(3):696-706). Further examples of such costimulatory molecules include CDS, ICAM-1, GITR, BAFFR, HVEM (LIGHTR), SLAMF7, NKp80 (KLRF1), NKp44, NKp30, NKp46, CD160, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAM1 (CD226), SLAMF4 (CD244, 2B4), CD84, CD96 (Tactile), NKG2D, CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, and CD19a.

The intracellular signaling sequences within the cytoplasmic portion of the CAR employed in the invention may be linked to each other in a random or specified order. Optionally, a short oligo- or polypeptide linker, for example, between 2 and 10 amino acids (e.g., 2, 3, 4, 5, 6, 7, 8, 9, or 10 amino acids) in length may form the linkage between intracellular signaling sequence. In one embodiment, a glycine-serine doublet can be used as a suitable linker. In one embodiment, a single amino acid, e.g., an alanine, a glycine, can be used as a suitable linker.

In one aspect, the intracellular signaling domain is designed to comprise two or more, e.g., 2, 3, 4, 5, or more, costimulatory signaling domains. In an embodiment, the two or more, e.g., 2, 3, 4, 5, or more, costimulatory signaling domains, are separated by a linker molecule, e.g., a linker molecule described herein. In one embodiment, the intracellular signaling domain comprises two costimulatory signaling domains. In some embodiments, the linker molecule is a glycine residue. In some embodiments, the linker is an alanine residue.

In one aspect, the intracellular signaling domain is designed to comprise the signaling domain of CD3-zeta and the signaling domain of CD28. In one aspect, the intracellular signaling domain is designed to comprise the signaling domain of CD3-zeta and the signaling domain of 4-1BB. In one aspect, the signaling domain of 4-1BB is a signaling domain of SEQ ID NO: 14. In one aspect, the signaling domain of CD3-zeta is a signaling domain of SEQ ID NO: 18.

In one aspect, the intracellular signaling domain is designed to comprise the signaling domain of CD3-zeta and the signaling domain of CD27. In one aspect, the signaling domain of CD27 comprises an amino acid sequence of QRRKYRSNKGESPVEPAEPCRYSCPREEEGSTIPIQEDYRKPEPACSP (SEQ ID NO: 16). In one aspect, the signalling domain of CD27 is encoded by a nucleic acid sequence of AGGAGTAAGAGGAGCAGGCTCCTGCACAGTGACTACATGAACATGACTCCCCG CCGCCCCGGGCCCACCCGCAAGCATTACCAGCCCTATGCCCCACCACGCGACTT CGCAGCCTATCGCTCC (SEQ ID NO:17).

In the present invention, the CAR-expressing cell comprises a second CAR that includes a different antigen binding domain, e.g., to the same target or a different target (e.g., a target other than a cancer associated antigen described herein or a different cancer associated antigen described herein). In one embodiment, the second CAR includes an antigen binding domain to a target expressed the same cancer cell type as the cancer associated antigen. In one embodiment, the CAR-expressing cell comprises a first CAR that targets a first antigen and includes an intracellular signaling domain having a costimulatory signaling domain but not a primary signaling domain, and a second CAR that targets a second, different, antigen and includes an intracellular signaling domain having a primary signaling domain but not a costimulatory signaling domain. While not wishing to be bound by theory, placement of a costimulatory signaling domain, e.g., 4-1BB, CD28, CD27 or OX-40, onto the first CAR, and the primary signaling domain, e.g., CD3 zeta, on the second CAR can limit the CAR activity to cells where both targets are expressed. In one embodiment, the CAR expressing cell comprises a first cancer associated antigen CAR that includes an antigen binding domain that binds a target antigen described herein, a transmembrane domain and a costimulatory domain and a second CAR that targets a different target antigen (e.g., an antigen expressed on that same cancer cell type as the first target antigen) and includes an antigen binding domain, a transmembrane domain and a primary signaling domain. In another embodiment, the CAR expressing cell comprises a first CAR that includes an antigen binding domain that binds a target antigen described herein, a transmembrane domain and a primary signaling domain and a second CAR that targets an antigen other than the first target antigen (e.g., an antigen expressed on the same cancer cell type as the first target antigen) and includes an antigen binding domain to the antigen, a transmembrane domain and a costimulatory signaling domain.

In one embodiment, the CAR-expressing cell comprises an XCAR described herein and an inhibitory CAR. In one embodiment, the inhibitory CAR comprises an antigen binding domain that binds an antigen found on normal cells but not cancer cells, e.g., normal cells that also express CLL. In one embodiment, the inhibitory CAR comprises the antigen binding domain, a transmembrane domain and an intracellular domain of an inhibitory molecule. For example, the intracellular domain of the inhibitory CAR can be an intracellular domain of PD1, PD-L1, CTLA4, TIM3, CEACAM (e.g., CEACAM-1, CEACAM-3 and/or CEACAM-5), LAG3, VISTA, BTLA, TIGIT, LAIR1, CD160, 2B4 or TGFR beta.

In the present invention, the CAR-expressing cell comprises two or more different CARs and the antigen binding domains of the different CARs can be such that the antigen binding domains do not interact with one another. For example, a cell expressing a first and second CAR can have an antigen binding domain of the second CAR as an scFv that does not form an association with the antigen binding domain of the first CAR that is a VHH

In some instances, the antigen binding domain comprises a single domain antigen binding (SDAB) molecules include molecules whose complementary determining regions are part of a single domain polypeptide. Examples include, but are not limited to, heavy chain variable domains, binding molecules naturally devoid of light chains, single domains derived from conventional 4-chain antibodies, engineered domains and single domain scaffolds other than those derived from antibodies. SDAB molecules may be any of the art, or any future single domain molecules. SDAB molecules may be derived from any species including, but not limited to mouse, human, camel, llama, lamprey, fish, shark, goat, rabbit, and bovine. This term also includes naturally occurring single domain antibody molecules from species other than Camelidae and sharks.

In one instance, an SDAB molecule can be derived from a variable region of the immunoglobulin found in fish, such as, for example, that which is derived from the immunoglobulin isotype known as Novel Antigen Receptor (NAR) found in the serum of shark. Methods of producing single domain molecules derived from a variable region of NAR ("IgNARs") are described in WO 03/014161 and Streltsov (2005) Protein Sci. 14:2901-2909.

According to another aspect, an SDAB molecule is a naturally occurring single domain antigen binding molecule known as heavy chain devoid of light chains. Such single domain molecules are disclosed in WO 9404678 and Hamers-Casterman, C. et al. (1993) Nature 363:446-448, for example. For clarity reasons, this variable domain derived from a heavy chain molecule naturally devoid of light chain is known herein as a VHH or nanobody to distinguish it from the conventional VH of four chain immunoglobulins. Such a VHH molecule can be derived from Camelidae species, for example in camel, llama, dromedary, alpaca and guanaco. Other species besides Camelidae may produce heavy chain molecules naturally devoid of light chain; such VHHs are within the scope of the invention.

The SDAB molecules can be recombinant, CDR-grafted, humanized, camelized, de-immunized and/or in vitro generated (e.g., selected by phage display).

It has also been discovered, that cells having a plurality of chimeric membrane embedded receptors comprising an antigen binding domain that interactions between the antigen binding domain of the receptors can be undesirable, e.g., because it inhibits the ability of one or more of the antigen binding domains to bind its cognate antigen. Accordingly, disclosed herein are cells having a first and a second non-naturally occurring chimeric membrane embedded receptor comprising antigen binding domains that minimize such interactions. Also disclosed herein are nucleic acids encoding a first and a second non-naturally occurring chimeric membrane embedded receptor comprising a antigen binding domains that minimize such interactions, as well as methods of making and using such cells and nucleic acids. In an instance the antigen binding domain of one of said first said second non-naturally occurring chimeric membrane embedded receptor, comprises an scFv, and the other comprises a single VH domain, e.g., a camelid, shark, or lamprey single VH domain, or a single VH domain derived from a human or mouse sequence.

In some instances, the disclosure comprises a first and second CAR, wherein the antigen binding domain of one of said first CAR said second CAR does not comprise a variable light domain and a variable heavy domain. In some instances, the antigen binding domain of one of said first CAR said second CAR is an scFv, and the other is not an scFv. In some instances, the antigen binding domain of one of said first CAR said second CAR comprises a single VH domain, e.g., a camelid, shark, or lamprey single VH domain, or a single VH domain derived from a human or mouse sequence. In some instances, the antigen binding domain of one of said first CAR said second CAR comprises a nanobody. In the present invention, the antigen binding domain of the first CAR comprises a VHH domain and the antigen binding domain of the second CAR comprises an scFv. In some embodiments, the antigen binding domain of the first CAR comprises a camelid VHH domain.

In some instances, the antigen binding domain of one of said first CAR said second CAR comprises an scFv, and the other comprises a single VH domain, e.g., a camelid, shark, or lamprey single VH domain, or a single VH domain derived from a human or mouse sequence. In some instances, the antigen binding domain of one of said first CAR said second CAR comprises an scFv, and the other comprises a nanobody. In some embodiments, the antigen binding domain of the second of said first CAR and said second CAR comprises comprises an scFv, and the other comprises a camelid VHH domain.

In some embodiments, when present on the surface of a cell, binding of the antigen binding domain of said first CAR to its cognate antigen is not substantially reduced by the presence of said second CAR. In some embodiments, binding of the antigen binding domain of said first CAR to its cognate antigen in the presence of said second CAR is 85%, 90%, 95%, 96%, 97%, 98% or 99% of binding of the antigen binding domain of said first CAR to its cognate antigen in the absence of said second CAR

In some embodiments, when present on the surface of a cell, the antigen binding domains of said first CAR said second CAR, associate with one another less than if both were scFv antigen binding domains. In some embodiments, the antigen binding domains of said first CAR said second CAR, associate with one another 85%, 90%, 95%, 96%, 97%, 98% or 99% less than if both were scFv antigen binding domains.

In another aspect, the CAR-expressing cell described herein can further express another agent, e.g., an agent which enhances the activity of a CAR-expressing cell. For example, in one embodiment, the agent can be an agent which inhibits an inhibitory molecule. Inhibitory molecules, e.g., PD1, can, in some embodiments, decrease the ability of a CAR-expressing cell to mount an immune effector response. Examples of inhibitory molecules include PD1, PD-L1, CTLA4, TIM3, CEACAM (e.g., CEACAM-1, CEACAM-3 and/or CEACAM-5), LAG3, VISTA, BTLA, TIGIT, LAIR1, CD160, 2B4 and TGFR beta. In one embodiment, the agent which inhibits an inhibitory molecule, e.g., is a molecule described herein, e.g., an agent that comprises a first polypeptide, e.g., an inhibitory molecule, associated with a second polypeptide that provides a positive signal to the cell, e.g., an intracellular signaling domain described herein. In one embodiment, the agent comprises a first polypeptide, e.g., of an inhibitory molecule such as PD1, PD-L1, CTLA4, TIM3, CEACAM (e.g., CEACAM-1, CEACAM-3 and/or CEACAM-5), LAG3, VISTA, BTLA, TIGIT, LAIR1, CD160, 2B4 or TGFR beta, or a fragment of any of these (e.g., at least a portion of an extracellular domain of any of these), and a second polypeptide which is an intracellular signaling domain described herein (e.g., comprising a costimulatory domain (e.g., 41BB, CD27 or CD28, e.g., as described herein) and/or a primary signaling domain (e.g., a CD3 zeta signaling domain described herein). In one embodiment, the agent comprises a first polypeptide of PD1 or a fragment thereof (e.g., at least a portion of an extracellular domain of PD1), and a second polypeptide of an intracellular signaling domain described herein (e.g., a CD28 signaling domain described herein and/or a CD3 zeta signaling domain described herein). PD1 is an inhibitory member of the CD28 family of receptors that also includes CD28, CTLA-4, ICOS, and BTLA. PD-1 is expressed on activated B cells, T cells and myeloid cells (Agata et al. 1996 Int. Immunol 8:765-75). Two ligands for PD1, PD-L1 and PD-L2 have been shown to downregulate T cell activation upon binding to PD1 (Freeman et a. 2000 J Exp Med 192:1027-34; Latchman et al. 2001 Nat Immunol 2:261-8; Carter et al. 2002 Eur J Immunol 32:634-43). PD-L1 is abundant in human cancers (Dong et al. 2003 J Mol Med 81:281-7; Blank et al. 2005 Cancer Immunol. Immunother 54:307-314; Konishi et al. 2004 Clin Cancer Res 10:5094). Immune suppression can be reversed by inhibiting the local interaction of PD1 with PD-L1.

In one embodiment, the agent comprises the extracellular domain (ECD) of an inhibitory molecule, e.g., Programmed Death 1 (PD1), fused to a transmembrane domain and intracellular signaling domains such as 41BB and CD3 zeta (also referred to herein as a PD1 CAR). In one embodiment, the PD1 CAR, when used in combinations with a XCAR described herein, improves the persistence of the T cell. In one embodiment, the CAR is a PD1 CAR comprising the extracellular domain of PD1 indicated as underlined in SEQ ID NO: 26. In one embodiment, the PD1 CAR comprises the amino acid sequence of SEQ ID NO:26.

Malpvtalllplalllhaarppgwfldspdrpwnpptfspallvvtegdnatftcsfsntsesfvlnwyrmspsn
qtdklaafpedrsqpgqdcrfrvtqlpngrdfhmsvvrarrndsgtylcgaislapkaqikeslraelrvterraevptahpspspr
pagqfqtlvtttpaprpptpaptiasqplslrpeacrpaaggavhtrgldfacdiyiwaplagtcgvlllslvitlyckrgrkkllyifk
qpfmrpvqttqeedgcscrfpeeeeggcelrvkfsrsadapaykqgqnqlynelnlgrreeydvldkrrgrdpemggkprrkn
pqeglynelqkdkmaeayseigmkgerrrgkghdglyqglstatkdtydalhmqalppr (SEQ ID NO:26).

In one embodiment, the PD1 CAR comprises the amino acid sequence provided below (SEQ ID NO:39).

pgwfldspdrpwnpptfspallvvtegdnatftcsfsntsesfvlnwyrmspsnqtdklaafpedrsqpgqdcr
frvtqlpngrdfhmsvvrarrndsgtylcgaislapkaqikeslraelrvterraevptahpspsprpagqfqtlvtttpaprpptpa
ptiasqplslrpeacrpaaggavhtrgldfacdiyiwaplagtcgvlllslvitlyckrgrkkllyifkqpfmrpvqttqeedgcscrf
peeeeggcelrvkfsrsadapaykqgqnqlynelnlgrreeydvldkrrgrdpemggkprrknpqeglynelqkdkmaeays
eigmkgerrrgkghdglyqglstatkdtydalhmqalppr (SEQ ID NO:39).

In one embodiment, the agent comprises a nucleic acid sequence encoding the PD1 CAR, e.g., the PD1 CAR described herein. In one embodiment, the nucleic acid sequence for the PD1 CAR is shown below, with the PD1 ECD underlined below in SEQ ID NO: 27

atggccctccctgtcactgccctgcttctccccctcgcactcctgctccacgccgctagaccacccggatggtttctg
gactctccggatcgcccgtggaatcccccaaccttctcaccggcactcttggttgtgactgagggcgataatgcgaccttcacgtgct
cgttctccaacacctccgaatcattcgtgctgaactggtaccgcatgagcccgtcaaaccagaccgacaagctcgccgcgtttccgg
aagatcggtcgcaaccgggacaggattgtcggttccgcgtgactcaactgccgaatggcagagacttccacatgagcgtggtccg
cgctaggcgaaacgactccgggacctacctgtgcggagccatctcgctggcgcctaaggcccaaatcaaagagagcttgagggc
cgaactgagagtgaccgagcgcagagctgaggtgccaactgcacatccatccccatcgcctcggcctgcggggcagtttcagacc
ctggtcacgaccactccggcgccgcgcccaccgactccggccccaactatcgcgagccagcccctgtcgctgaggccggaagc
atgccgccctgccgccggaggtgctgtgcatacccggggattggacttcgcatgcgacatctacatttgggctcctctcgccggaac
ttgtggcgtgctccttctgtccctggtcatcaccctgtactgcaagcggggtcggaaaaagcttctgtacattttcaagcagcccttcat
gaggcccgtgcaaaccacccaggaggaggacggttgctcctgccggttccccgaagaggaagaaggaggttgcgagctgcgcg
tgaagttctcccggagcgccgacgcccccgcctataagcagggccagaaccagctgtacaacgaactgaacctgggacggcgg
gaagagtacgatgtgctggacaagcggcgcggccgggaccccgaaatgggcgggaagcctagaagaaagaaccctcaggaag

gcctgtataacgagctgcagaaggacaagatggccgaggcctactccgaaattgggatgaagggagagcggcggaggggaaa
ggggcacgacggcctgtaccaaggactgtccaccgccaccaaggacacatacgatgccctgcacatgcaggcccttccccctcg
c (SEQ ID NO: 27).

In another aspect, the present invention provides a population of CAR-expressing cells, e.g., CART cells in accordance with the claims. In some instances, the population of CAR-expressing cells comprises a mixture of cells expressing different CARs. For example, in one instance, the population of CART cells can include a first cell expressing a CAR having an antigen binding domain to a cancer associated antigen described herein, and a second cell expressing a CAR having a different antigen binding domain, e.g., an antigen binding domain to a different a cancer associated antigen described herein, e.g., an antigen binding domain to a cancer associated antigen described herein that differs from the cancer associated antigen bound by the antigen binding domain of the CAR expressed by the first cell. As another example, the population of CAR-expressing cells can include a first cell expressing a CAR that includes an antigen binding domain to a cancer associated antigen described herein, and a second cell expressing a CAR that includes an antigen binding domain to a target other than a cancer associated antigen as described herein. In one instance, the population of CAR-expressing cells includes, e.g., a first cell expressing a CAR that includes a primary intracellular signaling domain, and a second cell expressing a CAR that includes a secondary signaling domain.

In another aspect, the present invention provides a population of cells in accordance with the claims wherein at least one cell in the population expresses a CAR having an antigen binding domain to a cancer associated antigen described herein, and a second cell expressing another agent, e.g., an agent which enhances the activity of a CAR-expressing cell. For example, in one embodiment, the agent can be an agent which inhibits an inhibitory molecule. Inhibitory molecules, e.g., PD-1, can, in some embodiments, decrease the ability of a CAR-expressing cell to mount an immune effector response. Examples of inhibitory molecules include PD-1, PD-L1, CTLA4, TIM3, CEACAM (e.g., CEACAM-1, CEACAM-3 and/or CEACAM-5), LAG3, VISTA, BTLA, TIGIT, LAIR1, CD160, 2B4 and TGFR beta. In one embodiment, the agent which inhibits an inhibitory molecule, e.g., is a molecule described herein, e.g., an agent that comprises a first polypeptide, e.g., an inhibitory molecule, associated with a second polypeptide that provides a positive signal to the cell, e.g., an intracellular signaling domain described herein. In one embodiment, the agent comprises a first polypeptide, e.g., of an inhibitory molecule such as PD-1, PD-L1, CTLA4, TIM3, CEACAM (e.g., CEACAM-1, CEACAM-3 and/or CEACAM-5), LAG3, VISTA, BTLA, TIGIT, LAIR1, CD160, 2B4 or TGFR beta, or a fragment of any of these, and a second polypeptide which is an intracellular signaling domain described herein (e.g., comprising a costimulatory domain (e.g., 41BB, CD27, OX40 or CD28, e.g., as described herein) and/or a primary signaling domain (e.g., a CD3 zeta signaling domain described herein). In one embodiment, the agent comprises a first polypeptide of PD-1 or a fragment thereof, and a second polypeptide of an intracellular signaling domain described herein (e.g., a CD28 signaling domain described herein and/or a CD3 zeta signaling domain described herein).

In one aspect, the present disclosure provides methods comprising administering a population of CAR-expressing cells, e.g., CART cells, e.g., a mixture of cells expressing different CARs, in combination with another agent, e.g., a kinase inhibitor, such as a kinase inhibitor described herein. In another aspect, the present disclosure provides methods comprising administering a population of cells wherein at least one cell in the population expresses a CAR having an antigen binding domain of a cancer associated antigen described herein, and a second cell expressing another agent, e.g., an agent which enhances the activity of a CAR-expressing cell, in combination with another agent, e.g., a kinase inhibitor, such as a kinase inhibitor described herein.

### Regulatable Chimeric Antigen Receptors

In some embodiments, a regulatable CAR (RCAR) where the CAR activity can be controlled is desirable to optimize the safety and efficacy of a CAR therapy. There are many ways CAR activities can be regulated. For example, inducible apoptosis using, e.g., a caspase fused to a dimerization domain (see, e.g., Di et al., N Egnl. J. Med. 2011 Nov. 3; 365(18):1673-1683), can be used as a safety switch in the CAR therapy of the instant invention. In an aspect, a RCAR comprises a set of polypeptides, typically two in the simplest embodiments, in which the components of a standard CAR described herein, e.g., an antigen binding domain and an intracellular signaling domain, are partitioned on separate polypeptides or members. In some embodiments, the set of polypeptides include a dimerization switch that, upon the presence of a dimerization molecule, can couple the polypeptides to one another, e.g., can couple an antigen binding domain to an intracellular signaling domain.

In an aspect, an RCAR comprises two polypeptides or members: 1) an intracellular signaling member comprising an intracellular signaling domain, e.g., a primary intracellular signaling domain described herein, and a first switch domain; 2) an antigen binding member comprising an antigen binding domain, e.g., that targets a tumor antigen described herein, as described herein and a second switch domain. Optionally, the RCAR comprises a transmembrane domain described herein. In an embodiment, a transmembrane domain can be disposed on the intracellular signaling member, on the antigen binding member, or on both. (Unless otherwise indicated, when members or elements of an RCAR are described herein, the order can be as provided, but other orders are included as well. In other words, in an embodiment, the order is as set out in the text, but in other embodiments, the order can be different. E.g., the order of elements on one side of a transmembrane region can be different from the example, e.g., the placement of a switch domain relative to a intracellular signaling domain can be different, e.g., reversed).

In an embodiment, the first and second switch domains can form an intracellular or an extracellular dimerization switch. In an embodiment, the dimerization switch can be a homodimerization switch, e.g., where the first and second switch domain are the same, or a heterodimerization switch, e.g., where the first and second switch domain are different from one another.

In embodiments, an RCAR can comprise a "multi switch." A multi switch can comprise heterodimerization switch domains or homodimerization switch domains. A multi switch comprises a plurality of, e.g., 2, 3, 4, 5, 6, 7, 8, 9, or 10, switch domains, independently, on a first member, e.g., an antigen binding member, and a second member, e.g., an intracellular signaling member. In an embodiment, the first member can comprise a plurality of first switch domains, e.g., FKBP-based switch domains, and the second member can comprise a plurality of second switch domains, e.g., FRB-based switch domains. In an embodiment, the first member can comprise a first and a second switch domain, e.g., a FKBP-based switch domain and a FRB-based switch domain, and the second member can comprise a first and a second switch domain, e.g., a FKBP-based switch domain and a FRB-based switch domain.

In an embodiment, the intracellular signaling member comprises one or more intracellular signaling domains, e.g., a primary intracellular signaling domain and one or more costimulatory signaling domains.

In an embodiment, the antigen binding member may comprise one or more intracellular signaling domains, e.g., one or more costimulatory signaling domains. In an embodiment, the antigen binding member comprises a plurality, e.g., 2 or 3 costimulatory signaling domains described herein, e.g., selected from 41BB, CD28, CD27, ICOS, and OX40, and in embodiments, no primary intracellular signaling domain. In an embodiment, the antigen binding member comprises the following costimulatory signaling domains, from the extracellular to intracellular direction: 41BB-CD27; 41BB-CD27; CD27-41BB; 41BB-CD28; CD28-41BB; OX40-CD28; CD28-OX40; CD28-41BB; or 41BB-CD28. In such embodiments, the intracellular binding member comprises a CD3zeta domain. In one such embodiment the RCAR comprises (1) an antigen binding member comprising, an antigen binding domain, a transmembrane domain, and two costimulatory domains and a first switch domain; and (2) an intracellular signaling domain comprising a transmembrane domain or membrane tethering domain and at least one primary intracellular signaling domain, and a second switch domain.

An embodiment provides RCARs wherein the antigen binding member is not tethered to the surface of the CAR cell. This allows a cell having an intracellular signaling member to be conveniently paired with one or more antigen binding domains, without transforming the cell with a sequence that encodes the antigen binding member. In such embodiments, the RCAR comprises: 1) an intracellular signaling member comprising: a first switch domain, a transmembrane domain, an intracellular signaling domain, e.g., a primary intracellular signaling domain, and a first switch domain; and 2) an antigen binding member comprising: an antigen binding domain, and a second switch domain, wherein the antigen binding member does not comprise a transmembrane domain or membrane tethering domain, and, optionally, does not comprise an intracellular signaling domain. In some embodiments, the RCAR may further comprise 3) a second antigen binding member comprising: a second antigen binding domain, e.g., a second antigen binding domain that binds a different antigen than is bound by the antigen binding domain; and a second switch domain.

Also provided herein are RCARs wherein the antigen binding member comprises bispecific activation and targeting capacity. In this embodiment, the antigen binding member can comprise a plurality, e.g., 2, 3, 4, or 5 antigen binding domains, e.g., scFvs, wherein each antigen binding domain binds to a target antigen, e.g. different antigens or the same antigen, e.g., the same or different epitopes on the same antigen. In an embodiment, the plurality of antigen binding domains are in tandem, and optionally, a linker or hinge region is disposed between each of the antigen binding domains. Suitable linkers and hinge regions are described herein.

An embodiment provides RCARs having a configuration that allows switching of proliferation. In this embodiment, the RCAR comprises: 1) an intracellular signaling member comprising: optionally, a transmembrane domain or membrane tethering domain; one or more co-stimulatory signaling domain, e.g., selected from 41BB, CD28, CD27, ICOS, and OX40, and a switch domain; and 2) an antigen binding member comprising: an antigen binding domain, a transmembrane domain, and a primary intracellular signaling domain, e.g., a CD3zeta domain, wherein the antigen binding member does not comprise a switch domain, or does not comprise a switch domain that dimerizes with a switch domain on the intracellular signaling member. In an embodiment, the antigen binding member does not comprise a co-stimulatory signaling domain. In an embodiment, the intracellular signaling member comprises a switch domain from a homodimerization switch. In an embodiment, the intracellular signaling member comprises a first switch domain of a heterodimerization switch and the RCAR comprises a second intracellular signaling member which comprises a second switch domain of the heterodimerization switch. In such embodiments, the second intracellular signaling member comprises the same intracellular signaling domains as the intracellular signaling member. In an embodiment, the dimerization switch is intracellular. In an embodiment, the dimerization switch is extracellular.

In any of the RCAR configurations described here, the first and second switch domains comprise a FKBP-FRB based switch as described herein.

Also provided herein are cells comprising an RCAR described herein. Any cell that is engineered to express a RCAR can be used as a RCARX cell. In an embodiment the RCARX cell is a T cell, and is referred to as a RCART cell. In an embodiment the RCARX cell is an NK cell, and is referred to as a RCARN cell.

Also provided herein are nucleic acids and vectors comprising RCAR encoding sequences. Sequence encoding various elements of an RCAR can be disposed on the same nucleic acid molecule, e.g., the same plasmid or vector, e.g., viral vector, e.g., lentiviral vector. In an embodiment, (i) sequence encoding an antigen binding member and (ii) sequence encoding an intracellular signaling member, can be present on the same nucleic acid, e.g., vector. Production of the corresponding proteins can be achieved, e.g., by the use of separate promoters, or by the use of a bicistronic transcription product (which can result in the production of two proteins by cleavage of a single translation product or by the translation of two separate protein products). In an embodiment, a sequence encoding a cleavable peptide, e.g., a P2A or F2A sequence, is disposed between (i) and (ii). In an embodiment, a sequence encoding an IRES, e.g., an EMCV or EV71 IRES, is disposed between (i) and (ii). In these embodiments, (i) and (ii) are transcribed as a single RNA. In an embodiment, a first promoter is operably linked to (i) and a second promoter is operably linked to (ii), such that (i) and (ii) are transcribed as separate mRNAs.

Alternatively, the sequence encoding various elements of an RCAR can be disposed on the different nucleic acid molecules, e.g., different plasmids or vectors, e.g., viral vector, e.g., lentiviral vector. E.g., the (i) sequence encoding an antigen binding member can be present on a first nucleic acid, e.g., a first vector, and the (ii) sequence encoding an intracellular signaling member can be present on the second nucleic acid, e.g., the second vector.

### Dimerization switches

Dimerization switches can be non-covalent or covalent. In a non-covalent dimerization switch, the dimerization molecule promotes a non-covalent interaction between the switch domains. In a covalent dimerization switch, the dimerization molecule promotes a covalent interaction between the switch domains.

In an embodiment, the RCAR comprises a FKBP/FRAP, or FKBP/FRB,-based dimerization switch. FKBP12 (FKBP, or FK506 binding protein) is an abundant cytoplasmic protein that serves as the initial intracellular target for the natural product immunosuppressive drug, rapamycin. Rapamycin binds to FKBP and to the large PI3K homolog FRAP (RAFT, mTOR). FRB is a 93 amino acid portion of FRAP, that is sufficient for binding the FKBP-rapamycin complex (Chen, J., Zheng, X. F., Brown, E. J. & Schreiber, S. L. (1995) Identification of an 11-kDa FKBP12-rapamycin-binding domain within the 289-kDa FKBP12-rapamycin-associated protein and characterization of a critical serine residue. Proc Natl Acad Sci USA 92: 4947-51.)

In embodiments, an FKBP/FRAP, e.g., an FKBP/FRB, based switch can use a dimerization molecule, e.g., rapamycin or a rapamycin analog.

The amino acid sequence of FKBP is as follows:

D V P D Y A S L G G P S S P K K K R K V S R G V Q V E T I S P G D G R T F P K R G
Q T C V V H Y T G M L E D G K K F D S S R D R N K P F K F M L G K Q E V I R G W

E E G V A Q M S V G Q R A K L T I S P D Y A Y G A T G H P G I I P P H A T L V F D
V E L L K L E T S Y (SEQ ID NO: 54)

In embodiments, an FKBP switch domain can comprise a fragment of FKBP having the ability to bind with FRB, or a fragment or analog thereof, in the presence of rapamycin or a rapalog, e.g., the underlined portion of SEQ ID NO: 54, which is:

V Q V E T I S P G D G R T F P K R G Q T C V V H Y T G M L E D G K K F D S S R D
R N K P F K F M L G K Q E V I R G W E E G V A Q M S V G Q R A K L T I S P D Y A
Y G A T G H P G I I P P H A T L V F D V E L L K L E T S (SEQ ID NO:55)

The amino acid sequence of FRB is as follows:


 (SEQ ID NO:
56)

"FKBP/FRAP, e.g., an FKBP/FRB, based switch" as that term is used herein, refers to a dimerization switch comprising: a first switch domain, which comprises an FKBP fragment or analog thereof having the ability to bind with FRB, or a fragment or analog thereof, in the presence of rapamycin or a rapalog, e.g., RAD001, and has at least 70, 75, 80, 85, 90, 95, 96, 97, 98, or 99% identity with, or differs by no more than 30, 25, 20, 15, 10, 5, 4, 3, 2, or 1 amino acid residues from, the FKBP sequence of SEQ ID NO: 54 or 55; and a second switch domain, which comprises an FRB fragment or analog thereof having the ability to bind with FRB, or a fragment or analog thereof, in the presence of rapamycin or a rapalog, and has at least 70, 75, 80, 85, 90, 95, 96, 97, 98, or 99% identity with, or differs by no more than 30, 25, 20, 15, 10, 5, 4, 3, 2, or 1 amino acid residues from, the FRB sequence of SEQ ID NO: 56. In an embodiment, a RCAR described herein comprises one switch domain comprises amino acid residues disclosed in SEQ ID NO: 54 (or SEQ ID NO: 55), and one switch domain comprises amino acid residues disclosed in SEQ ID NO: 56.

In embodiments, the FKBP/FRB dimerization switch comprises a modified FRB switch domain that exhibits altered, e.g., enhanced, complex formation between an FRB-based switch domain, e.g., the modified FRB switch domain, a FKBP-based switch domain, and the dimerization molecule, e.g., rapamycin or a rapalogue, e.g., RAD001. In an embodiment, the modified FRB switch domain comprises one or more mutations, e.g., 2, 3, 4, 5, 6, 7, 8, 9, 10 or more, selected from mutations at amino acid position(s) L2031, E2032, S2035, R2036, F2039, G2040, T2098, W2101, D2102, Y2105, and F2108, where the wild-type amino acid is mutated to any other naturally-occurring amino acid. In an embodiment, a mutant FRB comprises a mutation at E2032, where E2032 is mutated to phenylalanine (E2032F), methionine (E2032M), arginine (E2032R), valine (E2032V), tyrosine (E2032Y), isoleucine (E2032I), e.g., SEQ ID NO: 57, or leucine (E2032L), e.g., SEQ ID NO: 58. In an embodiment, a mutant FRB comprises a mutation at T2098, where T2098 is mutated to phenylalanine (T2098F) or leucine (T2098L), e.g., SEQ ID NO: 59. In an embodiment, a mutant FRB comprises a mutation at E2032 and at T2098, where E2032 is mutated to any amino acid, and where T2098 is mutated to any amino acid, e.g., SEQ ID NO: 60. In an embodiment, a mutant FRB comprises an E2032I and a T2098L mutation, e.g., SEQ ID NO: 61. In an embodiment, a mutant FRB comprises an E2032L and a T2098L mutation, e.g., SEQ ID NO: 62.

**Table 10. Exemplary mutant FRB having increased affinity for a dimerization molecule.**

| **FRB mutant** | **Amino Acid Sequence** | **SEQ ID NO:** |
|---|---|---|
| E2032I mutant | ILWHEMWHEGLIEASRLYFGERNVKGMFEVLEPLHAMMERGPQTLKETSFNQAYGR DLMEAQEWCRKYMKSGNVKDLTQAWDLYYHVFRRISKTS | 57 |
| E2032L mutant | ILWHEMWHEGLLEASRLYFGERNVKGMFEVLEPLHAMMERGPQTLKETSFNQAYGR DLMEAQEWCRKYMKSGNVKDLTQAWDLYYHVFRRISKTS | 58 |
| T2098L mutant | ILWHEMWHEGLEEASRLYFGERNVKGMFEVLEPLHAMMERGPQTLKETSFNQAYGR DLMEAQEWCRKYMKSGNVKDLLQAWDLYYHVFRRISKTS | 59 |
| E2032, T2098 mutant | ILWHEMWHEGLXEASRLYFGERNVKGMFEVLEPLHAMMERGPQTLKETSFNQAYGR DLMEAQEWCRKYMKSGNVKDLXQAWDLYYHVFRRISKTS | 60 |
| E2032I, T2098L mutant | ILWHEMWHEGLIEASRLYFGERNVKGMFEVLEPLHAMMERGPQTLKETSFNQAYGR DLMEAQEWCRKYMKSGNVKDLLQAWDLYYHVFRRISKTS | 61 |
| E2032L, T2098L mutant | ILWHEMWHEGLLEASRLYFGERNVKGMFEVLEPLHAMMERGPQTLKETSFNQAYGR DLMEAQEWCRKYMKSGNVKDLLQAWDLYYHVFRRISKTS | 62 |

Other suitable dimerization switches include a GyrB-GyrB based dimerization switch, a Gibberellin-based dimerization switch, a tag/binder dimerization switch, and a halo-tag/snap-tag dimerization switch. Following the guidance provided herein, such switches and relevant dimerization molecules will be apparent to one of ordinary skill.

### Dimerization molecule

Association between the switch domains is promoted by the dimerization molecule. In the presence of dimerization molecule interaction or association between switch domains allows for signal transduction between a polypeptide associated with, e.g., fused to, a first switch domain, and a polypeptide associated with, e.g., fused to, a second switch domain. In the presence of non-limiting levels of dimerization molecule signal transduction is increased by 1.1, 1.2, 1.3, 1.4, 1.5, 1.6, 1.7, 1.8, 1.9, 2, 5, 10, 50, 100 fold, e.g., as measured in a system described herein.

Rapamycin and rapamycin analogs (sometimes referred to as rapalogues), e.g., RAD001, can be used as dimerization molecules in a FKBP/FRB-based dimerization switch described herein. In an embodiment the dimerization molecule can be selected from rapamycin (sirolimus), RAD001 (everolimus), zotarolimus, temsirolimus, AP-23573 (ridaforolimus), biolimus and AP21967. Additional rapamycin analogs suitable for use with FKBP/FRB-based dimerization switches are further described in the section entitled "Combination Therapies", or in the subsection entitled "Exemplary mTOR inhibitors".

### Split CAR

In some embodiments, the CAR-expressing cell uses a split CAR. The split CAR approach is described in more detail in publications WO2014/055442 and WO2014/055657. Briefly, a split CAR system comprises a cell expressing a first CAR having a first antigen binding domain and a costimulatory domain (e.g., 41BB), and the cell also expresses a second CAR having a second antigen binding domain and an intracellular signaling domain (e.g., CD3 zeta). When the cell encounters the first antigen, the costimulatory domain is activated, and the cell proliferates. When the cell encounters the second antigen, the intracellular signaling domain is activated and cell-killing activity begins. Thus, the CAR-expressing cell is only fully activated in the presence of both antigens.

### RNA Transfection

Disclosed herein are methods for producing an in vitro transcribed RNA CAR. The present invention may employ a CAR encoding RNA construct that can be directly transfected into a cell. A method for generating mRNA for use in transfection can involve in vitro transcription (IVT) of a template with specially designed primers, followed by polyA addition, to produce a construct containing 3' and 5' untranslated sequence ("UTR"), a 5' cap and/or Internal Ribosome Entry Site (IRES), the nucleic acid to be expressed, and a polyA tail, typically 50-2000 bases in length (SEQ ID NO:32). RNA so produced can efficiently transfect different kinds of cells. In one aspect, the template includes sequences for the CAR

In one aspect, a CAR used in the present invention is encoded by a messenger RNA (mRNA). In one aspect, the mRNA encoding a CAR described herein is introduced into an immune effector cell, e.g., a T cell or a NK cell, for production of a CAR-expressing cell, e.g., a CART cell or a CAR NK cell.

The *in vitro* transcribed RNA CAR can be introduced to a cell as a form of transient transfection. The RNA is produced by *in vitro* transcription using a polymerase chain reaction (PCR)-generated template. DNA of interest from any source can be directly converted by PCR into a template for in vitro mRNA synthesis using appropriate primers and RNA polymerase. The source of the DNA can be, for example, genomic DNA, plasmid DNA, phage DNA, cDNA, synthetic DNA sequence or any other appropriate source of DNA. The desired temple for *in vitro* transcription is a CAR described herein. For example, the template for the RNA CAR comprises an extracellular region comprising a single chain variable domain of an antibody to a tumor associated antigen described herein; a hinge region (e.g., a hinge region described herein), a transmembrane domain (e.g., a transmembrane domain described herein such as a transmembrane domain of CD8a); and a cytoplasmic region that includes an intracellular signaling domain, e.g., an intracellular signaling domain described herein, e.g., comprising the signaling domain of CD3-zeta and the signaling domain of 4-1BB.

In one instance, the DNA to be used for PCR contains an open reading frame. The DNA can be from a naturally occurring DNA sequence from the genome of an organism. In one instance, the nucleic acid can include some or all of the 5' and/or 3' untranslated regions (UTRs). The nucleic acid can include exons and introns. In one instance, the DNA to be used for PCR is a human nucleic acid sequence. In another instance, the DNA to be used for PCR is a human nucleic acid sequence including the 5' and 3' UTRs. The DNA can alternatively be an artificial DNA sequence that is not normally expressed in a naturally occurring organism. An exemplary artificial DNA sequence is one that contains portions of genes that are ligated together to form an open reading frame that encodes a fusion protein. The portions of DNA that are ligated together can be from a single organism or from more than one organism.

PCR is used to generate a template for in vitro transcription of mRNA which is used for transfection. Methods for performing PCR are well known in the art. Primers for use in PCR are designed to have regions that are substantially complementary to regions of the DNA to be used as a template for the PCR. "Substantially complementary," as used herein, refers to sequences of nucleotides where a majority or all of the bases in the primer sequence are complementary, or one or more bases are non-complementary, or mismatched. Substantially complementary sequences are able to anneal or hybridize with the intended DNA target under annealing conditions used for PCR. The primers can be designed to be substantially complementary to any portion of the DNA template. For example, the primers can be designed to amplify the portion of a nucleic acid that is normally transcribed in cells (the open reading frame), including 5' and 3' UTRs. The primers can also be designed to amplify a portion of a nucleic acid that encodes a particular domain of interest. In one instance, the primers are designed to amplify the coding region of a human cDNA, including all or portions of the 5' and 3' UTRs. Primers useful for PCR can be generated by synthetic methods that are well known in the art. "Forward primers" are primers that contain a region of nucleotides that are substantially complementary to nucleotides on the DNA template that are upstream of the DNA sequence that is to be amplified. "Upstream" is used herein to refer to a location 5, to the DNA sequence to be amplified relative to the coding strand. "Reverse primers" are primers that contain a region of nucleotides that are substantially complementary to a double-stranded DNA template that are downstream of the DNA sequence that is to be amplified. "Downstream" is used herein to refer to a location 3' to the DNA sequence to be amplified relative to the coding strand.

Any DNA polymerase useful for PCR can be used in the methods disclosed herein. The reagents and polymerase are commercially available from a number of sources.

Chemical structures with the ability to promote stability and/or translation efficiency may also be used. The RNA preferably has 5' and 3' UTRs. In one embodiment, the 5' UTR is between one and 3000 nucleotides in length. The length of 5' and 3' UTR sequences to be added to the coding region can be altered by different methods, including, but not limited to, designing primers for PCR that anneal to different regions of the UTRs. Using this approach, one of ordinary skill in the art can modify the 5' and 3' UTR lengths required to achieve optimal translation efficiency following transfection of the transcribed RNA.

The 5' and 3' UTRs can be the naturally occurring, endogenous 5' and 3' UTRs for the nucleic acid of interest. Alternatively, UTR sequences that are not endogenous to the nucleic acid of interest can be added by incorporating the UTR sequences into the forward and reverse primers or by any other modifications of the template. The use of UTR sequences that are not endogenous to the nucleic acid of interest can be useful for modifying the stability and/or translation efficiency of the RNA. For example, it is known that AU-rich elements in 3' UTR sequences can decrease the stability of mRNA. Therefore, 3' UTRs can be selected or designed to increase the stability of the transcribed RNA based on properties of UTRs that are well known in the art.

In one embodiment, the 5' UTR can contain the Kozak sequence of the endogenous nucleic acid. Alternatively, when a 5' UTR that is not endogenous to the nucleic acid of interest is being added by PCR as described above, a consensus Kozak sequence can be redesigned by adding the 5' UTR sequence. Kozak sequences can increase the efficiency of translation of some RNA transcripts, but does not appear to be required for all RNAs to enable efficient translation. The requirement for Kozak sequences for many mRNAs is known in the art. In other embodiments the 5' UTR can be 5'UTR of an RNA virus whose RNA genome is stable in cells. In other embodiments various nucleotide analogues can be used in the 3' or 5' UTR to impede exonuclease degradation of the mRNA.

To enable synthesis of RNA from a DNA template without the need for gene cloning, a promoter of transcription should be attached to the DNA template upstream of the sequence to be transcribed. When a sequence that functions as a promoter for an RNA polymerase is added to the 5' end of the forward primer, the RNA polymerase promoter becomes incorporated into the PCR product upstream of the open reading frame that is to be transcribed. In one preferred instance, the promoter is a T7 polymerase promoter, as described elsewhere herein. Other useful promoters include, but are not limited to, T3 and SP6 RNA polymerase promoters. Consensus nucleotide sequences for T7, T3 and SP6 promoters are known in the art.

In a preferred embodiment, the mRNA has both a cap on the 5' end and a 3' poly(A) tail which determine ribosome binding, initiation of translation and stability mRNA in the cell. On a circular DNA template, for instance, plasmid DNA, RNA polymerase produces a long concatameric product which is not suitable for expression in eukaryotic cells. The transcription of plasmid DNA linearized at the end of the 3' UTR results in normal sized mRNA which is not effective in eukaryotic transfection even if it is polyadenylated after transcription.

On a linear DNA template, phage T7 RNA polymerase can extend the 3' end of the transcript beyond the last base of the template (Schenborn and Mierendorf, Nuc Acids Res., 13:6223-36 (1985); Nacheva and Berzal-Herranz, Eur. J. Biochem., 270:1485-65 (2003).

The conventional method of integration of polyA/T stretches into a DNA template is molecular cloning. However polyA/T sequence integrated into plasmid DNA can cause plasmid instability, which is why plasmid DNA templates obtained from bacterial cells are often highly contaminated with deletions and other aberrations. This makes cloning procedures not only laborious and time consuming but often not reliable. That is why a method which allows construction of DNA templates with polyA/T 3' stretch without cloning highly desirable.

The polyA/T segment of the transcriptional DNA template can be produced during PCR by using a reverse primer containing a polyT tail, such as 100T tail (SEQ ID NO: 35) (size can be 50-5000 T (SEQ ID NO: 36)), or after PCR by any other method, including, but not limited to, DNA ligation or in vitro recombination. Poly(A) tails also provide stability to RNAs and reduce their degradation. Generally, the length of a poly(A) tail positively correlates with the stability of the transcribed RNA. In one embodiment, the poly(A) tail is between 100 and 5000 adenosines (SEQ ID NO: 37).

Poly(A) tails of RNAs can be further extended following in vitro transcription with the use of a poly(A) polymerase, such as E. coli polyA polymerase (E-PAP). In one embodiment, increasing the length of a poly(A) tail from 100 nucleotides to between 300 and 400 nucleotides (SEQ ID NO: 38) results in about a two-fold increase in the translation efficiency of the RNA. Additionally, the attachment of different chemical groups to the 3' end can increase mRNA stability. Such attachment can contain modified/artificial nucleotides, aptamers and other compounds. For example, ATP analogs can be incorporated into the poly(A) tail using poly(A) polymerase. ATP analogs can further increase the stability of the RNA.

5' caps on also provide stability to RNA molecules. In a preferred embodiment, RNAs produced by the methods disclosed herein include a 5' cap. The 5' cap is provided using techniques known in the art and described herein (Cougot, et al., Trends in Biochem. Sci., 29:436-444 (2001); Stepinski, et al., RNA, 7:1468-95 (2001); Elango, et al., Biochim. Biophys. Res. Commun., 330:958-966 (2005)).

The RNAs produced by the methods disclosed herein can also contain an internal ribosome entry site (IRES) sequence. The IRES sequence may be any viral, chromosomal or artificially designed sequence which initiates cap-independent ribosome binding to mRNA and facilitates the initiation of translation. Any solutes suitable for cell electroporation, which can contain factors facilitating cellular permeability and viability such as sugars, peptides, lipids, proteins, antioxidants, and surfactants can be included.

RNA can be introduced into target cells using any of a number of different methods, for instance, commercially available methods which include, but are not limited to, electroporation (Amaxa Nucleofector-II (Amaxa Biosystems, Cologne, Germany)), (ECM 830 (BTX) (Harvard Instruments, Boston, Mass.) or the Gene Pulser II (BioRad, Denver, Colo.), Multiporator (Eppendort, Hamburg Germany), cationic liposome mediated transfection using lipofection, polymer encapsulation, peptide mediated transfection, or biolistic particle delivery systems such as "gene guns" (see, for example, Nishikawa, et al. Hum Gene Ther., 12(8):861-70 (2001).

### Non-viral delivery methods

In some aspects, non-viral methods can be used to deliver a nucleic acid encoding a CAR described herein into a cell or tissue or a subject.

In some instances, the non-viral method includes the use of a transposon (also called a transposable element). In some instances, a transposon is a piece of DNA that can insert itself at a location in a genome, for example, a piece of DNA that is capable of selfreplicating and inserting its copy into a genome, or a piece of DNA that can be spliced out of a longer nucleic acid and inserted into another place in a genome. For example, a transposon comprises a DNA sequence made up of inverted repeats flanking genes for transposition.

Exemplary methods of nucleic acid delivery using a transposon include a Sleeping Beauty transposon system (SBTS) and a piggyBac (PB) transposon system. See, e.g., Aronovich et al. Hum. Mol. Genet. 20.R1(2011):R14-20; Singh et al. Cancer Res. 15(2008):2961-2971; Huang et al. Mol. Ther. 16(2008):580-589; Grabundzija et al. Mol. Ther. 18(2010):1200-1209; Kebriaei et al. Blood. 122.21(2013):166; Williams. Molecular Therapy 16.9(2008):1515-16; Bell et al. Nat. Protoc. 2.12(2007):3153-65; and Ding et al. Cell. 122.3(2005):473-83.

The SBTS includes two components: 1) a transposon containing a transgene and 2) a source of transposase enzyme. The transposase can transpose the transposon from a carrier plasmid (or other donor DNA) to a target DNA, such as a host cell chromosome/genome. For example, the transposase binds to the carrier plasmid/donor DNA, cuts the transposon (including transgene(s)) out of the plasmid, and inserts it into the genome of the host cell. See, e.g., Aronovich et al. *supra.*

Exemplary transposons include a pT2-based transposon. See, e.g., Grabundzija et al. Nucleic Acids Res. 41.3(2013):1829-47; and Singh et al. Cancer Res. 68.8(2008): 2961-2971. Exemplary transposases include a Tc1/mariner-type transposase, e.g., the SB10 transposase or the SB11 transposase (a hyperactive transposase which can be expressed, e.g., from a cytomegalovirus promoter). See, e.g., Aronovich et al.; Kebriaei et al.; and Grabundzij a et al.

Use of the SBTS permits efficient integration and expression of a transgene, e.g., a nucleic acid encoding a CAR described herein. Provided herein are methods of generating a cell, e.g., T cell or NK cell, that stably expresses a CAR described herein, e.g., using a transposon system such as SBTS.

In accordance with methods described herein, in some instances, one or more nucleic acids, e.g., plasmids, containing the SBTS components are delivered to a cell (e.g., T or NK cell). For example, the nucleic acid(s) are delivered by standard methods of nucleic acid (e.g., plasmid DNA) delivery, e.g., methods described herein, e.g., electroporation, transfection, or lipofection. In some instances, the nucleic acid contains a transposon comprising a transgene, e.g., a nucleic acid encoding a CAR described herein. In some instances, the nucleic acid contains a transposon comprising a transgene (e.g., a nucleic acid encoding a CAR described herein) as well as a nucleic acid sequence encoding a transposase enzyme. In other instances, a system with two nucleic acids is provided, e.g., a dual-plasmid system, e.g., where a first plasmid contains a transposon comprising a transgene, and a second plasmid contains a nucleic acid sequence encoding a transposase enzyme. For example, the first and the second nucleic acids are co-delivered into a host cell.

In some instances, cells, e.g., T or NK cells, are generated that express a CAR described herein by using a combination of gene insertion using the SBTS and genetic editing using a nuclease (e.g., Zinc finger nucleases (ZFNs), Transcription Activator-Like Effector Nucleases (TALENs), the CRISPR/Cas system, or engineered meganuclease reengineered homing endonucleases).

In some instances, use of a non-viral method of delivery permits reprogramming of cells, e.g., T or NK cells, and direct infusion of the cells into a subject. Advantages of non-viral vectors include but are not limited to the ease and relatively low cost of producing sufficient amounts required to meet a patient population, stability during storage, and lack of immunogenicity.

### Nucleic Acid Constructs Encoding a CAR

The present invention also employs nucleic acid molecules encoding one or more CAR constructs in accordance with the claims. In one aspect, the nucleic acid molecule is provided as a messenger RNA transcript. In one aspect, the nucleic acid molecule is provided as a DNA construct.

Accordingly, in one aspect, the invention employs a nucleic acid molecule encoding a chimeric antigen receptor (CAR), wherein the CAR comprises an antigen binding domain that binds to a tumor antigen described herein, a transmembrane domain (e.g., a transmembrane domain described herein), and an intracellular signaling domain (e.g., an intracellular signaling domain described herein) comprising a stimulatory domain, e.g., a costimulatory signaling domain (e.g., a costimulatory signaling domain described herein) and/or a primary signaling domain (e.g., a primary signaling domain described herein, e.g., a zeta chain described herein). In one embodiment, the transmembrane domain is transmembrane domain of a protein selected from the group consisting of the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137 and CD154. In some embodiments, a transmembrane domain may include at least the transmembrane region(s) of, e.g., KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), NKp44, NKp30, NKp46, CD160, CD19, IL2R beta, IL2R gamma, IL7R α, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO-3), BLAME (SLAMF8), SELPLG (CD162), LTBR, PAG/Cbp.

In one embodiment, the transmembrane domain comprises a sequence of SEQ ID NO: 12, or a sequence with 95-99% identity thereof. In one embodiment, the antigen binding domain is connected to the transmembrane domain by a hinge region, e.g., a hinge described herein. In one embodiment, the hinge region comprises SEQ ID NO:4 or SEQ ID NO:6 or SEQ ID NO:8 or SEQ ID NO:10, or a sequence with 95-99% identity thereof. In one embodiment, the isolated nucleic acid molecule further comprises a sequence encoding a costimulatory domain. In one embodiment, the costimulatory domain is a functional signaling domain of a protein selected from the group consisting of OX40, CD27, CD28, CDS, ICAM-1, LFA-1 (CD11a/CD18), ICOS (CD278), and 4-1BB (CD137). Further examples of such costimulatory molecules include CDS, ICAM-1, GITR, BAFFR, HVEM (LIGHTR), SLAMF7, NKp80 (KLRF1), NKp44, NKp30, NKp46, CD160, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, and PAG/Cbp. In one embodiment, the costimulatory domain comprises a sequence of SEQ ID NO:16, or a sequence with 95-99% identity thereof. In one embodiment, the intracellular signaling domain comprises a functional signaling domain of 4-1BB and a functional signaling domain of CD3 zeta. In one embodiment, the intracellular signaling domain comprises the sequence of SEQ ID NO: 14 or SEQ ID NO:16, or a sequence with 95-99% identity thereof, and the sequence of SEQ ID NO: 18 or SEQ ID NO:20, or a sequence with 95-99% identity thereof, wherein the sequences comprising the intracellular signaling domain are expressed in the same frame and as a single polypeptide chain.

In another aspect, the invention employs an isolated nucleic acid molecule encoding a CAR construct comprising a leader sequence of SEQ ID NO: 2, a scFv domain as described herein, a hinge region of SEQ ID NO:4 or SEQ ID NO:6 or SEQ ID NO:8 or SEQ ID NO: 10 (or a sequence with 95-99% identity thereof), a transmembrane domain having a sequence of SEQ ID NO: 12 (or a sequence with 95-99% identity thereof), a 4-1BB costimulatory domain having a sequence of SEQ ID NO:14 or a CD27 costimulatory domain having a sequence of SEQ ID NO: 16 (or a sequence with 95-99% identity thereof), and a CD3 zeta stimulatory domain having a sequence of SEQ ID NO:18 or SEQ ID NO:20 (or a sequence with 95-99% identity thereof).

In another aspect, the invention employs a nucleic acid molecule encoding a chimeric antigen receptor (CAR) molecule that comprises an antigen binding domain, a transmembrane domain, and an intracellular signaling domain comprising a stimulatory domain, and wherein said antigen binding domain binds to a tumor antigen selected from a group consisting of: CD19, CD123, CD22, CD30, CD171, CS-1, CLL-1 (CLECL1), CD33, EGFRvIII, GD2, GD3, BCMA, Tn Ag, PSMA, ROR1, FLT3, FAP, TAG72, CD38, CD44v6, CEA, EPCAM, B7H3, KIT, IL-13Ra2, Mesothelin, IL-11Ra, PSCA, VEGFR2, LewisY, CD24, PDGFR-beta, SSEA-4, CD20, Folate receptor alpha, ERBB2 (Her2/neu), MUC1, EGFR, NCAM, Prostase, PRSS21, PAP, ELF2M, Ephrin B2, IGF-I receptor, CAIX, LMP2, gp100, bcr-abl, tyrosinase, EphA2, Fucosyl GM1, sLe, GM3, TGS5, HMWMAA, o-acetyl-GD2, Folate receptor beta, TEM1/CD248, TEM7R, CLDN6, TSHR, GPRC5D, CXORF61, CD97, CD179a, ALK, Polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6,E7, MAGE A1, ETV6-AML, sperm protein 17, XAGE1, Tie 2, MAD-CT-1, MAD-CT-2, Fos-related antigen 1, p53, p53 mutant, prostein, survivin and telomerase, PCTA-1/Galectin 8, MelanA/MART1, Ras mutant, hTERT, sarcoma translocation breakpoints, ML-IAP, ERG (TMPRSS2 ETS fusion gene), NA17, PAX3, Androgen receptor, Cyclin B1, MYCN, RhoC, TRP-2, CYP1B1, BORIS, SART3, PAX5, OY-TES1, LCK, AKAP-4, SSX2, RAGE-1, human telomerase reverse transcriptase, RU1, RU2, intestinal carboxyl esterase, mut hsp70-2, CD79a, CD79b, CD72, LAIR1, FCAR, LILRA2, CD300LF, CLEC12A, BST2, EMR2, LY75, GPC3, FCRL5, and IGLL1.

In one embodiment, the encoded CAR molecule further comprises a sequence encoding a costimulatory domain. In one embodiment, the costimulatory domain is a functional signaling domain of a protein selected from the group consisting of OX40, CD27, CD28, CDS, ICAM-1, LFA-1 (CD11a/CD18) and 4-1BB (CD137). In one embodiment, the costimulatory domain comprises a sequence of SEQ ID NO:14. In one embodiment, the transmembrane domain is a transmembrane domain of a protein selected from the group consisting of the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137 and CD154. In one embodiment, the transmembrane domain comprises a sequence of SEQ ID NO:12. In one embodiment, the intracellular signaling domain comprises a functional signaling domain of 4-1BB and a functional signaling domain of zeta. In one embodiment, the intracellular signaling domain comprises the sequence of SEQ ID NO: 14 and the sequence of SEQ ID NO: 18, wherein the sequences comprising the intracellular signaling domain are expressed in the same frame and as a single polypeptide chain. In one embodiment, the anti-a cancer associated antigen as described herein binding domain is connected to the transmembrane domain by a hinge region. In one embodiment, the hinge region comprises SEQ ID NO:4. In one embodiment, the hinge region comprises SEQ ID NO:6 or SEQ ID NO:8 or SEQ ID NO:10.

The nucleic acid sequences coding for the desired molecules can be obtained using recombinant methods known in the art, such as, for example by screening libraries from cells expressing the gene, by deriving the gene from a vector known to include the same, or by isolating directly from cells and tissues containing the same, using standard techniques. Alternatively, the gene of interest can be produced synthetically, rather than cloned.

The present invention also employs vectors in which a DNA in accordance with the claims is inserted. Vectors derived from retroviruses such as the lentivirus are suitable tools to achieve long-term gene transfer since they allow long-term, stable integration of a transgene and its propagation in daughter cells. Lentiviral vectors have the added advantage over vectors derived from onco-retroviruses such as murine leukemia viruses in that they can transduce non-proliferating cells, such as hepatocytes. They also have the added advantage of low immunogenicity. A retroviral vector may also be, e.g., a gammaretroviral vector. A gammaretroviral vector may include, e.g., a promoter, a packaging signal (ψ), a primer binding site (PBS), one or more (e.g., two) long terminal repeats (LTR), and a transgene of interest, e.g., a gene encoding a CAR. A gammaretroviral vector may lack viral structural gens such as gag, pol, and env. Exemplary gammaretroviral vectors include Murine Leukemia Virus (MLV), Spleen-Focus Forming Virus (SFFV), and Myeloproliferative Sarcoma Virus (MPSV), and vectors derived therefrom. Other gammaretroviral vectors are described, e.g., in Tobias Maetzig et al., "Gammaretroviral Vectors: Biology, Technology and Application" Viruses. 2011 Jun; 3(6): 677-713.

In another embodiment, the vector comprising the nucleic acid encoding the desired CAR is an adenoviral vector (A5/35). In another embodiment, the expression of nucleic acids encoding CARs can be accomplished using of transposons such as sleeping beauty, crisper, CAS9, and zinc finger nucleases. See below June et al. 2009Nature Reviews Immunology 9.10: 704-716.

In brief summary, the expression of natural or synthetic nucleic acids encoding CARs is typically achieved by operably linking a nucleic acid encoding the CAR polypeptide or portions thereof to a promoter, and incorporating the construct into an expression vector. The vectors can be suitable for replication and integration eukaryotes. Typical cloning vectors contain transcription and translation terminators, initiation sequences, and promoters useful for regulation of the expression of the desired nucleic acid sequence.

The expression constructs employed in the present invention may also be used for nucleic acid immunization and gene therapy, using standard gene delivery protocols. Methods for gene delivery are known in the art. See, e.g., U.S. Pat. Nos. 5,399,346, 5,580,859, 5,589,466. In another instance, the disclosure provides a gene therapy vector.

The nucleic acid can be cloned into a number of types of vectors. For example, the nucleic acid can be cloned into a vector including, but not limited to a plasmid, a phagemid, a phage derivative, an animal virus, and a cosmid. Vectors of particular interest include expression vectors, replication vectors, probe generation vectors, and sequencing vectors.

Further, the expression vector may be provided to a cell in the form of a viral vector. Viral vector technology is well known in the art and is described, for example, in Sambrook et al., 2012, MOLECULAR CLONING: A LABORATORY MANUAL, volumes 1 -4, Cold Spring Harbor Press, NY), and in other virology and molecular biology manuals. Viruses, which are useful as vectors include, but are not limited to, retroviruses, adenoviruses, adeno- associated viruses, herpes viruses, and lentiviruses. In general, a suitable vector contains an origin of replication functional in at least one organism, a promoter sequence, convenient restriction endonuclease sites, and one or more selectable markers, (e.g., WO 01/96584; WO 01/29058; and U.S. Pat. No. 6,326,193).

A number of viral based systems have been developed for gene transfer into mammalian cells. For example, retroviruses provide a convenient platform for gene delivery systems. A selected gene can be inserted into a vector and packaged in retroviral particles using techniques known in the art. The recombinant virus can then be isolated and delivered to cells of the subject either in vivo or ex vivo. A number of retroviral systems are known in the art. In some embodiments, adenovirus vectors are used. A number of adenovirus vectors are known in the art. In one embodiment, lentivirus vectors are used.

Additional promoter elements, e.g., enhancers, regulate the frequency of transcriptional initiation. Typically, these are located in the region 30-110 bp upstream of the start site, although a number of promoters have been shown to contain functional elements downstream of the start site as well. The spacing between promoter elements frequently is flexible, so that promoter function is preserved when elements are inverted or moved relative to one another. In the thymidine kinase (tk) promoter, the spacing between promoter elements can be increased to 50 bp apart before activity begins to decline. Depending on the promoter, it appears that individual elements can function either cooperatively or independently to activate transcription. Exemplary promoters include the CMV IE gene, EF-1α, ubiquitin C, or phosphoglycerokinase (PGK) promoters.

An example of a promoter that is capable of expressing a CAR encoding nucleic acid molecule in a mammalian T cell is the EFla promoter. The native EFla promoter drives expression of the alpha subunit of the elongation factor-1 complex, which is responsible for the enzymatic delivery of aminoacyl tRNAs to the ribosome. The EFla promoter has been extensively used in mammalian expression plasmids and has been shown to be effective in driving CAR expression from nucleic acid molecules cloned into a lentiviral vector. See, e.g., Milone et al., Mol. Ther. 17(8): 1453-1464 (2009). In one aspect, the EFla promoter comprises the sequence provided as SEQ ID NO:1.

Another example of a promoter is the immediate early cytomegalovirus (CMV) promoter sequence. This promoter sequence is a strong constitutive promoter sequence capable of driving high levels of expression of any polynucleotide sequence operatively linked thereto. However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the elongation factor-la promoter, the hemoglobin promoter, and the creatine kinase promoter. Further, the invention should not be limited to the use of constitutive promoters. Inducible promoters are also contemplated as part of the invention. The use of an inducible promoter provides a molecular switch capable of turning on expression of the polynucleotide sequence which it is operatively linked when such expression is desired, or turning off the expression when expression is not desired. Examples of inducible promoters include, but are not limited to a metallothionine promoter, a glucocorticoid promoter, a progesterone promoter, and a tetracycline promoter.

A vector may also include, e.g., a signal sequence to facilitate secretion, a polyadenylation signal and transcription terminator (e.g., from Bovine Growth Hormone (BGH) gene), an element allowing episomal replication and replication in prokaryotes (e.g. SV40 origin and ColE1 or others known in the art) and/or elements to allow selection (e.g., ampicillin resistance gene and/or zeocin marker).

In order to assess the expression of a CAR polypeptide or portions thereof, the expression vector to be introduced into a cell can also contain either a selectable marker gene or a reporter gene or both to facilitate identification and selection of expressing cells from the population of cells sought to be transfected or infected through viral vectors. In other aspects, the selectable marker may be carried on a separate piece of DNA and used in a co- transfection procedure. Both selectable markers and reporter genes may be flanked with appropriate regulatory sequences to enable expression in the host cells. Useful selectable markers include, for example, antibiotic-resistance genes, such as neo and the like.

Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, e.g., enzymatic activity. Expression of the reporter gene is assayed at a suitable time after the DNA has been introduced into the recipient cells. Suitable reporter genes may include genes encoding luciferase, beta-galactosidase, chloramphenicol acetyl transferase, secreted alkaline phosphatase, or the green fluorescent protein gene (e.g., Ui-Tei et al., 2000 FEBS Letters 479: 79-82). Suitable expression systems are well known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter- driven transcription.

Methods of introducing and expressing genes into a cell are known in the art. In the context of an expression vector, the vector can be readily introduced into a host cell, e.g., mammalian, bacterial, yeast, or insect cell by any method in the art. For example, the expression vector can be transferred into a host cell by physical, chemical, or biological means.

Physical methods for introducing a polynucleotide into a host cell include calcium phosphate precipitation, lipofection, particle bombardment, microinjection, electroporation, and the like. Methods for producing cells comprising vectors and/or exogenous nucleic acids are well-known in the art. See, for example, Sambrook et al., 2012, MOLECULAR CLONING: A LABORATORY MANUAL, volumes 1 -4, Cold Spring Harbor Press, NY). A preferred method for the introduction of a polynucleotide into a host cell is calcium phosphate transfection

Biological methods for introducing a polynucleotide of interest into a host cell include the use of DNA and RNA vectors. Viral vectors, and especially retroviral vectors, have become the most widely used method for inserting genes into mammalian, e.g., human cells. Other viral vectors can be derived from lentivirus, poxviruses, herpes simplex virus I, adenoviruses and adeno-associated viruses, and the like. See, for example, U.S. Pat. Nos. 5,350,674 and 5,585,362.

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle in vitro and in vivo is a liposome (e.g. , an artificial membrane vesicle). Other methods of state-of-the-art targeted delivery of nucleic acids are available, such as delivery of polynucleotides with targeted nanoparticles or other suitable sub-micron sized delivery system.

In the case where a non-viral delivery system is utilized, an exemplary delivery vehicle is a liposome. The use of lipid formulations is contemplated for the introduction of the nucleic acids into a host cell (in vitro, ex vivo or in vivo). In another aspect, the nucleic acid may be associated with a lipid. The nucleic acid associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/DNA or lipid/expression vector associated compositions are not limited to any particular structure in solution. For example, they may be present in a bilayer structure, as micelles, or with a "collapsed" structure. They may also simply be interspersed in a solution, possibly forming aggregates that are not uniform in size or shape. Lipids are fatty substances which may be naturally occurring or synthetic lipids. For example, lipids include the fatty droplets that naturally occur in the cytoplasm as well as the class of compounds which contain long-chain aliphatic hydrocarbons and their derivatives, such as fatty acids, alcohols, amines, amino alcohols, and aldehydes.

Lipids suitable for use can be obtained from commercial sources. For example, dimyristyl phosphatidylcholine ("DMPC") can be obtained from Sigma, St. Louis, MO; dicetyl phosphate ("DCP") can be obtained from K & K Laboratories (Plainview, NY); cholesterol ("Choi") can be obtained from Calbiochem-Behring; dimyristyl phosphatidylglycerol ("DMPG") and other lipids may be obtained from Avanti Polar Lipids, Inc. (Birmingham, AL.). Stock solutions of lipids in chloroform or chloroform/methanol can be stored at about -20°C. Chloroform is used as the only solvent since it is more readily evaporated than methanol. "Liposome" is a generic term encompassing a variety of single and multilamellar lipid vehicles formed by the generation of enclosed lipid bilayers or aggregates. Liposomes can be characterized as having vesicular structures with a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers (Ghosh et al., 1991 Glycobiology 5: 505-10). However, compositions that have different structures in solution than the normal vesicular structure are also encompassed. For example, the lipids may assume a micellar structure or merely exist as nonuniform aggregates of lipid molecules. Also contemplated are lipofectamine-nucleic acid complexes.

Regardless of the method used to introduce exogenous nucleic acids into a host cell or otherwise expose a cell to the inhibitor of the present disclosure, in order to confirm the presence of the recombinant DNA sequence in the host cell, a variety of assays may be performed. Such assays include, for example, "molecular biological" assays well known to those of skill in the art, such as Southern and Northern blotting, RT-PCR and PCR; "biochemical" assays, such as detecting the presence or absence of a particular peptide, e.g., by immunological means (ELISAs and Western blots) or by assays described herein to identify agents falling within the scope of the disclosure.

The present invention may also employ a vector comprising a CAR encoding nucleic acid molecule. In one aspect, a CAR vector can be directly transduced into a cell, *e.g.,* a T cell or a NK cell. In one aspect, the vector is a cloning or expression vector, *e.g.,* a vector including, but not limited to, one or more plasmids (e.g., expression plasmids, cloning vectors, minicircles, minivectors, double minute chromosomes), retroviral and lentiviral vector constructs. In one aspect, the vector is capable of expressing the CAR construct in mammalian immune effector cells (e.g., T cells, NK cells). In one aspect, the mammalian T cell is a human T cell. In one aspect, the mammalian NK cell is a human NK cell.

### Sources of Cells

Prior to expansion and genetic modification or other modification, a source of cells, e.g., T cells or natural killer (NK) cells, can be obtained from a subject. The term "subject" is intended to include living organisms in which an immune response can be elicited (e.g., mammals). Examples of subjects include humans, monkeys, chimpanzees, dogs, cats, mice, rats, and transgenic species thereof. T cells can be obtained from a number of sources, including peripheral blood mononuclear cells, bone marrow, lymph node tissue, cord blood, thymus tissue, tissue from a site of infection, ascites, pleural effusion, spleen tissue, and tumors.

In certain aspects of the present disclosure, immune effector cells, e.g., T cells, can be obtained from a unit of blood collected from a subject using any number of techniques known to the skilled artisan, such as Ficoll^{™} separation. In one preferred aspect, cells from the circulating blood of an individual are obtained by apheresis. The apheresis product typically contains lymphocytes, including T cells, monocytes, granulocytes, B cells, other nucleated white blood cells, red blood cells, and platelets. In one aspect, the cells collected by apheresis may be washed to remove the plasma fraction and, optionally, to place the cells in an appropriate buffer or media for subsequent processing steps. In one instance, the cells are washed with phosphate buffered saline (PBS). In an alternative instance, the wash solution lacks calcium and may lack magnesium or may lack many if not all divalent cations.

Initial activation steps in the absence of calcium can lead to magnified activation. As those of ordinary skill in the art would readily appreciate a washing step may be accomplished by methods known to those in the art, such as by using a semi-automated "flow-through" centrifuge (for example, the Cobe 2991 cell processor, the Baxter CytoMate, or the Haemonetics Cell Saver 5) according to the manufacturer's instructions. After washing, the cells may be resuspended in a variety of biocompatible buffers, such as, for example, Ca-free, Mg-free PBS, PlasmaLyte A, or other saline solution with or without buffer. Alternatively, the undesirable components of the apheresis sample may be removed and the cells directly resuspended in culture media.

It is recognized that the methods of the application can utilize culture media conditions comprising 5% or less, for example 2%, human AB serum, and employ known culture media conditions and compositions, for example those described in Smith et al., "Ex vivo expansion of human T cells for adoptive immunotherapy using the novel Xeno-free CTS Immune Cell Serum Replacement" Clinical & Translational Immunology (2015) 4, e31; doi: 10.1038/cti.2014.31.

In one aspect, T cells are isolated from peripheral blood lymphocytes by lysing the red blood cells and depleting the monocytes, for example, by centrifugation through a PERCOLL^{™} gradient or by counterflow centrifugal elutriation.

The methods described herein can include, e.g., selection of a specific subpopulation of immune effector cells, e.g., T cells, that are a T regulatory cell-depleted population, CD25+ depleted cells, using, e.g., a negative selection technique, e.g., described herein. Preferably, the population of T regulatory depleted cells contains less than 30%, 25%, 20%, 15%, 10%, 5%, 4%, 3%, 2%, 1% of CD25+ cells.

In one instance, T regulatory cells, e.g., CD25+ T cells, are removed from the population using an anti-CD25 antibody, or fragment thereof, or a CD25-binding ligand, IL-2. In one instance, the anti-CD25 antibody, or fragment thereof, or CD25-binding ligand is conjugated to a substrate, e.g., a bead, or is otherwise coated on a substrate, e.g., a bead. In one instance, the anti-CD25 antibody, or fragment thereof, is conjugated to a substrate as described herein.

In one instance, the T regulatory cells, e.g., CD25+ T cells, are removed from the population using CD25 depletion reagent from Miltenyi^{™}. In one instance, the ratio of cells to CD25 depletion reagent is 1e7 cells to 20 uL, or 1e7 cells to15 uL, or 1e7 cells to 10 uL, or 1e7 cells to 5 uL, or 1e7 cells to 2.5 uL, or 1e7 cells to 1.25 uL. In one instance, e.g., for T regulatory cells, e.g., CD25+ depletion, greater than 500 million cells/ml is used. In a further aspect, a concentration of cells of 600, 700, 800, or 900 million cells/ml is used.

In one instance, the population of immune effector cells to be depleted includes about 6 x 10⁹ CD25+ T cells. In other aspects, the population of immune effector cells to be depleted include about 1 x 10⁹ to 1x 10¹⁰ CD25+ T cell, and any integer value in between. In one instance, the resulting population T regulatory depleted cells has 2 x 10⁹ T regulatory cells, e.g., CD25+ cells, or less (e.g., 1 x 10⁹, 5 x 10⁸, 1 x 10⁸, 5 x 10⁷, 1 x 10⁷, or less CD25+ cells).

In one instance, the T regulatory cells, e.g., CD25+ cells, are removed from the population using the CliniMAC system with a depletion tubing set, such as, e.g., tubing 162-01. In one instance, the CliniMAC system is run on a depletion setting such as, e.g., DEPLETION2.1.

Without wishing to be bound by a particular theory, decreasing the level of negative regulators of immune cells (e.g., decreasing the number of unwanted immune cells, e.g., T_{REG} cells), in a subject prior to apheresis or during manufacturing of a CAR-expressing cell product can reduce the risk of subject relapse. For example, methods of depleting T_{REG} cells are known in the art. Methods of decreasing T_{REG} cells include, but are not limited to, cyclophosphamide, anti-GITR antibody (an anti-GITR antibody described herein), CD25-depletion, and combinations thereof.

In some instances, the manufacturing methods comprise reducing the number of (e.g., depleting) T_{REG} cells prior to manufacturing of the CAR-expressing cell. For example, manufacturing methods comprise contacting the sample, e.g., the apheresis sample, with an anti-GITR antibody and/or an anti-CD25 antibody (or fragment thereof, or a CD25-binding ligand), e.g., to deplete T_{REG} cells prior to manufacturing of the CAR-expressing cell (e.g., T cell, NK cell) product.

In an instance, a subject is pre-treated with one or more therapies that reduce T_{REG} cells prior to collection of cells for CAR-expressing cell product manufacturing, thereby reducing the risk of subject relapse to CAR-expressing cell treatment. In an instance, methods of decreasing T_{REG} cells include, but are not limited to, administration to the subject of one or more of cyclophosphamide, anti-GITR antibody, CD25-depletion, or a combination thereof. Administration of one or more of cyclophosphamide, anti-GITR antibody, CD25-depletion, or a combination thereof, can occur before, during or after an infusion of the CAR-expressing cell product.

In an instance, a subject is pre-treated with cyclophosphamide prior to collection of cells for CAR-expressing cell product manufacturing, thereby reducing the risk of subject relapse to CAR-expressing cell treatment. In an instance, a subject is pre-treated with an anti-GITR antibody prior to collection of cells for CAR-expressing cell product manufacturing, thereby reducing the risk of subject relapse to CAR-expressing cell treatment.

In one instance, the population of cells to be removed are neither the regulatory T cells or tumor cells, but cells that otherwise negatively affect the expansion and/or function of CART cells, e.g. cells expressing CD14, CD11b, CD33, CD15, or other markers expressed by potentially immune suppressive cells. In one instance, such cells are envisioned to be removed concurrently with regulatory T cells and/or tumor cells, or following said depletion, or in another order.

The methods described herein can include more than one selection step, e.g., more than one depletion step. Enrichment of a T cell population by negative selection can be accomplished, e.g., with a combination of antibodies directed to surface markers unique to the negatively selected cells. One method is cell sorting and/or selection via negative magnetic immunoadherence or flow cytometry that uses a cocktail of monoclonal antibodies directed to cell surface markers present on the cells negatively selected. For example, to enrich for CD4+ cells by negative selection, a monoclonal antibody cocktail can include antibodies to CD14, CD20, CD11b, CD16, HLA-DR, and CD8.

The methods described herein can further include removing cells from the population which express a tumor antigen, e.g., a tumor antigen that does not comprise CD25, e.g., CD19, CD30, CD38, CD123, CD20, CD14 or CD11b, to thereby provide a population of T regulatory depleted, e.g., CD25+ depleted, and tumor antigen depleted cells that are suitable for expression of a CAR, e.g., a CAR described herein. In one instance, tumor antigen expressing cells are removed simultaneously with the T regulatory, e.g., CD25+ cells. For example, an anti-CD25 antibody, or fragment thereof, and an anti-tumor antigen antibody, or fragment thereof, can be attached to the same substrate, e.g., bead, which can be used to remove the cells or an anti-CD25 antibody, or fragment thereof, or the anti-tumor antigen antibody, or fragment thereof, can be attached to separate beads, a mixture of which can be used to remove the cells. In other instances, the removal of T regulatory cells, e.g., CD25+ cells, and the removal of the tumor antigen expressing cells is sequential, and can occur, e.g., in either order.

Also provided are methods that include removing cells from the population which express a check point inhibitor, e.g., a check point inhibitor described herein, e.g., one or more of PD1+ cells, LAG3+ cells, and TIM3+ cells, to thereby provide a population of T regulatory depleted, e.g., CD25+ depleted cells, and check point inhibitor depleted cells, e.g., PD1+, LAG3+ and/or TIM3+ depleted cells. Exemplary check point inhibitors include B7-H1, B7-1, CD160, P1H, 2B4, PD1, TIM3, CEACAM (e.g., CEACAM-1, CEACAM-3 and/or CEACAM-5), LAG3, TIGIT, CTLA-4, BTLA and LAIR1. In one instance, check point inhibitor expressing cells are removed simultaneously with the T regulatory, e.g., CD25+ cells. For example, an anti-CD25 antibody, or fragment thereof, and an anti-check point inhibitor antibody, or fragment thereof, can be attached to the same bead which can be used to remove the cells, or an anti-CD25 antibody, or fragment thereof, and the anti-check point inhibitor antibody, or fragment there, can be attached to separate beads, a mixture of which can be used to remove the cells. In other instances, the removal of T regulatory cells, e.g., CD25+ cells, and the removal of the check point inhibitor expressing cells is sequential, and can occur, e.g., in either order.

Methods described herein can include a positive selection step. For example, T cells can isolated by incubation with anti-CD3/anti-CD28 (e.g., 3x28)-conjugated beads, such as DYNABEADS^{®} M-450 CD3/CD28 T, for a time period sufficient for positive selection of the desired T cells. In one instance, the time period is about 30 minutes. In a further instance, the time period ranges from 30 minutes to 36 hours or longer and all integer values there between. In a further instance, the time period is at least 1, 2, 3, 4, 5, or 6 hours. In yet another instance, the time period is 10 to 24 hours, e.g., 24 hours. Longer incubation times may be used to isolate T cells in any situation where there are few T cells as compared to other cell types, such in isolating tumor infiltrating lymphocytes (TIL) from tumor tissue or from immunocompromised individuals. Further, use of longer incubation times can increase the efficiency of capture of CD8+ T cells. Thus, by simply shortening or lengthening the time T cells are allowed to bind to the CD3/CD28 beads and/or by increasing or decreasing the ratio of beads to T cells (as described further herein), subpopulations of T cells can be preferentially selected for or against at culture initiation or at other time points during the process. Additionally, by increasing or decreasing the ratio of anti-CD3 and/or anti-CD28 antibodies on the beads or other surface, subpopulations of T cells can be preferentially selected for or against at culture initiation or at other desired time points.

In one instance, a T cell population can be selected that expresses one or more of IFN-^{γ}, TNFα, IL-17A, IL-2, IL-3, IL-4, GM-CSF, IL-10, IL-13, granzyme B, and perforin, or other appropriate molecules, e.g., other cytokines. Methods for screening for cell expression can be determined, e.g., by the methods described in PCT Publication No.: WO 2013/126712.

For isolation of a desired population of cells by positive or negative selection, the concentration of cells and surface (e.g., particles such as beads) can be varied. In certain aspects, it may be desirable to significantly decrease the volume in which beads and cells are mixed together (e.g., increase the concentration of cells), to ensure maximum contact of cells and beads. For example, in one aspect, a concentration of 10 billion cells/ml, 9 billion/ml, 8 billion/ml, 7 billion/ml, 6 billion/ml, or 5 billion/ml is used. In one aspect, a concentration of 1 billion cells/ml is used. In yet one aspect, a concentration of cells from 75, 80, 85, 90, 95, or 100 million cells/ml is used. In further aspects, concentrations of 125 or 150 million cells/ml can be used.

Using high concentrations can result in increased cell yield, cell activation, and cell expansion. Further, use of high cell concentrations allows more efficient capture of cells that may weakly express target antigens of interest, such as CD28-negative T cells, or from samples where there are many tumor cells present (e.g., leukemic blood, tumor tissue, etc.). Such populations of cells may have therapeutic value and would be desirable to obtain. For example, using high concentration of cells allows more efficient selection of CD8+ T cells that normally have weaker CD28 expression.

In a related aspect, it may be desirable to use lower concentrations of cells. By significantly diluting the mixture of T cells and surface (e.g., particles such as beads), interactions between the particles and cells is minimized. This selects for cells that express high amounts of desired antigens to be bound to the particles. For example, CD4+ T cells express higher levels of CD28 and are more efficiently captured than CD8+ T cells in dilute concentrations. In one aspect, the concentration of cells used is 5 x 10⁶/ml. In other aspects, the concentration used can be from about 1 x 10⁵/ml to 1 x 10⁶/ml, and any integer value in between.

In other aspects, the cells may be incubated on a rotator for varying lengths of time at varying speeds at either 2-10°C or at room temperature.

T cells for stimulation can also be frozen after a washing step. Wishing not to be bound by theory, the freeze and subsequent thaw step provides a more uniform product by removing granulocytes and to some extent monocytes in the cell population. After the washing step that removes plasma and platelets, the cells may be suspended in a freezing solution. While many freezing solutions and parameters are known in the art and will be useful in this context, one method involves using PBS containing 20% DMSO and 8% human serum albumin, or culture media containing 10% Dextran 40 and 5% Dextrose, 20% Human Serum Albumin and 7.5% DMSO, or 31.25% Plasmalyte-A, 31.25% Dextrose 5%, 0.45% NaCl, 10% Dextran 40 and 5% Dextrose, 20% Human Serum Albumin, and 7.5% DMSO or other suitable cell freezing media containing for example, Hespan and PlasmaLyte A, the cells then are frozen to -80°C at a rate of 1° per minute and stored in the vapor phase of a liquid nitrogen storage tank. Other methods of controlled freezing may be used as well as uncontrolled freezing immediately at -20° C or in liquid nitrogen.

In certain aspects, cryopreserved cells are thawed and washed as described herein and allowed to rest for one hour at room temperature prior to activation using the methods of the present disclosure.

Also contemplated in the context of the invention is the collection of blood samples or apheresis product from a subject at a time period prior to when the expanded cells as described herein might be needed. As such, the source of the cells to be expanded can be collected at any time point necessary, and desired cells, such as T cells, isolated and frozen for later use in immune effector cell therapy for any number of diseases or conditions that would benefit from immune effector cell therapy, such as those described herein. In one aspect a blood sample or an apheresis is taken from a generally healthy subject. In certain aspects, a blood sample or an apheresis is taken from a generally healthy subject who is at risk of developing a disease, but who has not yet developed a disease, and the cells of interest are isolated and frozen for later use. In certain aspects, the T cells may be expanded, frozen, and used at a later time. In certain aspects, samples are collected from a patient shortly after diagnosis of a particular disease as described herein but prior to any treatments. In a further aspect, the cells are isolated from a blood sample or an apheresis from a subject prior to any number of relevant treatment modalities, including but not limited to treatment with agents such as natalizumab, efalizumab, antiviral agents, chemotherapy, radiation, immunosuppressive agents, such as cyclosporin, azathioprine, methotrexate, mycophenolate, and FK506, antibodies, or other immunoablative agents such as CAMPATH, anti-CD3 antibodies, cytoxan, fludarabine, cyclosporin, FK506, rapamycin, mycophenolic acid, steroids, FR901228, and irradiation.

In a further aspect of the present disclosure, T cells are obtained from a patient directly following treatment that leaves the subject with functional T cells. In this regard, it has been observed that following certain cancer treatments, in particular treatments with drugs that damage the immune system, shortly after treatment during the period when patients would normally be recovering from the treatment, the quality of T cells obtained may be optimal or improved for their ability to expand ex vivo. Likewise, following ex vivo manipulation using the methods described herein, these cells may be in a preferred state for enhanced engraftment and in vivo expansion. Thus, it is contemplated within the context of the present disclosure to collect blood cells, including T cells, dendritic cells, or other cells of the hematopoietic lineage, during this recovery phase. Further, in certain aspects, mobilization (for example, mobilization with GM-CSF) and conditioning regimens can be used to create a condition in a subject wherein repopulation, recirculation, regeneration, and/or expansion of particular cell types is favored, especially during a defined window of time following therapy. Illustrative cell types include T cells, B cells, dendritic cells, and other cells of the immune system.

In one instance, the immune effector cells expressing a CAR molecule, e.g., a CAR molecule described herein, are obtained from a subject that has received a low, immune enhancing dose of an mTOR inhibitor. In an instance, the population of immune effector cells, e.g., T cells, to be engineered to express a CAR, are harvested after a sufficient time, or after sufficient dosing of the low, immune enhancing, dose of an mTOR inhibitor, such that the level of PD1 negative immune effector cells, e.g., T cells, or the ratio of PD1 negative immune effector cells, e.g., T cells/ PD1 positive immune effector cells, e.g., T cells, in the subject or harvested from the subject has been, at least transiently, increased.

In other instances, population of immune effector cells, e.g., T cells, which have, or will be engineered to express a CAR, can be treated ex vivo by contact with an amount of an mTOR inhibitor that increases the number of PD1 negative immune effector cells, e.g., T cells or increases the ratio of PD1 negative immune effector cells, e.g., T cells/ PD1 positive immune effector cells, e.g., T cells.

In one embodiment, a T cell population is diaglycerol kinase (DGK)-deficient. DGK-deficient cells include cells that do not express DGK RNA or protein, or have reduced or inhibited DGK activity. DGK-deficient cells can be generated by genetic approaches, e.g., administering RNA-interfering agents, e.g., siRNA, shRNA, miRNA, to reduce or prevent DGK expression. Alternatively, DGK-deficient cells can be generated by treatment with DGK inhibitors described herein.

In one embodiment, a T cell population is Ikaros-deficient. Ikaros-deficient cells include cells that do not express Ikaros RNA or protein, or have reduced or inhibited Ikaros activity, Ikaros-deficient cells can be generated by genetic approaches, e.g., administering RNA-interfering agents, e.g., siRNA, shRNA, miRNA, to reduce or prevent Ikaros expression. Alternatively, Ikaros-deficient cells can be generated by treatment with Ikaros inhibitors, e.g., lenalidomide.

In embodiments, a T cell population is DGK-deficient and Ikaros-deficient, e.g., does not express DGK and Ikaros, or has reduced or inhibited DGK and Ikaros activity. Such DGK and Ikaros-deficient cells can be generated by any of the methods described herein.

In an embodiment, the NK cells are obtained from the subject. In another embodiment, the NK cells are an NK cell line, e.g., NK-92 cell line (Conkwest).

### Allogeneic CAR

In embodiments described herein, the immune effector cell can be an allogeneic immune effector cell, e.g., T cell or NK cell. For example, the cell can be an allogeneic T cell, e.g., an allogeneic T cell lacking expression of a functional T cell receptor (TCR) and/or human leukocyte antigen (HLA), e.g., HLA class I and/or HLA class II.

A T cell lacking a functional TCR can be, e.g., engineered such that it does not express any functional TCR on its surface, engineered such that it does not express one or more subunits that comprise a functional TCR or engineered such that it produces very little functional TCR on its surface. Alternatively, the T cell can express a substantially impaired TCR, e.g., by expression of mutated or truncated forms of one or more of the subunits of the TCR. The term "substantially impaired TCR" means that this TCR will not elicit an adverse immune reaction in a host.

A T cell described herein can be, e.g., engineered such that it does not express a functional HLA on its surface. For example, a T cell described herein, can be engineered such that cell surface expression HLA, e.g., HLA class 1 and/or HLA class II, is downregulated.

In some embodiments, the T cell can lack a functional TCR and a functional HLA, e.g., HLA class I and/or HLA class II.

Modified T cells that lack expression of a functional TCR and/or HLA can be obtained by any suitable means, including a knock out or knock down of one or more subunit of TCR or HLA. For example, the T cell can include a knock down of TCR and/or HLA using siRNA, shRNA, clustered regularly interspaced short palindromic repeats (CRISPR) transcription-activator like effector nuclease (TALEN), or zinc finger endonuclease (ZFN).

In some embodiments, the allogeneic cell can be a cell which does not express or expresses at low levels an inhibitory molecule, e.g. by any mehod described herein. For example, the cell can be a cell that does not express or expresses at low levels an inhibitory molecule, e.g., that can decrease the ability of a CAR-expressing cell to mount an immune effector response. Examples of inhibitory molecules include PD1, PD-L1, CTLA4, TIM3, CEACAM (e.g., CEACAM-1, CEACAM-3 and/or CEACAM-5), LAG3, VISTA, BTLA, TIGIT, LAIR1, CD160, 2B4 and TGFR beta. Inhibition of an inhibitory molecule, e.g., by inhibition at the DNA, RNA or protein level, can optimize a CAR-expressing cell performance. In embodiments, an inhibitory nucleic acid, e.g., an inhibitory nucleic acid, e.g., a dsRNA, e.g., an siRNA or shRNA, a clustered regularly interspaced short palindromic repeats (CRISPR), a transcription-activator like effector nuclease (TALEN), or a zinc finger endonuclease (ZFN), e.g., as described herein, can be used.

### siRNA and shRNA to inhibit TCR or HLA

In some embodiments, TCR expression and/or HLA expression can be inhibited using siRNA or shRNA that targets a nucleic acid encoding a TCR and/or HLA in a T cell.

Expression of siRNA and shRNAs in T cells can be achieved using any conventional expression system, e.g., such as a lentiviral expression system.

Exemplary shRNAs that downregulate expression of components of the TCR are described, e.g., in US Publication No.: 2012/0321667. Exemplary siRNA and shRNA that downregulate expression of HLA class I and/or HLA class II genes are described, e.g., in U.S. publication No.: US 2007/0036773.

### CRISPR to inhibit TCR or HLA

"CRISPR" or "CRISPR to TCR and/or HLA" or "CRISPR to inhibit TCR and/or HLA" as used herein refers to a set of clustered regularly interspaced short palindromic repeats, or a system comprising such a set of repeats. "Cas", as used herein, refers to a CRISPR-associated protein. A "CRISPR/Cas" system refers to a system derived from CRISPR and Cas which can be used to silence or mutate a TCR and/or HLA gene.

Naturally-occurring CRISPR/Cas systems are found in approximately 40% of sequenced eubacteria genomes and 90% of sequenced archaea. Grissa et al. (2007) BMC Bioinformatics 8: 172. This system is a type of prokaryotic immune system that confers resistance to foreign genetic elements such as plasmids and phages and provides a form of acquired immunity. Barrangou et al. (2007) Science 315: 1709-1712; Marragini et al. ( 2008) Science 322: 1843-1845.

The CRISPR/Cas system has been modified for use in gene editing (silencing, enhancing or changing specific genes) in eukaryotes such as mice or primates. Wiedenheft et al. (2012) Nature 482: 331-8. This is accomplished by introducing into the eukaryotic cell a plasmid containing a specifically designed CRISPR and one or more appropriate Cas.

The CRISPR sequence, sometimes called a CRISPR locus, comprises alternating repeats and spacers. In a naturally-occurring CRISPR, the spacers usually comprise sequences foreign to the bacterium such as a plasmid or phage sequence; in the TCR and/or HLA CRISPR/Cas system, the spacers are derived from the TCR or HLA gene sequence.

RNA from the CRISPR locus is constitutively expressed and processed by Cas proteins into small RNAs. These comprise a spacer flanked by a repeat sequence. The RNAs guide other Cas proteins to silence exogenous genetic elements at the RNA or DNA level. Horvath et al. (2010) Science 327: 167-170; Makarova et al. (2006) Biology Direct 1: 7. The spacers thus serve as templates for RNA molecules, analogously to siRNAs. Pennisi (2013) Science 341: 833-836.

As these naturally occur in many different types of bacteria, the exact arrangements of the CRISPR and structure, function and number of Cas genes and their product differ somewhat from species to species. Haft et al. (2005) PLoS Comput. Biol. 1: e60; Kunin et al. (2007) Genome Biol. 8: R61; Mojica et al. (2005) J. Mol. Evol. 60: 174-182; Bolotin et al. (2005) Microbiol. 151: 2551-2561; Pourcel et al. (2005) Microbiol. 151: 653-663; and Stern et al. (2010) Trends. Genet. 28: 335-340. For example, the Cse (Cas subtype, E. coli) proteins (e.g., CasA) form a functional complex, Cascade, that processes CRISPR RNA transcripts into spacer-repeat units that Cascade retains. Brouns et al. (2008) Science 321: 960-964. In other prokaryotes, Cas6 processes the CRISPR transcript. The CRISPR-based phage inactivation in E. coli requires Cascade and Cas3, but not Cas1 or Cas2. The Cmr (Cas RAMP module) proteins in Pyrococcus furiosus and other prokaryotes form a functional complex with small CRISPR RNAs that recognizes and cleaves complementary target RNAs. A simpler CRISPR system relies on the protein Cas9, which is a nuclease with two active cutting sites, one for each strand of the double helix. Combining Cas9 and modified CRISPR locus RNA can be used in a system for gene editing. Pennisi (2013) Science 341: 833-836.

The CRISPR/Cas system can thus be used to edit a TCR and/or HLA gene (adding or deleting a basepair), or introducing a premature stop which thus decreases expression of a TCR and/or HLA. The CRISPR/Cas system can alternatively be used like RNA interference, turning off TCR and/or HLA gene in a reversible fashion. In a mammalian cell, for example, the RNA can guide the Cas protein to a TCR and/or HLA promoter, sterically blocking RNA polymerases.

Artificial CRISPR/Cas systems can be generated which inhibit TCR and/or HLA, using technology known in the art, e.g., that described in U.S. Publication No. 20140068797, and Cong (2013) Science 339: 819-823. Other artificial CRISPR/Cas systems that are known in the art may also be generated which inhibit TCR and/or HLA, e.g., that described in Tsai (2014) Nature Biotechnol., 32:6 569-576, U.S. Patent No.: 8,871,445; 8,865,406; 8,795,965; 8,771,945; and 8,697,359.

### TALEN to inhibit TCR and/or HLA

"TALEN" or "TALEN to HLA and/or TCR" or "TALEN to inhibit HLA and/or TCR" refers to a transcription activator-like effector nuclease, an artificial nuclease which can be used to edit the HLA and/or TCR gene.

TALENs are produced artificially by fusing a TAL effector DNA binding domain to a DNA cleavage domain. Transcription activator-like effects (TALEs) can be engineered to bind any desired DNA sequence, including a portion of the HLA or TCR gene. By combining an engineered TALE with a DNA cleavage domain, a restriction enzyme can be produced which is specific to any desired DNA sequence, including a HLA or TCR sequence. These can then be introduced into a cell, wherein they can be used for genome editing. Boch (2011) Nature Biotech. 29: 135-6; and Boch et al. (2009) Science 326: 1509-12; Moscou et al. (2009) Science 326: 3501.

TALEs are proteins secreted by Xanthomonas bacteria. The DNA binding domain contains a repeated, highly conserved 33-34 amino acid sequence, with the exception of the 12th and 13th amino acids. These two positions are highly variable, showing a strong correlation with specific nucleotide recognition. They can thus be engineered to bind to a desired DNA sequence.

To produce a TALEN, a TALE protein is fused to a nuclease (N), which is a wild-type or mutated Fokl endonuclease. Several mutations to Fokl have been made for its use in TALENs; these, for example, improve cleavage specificity or activity. Cermak et al. (2011) Nucl. Acids Res. 39: e82; Miller et al. (2011) Nature Biotech. 29: 143-8; Hockemeyer et al. (2011) Nature Biotech. 29: 731-734; Wood et al. (2011) Science 333: 307; Doyon et al. (2010) Nature Methods 8: 74-79; Szczepek et al. (2007) Nature Biotech. 25: 786-793; and Guo et al. (2010) J. Mol. Biol. 200: 96.

The Fokl domain functions as a dimer, requiring two constructs with unique DNA binding domains for sites in the target genome with proper orientation and spacing. Both the number of amino acid residues between the TALE DNA binding domain and the Fokl cleavage domain and the number of bases between the two individual TALEN binding sites appear to be important parameters for achieving high levels of activity. Miller et al. (2011) Nature Biotech. 29: 143-8.

A HLA or TCR TALEN can be used inside a cell to produce a double-stranded break (DSB). A mutation can be introduced at the break site if the repair mechanisms improperly repair the break via non-homologous end joining. For example, improper repair may introduce a frame shift mutation. Alternatively, foreign DNA can be introduced into the cell along with the TALEN; depending on the sequences of the foreign DNA and chromosomal sequence, this process can be used to correct a defect in the HLA or TCR gene or introduce such a defect into a wt HLA or TCR gene, thus decreasing expression of HLA or TCR.

TALENs specific to sequences in HLA or TCR can be constructed using any method known in the art, including various schemes using modular components. Zhang et al. (2011) Nature Biotech. 29: 149-53; Geibler et al. (2011) PLoS ONE 6: e19509.

### Zinc finger nuclease to inhibit HLA and/or TCR

"ZFN" or "Zinc Finger Nuclease" or "ZFN to HLA and/or TCR" or "ZFN to inhibit HLA and/or TCR" refer to a zinc finger nuclease, an artificial nuclease which can be used to edit the HLA and/or TCR gene.

Like a TALEN, a ZFN comprises a Fokl nuclease domain (or derivative thereof) fused to a DNA-binding domain. In the case of a ZFN, the DNA-binding domain comprises one or more zinc fingers. Carroll et al. (2011) Genetics Society of America 188: 773-782; and Kim et al. (1996) Proc. Natl. Acad. Sci. USA 93: 1156-1160.

A zinc finger is a small protein structural motif stabilized by one or more zinc ions. A zinc finger can comprise, for example, Cys2His2, and can recognize an approximately 3-bp sequence. Various zinc fingers of known specificity can be combined to produce multi-finger polypeptides which recognize about 6, 9, 12, 15 or 18-bp sequences. Various selection and modular assembly techniques are available to generate zinc fingers (and combinations thereof) recognizing specific sequences, including phage display, yeast one-hybrid systems, bacterial one-hybrid and two-hybrid systems, and mammalian cells.

Like a TALEN, a ZFN must dimerize to cleave DNA. Thus, a pair of ZFNs are required to target non-palindromic DNA sites. The two individual ZFNs must bind opposite strands of the DNA with their nucleases properly spaced apart. Bitinaite et al. (1998) Proc. Natl. Acad. Sci. USA 95: 10570-5.

Also like a TALEN, a ZFN can create a double-stranded break in the DNA, which can create a frame-shift mutation if improperly repaired, leading to a decrease in the expression and amount of HLA and/or TCR in a cell. ZFNs can also be used with homologous recombination to mutate in the HLA or TCR gene.

ZFNs specific to sequences in HLA AND/OR TCR can be constructed using any method known in the art. See, e.g., Provasi (2011) Nature Med. 18: 807-815; Torikai (2013) Blood 122: 1341-1349; Cathomen et al. (2008) Mol. Ther. 16: 1200-7; Guo et al. (2010) J. Mol. Biol. 400: 96; U.S. Patent Publication 2011/0158957; and U.S. Patent Publication 2012/0060230.

### Telomerase expression

While not wishing to be bound by any particular theory, in some embodiments, a therapeutic T cell has short term persistence in a patient, due to shortened telomeres in the T cell; accordingly, transfection with a telomerase gene can lengthen the telomeres of the T cell and improve persistence of the T cell in the patient. See Carl June, "Adoptive T cell therapy for cancer in the clinic", Journal of Clinical Investigation, 117:1466-1476 (2007). Thus, in an embodiment, an immune effector cell, e.g., a T cell, ectopically expresses a telomerase subunit, e.g., the catalytic subunit of telomerase, e.g., TERT, e.g., hTERT. In some aspects, this disclosure provides a method of producing a CAR-expressing cell, comprising contacting a cell with a nucleic acid encoding a telomerase subunit, e.g., the catalytic subunit of telomerase, e.g., TERT, e.g., hTERT. The cell may be contacted with the nucleic acid before, simultaneous with, or after being contacted with a construct encoding a CAR.

In one aspect, the disclosure features a method of making a population of immune effector cells (e.g., T cells, NK cells). In an instance, the method comprises: providing a population of immune effector cells (e.g., T cells or NK cells), contacting the population of immune effector cells with a nucleic acid encoding a CAR; and contacting the population of immune effector cells with a nucleic acid encoding a telomerase subunit, e.g., hTERT, under conditions that allow for CAR and telomerase expression.

In an instance, the nucleic acid encoding the telomerase subunit is DNA. In an instance, the nucleic acid encoding the telomerase subunit comprises a promoter capable of driving expression of the telomerase subunit.

In an embodiment, hTERT has the amino acid sequence of GenBank Protein ID AAC51724.1 (Meyerson et al., "hEST2, the Putative Human Telomerase Catalytic Subunit Gene, Is Up-Regulated in Tumor Cells and during Immortalization" Cell Volume 90, Issue 4, 22 August 1997, Pages 785-795) as follows:






















 (SEQ ID NO: 63)

In an embodiment, the hTERT has a sequence at least 80%, 85%, 90%, 95%, 96^, 97%, 98%, or 99% identical to the sequence of SEQ ID NO: 63. In an embodiment, the hTERT has a sequence of SEQ ID NO: 63. In an embodiment, the hTERT comprises a deletion (e.g., of no more than 5, 10, 15, 20, or 30 amino acids) at the N-terminus, the C-terminus, or both. In an embodiment, the hTERT comprises a transgenic amino acid sequence (e.g., of no more than 5, 10, 15, 20, or 30 amino acids) at the N-terminus, the C-terminus, or both.

In an embodiment, the hTERT is encoded by the nucleic acid sequence of GenBank Accession No. AF018167 (Meyerson et al., "hEST2, the Putative Human Telomerase Catalytic Subunit Gene, Is Up-Regulated in Tumor Cells and during Immortalization" Cell Volume 90, Issue 4, 22 August 1997, Pages 785-795):

1 caggcagcgt ggtcctgctg cgcacgtggg aagccctggc cccggccacc cccgcgatgc
61 cgcgcgctcc ccgctgccga gccgtgcgct ccctgctgcg cagccactac cgcgaggtgc
121 tgccgctggc cacgttcgtg cggcgcctgg ggccccaggg ctggcggctg gtgcagcgcg
181 gggacccggc ggctttccgc gcgctggtgg cccagtgcct ggtgtgcgtg ccctgggacg
241 cacggccgcc ccccgccgcc ccctccttcc gccaggtgtc ctgcctgaag gagctggtgg
301 cccgagtgct gcagaggctg tgcgagcgcg gcgcgaagaa cgtgctggcc ttcggcttcg
361 cgctgctgga cggggcccgc gggggccccc ccgaggcctt caccaccagc gtgcgcagct

421 acctgcccaa cacggtgacc gacgcactgc gggggagcgg ggcgtggggg ctgctgttgc
481 gccgcgtggg cgacgacgtg ctggttcacc tgctggcacg ctgcgcgctc tttgtgctgg
541 tggctcccag ctgcgcctac caggtgtgcg ggccgccgct gtaccagctc ggcgctgcca
601 ctcaggcccg gcccccgcca cacgctagtg gaccccgaag gcgtctggga tgcgaacggg
661 cctggaacca tagcgtcagg gaggccgggg tccccctggg cctgccagcc ccgggtgcga
721 ggaggcgcgg gggcagtgcc agccgaagtc tgccgttgcc caagaggccc aggcgtggcg
781 ctgcccctga gccggagcgg acgcccgttg ggcaggggtc ctgggcccac ccgggcagga
841 cgcgtggacc gagtgaccgt ggtttctgtg tggtgtcacc tgccagaccc gccgaagaag
901 ccacctcttt ggagggtgcg ctctctggca cgcgccactc ccacccatcc gtgggccgcc
961 agcaccacgc gggcccccca tccacatcgc ggccaccacg tccctgggac acgccttgtc
1021 ccccggtgta cgccgagacc aagcacttcc tctactcctc aggcgacaag gagcagctgc
1081 ggccctcctt cctactcagc tctctgaggc ccagcctgac tggcgctcgg aggctcgtgg
1141 agaccatctt tctgggttcc aggccctgga tgccagggac tccccgcagg ttgccccgcc
1201 tgccccagcg ctactggcaa atgcggcccc tgtttctgga gctgcttggg aaccacgcgc
1261 agtgccccta cggggtgctc ctcaagacgc actgcccgct gcgagctgcg gtcaccccag
1321 cagccggtgt ctgtgcccgg gagaagcccc agggctctgt ggcggccccc gaggaggagg
1381 acacagaccc ccgtcgcctg gtgcagctgc tccgccagca cagcagcccc tggcaggtgt
1441 acggcttcgt gcgggcctgc ctgcgccggc tggtgccccc aggcctctgg ggctccaggc
1501 acaacgaacg ccgcttcctc aggaacacca agaagttcat ctccctgggg aagcatgcca
1561 agctctcgct gcaggagctg acgtggaaga tgagcgtgcg gggctgcgct tggctgcgca
1621 ggagcccagg ggttggctgt gttccggccg cagagcaccg tctgcgtgag gagatcctgg
1681 ccaagttcct gcactggctg atgagtgtgt acgtcgtcga gctgctcagg tctttctttt
1741 atgtcacgga gaccacgttt caaaagaaca ggctcttttt ctaccggaag agtgtctgga
1801 gcaagttgca aagcattgga atcagacagc acttgaagag ggtgcagctg cgggagctgt
1861 cggaagcaga ggtcaggcag catcgggaag ccaggcccgc cctgctgacg tccagactcc
1921 gcttcatccc caagcctgac gggctgcggc cgattgtgaa catggactac gtcgtgggag
1981 ccagaacgtt ccgcagagaa aagagggccg agcgtctcac ctcgagggtg aaggcactgt
2041 tcagcgtgct caactacgag cgggcgcggc gccccggcct cctgggcgcc tctgtgctgg
2101 gcctggacga tatccacagg gcctggcgca ccttcgtgct gcgtgtgcgg gcccaggacc
2161 cgccgcctga gctgtacttt gtcaaggtgg atgtgacggg cgcgtacgac accatccccc
2221 aggacaggct cacggaggtc atcgccagca tcatcaaacc ccagaacacg tactgcgtgc
2281 gtcggtatgc cgtggtccag aaggccgccc atgggcacgt ccgcaaggcc ttcaagagcc
2341 acgtctctac cttgacagac ctccagccgt acatgcgaca gttcgtggct cacctgcagg
2401 agaccagccc gctgagggat gccgtcgtca tcgagcagag ctcctccctg aatgaggcca

2461 gcagtggcct cttcgacgtc ttcctacgct tcatgtgcca ccacgccgtg cgcatcaggg
2521 gcaagtccta cgtccagtgc caggggatcc cgcagggctc catcctctcc acgctgctct
2581 gcagcctgtg ctacggcgac atggagaaca agctgtttgc ggggattcgg cgggacgggc
2641 tgctcctgcg tttggtggat gatttcttgt tggtgacacc tcacctcacc cacgcgaaaa
2701 ccttcctcag gaccctggtc cgaggtgtcc ctgagtatgg ctgcgtggtg aacttgcgga
2761 agacagtggt gaacttccct gtagaagacg aggccctggg tggcacggct tttgttcaga
2821 tgccggccca cggcctattc ccctggtgcg gcctgctgct ggatacccgg accctggagg
2881 tgcagagcga ctactccagc tatgcccgga cctccatcag agccagtctc accttcaacc
2941 gcggcttcaa ggctgggagg aacatgcgtc gcaaactctt tggggtcttg cggctgaagt
3001 gtcacagcct gtttctggat ttgcaggtga acagcctcca gacggtgtgc accaacatct
3061 acaagatcct cctgctgcag gcgtacaggt ttcacgcatg tgtgctgcag ctcccatttc
3121 atcagcaagt ttggaagaac cccacatttt tcctgcgcgt catctctgac acggcctccc
3181 tctgctactc catcctgaaa gccaagaacg cagggatgtc gctgggggcc aagggcgccg
3241 ccggccctct gccctccgag gccgtgcagt ggctgtgcca ccaagcattc ctgctcaagc
3301 tgactcgaca ccgtgtcacc tacgtgccac tcctggggtc actcaggaca gcccagacgc
3361 agctgagtcg gaagctcccg gggacgacgc tgactgccct ggaggccgca gccaacccgg
3421 cactgccctc agacttcaag accatcctgg actgatggcc acccgcccac agccaggccg
3481 agagcagaca ccagcagccc tgtcacgccg ggctctacgt cccagggagg gaggggcggc
3541 ccacacccag gcccgcaccg ctgggagtct gaggcctgag tgagtgtttg gccgaggcct
3601 gcatgtccgg ctgaaggctg agtgtccggc tgaggcctga gcgagtgtcc agccaagggc
3661 tgagtgtcca gcacacctgc cgtcttcact tccccacagg ctggcgctcg gctccacccc
3721 agggccagct tttcctcacc aggagcccgg cttccactcc ccacatagga atagtccatc
3781 cccagattcg ccattgttca cccctcgccc tgccctcctt tgccttccac ccccaccatc
3841 caggtggaga ccctgagaag gaccctggga gctctgggaa tttggagtga ccaaaggtgt
3901 gccctgtaca caggcgagga ccctgcacct ggatgggggt ccctgtgggt caaattgggg
3961 ggaggtgctg tgggagtaaa atactgaata tatgagtttt tcagttttga aaaaaaaaaa
4021 aaaaaaa (SEQ ID NO: 64)

In an embodiment, the hTERT is encoded by a nucleic acid having a sequence at least 80%, 85%, 90%, 95%, 96, 97%, 98%, or 99% identical to the sequence of SEQ ID NO: 64. In an embodiment, the hTERT is encoded by a nucleic acid of SEQ ID NO: 64.

### Activation and Expansion of Immune Effector Cells (e.g., T Cells)

Immune effector cells such as T cells may be activated and expanded generally using methods as described, for example, in U.S. Patents 6,352,694; 6,534,055; 6,905,680; 6,692,964; 5,858,358; 6,887,466; 6,905,681; 7,144,575; 7,067,318; 7,172,869; 7,232,566; 7,175,843; 5,883,223; 6,905,874; 6,797,514; 6,867,041; and U.S. Patent Application Publication No. 20060121005.

Generally, a population of immune effector cells e.g., T regulatory cell depleted cells, may be expanded by contact with a surface having attached thereto an agent that stimulates a CD3/TCR complex associated signal and a ligand that stimulates a costimulatory molecule on the surface of the T cells. In particular, T cell populations may be stimulated as described herein, such as by contact with an anti-CD3 antibody, or antigen-binding fragment thereof, or an anti-CD2 antibody immobilized on a surface, or by contact with a protein kinase C activator (e.g., bryostatin) in conjunction with a calcium ionophore. For co-stimulation of an accessory molecule on the surface of the T cells, a ligand that binds the accessory molecule is used. For example, a population of T cells can be contacted with an anti-CD3 antibody and an anti-CD28 antibody, under conditions appropriate for stimulating proliferation of the T cells. To stimulate proliferation of either CD4+ T cells or CD8+ T cells, an anti-CD3 antibody and an anti-CD28 antibody can be used. Examples of an anti-CD28 antibody include 9.3, B-T3, XR-CD28 (Diaclone, Besançon, France) can be used as can other methods commonly known in the art (Berg et al., Transplant Proc. 30(8):3975-3977, 1998; Haanen et al., J. Exp. Med. 190(9):13191328, 1999; Garland et al., J. Immunol Meth. 227(1-2):53-63, 1999).

In certain aspects, the primary stimulatory signal and the costimulatory signal for the T cell may be provided by different protocols. For example, the agents providing each signal may be in solution or coupled to a surface. When coupled to a surface, the agents may be coupled to the same surface (i.e., in "cis" formation) or to separate surfaces (i.e., in "trans" formation). Alternatively, one agent may be coupled to a surface and the other agent in solution. In one aspect, the agent providing the costimulatory signal is bound to a cell surface and the agent providing the primary activation signal is in solution or coupled to a surface. In certain aspects, both agents can be in solution. In one aspect, the agents may be in soluble form, and then cross-linked to a surface, such as a cell expressing Fc receptors or an antibody or other binding agent which will bind to the agents. In this regard, see for example, U.S. Patent Application Publication Nos. 20040101519 and 20060034810 for artificial antigen presenting cells (aAPCs) that are contemplated for use in activating and expanding T cells in the present disclosure.

In one aspect, the two agents are immobilized on beads, either on the same bead, i.e., "cis," or to separate beads, i.e., "trans." By way of example, the agent providing the primary activation signal is an anti-CD3 antibody or an antigen-binding fragment thereof and the agent providing the costimulatory signal is an anti-CD28 antibody or antigen-binding fragment thereof; and both agents are co-immobilized to the same bead in equivalent molecular amounts. In one aspect, a 1:1 ratio of each antibody bound to the beads for CD4+ T cell expansion and T cell growth is used. In certain aspects of the present disclosure, a ratio of anti CD3:CD28 antibodies bound to the beads is used such that an increase in T cell expansion is observed as compared to the expansion observed using a ratio of 1:1. In one particular aspect an increase of from about 1 to about 3 fold is observed as compared to the expansion observed using a ratio of 1:1. In one aspect, the ratio of CD3 :CD28 antibody bound to the beads ranges from 100:1 to 1:100 and all integer values there between. In one aspect, more anti-CD28 antibody is bound to the particles than anti-CD3 antibody, i.e., the ratio of CD3:CD28 is less than one. In certain aspects, the ratio of anti CD28 antibody to anti CD3 antibody bound to the beads is greater than 2:1. In one particular aspect, a 1:100 CD3:CD28 ratio of antibody bound to beads is used. In one aspect, a 1:75 CD3:CD28 ratio of antibody bound to beads is used. In a further aspect, a 1:50 CD3:CD28 ratio of antibody bound to beads is used. In one aspect, a 1:30 CD3:CD28 ratio of antibody bound to beads is used. In one preferred aspect, a 1:10 CD3 :CD28 ratio of antibody bound to beads is used. In one aspect, a 1:3 CD3:CD28 ratio of antibody bound to the beads is used. In yet one aspect, a 3:1 CD3 :CD28 ratio of antibody bound to the beads is used.

Ratios of particles to cells from 1:500 to 500:1 and any integer values in between may be used to stimulate T cells or other target cells. As those of ordinary skill in the art can readily appreciate, the ratio of particles to cells may depend on particle size relative to the target cell. For example, small sized beads could only bind a few cells, while larger beads could bind many. In certain aspects the ratio of cells to particles ranges from 1:100 to 100:1 and any integer values in-between and in further aspects the ratio comprises 1:9 to 9:1 and any integer values in between, can also be used to stimulate T cells. The ratio of anti-CD3- and anti-CD28-coupled particles to T cells that result in T cell stimulation can vary as noted above, however certain preferred values include 1:100, 1:50, 1:40, 1:30, 1:20, 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1, and 15:1 with one preferred ratio being at least 1:1 particles per T cell. In one aspect, a ratio of particles to cells of 1:1 or less is used. In one particular aspect, a preferred particle: cell ratio is 1:5. In further aspects, the ratio of particles to cells can be varied depending on the day of stimulation. For example, in one aspect, the ratio of particles to cells is from 1:1 to 10:1 on the first day and additional particles are added to the cells every day or every other day thereafter for up to 10 days, at final ratios of from 1:1 to 1:10 (based on cell counts on the day of addition). In one particular aspect, the ratio of particles to cells is 1:1 on the first day of stimulation and adjusted to 1:5 on the third and fifth days of stimulation. In one aspect, particles are added on a daily or every other day basis to a final ratio of 1:1 on the first day, and 1:5 on the third and fifth days of stimulation. In one aspect, the ratio of particles to cells is 2:1 on the first day of stimulation and adjusted to 1:10 on the third and fifth days of stimulation. In one aspect, particles are added on a daily or every other day basis to a final ratio of 1:1 on the first day, and 1:10 on the third and fifth days of stimulation. One of skill in the art will appreciate that a variety of other ratios may be suitable for use in the present disclosure. In particular, ratios will vary depending on particle size and on cell size and type. In one aspect, the most typical ratios for use are in the neighborhood of 1:1, 2:1 and 3:1 on the first day.

In further aspects, the cells, such as T cells, are combined with agent-coated beads, the beads and the cells are subsequently separated, and then the cells are cultured. In an alternative aspect, prior to culture, the agent-coated beads and cells are not separated but are cultured together. In a further aspect, the beads and cells are first concentrated by application of a force, such as a magnetic force, resulting in increased ligation of cell surface markers, thereby inducing cell stimulation.

By way of example, cell surface proteins may be ligated by allowing paramagnetic beads to which anti-CD3 and anti-CD28 are attached (3x28 beads) to contact the T cells. In one aspect the cells (for example, 10⁴ to 10⁹ T cells) and beads (for example, DYNABEADS^{®} M-450 CD3/CD28 T paramagnetic beads at a ratio of 1:1) are combined in a buffer, for example PBS (without divalent cations such as, calcium and magnesium). Again, those of ordinary skill in the art can readily appreciate any cell concentration may be used. For example, the target cell may be very rare in the sample and comprise only 0.01% of the sample or the entire sample (i.e., 100%) may comprise the target cell of interest. Accordingly, any cell number is within the context of the present disclosure. In certain aspects, it may be desirable to significantly decrease the volume in which particles and cells are mixed together (i.e., increase the concentration of cells), to ensure maximum contact of cells and particles. For example, in one aspect, a concentration of about 10 billion cells/ml, 9 billion/ml, 8 billion/ml, 7 billion/ml, 6 billion/ml, 5 billion/ml, or 2 billion cells/ml is used. In one aspect, greater than 100 million cells/ml is used. In a further aspect, a concentration of cells of 10, 15, 20, 25, 30, 35, 40, 45, or 50 million cells/ml is used. In yet one aspect, a concentration of cells from 75, 80, 85, 90, 95, or 100 million cells/ml is used. In further aspects, concentrations of 125 or 150 million cells/ml can be used. Using high concentrations can result in increased cell yield, cell activation, and cell expansion. Further, use of high cell concentrations allows more efficient capture of cells that may weakly express target antigens of interest, such as CD28-negative T cells. Such populations of cells may have therapeutic value and would be desirable to obtain in certain aspects. For example, using high concentration of cells allows more efficient selection of CD8+ T cells that normally have weaker CD28 expression.

In one instance, cells transduced with a nucleic acid encoding a CAR, e.g., a CAR described herein, are expanded, e.g., by a method described herein. In one instance, the cells are expanded in culture for a period of several hours (e.g., about 2, 3, 4, 5, 6, 7, 8, 9, 10, 15, 18, 21 hours) to about 14 days (e.g., 1, 2, 3, 4, 5, 6, 7, 8, 9, 10, 11, 12, 13 or 14 days). In one instance, the cells are expanded for a period of 4 to 9 days. In one instance, the cells are expanded for a period of 8 days or less, e.g., 7, 6 or 5 days. In one instance, the cells, e.g., a CD19 CAR cell described herein, are expanded in culture for 5 days, and the resulting cells are more potent than the same cells expanded in culture for 9 days under the same culture conditions. Potency can be defined, e.g., by various T cell functions, e.g. proliferation, target cell killing, cytokine production, activation, migration, or combinations thereof. In one instance, the cells, e.g., a CD19 CAR cell described herein, expanded for 5 days show at least a one, two, three or four fold increase in cells doublings upon antigen stimulation as compared to the same cells expanded in culture for 9 days under the same culture conditions. In one instance, the cells, e.g., the cells expressing a CD19 CAR described herein, are expanded in culture for 5 days, and the resulting cells exhibit higher proinflammatory cytokine production, e.g., IFN-γ and/or GM-CSF levels, as compared to the same cells expanded in culture for 9 days under the same culture conditions. In one instance, the cells, e.g., a CD19 CAR cell described herein, expanded for 5 days show at least a one, two, three, four, five, ten fold or more increase in pg/ml of proinflammatory cytokine production, e.g., IFN-γ and/or GM-CSF levels, as compared to the same cells expanded in culture for 9 days under the same culture conditions.

Several cycles of stimulation may also be desired such that culture time of T cells can be 60 days or more. Conditions appropriate for T cell culture include an appropriate media (e.g., Minimal Essential Media or RPMI Media 1640 or, X-vivo 15, (Lonza)) that may contain factors necessary for proliferation and viability, including serum (e.g., fetal bovine or human serum), interleukin-2 (IL-2), insulin, IFN-γ, IL-4, IL-7, GM-CSF, IL-10, IL-12, IL-15, TGFβ, and TNF-α or any other additives for the growth of cells known to the skilled artisan. Other additives for the growth of cells include, but are not limited to, surfactant, plasmanate, and reducing agents such as N-acetyl-cysteine and 2-mercaptoethanol. Media can include RPMI 1640, AIM-V, DMEM, MEM, α-MEM, F-12, X-Vivo 15, and X-Vivo 20, Optimizer, with added amino acids, sodium pyruvate, and vitamins, either serum-free or supplemented with an appropriate amount of serum (or plasma) or a defined set of hormones, and/or an amount of cytokine(s) sufficient for the growth and expansion of T cells. Antibiotics, e.g., penicillin and streptomycin, are included only in experimental cultures, not in cultures of cells that are to be infused into a subject. The target cells are maintained under conditions necessary to support growth, for example, an appropriate temperature (e.g., 37° C) and atmosphere (e.g., air plus 5% CO₂).

In one instance, the cells are expanded in an appropriate media (e.g., media described herein) that includes one or more interleukin that result in at least a 200-fold (e.g., 200-fold, 250-fold, 300-fold, 350-fold) increase in cells over a 14 day expansion period, e.g., as measured by a method described herein such as flow cytometry. In one instance, the cells are expanded in the presence of IL-15 and/or IL-7 (e.g., IL-15 and IL-7).

In instances, methods described herein, e.g., CAR-expressing cell manufacturing methods, comprise removing T regulatory cells, e.g., CD25+ T cells, from a cell population, e.g., using an anti-CD25 antibody, or fragment thereof, or a CD25-binding ligand, IL-2. Methods of removing T regulatory cells, e.g., CD25+ T cells, from a cell population are described herein. In instances, the methods, e.g., manufacturing methods, further comprise contacting a cell population (e.g., a cell population in which T regulatory cells, such as CD25+ T cells, have been depleted; or a cell population that has previously contacted an anti-CD25 antibody, fragment thereof, or CD25-binding ligand) with IL-15 and/or IL-7. For example, the cell population (e.g., that has previously contacted an anti-CD25 antibody, fragment thereof, or CD25-binding ligand) is expanded in the presence of IL-15 and/or IL-7.

In some instances a CAR-expressing cell described herein is contacted with a composition comprising a interleukin-15 (IL-15) polypeptide, a interleukin-15 receptor alpha (IL-15Ra) polypeptide, or a combination of both a IL-15 polypeptide and a IL-15Ra polypeptide e.g., hetIL-15, during the manufacturing of the CAR-expressing cell, e.g., ex vivo. In instances, a CAR-expressing cell described herein is contacted with a composition comprising a IL-15 polypeptide during the manufacturing of the CAR-expressing cell, e.g., ex vivo. In instances, a CAR-expressing cell described herein is contacted with a composition comprising a combination of both a IL-15 polypeptide and a IL-15 Ra polypeptide during the manufacturing of the CAR-expressing cell, e.g., ex vivo. In instances, a CAR-expressing cell described herein is contacted with a composition comprising hetIL-15 during the manufacturing of the CAR-expressing cell, e.g., ex vivo.

In one instance the CAR-expressing cell described herein is contacted with a composition comprising hetIL-15 during ex vivo expansion. In an instance, the CAR-expressing cell described herein is contacted with a composition comprising an IL-15 polypeptide during ex vivo expansion. In an instance, the CAR-expressing cell described herein is contacted with a composition comprising both an IL-15 polypeptide and an IL-15Ra polypeptide during ex vivo expansion. In one instance the contacting results in the survival and proliferation of a lymphocyte subpopulation, e.g., CD8+ T cells.

T cells that have been exposed to varied stimulation times may exhibit different characteristics. For example, typical blood or apheresed peripheral blood mononuclear cell products have a helper T cell population (TH, CD4+) that is greater than the cytotoxic or suppressor T cell population (TC, CD8+). Ex vivo expansion of T cells by stimulating CD3 and CD28 receptors produces a population of T cells that prior to about days 8-9 consists predominately of TH cells, while after about days 8-9, the population of T cells comprises an increasingly greater population of TC cells. Accordingly, depending on the purpose of treatment, infusing a subject with a T cell population comprising predominately of TH cells may be advantageous. Similarly, if an antigen-specific subset of TC cells has been isolated it may be beneficial to expand this subset to a greater degree.

Further, in addition to CD4 and CD8 markers, other phenotypic markers vary significantly, but in large part, reproducibly during the course of the cell expansion process. Thus, such reproducibility enables the ability to tailor an activated T cell product for specific purposes.

Once a CAR described herein is constructed, various assays can be used to evaluate the activity of the molecule, such as but not limited to, the ability to expand T cells following antigen stimulation, sustain T cell expansion in the absence of re-stimulation, and anti-cancer activities in appropriate in vitro and animal models. Assays to evaluate the effects of a cars of the present disclosure are described in further detail below

Western blot analysis of CAR expression in primary T cells can be used to detect the presence of monomers and dimers. See, *e.g.,* Milone et al., Molecular Therapy 17(8): 1453-1464 (2009). Very briefly, T cells (1:1 mixture of CD4⁺ and CD8⁺ T cells) expressing the CARs are expanded *in vitro* for more than 10 days followed by lysis and SDS-PAGE under reducing conditions. CARs containing the full length TCR-ζ cytoplasmic domain and the endogenous TCR-ζ chain are detected by western blotting using an antibody to the TCR-ζ chain. The same T cell subsets are used for SDS-PAGE analysis under non-reducing conditions to permit evaluation of covalent dimer formation.

*In vitro* expansion of CAR⁺ T cells following antigen stimulation can be measured by flow cytometry. For example, a mixture of CD4⁺ and CD8⁺ T cells are stimulated with αCD3/αCD28 aAPCs followed by transduction with lentiviral vectors expressing GFP under the control of the promoters to be analyzed. Exemplary promoters include the CMV IE gene, EF-1α, ubiquitin C, or phosphoglycerokinase (PGK) promoters. GFP fluorescence is evaluated on day 6 of culture in the CD4⁺ and/or CD8⁺ T cell subsets by flow cytometry. See, *e.g.,* Milone et al., Molecular Therapy 17(8): 1453-1464 (2009). Alternatively, a mixture of CD4⁺ and CD8⁺ T cells are stimulated with αCD3/αCD28 coated magnetic beads on day 0, and transduced with CAR on day 1 using a bicistronic lentiviral vector expressing CAR along with eGFP using a 2A ribosomal skipping sequence. Cultures are re-stimulated with either a cancer associated antigen as described herein⁺ K562 cells (K562 expressing a cancer associated antigen as described herein), wild-type K562 cells (K562 wild type) or K562 cells expressing hCD32 and 4-1BBL in the presence of antiCD3 and anti-CD28 antibody (K562-BBL-3/28) following washing. Exogenous IL-2 is added to the cultures every other day at 100 IU/ml. GFP⁺ T cells are enumerated by flow cytometry using bead-based counting. See, *e.g.,* Milone et al., Molecular Therapy 17(8): 1453-1464 (2009).

Sustained CAR⁺ T cell expansion in the absence of re-stimulation can also be measured. See, *e.g.,* Milone et al., Molecular Therapy 17(8): 1453-1464 (2009). Briefly, mean T cell volume (fl) is measured on day 8 of culture using a Coulter Multisizer III particle counter, a Nexcelom Cellometer Vision or Millipore Scepter, following stimulation with αCD3/αCD28 coated magnetic beads on day 0, and transduction with the indicated CAR on day 1.

Animal models can also be used to measure a CART activity. For example, xenograft model using human a cancer associated antigen described herein-specific CAR⁺ T cells to treat a primary human pre-B ALL in immunodeficient mice can be used. See, *e.g.,* Milone et al., Molecular Therapy 17(8): 1453-1464 (2009). Very briefly, after establishment of ALL, mice are randomized as to treatment groups. Different numbers of a cancer associated antigen -specific CARengineered T cells are coinjected at a 1:1 ratio into NOD-SCID-γ^{-/-} mice bearing B-ALL. The number of copies of a cancer associated antigen -specific CAR vector in spleen DNA from mice is evaluated at various times following T cell injection. Animals are assessed for leukemia at weekly intervals. Peripheral blood a cancer associate antigen as described herein⁺ B-ALL blast cell counts are measured in mice that are injected with a cancer associated antigen described herein-ζ CAR⁺ T cells or mock-transduced T cells. Survival curves for the groups are compared using the log-rank test. In addition, absolute peripheral blood CD4⁺ and CD8⁺ T cell counts 4 weeks following T cell injection in NOD-SCID-γ^{-/-} mice can also be analyzed. Mice are injected with leukemic cells and 3 weeks later are injected with T cells engineered to express CAR by a bicistronic lentiviral vector that encodes the CAR linked to eGFP. T cells are normalized to 45-50% input GFP⁺ T cells by mixing with mock-transduced cells prior to injection, and confirmed by flow cytometry. Animals are assessed for leukemia at 1-week intervals. Survival curves for the CAR⁺ T cell groups are compared using the log-rank test.

Dose dependent CAR treatment response can be evaluated. See, *e.g.,* Milone et al., Molecular Therapy 17(8): 1453-1464 (2009). For example, peripheral blood is obtained 35-70 days after establishing leukemia in mice injected on day 21 with CAR T cells, an equivalent number of mock-transduced T cells, or no T cells. Mice from each group are randomly bled for determination of peripheral blood a cancer associate antigen as described herein⁺ ALL blast counts and then killed on days 35 and 49. The remaining animals are evaluated on days 57 and 70.

Assessment of cell proliferation and cytokine production has been previously described, *e.g.,* at Milone et al., Molecular Therapy 17(8): 1453-1464 (2009). Briefly, assessment of CAR-mediated proliferation is performed in microtiter plates by mixing washed T cells with K562 cells expressing a cancer associated antigen described herein (K19) or CD32 and CD137 (KT32-BBL) for a final T-cell:K562 ratio of 2:1. K562 cells are irradiated with gamma-radiation prior to use. Anti-CD3 (clone OKT3) and anti- CD28 (clone 9.3) monoclonal antibodies are added to cultures with KT32-BBL cells to serve as a positive control for stimulating T-cell proliferation since these signals support long-term CD8⁺ T cell expansion *ex vivo.* T cells are enumerated in cultures using CountBright^{™} fluorescent beads (Invitrogen, Carlsbad, CA) and flow cytometry as described by the manufacturer. CAR⁺ T cells are identified by GFP expression using T cells that are engineered with eGFP-2A linked CAR-expressing lentiviral vectors. For CAR+ T cells not expressing GFP, the CAR+ T cells are detected with biotinylated recombinant a cancer associate antigen as described herein protein and a secondary avidin-PE conjugate. CD4+ and CD8⁺ expression on T cells are also simultaneously detected with specific monoclonal antibodies (BD Biosciences). Cytokine measurements are performed on supernatants collected 24 hours following re-stimulation using the human TH1/TH2 cytokine cytometric bead array kit (BD Biosciences, San Diego, CA) according the manufacturer's instructions. Fluorescence is assessed using a FACScalibur flow cytometer, and data is analyzed according to the manufacturer's instructions.

Cytotoxicity can be assessed by a standard 51Cr-release assay. See, *e.g.,* Milone et al., Molecular Therapy 17(8): 1453-1464 (2009). Briefly, target cells (K562 lines and primary pro-B-ALL cells) are loaded with 51Cr (as NaCrO4, New England Nuclear, Boston, MA) at 37°C for 2 hours with frequent agitation, washed twice in complete RPMI and plated into microtiter plates. Effector T cells are mixed with target cells in the wells in complete RPMI at varying ratios of effector cell:target cell (E:T). Additional wells containing media only (spontaneous release, SR) or a 1% solution of triton-X 100 detergent (total release, TR) are also prepared. After 4 hours of incubation at 37°C, supernatant from each well is harvested. Released 51Cr is then measured using a gamma particle counter (Packard Instrument Co., Waltham, MA). Each condition is performed in at least triplicate, and the percentage of lysis is calculated using the formula: % Lysis = (ER- SR) / (TR - SR), where ER represents the average 51Cr released for each experimental condition.

Imaging technologies can be used to evaluate specific trafficking and proliferation of CARs in tumor-bearing animal models. Such assays have been described, for example, in Barrett et al., Human Gene Therapy 22:1575-1586 (2011). Briefly, NOD/SCID/γc^{-/-} (NSG) mice are injected IV with Nalm-6 cells followed 7 days later with T cells 4 hour after electroporation with the CAR constructs. The T cells are stably transfected with a lentiviral construct to express firefly luciferase, and mice are imaged for bioluminescence. Alternatively, therapeutic efficacy and specificity of a single injection of CAR⁺ T cells in Nalm-6 xenograft model can be measured as the following: NSG mice are injected with Nalm-6 transduced to stably express firefly luciferase, followed by a single tail-vein injection of T cells electroporated with cars of the present disclosure 7 days later. Animals are imaged at various time points post injection. For example, photon-density heat maps of firefly luciferasepositive leukemia in representative mice at day 5 (2 days before treatment) and day 8 (24 hr post CAR⁺PBLs) can be generated.

Other assays, including those described in the Example section herein as well as those that are known in the art can also be used to evaluate the CARs described herein.

### Therapeutic Application

In one aspect, the disclosure provides methods for treating a disease associated with expression of a cancer associated antigen described herein.

In one aspect, the present disclosure provides methods of treating cancer by providing to the subject in need thereof immune effector cells (e.g., T cells, NK cells) that are engineered to express an XCAR, wherein X represents a tumor antigen as described herein, and wherein the cancer cells express said X tumor antigen.

In one aspect, the present disclosure provides methods of treating cancer by providing to the subject in need thereof immune effector cells (e.g., T cells, NK cells) that are engineered to express a XCAR described herein, wherein the cancer cells express X. In one instance, X is expressed on both normal cells and cancers cells, but is expressed at lower levels on normal cells. In one instance, the method further comprises selecting a CAR that binds X with an affinity that allows the XCAR to bind and kill the cancer cells expressing X but less than 30%, 25%, 20%, 15%, 10%, 5% or less of the normal cells expressing X are killed, e.g., as determined by an assay described herein. For example, the assay described in Figure 13 can be used or a killing assay such as flow cytometry based on Cr51 CTL. In one instance, the selected CAR has an antigen binding domain that has a binding affinity KD of 10⁻⁴ M to 10⁻⁸ M, e.g., 10⁻⁵ M to 10⁻⁷ M, e.g., 10⁻⁶ M or 10⁻⁷ M, for the target antigen. In one instance, the selected antigen binding domain has a binding affinity that is at least five-fold, 10-fold, 20-fold, 30-fold, 50-fold, 100-fold or 1,000-fold less than a reference antibody, e.g., an antibody described herein.

In one instance, the present disclosure provides methods of treating cancer by providing to the subject in need thereof immune effector cells (e.g., T cells, NK cells) that are engineered to express CD19 CAR, wherein the cancer cells express CD19. In one instance, the cancer to be treated is ALL (acute lymphoblastic leukemia), CLL (chronic lymphocytic leukemia), DLBCL (diffuse large B-cell lymphoma), MCL (Mantle cell lymphoma, or MM (multiple myeloma).

In one aspect, the present disclosure provides methods of treating cancer by providing to the subject in need thereof immune effector cells (e.g., T cells, NK cells) that are engineered to express an EGFRvIIICAR, wherein the cancer cells express EGFRvIII. In one instance, the cancer to be treated is glioblastoma.

In one aspect, the present disclosure provides methods of treating cancer by providing to the subject in need thereof immune effector cells (e.g., T cells, NK cells) that are engineered to express a mesothelinCAR, wherein the cancer cells express mesothelin. In one instance, the cancer to be treated is mesothelioma, pancreatic cancer, or ovarian cancer.

In one aspect, the present disclosure provides methods of treating cancer by providing to the subject in need thereof immune effector cells (e.g., T cells, NK cells) that are engineered to express a CD123CAR, wherein the cancer cells express CD123. In one instance, the cancer to be treated is AML.

In one aspect, the present disclosure provides methods of treating cancer by providing to the subject in need thereof immune effector cells (e.g., T cells, NK cells) that are engineered to express a CD22CAR, wherein the cancer cells express CD22. In one instance, the cancer to be treated is B cell malignancies.

In one aspect, the present disclosure provides methods of treating cancer by providing to the subject in need thereof immune effector cells (e.g., T cells, NK cells) that are engineered to express a CS-1CAR, wherein the cancer cells express CS-1. In one instance, the cancer to be treated is multiple myeloma.

In one aspect, the present disclosure provides methods of treating cancer by providing to the subject in need thereof immune effector cells (e.g., T cells, NK cells) that are engineered to express a CLL-1CAR, wherein the cancer cells express CLL-1. In one instance, the cancer to be treated is AML.

In one aspect, the present disclosure provides methods of treating cancer by providing to the subject in need thereof immune effector cells (e.g., T cells, NK cells) that are engineered to express a CD33CAR, wherein the cancer cells express CD33. In one instance, the cancer to be treated is AML.

In one aspect, the present disclosure provides methods of treating cancer by providing to the subject in need thereof immune effector cells (e.g., T cells, NK cells) that are engineered to express a GD2CAR, wherein the cancer cells express GD2. In one instance, the cancer to be treated is neuroblastoma.

In one aspect, the present disclosure provides methods of treating cancer by providing to the subject in need thereof immune effector cells (e.g., T cells, NK cells) that are engineered to express a BCMACAR, wherein the cancer cells express BCMA. In one instance, the cancer to be treated is multiple myeloma.

In one aspect, the present disclosure provides methods of treating cancer by providing to the subject in need thereof immune effector cells (e.g., T cells, NK cells) that are engineered to express a TnCAR, wherein the cancer cells express Tn antigen. In one instance, the cancer to be treated is ovarian cancer.

In one aspect, the present disclosure provides methods of treating cancer by providing to the subject in need thereof immune effector cells (e.g., T cells, NK cells) that are engineered to express a PSMACAR, wherein the cancer cells express PSMA. In one instance, the cancer to be treated is prostate cancer.

In one aspect, the present disclosure provides methods of treating cancer by providing to the subject in need thereof immune effector cells (e.g., T cells, NK cells) that are engineered to express a ROR1CAR, wherein the cancer cells express ROR1. In one instance, the cancer to be treated is B cell malignancies.

In one aspect, the present disclosure provides methods of treating cancer by providing to the subject in need thereof immune effector cells (e.g., T cells, NK cells) that are engineered to express a FLT3 CAR, wherein the cancer cells express FLT3. In one instance, the cancer to be treated is AML.

In one aspect, the present disclosure provides methods of treating cancer by providing to the subject in need thereof immune effector cells (e.g., T cells, NK cells) that are engineered to express a TAG72CAR, wherein the cancer cells express TAG72. In one instance, the cancer to be treated is gastrointestinal cancer.

In one aspect, the present disclosure provides methods of treating cancer by providing to the subject in need thereof immune effector cells (e.g., T cells, NK cells) that are engineered to express a CD38CAR, wherein the cancer cells express CD38. In one instance, the cancer to be treated is multiple myeloma.

In one aspect, the present disclosure provides methods of treating cancer by providing to the subject in need thereof immune effector cells (e.g., T cells, NK cells) that are engineered to express a CD44v6CAR, wherein the cancer cells express CD44v6. In one instance, the cancer to be treated is cervical cancer, AML, or MM.

In one aspect, the present disclosure provides methods of treating cancer by providing to the subject in need thereof immune effector cells (e.g., T cells, NK cells) that are engineered to express a CEACAR, wherein the cancer cells express CEA. In one instance, the cancer to be treated is pastrointestinal cancer, or pancreatic cancer.

In one aspect, the present disclosure provides methods of treating cancer by providing to the subject in need thereof immune effector cells (e.g., T cells, NK cells) that are engineered to express an EPCAMCAR, wherein the cancer cells express EPCAM. In one instance, the cancer to be treated is gastrointestinal cancer.

In one aspect, the present disclosure provides methods of treating cancer by providing to the subject in need thereof immune effector cells (e.g., T cells, NK cells) that are engineered to express a B7H3CAR, wherein the cancer cells express B7H3.

In one aspect, the present disclosure provides methods of treating cancer by providing to the subject in need thereof immune effector cells (e.g., T cells, NK cells) that are engineered to express a KITCAR, wherein the cancer cells express KIT. In one instance, the cancer to be treated is gastrointestinal cancer.

In one aspect, the present disclosure provides methods of treating cancer by providing to the subject in need thereof immune effector cells (e.g., T cells, NK cells) that are engineered to express an IL-13Ra2CAR, wherein the cancer cells express IL-13Ra2. In one instance, the cancer to be treated is glioblastoma.

In one aspect, the present disclosure provides methods of treating cancer by providing to the subject in need thereof immune effector cells (e.g., T cells, NK cells) that are engineered to express a PRSS21CAR, wherein the cancer cells express PRSS21. In one instance, the cancer to be treated is selected from ovarian, pancreatic, lung and breast cancer.

In one aspect, the present disclosure provides methods of treating cancer by providing to the subject in need thereof immune effector cells (e.g., T cells, NK cells) that are engineered to express a CD30CAR, wherein the cancer cells express CD30. In one instance, the cancer to be treated is lymphomas, or leukemias.

In one aspect, the present disclosure provides methods of treating cancer by providing to the subject in need thereof immune effector cells (e.g., T cells, NK cells) that are engineered to express a GD3CAR, wherein the cancer cells express GD3. In one instance, the cancer to be treated is melanoma.

In one aspect, the present disclosure provides methods of treating cancer by providing to the subject in need thereof immune effector cells (e.g., T cells, NK cells) that are engineered to express a CD171CAR, wherein the cancer cells express CD171. In one instance, the cancer to be treated is neuroblastoma, ovarian cancer, melanoma, breast cancer, pancreatic cancer, colon cancers, or NSCLC (non-small cell lung cancer).

In one aspect, the present disclosure provides methods of treating cancer by providing to the subject in need thereof immune effector cells (e.g., T cells, NK cells) that are engineered to express an IL-11RaCAR, wherein the cancer cells express IL-11Ra. In one instance, the cancer to be treated is osteosarcoma.

In one aspect, the present disclosure provides methods of treating cancer by providing to the subject in need thereof immune effector cells (e.g., T cells, NK cells) that are engineered to express a PSCACAR, wherein the cancer cells express PSCA. In one instance, the cancer to be treated is prostate cancer.

In one aspect, the present disclosure provides methods of treating cancer by providing to the subject in need thereof immune effector cells (e.g., T cells, NK cells) that are engineered to express a VEGFR2CAR, wherein the cancer cells express VEGFR2. In one instance, the cancer to be treated is a solid tumor.

In one aspect, the present disclosure provides methods of treating cancer by providing to the subject in need thereof immune effector cells (e.g., T cells, NK cells) that are engineered to express a LewisYCAR, wherein the cancer cells express LewisY. In one instance, the cancer to be treated is ovarian cancer, or AML.

In one aspect, the present disclosure provides methods of treating cancer by providing to the subject in need thereof immune effector cells (e.g., T cells, NK cells) that are engineered to express a CD24CAR, wherein the cancer cells express CD24. In one instance, the cancer to be treated is pancreatic cancer.

In one aspect, the present disclosure provides methods of treating cancer by providing to the subject in need thereof immune effector cells (e.g., T cells, NK cells) that are engineered to express a PDGFR-betaCAR, wherein the cancer cells express PDGFR-beta. In one instance, the cancer to be treated is breast cancer, prostate cancer, GIST (gastrointestinal stromal tumor), CML, DFSP (dermatofibrosarcoma protuberans), or glioma.

In one aspect, the present disclosure provides methods of treating cancer by providing to the subject in need thereof immune effector cells (e.g., T cells, NK cells) that are engineered to express a SSEA-4CAR, wherein the cancer cells express SSEA-4. In one instance, the cancer to be treated is glioblastoma, breast cancer, lung cancer, or stem cell cancer.

In one aspect, the present disclosure provides methods of treating cancer by providing to the subject in need thereof immune effector cells (e.g., T cells, NK cells) that are engineered to express a CD20CAR, wherein the cancer cells express CD20. In one instance, the cancer to be treated is B cell malignancies.

In one aspect, the present disclosure provides methods of treating cancer by providing to the subject in need thereof immune effector cells (e.g., T cells, NK cells) that are engineered to express a Folate receptor alphaCAR, wherein the cancer cells express folate receptor alpha. In one instance the cancer to be treated is ovarian cancer, NSCLC, endometrial cancer, renal cancer, or other solid tumors.

In one aspect, the present disclosure provides methods of treating cancer by providing to the subject in need thereof immune effector cells (e.g., T cells, NK cells) that are engineered to express an ERBB2CAR, wherein the cancer cells express ERBB2 (Her2/neu). In one instance, the cancer to be treated is breast cancer, gastric cancer, colorectal cancer, lung cancer, or other solid tumors.

In one aspect, the present disclosure provides methods of treating cancer by providing to the subject in need thereof immune effector cells (e.g., T cells, NK cells) that are engineered to express a MUC1CAR, wherein the cancer cells express MUC1. In one instance, the cancer to be treated is breast cancer, lung cancer, or other solid tumors.

In one aspect, the present disclosure provides methods of treating cancer by providing to the subject in need thereof immune effector cells (e.g., T cells, NK cells) that are engineered to express an EGFRCAR, wherein the cancer cells express EGFR. In one instance, the cancer to be treated is glioblastoma, SCLC (small cell lung cancer), SCCHN (squamous cell carcinoma of the head and neck), NSCLC, or other solid tumors.

In one aspect, the present disclosure provides methods of treating cancer by providing to the subject in need thereof immune effector cells (e.g., T cells, NK cells) that are engineered to express a NCAMCAR, wherein the cancer cells express NCAM. In one instance, the cancer to be treated is neuroblastoma, or other solid tumors.

In one aspect, the present disclosure provides methods of treating cancer by providing to the subject in need thereof immune effector cells (e.g., T cells, NK cells) that are engineered to express a CAIXCAR, wherein the cancer cells express CAIX. In one instance, the cancer to be treated is renal cancer, CRC, cervical cancer, or other solid tumors.

In one aspect, the present disclosure provides methods of treating cancer by providing to the subject in need thereof immune effector cells (e.g., T cells, NK cells) that are engineered to express an EphA2CAR, wherein the cancer cells express EphA2. In one instance, the cancer to be treated is GBM.

In one aspect, the present disclosure provides methods of treating cancer by providing to the subject in need thereof immune effector cells (e.g., T cells, NK cells) that are engineered to express a GD3CAR, wherein the cancer cells express GD3. In one instance, the cancer to be treated is melanoma.

In one aspect, the present disclosure provides methods of treating cancer by providing to the subject in need thereof immune effector cells (e.g., T cells, NK cells) that are engineered to express a Fucosyl GM1CAR, wherein the cancer cells express Fucosyl GM

In one aspect, the present disclosure provides methods of treating cancer by providing to the subject in need thereof immune effector cells (e.g., T cells, NK cells) that are engineered to express a sLeCAR, wherein the cancer cells express sLe. In one instance, the cancer to be treated is NSCLC, or AML.

In one aspect, the present disclosure provides methods of treating cancer by providing to the subject in need thereof immune effector cells (e.g., T cells, NK cells) that are engineered to express a GM3CAR, wherein the cancer cells express GM3.

In one aspect, the present disclosure provides methods of treating cancer by providing to the subject in need thereof immune effector cells (e.g., T cells, NK cells) that are engineered to express a TGS5CAR, wherein the cancer cells express TGS5.

In one aspect, the present disclosure provides methods of treating cancer by providing to the subject in need thereof immune effector cells (e.g., T cells, NK cells) that are engineered to express a HMWMAACAR, wherein the cancer cells express HMWMAA. In one instance, the cancer to be treated is melanoma, glioblastoma, or breast cancer.

In one aspect, the present disclosure provides methods of treating cancer by providing to the subject in need thereof immune effector cells (e.g., T cells, NK cells) that are engineered to express an o-acetyl-GD2CAR, wherein the cancer cells express o-acetyl-GD2. In one instance, the cancer to be treated is neuroblastoma, or melanoma.

In one aspect, the present disclosure provides methods of treating cancer by providing to the subject in need thereof immune effector cells (e.g., T cells, NK cells) that are engineered to express a CD19CAR, wherein the cancer cells express CD19. In one instance, the cancer to be treated is Folate receptor beta AML, myeloma

In one aspect, the present disclosure provides methods of treating cancer by providing to the subject in need thereof immune effector cells (e.g., T cells, NK cells) that are engineered to express a TEM1/CD248CAR, wherein the cancer cells express TEM1/CD248. In one instance, the cancer to be treated is a solid tumor.

In one aspect, the present disclosure provides methods of treating cancer by providing to the subject in need thereof immune effector cells (e.g., T cells, NK cells) that are engineered to express a TEM7RCAR, wherein the cancer cells express TEM7R. In one instance, the cancer to be treated is solid tumor.

In one aspect, the present disclosure provides methods of treating cancer by providing to the subject in need thereof immune effector cells (e.g., T cells, NK cells) that are engineered to express a CLDN6CAR, wherein the cancer cells express CLDN6. In one instance, the cancer to be treated is ovarian cancer, lung cancer, or breast cancer.

In one aspect, the present disclosure provides methods of treating cancer by providing to the subject in need thereof immune effector cells (e.g., T cells, NK cells) that are engineered to express a TSHRCAR, wherein the cancer cells express TSHR. In one instance, the cancer to be treated is thyroid cancer, or multiple myeloma.

In one aspect, the present disclosure provides methods of treating cancer by providing to the subject in need thereof immune effector cells (e.g., T cells, NK cells) that are engineered to express a GPRC5DCAR, wherein the cancer cells express GPRC5D. In one instance, the cancer to be treated is multiple myeloma.

In one aspect, the present disclosure provides methods of treating cancer by providing to the subject in need thereof immune effector cells (e.g., T cells, NK cells) that are engineered to express a CXORF61CAR, wherein the cancer cells express CXORF61.

In one aspect, the present disclosure provides methods of treating cancer by providing to the subject in need thereof immune effector cells (e.g., T cells, NK cells) that are engineered to express a CD97CAR, wherein the cancer cells express CD97. In one instance, the cancer to be treated is B cell malignancies, gastric cancer, pancreatic cancer, esophageal cancer, glioblastoma, breast cancer, or colorectal cancer.

In one aspect, the present disclosure provides methods of treating cancer by providing to the subject in need thereof immune effector cells (e.g., T cells, NK cells) that are engineered to express a CD179aCAR, wherein the cancer cells express CD179a. In one instance, the cancer to be treated is B cell malignancies.

In one aspect, the present disclosure provides methods of treating cancer by providing to the subject in need thereof immune effector cells (e.g., T cells, NK cells) that are engineered to express an ALK CAR, wherein the cancer cells express ALK. In one instance, the cancer to be treated is NSCLC, ALCL (anaplastic large cell lymphoma), IMT (inflammatory myofibroblastic tumor), or neuroblastoma.

In one aspect, the present disclosure provides methods of treating cancer by providing to the subject in need thereof immune effector cells (e.g., T cells, NK cells) that are engineered to express a Polysialic acid CAR, wherein the cancer cells express Polysialic acid. In one instance, the cancer to be treated is small cell lung cancer.

In one aspect, the present disclosure provides methods of treating cancer by providing to the subject in need thereof immune effector cells (e.g., T cells, NK cells) that are engineered to express a PLAC1CAR, wherein the cancer cells express PLAC1. In one instance, the cancer to be treated is HCC (hepatocellular carcinoma).

In one aspect, the present disclosure provides methods of treating cancer by providing to the subject in need thereof immune effector cells (e.g., T cells, NK cells) that are engineered to express a GloboHCAR, wherein the cancer cells express GloboH. In one instance, the cancer to be treated is ovarian cancer, gastric cancer, prostate cancer, lung cancer, breast cancer, or pancreatic cancer.

In one aspect, the present disclosure provides methods of treating cancer by providing to the subject in need thereof immune effector cells (e.g., T cells, NK cells) that are engineered to express a NY-BR-1CAR, wherein the cancer cells express NY-BR-1. In one instance, the cancer to be treated is breast cancer.

In one aspect, the present disclosure provides methods of treating cancer by providing to the subject in need thereof immune effector cells (e.g., T cells, NK cells) that are engineered to express a UPK2CAR, wherein the cancer cells express UPK2. In one instance, the cancer to be treated is bladder cancer.

In one aspect, the present disclosure provides methods of treating cancer by providing to the subject in need thereof immune effector cells (e.g., T cells, NK cells) that are engineered to express a HAVCR1CAR, wherein the cancer cells express HAVCR1. In one instance, the cancer to be treated is renal cancer.

In one aspect, the present disclosure provides methods of treating cancer by providing to the subject in need thereof immune effector cells (e.g., T cells, NK cells) that are engineered to express a ADRB3CAR, wherein the cancer cells express ADRB3. In one instance, the cancer to be treated is Ewing sarcoma.

In one aspect, the present disclosure provides methods of treating cancer by providing to the subject in need thereof immune effector cells (e.g., T cells, NK cells) that are engineered to express a PANX3CAR, wherein the cancer cells express PANX3. In one instance, the cancer to be treated is osteosarcoma.

In one aspect, the present disclosure provides methods of treating cancer by providing to the subject in need thereof immune effector cells (e.g., T cells, NK cells) that are engineered to express a GPR20CAR, wherein the cancer cells express GPR20. In one instance, the cancer to be treated is GIST.

In one aspect, the present disclosure provides methods of treating cancer by providing to the subject in need thereof immune effector cells (e.g., T cells, NK cells) that are engineered to express a LY6KCAR, wherein the cancer cells express LY6K. In one instance, the cancer to be treated is breast cancer, lung cancer, ovary caner, or cervix cancer.

In one aspect, the present disclosure provides methods of treating cancer by providing to the subject in need thereof immune effector cells (e.g., T cells, NK cells) that are engineered to express a OR51E2CAR, wherein the cancer cells express OR51E2. In one instance, the cancer to be treated is prostate cancer.

In one aspect, the present disclosure provides methods of treating cancer by providing to the subject in need thereof immune effector cells (e.g., T cells, NK cells) that are engineered to express a TARPCAR, wherein the cancer cells express TARP. In one instance, the cancer to be treated is prostate cancer.

In one aspect, the present disclosure provides methods of treating cancer by providing to the subject in need thereof immune effector cells (e.g., T cells, NK cells) that are engineered to express a WT1CAR, wherein the cancer cells express WT1.

In one aspect, the present disclosure provides methods of treating cancer by providing to the subject in need thereof immune effector cells (e.g., T cells, NK cells) that are engineered to express a NY-ESO-1CAR, wherein the cancer cells express NY-ESO-1.

In one aspect, the present disclosure provides methods of treating cancer by providing to the subject in need thereof immune effector cells (e.g., T cells, NK cells) that are engineered to express a LAGE-1a CAR, wherein the cancer cells express LAGE-1a.

In one aspect, the present disclosure provides methods of treating cancer by providing to the subject in need thereof immune effector cells (e.g., T cells, NK cells) that are engineered to express a MAGE-A1CAR, wherein the cancer cells express MAGE-A1. In one instance, the cancer to be treated is melanoma.

In one aspect, the present disclosure provides methods of treating cancer by providing to the subject in need thereof immune effector cells (e.g., T cells, NK cells) that are engineered to express a MAGE A1CAR, wherein the cancer cells express MAGE A1.

In one aspect, the present disclosure provides methods of treating cancer by providing to the subject in need thereof immune effector cells (e.g., T cells, NK cells) that are engineered to express a ETV6-AML CAR, wherein the cancer cells express ETV6-AML.

In one aspect, the present disclosure provides methods of treating cancer by providing to the subject in need thereof immune effector cells (e.g., T cells, NK cells) that are engineered to express a sperm protein 17 CAR, wherein the cancer cells express sperm protein 17. In one instance, the cancer to be treated is ovarian cancer, HCC, or NSCLC.

In one aspect, the present disclosure provides methods of treating cancer by providing to the subject in need thereof immune effector cells (e.g., T cells, NK cells) that are engineered to express a XAGE1CAR, wherein the cancer cells express XAGE1. In one instance, the cancer to be treated is Ewings, or rhabdo cancer.

In one aspect, the present disclosure provides methods of treating cancer by providing to the subject in need thereof immune effector cells (e.g., T cells, NK cells) that are engineered to express a Tie 2 CAR, wherein the cancer cells express Tie 2. In one instance, the cancer to be treated is a solid tumor.

In one aspect, the present disclosure provides methods of treating cancer by providing to the subject in need thereof immune effector cells (e.g., T cells, NK cells) that are engineered to express a MAD-CT-1CAR, wherein the cancer cells express MAD-CT-1. In one instance, the cancer to be treated is prostate cancer, or melanoma.

In one aspect, the present disclosure provides methods of treating cancer by providing to the subject in need thereof immune effector cells (e.g., T cells, NK cells) that are engineered to express a MAD-CT-2CAR, wherein the cancer cells express MAD-CT-2. In one instance, the cancer to be treated is prostate cancer, melanoma.

In one aspect, the present disclosure provides methods of treating cancer by providing to the subject in need thereof immune effector cells (e.g., T cells, NK cells) that are engineered to express a Fos-related antigen 1 CAR, wherein the cancer cells express Fos-related antigen 1. In one instance, the cancer to be treated is glioma, squamous cell cancer, or pancreatic cancer.

In one aspect, the present disclosure provides methods of treating cancer by providing to the subject in need thereof immune effector cells (e.g., T cells, NK cells) that are engineered to express a p53CAR, wherein the cancer cells express p53.

In one aspect, the present disclosure provides methods of treating cancer by providing to the subject in need thereof immune effector cells (e.g., T cells, NK cells) that are engineered to express a prostein CAR, wherein the cancer cells express prostein.

In one aspect, the present disclosure provides methods of treating cancer by providing to the subject in need thereof immune effector cells (e.g., T cells, NK cells) that are engineered to express a survivin and telomerase CAR, wherein the cancer cells express survivin and telomerase.

In one aspect, the present disclosure provides methods of treating cancer by providing to the subject in need thereof immune effector cells (e.g., T cells, NK cells) that are engineered to express a PCTA-1/Galectin 8 CAR, wherein the cancer cells express PCTA-1/Galectin 8.

In one aspect, the present disclosure provides methods of treating cancer by providing to the subject in need thereof immune effector cells (e.g., T cells, NK cells) that are engineered to express a MelanA/MART1CAR, wherein the cancer cells express MelanA/MART1.

In one aspect, the present disclosure provides methods of treating cancer by providing to the subject in need thereof immune effector cells (e.g., T cells, NK cells) that are engineered to express a Ras mutant CAR, wherein the cancer cells express Ras mutant.

In one aspect, the present disclosure provides methods of treating cancer by providing to the subject in need thereof immune effector cells (e.g., T cells, NK cells) that are engineered to express a p53 mutant CAR, wherein the cancer cells express p53 mutant.

In one aspect, the present disclosure provides methods of treating cancer by providing to the subject in need thereof immune effector cells (e.g., T cells, NK cells) that are engineered to express a hTERT CAR, wherein the cancer cells express hTERT.

In one aspect, the present disclosure provides methods of treating cancer by providing to the subject in need thereof immune effector cells (e.g., T cells, NK cells) that are engineered to express a sarcoma translocation breakpoints CAR, wherein the cancer cells express sarcoma translocation breakpoints. In one instance, the cancer to be treated is sarcoma.

In one aspect, the present disclosure provides methods of treating cancer by providing to the subject in need thereof immune effector cells (e.g., T cells, NK cells) that are engineered to express a ML-IAP CAR, wherein the cancer cells express ML-IAP. In one instance, the cancer to be treated is melanoma.

In one aspect, the present disclosure provides methods of treating cancer by providing to the subject in need thereof immune effector cells (e.g., T cells, NK cells) that are engineered to express an ERGCAR, wherein the cancer cells express ERG (TMPRSS2 ETS fusion gene).

In one aspect, the present disclosure provides methods of treating cancer by providing to the subject in need thereof immune effector cells (e.g., T cells, NK cells) that are engineered to express a NA17CAR, wherein the cancer cells express NA17. In one instance, the cancer to be treated is melanoma.

In one aspect, the present disclosure provides methods of treating cancer by providing to the subject in need thereof immune effector cells (e.g., T cells, NK cells) that are engineered to express a PAX3CAR, wherein the cancer cells express PAX3. In one instance, the cancer to be treated is alveolar rhabdomyosarcoma.

In one aspect, the present disclosure provides methods of treating cancer by providing to the subject in need thereof immune effector cells (e.g., T cells, NK cells) that are engineered to express an androgen receptor CAR, wherein the cancer cells express androgen receptor. In one instance, the cancer to be treated is metastatic prostate cancer.

In one aspect, the present disclosure provides methods of treating cancer by providing to the subject in need thereof immune effector cells (e.g., T cells, NK cells) that are engineered to express a Cyclin B1CAR, wherein the cancer cells express Cyclin B1.

In one aspect, the present disclosure provides methods of treating cancer by providing to the subject in need thereof immune effector cells (e.g., T cells, NK cells) that are engineered to express a MYCNCAR, wherein the cancer cells express MYCN.

In one aspect, the present disclosure provides methods of treating cancer by providing to the subject in need thereof immune effector cells (e.g., T cells, NK cells) that are engineered to express a RhoC CAR, wherein the cancer cells express RhoC.

In one aspect, the present disclosure provides methods of treating cancer by providing to the subject in need thereof immune effector cells (e.g., T cells, NK cells) that are engineered to express a TRP-2CAR, wherein the cancer cells express TRP-2. In one instance, the cancer to be treated is melanoma.

In one aspect, the present disclosure provides methods of treating cancer by providing to the subject in need thereof immune effector cells (e.g., T cells, NK cells) that are engineered to express a CYP1B1CAR, wherein the cancer cells express CYP1B1. In one instance, the cancer to be treated is breast cancer, colon cancer, lung cancer, esophagus cancer, skin cancer, lymph node cancer, brain cancer, or testis cancer.

In one aspect, the present disclosure provides methods of treating cancer by providing to the subject in need thereof immune effector cells (e.g., T cells, NK cells) that are engineered to express a BORIS CAR, wherein the cancer cells express BORIS. In one instance, the cancer to be treated is lung cancer.

In one aspect, the present disclosure provides methods of treating cancer by providing to the subject in need thereof immune effector cells (e.g., T cells, NK cells) that are engineered to express a SART3CAR, wherein the cancer cells express SART3

In one aspect, the present disclosure provides methods of treating cancer by providing to the subject in need thereof immune effector cells (e.g., T cells, NK cells) that are engineered to express a PAX5CAR, wherein the cancer cells express PAX5.

In one aspect, the present disclosure provides methods of treating cancer by providing to the subject in need thereof immune effector cells (e.g., T cells, NK cells) that are engineered to express a OY-TES1CAR, wherein the cancer cells express OY-TES1.

In one aspect, the present disclosure provides methods of treating cancer by providing to the subject in need thereof immune effector cells (e.g., T cells, NK cells) that are engineered to express a LCK CAR, wherein the cancer cells express LCK.

In one aspect, the present disclosure provides methods of treating cancer by providing to the subject in need thereof immune effector cells (e.g., T cells, NK cells) that are engineered to express a AKAP-4CAR, wherein the cancer cells express AKAP-4.

In one aspect, the present disclosure provides methods of treating cancer by providing to the subject in need thereof immune effector cells (e.g., T cells, NK cells) that are engineered to express a SSX2CAR, wherein the cancer cells express SSX2.

In one aspect, the present disclosure provides methods of treating cancer by providing to the subject in need thereof immune effector cells (e.g., T cells, NK cells) that are engineered to express a RAGE-1CAR, wherein the cancer cells express RAGE-1. In one instance, the cancer to be treated is RCC (renal cell cancer), or other solid tumors

In one aspect, the present disclosure provides methods of treating cancer by providing to the subject in need thereof immune effector cells (e.g., T cells, NK cells) that are engineered to express a human telomerase reverse transcriptase CAR, wherein the cancer cells express human telomerase reverse transcriptase. In one instance, the cancer to be treated is solid tumors.

In one aspect, the present disclosure provides methods of treating cancer by providing to the subject in need thereof immune effector cells (e.g., T cells, NK cells) that are engineered to express a RU1CAR, wherein the cancer cells express RU1.

In one aspect, the present disclosure provides methods of treating cancer by providing to the subject in need thereof immune effector cells (e.g., T cells, NK cells) that are engineered to express a RU2CAR, wherein the cancer cells express RU2.

In one aspect, the present disclosure provides methods of treating cancer by providing to the subject in need thereof immune effector cells (e.g., T cells, NK cells) that are engineered to express an intestinal carboxyl esterase CAR, wherein the cancer cells express intestinal carboxyl esterase. In one instance, the cancer to be treated is thyroid cancer, RCC, CRC (colorectal cancer), breast cancer, or other solid tumors.

In one aspect, the present disclosure provides methods of treating cancer by providing to the subject in need thereof immune effector cells (e.g., T cells, NK cells) that are engineered to express a Prostase CAR, wherein the cancer cells express Prostase.

In one aspect, the present disclosure provides methods of treating cancer by providing to the subject in need thereof immune effector cells (e.g., T cells, NK cells) that are engineered to express a PAPCAR, wherein the cancer cells express PAP.

In one aspect, the present disclosure provides methods of treating cancer by providing to the subject in need thereof immune effector cells (e.g., T cells, NK cells) that are engineered to express an IGF-I receptor CAR, wherein the cancer cells express IGF-I receptor.

In one aspect, the present disclosure provides methods of treating cancer by providing to the subject in need thereof immune effector cells (e.g., T cells, NK cells) that are engineered to express a gp100 CAR, wherein the cancer cells express gp100.

In one aspect, the present disclosure provides methods of treating cancer by providing to the subject in need thereof immune effector cells (e.g., T cells, NK cells) that are engineered to express a bcr-abl CAR, wherein the cancer cells express bcr-abl.

In one aspect, the present disclosure provides methods of treating cancer by providing to the subject in need thereof immune effector cells (e.g., T cells, NK cells) that are engineered to express a tyrosinase CAR, wherein the cancer cells express tyrosinase.

In one aspect, the present disclosure provides methods of treating cancer by providing to the subject in need thereof immune effector cells (e.g., T cells, NK cells) that are engineered to express a Fucosyl GM1CAR, wherein the cancer cells express Fucosyl GM1.

In one aspect, the present disclosure provides methods of treating cancer by providing to the subject in need thereof immune effector cells (e.g., T cells, NK cells) that are engineered to express a mut hsp70-2CAR, wherein the cancer cells express mut hsp70-2. In one instance, the cancer to be treated is melanoma.

In one aspect, the present disclosure provides methods of treating cancer by providing to the subject in need thereof immune effector cells (e.g., T cells, NK cells) that are engineered to express a CD79a CAR, wherein the cancer cells express CD79a.

In one aspect, the present disclosure provides methods of treating cancer by providing to the subject in need thereof immune effector cells (e.g., T cells, NK cells) that are engineered to express a CD79b CAR, wherein the cancer cells express CD79b.

In one aspect, the present disclosure provides methods of treating cancer by providing to the subject in need thereof immune effector cells (e.g., T cells, NK cells) that are engineered to express a CD72 CAR, wherein the cancer cells express CD72.

In one aspect, the present disclosure provides methods of treating cancer by providing to the subject in need thereof immune effector cells (e.g., T cells, NK cells) that are engineered to express a LAIR1 CAR, wherein the cancer cells express LAIR1.

In one aspect, the present disclosure provides methods of treating cancer by providing to the subject in need thereof immune effector cells (e.g., T cells, NK cells) that are engineered to express a FCAR CAR, wherein the cancer cells express FCAR

In one aspect, the present disclosure provides methods of treating cancer by providing to the subject in need thereof immune effector cells (e.g., T cells, NK cells) that are engineered to express a LILRA2 CAR, wherein the cancer cells express LILRA2.

In one aspect, the present disclosure provides methods of treating cancer by providing to the subject in need thereof immune effector cells (e.g., T cells, NK cells) that are engineered to express a CD300LF CAR, wherein the cancer cells express CD300LF.

In one aspect, the present disclosure provides methods of treating cancer by providing to the subject in need thereof immune effector cells (e.g., T cells, NK cells) that are engineered to express a CLEC12A CAR, wherein the cancer cells express CLEC12A.

In one aspect, the present disclosure provides methods of treating cancer by providing to the subject in need thereof immune effector cells (e.g., T cells, NK cells) that are engineered to express a BST2 CAR, wherein the cancer cells express BST2.

In one aspect, the present disclosure provides methods of treating cancer by providing to the subject in need thereof immune effector cells (e.g., T cells, NK cells) that are engineered to express an EMR2 CAR, wherein the cancer cells express EMR2.

In one aspect, the present disclosure provides methods of treating cancer by providing to the subject in need thereof immune effector cells (e.g., T cells, NK cells) that are engineered to express a LY75 CAR, wherein the cancer cells express LY75.

In one aspect, the present disclosure provides methods of treating cancer by providing to the subject in need thereof immune effector cells (e.g., T cells, NK cells) that are engineered to express a GPC3 CAR, wherein the cancer cells express GPC3.

In one aspect, the present disclosure provides methods of treating cancer by providing to the subject in need thereof immune effector cells (e.g., T cells, NK cells) that are engineered to express a FCRL5 CAR, wherein the cancer cells express FCRL5.

In one aspect, the present disclosure provides methods of treating cancer by providing to the subject in need thereof immune effector cells (e.g., T cells, NK cells) that are engineered to express an IGLL1 CAR, wherein the cancer cells express IGLL1.

In one aspect, the present disclosure relates to treatment of a subject in vivo using an PD1 CAR such that growth of cancerous tumors is inhibited. A PD1 CAR may be used alone to inhibit the growth of cancerous tumors. Alternatively, PD1 CAR may be used in conjunction with other CARs, immunogenic agents, standard cancer treatments, or other antibodies. In one instance, the subject is treated with a PD1 CAR and an XCAR described herein. In an instance, a PD1 CAR is used in conjunction with another CAR, e.g., a CAR described herein, and a kinase inhibitor, e.g., a kinase inhibitor described herein.

In another aspect, a method of treating a subject, e.g., reducing or ameliorating, a hyperproliferative condition or disorder (e.g., a cancer), e.g., solid tumor, a soft tissue tumor, or a metastatic lesion, in a subject is disclosed. As used herein, the term "cancer" is meant to include all types of cancerous growths or oncogenic processes, metastatic tissues or malignantly transformed cells, tissues, or organs, irrespective of histopathologic type or stage of invasiveness. Examples of solid tumors include malignancies, e.g., sarcomas, adenocarcinomas, and carcinomas, of the various organ systems, such as those affecting liver, lung, breast, lymphoid, gastrointestinal (e.g., colon), genitourinary tract (e.g., renal, urothelial cells), prostate and pharynx. Adenocarcinomas include malignancies such as most colon cancers, rectal cancer, renal-cell carcinoma, liver cancer, non-small cell carcinoma of the lung, cancer of the small intestine and cancer of the esophagus. In one embodiment, the cancer is a melanoma, e.g., an advanced stage melanoma. Metastatic lesions of the aforementioned cancers can also be treated or prevented using the methods of the disclosure and compositions of the invention. Examples of other cancers that can be treated include bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, Hodgkin Disease, non-Hodgkin lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, chronic or acute leukemias including acute myeloid leukemia, chronic myeloid leukemia, acute lymphoblastic leukemia, chronic lymphocytic leukemia, solid tumors of childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally induced cancers including those induced by asbestos, and combinations of said cancers. Treatment of metastatic cancers, e.g., metastatic cancers that express PD-L1 (Iwai et al. (2005) Int. Immunol. 17:133-144) can be effected using the antibody molecules described herein.

Exemplary cancers whose growth can be inhibited include cancers typically responsive to immunotherapy. Non-limiting examples of cancers for treatment include melanoma (e.g., metastatic malignant melanoma), renal cancer (e.g. clear cell carcinoma), prostate cancer (e.g. hormone refractory prostate adenocarcinoma), breast cancer, colon cancer and lung cancer (e.g. non-small cell lung cancer). Additionally, refractory or recurrent malignancies can be treated using the molecules described herein.

In one aspect, the invention employs a vector comprising a CAR operably linked to promoter for expression in mammalian immune effector cells (e.g., T cells, NK cells). In one aspect, the invention provides a recombinant immune effector cell in accordance with the claims for use in treating cancer expressing a cancer associate antigen as described herein. In one aspect, CAR-expressing cells of the invention are capable of contacting a tumor cell with at least one cancer associated antigen expressed on its surface such that the CAR-expressing cell targets the cancer cell and growth of the cancer is inhibited.

In one aspect, the disclosure pertains to a method of inhibiting growth of a cancer, comprising contacting the cancer cell with a CAR-expressing cell of the present invention such that the CART is activated in response to the antigen and targets the cancer cell, wherein the growth of the tumor is inhibited.

In one aspect, the disclosure pertains to a method of treating cancer in a subject. The method comprises administering to the subject CAR-expressing cell of the present invention such that the cancer is treated in the subject. In one aspect, the cancer associated with expression of a cancer associate antigen as described herein is a hematological cancer. In one aspect, the hematological cancer is a leukemia or a lymphoma. In one aspect, a cancer associated with expression of a cancer associate antigen as described herein includes cancers and malignancies including, but not limited to, e.g., one or more acute leukemias including but not limited to, e.g., B-cell acute Lymphoid Leukemia ("BALL"), T-cell acute Lymphoid Leukemia ("TALL"), acute lymphoid leukemia (ALL); one or more chronic leukemias including but not limited to, e.g., chronic myelogenous leukemia (CML), Chronic Lymphoid Leukemia (CLL). Additional cancers or hematologic conditions associated with expression of a cancer associate antigen as described herein include, but are not limited to, e.g., B cell prolymphocytic leukemia, blastic plasmacytoid dendritic cell neoplasm, Burkitt's lymphoma, diffuse large B cell lymphoma, Follicular lymphoma, Hairy cell leukemia, small cell- or a large cell-follicular lymphoma, malignant lymphoproliferative conditions, MALT lymphoma, mantle cell lymphoma, Marginal zone lymphoma, multiple myeloma, myelodysplasia and myelodysplastic syndrome, non-Hodgkin lymphoma, plasmablastic lymphoma, plasmacytoid dendritic cell neoplasm, Waldenstrom macroglobulinemia, and "preleukemia" which are a diverse collection of hematological conditions united by ineffective production (or dysplasia) of myeloid blood cells, and the like. Further a disease associated with a cancer associate antigen as described herein expression include, but not limited to, e.g., atypical and/or non-classical cancers, malignancies, precancerous conditions or proliferative diseases associated with expression of a cancer associate antigen as described herein.

In some embodiments, a cancer that can be treated with CAR-expressing cell of the present invention is multiple myeloma. Multiple myeloma is a cancer of the blood, characterized by accumulation of a plasma cell clone in the bone marrow. Current therapies for multiple myeloma include, but are not limited to, treatment with lenalidomide, which is an analog of thalidomide. Lenalidomide has activities which include anti-tumor activity, angiogenesis inhibition, and immunomodulation. Generally, myeloma cells are thought to be negative for a cancer associate antigen as described herein expression by flow cytometry. Thus, in some embodiments, a CD19 CAR, e.g., as described herein, may be used to target myeloma cells. In some embodiments, cars employed in the present invention therapy can be used in combination with one or more additional therapies, e.g., lenalidomide treatment.

The invention includes a type of cellular therapy where immune effector cells (e.g., T cells, NK cells) are genetically modified to express a chimeric antigen receptor (CAR) and the CAR-expressing T cell or NK cell is infused to a recipient in need thereof. The infused cell is able to kill tumor cells in the recipient. Unlike antibody therapies, CAR-modified immune effector cells (e.g., T cells, NK cells) are able to replicate in vivo resulting in long-term persistence that can lead to sustained tumor control. In various aspects, the immune effector cells (e.g., T cells, NK cells) administered to the patient, or their progeny, persist in the patient for at least four months, five months, six months, seven months, eight months, nine months, ten months, eleven months, twelve months, thirteen months, fourteen month, fifteen months, sixteen months, seventeen months, eighteen months, nineteen months, twenty months, twenty-one months, twenty-two months, twenty-three months, two years, three years, four years, or five years after administration of the T cell or NK cell to the patient.

The invention also includes a type of cellular therapy where immune effector cells (e.g., T cells, NK cells) are modified, e.g., by in vitro transcribed RNA, to transiently express a chimeric antigen receptor (CAR) and the CAR T cell or NK cell is infused to a recipient in need thereof. The infused cell is able to kill tumor cells in the recipient. Thus, in various aspects, the immune effector cells (e.g., T cells, NK cells) administered to the patient, is present for less than one month, e.g., three weeks, two weeks, one week, after administration of the T cell or NK cell to the patient.

Without wishing to be bound by any particular theory, the anti-tumor immunity response elicited by the CAR-modified immune effector cells (e.g., T cells, NK cells) may be an active or a passive immune response, or alternatively may be due to a direct vs indirect immune response. In one aspect, the CAR transduced immune effector cells (e.g., T cells, NK cells) exhibit specific proinflammatory cytokine secretion and potent cytolytic activity in response to human cancer cells expressing the a cancer associate antigen as described herein, resist soluble a cancer associate antigen as described herein inhibition, mediate bystander killing and mediate regression of an established human tumor. For example, antigen-less tumor cells within a heterogeneous field of a cancer associate antigen as described herein-expressing tumor may be susceptible to indirect destruction by a cancer associate antigen as described herein-redirected immune effector cells (e.g., T cells, NK cells) that has previously reacted against adjacent antigen-positive cancer cells.

In one aspect, the fully-human CAR-modified immune effector cells (e.g., T cells, NK cells) of the invention may be a type of vaccine for ex vivo immunization and/or in vivo therapy in a mammal. In one aspect, the mammal is a human.

With respect to ex vivo immunization, at least one of the following occurs in vitro prior to administering the cell into a mammal: i) expansion of the cells, ii) introducing a nucleic acid encoding a CAR to the cells or iii) cryopreservation of the cells.

Ex vivo procedures are well known in the art and are discussed more fully below. Briefly, cells are isolated from a mammal (e.g., a human) and genetically modified (i.e., transduced or transfected in vitro) with a vector expressing a CAR disclosed herein. The CAR-modified cell can be administered to a mammalian recipient to provide a therapeutic benefit. The mammalian recipient may be a human and the CAR-modified cell can be autologous with respect to the recipient. Alternatively, the cells can be allogeneic, syngeneic or xenogeneic with respect to the recipient.

The procedure for ex vivo expansion of hematopoietic stem and progenitor cells is described in U.S. Pat. No. 5,199,942 can be applied to the cells of the present invention. Other suitable methods are known in the art, therefore the present invention is not limited to any particular method of ex vivo expansion of the cells. Briefly, ex vivo culture and expansion of immune effector cells (e.g., T cells, NK cells) comprises: (1) collecting CD34+ hematopoietic stem and progenitor cells from a mammal from peripheral blood harvest or bone marrow explants; and (2) expanding such cells ex vivo. In addition to the cellular growth factors described in U.S. Pat. No. 5,199,942, other factors such as flt3-L, IL-1, IL-3 and c-kit ligand, can be used for culturing and expansion of the cells.

In addition to using a cell-based vaccine in terms of ex vivo immunization, the present disclosure also provides compositions and methods for in vivo immunization to elicit an immune response directed against an antigen in a patient.

Generally, the cells activated and expanded as described herein may be utilized in the treatment and prevention of diseases that arise in individuals who are immunocompromised. In particular, the CAR-modified immune effector cells (e.g., T cells, NK cells) of the invention are used in the treatment of diseases, disorders and conditions associated with expression of a cancer associated antigen as described herein. In certain aspects, the cells of the invention are used in the treatment of patients at risk for developing diseases, disorders and conditions associated with expression of a cancer associate antigen as described herein. Thus, the present disclosure provides methods for the treatment or prevention of diseases, disorders and conditions associated with expression of a cancer associated antigen as described herein comprising administering to a subject in need thereof, a therapeutically effective amount of the CAR-modified immune effector cells (e.g., T cells, NK cells) of the invention.

In one aspect the CAR-expressing cells of the inventions may be used to treat a proliferative disease such as a cancer or malignancy or is a precancerous condition such as a myelodysplasia, a myelodysplastic syndrome or a preleukemia. Further a disease associated with a cancer associate antigen as described herein expression include, but not limited to, e.g., atypical and/or non-classical cancers, malignancies, precancerous conditions or proliferative diseases expressing a cancer associated antigen as described herein. Non-cancer related indications associated with expression of a cancer associate antigen as described herein include, but are not limited to, e.g., autoimmune disease, (e.g., lupus), inflammatory disorders (allergy and asthma) and transplantation.

The CAR-modified immune effector cells (e.g., T cells, NK cells) of the present invention may be administered either alone, or as a pharmaceutical composition in combination with diluents and/or with other components such as IL-2 or other cytokines or cell populations.

### Hematologic Cancer

Hematological cancer conditions are the types of cancer such as leukemia, lymphoma, and malignant lymphoproliferative conditions that affect blood, bone marrow and the lymphatic system.

Leukemia can be classified as acute leukemia and chronic leukemia. Acute leukemia can be further classified as acute myelogenous leukemia (AML) and acute lymphoid leukemia (ALL). Chronic leukemia includes chronic myelogenous leukemia (CML) and chronic lymphoid leukemia (CLL). Other related conditions include myelodysplastic syndromes (MDS, formerly known as "preleukemia") which are a diverse collection of hematological conditions united by ineffective production (or dysplasia) of myeloid blood cells and risk of transformation to AML

Lymphoma is a group of blood cell tumors that develop from lymphocytes. Exemplary lymphomas include non-Hodgkin lymphoma and Hodgkin lymphoma.

The present invention provides for compositions for treating cancer. In one aspect, the cancer is a hematologic cancer including but is not limited to hematolical cancer is a leukemia or a lymphoma. In one aspect, the CAR-expressing cells of the invention may be used to treat cancers and malignancies such as, but not limited to, e.g., acute leukemias including but not limited to, e.g., B-cell acute lymphoid leukemia ("BALL"), T-cell acute lymphoid leukemia ("TALL"), acute lymphoid leukemia (ALL); one or more chronic leukemias including but not limited to, e.g., chronic myelogenous leukemia (CML), chronic lymphocytic leukemia (CLL); additional hematologic cancers or hematologic conditions including, but not limited to, e.g., B cell prolymphocytic leukemia, blastic plasmacytoid dendritic cell neoplasm, Burkitt's lymphoma, diffuse large B cell lymphoma, Follicular lymphoma, Hairy cell leukemia, small cell- or a large cell-follicular lymphoma, malignant lymphoproliferative conditions, MALT lymphoma, mantle cell lymphoma, Marginal zone lymphoma, multiple myeloma, myelodysplasia and myelodysplastic syndrome, non-Hodgkin lymphoma, plasmablastic lymphoma, plasmacytoid dendritic cell neoplasm, Waldenstrom macroglobulinemia, and "preleukemia" which are a diverse collection of hematological conditions united by ineffective production (or dysplasia) of myeloid blood cells, and the like. Further a disease associated with a cancer associate antigen as described herein expression includes, but not limited to, e.g., atypical and/or non-classical cancers, malignancies, precancerous conditions or proliferative diseases expressing a cancer associate antigen as described herein.

The present disclosure also provides methods for inhibiting the proliferation or reducing a cancer associated antigen as described herein-expressing cell population, the methods comprising contacting a population of cells comprising a cancer associated antigen as described herein-expressing cell with a CAR-expressing T cell or NK cell of the invention that binds to the a cancer associate antigen as described herein-expressing cell. In a specific aspect, the present disclosure provides methods for inhibiting the proliferation or reducing the population of cancer cells expressing a cancer associated antigen as described herein, the methods comprising contacting a cancer associate antigen as described herein-expressing cancer cell population with a CAR-expressing T cell or NK cell of the invention that binds to a cancer associated antigen as described herein-expressing cell. In one aspect, the present disclosure provides methods for inhibiting the proliferation or reducing the population of cancer cells expressing a cancer associated antigen as described herein, the methods comprising contacting a cancer associated antigen as described herein-expressing cancer cell population with a CAR-expressing T cell or NK cell of the invention that binds to a cancer associated antigen as described herein-expressing cell. In certain aspects, a CAR-expressing T cell or NK cell of the invention reduces the quantity, number, amount or percentage of cells and/or cancer cells by at least 25%, at least 30%, at least 40%, at least 50%, at least 65%, at least 75%, at least 85%, at least 95%, or at least 99% in a subject with or animal model for myeloid leukemia or another cancer associated with a cancer associated antigen as described herein-expressing cells relative to a negative control. In one aspect, the subject is a human.

The present disclosure also provides methods for preventing, treating and/or managing a disease associated with a cancer associated antigen as described herein-expressing cells (e.g., a hematologic cancer or atypical cancer expessing a cancer associated antigen as described herein), the methods comprising administering to a subject in need a CAR T cell or NK cell of the invention that binds to a cancer associated antigen as described herein-expressing cell. In one aspect, the subject is a human. Non-limiting examples of disorders associated with a cancer associated antigen as described herein-expressing cells include autoimmune disorders (such as lupus), inflammatory disorders (such as allergies and asthma) and cancers (such as hematological cancers or atypical cancers expessing a cancer associated antigen as described herein).

The present disclosure also provides methods for preventing, treating and/or managing a disease associated with a cancer associated antigen as described herein-expressing cells, the methods comprising administering to a subject in need a CAR T cell or NK cell of the invention that binds to a cancer associated antigen as described herein-expressing cell. In one aspect, the subject is a human.

The present disclosure provides methods for preventing relapse of cancer associated with a cancer associated antigen as described herein-expressing cells, the methods comprising administering to a subject in need thereof aCAR T cell or NK cell of the invention that binds to a cancer associated antigen as described herein-expressing cell. In one aspect, the methods comprise administering to the subject in need thereof an effective amount of a CAR-expressingT cell or NK cell described herein that binds to a cancer associated antigen as described herein-expressing cell in combination with an effective amount of another therapy.

### Combination Therapies

A CAR-expressing cell described herein may be used in combination with other known agents and therapies. Administered "in combination", as used herein, means that two (or more) different treatments are delivered to the subject during the course of the subject's affliction with the disorder, e.g., the two or more treatments are delivered after the subject has been diagnosed with the disorder and before the disorder has been cured or eliminated or treatment has ceased for other reasons. In some embodiments, the delivery of one treatment is still occurring when the delivery of the second begins, so that there is overlap in terms of administration. This is sometimes referred to herein as "simultaneous" or "concurrent delivery". In other embodiments, the delivery of one treatment ends before the delivery of the other treatment begins. In some embodiments of either case, the treatment is more effective because of combined administration. For example, the second treatment is more effective, e.g., an equivalent effect is seen with less of the second treatment, or the second treatment reduces symptoms to a greater extent, than would be seen if the second treatment were administered in the absence of the first treatment, or the analogous situation is seen with the first treatment. In some embodiments, delivery is such that the reduction in a symptom, or other parameter related to the disorder is greater than what would be observed with one treatment delivered in the absence of the other. The effect of the two treatments can be partially additive, wholly additive, or greater than additive. The delivery can be such that an effect of the first treatment delivered is still detectable when the second is delivered.

A CAR-expressing cell described herein and the at least one additional therapeutic agent can be administered simultaneously, in the same or in separate compositions, or sequentially. For sequential administration, the CAR-expressing cell described herein can be administered first, and the additional agent can be administered second, or the order of administration can be reversed.

The CAR therapy and/or other therapeutic agents, procedures or modalities can be administered during periods of active disorder, or during a period of remission or less active disease. The CAR therapy can be administered before the other treatment, concurrently with the treatment, post-treatment, or during remission of the disorder.

When administered in combination, the CAR therapy and the additional agent (e.g., second or third agent), or all, can be administered in an amount or dose that is higher, lower or the same than the amount or dosage of each agent used individually, e.g., as a monotherapy. In certain embodiments, the administered amount or dosage of the CAR therapy, the additional agent (e.g., second or third agent), or all, is lower (e.g., at least 20%, at least 30%, at least 40%, or at least 50%) than the amount or dosage of each agent used individually, e.g., as a monotherapy. In other embodiments, the amount or dosage of the CAR therapy, the additional agent (e.g., second or third agent), or all, that results in a desired effect (e.g., treatment of cancer) is lower (e.g., at least 20%, at least 30%, at least 40%, or at least 50% lower) than the amount or dosage of each agent used individually, e.g., as a monotherapy, required to achieve the same therapeutic effect.

In further aspects, a CAR-expressing cell described herein may be used in a treatment regimen in combination with surgery, chemotherapy, radiation, immunosuppressive agents, such as cyclosporin, azathioprine, methotrexate, mycophenolate, and FK506, antibodies, or other immunoablative agents such as CAMPATH, anti-CD3 antibodies or other antibody therapies, cytoxin, fludarabine, cyclosporin, FK506, rapamycin, mycophenolic acid, steroids, FR901228, cytokines, and irradiation. peptide vaccine, such as that described in Izumoto et al. 2008 J Neurosurg 108:963-971.

In one embodiment, a CAR-expressing cell can be used in combination with a chemotherapeutic agent. Exemplary chemotherapeutic agents include an anthracycline (e.g., doxorubicin (e.g., liposomal doxorubicin)). a vinca alkaloid (e.g., vinblastine, vincristine, vindesine, vinorelbine), an alkylating agent (e.g., cyclophosphamide, decarbazine, melphalan, ifosfamide, temozolomide), an immune cell antibody (e.g., alemtuzamab, gemtuzumab, rituximab, ofatumumab, tositumomab, brentuximab), an antimetabolite (including, e.g., folic acid antagonists, pyrimidine analogs, purine analogs and adenosine deaminase inhibitors (e.g., fludarabine)), an mTOR inhibitor, a TNFR glucocorticoid induced TNFR related protein (GITR) agonist, a proteasome inhibitor (e.g., aclacinomycin A, gliotoxin or bortezomib), an immunomodulator such as thalidomide or a thalidomide derivative (e.g., lenalidomide).

General Chemotherapeutic agents considered for use in combination therapies include anastrozole (Arimidex^{®}), bicalutamide (Casodex^{®}), bleomycin sulfate (Blenoxane^{®}), busulfan (Myleran^{®}), busulfan injection (Busulfex^{®}), capecitabine (Xeloda^{®}), N4-pentoxycarbonyl-5-deoxy-5-fluorocytidine, carboplatin (Paraplatin^{®}), carmustine (BiCNU^{®}), chlorambucil (Leukeran^{®}), cisplatin (Platinol^{®}), cladribine (Leustatin^{®}), cyclophosphamide (Cytoxan^{®} or Neosar^{®}), cytarabine, cytosine arabinoside (Cytosar-U^{®}), cytarabine liposome injection (DepoCyt^{®}), dacarbazine (DTIC-Dome^{®}), dactinomycin (Actinomycin D, Cosmegan), daunorubicin hydrochloride (Cerubidine^{®}), daunorubicin citrate liposome injection (DaunoXome^{®}), dexamethasone, docetaxel (Taxotere^{®}), doxorubicin hydrochloride (Adriamycin^{®}, Rubex^{®}), etoposide (Vepesid^{®}), fludarabine phosphate (Fludara^{®}), 5-fluorouracil (Adrucil^{®}, Efudex^{®}), flutamide (Eulexin^{®}), tezacitibine, Gemcitabine (difluorodeoxycitidine), hydroxyurea (Hydrea^{®}), Idarubicin (Idamycin^{®}), ifosfamide (IFEX^{®}), irinotecan (Camptosar^{®}), L-asparaginase (ELSPAR^{®}), leucovorin calcium, melphalan (Alkeran^{®}), 6-mercaptopurine (Purinethol^{®}), methotrexate (Folex^{®}), mitoxantrone (Novantrone^{®}), mylotarg, paclitaxel (Taxol^{®}), phoenix (Yttrium90/MX-DTPA), pentostatin, polifeprosan 20 with carmustine implant (Gliadel^{®}), tamoxifen citrate (Nolvadex^{®}), teniposide (Vumon^{®}), 6-thioguanine, thiotepa, tirapazamine (Tirazone^{®}), topotecan hydrochloride for injection (Hycamptin^{®}), vinblastine (Velban^{®}), vincristine (Oncovin^{®}), and vinorelbine (Navelbine^{®}).

Exemplary alkylating agents include, without limitation, nitrogen mustards, ethylenimine derivatives, alkyl sulfonates, nitrosoureas and triazenes): uracil mustard (Aminouracil Mustard^{®}, Chlorethaminacil^{®}, Demethyldopan^{®}, Desmethyldopan^{®}, Haemanthamine^{®}, Nordopan^{®}, Uracil nitrogen mustard^{®}, Uracillost^{®}, Uracilmostaza^{®}, Uramustin^{®}, Uramustine^{®}), chlormethine (Mustargen^{®}), cyclophosphamide (Cytoxan^{®}, Neosar^{®}, Clafen^{®}, Endoxan^{®}, Procytox^{®}, Revimmune^{™}), ifosfamide (Mitoxana^{®}), melphalan (Alkeran^{®}), Chlorambucil (Leukeran^{®}), pipobroman (Amedel^{®}, Vercyte^{®}), triethylenemelamine (Hemel^{®}, Hexalen^{®}, Hexastat^{®}), triethylenethiophosphoramine, Temozolomide (Temodar^{®}), thiotepa (Thioplex^{®}), busulfan (Busilvex^{®}, Myleran^{®}), carmustine (BiCNU^{®}), lomustine (CeeNU^{®}), streptozocin (Zanosar^{®}), and Dacarbazine (DTIC-Dome^{®}). Additional exemplary alkylating agents include, without limitation, Oxaliplatin (Eloxatin^{®}); Temozolomide (Temodar^{®} and Temodal^{®}); Dactinomycin (also known as actinomycin-D, Cosmegen^{®}); Melphalan (also known as L-PAM, L-sarcolysin, and phenylalanine mustard, Alkeran^{®}); Altretamine (also known as hexamethylmelamine (HMM), Hexalen^{®}); Carmustine (BiCNU^{®}); Bendamustine (Treanda^{®}); Busulfan (Busulfex^{®} and Myleran^{®}); Carboplatin (Paraplatin^{®}); Lomustine (also known as CCNU, CeeNU^{®}); Cisplatin (also known as CDDP, Platinol^{®} and Platinol^{®}-AQ); Chlorambucil (Leukeran^{®}); Cyclophosphamide (Cytoxan^{®} and Neosar^{®}); Dacarbazine (also known as DTIC, DIC and imidazole carboxamide, DTIC-Dome^{®}); Altretamine (also known as hexamethylmelamine (HMM), Hexalen^{®}); Ifosfamide (Ifex^{®}); Prednumustine; Procarbazine (Matulane^{®}); Mechlorethamine (also known as nitrogen mustard, mustine and mechloroethamine hydrochloride, Mustargen^{®}); Streptozocin (Zanosar^{®}); Thiotepa (also known as thiophosphoamide, TESPA and TSPA, Thioplex^{®}); Cyclophosphamide (Endoxan^{®}, Cytoxan^{®}, Neosar^{®}, Procytox^{®}, Revimmune^{®}); and Bendamustine HCl (Treanda^{®}).

In embodiments, a CAR-expressing cell is administered to a subject in combination with fludarabine, cyclophosphamide, and/or rituximab. In embodiments, a CAR-expressing cell is administered to a subject in combination with fludarabine, cyclophosphamide, and rituximab (FCR). In embodiments, the subject has CLL. For example, the subject has a deletion in the short arm of chromosome 17 (del(17p), e.g., in a leukemic cell). In other examples, the subject does not have a del(17p). In embodiments, the subject comprises a leukemic cell comprising a mutation in the immunoglobulin heavy-chain variable-region (*IgV_{H}*) gene. In other embodiments, the subject does not comprise a leukemic cell comprising a mutation in the immunoglobulin heavy-chain variable-region (*IgV_{H}*) gene. In embodiments, the fludarabine is administered at a dosage of about 10-50 mg/m² (e.g., about 10-15, 15-20, 20-25, 25-30, 30-35, 35-40, 40-45, or 45-50 mg/m²), e.g., intravenously. In embodiments, the cyclophosphamide is administered at a dosage of about 200-300 mg/m² (e.g., about 200-225, 225-250, 250-275, or 275-300 mg/m²), e.g., intravenously. In embodiments, the rituximab is administered at a dosage of about 400-600 mg/m2 (e.g., 400-450, 450-500, 500-550, or 550-600 mg/m²), e.g., intravenously.

In embodiments, a CAR-expressing cell is administered to a subject in combination with bendamustine and rituximab. In embodiments, the subject has CLL. For example, the subject has a deletion in the short arm of chromosome 17 (del(17p), e.g., in a leukemic cell). In other examples, the subject does not have a del(17p). In embodiments, the subject comprises a leukemic cell comprising a mutation in the immunoglobulin heavy-chain variable-region (*IgV_{H}*) gene. In other embodiments, the subject does not comprise a leukemic cell comprising a mutation in the immunoglobulin heavy-chain variable-region (*IgV_{H}*) gene. In embodiments, the bendamustine is administered at a dosage of about 70-110 mg/m2 (e.g., 70-80, 80-90, 90-100, or 100-110 mg/m2), e.g., intravenously. In embodiments, the rituximab is administered at a dosage of about 400-600 mg/m2 (e.g., 400-450, 450-500, 500-550, or 550-600 mg/m²), e.g., intravenously.

In embodiments, a CAR-expressing cell is administered to a subject in combination with rituximab, cyclophosphamide, doxorubicine, vincristine, and/or a corticosteroid (e.g., prednisone). In embodiments, a CAR-expressing cell is administered to a subject in combination with rituximab, cyclophosphamide, doxorubicine, vincristine, and prednisone (R-CHOP). In embodiments, the subject has diffuse large B-cell lymphoma (DLBCL). In embodiments, the subject has nonbulky limited-stage DLBCL (e.g., comprises a tumor having a size/diameter of less than 7 cm). In embodiments, the subject is treated with radiation in combination with the R-CHOP. For example, the subject is administered R-CHOP (e.g., 1-6 cycles, e.g., 1, 2, 3, 4, 5, or 6 cycles of R-CHOP), followed by radiation. In some cases, the subject is administered R-CHOP (e.g., 1-6 cycles, e.g., 1, 2, 3, 4, 5, or 6 cycles of R-CHOP) following radiation.

In embodiments, a CAR-expressing cell is administered to a subject in combination with etoposide, prednisone, vincristine, cyclophosphamide, doxorubicin, and/or rituximab. In embodiments, a CAR-expressing cell is administered to a subject in combination with etoposide, prednisone, vincristine, cyclophosphamide, doxorubicin, and rituximab (EPOCH-R). In embodiments, a CAR-expressing cell described herein is administered to a subject in combination with dose-adjusted EPOCH-R (DA-EPOCH-R). In embodiments, the subject has a B cell lymphoma, e.g., a Myc-rearranged aggressive B cell lymphoma.

In embodiments, a CAR-expressing cell is administered to a subject in combination with rituximab and/or lenalidomide. Lenalidomide ((*RS*)-3-(4-Amino-1-oxo 1,3-dihydro-2*H*-isoindol- 2-yl)piperidine-2,6-dione) is an immunomodulator. In embodiments, a CAR-expressing cell is administered to a subject in combination with rituximab and lenalidomide. In embodiments, the subject has follicular lymphoma (FL) or mantle cell lymphoma (MCL). In embodiments, the subject has FL and has not previously been treated with a cancer therapy. In embodiments, lenalidomide is administered at a dosage of about 10-20 mg (e.g., 10-15 or 15-20 mg), e.g., daily. In embodiments, rituximab is administered at a dosage of about 350-550 mg/m² (e.g., 350-375, 375-400, 400-425, 425-450, 450-475, or 475-500 mg/m²), e.g., intravenously.

Exemplary mTOR inhibitors include, e.g., temsirolimus; ridaforolimus (formally known as deferolimus, (1*R*,2*R*,4*S*)-4-[(2*R*)-2 [(1*R*,9*S*,12*S*,15*R*,16*E*,18*R*,19*R*,21*R*, 23*S*,24*E*,26*E*,28*Z*,30*S*,32*S*,35*R*)-1,18-dihydroxy-19,30-dimethoxy-15,17,21,23, 29,35-hexamethyl-2,3,10,14,20-pentaoxo-11,36-dioxa-4-azatricyclo[30.3.1.0^{4,9}] hexatriaconta-16,24,26,28-tetraen-12-yl]propyl]-2-methoxycyclohexyl dimethylphosphinate, also known as AP23573 and MK8669, and described in PCT Publication No. WO 03/064383); everolimus (Afinitor^{®} or RAD001); rapamycin (AY22989, Sirolimus^{®}); simapimod (CAS 164301-51-3); emsirolimus, (5-{2,4-Bis[(3*S*)-3-methylmorpholin-4-yl]pyrido[2,3-*d*]pyrimidin-7-yl}-2-methoxyphenyl)methanol (AZD8055); 2-Amino-8-[*trans*-4-(2-hydroxyethoxy)cyclohexyl]-6-(6-methoxy-3-pyridinyl)-4-methyl-pyrido[2,3-*d*]pyrimidin-7(8*H*)-one (PF04691502, CAS 1013101-36-4); and *N*²-[1,4-dioxo-4-[[4-(4-oxo-8-phenyl-4*H*-1-benzopyran-2-yl)morpholinium-4-yl]methoxy]butyl]-L-arginylglycyl-L-α-aspartylL-serine-, inner salt (SF1126, CAS 936487-67-1), and XL765.

Exemplary immunomodulators include, e.g., afutuzumab (available from Roche^{®}); pegfilgrastim (Neulasta^{®}); lenalidomide (CC-5013, Revlimid^{®}); thalidomide (Thalomid^{®}), actimid (CC4047); and IRX-2 (mixture of human cytokines including interleukin 1, interleukin 2, and interferon γ, CAS 951209-71-5, available from IRX Therapeutics).

Exemplary anthracyclines include, e.g., doxorubicin (Adriamycin^{®} and Rubex^{®}); bleomycin (lenoxane^{®}); daunorubicin (dauorubicin hydrochloride, daunomycin, and rubidomycin hydrochloride, Cerubidine^{®}); daunorubicin liposomal (daunorubicin citrate liposome, DaunoXome^{®}); mitoxantrone (DHAD, Novantrone^{®}); epirubicin (Ellence^{™}); idarubicin (Idamycin^{®}, Idamycin PFS^{®}); mitomycin C (Mutamycin^{®}); geldanamycin; herbimycin; ravidomycin; and desacetylravidomycin.

Exemplary vinca alkaloids include, e.g., vinorelbine tartrate (Navelbine^{®}), Vincristine (Oncovin^{®}), and Vindesine (Eldisine^{®})); vinblastine (also known as vinblastine sulfate, vincaleukoblastine and VLB, Alkaban-AQ^{®} and Velban^{®}); and vinorelbine (Navelbine^{®}).

Exemplary proteosome inhibitors include bortezomib (Velcade^{®}); carfilzomib (PX-171-007, (*S*)-4-Methyl-*N*-((*S*)-1-(((*S*)-4-methyl-1-((*R*)-2-methyloxiran-2-yl)-1-oxopentan-2-yl)amino)-1-oxo-3-phenylpropan-2-yl)-2-((*S*)-2-(2-morpholinoacetamido)-4-phenylbutanamido)-pentanamide); marizomib (NPI-0052); ixazomib citrate (MLN-9708); delanzomib (CEP-18770); and *O*-Methyl-*N*-[(2-methyl-5-thiazolyl)carbonyl]-L-seryl-*O-*methyl-*N*-[(1*S*)-2-[(2*R*)-2-methyl-2-oxiranyl]-2-oxo-1-(phenylmethyl)ethyl]-L-serinamide (ONX-0912).

In embodiments, a CAR-expressing cell is administered to a subject in combination with brentuximab. Brentuximab is an antibody-drug conjugate of anti-CD30 antibody and monomethyl auristatin E. In embodiments, the subject has Hodgkin's lymphoma (HL), e.g., relapsed or refractory HL. In embodiments, the subject comprises CD30+ HL. In embodiments, the subject has undergone an autologous stem cell transplant (ASCT). In embodiments, the subject has not undergone an ASCT. In embodiments, brentuximab is administered at a dosage of about 1-3 mg/kg (e.g., about 1-1.5, 1.5-2, 2-2.5, or 2.5-3 mg/kg), e.g., intravenously, e.g., every 3 weeks.

In embodiments, a CAR-expressing cell is administered to a subject in combination with brentuximab and dacarbazine or in combination with brentuximab and bendamustine. Dacarbazine is an alkylating agent with a chemical name of 5-(3,3-Dimethyl-1-triazenyl)imidazole-4-carboxamide. Bendamustine is an alkylating agent with a chemical name of 4-[5-[Bis(2-chloroethyl)amino]-1-methylbenzimidazol-2-yl]butanoic acid. In embodiments, the subject has Hodgkin's lymphoma (HL). In embodiments, the subject has not previously been treated with a cancer therapy. In embodiments, the subject is at least 60 years of age, e.g., 60, 65, 70, 75, 80, 85, or older. In embodiments, dacarbazine is administered at a dosage of about 300-450 mg/m² (e.g., about 300-325, 325-350, 350-375, 375-400, 400-425, or 425-450 mg/m²), e.g., intravenously. In embodiments, bendamustine is administered at a dosage of about 75-125 mg/m2 (e.g., 75-100 or 100-125 mg/m², e.g., about 90 mg/m²), e.g., intravenously. In embodiments, brentuximab is administered at a dosage of about 1-3 mg/kg (e.g., about 1-1.5, 1.5-2, 2-2.5, or 2.5-3 mg/kg), e.g., intravenously, e.g., every 3 weeks.

In some embodiments, a CAR-expressing cell is administered to a subject in combination with a CD20 inhibitor, e.g., an anti-CD20 antibody (e.g., an anti-CD20 monoor bispecific antibody) or a fragment thereof. Exemplary anti-CD20 antibodies include but are not limited to rituximab, ofatumumab, ocrelizumab, veltuzumab, obinutuzumab, TRU-015 (Trubion Pharmaceuticals), ocaratuzumab, and Pro131921 (Genentech). See, e.g., Lim et al. Haematologica. 95.1(2010):135-43.

In some embodiments, the anti-CD20 antibody comprises rituximab. Rituximab is a chimeric mouse/human monoclonal antibody IgG1 kappa that binds to CD20 and causes cytolysis of a CD20 expressing cell, e.g., as described in www.accessdata.fda.gov/drugsatfda_docs/label/2010/103705s5311lbl.pdf. In embodiments, a CAR-expressing cell is administered to a subject in combination with rituximab. In embodiments, the subject has CLL or SLL.

In some embodiments, rituximab is administered intravenously, e.g., as an intravenous infusion. For example, each infusion provides about 500-2000 mg (e.g., about 500-550, 550-600, 600-650, 650-700, 700-750, 750-800, 800-850, 850-900, 900-950, 950-1000, 1000-1100, 1100-1200, 1200-1300, 1300-1400, 1400-1500, 1500-1600, 1600-1700, 1700-1800, 1800-1900, or 1900-2000 mg) of rituximab. In some embodiments, rituximab is administered at a dose of 150 mg/m² to 750 mg/m², e.g., about 150-175 mg/m², 175-200 mg/m², 200-225 mg/m², 225-250 mg/m², 250-300 mg/m², 300-325 mg/m², 325-350 mg/m², 350-375 mg/m², 375-400 mg/m², 400-425 mg/m², 425-450 mg/m², 450-475 mg/m², 475-500 mg/m², 500-525 mg/m², 525-550 mg/m², 550-575 mg/m², 575-600 mg/m², 600-625 mg/m², 625-650 mg/m², 650-675 mg/m², or 675-700 mg/m², where m² indicates the body surface area of the subject. In some embodiments, rituximab is administered at a dosing interval of at least 4 days, e.g., 4, 7, 14, 21, 28, 35 days, or more. For example, rituximab is administered at a dosing interval of at least 0.5 weeks, e.g., 0.5, 1, 2, 3, 4, 5, 6, 7, 8 weeks, or more. In some embodiments, rituximab is administered at a dose and dosing interval described herein for a period of time, e.g., at least 2 weeks, e.g., at least 2, 3, 4, 5, 6, 7, 8, 9, 10, 11, 12, 13, 14, 15, 16, 17, 18, 19, 20 weeks, or greater. For example, rituximab is administered at a dose and dosing interval described herein for a total of at least 4 doses per treatment cycle (e.g., at least 4, 5, 6, 7, 8, 9, 10, 11, 12, 13, 14, 15, 16, or more doses per treatment cycle).

In some embodiments, the anti-CD20 antibody comprises ofatumumab. Ofatumumab is an anti-CD20 IgG1κ human monoclonal antibody with a molecular weight of approximately 149 kDa. For example, ofatumumab is generated using transgenic mouse and hybridoma technology and is expressed and purified from a recombinant murine cell line (NS0). See, e.g., www.accessdata.fda.gov/drugsatfda_docs/label/2009/125326lbl.pdf; and Clinical Trial Identifier number NCT01363128, NCT01515176, NCT01626352, and NCT01397591. In embodiments, a CAR-expressing cell is administered to a subject in combination with ofatumumab. In embodiments, the subject has CLL or SLL.

In some embodiments, ofatumumab is administered as an intravenous infusion. For example, each infusion provides about 150-3000 mg (e.g., about 150-200, 200-250, 250-300, 300-350, 350-400, 400-450, 450-500, 500-550, 550-600, 600-650, 650-700, 700-750, 750-800, 800-850, 850-900, 900-950, 950-1000, 1000-1200, 1200-1400, 1400-1600, 1600-1800, 1800-2000, 2000-2200, 2200-2400, 2400-2600, 2600-2800, or 2800-3000 mg) of ofatumumab. In embodiments, ofatumumab is administered at a starting dosage of about 300 mg, followed by 2000 mg, e.g., for about 11 doses, e.g., for 24 weeks. In some embodiments, ofatumumab is administered at a dosing interval of at least 4 days, e.g., 4, 7, 14, 21, 28, 35 days, or more. For example, ofatumumab is administered at a dosing interval of at least 1 week, e.g., 1, 2, 3, 4, 5, 6, 7, 8, 9, 10, 11, 12, 24, 26, 28, 20, 22, 24, 26, 28, 30 weeks, or more. In some embodiments, ofatumumab is administered at a dose and dosing interval described herein for a period of time, e.g., at least 1 week, e.g., 1, 2, 3, 4, 5, 6, 7, 8, 9, 10, 11, 12, 13, 14, 15, 16, 17, 18, 19, 20, 22, 24, 26, 28, 30, 40, 50, 60 weeks or greater, or 1, 2, 3, 4, 5, 6, 7, 8, 9, 10, 11, 12 months or greater, or 1, 2, 3, 4, 5 years or greater. For example, ofatumumab is administered at a dose and dosing interval described herein for a total of at least 2 doses per treatment cycle (e.g., at least 2, 3, 4, 5, 6, 7, 8, 9, 10, 11, 12, 13, 14, 15, 16, 18, 20, or more doses per treatment cycle).

In some cases, the anti-CD20 antibody comprises ocrelizumab. Ocrelizumab is a humanized anti-CD20 monoclonal antibody, e.g., as described in Clinical Trials Identifier Nos. NCT00077870, NCT01412333, NCT00779220, NCT00673920, NCT01194570, and Kappos et al. Lancet. 19.378(2011):1779-87.

In some cases, the anti-CD20 antibody comprises veltuzumab. Veltuzumab is a humanized monoclonal antibody against CD20. See, e.g., Clinical Trial Identifier No. NCT00547066, NCT00546793, NCT01101581, and Goldenberg et al. Leuk Lymphoma. 51(5)(2010):747-55.

In some cases, the anti-CD20 antibody comprises GA101. GA101 (also called obinutuzumab or RO5072759) is a humanized and glyco-engineered anti-CD20 monoclonal antibody. See, e.g., Robak. Curr. Opin. Investig. Drugs. 10.6(2009):588-96; Clinical Trial Identifier Numbers: NCT01995669, NCT01889797, NCT02229422, and NCT01414205; and www.accessdata.fda.gov/drugsatfda_docs/label/2013/125486s000lbl.pdf.

In some cases, the anti-CD20 antibody comprises AME-133v. AME-133v (also called LY2469298 or ocaratuzumab) is a humanized IgG1 monoclonal antibody against CD20 with increased affinity for the FcγRIIIa receptor and an enhanced antibody dependent cellular cytotoxicity (ADCC) activity compared with rituximab. See, e.g., Robak et al. BioDrugs 25.1(2011):13-25; and Forero-Torres et al. Clin Cancer Res. 18.5(2012):1395-403.

In some cases, the anti-CD20 antibody comprises PRO 131921. PRO131921 is a humanized anti-CD20 monoclonal antibody engineered to have better binding to FcγRIIIa and enhanced ADCC compared with rituximab. See, e.g., Robak et al. BioDrugs 25.1(2011):13-25; and Casulo et al. Clin Immunol. 154.1(2014):37-46; and Clinical Trial Identifier No. NCT00452127.

In some cases, the anti-CD20 antibody comprises TRU-015. TRU-015 is an anti-CD20 fusion protein derived from domains of an antibody against CD20. TRU-015 is smaller than monoclonal antibodies, but retains Fc-mediated effector functions. See, e.g., Robak et al. BioDrugs 25.1(2011):13-25. TRU-015 contains an anti-CD20 single-chain variable fragment (scFv) linked to human IgG1 hinge, CH2, and CH3 domains but lacks CH1 and CL domains.

In some embodiments, an anti-CD20 antibody described herein is conjugated or otherwise bound to a therapeutic agent, e.g., a chemotherapeutic agent (e.g., cytoxan, fludarabine, histone deacetylase inhibitor, demethylating agent, peptide vaccine, anti-tumor antibiotic, tyrosine kinase inhibitor, alkylating agent, anti-microtubule or anti-mitotic agent), anti-allergic agent, anti-nausea agent (or anti-emetic), pain reliever, or cytoprotective agent described herein.

In embodiments, a CAR-expressing cell is administered to a subject in combination with a B-cell lymphoma 2 (BCL-2) inhibitor (e.g., venetoclax, also called ABT-199 or GDC-0199;) and/or rituximab. In embodiments, a CAR-expressing cell is administered to a subject in combination with venetoclax and rituximab. Venetoclax is a small molecule that inhibits the anti-apoptotic protein, BCL-2. The structure of venetoclax (4-(4-{[2-(4-chlorophenyl)-4,4-dimethylcyclohex-1-en-1-yl]methyl}piperazin-1-yl)-*N*-({3-nitro-4-[(tetrahydro-2*H*-pyran-4-ylmethyl)amino]phenyl}sulfonyl)-2-(1*H*-pyrrolo[2,3-*b*]pyridin-5-yloxy)benzamide) is shown below. 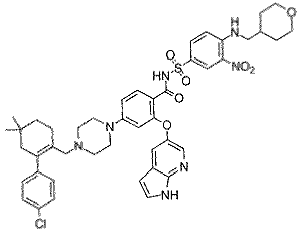 In embodiments, the subject has CLL. In embodiments, the subject has relapsed CLL, e.g., the subject has previously been administered a cancer therapy. In embodiments, venetoclax is administered at a dosage of about 15-600 mg (e.g., 15-20, 20-50, 50-75, 75-100, 100-200, 200-300, 300-400, 400-500, or 500-600 mg), e.g., daily. In embodiments, rituximab is administered at a dosage of about 350-550 mg/m2 (e.g., 350-375, 375-400, 400-425, 425-450, 450-475, or 475-500 mg/m2), e.g., intravenously, e.g., monthly

In an embodiment, cells expressing a CAR are administered to a subject in combination with a molecule that decreases the Treg cell population. Methods that decrease the number of (e.g., deplete) Treg cells are known in the art and include, e.g., CD25 depletion, cyclophosphamide administration, modulating GITR function. Without wishing to be bound by theory, it is believed that reducing the number of Treg cells in a subject prior to apheresis or prior to administration of a CAR-expressing cell described herein reduces the number of unwanted immune cells (e.g., Tregs) in the tumor microenvironment and reduces the subject's risk of relapse. In one embodiment, cells expressing a CAR are administered to a subject in combination with a molecule targeting GITR and/or modulating GITR functions, such as a GITR agonist and/or a GITR antibody that depletes regulatory T cells (Tregs). In embodiments, cells expressing a CAR are administered to a subject in combination with cyclophosphamide. In one embodiment, the GITR binding molecules and/or molecules modulating GITR functions (e.g., GITR agonist and/or Treg depleting GITR antibodies) are administered prior to administration of the CAR-expressing cell. For example, in one embodiment, the GITR agonist can be administered prior to apheresis of the cells. In embodiments, cyclophosphamide is administered to the subject prior to administration (e.g., infusion or re-infusion) of the CAR-expressing cell or prior to aphersis of the cells. In embodiments, cyclophosphamide and an anti-GITR antibody are administered to the subject prior to administration (e.g., infusion or re-infusion) of the CAR-expressing cell or prior to apheresis of the cells. In one embodiment, the subject has cancer (e.g., a solid cancer or a hematological cancer such as ALL or CLL). In an embodiment, the subject has CLL. In embodiments, the subject has ALL. In embodiments, the subject has a solid cancer, e.g., a solid cancer described herein. Exemplary GITR agonists include, e.g., GITR fusion proteins and anti-GITR antibodies (e.g., bivalent anti-GITR antibodies) such as, e.g., a GITR fusion protein described in U.S. Patent No.: 6,111,090, European Patent No.: 090505B1, U.S Patent No.: 8,586,023, PCT Publication Nos.: WO 2010/003118 and 2011/090754, or an anti-GITR antibody described, e.g., in U.S. Patent No.: 7,025,962, European Patent No.: 1947183B1, U.S. Patent No.: 7,812,135, U.S. Patent No.: 8,388,967, U.S. Patent No.: 8,591,886, European Patent No.: EP 1866339, PCT Publication No.: WO 2011/028683, PCT Publication No.:WO 2013/039954, PCT Publication No.: WO2005/007190, PCT Publication No.: WO 2007/133822, PCT Publication No.: WO2005/055808, PCT Publication No.: WO 99/40196, PCT Publication No.: WO 2001/03720, PCT Publication No.: WO99/20758, PCT Publication No.: WO2006/083289, PCT Publication No.: WO 2005/115451, U.S. Patent No.: 7,618,632, and PCT Publication No.: WO 2011/051726.

In one embodiment, a CAR expressing cell is administered to a subject in combination with an mTOR inhibitor, e.g., an mTOR inhibitor described herein, e.g., a rapalog such as everolimus. In one embodiment, the mTOR inhibitor is administered prior to the CAR-expressing cell. For example, in one embodiment, the mTOR inhibitor can be administered prior to apheresis of the cells. In one embodiment, the subject has CLL.

In one embodiment, a CAR expressing cell is administered to a subject in combination with a GITR agonist, e.g., a GITR agonist described herein. In one embodiment, the GITR agonist is administered prior to the CAR-expressing cell. For example, in one embodiment, the GITR agonist can be administered prior to apheresis of the cells. In one embodiment, the subject has CLL.

In one embodiment, a CAR-expressing cell can be used in combination with a kinase inhibitor. In one embodiment, the kinase inhibitor is a CDK4 inhibitor, e.g., a CDK4 inhibitor described herein, e.g., a CD4/6 inhibitor, such as, e.g., 6-Acetyl-8-cyclopentyl-5-methyl-2-(5-piperazin-1-yl-pyridin-2-ylamino)-8*H*-pyrido[2,3-*d*]pyrimidin-7-one, hydrochloride (also referred to as palbociclib or PD0332991). In one embodiment, the kinase inhibitor is a BTK inhibitor, e.g., a BTK inhibitor described herein, such as, e.g., ibrutinib. In one embodiment, the kinase inhibitor is an mTOR inhibitor, e.g., an mTOR inhibitor described herein, such as, e.g., rapamycin, a rapamycin analog, OSI-027. The mTOR inhibitor can be, e.g., an mTORC1 inhibitor and/or an mTORC2 inhibitor, e.g., an mTORC1 inhibitor and/or mTORC2 inhibitor described herein. In one embodiment, the kinase inhibitor is a MNK inhibitor, e.g., a MNK inhibitor described herein, such as, e.g., 4-amino-5-(4-fluoroanilino)-pyrazolo [3,4-*d*] pyrimidine. The MNK inhibitor can be, e.g., a MNK1a, MNK1b, MNK2a and/or MNK2b inhibitor. In one embodiment, the kinase inhibitor is a dual PI3K/mTOR inhibitor described herein, such as, e.g., PF-04695102.

In one embodiment, the kinase inhibitor is a CDK4 inhibitor selected from aloisine A; flavopiridol or HMR-1275, 2-(2-chlorophenyl)-5,7-dihydroxy-8-[(3S,4R)-3-hydroxy-1-methyl-4-piperidinyl]-4-chromenone; crizotinib (PF-02341066; 2-(2-Chlorophenyl)-5,7-dihydroxy-8-[(2*R*,3*S*)-2-(hydroxymethyl)-1-methyl-3-pyrrolidinyl]- 4H-1-benzopyran-4-one, hydrochloride (P276-00); 1-methyl-5-[[2-[5-(trifluoromethyl)-1*H-*imidazol-2-yl]-4-pyridinyl]oxy]-*N*-[4-(trifluoromethyl)phenyl]-1*H*-benzimidazol-2-amine (RAF265); indisulam (E7070); roscovitine (CYC202); palbociclib (PD0332991); dinaciclib (SCH727965); N-[5-[[(5-*tert*-butyloxazol-2-yl)methyl]thio]thiazol-2-yl]piperidine-4-carboxamide (BMS 387032); 4-[[9-chloro-7-(2,6-difluorophenyl)-5*H-*pyrimido[5,4-*d*][2]benzazepin-2-yl]amino]-benzoic acid (MLN8054); 5-[3-(4,6-difluoro-1H-benzimidazol-2-yl)-1H-indazol-5-yl]-N-ethyl-4-methyl-3-pyridinemethanamine (AG-024322); 4-(2,6-dichlorobenzoylamino)-1H-pyrazole-3-carboxylic acid N-(piperidin-4-yl)amide (AT7519); 4-[2-methyl-1-(1-methylethyl)-1*H*-imidazol-5-yl]-*N*-[4-(methylsulfonyl)phenyl]- 2-pyrimidinamine (AZD5438); and XL281 (BMS908662).

In one embodiment, the kinase inhibitor is a CDK4 inhibitor, e.g., palbociclib (PD0332991), and the palbociclib is administered at a dose of about 50 mg, 60 mg, 70 mg, 75 mg, 80 mg, 90 mg, 100 mg, 105 mg, 110 mg, 115 mg, 120 mg, 125 mg, 130 mg, 135 mg (e.g., 75 mg, 100 mg or 125 mg) daily for a period of time, e.g., daily for 14-21 days of a 28 day cycle, or daily for 7-12 days of a 21 day cycle. In one embodiment, 1, 2, 3, 4, 5, 6, 7, 8, 9, 10, 11, 12 or more cycles of palbociclib are administered.

In embodiments, a CAR-expressing cell is administered to a subject in combination with a cyclin-dependent kinase (CDK) 4 or 6 inhibitor, e.g., a CDK4 inhibitor or a CDK6 inhibitor described herein. In embodiments, a CAR-expressing cell is administered to a subject in combination with a CDK4/6 inhibitor (e.g., an inhibitor that targets both CDK4 and CDK6), e.g., a CDK4/6 inhibitor described herein. In an embodiment, the subject has MCL. MCL is an aggressive cancer that is poorly responsive to currently available therapies, i.e., essentially incurable. In many cases of MCL, cyclin D1 (a regulator of CDK4/6) is expressed (e.g., due to chromosomal translocation involving immunoglobulin and Cyclin D1 genes) in MCL cells. Thus, without being bound by theory, it is thought that MCL cells are highly sensitive to CDK4/6 inhibition with high specificity (i.e., minimal effect on normal immune cells). CDK4/6 inhibitors alone have had some efficacy in treating MCL, but have only achieved partial remission with a high relapse rate. An exemplary CDK4/6 inhibitor is LEE011 (also called ribociclib), the structure of which is shown below. 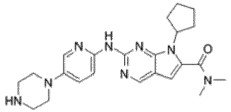 Without being bound by theory, it is believed that administration of a CAR-expressing cell described herein with a CDK4/6 inhibitor (e.g., LEE011 or other CDK4/6 inhibitor described herein) can achieve higher responsiveness, e.g., with higher remission rates and/or lower relapse rates, e.g., compared to a CDK4/6 inhibitor alone.

In one embodiment, the kinase inhibitor is a BTK inhibitor selected from ibrutinib (PCI-32765); GDC-0834; RN-486; CGI-560; CGI-1764; HM-71224; CC-292; ONO-4059; CNX-774; and LFM-A13. In a preferred embodiment, the BTK inhibitor does not reduce or inhibit the kinase activity of interleukin-2-inducible kinase (ITK), and is selected from GDC-0834; RN-486; CGI-560; CGI-1764; HM-71224; CC-292; ONO-4059; CNX-774; and LFM-A13.

In one embodiment, the kinase inhibitor is a BTK inhibitor, e.g., ibrutinib (PCI-32765). In embodiments, a CAR-expressing cell is administered to a subject in combination with a BTK inhibitor (e.g., ibrutinib). In embodiments, a CAR-expressing cell described herein is administered to a subject in combination with ibrutinib (also called PCI-32765). The structure of ibrutinib (1-[(3*R*)-3-[4-Amino-3-(4-phenoxyphenyl)-1*H*-pyrazolo[3,4-d]pyrimidin-1-yl]piperidin-1-yl]prop-2-en-1-one) is shown below. 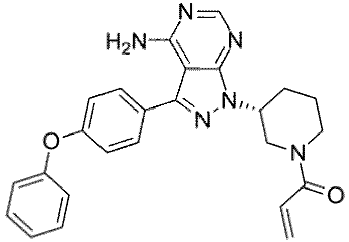 In embodiments, the subject has CLL, mantle cell lymphoma (MCL), or small lymphocytic lymphoma (SLL). For example, the subject has a deletion in the short arm of chromosome 17 (del(17p), e.g., in a leukemic cell). In other examples, the subject does not have a del(17p). In embodiments, the subject has relapsed CLL or SLL, e.g., the subject has previously been administered a cancer therapy (e.g., previously been administered one, two, three, or four prior cancer therapies). In embodiments, the subject has refractory CLL or SLL. In other embodiments, the subject has follicular lymphoma, e.g., relapse or refractory follicular lymphoma. In some embodiments, ibrutinib is administered at a dosage of about 300-600 mg/day (e.g., about 300-350, 350-400, 400-450, 450-500, 500-550, or 550-600 mg/day, e.g., about 420 mg/day or about 560 mg/day), e.g., orally. In embodiments, the ibrutinib is administered at a dose of about 250 mg, 300 mg, 350 mg, 400 mg, 420 mg, 440 mg, 460 mg, 480 mg, 500 mg, 520 mg, 540 mg, 560 mg, 580 mg, 600 mg (e.g., 250 mg, 420 mg or 560 mg) daily for a period of time, e.g., daily for 21 day cycle cycle, or daily for 28 day cycle. In one embodiment, 1, 2, 3, 4, 5, 6, 7, 8, 9, 10, 11, 12 or more cycles of ibrutinib are administered. Without being bound by theory, it is thought that the addition of ibrutinib enhances the T cell proliferative response and may shift T cells from a T-helper-2 (Th2) to T-helper-1 (Th1) phenotype. Th1 and Th2 are phenotypes of helper T cells, with Th1 versus Th2 directing different immune response pathways. A Th1 phenotype is associated with proinflammatory responses, e.g., for killing cells, such as intracellular pathogens/viruses or cancerous cells, or perpetuating autoimmune responses. A Th2 phenotype is associated with eosinophil accumulation and anti-inflammatory responses.

In one embodiment, the kinase inhibitor is an mTOR inhibitor selected from temsirolimus; ridaforolimus (1*R*,2*R*,4*S*)-4-[(2*R*)-2 [(1*R*,9*S*,12*S*,15*R*,16*E*,18*R*,19*R*,21*R*, 23*S*,24*E*,26*E*,28*Z*,30*S*,32*S*,35*R*)-1,18-dihydroxy-19,30-dimethoxy-15,17,21,23, 29,35-hexamethyl-2,3,10,14,20-pentaoxo-11,36-dioxa-4-azatricyclo[30.3.1.0^{4,9}] hexatriaconta-16,24,26,28-tetraen-12-yl]propyl]-2-methoxycyclohexyl dimethylphosphinate, also known as AP23573 and MK8669; everolimus (RAD001); rapamycin (AY22989); simapimod; (5-{2,4-bis[(3*S*)-3-methylmorpholin-4-yl]pyrido[2,3-*d*]pyrimidin-7-yl}-2-methoxyphenyl)methanol (AZD8055); 2-amino-8-[*trans*-4-(2-hydroxyethoxy)cyclohexyl]-6-(6-methoxy-3-pyridinyl)-4-methyl-pyrido[2,3-*d*]pyrimidin-7(8*H*)-one (PF04691502); and *N*²-[1,4-dioxo-4-[[4-(4-oxo-8-phenyl-4*H*-1-benzopyran-2-yl)morpholinium-4-yl]methoxy]butyl]-L-arginylglycyl-L-α-aspartylL-serine-, inner salt (SF1126); and XL765.

In one embodiment, the kinase inhibitor is an mTOR inhibitor, e.g., rapamycin, and the rapamycin is administered at a dose of about 3 mg, 4 mg, 5 mg, 6 mg, 7 mg, 8 mg, 9 mg, 10 mg (e.g., 6 mg) daily for a period of time, e.g., daily for 21 day cycle cycle, or daily for 28 day cycle. In one embodiment, 1, 2, 3, 4, 5, 6, 7, 8, 9, 10, 11, 12 or more cycles of rapamycin are administered. In one embodiment, the kinase inhibitor is an mTOR inhibitor, e.g., everolimus and the everolimus is administered at a dose of about 2 mg, 2.5 mg, 3 mg, 4 mg, 5 mg, 6 mg, 7 mg, 8 mg, 9 mg, 10 mg, 11 mg, 12 mg, 13 mg, 14 mg, 15 mg (e.g., 10 mg) daily for a period of time, e.g., daily for 28 day cycle. In one embodiment, 1, 2, 3, 4, 5, 6, 7, 8, 9, 10, 11, 12 or more cycles of everolimus are administered.

In one embodiment, the kinase inhibitor is an MNK inhibitor selected from CGP052088; 4-amino-3-(p-fluorophenylamino)-pyrazolo [3,4-*d*] pyrimidine (CGP57380); cercosporamide; ETC-1780445-2; and 4-amino-5-(4-fluoroanilino)-pyrazolo [3,4-*d*] pyrimidine.

In embodiments, a CAR-expressing cell is administered to a subject in combination with a phosphoinositide 3-kinase (PI3K) inhibitor (e.g., a PI3K inhibitor described herein, e.g., idelalisib or duvelisib) and/or rituximab. In embodiments, a CAR-expressing cell described herein is administered to a subject in combination with idelalisib and rituximab. In embodiments, a CAR-expressing cell is administered to a subject in combination with duvelisib and rituximab. Idelalisib (also called GS-1101 or CAL-101; Gilead) is a small molecule that blocks the delta isoform of PI3K. The structure of idelalisib (5-Fluoro-3-phenyl-2-[(1S)-1-(7*H*-purin-6-ylamino)propyl]-4(3*H*)-quinazolinone) is shown below. 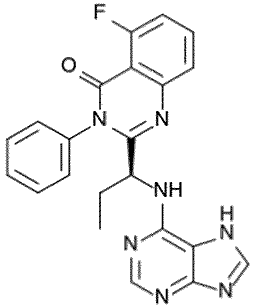 Duvelisib (also called IPI-145; Infinity Pharmaceuticals and Abbvie) is a small molecule that blocks PI3K-δ,γ. The structure of duvelisib (8-Chloro-2-phenyl-3-[(1S)-1-(9H-purin-6-ylamino)ethyl]-1(2H)-isoquinolinone) is shown below. 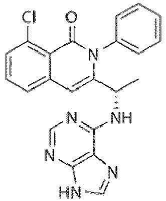 In embodiments, the subject has CLL. In embodiments, the subject has relapsed CLL, e.g., the subject has previously been administered a cancer therapy (e.g., previously been administered an anti-CD20 antibody or previously been administered ibrutinib). For example, the subject has a deletion in the short arm of chromosome 17 (del(17p), e.g., in a leukemic cell). In other examples, the subject does not have a del(17p). In embodiments, the subject comprises a leukemic cell comprising a mutation in the immunoglobulin heavy-chain variable-region (*IgV_{H}*) gene. In other embodiments, the subject does not comprise a leukemic cell comprising a mutation in the immunoglobulin heavy-chain variable-region (*IgV_{H}*) gene. In embodiments, the subject has a deletion in the long arm of chromosome 11 (del(l 1q)). In other embodiments, the subject does not have a del(11q). In embodiments, idelalisib is administered at a dosage of about 100-400 mg (e.g., 100-125, 125-150, 150-175, 175-200, 200-225, 225-250, 250-275, 275-300, 325-350, 350-375, or 375-400 mg), e.g., BID. In embodiments, duvelisib is administered at a dosage of about 15-100 mg (e.g., about 15-25, 25-50, 50-75, or 75-100 mg), e.g., twice a day. In embodiments, rituximab is administered at a dosage of about 350-550 mg/m² (e.g., 350-375, 375-400, 400-425, 425-450, 450-475, or 475-500 mg/m²), e.g., intravenously.

In one embodiment, the kinase inhibitor is a dual phosphatidylinositol 3-kinase (PI3K) and mTOR inhibitor selected from 2-Amino-8-[*trans*-4-(2-hydroxyethoxy)cyclohexyl]-6-(6-methoxy-3-pyridinyl)-4-methyl-pyrido[2,3-*d*]pyrimidin-7(8*H*)-one (PF-04691502); *N*-[4-[[4-(Dimethylamino)-1-piperidinyl]carbonyl]phenyl]-*N*'-[4-(4,6-di-4-morpholinyl-1,3,5-triazin-2-yl)phenyl]urea (PF-05212384, PKI-587); 2-Methyl-2-{4-[3-methyl-2-oxo-8-(quinolin-3-yl)-2,3-dihydro-1*H*-imidazo[4,5-c]quinolin-1-yl]phenyl}propanenitrile (BEZ-235); apitolisib (GDC-0980, RG7422); 2,4-Difluoro-N-{2-(methyloxy)-5-[4-(4-pyridazinyl)-6-quinolinyl]-3-pyridinyl}benzenesulfonamide (GSK2126458); 8-(6-methoxypyridin-3-yl)-3-methyl-1-(4-(piperazin-1-yl)-3-(trifluoromethyl)phenyl)-1H-imidazo[4,5-c]quinolin-2(3H)-one Maleic acid (NVP-BGT226); 3-[4-(4-Morpholinylpyrido[3',2':4,5]furo[3,2-d]pyrimidin-2-yl]phenol (PI-103); 5-(9-isopropyl-8-methyl-2-morpholino-9H-purin-6-yl)pyrimidin-2-amine (VS-5584, SB2343); and N-[2-[(3,5-Dimethoxyphenyl)amino]quinoxalin-3-yl]-4-[(4-methyl-3-methoxyphenyl)carbonyl]aminophenylsulfonamide (XL765).

In embodiments, a CAR-expressing cell is administered to a subject in combination with an anaplastic lymphoma kinase (ALK) inhibitor. Exemplary ALK kinases include but are not limited to crizotinib (Pfizer), ceritinib (Novartis), alectinib (Chugai), brigatinib (also called AP26113; Ariad), entrectinib (Ignyta), PF-06463922 (Pfizer), TSR-011 (Tesaro) (see, e.g., Clinical Trial Identifier No. NCT02048488), CEP-37440 (Teva), and X-396 (Xcovery). In some embodiments, the subject has a solid cancer, e.g., a solid cancer described herein, e.g., lung cancer.

The chemical name of crizotinib is 3-[(1*R*)-1-(2,6-dichloro-3-fluorophenyl)ethoxy]-5-(1-piperidin-4-ylpyrazol-4-yl)pyridin-2-amine. The chemical name of ceritinib is 5-Chloro-*N*²-[2-isopropoxy-5-methyl-4-(4-pipelidinyl)phenyl]-*N*⁴-[2-(isopropylsulfonyl)phenyl]-2,4-pyrimidinediamine. The chemical name of alectinib is 9-ethyl-6,6-dimethyl-8-(4-morpholinopiperidin-1-yl)-11-oxo-6,11-dihydro-5H-benzo[b]carbazole-3-carbonitrile. The chemical name of brigatinib is 5-Chloro-N²-{4-[4-(dimethylamino)-1-piperidinyl]-2-methoxyphenyl}-N⁴-[2-(dimethylphosphoryl)phenyl]-2,4-pyrimidinediamine. The chemical name of entrectinib is N-(5-(3,5-difluorobenzyl)-1H-indazol-3-yl)-4-(4-methylpiperazin-1-yl)-2-((tetrahydro-2H-pyran-4-yl)amino)benzamide. The chemical name of PF-06463922 is (10R)-7-Amino-12-fluoro-2,10,16-trimethyl-15-oxo-10,15,16,17-tetrahydro-2H-8,4-(metheno)pyrazolo[4,3-h][2,5,11]-benzoxadiazacyclotetradecine-3-carbonitrile. The chemical structure of CEP-37440 is (S)-2-((5-chloro-2-((6-(4-(2-hydroxyethyl)piperazin-1-yl)-1-methoxy-6,7,8,9-tetrahydro-5H-benzo[7] annul en-2-yl)amino)pyrimidin-4-yl)amino)-N-methylbenzamide. The chemical name of X-396 is (R)-6-amino-5-(1-(2,6-dichloro-3-fluorophenyl)ethoxy)-N-(4-(4-methylpiperazine-1-carbonyl)phenyl)pyridazine-3-carboxamide.

Drugs that inhibit either the calcium dependent phosphatase calcineurin (cyclosporine and FK506) or inhibit the p70S6 kinase that is important for growth factor induced signaling (rapamycin). (Liu et al., Cell 66:807-815, 1991; Henderson et al., Immun. 73:316-321, 1991; Bierer et al., Curr. Opin. Immun. 5:763-773, 1993) can also be used. In a further aspect, the cell compositions of the present invention may be administered to a patient in conjunction with (e.g., before, simultaneously or following) bone marrow transplantation, T cell ablative therapy using chemotherapy agents such as, fludarabine, external-beam radiation therapy (XRT), cyclophosphamide, and/or antibodies such as OKT3 or CAMPATH. In one aspect, the cell compositions of the present invention are administered following B-cell ablative therapy such as agents that react with CD20, e.g., Rituxan. For example, in one embodiment, subjects may undergo standard treatment with high dose chemotherapy followed by peripheral blood stem cell transplantation. In certain embodiments, following the transplant, subjects receive an infusion of the expanded immune cells of the present invention. In an additional embodiment, expanded cells are administered before or following surgery.

In embodiments, a CAR-expressing cell is administered to a subject in combination with an indoleamine 2,3-dioxygenase (IDO) inhibitor. IDO is an enzyme that catalyzes the degradation of the amino acid, L-tryptophan, to kynurenine. Many cancers overexpress IDO, e.g., prostatic, colorectal, pancreatic, cervical, gastric, ovarian, head, and lung cancer. pDCs, macrophages, and dendritic cells (DCs) can express IDO. Without being bound by theory, it is thought that a decrease in L-tryptophan (e.g., catalyzed by IDO) results in an immunosuppressive milieu by inducing T-cell anergy and apoptosis. Thus, without being bound by theory, it is thought that an IDO inhibitor can enhance the efficacy of a CAR-expressing cell described herein, e.g., by decreasing the suppression or death of a CAR-expressing immune cell. In embodiments, the subject has a solid tumor, e.g., a solid tumor described herein, e.g., prostatic, colorectal, pancreatic, cervical, gastric, ovarian, head, or lung cancer. Exemplary inhibitors of IDO include but are not limited to 1-methyltryptophan, indoximod (NewLink Genetics) (see, e.g., Clinical Trial Identifier Nos. NCT01191216; NCT01792050), and INCB024360 (Incyte Corp.) (see, e.g., Clinical Trial Identifier Nos. NCT01604889; NCT01685255)

In embodiments, a CAR-expressing cell is administered to a subject in combination with a modulator of myeloid-derived suppressor cells (MDSCs). MDSCs accumulate in the periphery and at the tumor site of many solid tumors. These cells suppress T cell responses, thereby hindering the efficacy of CAR-expressing cell therapy. Without being bound by theory, it is thought that administration of a MDSC modulator enhances the efficacy of a CAR-expressing cell described herein. In an embodiment, the subject has a solid tumor, e.g., a solid tumor described herein, e.g., glioblastoma. Exemplary modulators of MDSCs include but are not limited to MCS110 and BLZ945. MCS110 is a monoclonal antibody (mAb) against macrophage colony-stimulating factor (M-CSF). See, e.g., Clinical Trial Identifier No. NCT00757757. BLZ945 is a small molecule inhibitor of colony stimulating factor 1 receptor (CSF1R). See, e.g., Pyonteck et al. Nat. Med. 19(2013):1264-72. The structure of BLZ945 is shown below.

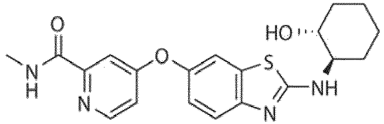

In embodiments, a CAR-expressing cell is administered to a subject in combination with a CD19 CART cell (e.g., CTL019, e.g., as described in WO2012/079000). In embodiments, the subject has a CD19+ lymphoma, e.g., a CD19+ Non-Hodgkin's Lymphoma (NHL), a CD19+ FL, or a CD19+ DLBCL. In embodiments, the subject has a relapsed or refractory CD19+ lymphoma. In embodiments, a lymphodepleting chemotherapy is administered to the subject prior to, concurrently with, or after administration (e.g., infusion) of CD19 CART cells. In an example, the lymphodepleting chemotherapy is administered to the subject prior to administration of CD19 CART cells. For example, the lymphodepleting chemotherapy ends 1-4 days (e.g,. 1, 2, 3, or 4 days) prior to CD19 CART cell infusion. In embodiments, multiple doses of CD19 CART cells are administered, e.g., as described herein. For example, a single dose comprises about 5 × 10⁸ CD19 CART cells. In embodiments, a lymphodepleting chemotherapy is administered to the subject prior to, concurrently with, or after administration (e.g., infusion) of a CAR-expressing cell described herein, e.g., a non-CD19 CAR-expresing cell. In embodiments, a CD19 CART is administered to the subject prior to, concurrently with, or after administration (e.g., infusion) of a non-CD19 CAR-expressing cell, e.g., a non-CD19 CAR-expressing cell described herein.

In some embodiments, a CAR-expressing cell is administered to a subject in combination with a interleukin-15 (IL-15) polypeptide, a interleukin-15 receptor alpha (IL-15Ra) polypeptide, or a combination of both a IL-15 polypeptide and a IL-15Ra polypeptide e.g., hetIL-15 (Admune Therapeutics, LLC). hetIL-15 is a heterodimeric non-covalent complex of IL-15 and IL-15Ra. hetIL-15 is described in, e.g., U.S. 8,124,084, U.S. 2012/0177598, U.S. 2009/0082299, U.S. 2012/0141413, and U.S. 2011/0081311. In embodiments, het-IL-15 is administered subcutaneously. In embodiments, the subject has a cancer, e.g., solid cancer, e.g., melanoma or colon cancer. In embodiments, the subject has a metastatic cancer.

In one embodiment, the subject can be administered an agent which reduces or ameliorates a side effect associated with the administration of a CAR-expressing cell. Side effects associated with the administration of a CAR-expressing cell include, but are not limited to CRS, and hemophagocytic lymphohistiocytosis (HLH), also termed Macrophage Activation Syndrome (MAS). Symptoms of CRS include high fevers, nausea, transient hypotension, hypoxia, and the like. CRS may include clinical constitutional signs and symptoms such as fever, fatigue, anorexia, myalgias, arthalgias, nausea, vomiting, and headache. CRS may include clinical skin signs and symptoms such as rash. CRS may include clinical gastrointestinal signs and symsptoms such as nausea, vomiting and diarrhea. CRS may include clinical respiratory signs and symptoms such as tachypnea and hypoxemia. CRS may include clinical cardiovascular signs and symptoms such as tachycardia, widened pulse pressure, hypotension, increased cardac output (early) and potentially diminished cardiac output (late). CRS may include clinical coagulation signs and symptoms such as elevated d-dimer, hypofibrinogenemia with or without bleeding. CRS may include clinical renal signs and symptoms such as azotemia. CRS may include clinical hepatic signs and symptoms such as transaminitis and hyperbilirubinemia. CRS may include clinical neurologic signs and symptoms such as headache, mental status changes, confusion, delirium, word finding difficulty or frank aphasia, hallucinations, tremor, dymetria, altered gait, and seizures.

Accordingly, the methods described herein can comprise administering a CAR-expressing cell described herein to a subject and further administering one or more agents to manage elevated levels of a soluble factor resulting from treatment with a CAR-expressing cell. In one instance, the soluble factor elevated in the subject is one or more of IFN-γ, TNFα, IL-2 and IL-6. In an instance, the factor elevated in the subject is one or more of IL-1, GM-CSF, IL-10, IL-8, IL-5 and fraktalkine. Therefore, an agent administered to treat this side effect can be an agent that neutralizes one or more of these soluble factors. In one embodiment, the agent that neutralizes one or more of these soluble forms is an antibody or antigen binding fragment thereof. Examples of such agents include, but are not limited to a steroid (e.g., corticosteroid), an inhibitor of TNFα, and an inhibitor of IL-6. An example of a TNFα inhibitor is an anti-TNFa antibody molecule such as, infliximab, adalimumab, certolizumab pegol, and golimumab. Another example of a TNFα inhibitor is a fusion protein such as entanercept. Small molecule inhibitors of TNFα include, but are not limited to, xanthine derivatives (e.g. pentoxifylline) and bupropion. An example of an IL-6 inhibitor is an anti-IL-6 antibody molecule or an anti-IL-6 receptor antibody molecule such as tocilizumab (toc), sarilumab, elsilimomab, CNTO 328, ALD518/BMS-945429, CNTO 136, CPSI-2364, CDP6038, VX30, ARGX-109, FE301, and FM101. In one embodiment, the anti-IL-6 receptor antibody molecule is tocilizumab. An example of an IL-1R based inhibitor is anakinra.

In one embodiment, the subject can be administered an agent which enhances the activity of a CAR-expressing cell. For example, in one embodiment, the agent can be an agent which inhibits an inhibitory molecule. Inhibitory molecules, e.g., Programmed Death 1 (PD-1), can, in some embodiments, decrease the ability of a CAR-expressing cell to mount an immune effector response. Examples of inhibitory molecules include PD-1, PD-L1, CTLA-4, TIM-3, CEACAM (e.g., CEACAM-1, CEACAM-3 and/or CEACAM-5), LAG-3, VISTA, BTLA, TIGIT, LAIR1, CD160, 2B4 and TGFR beta. Inhibition of an inhibitory molecule, e.g., by inhibition at the DNA, RNA or protein level, can optimize a CAR-expressing cell performance. In embodiments, an inhibitory nucleic acid, e.g., an inhibitory nucleic acid, e.g., a dsRNA, e.g., an siRNA or shRNA, a clustered regularly interspaced short palindromic repeats (CRISPR), a transcription-activator like effector nuclease (TALEN), or a zinc finger endonuclease (ZFN), e.g., as described herein, can be used to inhibit expression of an inhibitory molecule in the CAR-expressing cell. In an embodiment the inhibitor is an shRNA. In an embodiment, the inhibitory molecule is inhibited within a CAR-expressing cell. In these embodiments, a dsRNA molecule that inhibits expression of the inhibitory molecule is linked to the nucleic acid that encodes a component, e.g., all of the components, of the CAR. In one embodiment, the inhibitor of an inhibitory signal can be, e.g., an antibody or antibody fragment that binds to an inhibitory molecule. For example, the agent can be an antibody or antibody fragment that binds to PD-1, PD-L1, PD-L2 or CTLA4 (e.g., ipilimumab (also referred to as MDX-010 and MDX-101, and marketed as Yervoy^{®}; Bristol-Myers Squibb; Tremelimumab (IgG2 monoclonal antibody available from Pfizer, formerly known as ticilimumab, CP-675,206).). In an embodiment, the agent is an antibody or antibody fragment that binds to TIM3. In an embodiment, the agent is an antibody or antibody fragment that binds to CEACAM (CEACAM-1, CEACAM-3, and/or CEACAM-5). In an embodiment, the agent is an antibody or antibody fragment that binds to LAG3.

PD-1 is an inhibitory member of the CD28 family of receptors that also includes CD28, CTLA-4, ICOS, and BTLA. PD-1 is expressed on activated B cells, T cells and myeloid cells (Agata et al. 1996 Int. Immunol 8:765-75). Two ligands for PD-1, PD-L1 and PD-L2 have been shown to downregulate T cell activation upon binding to PD-1 (Freeman et a. 2000 J Exp Med 192:1027-34; Latchman et al. 2001 Nat Immunol 2:261-8; Carter et al. 2002 Eur J Immunol 32:634-43). PD-L1 is abundant in human cancers (Dong et al. 2003 J Mol Med 81:281-7; Blank et al. 2005 Cancer Immunol. Immunother 54:307-314; Konishi et al. 2004 Clin Cancer Res 10:5094). Immune suppression can be reversed by inhibiting the local interaction of PD-1 with PD-L1. Antibodies, antibody fragments, and other inhibitors of PD-1, PD-L1 and PD-L2 are available in the art and may be used combination with the cars used in the present invention . For example, nivolumab (also referred to as BMS-936558 or MDX1106; Bristol-Myers Squibb) is a fully human IgG4 monoclonal antibody which specifically blocks PD-1. Nivolumab (clone 5C4) and other human monoclonal antibodies that specifically bind to PD-1 are disclosed in US 8,008,449 and WO2006/121168. Pidilizumab (CT-011; Cure Tech) is a humanized IgGlk monoclonal antibody that binds to PD-1. Pidilizumab and other humanized anti-PD-1 monoclonal antibodies are disclosed in WO2009/101611. Pembrolizumab (formerly known as lambrolizumab, and also referred to as MK03475; Merck) is a humanized IgG4 monoclonal antibody that binds to PD-1. Pembrolizumab and other humanized anti-PD-1 antibodies are disclosed in US 8,354,509 and WO2009/114335. MEDI4736 (Medimmune) is a human monoclonal antibody that binds to PDL1, and inhibits interaction of the ligand with PD1. MDPL3280A (Genentech / Roche) is a human Fc optimized IgG1 monoclonal antibody that binds to PD-L1. MDPL3280A and other human monoclonal antibodies to PD-L1 are disclosed in U.S. Patent No.: 7,943,743 and U.S Publication No.: 20120039906. Other anti-PD-L1 binding agents include YW243.55.S70 (heavy and light chain variable regions are shown in SEQ ID NOs 20 and 21 in WO2010/077634) and MDX-1 105 (also referred to as BMS-936559, and, e.g., anti-PD-L1 binding agents disclosed in WO2007/005874). AMP-224 (B7-DCIg; Amplimmune; e.g., disclosed in WO2010/027827 and WO2011/066342), is a PD-L2 Fc fusion soluble receptor that blocks the interaction between PD-1 and B7-H1. Other anti-PD-1 antibodies include AMP 514 (Amplimmune), among others, e.g., anti-PD-1 antibodies disclosed in US 8,609,089, US 2010028330, and/or US 20120114649.

TIM-3 (T cell immunoglobulin-3) also negatively regulates T cell function, particularly in IFN-g-secreting CD4+ T helper 1 and CD8+ T cytotoxic 1 cells, and plays a critical role in T cell exhaustion. Inhibition of the interaction between TIM3 and its ligands, e.g., galectin-9 (Gal9), phosphotidylserine (PS), and HMGB1, can increase immune response. Antibodies, antibody fragments, and other inhibitors of TIM3 and its ligands are available in the art and may be used combination with a CD19 CAR described herein. For example, antibodies, antibody fragments, small molecules, or peptide inhibitors that target TIM3 binds to the IgV domain of TIM3 to inhibit interaction with its ligands. Antibodies and peptides that inhibit TIM3 are disclosed in WO2013/006490 and US20100247521. Other anti-TIM3 antibodies include humanized versions of RMT3-23 (disclosed in Ngiow et al., 2011, Cancer Res, 71:3540-3551), and clone 8B.2C12 (disclosed in Monney et al., 2002, Nature, 415:536-541). Bi-specific antibodies that inhibit TIM3 and PD-1 are disclosed in US20130156774.

In other embodiments, the agent that enhances the activity of a CAR-expressing cell is a CEACAM inhibitor (e.g., CEACAM-1, CEACAM-3, and/or CEACAM-5 inhibitor). In one embodiment, the inhibitor of CEACAM is an anti-CEACAM antibody molecule. Exemplary anti-CEACAM-1 antibodies are described in WO 2010/125571, WO 2013/082366 WO 2014/059251 and WO 2014/022332, *e.g.,* a monoclonal antibody 34B1, 26H7, and 5F4; or a recombinant form thereof, as described in, *e.g.,* US 2004/0047858, US 7,132,255 and WO 99/052552. In other embodiments, the anti-CEACAM antibody binds to CEACAM-5 as described in, *e.g.,* Zheng et al. PLoS One. 2010 Sep 2;5(9). pii: e12529 (DOI:10:1371/journal.pone.0021146), or crossreacts with CEACAM-1 and CEACAM-5 as described in, *e.g.,* WO 2013/054331 and US 2014/0271618.

Without wishing to be bound by theory, carcinoembryonic antigen cell adhesion molecules (CEACAM), such as CEACAM-1 and CEACAM-5, are believed to mediate, at least in part, inhibition of an anti-tumor immune response (*see e.g.,* Markel et al. J Immunol. 2002 Mar 15;168(6):2803-10; Markel et al. J Immunol. 2006 Nov 1;177(9):6062-71; Markel et al. Immunology. 2009 Feb;126(2):186-200; Markel et al. Cancer Immunol Immunother. 2010 Feb;59(2):215-30; Ortenberg et al. Mol Cancer Ther. 2012 Jun; 11(6):1300-10; Stern et al. J Immunol. 2005 Jun 1;174(11):6692-701; Zheng et al. PLoS One. 2010 Sep 2;5(9). pii: e12529). For example, CEACAM-1 has been described as a heterophilic ligand for TIM-3 and as playing a role in TIM-3-mediated T cell tolerance and exhaustion *(see e.g.,* WO 2014/022332; Huang, et al. (2014) Nature doi:10.1038/nature13848). In embodiments, co-blockade of CEACAM-1 and TIM-3 has been shown to enhance an anti-tumor immune response in xenograft colorectal cancer models *(see e.g.,* WO 2014/022332; Huang, *et al.* (2014), *supra).* In other embodiments, co-blockade of CEACAM-1 and PD-1 reduce T cell tolerance as described, *e.g.,* in WO 2014/059251. Thus, CEACAM inhibitors can be used with the other immunomodulators described herein (e*.g.,* anti-PD-1 and/or anti-TIM-3 inhibitors) to enhance an immune response against a cancer, *e.g.,* a melanoma, a lung cancer (e.g., NSCLC), a bladder cancer, a colon cancer an ovarian cancer, and other cancers as described herein.

LAG-3 (lymphocyte activation gene-3 or CD223) is a cell surface molecule expressed on activated T cells and B cells that has been shown to play a role in CD8+ T cell exhaustion. Antibodies, antibody fragments, and other inhibitors of LAG-3 and its ligands are available in the art and may be used combination with a CD19 CAR described herein. For example, BMS-986016 (Bristol-Myers Squib) is a monoclonal antibody that targets LAG3. IMP701 (Immutep) is an antagonist LAG-3 antibody and IMP731 (Immutep and GlaxoSmithKline) is a depleting LAG-3 antibody. Other LAG-3 inhibitors include IMP321 (Immutep), which is a recombinant fusion protein of a soluble portion of LAG3 and Ig that binds to MHC class II molecules and activates antigen presenting cells (APC). Other antibodies are disclosed, e.g., in WO2010/019570.

In some embodiments, the agent which enhances the activity of a CAR-expressing cell can be, e.g., a fusion protein comprising a first domain and a second domain, wherein the first domain is an inhibitory molecule, or fragment thereof, and the second domain is a polypeptide that is associated with a positive signal, e.g., a polypeptide comrpsing an antracellular signaling domain as described herein. In some embodiments, the polypeptide that is associated with a positive signal can include a costimulatory domain of CD28, CD27, ICOS, e.g., an intracellular signaling domain of CD28, CD27 and/or ICOS, and/or a primary signaling domain, e.g., of CD3 zeta, e.g., described herein. In one embodiment, the fusion protein is expressed by the same cell that expressed the CAR. In another embodiment, the fusion protein is expressed by a cell, e.g., a T cell that does not express a CAR of the present disclosure.

In one embodiment, the agent which enhances activity of a CAR-expressing cell described herein is miR-17-92.

In one embodiment, the agent which enhances activity of a CAR-described herein is a cytokine. Cytokines have important functions related to T cell expansion, differentiation, survival, and homeostatis. Cytokines that can be administered to the subject receiving a CAR-expressing cell described herein include: IL-2, IL-4, IL-7, IL-9, IL-15, IL-18, and IL-21, or a combination thereof. In preferred embodiments, the cytokine administered is IL-7, IL-15, or IL-21, or a combination thereof. The cytokine can be administered once a day or more than once a day, e.g., twice a day, three times a day, or four times a day. The cytokine can be administered for more than one day, e.g. the cytokine is administered for 2 days, 3 days, 4 days, 5 days, 6 days, 1 week, 2 weeks, 3 weeks, or 4 weeks. For example, the cytokine is administered once a day for 7 days.

In embodiments, the cytokine is administered in combination with CAR-expressing T cells. The cytokine can be administered simultaneously or concurrently with the CAR-expressing T cells, e.g., administered on the same day. The cytokine may be prepared in the same pharmaceutical composition as the CAR-expressing T cells, or may be prepared in a separate pharmaceutical composition. Alternatively, the cytokine can be administered shortly after administration of the CAR-expressing T cells, e.g., 1 day, 2 days, 3 days, 4 days, 5 days, 6 days, or 7 days after administration of the CAR-expressing T cells. In embodiments where the cytokine is administered in a dosing regimen that occurs over more than one day, the first day of the cytokine dosing regimen can be on the same day as administration with the CAR-expressing T cells, or the first day of the cytokine dosing regimen can be 1 day, 2 days, 3 days, 4 days, 5 days, 6 days, or 7 days after administration of the CAR-expressing T cells. In one embodiment, on the first day, the CAR-expressing T cells are administered to the subject, and on the second day, a cytokine is administered once a day for the next 7 days. In a preferred embodiment, the cytokine to be administered in combination with CAR-expressing T cells is IL-7, IL-15, or IL-21.

In other embodiments, the cytokine is administered a period of time after administration of CAR-expressing cells, e.g., at least 2 weeks, 3 weeks, 4 weeks, 6 weeks, 8 weeks, 10 weeks, 12 weeks, 4 months, 5 months, 6 months, 7 months, 8 months, 9 months, 10 months, 11 months, or 1 year or more after administration of CAR-expressing cells. In one embodiment, the cytokine is administered after assessment of the subject's response to the CAR-expressing cells. For example, the subject is administered CAR-expressing cells according to the dosage and regimens described herein. The response of the subject to CAR-expressing cell therapy is assessed at 2 weeks, 3 weeks, 4 weeks, 6 weeks, 8 weeks, 10 weeks, 12 weeks, 4 months, 5 months, 6 months, 7 months, 8 months, 9 months, 10 months, 11 months, or 1 year or more after administration of CAR-expressing cells, using any of the methods described herein, including inhibition of tumor growth, reduction of circulating tumor cells, or tumor regression. Subjects that do not exhibit a sufficient response to CAR-expressing cell therapy can be administered a cytokine. Administration of the cytokine to the subject that has sub-optimal response to the CAR-expressing cell therapy improves CAR-expressing cell efficacy or anti-cancer activity. In a preferred embodiment, the cytokine administered after administration of CAR-expressing cells is IL-7.

### Combination with a low dose of an mTOR inhibitor

In one embodiment, the cells expressing a CAR molecule are administered in combination with a low, immune enhancing dose of an mTOR inhibitor.

In an embodiment, a dose of an mTOR inhibitor is associated with, or provides, mTOR inhibition of at least 5 but no more than 90%, at least 10 but no more than 90%, at least 15, but no more than 90%, at least 20 but no more than 90%, at least 30 but no more than 90%, at least 40 but no more than 90%, at least 50 but no more than 90%, at least 60 but no more than 90%, or at least 70 but no more than 90%.

In an embodiment, a dose of an mTOR inhibitor is associated with, or provides, mTOR inhibition of at least 5 but no more than 80%, at least 10 but no more than 80%, at least 15, but no more than 80%, at least 20 but no more than 80%, at least 30 but no more than 80%, at least 40 but no more than 80%, at least 50 but no more than 80%, or at least 60 but no more than 80%.

In an embodiment, a dose of an mTOR inhibitor is associated with, or provides, mTOR inhibition of at least 5 but no more than 70%, at least 10 but no more than 70%, at least 15, but no more than 70%, at least 20 but no more than 70%, at least 30 but no more than 70%, at least 40 but no more than 70%, or at least 50 but no more than 70%.

In an embodiment, a dose of an mTOR inhibitor is associated with, or provides, mTOR inhibition of at least 5 but no more than 60%, at least 10 but no more than 60%, at least 15, but no more than 60%, at least 20 but no more than 60%, at least 30 but no more than 60%, or at least 40 but no more than 60%.

In an embodiment, a dose of an mTOR inhibitor is associated with, or provides, mTOR inhibition of at least 5 but no more than 50%, at least 10 but no more than 50%, at least 15, but no more than 50%, at least 20 but no more than 50%, at least 30 but no more than 50%, or at least 40 but no more than 50%.

In an embodiment, a dose of an mTOR inhibitor is associated with, or provides, mTOR inhibition of at least 5 but no more than 40%, at least 10 but no more than 40%, at least 15, but no more than 40%, at least 20 but no more than 40%, at least 30 but no more than 40%, or at least 35 but no more than 40%.

In an embodiment, a dose of an mTOR inhibitor is associated with, or provides, mTOR inhibition of at least 5 but no more than 30%, at least 10 but no more than 30%, at least 15, but no more than 30%, at least 20 but no more than 30%, or at least 25 but no more than 30%.

In an embodiment, a dose of an mTOR inhibitor is associated with, or provides, mTOR inhibition of at least 1, 2, 3, 4 or 5 but no more than 20%, at least 1, 2, 3, 4 or 5 but no more than 30%, at least 1, 2, 3, 4 or 5, but no more than 35, at least 1, 2, 3, 4 or 5 but no more than 40%, or at least 1, 2, 3, 4 or 5 but no more than 45%.

In an embodiment, a dose of an mTOR inhibitor is associated with, or provides, mTOR inhibition of at least 1, 2, 3, 4 or 5 but no more than 90%.

As is discussed herein, the extent of mTOR inhibition can be expressed as the extent of P70 S6 kinase inhibition, e.g., the extent of mTOR inhibition can be determined by the level of decrease in P70 S6 kinase activity, e.g., by the decrease in phosphorylation of a P70 S6 kinase substrate. The level of mTOR inhibition can be evaluated by a method described herein, e.g. by the Boulay assay, or measurement of phosphorylated S6 levels by western blot.

### EXEMPLARY MTOR INHIBITORS

As used herein, the term "mTOR inhibitor" refers to a compound or ligand, or a pharmaceutically acceptable salt thereof, which inhibits the mTOR kinase in a cell. In an embodiment an mTOR inhibitor is an allosteric inhibitor. In an embodiment an mTOR inhibitor is a catalytic inhibitor.

Allosteric mTOR inhibitors include the neutral tricyclic compound rapamycin (sirolimus), rapamycin-related compounds, that is compounds having structural and functional similarity to rapamycin including, e.g., rapamycin derivatives, rapamycin analogs (also referred to as rapalogs) and other macrolide compounds that inhibit mTOR activity.

Rapamycin is a known macrolide antibiotic produced by Streptomyces hygroscopicus having the structure shown in Formula A.

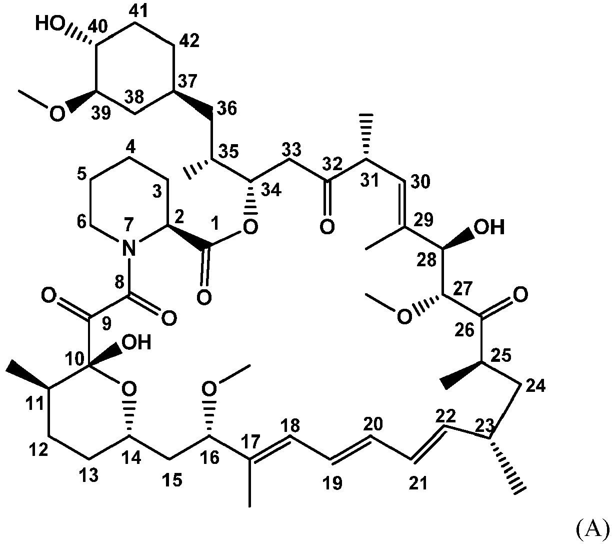

See, e.g., McAlpine, J.B., et al., J. Antibiotics (1991) 44: 688; Schreiber, S.L., et al., J. Am. Chem. Soc. (1991) 113: 7433; U.S. Patent No. 3,929,992. There are various numbering schemes proposed for rapamycin. To avoid confusion, when specific rapamycin analogs are named herein, the names are given with reference to rapamycin using the numbering scheme of formula A.

Rapamycin analogs useful in the invention are, for example, O-substituted analogs in which the hydroxyl group on the cyclohexyl ring of rapamycin is replaced by ORi in which Ri is hydroxyalkyl, hydroxyalkoxyalkyl, acylaminoalkyl, or aminoalkyl; e.g. RAD001, also known as, everolimus as described in US 5,665,772 and WO94/09010. Other suitable rapamycin analogs include those substituted at the 26- or 28-position. The rapamycin analog may be an epimer of an analog mentioned above, particularly an epimer of an analog substituted in position 40, 28 or 26, and may optionally be further hydrogenated, e.g. as described in US 6,015,815, WO95/14023 and WO99/15530, e.g. ABT578 also known as zotarolimus or a rapamycin analog described in US 7,091,213, WO98/02441 and WO01/14387, e.g. AP23573 also known as ridaforolimus.

Examples of rapamycin analogs suitable for use in the present invention from US 5,665,772 include, but are not limited to, 40-O-benzyl-rapamycin, 40-O-(4'-hydroxymethyl)benzyl-rapamycin, 40-O-[4'-(1,2-dihydroxyethyl)]benzyl-rapamycin, 40-O-allyl-rapamycin, 40-O-[3'-(2,2-dimethyl-1,3-dioxolan-4(S)-yl)-prop-2'-en-1'-yl]-rapamycin, (2'E,4'S)-40-O-(4',5'-dihydroxypent-2'-en-1'-yl)-rapamycin, 40-O-(2-hydroxy)ethoxycarbonylmethyl-rapamycin, 40-O-(2-hydroxy)ethyl-rapamycin, 40-O-(3-hydroxy)propyl-rapamycin, 40-O-(6-hydroxy)hexyl-rapamycin, 40-O-[2-(2-hydroxy)ethoxy]ethyl-rapamycin, 40-O-[(3S)-2,2-dimethyldioxolan-3-yl]methyl-rapamycin, 40-O-[(2S)-2,3-dihydroxyprop-1-yl]-rapamycin, 40-O-(2-acetoxy)ethyl-rapamycin, 40-O-(2-nicotinoyloxy)ethyl-rapamycin, 40-O-[2-(N-morpholino)acetoxy]ethyl-rapamycin, 40-O-(2-N-imidazolylacetoxy)ethyl-rapamycin, 40-O-[2-(N-methyl-N'-piperazinyl)acetoxy]ethyl-rapamycin, 39-O-desmethyl-39,40-O,O-ethylene-rapamycin, (26R)-26-dihydro-40-O-(2-hydroxy)ethyl-rapamycin, 40-O-(2-aminoethyl)-rapamycin, 40-O-(2-acetaminoethyl)-rapamycin, 40-O-(2-nicotinamidoethyl)-rapamycin, 40-O-(2-(N-methyl-imidazo-2'-ylcarbethoxamido)ethyl)-rapamycin, 40-O-(2-ethoxycarbonylaminoethyl)-rapamycin, 40-O-(2-tolylsulfonamidoethyl)-rapamycin and 40-O-[2-(4',5'-dicarboethoxy-1',2',3'-triazol-1'-yl)-ethyl]-rapamycin.

Other rapamycin analogs useful in the present invention are analogs where the hydroxyl group on the cyclohexyl ring of rapamycin and/or the hydroxy group at the 28 position is replaced with an hydroxyester group are known, for example, rapamycin analogs found in US RE44,768, e.g. temsirolimus.

Other rapamycin analogs useful in the preset invention include those wherein the methoxy group at the 16 position is replaced with another substituent, preferably (optionally hydroxy-substituted) alkynyloxy, benzyl, orthomethoxybenzyl or chlorobenzyl and/or wherein the mexthoxy group at the 39 position is deleted together with the 39 carbon so that the cyclohexyl ring of rapamycin becomes a cyclopentyl ring lacking the 39 position methyoxy group; e.g. as described in WO95/16691 and WO96/41807. The analogs can be further modified such that the hydroxy at the 40-position of rapamycin is alkylated and/or the 32-carbonyl is reduced.

Rapamycin analogs from WO95/16691 include, but are not limited to, 16-demthoxy-16-(pent-2-ynyl)oxy-rapamycin, 16-demthoxy-16-(but-2-ynyl)oxy-rapamycin, 16-demthoxy-16-(propargyl)oxy-rapamycin, 16-demethoxy-16-(4-hydroxy-but-2-ynyl)oxy-rapamycin, 16-demthoxy-16-benzyloxy-40-O-(2-hydroxyethyl)-rapamycin, 16-demthoxy-16-benzyloxy-rapamycin, 16-demethoxy-16-ortho-methoxybenzyl-rapamycin, 16-demethoxy-40-O-(2-methoxyethyl)-16-pent-2-ynyl)oxy-rapamycin, 3 9-demethoxy-40-desoxy-39-formyl-42-nor-rapamycin, 39-demethoxy-40-desoxy-39-hydroxymethyl-42-nor-rapamycin, 39-demethoxy-40-desoxy-39-carboxy-42-nor-rapamycin, 39-demethoxy-40-desoxy-39-(4-methyl-piperazin-1-yl)carbonyl-42-nor-rapamycin, 39-demethoxy-40-desoxy-39-(morpholin-4-yl)carbonyl-42-nor-rapamycin, 39-demethoxy-40-desoxy-39-[N-methyl, N-(2-pyridin-2-yl-ethyl)]carbamoyl-42-nor-rapamycin and 39-demethoxy-40-desoxy-39-(p-toluenesulfonylhydrazonomethyl)-42-nor-rapamycin.

Rapamycin analogs from WO96/41807 include, but are not limited to, 32-deoxo-rapamycin, 16-O-pent-2-ynyl-32-deoxo-rapamycin, 16-O-pent-2-ynyl-32-deoxo-40-O-(2-hydroxy-ethyl)-rapamycin, 16-O-pent-2-ynyl-32-(S)-dihydro-40-O-(2-hydroxyethyl)-rapamycin, 32(S)-dihydro-40-O-(2-methoxy)ethyl-rapamycin and 32(S)-dihydro-40-O-(2-hydroxyethyl)-rapamycin.

Another suitable rapamycin analog is umirolimus as described in US2005/0101624.

RAD001, otherwise known as everolimus (Afinitor^{®}), has the chemical name (1R,9S,12S,15R,16E,18R,19R,21R,23S,24E,26E,28E,30S,32S,35R)-1,18-dihydroxy-12-{(1R)-2-[(1S,3R,4R)-4-(2-hydroxy ethoxy)-3-methoxycyclohexyl]-1-methylethyl}-19,30-dimethoxy-15,17,21,23,29,35-hexamethyl-11,36-dioxa-4-aza-tricyclo[30.3.1.04,9]hexatriaconta-16,24,26,28-tetraene-2,3,10,14,20-pentaone

Further examples of allosteric mTOR inhibitors include sirolimus (rapamycin, AY-22989), 40-[3-hydroxy-2-(hydroxymethyl)-2-methylpropanoate]-rapamycin (also called temsirolimus or CCI-779) and ridaforolimus (AP-23573/MK-8669). Other examples of allosteric mTor inhibtors include zotarolimus (ABT578) and umirolimus.

Alternatively or additionally, catalytic, ATP-competitive mTOR inhibitors have been found to target the mTOR kinase domain directly and target both mTORC1 and mTORC2. These are also more effective inhibitors of mTORC1 than such allosteric mTOR inhibitors as rapamycin, because they modulate rapamycin-resistant mTORC1 outputs such as 4EBP1-T37/46 phosphorylation and cap-dependent translation.

Catalytic inhibitors include: BEZ235 or 2-methyl-2-[4-(3-methyl-2-oxo-8-quinolin-3-yl-2,3-dihydro-imidazo[4,5-c]quinolin-1-yl)-phenyl]-propionitrile, or the monotosylate salt form. the synthesis of BEZ235 is described in WO2006/122806; CCG168 (otherwise known as AZD-8055, Chresta, C.M., et al., Cancer Res, 2010, 70(1), 288-298) which has the chemical name {5-[2,4-bis-((S)-3-methyl-morpholin-4-yl)-pyrido[2,3d]pyrimidin-7-yl]-2-methoxy-phenyl}-methanol; 3-[2,4-bis[(3S)-3-methylmorpholin-4-yl]pyrido[2,3-d]pyrimidin-7-yl]-N-methylbenzamide (WO09104019); 3-(2-aminobenzo[d]oxazol-5-yl)-1-isopropyl-1H-pyrazolo[3,4-d]pyrimidin-4-amine (WO10051043 and WO2013023184); A N-(3-(N-(3-((3,5-dimethoxyphenyl)amino)quinoxaline-2-yl)sulfamoyl)phenyl)-3-methoxy-4-methylbenzamide (WO07044729 and WO12006552); PKI-587 (Venkatesan, A.M., J. Med.Chem., 2010, 53, 2636-2645) which has the chemical name 1-[4-[4-(dimethylamino)piperidine-1-carbonyl]phenyl]-3-[4-(4,6-dimorpholino-1,3,5-triazin-2-yl)phenyl]urea; GSK-2126458 (ACS Med. Chem. Lett., 2010, 1, 39-43) which has the chemical name 2,4-difluoro-N-{2-methoxy-5-[4-(4-pyridazinyl)-6-quinolinyl]-3-pyridinyl}benzenesulfonamide; ; 5-(9-isopropyl-8-methyl-2-morpholino-9H-purin-6-yl)pyrimidin-2-amine (WO10114484); (E)-N-(8-(6-amino-5-(trifluoromethyl)pyridin-3-yl)-1-(6-(2-cyanopropan-2-yl)pyridin-3-yl)-3-methyl-1H-imidazo[4,5-c]quinolin-2(3H)-ylidene)cyanamide (WO12007926).

Further examples of catalytic mTOR inhibitors include 8-(6-methoxy-pyridin-3-yl)-3 -methyl-1-(4-piperazin-1-yl-3-trifluoromethyl-phenyl)-1,3-dihydro-imidazo[4,5-c]quinolin-2-one (WO2006/122806) and Ku-0063794 (Garcia-Martinez JM, et al.,Biochem J., 2009, 421(1), 29-42.. Ku-0063794 is a specific inhibitor of the mammalian target of rapamycin (mTOR).) WYE-354 is another example of a catalytic mTor inhibitor (Yu K, et al. (2009). Biochemical, Cellular, and In vivo Activity of Novel ATP-Competitive and Selective Inhibitors of the Mammalian Target of Rapamycin. Cancer Res. 69(15): 6232-6240).

mTOR inhibitors useful according to the present invention also include prodrugs, derivatives, pharmaceutically acceptable salts, or analogs thereof of any of the foregoing.

mTOR inhibitors, such as RAD001, may be formulated for delivery based on well-established methods in the art based on the particular dosages described herein. In particular, US Patent 6,004,973 provides examples of formulations useable with the mTOR inhibitors described herein.

### EVALUATION OF MTOR INHIBITION

mTOR phosphorylates the kinase P70 S6, thereby activating P70 S6 kinase and allowing it to phosphorylate its substrate. The extent of mTOR inhibition can be expressed as the extent of P70 S6 kinase inhibition, e.g., the extent of mTOR inhibition can be determined by the level of decrease in P70 S6 kinase activity, e.g., by the decrease in phosphorylation of a P70 S6 kinase substrate. One can determine the level of mTOR inhibition, by measuring P70 S6 kinase activity (the ability of P70 S6 kinase to phsophorylate a substrate), in the absence of inhibitor, e.g., prior to administration of inhibitor, and in the presences of inhibitor, or after the administration of inhibitor. The level of inhibition of P70 S6 kinase gives the level of mTOR inhibition. Thus, if P70 S6 kinase is inhibited by 40%, mTOR activity, as measured by P70 S6 kinase activity, is inhibited by 40%. The extent or level of inhibition referred to herein is the average level of inhibition over the dosage interval. By way of example, if the inhibitor is given once per week, the level of inhibition is given by the average level of inhibition over that interval, namely a week.

Boulay et al., Cancer Res, 2004, 64:252-61 teaches an assay that can be used to assess the level of mTOR inhibition (referred to herein as the Boulay assay). In an instance, the assay relies on the measurement of P70 S6 kinase activity from biological samples before and after administration of an mTOR inhibitor, e.g., RAD001. Samples can be taken at preselected times after treatment with an mTOR ihibitor, e.g., 24, 48, and 72 hours after treatment. Biological samples, e.g., from skin or peripheral blood mononuclear cells (PBMCs) can be used. Total protein extracts are prepared from the samples. P70 S6 kinase is isolated from the protein extracts by immunoprecipitation using an antibody that specifically recognizes the P70 S6 kinase. Activity of the isolated P70 S6 kinase can be measured in an in vitro kinase assay. The isolated kinase can be incubated with 40S ribosomal subunit substrates (which is an endogenous substrate of P70 S6 kinase) and gamma-³²P under conditions that allow phosphorylation of the substrate. Then the reaction mixture can be resolved on an SDS-PAGE gel, and ³²P signal analyzed using a PhosphorImager. A ³²P signal corresponding to the size of the 40S ribosomal subunit indicates phosphorylated substrate and the activity of P70 S6 kinase. Increases and decreases in kinase activity can be calculated by quantifying the area and intensity of the ³²P signal of the phosphorylated substrate (e.g., using ImageQuant, Molecular Dynamics), assigning arbitrary unit values to the quantified signal, and comparing the values from after administration with values from before administration or with a reference value. For example, percent inhibition of kinase activity can be calculated with the following formula: 1-(value obtained after administration/value obtained before administration) X 100. As described above, the extent or level of inhibition referred to herein is the average level of inhibition over the dosage interval.

Methods for the evaluation of kinase activity, e.g., P70 S6 kinase activity, are also provided in US 7,727,950.

The level of mTOR inhibition can also be evaluated by a change in the ration of PD1 negative to PD1 positive T cells. T cells from peripheral blood can be identified as PD1 negative or positive by art-known methods.

### Low-Dose mTOR Inhibitors

Methods described herein use low, immune enhancing, dose mTOR inhibitors, doses of mTOR inhibitors, e.g., allosteric mTOR inhibitors, including rapalogs such as RAD001. In contrast, levels of inhibitor that fully or near fully inhibit the mTOR pathway are immunosuppressive and are used, e.g., to prevent organ transplant rejection. In addition, high doses of rapalogs that fully inhibit mTOR also inhibit tumor cell growth and are used to treat a variety of cancers (See, e.g., Antineoplastic effects of mammalian target of rapamycine inhibitors. Salvadori M. World J Transplant. 2012 Oct 24;2(5):74-83; Current and Future Treatment Strategies for Patients with Advanced Hepatocellular Carcinoma: Role of mTOR Inhibition. Finn RS. Liver Cancer. 2012 Nov;1(3-4):247-256; Emerging Signaling Pathways in Hepatocellular Carcinoma. Moeini A, Cornellà H, Villanueva A. Liver Cancer. 2012 Sep;1(2):83-93; Targeted cancer therapy - Are the days of systemic chemotherapy numbered? Joo WD, Visintin I, Mor G. Maturitas. 2013 Sep 20.; *Role of natural and adaptive immunity in renal cell carcinoma response to VEGFR-TKIS and mTOR inhibitor.* Santoni M, Berardi R, Amantini C, Burattini L, Santini D, Santoni G, Cascinu S. Int J Cancer. 2013 Oct 2).

The present disclosure is based, at least in part, on the surprising finding that doses of mTOR inhibitors well below those used in current clinical settings had a superior effect in increasing an immune response in a subject and increasing the ratio of PD-1 negative T cells/PD-1 positive T cells. It was surprising that low doses of mTOR inhibitors, producing only partial inhibition of mTOR activity, were able to effectively improve immune responses in human human subjects and increase the ratio of PD-1 negative T cells/PD-1 positive T cells.

Alternatively, or in addition, without wishing to be bound by any theory, it is believed that low, a low, immune enhancing, dose of an mTOR inhibitor can increase naive T cell numbers, e.g., at least transiently, e.g., as compared to a non-treated subject. Alternatively or additionally, again while not wishing to be bound by theory, it is believed that treatment with an mTOR inhibitor after a sufficient amount of time or sufficient dosing results in one or more of the following:
an increase in the expression of one or more of the following markers: CD62L^{high}, CD127^{high}, CD27⁺, and BCL2, e.g., on memory T cells, e.g., memory T cell precursors;
a decrease in the expression of KLRG1, e.g., on memory T cells, e.g., memory T cell precursors; and
an increase in the number of memory T cell precursors, e.g., cells with any one or combination of the following characteristics: increased CD62L^{high}, increased CD127^{high}, increased CD27⁺, decreased KLRG1, and increased BCL2;
and wherein any of the changes described above occurs, e.g., at least transiently, e.g., as compared to a non-treated subject (Araki, K et al. (2009) Nature 460:108-112). Memory T cell precursors are memory T cells that are early in the differentiation program. For example, memory T cells have one or more of the following characteristics: increased CD62L^{high}, increased CD127^{high}, increased CD27⁺, decreased KLRG1, and/or increased BCL2.

In an embodiment, the invention may employ a composition, or dosage form, of an mTOR inhibitor, e.g., an allosteric mTOR inhibitor, e.g., a rapalog, rapamycin, or RAD001, or a catalytic mTOR inhibitor, which, when administered on a selected dosing regimen, e.g., once daily or once weekly, is associated with: a level of mTOR inhibition that is not associated with complete, or significant immune suppression, but is associated with enhancement of the immune response.

An mTOR inhibitor, e.g., an allosteric mTOR inhibitor, e.g., a rapalog, rapamycin, or RAD001, or a catalytic mTOR inhibitor, can be provided in a sustained relase formulation. Any of the compositions or unit dosage forms described herein can be provided in a sustained release formulation. In some embodiments, a sustained release formulation will have lower bioavailability than an immediate release formulation. E.g., in embodiments, to attain a similar therapeutic effect of an immediate release forlation a sustained release formulation will have from about 2 to about 5, about 2.5 to about 3.5, or about 3 times the amount of inhibitor provided in the immediate release formulation.

In an embodiment, immediate release forms, e.g., of RAD001, typically used for one administration per week, having 0.1 to 20, 0.5 to 10, 2.5 to 7.5, 3 to 6, or about 5, mgs per unit dosage form, are provided. For once per week administrations, these immediate release formulations correspond to sustained release forms, having, respectively, 0.3 to 60, 1.5 to 30, 7.5 to 22.5, 9 to 18, or about 15 mgs of an mTOR inhibitor, e.g., an allosteric mTOR inhibitor, e.g., rapamycin or RAD001. In embodiments both forms are administered on a once/week basis.

In an embodiment, immediate release forms, e.g., of RAD001, typically used for one administration per day, having having 0.005 to 1.5, 0.01 to 1.5, 0.1 to 1.5, 0.2 to 1.5, 0.3 to 1.5, 0.4 to 1.5, 0.5 to 1.5, 0.6 to 1.5, 0.7 to 1.5, 0.8 to 1.5, 1.0 to 1.5, 0.3 to 0.6, or about 0.5 mgs per unit dosage form, are provided. For once per day administrations, these immediate release forms correspond to sustained release forms, having, respectively, 0.015 to 4.5, 0.03 to 4.5, 0.3 to 4.5, 0.6 to 4.5, 0.9 to 4.5, 1.2 to 4.5, 1.5 to 4.5, 1.8 to 4.5, 2.1 to 4.5, 2.4 to 4.5, 3.0 to 4.5, 0.9 to 1.8, or about 1.5 mgs of an mTOR inhibitor, e.g., an allosteric mTOR inhibitor, e.g., rapamycin or RAD001. For once per week administrations, these immediate release forms correspond to sustained release forms, having, respectively, 0.1 to 30, 0.2 to 30, 2 to 30, 4 to 30, 6 to 30, 8 to 30, 10 to 30, 1.2 to 30, 14 to 30, 16 to 30, 20 to 30, 6 to 12, or about 10 mgs of an mTOR inhibitor, e.g., an allosteric mTOR inhibitor, e.g., rapamycin or RAD001.

In an embodiment, immediate release forms, e.g., of RAD001, typically used for one administration per day, having having 0.01 to 1.0 mgs per unit dosage form, are provided. For once per day administrations, these immediate release forms correspond to sustained release forms, having, respectively, 0.03 to 3 mgs of an mTOR inhibitor, e.g., an allosteric mTOR inhibitor, e.g., rapamycin or RAD001.For once per week administrations, these immediate release forms correspond to sustained release forms, having, respectively, 0.2 to 20 mgs of an mTOR inhibitor, e.g., an allosteric mTOR inhibitor, e.g., rapamycin or RAD001.

In an embodiment, immediate release forms, e.g., of RAD001, typically used for one administration per week, having having 0.5 to 5.0 mgs per unit dosage form, are provided. For once per week administrations, these immediate release forms correspond to sustained release forms, having, respectively, 1.5 to 15 mgs of an mTOR inhibitor, e.g., an allosteric mTOR inhibitor, e.g., rapamycin or RAD001.

As described above, one target of the mTOR pathway is the P70 S6 kinase. Thus, doses of mTOR inhibitors which are useful in the methods and compositions described herein are those which are sufficient to achieve no greater than 80% inhibition of P70 S6 kinase activity relative to the activity of the P70 S6 kinase in the absence of an mTOR inhibitor, e.g., as measured by an assay described herein, e.g., the Boulay assay. In a further aspect, the invention may employ an amount of an mTOR inhibitor sufficient to achieve no greater than 38% inhibition of P70 S6 kinase activity relative to P70 S6 kinase activity in the absence of an mTOR inhibitor.

In one aspect the dose of mTOR inhibitor useful in the methods and compositions of the invention is sufficient to achieve, e.g., when administered to a human subject, 90 +/-5 % (i.e., 85-95%), 89+/-5 %, 88+/-5 %, 87+/-5 %, 86+/-5 %, 85+/-5 %, 84+/-5 %, 83+/-5 %, 82+/-5 %, 81+/-5 %, 80+/-5 %, 79+/-5 %, 78+/-5 %, 77+/-5 %, 76+/-5 %, 75+/-5 %, 74+/-5 %, 73+/-5 %, 72 +/-5%, 71 +/-5%, 70 +/-5%, 69 +/-5%, 68 +/-5%, 67 +/-5%, 66 +/-5%, 65 +/-5%, 64 +/-5%, 63 +/-5%, 62 +/-5%, 61 +/-5%, 60 +/-5%, 59 +/-5%, 58 +/-5%, 57 +/-5%, 56 +/-5%, 55 +/-5%, 54 +/-5%, 54 +/-5%, 53 +/-5%, 52 +/-5%, 51 +/-5%, 50 +/-5%, 49 +/-5%, 48 +/-5%, 47 +/-5%, 46 +/-5%, 45 +/-5%, 44 +/-5%, 43 +/-5%, 42 +/-5%, 41 +/-5%, 40 +/-5%, 39 +/-5%, 38 +/-5%, 37 +/-5%, 36 +/-5%, 35 +/-5%, 34 +/-5%, 33 +/-5%, 32 +/-5%, 31 +/-5%, 30 +/-5%, 29 +/-5%, 28 +/-5%, 27 +/-5%, 26 +/-5%, 25 +/-5%, 24 +/-5%, 23 +/-5%, 22 +/-5%, 21 +/-5%, 20 +/-5%, 19 +/-5%, 18 +/-5%, 17 +/-5%, 16 +/-5%, 15 +/-5%, 14 +/-5%, 13 +/-5%, 12 +/-5%, 11 +/-5%, or 10 +/-5%, inhibition of P70 S6 kinase activity , e.g., as measured by an assay described herein, e.g., the Boulay assay.

P70 S6 kinase activity in a subject may be measured using methods known in the art, such as, for example, according to the methods described in U.S. Pat. 7,727,950, by immunoblot analysis of phosphoP70 S6K levels and/or phosphoP70 S6 levels or by in vitro kinase activity assays.

As used herein, the term "about" in reference to a dose of mTOR inhibitor refers to up to a +/- 10% variability in the amount of mTOR inhibitor, but can include no variability around the stated dose.

In some instances, the disclosure provides methods comprising administering to a subject an mTOR inhibitor, e.g., an allosteric inhibitor, e.g., RAD001, at a dosage within a target trough level. In some instances, the trough level is significantly lower than trough levels associated with dosing regimens used in organ transplant and cancer patients. In an instance mTOR inhibitor, e.g., RAD001, or rapamycin, is administerd to result in a trough level that is less than ½, 1/4, 1/10, or 1/20 of the trough level that results in immunosuppression or an anticancer effect. In an instance mTOR inhibitor, e.g., RAD001, or rapamycin, is administerd to result in a trough level that is less than ½, 1/4, 1/10, or 1/20 of the trough level provided on the FDA approved packaging insert for use in immunosuppression or an anticancer indications.

In an instance a method disclosed herein comprises administering to a subject an mTOR inhibitor, e.g., an allosteric inhibitor, e.g., RAD001, at a dosage that provides a target trough level of 0.1 to 10 ng/ml, 0.1 to 5 ng/ml, 0.1 to 3ng/ml, 0.1 to 2 ng/ml, or 0.1 to 1 ng/ml.

In an instance a method disclosed herein comprises administering to a subject an mTOR inhibitor, e.g., an allosteric inhibitor, e.g., RAD001, at a dosage that provides a target trough level of 0.2 to 10 ng/ml, 0.2 to 5 ng/ml, 0.2 to 3ng/ml, 0.2 to 2 ng/ml, or 0.2 to 1 ng/ml.

In an instance a method disclosed herein comprises administering to a subject an mTOR inhibitor, e.g. an, allosteric inhibitor, e.g., RAD001, at a dosage that provides a target trough level of 0.3 to 10 ng/ml, 0.3 to 5 ng/ml, 0.3 to 3ng/ml, 0.3 to 2 ng/ml, or 0.3 to 1 ng/ml.

In an instance a method disclosed herein comprises administering to a subject an mTOR inhibitor, e.g., an allosteric inhibitor, e.g., RAD001, at a dosage that provides a target trough level of 0.4 to 10 ng/ml, 0.4 to 5 ng/ml, 0.4 to 3ng/ml, 0.4 to 2 ng/ml, or 0.4 to 1 ng/ml.

In an instance a method disclosed herein comprises administering to a subject an mTOR inhibitor, e.g., an allosteric inhibitor, e.g., RAD001, at a dosage that provides a target trough level of 0.5 to 10 ng/ml, 0.5 to 5 ng/ml, 0.5 to 3ng/ml, 0.5 to 2 ng/ml, or 0.5 to 1 ng/ml.

In an instance a method disclosed herein comprises administering to a subject an mTOR inhibitor, e.g., an allosteric inhibitor, e.g., RAD001, at a dosage that provides a target trough level of 1 to 10 ng/ml, 1 to 5 ng/ml, 1 to 3ng/ml, or 1 to 2 ng/ml.

As used herein, the term "trough level" refers to the concentration of a drug in plasma just before the next dose, or the minimum drug concentration between two doses.

In some instances, a target trough level of RAD001 is in a range of between about 0.1 and 4.9 ng/ml. In an instance, the target trough level is below 3ng/ml, e.g., is between 0.3 or less and 3 ng/ml. In an instance, the target trough level is below 3ng/ml, e.g., is between 0.3 or less and 1 ng/ml.

In a further aspect, the invention can utilize an mTOR inhibitor other than RAD001 in an amount that is associated with a target trough level that is bioequivalent to the specified target trough level for RAD001. In an embodiment, the target trough level for an mTOR inhibitor other than RAD001, is a level that gives the same level of mTOR inhibition (e.g., as measured by a method described herein, e.g., the inhibition of P70 S6) as does a trough level of RAD001 described herein.

### Pharmaceutical compositions: mTOR Inhibitors

In one aspect, the present invention may employ pharmaceutical compositions comprising an mTOR inhibitor, e.g., an mTOR inhibitor as described herein, formulated for use in combination with CAR cells in accordance with the claims.

In some embodiments, the mTOR inhibitor is formulated for administration in combination with an additional, e.g., as described herein.

In general, compounds of the disclosure will be administered in therapeutically effective amounts as described above via any of the usual and acceptable modes known in the art, either singly or in combination with one or more therapeutic agents.

The pharmaceutical formulations may be prepared using conventional dissolution and mixing procedures. For example, the bulk drug substance (e.g., an mTOR inhibitor or stabilized form of the compound (e.g., complex with a cyclodextrin derivative or other known complexation agent) is dissolved in a suitable solvent in the presence of one or more of the excipients described herein. The mTOR inhibitor is typically formulated into pharmaceutical dosage forms to provide an easily controllable dosage of the drug and to give the patient an elegant and easily handleable product.

Compounds of the disclosure can be administered as pharmaceutical compositions by any conventional route, in particular enterally, e.g., orally, e.g., in the form of tablets or capsules, or parenterally, e.g., in the form of injectable solutions or suspensions, topically, e.g., in the form of lotions, gels, ointments or creams, or in a nasal or suppository form. Where an mTOR inhibitor is administered in combination with (either simultaneously with or separately from) another agent as described herein, in one aspect, both components can be administered by the same route (e.g., parenterally). Alternatively, another agent may be administered by a different route relative to the mTOR inhibitor. For example, an mTOR inhibitor may be administered orally and the other agent may be administered parenterally.

### SUSTAINED RELEASE

mTOR inhibitors, e.g., allosteric mTOR inhibitors or catalytic mTOR inhibitors, disclosed herein can be provided as pharmaceutical formulations in form of oral solid dosage forms comprising an mTOR inhibitor disclosed herein, e.g., rapamycin or RAD001, which satisfy product stability requirements and/or have favorable pharmacokinetic properties over the immediate release (IR) tablets, such as reduced average plasma peak concentrations, reduced inter- and intra-patient variability in the extent of drug absorption and in the plasma peak concentration, reduced Cₘₐₓ / Cₘᵢₙ ratio and/or reduced food effects. Provided pharmaceutical formulations may allow for more precise dose adjustment and/or reduce frequency of adverse events thus providing safer treatments for patients with an mTOR inhibitor disclosed herein, e.g., rapamycin or RAD001.

In some instances, the present disclosure provides stable extended release formulations of an mTOR inhibitor disclosed herein, e.g., rapamycin or RAD001, which are multi-particulate systems and may have functional layers and coatings.

The term "extended release, multi-particulate formulation as used herein refers to a formulation which enables release of an mTOR inhibitor disclosed herein, e.g., rapamycin or RAD001, over an extended period of time e.g. over at least 1, 2, 3, 4, 5 or 6 hours. The extended release formulation may contain matrices and coatings made of special excipients, e.g., as described herein, which are formulated in a manner as to make the active ingredient available over an extended period of time following ingestion.

The term "extended release" can be interchangeably used with the terms "sustained release" (SR) or "prolonged release". The term "extended release" relates to a pharmaceutical formulation that does not release active drug substance immediately after oral dosing but over an extended in accordance with the definition in the pharmacopoeias Ph. Eur. (7th edition) mongraph for tablets and capsules and USP general chapter <1151> for pharmaceutical dosage forms. The term "Immediate Release" (IR) as used herein refers to a pharmaceutical formulation which releases 85% of the active drug substance within less than 60 minutes in accordance with the definition of "Guidance for Industry: "Dissolution Testing of Immediate Release Solid Oral Dosage Forms" (FDA CDER, 1997). In some embodiments, the term "immediate release" means release of everolismus from tablets within the time of 30 minutes, e.g., as measured in the dissolution assay described herein.

Stable extended release formulations of an mTOR inhibitor disclosed herein, e.g., rapamycin or RAD001, can be characterized by an in-vitro release profile using assays known in the art, such as a dissolution assay as described herein: a dissolution vessel filled with 900 mL phosphate buffer pH 6.8 containing sodium dodecyl sulfate 0.2% at 37°C and the dissolution is performed using a paddle method at 75 rpm according to USP by according to USP testing monograph 711, and Ph.Eur. testing monograph 2.9.3. respectively.

In some embodiments, stable extended release formulations of an mTOR inhibitor disclosed herein, e.g., rapamycin or RAD001, release the mTOR inhibitor in the in-vitro release assay according to following release specifications:
0.5h: <45%, or <40, e.g., <30%
1h: 20-80%, e.g., 30-60%
2h: >50%, or >70%, e.g., >75%
3h: >60%, or >65%, e.g., >85%, e.g., >90%.

In some embodiments, stable extended release formulations of an mTOR inhibitor disclosed herein, e.g., rapamycin or RAD001, release 50% of the mTOR inhibitor not earlier than 45, 60, 75, 90, 105 min or 120 min in the in-vitro dissolution assay.

### Biopolymer delivery methods

In some embodiments, one or more CAR-expressing cells as disclosed herein can be administered or delivered to the subject via a biopolymer scaffold, e.g., a biopolymer implant. Biopolymer scaffolds can support or enhance the delivery, expansion, and/or dispersion of the CAR-expressing cells described herein. A biopolymer scaffold comprises a biocompatible (e.g., does not substantially induce an inflammatory or immune response) and/or a biodegradable polymer that can be naturally occurring or synthetic.

Examples of suitable biopolymers include, but are not limited to, agar, agarose, alginate, alginate/calcium phosphate cement (CPC), beta-galactosidase (β-GAL), (1 ,2,3,4,6-pentaacetyl α-D-galactose), cellulose, chitin, chitosan, collagen, elastin, gelatin, hyaluronic acid collagen, hydroxyapatite, poly(3-hydroxybutyrate-co-3-hydroxy-hexanoate) (PHBHHx), poly(lactide), poly(caprolactone) (PCL), poly(lactide-co-glycolide) (PLG), polyethylene oxide (PEO), poly(lactic-co-glycolic acid) (PLGA), polypropylene oxide (PPO), polyvinyl alcohol) (PVA), silk, soy protein, and soy protein isolate, alone or in combination with any other polymer composition, in any concentration and in any ratio. The biopolymer can be augmented or modified with adhesion- or migration-promoting molecules, e.g., collagen-mimetic peptides that bind to the collagen receptor of lymphocytes, and/or stimulatory molecules to enhance the delivery, expansion, or function, e.g., anti-cancer activity, of the cells to be delivered. The biopolymer scaffold can be an injectable, e.g., a gel or a semi-solid, or a solid composition.

In some embodiments, CAR-expressing cells described herein are seeded onto the biopolymer scaffold prior to delivery to the subject. In embodiments, the biopolymer scaffold further comprises one or more additional therapeutic agents described herein (e.g., another CAR-expressing cell, an antibody, or a small molecule) or agents that enhance the activity of a CAR-expressing cell, e.g., incorporated or conjugated to the biopolymers of the scaffold. In embodiments, the biopolymer scaffold is injected, e.g., intratumorally, or surgically implanted at the tumor or within a proximity of the tumor sufficient to mediate an anti-tumor effect. Additional examples of biopolymer compositions and methods for their delivery are described in Stephan et al., Nature Biotechnology, 2015, 33:97-101; and WO2014/110591.

### Pharmaceutical compositions and treatments

Pharmaceutical compositions employed in the present invention may comprise a CAR-expressing cell, e.g., a plurality of CAR-expressing cells, in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions employed in the present invention are in one aspect formulated for intravenous administration.

Pharmaceutical compositions employed in the present invention may be administered in a manner appropriate to the disease to be treated (or prevented). The quantity and frequency of administration will be determined by such factors as the condition of the patient, and the type and severity of the patient's disease, although appropriate dosages may be determined by clinical trials.

In one embodiment, the pharmaceutical composition is substantially free of, e.g., there are no detectable levels of a contaminant, e.g., selected from the group consisting of endotoxin, mycoplasma, replication competent lentivirus (RCL), p24, VSV-G nucleic acid, HIV gag, residual anti-CD3/anti-CD28 coated beads, mouse antibodies, pooled human serum, bovine serum albumin, bovine serum, culture media components, vector packaging cell or plasmid components, a bacterium and a fungus. In one embodiment, the bacterium is at least one selected from the group consisting of Alcaligenes faecalis, Candida albicans, Escherichia coli, Haemophilus influenza, Neisseria meningitides, Pseudomonas aeruginosa, Staphylococcus aureus, Streptococcus pneumonia, and Streptococcus pyogenes group A.

When "an immunologically effective amount," "an anti-tumor effective amount," "a tumor-inhibiting effective amount," or "therapeutic amount" is indicated, the precise amount of the compositions employed in the present invention to be administered can be determined by a physician with consideration of individual differences in age, weight, tumor size, extent of infection or metastasis, and condition of the patient (subject). It can generally be stated that a pharmaceutical composition comprising the immune effector cells (e.g., T cells, NK cells) described herein may be administered at a dosage of 10⁴ to 10⁹ cells/kg body weight, in some instances 10⁵ to 10⁶ cells/kg body weight, including all integer values within those ranges. T cell compositions may also be administered multiple times at these dosages. The cells can be administered by using infusion techniques that are commonly known in immunotherapy (see, e.g., Rosenberg et al., New Eng. J. of Med. 319:1676, 1988).

In certain aspects, it may be desired to administer activated immune effector cells (e.g., T cells, NK cells) to a subject and then subsequently redraw blood (or have an apheresis performed), activate immune effector cells (e.g., T cells, NK cells) therefrom according to the present disclosure, and reinfuse the patient with these activated and expanded immune effector cells (e.g., T cells, NK cells). This process can be carried out multiple times every few weeks. In certain aspects, immune effector cells (e.g., T cells, NK cells) can be activated from blood draws of from 10cc to 400cc. In certain aspects, immune effector cells (e.g., T cells, NK cells) are activated from blood draws of 20cc, 30cc, 40cc, 50cc, 60cc, 70cc, 80cc, 90cc, or 100cc.

The administration of the subject compositions may be carried out in any convenient manner, including by aerosol inhalation, injection, ingestion, transfusion, implantation or transplantation. The compositions described herein may be administered to a patient trans arterially, subcutaneously, intradermally, intratumorally, intranodally, intramedullary, intramuscularly, by intravenous (i.v.) injection, or intraperitoneally. In one aspect, the T cell compositions of the present invention are administered to a patient by intradermal or subcutaneous injection. In one aspect, the T cell compositions of the present invention are administered by i.v. injection. The compositions of immune effector cells (e.g., T cells, NK cells) may be injected directly into a tumor, lymph node, or site of infection.

In a particular exemplary aspect, subjects may undergo leukapheresis, wherein leukocytes are collected, enriched, or depleted ex vivo to select and/or isolate the cells of interest, e.g., T cells. These T cell isolates may be expanded by methods known in the art and treated such that one or more CAR constructs employed in the invention may be introduced, thereby creating a CAR T cell of the invention. Subjects in need thereof may subsequently undergo standard treatment with high dose chemotherapy followed by peripheral blood stem cell transplantation. In certain aspects, following or concurrent with the transplant, subjects receive an infusion of the expanded CAR T cells of the present invention. In an additional aspect, expanded cells are administered before or following surgery.

The dosage of the above treatments to be administered to a patient will vary with the precise nature of the condition being treated and the recipient of the treatment. The scaling of dosages for human administration can be performed according to art-accepted practices. The dose for CAMPATH, for example, will generally be in the range 1 to about 100 mg for an adult patient, usually administered daily for a period between 1 and 30 days. The preferred daily dose is 1 to 10 mg per day although in some instances larger doses of up to 40 mg per day may be used (described in U.S. Patent No. 6,120,766).

In one embodiment, the CAR is introduced into immune effector cells (e.g., T cells, NK cells), e.g., using in vitro transcription, and the subject (e.g., human) receives an initial administration of CAR immune effector cells (e.g., T cells, NK cells) of the invention, and one or more subsequent administrations of the CAR immune effector cells (e.g., T cells, NK cells) of the invention, wherein the one or more subsequent administrations are administered less than 15 days, e.g., 14, 13, 12, 11, 10, 9, 8, 7, 6, 5, 4, 3, or 2 days after the previous administration. In one embodiment, more than one administration of the CAR immune effector cells (e.g., T cells, NK cells) of the invention are administered to the subject (e.g., human) per week, e.g., 2, 3, or 4 administrations of the CAR immune effector cells (e.g., T cells, NK cells) of the invention are administered per week. In one embodiment, the subject (e.g., human subject) receives more than one administration of the CAR immune effector cells (e.g., T cells, NK cells) per week (e.g., 2, 3 or 4 administrations per week) (also referred to herein as a cycle), followed by a week of no CAR immune effector cells (e.g., T cells, NK cells) administrations, and then one or more additional administration of the CAR immune effector cells (e.g., T cells, NK cells) (e.g., more than one administration of the CAR immune effector cells (e.g., T cells, NK cells) per week) is administered to the subject. In another embodiment, the subject (e.g., human subject) receives more than one cycle of CAR immune effector cells (e.g., T cells, NK cells), and the time between each cycle is less than 10, 9, 8, 7, 6, 5, 4, or 3 days. In one embodiment, the CAR immune effector cells (e.g., T cells, NK cells) are administered every other day for 3 administrations per week. In one embodiment, the CAR immune effector cells (e.g., T cells, NK cells) of the invention are administered for at least two, three, four, five, six, seven, eight or more weeks.

In one aspect, CAR-expressing cells of the present inventions are generated using lentiviral viral vectors, such as lentivirus. Cells, e.g., CARTs, generated that way will have stable CAR expression.

In one aspect, CAR-expressing cells, e.g., CARTs, are generated using a viral vector such as a gammaretroviral vector, e.g., a gammaretroviral vector described herein. CARTs generated using these vectors can have stable CAR expression.

In one aspect, CARTs transiently express CAR vectors for 4, 5, 6, 7, 8, 9, 10, 11, 12, 13, 14, 15 days after transduction. Transient expression of CARs can be effected by RNA CAR vector delivery. In one aspect, the CAR RNA is transduced into the T cell by electroporation.

A potential issue that can arise in patients being treated using transiently expressing CAR immune effector cells (e.g., T cells, NK cells) (particularly with murine scFv bearing CARTs) is anaphylaxis after multiple treatments.

Without being bound by this theory, it is believed that such an anaphylactic response might be caused by a patient developing humoral anti-CAR response, i.e., anti-CAR antibodies having an anti-IgE isotype. It is thought that a patient's antibody producing cells undergo a class switch from IgG isotype (that does not cause anaphylaxis) to IgE isotype when there is a ten to fourteen day break in exposure to antigen.

If a patient is at high risk of generating an anti-CAR antibody response during the course of transient CAR therapy (such as those generated by RNA transductions), CART infusion breaks should not last more than ten to fourteen days.

### EXAMPLES

The invention is further described in detail by reference to the following experimental examples. These examples are provided for purposes of illustration only, and are not intended to be limiting unless otherwise The invention is defined by the attached claims.

### Example 1: PD-1 CAR

In one embodiment, the extracellular domain (ECD) of inhibitory molecules, e.g., Programmed Death 1 (PD-1), can be fused to a transmembrane domain and intracellular signaling domains such as 4-1BB and CD3 zeta. In one instance, the PD-1 CAR can be used alone. In one instance, the PD-1 CAR can be used in combination with another CAR, e.g., CD19CAR. In one embodiment, the PD-1 CAR improves the persistence of the T cell. In one embodiment, the CAR is a PD-1 CAR comprising the extracellular domain of PD-1 indicated as underlined in SEQ ID NO: 26 (PD-1 domain is underlined)

Malpvtalllplalllhaarppgwfldspdrpwnpptfspallvvtegdnatftcsfsntsesfvlnwyrmspsn
qtdklaafpedrsqpgqdcrfrvtqlpngrdfhmsvvrarrndsgtylcgaislapkaqikeslraelrvterraevptahpspspr
pagqfqtlvtttpaprpptpaptiasqplslrpeacrpaaggavhtrgldfacdiyiwaplagtcgvlllslvitlyckrgrkkllyifk
qpfmrpvqttqeedgcscrfpeeeeggcelrvkfsrsadapaykqgqnqlynelnlgrreeydvldkrrgrdpemggkprrkn
pqeglynelqkdkmaeayseigmkgerrrgkghdglyqglstatkdtydalhmqalppr SEQ ID NO: 26

The corresponding nucleotide sequence for the PD-1 CAR is shown below, with the PD-1 ECD underlined below in SEQ ID NO: 27 (PD-1 domian is underlined)

Atggccctccctgtcactgccctgcttctccccctcgcactcctgctccacgccgctagaccacccggatggtttct
ggactctccggatcgcccgtggaatcccccaaccttctcaccggcactcttggttgtgactgagggcgataatgcgaccttcacgtg
ctcgttctccaacacctccgaatcattcgtgctgaactggtaccgcatgagcccgtcaaaccagaccgacaagctcgccgcgtttcc
ggaagatcggtcgcaaccgggacaggattgtcggttccgcgtgactcaactgccgaatggcagagacttccacatgagcgtggtc

cgcgctaggcgaaacgactccgggacctacctgtgcggagccatctcgctggcgcctaaggcccaaatcaaagagagcttgagg
gccgaactgagagtgaccgagcgcagagctgaggtgccaactgcacatccatccccatcgcctcggcctgcggggcagtttcag
accctggtcacgaccactccggcgccgcgcccaccgactccggccccaactatcgcgagccagcccctgtcgctgaggccgga
agcatgccgccctgccgccggaggtgctgtgcatacccggggattggacttcgcatgcgacatctacatttgggctcctctcgccgg
aacttgtggcgtgctccttctgtccctggtcatcaccctgtactgcaagcggggtcggaaaaagcttctgtacattttcaagcagccctt
catgaggcccgtgcaaaccacccaggaggaggacggttgctcctgccggttccccgaagaggaagaaggaggttgcgagctgc
gcgtgaagttctcccggagcgccgacgcccccgcctataagcagggccagaaccagctgtacaacgaactgaacctgggacgg
cgggaagagtacgatgtgctggacaagcggcgcggccgggaccccgaaatgggcgggaagcctagaagaaagaaccctcag
gaaggcctgtataacgagctgcagaaggacaagatggccgaggcctactccgaaattgggatgaagggagagcggcggaggg
gaaaggggcacgacggcctgtaccaaggactgtccaccgccaccaaggacacatacgatgccctgcacatgcaggcccttcccc
ctcgc SEQ ID NO: 27

Other examples of inhibitory molecules in include PD1, CTLA4, TIM3, LAG3, VISTA, BTLA, TIGIT, LAIR1, CD160, 2B4 and TGFR beta. PD1 is an inhibitory member of the CD28 family of receptors that also includes CD28, CTLA-4, ICOS, and BTLA. PD-1 is expressed on activated B cells, T cells and myeloid cells (Agata et al. 1996 Int. Immunol 8:765-75). Two ligands for PD1, PD-L1 and PD-L2 have been shown to downregulate T cell activation upon binding to PD1 (Freeman et a. 2000 J Exp Med 192:1027-34; Latchman et al. 2001 Nat Immunol 2:261-8; Carter et al. 2002 Eur J Immunol 32:634-43). PD-L1 is abundant in human cancers (Dong et al. 2003 J Mol Med 81:281-7; Blank et al. 2005 Cancer Immunol. Immunother 54:307-314; Konishi et al. 2004 Clin Cancer Res 10:5094). Immune suppression can be reversed by inhibiting the local interaction of PD1 with PD-L1.

Jurkat cells with NFAT-LUC reporter (JNL) were grown to the density of 0.5 × 10⁶/ml in Jurkat cell growth media with puromycin at 0.5 µg/ml. For each transfection 3 × 10⁶ cells were spin down at 100g for 10 minutes. Four µg DNA per construct were used per transfection. Amaxa Nucleofector solution V and supplement I was mixed and 100 µl was added into the tube with DNA construct. The mixture was then added to the cells and transferred to the electroporation cuvette. Electroporation was done under setting X-001 using Amaxa Nucleofector II Device. 0.5 ml of growth media was added immediately after eletroporation and the mixture were transferred into 2 ml growth media in one well of the 6-well plate. After two hours, the rapalogue compound at various concentrations was added to cells. The cells were applied to tissue culture plate wells that were coated by the target. Tissue culture plate was coated with 5 µg/ml of PDL1-Fc or IgG1-Fc or any target for 2 hrs at 37°C, then blocked with the blocking buffer (DPBS with 5% serum) for 30 minutes. The transfected cells were added to the target plate with 100 µl per well and incubated further for 16 hrs. Luciferase One Glo reagent 100 µl was added per well. The samples were incubated for 5 min at 37°C and then luminescence is measured using Envision plate reader.

The PD1 CAR construct comprises PD1-ECD -TM - 4-1BB - CD3zeta. This construct may improve the persistence of cells transfected with the construct, e.g., CART cells transfected with PD1 CAR.

As shown in FIG. 32: PD1 CAR showed significant PD1 induced activation of NFAT inducible promoter driven luciferase activity, as compared to the control treatment by IgG1-Fc. This suggest that PD1 interaction with PDL-1 is sufficient in causing clustering of PD1 on Jurkat cell surface and triggers the strong activation of the NFAT pathway.

### Example 2: A camelid single VHH domain-based CAR can be expressed on a T cell surface in combination with a scFv-based CAR without appreciable receptor interaction

Material and Method: Jurkat T cells expressing GFP under an NFAT-dependent promoter (NF-GFP) were transduced with either a mesothelin-specific activating CAR (SS1-CAR), CD19-specific activating (19-CAR) or a CAR generated using a camelid VHH domain specific to EGFR (VHH-CAR). Following transduction with the activating CAR, the cells were then transduced with an additional inhibitory CAR recognizing CD19 (19-PD1) to generate cells co-expressing both the activating and inhibitory CAR (SS1+19PD1, 19+19PD1 or VHH+19PD1). The transduced Jurkat T cells were co-cultured for 24 hours with different cell lines that are either 1) devoid of all target antigens (K562), 2) express mesothelin (K-meso), CD19 (K-19) or EGFR (A431) only, 3) express a combination of EGFR and mesothelin (A431-mesothelin) or CD19 (A431-CD19) or 4) express a combination of CD19 and mesothelin (K-19/meso). Additional conditions that include either no stimulator cells (no stim) or K562 with 1 ug/mL of OKT3 (OKT3) were also included as negative and positive controls for NFAT activation, respectively. GFP expression, as a marker of NFAT activation, was assessed by flow cytometry.

Result: Camels and related species (e.g. Llama) naturally produce antibodies that have a single heavy-chain like variable domain. This domain, known as a camelid VHH domain, has evolved to exist without pairing to a light chain variable domain. It was found that the possibility that two heterologous scFv molecules can dissociate and re-associate with one another when displayed on the surface of a cell as demonstrated by the disruption in scFv binding to cognate ligand during receptor co-expression. The present example showed the expected reduced interaction between a scFv CAR displayed on the surface of a cell in combination with a VHH domain-based CAR. It was found that coexpression of two scFv-based CARs (SS1-z activating CAR and CD19-PD1 inhibitory CAR) on the surface of a Jurkat leads to the inability of the activating CAR (SS1-z) to recognize its cognate ligand on the target cell and trigger T cell activation despite the absence of the inhibitory receptor's ligand. This is consistent with the observed reduced ligand binding on the surface. In contrast, the coexpression of the same inhibitory CAR (CD19-PD1) with a camelid VHH-based activating CAR (VHH-z) has no impact on the ability of the VHH-based activating CAR to recognize its cognate ligand. These data support the model that a VHH-based activating CAR can be expressed with an scFv-based CAR without significant interaction between the receptors due to the reduced ability of the scFv and VHH domains to interact.

### Example 3: CART targeting folate receptor-alpha expressing tumor

Folate receptor α (FRA) is over expressed in approximately 90% of ovarian carcinomas, as well as in cancers of the endometrium, kidney, breast, lung, pancreas, colorectal cancer and mesothelioma, and its expression is not affected by prior administration of chemotherapy (see, e.g., Despierre et al., Gynecol Oncol 130, 192-199 (2013)). In normal tissues, FRA expression is null or low and restricted to the apical surface of polarized epithelial cells (Kelemen et al., International journal of cancer 119, 243-250 (2006)), where it appears to be inaccessible to circulating anti-FR drugs.

CAR-T cell therapy in oncology was first tested in ovarian cancer, where administration of T cells engineered to express an anti-FRA CAR composed of the murine MOv18 scFv and a CD3z endodomain was shown to be feasible but did not induce tumor regression due to the poor persistence of the gene-modified T cells (Kershaw et al, Clin Cancer Res 12, 6106-6115 (2006)).

In this example, we constructed a fully human anti-FRA C4 CAR to reduce the risk of potential CAR transgene immunogenicity. Although the binding affinity of the human C4 Fab fragment (2 ×10⁷ M⁻¹) is approximately five-fold less than that of the high affinity murine MOv19 antibody (Figini, M. et al. (1998) Cancer Res 58, 991-996), it retains its specificity for FRA and its K(d) of <10⁸ M⁻¹ is predicted to confer exclusive activation of CAR upon encounter with tumor cells bearing high amounts of surface FRA. The targeting domain is linked to a combined intracellular CD27 and CD3z signaling chain to further enhance the efficacy of this receptor (referred to hereafter as "C4-27z"). In addition, the C4-27z CAR has reduced activity against normal cells bearing low level antigen and decreases the potential risk of on-antigen, off-tumor toxicity. These results lead to fully human C4 CAR T cell therapy for the safe and effective treatment of a wide spectrum of FRA-expressing malignancies.

### Material and Methods

***Cell lines.*** Lentivirus packaging was performed in the immortalized normal fetal renal 293T cell line purchased from ATCC. Human cell lines used in immune based assays include the established human ovarian cancer cell lines SKOV3, A1847, OVCAR2, OVCAR3, OVCAR4, OVCAR5, A2780, A2008, C30, and PEO-1. The human lymphoid cell lines SUP-T1 was used for lentivirus titer analysis. For bioluminescence assays, target cancer cell lines were transfected to express firefly luciferase (fLuc). The mouse malignant mesothelioma cell line, AE17 (kindly provided by Steven Albelda, University of Pennsylvania) was used as negative control. All cell lines were maintained in R10 medium: RPMI-1640 supplemented with 10% heat inactivated FBS, 100U/mL penicillin, 100mg/mL streptomycin sulfate, 10mmol/L HEPES).

***CAR construction and lentivirus production**.* The pHEN2 plasmid containing the anti-FRa C4/AFRA4 scFv kindly provided by Dr. Silvana Canevari (Figini, M. et al. (1998) Cancer Res 58, 991-996) was used as a template for PCR amplification of a 729-bp C4 fragment using the following primers: 5'-ataggatcccagctggtggagtctgggggaggc-3' (BamHI is underlined) and 5'-atagctagcacctaggacggtcagcttggtccc-3' (Nhel is underlined). Third generation self-inactivating lentiviral expression vectors pELNS previously described were digested with BamHI and Nhel and gel purified. The digested PCR products were then inserted into the pELNS vector containing CD3z or CD27-CD3z T cell signaling domains in which transgene expression is driven by the elongation factor-la (EF-1α) promoter. The resulting construct was designated pELNS-C4-z or C4-27z. High-titer replication-defective lentiviral vectors were produced and concentrated as previously described in Parry, R.V., et al. (2003) The Journal of Immunology 171, 166-174. Briefly, 293T cells were seeded in 150 cm² flask and transfected using Express In (Open Biosystems) according to manufacturer's instructions. Fifteen micrograms of FR-specific CAR transgene plasmid were cotransfected with 7ug pVSV-G (VSV glycoprotein expression plasmid), 18ug pRSV.REV (Rev expression plasmid) and 18ug pMDLg/p.RRE (Gag/Pol expression plasmid) with 174ul Express In (1ug/ul) per flask. Supernatants were collected at 24h and 48h after transfection, concentrated 10-fold by ultracentrifugation for 2 hours at 28,000 rpm with a Beckman SW32Ti rotor (Beckman Coulter). Alternatively, a single collection of the media was done 30 hr after media change. Virus containing media is alternatively used unconcentrated or concentrated by Lenti-X concentrator (Clontech, Cat# 631232). The viruses were aliquoted into tubes and stored at -80°C until ready to use for titering or experiments. All lentiviruses used in the experiments were from concentrated stocks.

***Determination of lentiviral titer.*** Titers of concentrated lentiviral vectors encoding FRA CAR were determined by serially (3-fold) diluting vector preparations in R10 medium and transduce SUP-T1 cells. Briefly, SUP-T1 cells (20,000cells/100ul/well) were seeded in a single well of a 96-well plate and 50ul 3-fold diluted vector supernatant was transferred and incubated overnight. The next day, feed the cells with 100ul prewarmed R10 medium. Two days post transduction, vector titers were determined by flow cytometry applying standard flow cytometric methods for analysis of CAR expression. The titers (transducing units [TU] = (% positive/100) x 2E4 x 20 x dilution. All the experiments were repeated at least three times and average titers obtained from the experiments were used for data analysis.

***Human T cells and transfection**.* Primary human CD4+ and CD8+ T cells, purchased from the Human Immunology Core at University of Pennsylvania, were isolated from healthy volunteer donors following leukapheresis by negative selection. All specimens were collected under a protocol approved by a University Institutional Review Board, and written informed consent was obtained from each donor. T cells were cultured in R10 medium and stimulated with anti-CD3 and anti-CD28 monoclonal antibodies (mAb)-coated beads (Invitrogen). Eighteen to 24 hours after activation, human T cells were transduced using a spinoculation procedure. Briefly, 0.5×10⁶ T cells were infected with a multiplicity of infection (MOI) of 2 and 5 of concentrated C4-27z and MOv19-27z vector, respectively. Mixtures of cells and vectors were centrifuged at room temperature for 90min (2,500rpm) in a table-top centrifuge (Sorvall ST 40). Human recombinant interleukin-2 (IL-2; Novartis) was added every 2-3 days to a 100IU/mL final concentration and a cell density of 0.5 ×10⁶ to 1 ×10⁶ cells/mL was maintained. Once engineered T-cell cultures appeared to rest down, as determined by both decreased growth kinetics and cell-sizing determined using the Multisizer 3 Coulter Counter (Beckman Coulter), a Nexcelom Cellometer Vision or Millipore Scepter, the T cells were used for functional analysis.

***Flow cytometric analysis.*** The following monoclonal antibodies were used for phenotypic analysis: APC-Cy7 anti-human CD3; FITC antihuman CD4; APC anti-human CD8; PE-anti-human CD45; PE anti-human CD137. 7-Aminoactinomycin D (7-AAD) was used for viability staining. All monoclonal antibodies were purchased from BD Biosciences. In T cell transfer experiments, peripheral blood was obtained via retro-orbital bleeding and stained for the presence of human CD45, CD4, and CD8 T cells. After gating on the human CD45+ population, the CD4+ and CD8+ subsets were quantified using TruCount tubes (BD Biosciences) with known numbers of fluorescent beads as described in the manufacturer's instructions. Tumor cell surface expression of FRa was performed using MOv18 mAb followed by APC-labeled goat anti mouse Ab. T cell surface expression of the both C4 and MOv19 CAR was evaluated using biotin-labeled recombinant FRa protein (R&D Systems, Inc) followed by Streptavidin-APC (eBioscience, Inc.) or biotin-labeled rabbit anti-human IgG and goat anti-Mouse IgG F(ab')₂ fragment followed by Streptavidin-APC, respectively. For intracellular cytokine staining, cells were stimulated in culture medium containing phosphomolybdic acid (PMA) (30 ng/ mL) (Sigma-Aldrich), ionomycin (500 ng/mL) (Sigma-Aldrich), and monensin (GolgiStop) (1µL/mL) (BD Biosciences) in a cell incubator with 10% CO2 at 37 °C for 4 h. To determine cytokine production in CAR T cells, cells were cocultured with FR^{pos} ovarian cancer cells for 5 h. After surface markers were stained, cells were fixed and permeabilized using Cytofix/Cytoperm and Perm/Wash buffer (BD Biosciences) according to the manufacturer's instructions. Then cells were stained with fluorescence-conjugated cytokine antibodies including PE anti-human IFN-γ, Pacific blue anti-human TNF-α or FITC anti-human IL-2 before analysis. Flow cytometry was performed with a BD FACSCanto II flow cytometer (BD Biosciences) and flow cytometric data were analyzed with FlowJo version 7.2.5 software (Tree Star, Ashland, OR).

***Cytokine release assays.*** Cytokine release assays were performed by coculture of 1×10⁵ T cells with 1×10⁵ target cells per well in triplicate in 96-well flat bottom plates in a 200ul volume of R10 medium. After 20-24 hours, coculture supernatants were assayed for presence of IFN-γ using an ELISA Kit, according to manufacturer's instructions (Biolegend, San Diego, CA). Values represent the mean of triplicate wells.

*Cytotoxicity assays.* For the cell-based bioluminescence assays, 5 × 10⁴ firefly Luciferase (fLuc)-expressing tumor cells were cultured with R10 media in the presence of different ratios of transduced T cells with the use of a 96-well Microplate (BD Biosciences). After incubation for ~20 hours at 37°C, each well was filled with 50 uL of DPBS resuspended with 1 ul of D-luciferin (0.015 g/mL) and imaged with the Xenogen IVIS Spectrum. Percent tumor cell viability was calculated as the mean luminescence of the experimental sample minus background divided by the mean luminescence of the input number of target cells used in the assay minus background times 100. All data are represented as a mean of triplicate wells.

CAR T cells (5 × 10⁵) were cocultured with 5 × 10⁵ FR^{pos} A1847 cancer cells or FR^{neg} AE17 cells in 1ml in 24-well plate. GolgiStop (BD Biosciences) was added after coculture. Cells were then cultured for an additional 4 h. Cultures were stained for MOv19 or C4 scFv, followed by CD3 and CD8. Permeabilized cells were then stained intracellularly for IFN-g, TNF-a, and IL-2 production. T cells were gated on CD3 and CD8 expression and further analyzed for cytokine expression using a Boolean gate platform to assess all of the possible patterns of cytokine responses.

***Xenograft model of ovarian cancer**.* All animals were obtained from the Stem Cell and Xenograft Core of the Abramson Cancer Center, University of Pennsylvania. Six to 12-week-old NOD/SCID/y-chain-/- (NSG) mice were bred, treated and maintained under pathogen-free conditions in-house under University of Pennsylvania IACUC approved protocols. For an established ovarian cancer model, 6 to 12-week-old female NSG mice were inoculated s.c. with 3 × 10⁶ SKOV3 fLuc+ cells on the flank on day 0. After tumors become palpable at about 1 month, human primary T cell (CD4+ and CD8+T cells used were mixed at 1:1 ratio) were activated, and transduced as described above. After 2 weeks T cell expansion, when the tumor burden was ~200-300 mm³, mice were treated with T cells. The route, dose, and timing of T-cell injections is indicated in the individual figure legends. Tumor dimensions were measured with calipers, and tumor volumes calculated using the formula V = 1/2(length × width²), where length is greatest longitudinal diameter and width is greatest transverse diameter. Animals were imaged prior to T cell transfer and about every week thereafter to evaluate tumor growth. Photon emission from fLuc+ cells was quantified using the "Living Image" software (Xenogen) for all *in vivo* experiments. Tumors were resected immediately after euthanasia approximately 40 days after first T cell dose for size measurement and immunohistochemistry.

For the intraperitoneal model of ovarian cancer, NSG mice were injected i.p. with 5 × 10⁶ SKOV3 fLuc+ cells. Twenty days after peritoneal inoculation, mice bearing well-established SKOV3 tumors were divided into groups and treated. Mice were sacrificed and necropsied when the mice became distressed and moribund. To monitor the extent of tumor progression, the mice were imaged weekly or biweekly and body weights of the mice were measured. In all models, 4-5 mice were randomized per group prior to treatment.

***Bioluminescence imaging.*** Tumor growth was also monitored by Bioluminescent imaging (BLI). BLI was done using Xenogen IVIS imaging system and the photons emitted from fLuc-expressing cells within the animal body were quantified using Living Image software (Xenogen). Briefly, mice bearing SKOV3 fLuc+ tumor cells were injected intraperitoneally with D-luciferin (150 mg/kg stock, 100 µL of D-luciferin per 10 grams of mouse body weight) suspended in PBS and imaged under isoflurane anesthesia after 5~10 minutes. A pseudocolor image representing light intensity (blue, least intense; red, most intense) was generated using Living Image. BLI findings were confirmed at necropsy.

***Statistical analysis.*** The data are reported as means and SD. Statistical analysis was performed by the use of 2-way repeated-measures ANOVA for the tumor burden (tumor volume, photon counts). Student t test was used to evaluate differences in absolute numbers of transferred T cells, cytokine secretion, and specific cytolysis. GraphPad Prism 5.0 (GraphPad Software) was used for the statistical calculations. *P<0.05* was considered significant.

### Results

### 1. Enhanced function of the human C4 CAR compared to murine MOvl9 CAR in vitro

Using the production and concentration protocols described above, we found that the C4 CAR-encoding lentivirus has a higher effective titer than the murine MOv19 CAR, possibly the result of more efficient expression of the human scFv on human T cells (Figure 37a). Indeed, we observed a multiplicity of infection (MOI) of C4 CAR lentivirus as low as 1 is sufficient to infect >20% human T cells, while the MOv19 CAR lentivirus required a MOI of 5 (Figure 37b). Thus, for the following experiments, T cells were infected with a MOI of 2 and 5 of concentrated C4-27z and MOv19-27z vector, respectively, and both C4 and MOv19 CAR surface expression on T cells were detected via recombinant FRA protein staining (Figure 34a).

ScFvs used for CAR construction require a minimal antigen affinity to achieve activation threshold for the engineered T cell, however, higher affinity scFvs do not necessarily induce a more potent activation of CAR T cells than low affinity scFvs. Since the binding affinity of the human C4 Fab fragment (2 ×10⁷ M⁻¹) is approximately five-fold weaker than that of the murine MOv19, we examined whether the lower affinity of the C4 scFv used to construct the fully human C4 CAR might influence redirected T-cell function via comparison to the MOv19 CAR containing a higher affinity anti-FRA scFv. T cells modified to express either the C4-27z or MOv19-27z CAR specifically lysed FRA^{pos} SKOV3 and A1847 tumor cells with approximately equivalent efficiency in overnight cocultures (Figure 34b). However, *in vitro* cytokine production analysis showed that MOv19-27z CAR T cells secreted significantly less IFN-γ than C4 CAR T cells at an equivalent 1:1 E:T ratio after overnight co-culture (Figure 34c). This result was validated by 5-hour intracellular cytokine production assays. Representative fluorescence activated cell sorter (FACS) plots of 5-hour intracellular cytokine expression by tumor-activated CAR T cells show that both C4 and MOv19 CAR T cells produce IFN-γ, TNF-α and IL-2 cytokines when incubated overnight with FRA^{pos} SKOV3 ovarian cancer cells, but MOv19 CAR T cells produced less of these cytokines than C4 CAR T cells (Figure 34d). The frequency of C4 CAR T cells expressing cytokine was 5.6-fold higher for IFN-γ, 6.1-fold for higher TNF-α and 9-fold higher for IL-2, than that observed in MOv19 CAR T cells *in vitro.* Untransduced T cells cocultured with FRA^{pos} or FRA-negative cancer cells, or CAR T cells cocultured with FRA-negative cancer cells, did not produced proinflammatory cytokines (Figure 38).

Our results *in vitro* suggested that C4 CAR T cells with an intermediate affinity for FRA may be functionally superior to MOv19 CAR T cells with a higher affinity scFv. To understand the mechanisms accounting for reduced function by high affinity MOv19 CAR T cells, we carefully analyzed CAR expression on T cells after co-incubation with antigen-expressing tumor cells. Stimulation with SKOV3 cancer cells, which express a high level of FRA, induced a rapid and marked down-modulation of surface MOv19 CAR expression following antigen engagement (Figure 39). Five hours after exposure to tumor cells, MOv19 CAR frequency was rapidly down-modulated from about 65% of T cells to ~1%. This finding was also confirmed by using FRA^{pos} A1847 cells and breast cancer cell line T47D, which also express high levels of FRA (data not shown). By comparison, the C4 CAR was not markedly down-modulated (Figure 39). Intracellular cytokine expression analysis showed that T cells with maintained C4 CAR surface expression produced IFN-γ, TNF-α and IL-2, while cytokine production was exclusively detected in the CAR-negative fraction of the MOv19 group, indicating that CAR down-modulation and cytokine production had occurred following antigen encounter.

There was a similar frequency of Annexin V+/7-AAD+ as measured by apoptosis staining in T cells modified with C4 compared with MOv19-CARs after stimulation with SKOV3 cells, respectively. CARs with CD28 domain had lower AICD compared with 4-1BB (R12: 16.4%/18.4% vs. 2A2 38.1%/39.6%).

Overall avidity between CAR and target molecule may account for this observed difference in CAR expression. We evaluated the impact of T cell to target cell ratio on relative CAR expression by C4 or MOv19 CAR T cells following co-culture with SKOV3 cells. At lower E:T ratios of 1:10, 1:3 and 1:1, MOv19 CAR T cells showed a marked, dose-dependent down-modulation in CAR expression compared with C4 CAR, which maintained ~50% of initial CAR expression at the lowest E:T ratio tested. However, at high E:T ratios of 3:1 and 10:1 where tumor antigen is more limiting, T cells bearing either C4 or MOv19 CAR maintained high CAR expression (Figure 40). Consistent with changes in CAR expression after antigen stimulation, C4 CAR T cells released more IFN-γ than MOv19 CAR T cells at E:T ratios of 1:10, 1:3 and 1:1, but similar amounts at E:T ratios of 3:1 and 10:1 (Figure 34e). Thus, CAR down-modulation occurs in an antigen dose-dependent fashion with anti-FRA CAR T cells bearing the high affinity MOv19 scFv being more sensitive to low antigen level.

### 2. Comparable antitumor activity of C4 and MOv19 CAR T cells in vivo

To compare the antitumor capacity of C4 CAR T cells with MOv19 CAR T cells *in vivo,* NSG mice with large, established subcutaneous SKOV3 tumors (~300 mm³) received intravenous injections of 10⁷ CAR+ T cells on days 40 and 47 post-tumor inoculation. Tumors in animals treated with saline, untransduced T cells or CD19-27z CAR T cells continued to grow rapidly. In contrast, mice receiving C4-27z or MOv19-27z CAR T cells experienced tumor regression (*p*<0.0001), compared with all 3 control groups at the latest evaluated time point. The antitumor activity of MOv19-27z CAR T cells appeared slightly better than that of C4-27z CAR T cells, but not at a significant level (*p*=0.058; Figure 35a). BLI of tumor xenografts before and 3 weeks after T cells injection showed progressive growth of tumors in all animals receiving control T cells but not in CAR T cells groups (Figure 35b). Tumor BLI results were consistent with the size of resected residual tumors (Figure 35c). Next, we analyzed the persistence of transferred T-cells in the peripheral blood 3 weeks following adoptive transfer and detected higher numbers of CD4+ and CD8+ T cells in mice treated with both the C4 and MOv19 CAR T cells groups compared with the UNT and CD19-27z CAR T cells treatment group (Figure 35d), suggesting that tumor antigen recognition drives the survival of the adoptively transferred T cells in vivo. These results demonstrated that the anti-tumor activity of C4 CAR are comparable to MOv19 CAR which was well described previously (see, e.g., Song et al., Blood 119, 696-706 (2012); Song et al., Cancer Res 71, 4617-4627 (2011)) and confirm that the C4 CAR, despite its decreased affinity, is suitable for *in vivo* application.

### 3. Anti-FRA CAR with lower affinity may decrease the risk of "on-target" toxicity

On-target toxicities have been observed in clinical trials with CAR T-cells specific for tumor associated antigens that are expressed at low levels on normal cells, and a critical issue to be addressed is whether CARs with higher affinity may increase the risk of toxicity. To investigate the functional effect of primary human T cells modified with C4 CAR and MOv19 CAR on normal cells expressing low levels of FRA, we analyzed cytokine production of C4 CAR and MOv19 CAR T-cells after co-culture with human embryonic kidney 293T cells or normal epithelial ovarian cell line IOSE 6, which express low but detectable levels of FRA, and FRA^{pos} SKOV3 cells (Figure 36a). C4 and MOv19 CAR T cells responded against SKOV3 with greater activity observed again from C4 CAR T cells. However, greater IFN-γ cytokine production was observed from the MOv19 CAR T cells in response to low antigen expressing cells, suggesting that MOv19 CAR T cells are more functionally avid and sensitive to low antigen (Figure 36b). Similar to what we observed in overnight IFN-γ release assays, 5-hour intracellular cytokine secretion assays showed that more MOv19 CAR T cells produced IFN-γ and TNF-α in response to low antigen on normal cells (Figure 36c), which is one primary proposed contributors to the "on-target" cytokine storm²⁸, as compared with C4 CAR T cells. These data suggest that the new described C4 CAR may have a more appropriate affinity for the delivery of safe and effective engineered T cell therapy.

### 4. C4 CAR with lower affinity for soluble aFR antigen than MOv19 CAR

In vitro results described herein suggested that fully human C4 CAR T cells may be functionally superior to MOv19 CAR T cells and that CAR down-modulation may impair the antitumor activity of MOv19 CAR but not C4 CAR. To understand the mechanisms accounting for reduced function by MOv19 CAR T cells, experiments were performed to measure relative binding to recombinant αFR protein. C4 and MOv19 CAR T cells were pre-loaded with biotin labeled recombinant αFR protein, and measured for surface protein dissociation over time at either 4 or 37 °C in the presence of ten-fold excess non-biotinylated αFR competitor. Antigen retention on the cell surface was assessed by flow cytometry by adding phycoerythrin (PE)-conjugated streptavidin (SA) after the end of each culture period. Within one hour, less αFR protein was detectable on the surface of C4 CAR T cells, in comparison to MOv19 CAR, at either temperature. The level of dissociation was dependent on both time and temperature and was higher in C4 CAR T cells under all conditions tested (Fig. 88A, Fig. 88B). Similar results were obtained in a titration analysis on the binding of biotinylated αFR protein to MOv19 and C4 CAR T cells (Fig. 89). Activated T cells were transduced with lentiviral vector expressing MOv19-27z or C4-27z-CAR and analyzed for CAR expression on day 14. One hundred thousand untransduced (UNT) or CAR T cells were stained with 0.2, 0.5, 1, 2, 5, 10, 20, 50 or 120 nM/sample of biotinylated αFR. T cells were then washed and stained with PE-SA. T cells were analyzed using flow cytometer and the data analyzed with FlowJo software. These results suggest that C4 in the CAR construct had a lower affinity for soluble αFR antigen than MOv19 CAR.

### Conclusion:

The decreased affinity of the fully human C4 scFv selected for designing a CAR could affect T-cell recognition. However, a direct comparison of cytokine production after tumor engagement by T cells modified with the C4 and MOv19 CARs showed that the C4 CAR with lower affinity was superior at an E/T ratio of 1:1. It may be due to the rapid internalization of MOv19 CAR with higher affinity encountering with high levels of antigen. When we increase the E/T ratios, the anti-tumor activity of C4 CAR T cells is comparable to MOv19 CAR T cells. To further compare the antitumor activity in vivo, we found that T cells expressing the high-affinity MOv19 CAR mediated slightly superior activity in vivo compared with the C4 CAR. However, this difference is not statistically significant, suggesting that the affinity of C4 CAR is adequate for in vivo application.

Possible on-target, off-tumor toxicities resulting from the expression of TAAs on normal tissues need to be considered in the application of CAR approach. The development of high affinity CAR or TCR with great anti-tumor activity can lead to severe toxicity. Our study showed that C4 CAR T cells release minimal cytokine compared with MOv19 CAR T cells when encountering with normal cells expressing low levels of FRA. Thus, the relative lower affinity C4 CAR could decrease the risk of on-target toxicity, while the higher affinity MOv19 CAR could increase this risk in vivo.

### Example 4: Decreasing the affinity of CAR increases therapeutic efficacy

Adoptive cell therapy (ACT) with CAR engineered T cells can target and kill widespread malignant cells thereby inducing durable clinical responses in treating some hematopoietic malignancies (Kochenderfer, J.N., et al. (2010) Blood 116:4099-4102; Porter, D.L., et al. (2011) N Engl J Med 365:725-733; and Brentjens, R.J., et al. (2013) Sci Transl Med 5:177ra138). However, many commonly targeted tumor antigens are also expressed by healthy tissues and on target off tumor toxicity from T cell-mediated destruction of normal tissue has limited the development and adoption of this otherwise promising type of cancer therapy. Recent reports on severe adverse events associated with treatment of cancer patients with CAR- or TCR-engineered T lymphocytes further illustrate the critical importantance of target selection for safe and efficient therapy (Lamers et al., 2006, J Clin Oncol. 24:e20; Parkhurst et al., 2011, Molecular therapy: the journal of the American Society of Gene Therapy. 19:620-6; Morgan et al., 2013, J Immunotherapy. 36:133-151; Linette et al., 2013, Blood. 122:863-71). In specific, the targeting of ErbB2 (Her2/neu or CD340) with high affinity CARTs led to serious toxicity due to target recognition on normal cardiopulmonary tissue (Morgan et al., 2013, Mol Therapy. 18:843-851), and similarly, the presence of relatively high levels of EGFR in healthy skin leads to dose-limiting skin toxicity (Perez-Soler et al., 2010, J Clin Oncol. 23:5235-46).

Selecting highly tissue-restricted antigens, cancer testis antigens, mutated gene products or viral proteins as targets could significantly improve the safety profile of using CART cells. However, none of these antigens is present with high frequency in common cancers, constitutively expressed exclusively by malignant cells, functionally important for tumor growth, and targetable with CART. Most of the top-ranked target antigens that could be targeted by CART are expressed in potentially important normal tissues, such as ErbB2, EGFR, MUC1, PSMA, and GD2 (Cheever et al., 2009, Clinical Cancer Research. 15:5323-37). Current strategies for generating CARs consist of selecting scFv with high affinity, as previous studies have shown that the activation threshold inversely correlated with the affinity of the scFv (Chmielewski et al., 2004, J Clin Oncol. 173:7647-53; and Hudecek et al., 2013, Clincial Cancer Research. 19:3153-64. Studies indicate that the costimulatory domain of CARs does not influence the activation threshold (Chmielewski et al., 2011, Gene Therapy. 18:62-72). After TCR stimulation there is a narrow window of affinity for optimal T cell activation, and increasing the affinity of the TCRs does not necessarily improve treatment efficacy (Zhong et al., 2013, Proc Natl Acad Sci USA. 110:6973-8 ; and Schmid et al., 2010, J Immunol. 184:4936-46).

In this example, it was determined whether equipping T cells with high affinity scFv may limit the utility of CARs, due to poor discrimination of the CART for tumors and normal tissues that express the same antigen at lower levels. It was also determined whether fine-tuning the affinity of the scFv could increase the ability of CART cells to discriminate tumors from normal tissues expressing the same antigen at lower levels. CARs with affinities against two validated targets, ErbB2 and EGFR, which are amplified or overexpressed in variety of cancers but are also expressed, at lower levels by normal tissues, were tested extensively against multiple tumor lines, as well as primary cell lines from normal tissues and organs. It was found that decreasing the affinity of the scFv could significantly increase the therapeutic index of CARs while maintaining robust antitumor efficacy.

The following materials and methods were used in the experiments described in this example:

### Cell lines and primary human lymphocytes

SK-BR3, SK-OV3, BT-474, MCF7, MDA231, MDA468, HCC2281, MDA-361, MDA-453, HCC-1419, HCC-1569, UACC-812, LnCap, MDA-175, MCF-10A, HCC38 and HG261 cell lines were purchased from American Type Culture Collection and cultured as instructed. Seven primary cell lines (keratinocytes, osteoblast, renal epithelial, pulmonary artery endothelial cells, pulmonary artery smooth muscle, neural progenitor, CD34+ enriched PBMC) were obtained from Promocell and cultured according to their protocols. Primary lymphocytes were isolated from normal donors by the University of Pennsylvania Human Immunology Core and cultured in R10 medium (RPMI 1640 supplemented with 10% fetal calf serum; Invitrogen). Primary lymphocytes were stimulated with microbeads coated with CD3 and CD28 stimulatory antibodies (Life Technologies, Grand Island, NY, Catalog) as described (Barrett et al., 2009, Proc Nat Acad Sci USA, 106:3360). T cells were cryopreserved at day 10 in a solution of 90% fetal calf serum and 10% dimethylsulfoxide (DMSO) at 1 × 10⁸ cells /vial.

### Generation of CAR constructs for mRNA electroporation and lentiviral transduction.

CAR scFv domains against ErbB2 or EGFR were synthesised and/or amplified by PCR, based on sequencing information provided by the relevant publications (Carter et al., 1992, Proc Nat Acad Sci USA, 89:4285; Zhou et al., 2007, J Mol Bio, 371:934), linked to CD8 transmembrane domain and 4-1BB and CD3Z intracellular signaling domains, and subcloned into pGEM.64A RNA based vector (Zhao et al., 2010, Cancer Res, 70:9053) or pTRPE lentiviral vectors (Carpenito et al., 2009, Proc Nat Acad Sci USA, 106:3360.

### Biacore assay

Biotinylated ErbB2 was mobililzed to a streptavidin coated sensor chip at a density of 200 RU. Binding affinity of the parental 4D5 antibody (Carter et al., 1992, Proc Nat Acad Sci USA, 89:4285) were compared to recombinant scFv. The purity and atomic mass of the scFv were verified by liquid chromatography-mass spe ctrometry. ScFv samples were serial diluted 3-fold and injected over the chip at a constant flow rate. Association and dissociatioin rates of the protein complex were monitored for 270 s and 400 s, respectively. Double referencing was performed against a blank immobilized flow cell and a buffer blank and the data was fit using a 1:1 Langmuir model or steady state affinity where appropriate with the Biacore T200 evaluation software.

### mRNA in vitro transcription and T cell electroporation

T7 mScript systems kit (CellScript) was used to generate IVT RNA. CD3/CD28 bead stimulated T cells were electroporated with IVT RNA using BTX EM830 (Harvard Apparatus BTX) as previously described (Zhao et al., 2010, Cancer Res, 70:9053). Briefely, T cells were washed three times and resuspended in OPTI-MEM (Invitrogen) at a final concentration of 1-3 × 10⁸ cells /ml. Subsequently, 0.1 ml of cells were mixed with 10ug IVT RNA (or as indicated) and electroporated in a 2mm cuvette.

### Flow cytometry analysis

Antibodies were obtained from the following suppliers: anti-human CD3 (BD Biosciences, 555335), anti-human CD8 (BD Biosciences 555366), anti-human CD107a (BD Biosciences 555801), anti-human CD137 (BD Biosciences 555956). Cell surface expression of ErbB2 was detected by biotylated anti-ErbB2 Affibody (Abcam, ab31890), and EGFR by FITC conjugated anti-EGFR affibody (Abcam, ab81872). ErbB2, EGFR and CD19 specific CAR T cell expression were detected by ErbB2-Fc fusion protein (R&D system, 1129-ER), EGFR-Fc fusion protein and biotin-labeled polyclonal goat anti-mouse F(ab)2 antibodies (Jackson Immunoresearch, 115-066-072) respectively, incubated at 4°C for 25 minutes and washed twice (PBS with 2% FBS). Samples were then stained with PE-conjugated anti-human IgG Fc Ab (eBioscience, 12-4998-82) or phycoerythrin-labeled streptavidin (eBioscience, 17-4317-82), incubated at 4°C for 25 minutes and washed once. Flow cytometry acquisition was performed on either a BD FacsCalibur or Accuri C6 Cytometer (BD Biosciences). Analysis was performed using FlowJo software (Treestar).

### ELISA assays

Target cells were washed and suspended at 1× 10⁶ cells/ml in R10 medium (RPMI 1640 supplemented with 10% fetal calf serum; Invitrogen). 100ul each target cell type were added in duplicate to a 96 well round bottom plate (Corning). Effector T cells were washed, and re-suspended at 1× 10⁶ cells/ml in R10 medium and then 100ul of T cells were combined with target cells in the indicated wells. In addition, wells containing T cells alone were prepared. The plates were incubated at 37°C for 18 to 20 hours. After the incubation, supernatant was harvested and subjected to an ELISA assay (eBioscience, 88-7316-77; 88-7025-77).

### CD107a staining

Cells were plated at an E:T of 1: 1 (1 × 10⁵ effectors: 1 × 10⁵ targets) in 160 µl of complete RPMI medium in a 96 well plate. 20µl of phycoerythrin-labeled anti-CD107a Ab (BD Biosciences, 555801) was added and the plate was incubated at 37°C for 1 hour before adding Golgi Stop (2ul Golgi Stop in 3ml RPMI medium, 20ul/well; BD Biosciences, 51-2092KZ ) and incubating for another 2.5 hours. Then 5 µl FITC-anti-CD8 and 5ul APC-anti-CD3 were added and incubated at 37°C for 30 min. After incubation, the samples were washed with FACS buffer and analyzed by flow cytometry.

### CFSE based T cells proliferation assay

Resting CD4 T cells were washed and suspended at a concentration of 1 × 10⁷ cells / ml in PBS. Then 120 ul CFSE working solution (25 µM CFSE) was added to 1 × 10⁷ cells for 3.5 min at 25°C. The labeling was stopped with 5% FBS (in PBS), washed twice with 5% FBS and cultured in R10 with 10 IU/ml IL2. After overnight culture, the CFSE labeled T cells were electroporated with different affinity ErbB2 CAR RNA. Two to four hours after electroporation, T cells were suspended at concentration of 1 × 10⁶/ ml in R10 medium (with 10 IU/ml IL2). Tumor or K562 cell lines were irradiated and suspended at 1 × 10⁶/ mL in R10 medium. Cells were plated at an E:T of 1:1 (5 × 10⁵ effectors: 5 × 10⁵ targets) in 1ml of complete RPMI medium in a 48 well plate. T cells were then counted and fed every 2 days from day 3. CFSE dilution was monitored by flow cytometry at day 3, day 5 and day 7.

### Luciferase based CTL assay.

Nalm6-CBG tumor cells were generated and employed in a modified version of a luciferase based CTL assay as follows: Click beetle green luciferase (CBG) was cloned into the pELNS vector, packaged into lentivirus, transduced into NALM6 tumor cells and sorted for CBG expression. Resulting Nalm6-CBG cells were washed and resuspended at 1 × 10⁵ cells /ml in R10 medium, and 100 ul of CBG-labeled cells were incubated with different ratios of T cells (e.g. 30:1, 15:1, etc) overnight at 37°C. 100µl of the mixture was transferred to a 96 well white luminometerplate, 100ul of substrate was added and the the luminescence was immediately determined.

### Mouse xenograft studies

Studies were performed as previously described with certain modifications (Barrett et al., 2011, Human Gene Therapy, 22:1575; and Carpenito et al., 2009, PNAS, 106:336). Briefly, 6-10 week old NOD scid gamma (NSG) mice were injected subcutaneously with 1 × 10⁶ PC3-CBG tumors cells on the right flank at day 0 and the same mice were given SK-OV3-CBG tumor cells (5 × 10⁶ cells/mouse, s.c.) on the left flank at day 5. The mice were treated with T cells via the tail vein at day 23 post PC3-CBG tumor inoculation such that both tumors were approximately 200 mm³ in volume. Lentivirally transduced T cells were given at 1 × 10⁷ cells /mouse (10M), or 3 × 10⁶ cells /mouse (3M). RNA electroporated T cells were given at 5 × 10⁷ cells/mouse for the 1st treatment, followed by 3 treatments at days 26, 30 and 33 in the dose of 1 × 10⁷ RNA electroporated T cells/mouse.

### RESULTS

### Lowering the affinity of the anti-ErbB2 scFv improves the therapeutic index of ErbB2 CAR T cells in vitro

A panel of tumor lines with a wide range of ErbB2 expression as measured by flow cytometry was compiled (Fig. 1). SK-OV3 (ovarian cancer), SK-BR3 (breast cancer), BT-474 (breast cancer) over-express ErbB2, while EM-Meso (mesothelioma), MCF7 (breast cancer), 293T (embryonic kidney 293 cell), A549 (lung cancer), 624mel (melanoma), PC3 (prostate cancer), MDA231 (breast cancer) express ErbB2 at lower levels and ErbB2 was not detected in MDA468 (breast cancer). ErbB2 mRNA levels were also measured by real time PCR and there was a strong correlation between the two techniques (Fig. 2).

A panel of ErbB2 CARs was constructed making use scFv derived from the published mutations of the parental 4D5 antibody (Carter et al. (1992) Proc Natl Acad Sci USA 89:4285-4289). The sequences encoding the CARs against ErbB2 are provided in Table 2.

**Table 2: Nucleic acid sequences encoding CARs against ErbB2**

| CAR Designation | Nucleic Acid Sequence | SEQ ID NO: |
|---|---|---|
| 4D5-BZZ | atg gac ttc cag gtt cag atc ttt tcg ttc ctg ctg atc agc gcc tct gtt atc atg tcg cgc ggc gac atc cag atg acc cag tcc cct tcc tcc ctc tct gcc tct gtg gga gac cgc gtt acc atc aca tgc cga gct tcc cag gac gtg aac aca gcc gtg gcc tgg tac cag cag aag ccc ggg aag gca ccc aaa ctc ctc atc tac tcc gcc tcc ttc cta tac agt ggc gtg cct tcc cga ttc tcc ggc tcc agg agt ggc acg gac ttt acg ctc acc att agt agc ctg cag ccc gaa gac ttc gcg acc tac tat tgt cag caa cac tac acg acg cca cca act ttc ggc cag ggt acc aag gtc gag att aag cga acc ggc agt acc agt ggg tct ggc aag ccc ggc agc ggc gag gga tcc gag gtc cag ctg gtc gag tcc ggc ggg ggc ctg gtg cag ccg ggc ggc tcg ctg agg tta tct tgc gcc gcc agt ggc ttc aac atc aag gat act tac atc cac tgg gtg agg cag gct ccg ggc aag ggc ctg gaa tgg gtg gct agg atc tac cct act aac ggg tac aca cgc tac gca gat tcg gtg aaa ggc cgc ttc act atc tcc gcc gac acc tcg aag aac act gct tac ctg cag atg aac tcc ctc agg gcc gaa gat act gca gtc tac tac tgc tcc cgc tgg ggt ggg gac ggc ttc tac gcc atg gac gtg tgg ggt cag ggc act cta gtt aca gtg tca tcc acc acg acg cca gcg ccg cga cca cca aca ccg gcg ccc acc atc gcg tcg cag ccc ctg tcc ctg cgc cca gag gcg tgc cgg cca gcg gcg ggg ggc gca gtg cac acg agg ggg ctg gac ttc gcc tgt gat atc tac atc tgg gcg ccc ttg gcc ggg act tgt ggg gtc ctt ctc ctg tca ctg gtt atc acc ctt tac tgc aaa cgg ggc aga aag aaa ctc ctg tat ata ttc aaa caa cca ttt atg aga cca gta caa act act caa gag gaa gat ggc tgt agc tgc cga ttt cca gaa gaa gaa gaa gga gga tgt gaa ctg aga gtg aag ttc agc agg agc gca gac gcc ccc gcg tac aag cag ggc cag aac cag ctc tat aac gag ctc aat cta gga cga aga gag gag tac gac gtt ttg gac aag aga cgt ggc cgg gac cct gag atg ggg gga aag ccg aga agg aag aac cct cag gaa ggc ctg tac aat gaa ctg cag aaa gat aag atg gcg gag gcc tac agt gag att ggg atg aaa ggc gag cgc cgg agg ggc aag ggg cac gat ggc ctt tac cag ggt ctc agt aca gcc acc aag gac acc tac gac gcc ctt cac atg cag gcc ctg ccc cct cgc taa | 40 |
| 4D5-1-BBZ | atg gac ttc cag gtt cag atc ttt tcg ttc ctg ctg atc agc gcc tct gtt atc atg tcg cgc ggc gac atc cag atg acc cag tcc cct tcc tcc ctc tct gcc tct gtg gga gac cgc gtt acc atc aca tgc cga gct tcc cag gac gtg aac aca gcc gtg gcc tgg tac cag cag aag ccc ggg aag gca ccc aaa ctc ctc atc tac tcc gcc tcc ttc cta gag agt ggc gtg cct tcc cga ttc tcc ggc tcc ggc agt ggc acg gac ttt acg ctc acc att agt agc ctg cag ccc gaa gac ttc gcg acc tac tat tgt cag caa cac tac acg acg cca cca act ttc ggc cag ggt acc aag gtc gag att aag | 41 |
| | cga acc ggc agt acc agt ggg tct ggc aag ccc ggc agc ggc gag gga tcc gag gtc cag ctg gtc gag tcc ggc ggg ggc ctg gtg cag ccg ggc ggc tcg ctg agg tta tct tgc gcc gcc agt ggc ttc aac atc aag gat act tac atc cac tgg gtg agg cag gct ccg ggc aag ggc ctg gaa tgg gtg gct agg atc tac cct act aac ggg tac aca cgc tac gca gat tcg gtg aaa ggc cgc ttc act atc tcc agg gac gac tcg aag aac act ctg tac ctg cag atg aac tcc ctc agg gcc gaa gat act gca gtc tac tac tgc gcc cgc tgg ggt ggg gac ggc ttc gta gcc atg gac gtg tgg ggt cag ggc act cta gtt aca gtg tca tcc acc acg acg cca gcg ccg cga cca cca aca ccg gcg ccc acc atc gcg tcg cag ccc ctg tcc ctg cgc cca gag gcg tgc cgg cca gcg gcg ggg ggc gca gtg cac acg agg ggg ctg gac ttc gcc tgt gat atc tac atc tgg gcg ccc ttg gcc ggg act tgt ggg gtc ctt ctc ctg tca ctg gtt atc acc ctt tac tgc aaa cgg ggc aga aag aaa ctc ctg tat ata ttc aaa caa cca ttt atg aga cca gta caa act act caa gag gaa gat ggc tgt agc tgc cga ttt cca gaa gaa gaa gaa gga gga tgt gaa ctg aga atg gac ttc cag gtt cag atc ttt tcg ttc ctg ctg atc agc gcc tct gtt atc atg tcg cgc ggc gac atc cag atg acc cag tcc cct tcc tcc ctc tct gcc tct gtg gga gac cgc gtt acc atc aca tgc cga gct tcc cag gac gtg aac aca gcc gtg gcc tgg tac cag cag aag ccc ggg aag gca ccc aaa ctc ctc atc tac tcc gcc tcc ttc cta gag agt ggc gtg cct tcc cga ttc tcc ggc tcc ggc agt ggc acg gac ttt acg ctc acc att agt agc ctg cag ccc gaa gac ttc gcg acc tac tat tgt cag caa cac tac acg acg cca cca act ttc ggc cag ggt acc aag gtc gag att aag cga acc ggc agt acc agt ggg tct ggc aag ccc ggc agc ggc gag gga tcc gag gtc cag ctg gtc gag tcc ggc ggg ggc ctg gtg cag ccg ggc ggc tcg ctg agg tta tct tgc gcc gcc agt ggc ttc aac atc aag gat act tac atc cac tgg gtg agg cag gct ccg ggc aag ggc ctg gaa tgg gtg gct agg atc tac cct act aac ggg tac aca cgc tac gca gat tcg gtg aaa ggc cgc ttc act atc tcc agg gac gac tcg aag aac act ctg tac ctg cag atg aac tcc ctc agg gcc gaa gat act gca gtc tac tac tgc gcc cgc tgg ggt ggg gac ggc ttc gta gcc atg gac gtg tgg ggt cag ggc act cta gtt aca gtg tca tcc gtg aag ttc agc agg agc gca gac gcc ccc gcg tac aag cag ggc cag aac cag ctc tat aac gag ctc aat cta gga cga aga gag gag tac gac gtt ttg gac aag aga cgt ggc cgg gac cct gag atg ggg gga aag ccg aga agg aag aac cct cag gaa ggc ctg tac aat gaa ctg cag aaa gat aag atg gcg gag gcc tac agt gag att ggg atg aaa ggc gag cgc cgg agg ggc aag ggg cac gat ggc ctt tac cag ggt ctc agt aca gcc acc aag gac acc tac gac gcc ctt cac atg cag gcc ctg ccc cct cgc taa | |
| 4D5-3-BBZ | acc acg acg cca gcg ccg cga cca cca aca ccg gcg ccc acc atc gcg tcg cag ccc ctg tcc ctg cgc cca gag gcg tgc cgg cca gcg gcg ggg ggc gca gtg cac acg agg ggg ctg gac ttc gcc tgt gat atc tac atc tgg gcg ccc ttg gcc ggg act tgt ggg gtc ctt ctc ctg tca ctg gtt atc acc ctt tac tgc aaa cgg ggc aga aag aaa ctc ctg tat ata ttc aaa caa cca ttt atg aga cca gta caa act act caa gag gaa gat ggc tgt agc tgc cga ttt cca gaa gaa gaa atg gac ttc cag gtt cag atc ttt tcg ttc ctg ctg atc agc gcc tct gtt atc atg tcg cgc ggc gac atc cag atg acc cag tcc cct tcc tcc ctc tct gcc tct gtg gga gac cgc gtt acc atc aca tgc cga gct tcc cag gac gtg aac aca gcc gtg gcc tgg tac cag cag aag ccc ggg aag gca ccc aaa ctc ctc atc tac tcc gcc tcc ttc cta gag agt ggc gtg cct tcc cga ttc tcc ggc tcc ggc agt ggc acg gac ttt acg ctc acc att | 42 |
| | agt agc ctg cag ccc gaa gac ttc gcg acc tac tat tgt cag caa cac tac acg acg cca cca act ttc ggc cag ggt acc aag gtc gag att aag cga acc ggc agt acc agt ggg tct ggc aag ccc ggc agc ggc gag gga tcc gag gtc cag ctg gtc gag tcc ggc ggg ggc ctg gtg cag ccg ggc ggc tcg ctg agg tta tct tgc gcc gcc agt ggc ttc aac atc aag gat act tac atc cac tgg gtg agg cag gct ccg ggc aag ggc ctg gaa tgg gtg gct agg atc tac cct act aac ggg tac aca cgc tac gca gat tcg gtg aaa ggc cgc ttc act atc tcc gcc gac acc tcg aag aac act gct tac ctg cag atg aac tcc ctc agg gcc gaa gat act gca gtc tac tac tgc tcc cgc tgg ggt ggg gac ggc ttc gta gcc atg gac gtg tgg ggt cag ggc act cta gtt aca gtg tca tcc gaa gga gga tgt gaa ctg aga gtg aag ttc agc agg agc gca gac gcc ccc gcg tac aag cag ggc cag aac cag ctc tat aac gag ctc aat cta gga cga aga gag gag tac gac gtt ttg gac aag aga cgt ggc cgg gac cct gag atg ggg gga aag ccg aga agg aag aac cct cag gaa ggc ctg tac aat gaa ctg cag aaa gat aag atg gcg gag gcc tac agt gag att ggg atg aaa ggc gag cgc cgg agg ggc aag ggg cac gat ggc ctt tac cag ggt ctc agt aca gcc acc aag gac acc tac gac gcc ctt cac atg cag gcc ctg ccc cct cgc taa | |
| 4D5-5-BBZ | atg gac ttc cag gtt cag atc ttt tcg ttc ctg ctg atc agc gcc tct gtt atc atg tcg cgc ggc gac atc cag atg acc cag tcc cct tcc tcc ctc tct gcc tct gtg gga gac cgc gtt acc atc aca tgc cga gct tcc cag gac gtg aac aca gcc gtg gcc tgg tac cag cag aag ccc ggg aag gca ccc aaa ctc ctc atc tac tcc gcc tcc ttc cta gag agt ggc gtg cct tcc cga ttc tcc ggc tcc agg agt ggc acg gac ttt acg ctc acc att agt agc ctg cag ccc gaa gac ttc gcg acc tac tat tgt cag caa cac tac acg acg cca cca act ttc ggc cag ggt acc aag gtc gag att aag cga acc ggc agt acc agt ggg tct ggc aag ccc ggc agc ggc gag gga tcc gag gtc cag ctg gtc gag tcc ggc ggg ggc ctg gtg cag ccg ggc ggc tcg ctg agg tta tct tgc gcc gcc agt ggc ttc aac atc aag gat act tac atc cac tgg gtg agg cag gct ccg ggc aag ggc ctg gaa tgg gtg gct agg atc tac cct act aac ggg tac aca cgc tac gca gat tcg gtg aaa ggc cgc ttc act atc tcc gcc gac acc tcg aag aac act gct tac ctg cag atg aac tcc ctc agg gcc gaa gat act gca gtc tac tac tgc tcc cgc tgg ggt ggg gac ggc ttc gta gcc atg gac gtg tgg ggt cag ggc act cta gtt aca gtg tca tcc acc acg acg cca gcg ccg cga cca cca aca ccg gcg ccc acc atc gcg tcg cag ccc ctg tcc ctg cgc cca gag gcg tgc cgg cca gcg gcg ggg ggc gca gtg cac acg agg ggg ctg gac ttc gcc tgt gat atc tac atc tgg gcg ccc ttg gcc ggg act tgt ggg gtc ctt ctc ctg tca ctg gtt atc acc ctt tac tgc aaa cgg ggc aga aag aaa ctc ctg tat ata ttc aaa caa cca ttt atg aga cca gta caa act act caa gag gaa gat ggc tgt agc tgc cga ttt cca gaa gaa gaa gaa gga gga tgt gaa ctg aga gtg aag ttc agc agg agc gca gac gcc ccc gcg tac aag cag ggc cag aac cag ctc tat aac gag ctc aat cta gga cga aga gag gag tac gac gtt ttg gac aag aga cgt ggc cgg gac cct gag atg ggg gga aag ccg aga agg aag aac cct cag gaa ggc ctg tac aat gaa ctg cag aaa gat aag atg gcg gag gcc tac agt gag att ggg atg aaa ggc gag cgc cgg agg ggc aag ggg cac gat ggc ctt tac cag ggt ctc agt aca gcc acc aag gac acc tac gac gcc ctt cac atg cag gcc ctg ccc cct cgc taa | 43 |
| 4D5-7-BBZ | atg gac ttc cag gtt cag atc ttt tcg ttc ctg ctg atc agc gcc tct gtt atc atg tcg cgc ggc gac atc cag atg acc cag tcc cct tcc tcc ctc tct gcc tct gtg gga | 44 |
| | gac cgc gtt acc atc aca tgc cga gct tcc cag gac gtg aac aca gcc gtg gcc tgg tac cag cag aag ccc ggg aag gca ccc aaa ctc ctc atc tac tcc gcc tcc ttc cta gag agt ggc gtg cct tcc cga ttc tcc ggc tcc agg agt ggc acg gac ttt acg ctc acc att agt agc ctg cag ccc gaa gac ttc gcg acc tac tat tgt cag caa cac tac acg acg cca cca act ttc ggc cag ggt acc aag gtc gag att aag cga acc ggc agt acc agt ggg tct ggc aag ccc ggc agc ggc gag gga tcc gag gtc cag ctg gtc gag tcc ggc ggg ggc ctg gtg cag ccg ggc ggc tcg ctg agg tta tct tgc gcc gcc agt ggc ttc aac atc aag gat act tac atc cac tgg gtg agg cag gct ccg ggc aag ggc ctg gaa tgg gtg gct agg atc tac cct act aac ggg tac aca cgc tac gca gat tcg gtg aaa ggc cgc ttc act atc tcc gcc gac acc tcg aag aac act gct tac ctg cag atg aac tcc ctc agg gcc gaa gat act gca gtc tac acc acg acg cca gcg ccg cga cca cca aca ccg gcg ccc acc atc gcg tcg cag ccc ctg tcc ctg cgc cca gag gcg tgc cgg cca gcg gcg ggg ggc gca gtg cac acg agg ggg ctg gac ttc gcc tgt gat atc tac atc tgg gcg ccc ttg gcc ggg act tgt ggg gtc ctt ctc ctg tca ctg gtt atc acc ctt tac tgc aaa cgg ggc aga aag aaa ctc ctg tat ata ttc aaa caa cca ttt atg aga cca gta caa act act caa gag gaa gat ggc tgt agc tgc cga ttt cca gaa gaa gaa gaa gga gga tgt gaa ctg aga gtg aag ttc agc agg agc gca gac gcc ccc gcg tac aag cag ggc cag aac cag ctc tat aac gag ctc aat cta gga cga aga gag gag tac gac gtt ttg gac aag aga cgt ggc cgg gac cct gag atg ggg gga aag ccg aga agg aag aac cct cag gaa ggc ctg tac aat gaa ctg cag aaa gat aag atg gcg gag gcc tac agt gag att ggg atg aaa ggc gag cgc cgg agg ggc aag ggg cac gat ggc ctt tac cag ggt ctc agt aca gcc acc aag gac acc tac gac gcc ctt cac atg cag gcc ctg ccc cct cgc taa | |

The monovalent affinities of the ErbB2 scFvs varied by approximately 3 orders of magnitude (Table 3), in contrast to the corresponding mutant antibodies that retained binding affinities within 10-fold of each other (Carter, P., et al. 1992).

**Table 3. Comparison of measured affinities of the wild type 4D5 and mutated antibody with the corresponding scFv**

| **Sample** | **Mutation** | **Antibody KD (nM)** | **scFv KD (nM)** |
|---|---|---|---|
| 4D5 | Wild Type | 0.3 | 0.58 |
| 4D5-7 | 1 in CDR2 | 0.62 | 3.2 |
| 4D5-5 | 1 in CDR3, 1 in CDR2 | 1.1 | 1119 |
| 4D5-3 | 1 in framework, 1 in CDR3, 1 in CDR2 | 4.4 | 3910 |

CARs were constructed by linking the various scFv to the CD8 alpha hinge and transmembrane domain followed by the 4-1BB and CD3ζ intracellular signaling domains. The CARs were expressed by lentiviral vector technology or by cloning into an RNA-based vector (Zhao et al., 2010, Cancer Res, 70:9053). After production of mRNA by *in vitro* transcription and electroporation into T cells, the surface expression of the panel of affinity-modified ErbB2 RNA CARs was similar (Fig. 3). To compare recognition thresholds, the panel of ErbB2 CAR T cells was stimulated with ErbB2 high expressing (SK-BR3, SK-OV3 and BT-474) or low expressing tumor cell lines (MCF7, 293T, A549, 624Mel, PC3, MDA231 and MDA468) and T cell activation was assessed by upregulation of CD137 (4-1BB; Fig. 4), secretion of IFN-γ (Fig. 5) and IL-2 (Fig. 6) and induction of surface CD107a expression (Fig. 7). T cells expressing a CD19-specific CAR served as control for allogeneic reactivity. Lower affinity CAR T cells (4D5-5 and 4D5-3) were strongly reactive to tumors with amplified ErbB2 expression and exhibited undetectable or low reactivity to the tumor lines that expressed ErbB2 at lower levels. In contrast, higher affinity CAR T cells (4D5 and 4D5-7) showed strong reactivity to tumor lines expressing high and low levels of ErbB2, as evidenced by CD137 up-regulation, cytokine secretion and CD107a translocation. These results were extended by assaying additional ErbB2-expressing cell lines (Fig. 8 and 9). Interestingly, higher affinity CAR T cells secreted greater levels of IFN-γ and IL-2 when exposed to targets expressing low levels of ErbB2, while lower affinity CAR T cells secreted more cytokines when exposed to cells expressing high levels of target (Fig. 5 and 6). As expected, the CD19-BBζ CAR was not reactive against ErbB2-expressing cell lines. In summary, higher affinity 405-BBζ T cells recognized all the *ErbB2* expressing lines tested, whereas CARs with lower affinity scFvs, 405-5-BBζ or 4D5-3- BBζ, were highly reactive to all tumor lines with overexpressed ErbB2, but displayed negligible reactivity to cell lines expressing low or undetectable levels of ErbB2.

### ErbB2 CARs with lower affinity scFvs discriminate between tumor cells expressing low and high levels of ErbB2.

To exclude any tumor-specific effects that might contribute to the above results, the activity of the panel of ErbB2-BBζ CAR T cells was assayed against a single tumor line expressing varying levels of ErbB2 (K562 cells electroporated with varying amounts of *ErbB2* RNA). In agreement, it was observed that T cells expressing higher affinity scFvs (4D5 and 4D5-7) recognized K562 cells electroporated with *ErbB2* RNA at doses as low as 0.001µg, which is 100 fold lower than the flow cytometrically detectable level of 0.1µg mRNA (Fig. 10, 11A, and 11B). In contrast the CARs with lower affinity scFvs (4D5-5 and 4D5-3) only recognized K562 electroporated *ErbB2* RNA at doses of 0.5 µg (4D5-5; 10) or higher, indicating that CAR T cell sensitivity was decreased by 2000- (4D5-3) to 500- fold (4D5-5) compared to the high affinity 4D5 CAR T cells. Moreover, the antigen dose associated reactivity observed with lower affinity ErbB2 CARs (4D5-5 and 4D5-3; Fig.11A and 11B), was confirmed by performing a CFSE-based proliferation assay (Fig. 12). Interestingly, decreasing the CAR RNA dose 5 fold (from 10µg RNA/100µl T cells to 2µg RNA/100 µl T cells), further increased the antigen recognition threshold of the T cells with lower and high affinity CARs as assessed by cytokine secretion, suggesting that fine tuning of CAR density on the surface of the T cells is an important variable, or that doses above 2µg of mRNA may have some toxicity on overall T cell activity.

A luciferase based cytolytic T cell (CTL) assay was used to determine whether T cells with affinity decreased CARs could maintain potent killing activity against ErbB2 over expressing targets while sparing cells expressing lower ErbB2 levels. When Nalm6 target cells were transfected with 10µg *ErbB2* RNA, T cells with either higher or lower affinity ErbB2 CARs effectively lysed target cells (Fig. 13A). CARs with higher affinity scFv (4D5 and 4D5-7) exhibit potent lytic activity against target cells transfected with 1µg *ErbB2* RNA, but lower affinity scFvs (4D5-5 and 4D5-3) showed decreased killing activity (Fig. 13B). Finally, only CARs with higher affinity scFvs were able to kill target cells expressing very low amounts of target after electroporation with 0.1 µg ErbB2 RNA (Fig. 13C). Since Nalm6 is a CD19 positive cell line, CART19 maintained cytolytic activity independent of levels of transfected *ErbB2* RNA. These data indicate that that fine-tuning the affinity of ErbB2 CAR T cells enhances discrimination of ErbB2 over-expressing tumor from tumor cells that have low or undetectable levels of ErbB2 expression.

### Affinity decreased ErbB2 CAR T cells fail to recognize physiological levels of ErbB2

Given the previous serious adverse event which occurred upon administration of the high affinity ErbB2 CAR that incorporated the scFv from the parental 4D5 trastuzumab antibody (Morgan et al., 2010, Mol Therapy, 18:843), it is of paramount importance to evaluate potential reactivity of the reduced affinity ErbB2 CAR T cells to physiological levels of ErbB2 expression. To address this, seven primary cell lines isolated from different organs were tested for ErbB2 expression. Most of the primary lines had detectable levels of surface ErbB2, with the neural progenitor line expressing the highest levels of ErbB2 (Fig. 14). T cells expressing the high affinity 4D5 CAR were strongly reactive to all primary lines tested, as evidenced by levels of CD107a up-regulation (Fig. 15). However, T cells expressing the affinity decreased ErbB2 CARs 4D5-5 and 4D5-3 exhibited no reactivity to the primary lines with the exception of weak reactivity to the neural progenitor line. These results were confirmed by analysis of a larger panel of cell lines that had low or undetectable levels of ErbB2 by flow cytometry (Figs. 8 and 9).

### Comparable effects with affinity-tuned ErbB2 CARs expressed using lentiviral transduction or RNA electroporation

To establish comparability between T cells permanently expressing CARs by lentiviral transduction with mRNA electroporated CAR T cells, the panel of affinity-tuned CARs was expressed in T cells from the same normal donor using either lentiviral transduction or mRNA electroporation (Fig 16A). T cells were stimulated with tumor cell lines (Fig. 16B), or K562 cells, expressing varying amounts of ErbB2 (Fig. 16C). CAR T cell recognition and activation was monitored by CD107a upregulation (Fig. 17 and 18), CD137 upregulation (Fig. 19) and IFN-γ secretion (Fig. 20 and 21). In agreement with the previous ErbB2 mRNA CAR T cell results, T cells that constitutively expressed high affinity CARs showed strong reactivity to all cell lines expressing ErbB2; no correlation was observed between antigen expression levels and T cell- activity. In contrast, T cells with low affinity CARs expressed by lentiviral technology demonstrated a robust correlation between target antigen expression and activation (Fig. 17, 18, 20, and 21). These results confirm that the sensitivity of ErbB2 antigen recognition is dependent on scFv affinity using both mRNA electroporated and lentiviral transduced CAR T cells.

### Affinity decreased ErbB2 CAR T cells eliminate tumor in vivo and ignore tissues expressing physiological levels of ErbB2

To extend the above *in vitro* results, a series of experiments were conducted in NSG mice with advanced vascularized tumor xenografts. Based on data above in Figure 1, the human ovarian cancer cell line SK-OV3 was selected as a representative ErbB2 over-expressing tumor and PC3, a human prostate cancer line, was chosen to model normal tissue ErbB2 levels. The antitumor efficacy of ErbB2 CAR T cells expressing either the high affinity 4D5 scFv or the low affinity 45D-5 scFv in NSG mice was compared with day 18 established flank SK-OV3 tumors (Fig. 22). Serial bioluminescence imaging revealed that both the high and low affinity CAR T cells resulted in the rapid elimination of the tumors.

To further evaluate the therapeutic index of the low affinity ErbB2 CAR T cells *in vivo,* a mouse model was designed to simultaneously compare the efficacy and normal tissue toxicity of the high affinity (4D5:BBζ) and low affinity (4D5-5:BBζ) ErbB2 CARs. SK-OV3 and PC3 tumor cell lines were injected subcutaneously into opposite flanks of the same NSG mouse and T cells were administered when tumor volumes reached approximately 200 mm³. Mice were injected (i.v.) with either 3×10⁶ or 1×10⁷ CAR T cells on day 22 and serial bioluminescence imaging and tumor size assessements were conducted. Mice treated with either dose of the CAR T cells exhibited nearly complete regression of the ErbB2 overexpressing SK-OV3 tumor (Fig. 23, 24, and 25). In addition, almost complete regression of the PC3 tumor expressing ErbB2 at low levels on the opposite flank was also seen for the mice treated with high affinity 4D5-based CAR T cells. In contrast, the progressive tumor growth of PC3 was observed in the mice treated with low affinity 4D5-5-based CAR T cells, indicating that whereas the lower affinity CAR T cells were efficacious against ErbB2 overexpressing tumor, they show limited or no detectable reactivity against cells expressing ErbB2 at physiological levels. Moreover, the selective tumor elimination was observed in mice treated at both high and low doses of CAR T cells. The above effects were not due to allorecognition because progressive tumor growth of of both tumors was observed in mice treated with mock transduced T cells.

### Affinity-tuning of scFv increases the therapeutic index of EGFR CAR T cells

To test the broader applicability the strategy to fine tune the affinity of the scFv, we evaluated a panel of EGFR CARs. EGFR:BBζ CARs were constructed from scFvs derived from the parental human anti-EGFR antibody C10 (Heitner et al., 2001, J Immunol Methods, 248:17-30. The nucleic acid sequences encoding the EGFR CARs are provided in Table 4.

**Table 4: Nucleic Acid Sequences of Exemplary EGFR CARs**

| CAR designation | Nucleic Acid Sequence | SEQ ID NO: |
|---|---|---|
| C10-BBZ | atg ggt tgg tcg tgc att atc ctc ttc ctc gtc gca acc gct acc ggc gtt cac tcg gat tac aag gat gac gac gac aaa gag gta cag ctg gtg cag agc ggg gcc gag gtt aag aag ccc ggg tct tcc gta aag gtg tcc tgc aag gcc tcg ggg ggc aca ttc tca tcg tac gca ata tcg tgg gtg cgg cag gcc ccc ggg cag ggg ctg gaa tgg atg ggc gga att atc cca atc ttc ggg acc gcc aac tat gcc cag aag ttt cag ggt cgt gtg acc att act gcc gac gag tcc acc agt acg gcc tac atg gag ctg agt agt | 45 |
| | ctg cgt agc gag gat act gcc gtt tat tat tgc gcc cgg gaa gag gga ccg tac tgc tcg tcg acc tca tgt tac ggc gcc ttc gac atc tgg ggc caa ggc acc ctg gtg acg gtg tcc tcc ggt ggt ggc gga agt ggc ggc ggg ggg tcc ggc ggg ggc ggt tca cag tcc gtc ctg acc cag gat ccc gcg gtg tcg gtc gcg ctg ggt cag aca gta aag ata aca tgc cag ggc gat tct ctg cgc agt tat ttc gcc tcg tgg tac cag cag aaa ccc ggc cag gct cct acc ctt gtt atg tac gcg cgc aat gac aga ccc gcg ggc gtg ccc gac cgc ttc tcc ggc tca aag agc ggg acc tcc gcc tcc ctg gcc atc tcc ggg ctc cag tct gag gat gag gcc gat tac tac tgc gct gct tgg gac gac tcc ctc aat ggc tat ctg ttt ggc gca ggc aca aag ctg acc gtg ctc acc acg acg cca gcg ccg cga cca cca aca ccg gcg ccc acc atc gcg tcg cag ccc ctg tcc ctg cgc cca gag gcg tgc cgg cca gcg gcg ggg ggc gca gtg cac acg agg ggg ctg gac ttc gcc tgt gat atc tac atc tgg gcg ccc ttg gcc ggg act tgt ggg gtc ctt ctc ctg tca ctg gtt atc acc ctt tac tgc aaa cgg ggc aga aag aaa ctc ctg tat ata ttc aaa caa cca ttt atg aga cca gta caa act act caa gag gaa gat ggc tgt agc tgc cga ttt cca gaa gaa gaa gaa gga gga tgt gaa ctg aga gtg aag ttc agc agg agc gca gac gcc ccc gcg tac aag cag ggc cag aac cag ctc tat aac gag ctc aat cta gga cga aga gag gag tac gac gtt ttg gac aag aga cgt ggc cgg gac cct gag atg ggg gga aag ccg aga agg aag aac cct cag gaa ggc ctg tac aat gaa ctg cag aaa gat aag atg gcg gag gcc tac agt gag att ggg atg aaa ggc gag cgc cgg agg ggc aag ggg cac gat ggc ctt tac cag ggt ctc agt aca gcc acc aag gac acc tac gac gcc ctt cac atg cag gcc ctg ccc cct cgc taa | |
| 2224-BBZ | atg ggt tgg tcg tgc att atc ctc ttc ctc gtc gca acc gct acc ggc gtt cac tcg gat tac aag gat gac gac gac aaa gag gta cag ctg gtg cag agc ggg gcc gag gtt aag aag ccc ggg tct tcc gta aag gtg tcc tgc aag gcc tcg ggg ggc aca ttc tca tcg tac gca ata ggt tgg gtg cgg cag gcc ccc ggg cag ggg ctg gaa tgg atg ggc gga att atc cca atc ttc ggg atc gcc aac tat gcc cag aag ttt cag ggt cgt gtg acc att act gcc gac gag tcc acc agt agt gcc tac atg gag ctg agt agt ctg cgt agc gag gat act gcc gtt tat tat tgc gcc cgg gaa gag gga ccg tac tgc tcg tcg acc tca tgt tac gca gcc ttc gac atc tgg ggc caa ggc acc ctg gtg acg gtg tcc tcc ggt ggt ggc gga agt ggc ggc ggg ggg tcc ggc ggg ggc ggt tca cag tcc gtc ctg acc cag gat ccc gcg gtg tcg gtc gcg ctg ggt cag aca gta aag ata aca tgc cag ggc gat tct ctg cgc agt tat ttc gcc tcg tgg tac cag cag aaa ccc ggc cag gct cct acc ctt gtt atg tac gcg cgc aat gac aga ccc gcg ggc gtg ccc gac cgc ttc tcc ggc tca aag agc ggg acc tcc gcc tcc ctg gcc atc tcc ggg ctc cag ccc gag gat gag gcc gat tac tac tgc gct gct tgg gac gac tcc ctc aat ggc tat ctg ttt ggc gca ggc aca aag ctg acc gtg ctc acc acg acg cca gcg ccg cga cca cca aca ccg gcg ccc acc atc gcg tcg cag ccc ctg tcc ctg cgc cca gag gcg tgc cgg cca gcg gcg ggg ggc gca gtg cac acg agg ggg ctg gac ttc gcc tgt gat atc tac atc tgg gcg ccc ttg gcc ggg act tgt ggg gtc ctt ctc ctg tca ctg gtt atc acc ctt tac tgc aaa cgg ggc aga aag aaa ctc ctg tat ata ttc aaa caa cca ttt atg aga cca gta caa act act caa gag gaa gat ggc tgt agc tgc cga ttt cca gaa gaa gaa gaa gga gga tgt gaa ctg aga gtg aag ttc agc agg agc gca gac gcc ccc gcg tac aag cag ggc cag aac cag ctc tat aac gag ctc aat cta gga cga aga gag gag tac gac gtt ttg gac aag aga cgt ggc cgg gac cct gag atg ggg gga aag ccg aga agg aag aac cct cag gaa ggc ctg tac aat gaa ctg cag aaa gat aag atg gcg gag gcc tac agt gag att ggg | 46 |
| | atg aaa ggc gag cgc cgg agg ggc aag ggg cac gat ggc ctt tac cag ggt ctc agt aca gcc acc aag gac acc tac gac gcc ctt cac atg cag gcc ctg ccc cct cgc taa | |
| 3524-BBZ | atg ggt tgg tcg tgc att atc ctc ttc ctc gtc gca acc gct acc ggc gtt cac tcg gat tac aag gat gac gac gac aaa gag gta cag ctg gtg cag agc ggg gcc gag gtt aag aag ccc ggg tct tcc gta aag gtg tcc tgc aag gcc tcg ggg ggc aca ttc tca tcg tac gca ata tcg tgg gtg cgg cag gcc ccc ggg cag ggg ctg gaa tgg gtc ggc gga att atc cca atc ttc ggg acc gcc aac tat gcc cag aag ttt cag ggt cgt gtg aag att act gcc gac gag tcc gca agt acg gcc tac atg gag ctg agt agt ctg cgt agc gag gat act gcc gtt tat tat tgc gcc cgg gaa gag gga ccg tac tgc tcg tcg acc tca tgt tac gca gcc ttc gac atc tgg ggc caa ggc acc ctg gtg acg gtg tcc tcc ggt ggt ggc gga agt ggc ggc ggg ggg tcc ggc ggg ggc ggt tca cag tcc gtc ctg acc cag gat ccc gcg gtg tcg gtc gcg ctg ggt cag aca gta aag ata aca tgc cag ggc gat tct ctg cgc agt tat ctg gcc tcg tgg tac cag cag aaa ccc ggc cag gct cct acc ctt gtt acc tac gcg cgc aat gac aga ccc gcg ggc gtg ccc gac cgc ttc tcc ggc tca aag agc ggg acc tcc gcc tcc ctg gcc atc tcc ggg ctc cag tct gag gat gag gcc gat tac tac tgc gct gct tgg gac gac tcc ctc aat ggc tat ctg ttt ggc gca ggc aca aag ctg acc gtg ctc acc acg acg cca gcg ccg cga cca cca aca ccg gcg ccc acc atc gcg tcg cag ccc ctg tcc ctg cgc cca gag gcg tgc cgg cca gcg gcg ggg ggc gca gtg cac acg agg ggg ctg gac ttc gcc tgt gat atc tac atc tgg gcg ccc ttg gcc ggg act tgt ggg gtc ctt ctc ctg tca ctg gtt atc acc ctt tac tgc aaa cgg ggc aga aag aaa ctc ctg tat ata ttc aaa caa cca ttt atg aga cca gta caa act act caa gag gaa gat ggc tgt agc tgc cga ttt cca gaa gaa gaa gaa gga gga tgt gaa ctg aga gtg aag ttc agc agg agc gca gac gcc ccc gcg tac aag cag ggc cag aac cag ctc tat aac gag ctc aat cta gga cga aga gag gag tac gac gtt ttg gac aag aga cgt ggc cgg gac cct gag atg ggg gga aag ccg aga agg aag aac cct cag gaa ggc ctg tac aat gaa ctg cag aaa gat aag atg gcg gag gcc tac agt gag att ggg atg aaa ggc gag cgc cgg agg ggc aag ggg cac gat ggc ctt tac cag ggt ctc agt aca gcc acc aag gac acc tac gac gcc ctt cac atg cag gcc ctg ccc cct cgc taa | 47 |
| P3-5BBZ | atg ggt tgg tcg tgc att atc ctc ttc ctc gtc gca acc gct acc ggc gtt cac tcg gat tac aag gat gac gac gac aaa gag gta cag ctg gtg cag agc ggg gcc gag gtt aag aag ccc ggg tct tcc gta aag gtg tcc tgc aag gcc tcg ggg ggc aca ttc tca tcg tac gca ata tcg tgg gtg cgg cag gcc ccc ggg cag ggg ctg gaa tgg gtc ggc gga att atc cca atc ttc ggg acc gcc aac tat gcc cag aag ttt cag ggt cgt gtg aag att act gcc gac gag tcc gca agt acg gcc tac atg gag ctg agt agt ctg cgt agc gag gat act gcc gtt tat tat tgc gcc cgg gaa gag gga ccg tac tgc tcg tcg acc tca tgt tac ggc gcc ttc gac atc tgg ggc caa ggc acc ctg gtg acg gtg tcc tcc ggt ggt ggc gga agt ggc ggc ggg ggg tcc ggc ggg ggc ggt tca cag tcc gtc ctg acc cag gat ccc gcg gtg tcg gtc gcg ctg ggt cag aca gta aag ata aca tgc cag ggc gat tct ctg cgc agt tat ctg gcc tcg tgg tac cag cag aaa ccc ggc cag gct cct acc ctt gtt acc tac gcg cgc aat gac aga ccc gcg ggc gtg ccc gac cgc ttc tcc ggc tca aag agc ggg acc tcc gcc tcc ctg gcc atc tcc ggg ctc cag tct gag gat gag gcc gat tac tac tgc gct gct tgg gac gac tcc | 48 |
| | ctc aat ggc tat ctg ttt ggc gca ggc aca aag ctg acc gtg ctc acc acg acg cca gcg ccg cga cca cca aca ccg gcg ccc acc atc gcg tcg cag ccc ctg tcc ctg cgc cca gag gcg tgc cgg cca gcg gcg ggg ggc gca gtg cac acg agg ggg ctg gac ttc gcc tgt gat atc tac atc tgg gcg ccc ttg gcc ggg act tgt ggg gtc ctt ctc ctg tca ctg gtt atc acc ctt tac tgc aaa cgg ggc aga aag aaa ctc ctg tat ata ttc aaa caa cca ttt atg aga cca gta caa act act caa gag gaa gat ggc tgt agc tgc cga ttt cca gaa gaa gaa gaa gga gga tgt gaa ctg aga gtg aag ttc agc agg agc gca gac gcc ccc gcg tac aag cag ggc cag aac cag ctc tat aac gag ctc aat cta gga cga aga gag gag tac gac gtt ttg gac aag aga cgt ggc cgg gac cct gag atg ggg gga aag ccg aga agg aag aac cct cag gaa ggc ctg tac aat gaa ctg cag aaa gat aag atg gcg gag gcc tac agt gag att ggg atg aaa ggc gag cgc cgg agg ggc aag ggg cac gat ggc ctt tac cag ggt ctc agt aca gcc acc aag gac acc tac gac gcc ctt cac atg cag gcc ctg ccc cct cgc taa | |
| P2-4BBZ | atg ggt tgg tcg tgc att atc ctc ttc ctc gtc gca acc gct acc ggc gtt cac tcg gat tac aag gat gac gac gac aaa gag gta cag ctg gtg cag agc ggg gcc gag gtt aag aag ccc ggg tct tcc gta aag gtg tcc tgc aag gcc tcg ggg ggc aca ttc tca tcg tac gca ata tcg tgg gtg cgg cag gcc ccc ggg cag ggg ctg gaa tgg atg ggc gga att atc cca atc ttc ggg acc gcc aac tat gcc cag aag ttt cag ggt cgt gtg acc att act gcc gac gag tcc acc agt acg gcc tac atg gag ctg agt agt ctg cgt agc gag gat act gcc gtt tat tat tgc gcc cgg gaa gag gga ccg tac tgc tcg tcg acc tca tgt tac gca gcc ttc gac atc tgg ggc caa ggc acc ctg gtg acg gtg tcc tcc ggt ggt ggc gga agt ggc ggc ggg ggg tcc ggc ggg ggc ggt tca cag tcc gtc ctg acc cag gat ccc gcg gca tcg gtc gcg ctg ggt cag aca gta aag ata aca tgc cag ggc gat tct ctg cgc agt tat ttc gcc tcg tgg tac cag cag aaa ccc ggc cag gct cct acc ctt gtt atg tac gcg cgc aat gac aga ccc gcg ggc gtg ccc gac cgc ttc tcc ggc tca aag agc ggg acc tcc gcc tcc ctg gcc atc tcc ggg ctc cag tct gag gat gag gcc gat tac tac tgc gct gct tgg gac gac tcc ctc aat ggc tat ctg ttt ggc gca ggc aca aag ctg acc gtg ctc acc acg acg cca gcg ccg cga cca cca aca ccg gcg ccc acc atc gcg tcg cag ccc ctg tcc ctg cgc cca gag gcg tgc cgg cca gcg gcg ggg ggc gca gtg cac acg agg ggg ctg gac ttc gcc tgt gat atc tac atc tgg gcg ccc ttg gcc ggg act tgt ggg gtc ctt ctc ctg tca ctg gtt atc acc ctt tac tgc aaa cgg ggc aga aag aaa ctc ctg tat ata ttc aaa caa cca ttt atg aga cca gta caa act act caa gag gaa gat ggc tgt agc tgc cga ttt cca gaa gaa gaa gaa gga gga tgt gaa ctg aga gtg aag ttc agc agg agc gca gac gcc ccc gcg tac aag cag ggc cag aac cag ctc tat aac gag ctc aat cta gga cga aga gag gag tac gac gtt ttg gac aag aga cgt ggc cgg gac cct gag atg ggg gga aag ccg aga agg aag aac cct cag gaa ggc ctg tac aat gaa ctg cag aaa gat aag atg gcg gag gcc tac agt gag att ggg atg aaa ggc gag cgc cgg agg ggc aag ggg cac gat ggc ctt tac cag ggt ctc agt aca gcc acc aag gac acc tac gac gcc ctt cac atg cag gcc ctg ccc cct cgc taa | 49 |

The monovalent affinities of the panel of EGFR-specific scFvs varied over a range of approximately 300-fold (Zhoe et al., 2007, J Mol Biol, 371:934). The 2224, P2-4, P3-5 and C10 scFvs were cloned into an RNA-based vector and *in vitro* transcribed for T cell mRNA electroporation. Levels of CAR surface expression were assayed and found to be similar among the EGFR constructs (Fig. 26A). To compare reactivities of the panel of EGFR CARs, CAR T cells were stimulated with EGFR-expressing tumor cell lines that have a broad range of EGFR expression at the cell surface (Fig. 26B). CAR T cell activation was evaluated by levels of CD107a up-regulation; the data is summarized in Fig. 27. Higher affinity EGFR CARs (2224:BBζ and P2-4:BBζ) responded to all EGFR positive tumor lines (MDA468, MDA231 and SK-OV3) regardless of EGFR expression levels (Fig. 27). However, the reactivity exhibited by lower affinity EGFR CARs (P3-5.BBZ and C10.BBZ) against EGFR-expressing tumor lines did correlate with the levels of EGFR expression. Furthermore, lower affinity EGFR CARs displayed more potent reactivity to the EGFR overexpressing tumor, MDA468, than the higher affinity EGFR CARs, while provoking a much weaker response to EGFR low expressing cells (Fig. 27). None of the EGFR CAR T cells reacted to the EGFR negative tumor line K562.

To confirm that the level of response was related to scFv affinity and the level of EGFR expression, and to exclude tumor-specific effects, the panel of EGFR CAR T cells was co-cultured with K562 cells expressing varying levels of EGFR after electroporation with *EGFR* mRNA (Fig. 28). The higher affinity EGFR CARs did not discriminate between target cells with different levels of EGFR expression (Fig. 29). For example, T cells expressing CAR 2224 responded equally well to K562 cells electroporated with a 200-fold difference in EGFR mRNA (0.1 µg to 20µg). Howver in agreement with the above ErbB2 CAR results, the lower affinity EGFR CARs (P3-5 and C10) exhibited a high correlation between T cell responses and EGFR expression levels; data are summarized in Fig. 29.

To confirm the increased safety profile of the lower affinity EGFR CARs, we tested the reactivities of EGFR CARs against primary cells derived from different organs. Five primary cell lines and five tumor cell lines were tested for both surface levels of EGFR (Fig. 30) and ability to trigger CAR T cell reactivity (Fig. 31). Three of the primary cell lines examined express detectable levels of EGFR and two did not (pulmonary artery smooth muscle and PBMC). Two of the tumor cell lines (MCF7 and Raji) did not express detectable EGFR on the cell surface. Comparing EGFR CAR T cells to CD19 CAR T cells, T cells with higher EGFR affinity CARs (2224 and P2-4) reacted to all the primary lines tested and all of the tumors except Raji (Fig. 31). However, T cells with the affinity decreased EGFR CAR T cells P3-5 and C10 were not reactive to any of the five primary cells tested (Fig. 31). CD19 specific CAR T cells reacted to the CD19+ line Raji, and to PBMCs, presumably to the B cells in PBMC, but did not respond to any of the tumor lines or other primary cell lines. These data demonstrate that affinity tuning of scFv can increase the therapeutic index for CAR T cells that target either ErbB2 or EGFR.

### DISCUSSION

The efficacy of CAR T cells is dictated in part by the differential expression of the target antigen in tumor versus normal tissue. The results described above demonstrate that CARs with known severe on-target toxicities can be reengineered by affinity tuning, retaining potent *in vivo* efficacy while eliminating or reducing toxicity. In particular, the 4D5 CAR based on trastuzumab had lethal toxicity (Morgan et al., 2010, Mol Ther, 18:843), due to recognition of physiological levels of ErbB2 expressed in cardiopulmonary tissues (Press et al., 1990, Oncogene, 5:953). It was shown that by reducing the K_{D} of scFv employed in CAR T cells by 2- to 3-log, a substantial improvement in the therapeutic index was demonstrated for ErbB2 and EGFR CAR T cells. CAR T cells with lower affinity scFv showed equally robust anti-tumor activity against ErbB2 overexpressing tumors as compared to the high affinity CARs, but displayed little reactivity against physiological levels of ErbB2.

CARs specific for the B cell lineage antigens CD19 and CD20 have been tested by a variety of groups and have displayed potent efficacy in B cell malignancies (Maus et al., 2014, Blood, 123:2625). However in solid tumors, with the exception of tumor-specific isoforms such as EGFRviii (Morgan et al., 2012, Human Gene Therapy)*,* on-target toxicity is anticipated to be a severe limitation for CAR T cells. This limitation is expected to be more serious with CARs than with antibody therapies using intact antibodies or antibody drug conjugates, due to the lower limit of target sensitivity for CAR T cells compared to antibody based therapies that differes by several orders of magnitude. The present studies using target cells electroporated with *ErbB2* or *EGFR* mRNA are consistent with previous studies indicating that CAR T cells can recognize tumor cells with ~100 targets per cell (Stone et al., 2012, Oncoimmunology, 1:863). In contrast, amplification of *ErbB2* occurs in approximately 20% to 25% of primary human breast cancers and typically results in overexpression of ErbB2 protein at > 1 million copies per cell (Robertson et al., 1996, Cancer Res, 56:3823; and Vogel et al., 2002, J Clin Oncol, 20:719). At present, available data indicate that cancer cells do not lose ErbB2 expression when they become refractory to ErbB2 directed therapies (Ritter et al., 2007, Clin Cancer Res, 13:4909).

These findings support previous work from Chmielewski (Chmielewski et al., 2004, J Immunol, 173:7647), suggesting that the high affinity CARs exhibit less discrimination between target cells with high or low target expression levels. However, the present results differ from Chmielewski and coworkers in that none of the higher affinity CARs (with K_{D} ranging from 15pM to 16nM) in their report were reactive to cells with low level expression of *ErbB2* and their lower affinity CAR that only recognized tumors with amplified *ErbB2* showed a substantial reduction in T cell efficacy compared to the higher affinitiy CARs. In contrast, it was found that the ErbB2 CAR using the 4DF5 scFv with K_{D} at 0.3nM was strongly reactive to keratinocytes and even to cell lines transfected with extremely low amounts ErbB2 mRNA that were 100 times below detectable levels, while affinity-tuned CAR T cells retained reactivity to ErbB2 amplified tumors that was at least as potent as the high affinity CAR, both in vitro and in aggressive mouse tumor models. Some variables that may explain these differences include the use of different scFvs (C5.6 versus 4D5) that may recognize different epitopes, distinct CAR signaling domain configuration (zeta alone versus 4-1BB-zeta), and different gene transfer approaches (retroviral transduction versus RNA electroporation or lentiviral transduction) that affect CAR surface expression levels on the T cells. Together, this suggests that each of these factors should be considered when selecting the affinity of a CAR in relevant clinical situations.

The findings described in this example also demonstrated the importance of selecting the right affinity for a CAR targeting a particular tumor-associated antigen (TAA). The in vitro and in vivo results were consistent with each other, demonstrating that CARs having lower affinity was at least as potent as the high affinity CAR (for both lentivirally-transduced and RNA-electroporated CAR T cells), but had minimal impact on cells that had low expression of a TAA (ErbB2), representing normal tissue. In contrast, CARs with high affinity were more reactive to the low-expressing TAA cells representing normal tissue. Thus, CARs with high affinity may not be preferred for cancers where the TAA is also expressed in normal tissue, as these results demonstrate that CARs with high affinity may also target normal tissues and therefore would result in adverse side effects. Taken together, these results indicate that the affinity of the CARs must be considered with respect to the nature of the cancer (e.g., whether the TAA is expressed only in cancer cells or whether the TAA is expressed higher in cancer cells, but is also expressed at a low level in normal tissue) for potency and safety reasons.

The advent of more potent adoptive transfer strategies has prompted a reassessment of targets previously considered as safe using weaker immunotherapeutic strategies (Hinrichs et al., 2013, Nature Biotechnology, 31:999). Strategies to maximize the therapeutic index of CAR T cells include target selection, CAR design, cell manufacturing and gene transfer techniques. In addition to affinity tuning, other strategies being developed to manage on-target toxicity include the use of dual CAR T cell approaches (Kloss et al., 2013, Nat Biotech, 31:999; and Lanitis et al., 2013, Cancer Immunol Res, 1:43), conditional deletion and suicide systems (Di Stasi et al., 2011, NEJM, 365:1673; and Wang et al., 2011, Blood, 118:1255), and repeated infusions of T cells having mRNA CARs that have transient expression and self limiting toxicity (Beatty et al., 2014, Cancer Immunol Res, 2:112).

These results demonstrate that affinity-tuning can increase the therapeutic index for ErbB2 and EGFR. In addition to scFv affinity, other variables that require examination to increase the therapeutic index for other targets include the location of the target epitope, the length of the hinge and the nature of the signaling domain (Hudecek et al., 2013, Clin Cancer Res, 15:5323; and Guedan et al., 2014, Blood, 124:1070).

In summary, ErbB2 and EGFR have previously been considered as undruggable targets for CAR T cells. Given that dysregulation of the expression of ErbB2 and EGFR occurs frequently in multiple human carcinomas including breast, glioblastoma, lung, pancreatic, ovarian, head and neck squamous cell cancer and colon cancer, these findings have considerable clinical importance. This affinity-tuning strategy has the potential not only to improve the safety profile and clinical outcome of CARs directed against validated targets but also to expand the landscape to targets not previously druggable with CAR T cells because of on-target toxicities. More generally, these findings suggest that affinity-tuning suggests that safer and more potent CARs can be designed by employing affinity-decreased scFvs for a variety of common carcinomas.

### Example 5: Effects of mTOR Inhibition on Immunosenescence in the Elderly

One of the pathways most clearly linked to aging is the mTOR pathway. The mTOR inhibitor rapamycin has been shown to extend lifespan in mice and improve a variety of aging-related conditions in old mice (Harrison, DE et al. (2009) Nature 460:392-395; Wilkinson JE et al. (2012) Aging Cell 11:675-682; and Flynn, JM et al. (2013) Aging Cell 12:851-862). Thus, these findings indicate that mTOR inhibitors may have beneficial effects on aging and aging-related conditions in humans.

An age-related phenotype that can be studied in a short clinical trial timeframe is immunosenescence. Immunosenescence is the decline in immune function that occurs in the elderly, leading to an increased susceptibility to infection and a decreased response to vaccination, including influenza vaccination. The decline in immune function with age is due to an accumulation of immune defects, including a decrease in the ability of hematopoietic stem cells (HSCs) to generate naive lymphocytes, and an increase in the numbers of exhausted PD-1 positive lymphocytes that have defective responses to antigenic stimulation (Boraschi, D et al. (2013) Sci. Transl. Med.5:185ps8; Lages, CS et al. (2010) Aging Cell 9:785-798; and Shimatani, K et al., (2009) Proc. Natl. Acad. Sci. USA 106:15807-15812). Studies in elderly mice showed that 6 weeks of treatment with the mTOR inhibitor rapamycin rejuvenated HSC function leading to increased production of naive lymphocytes, improved response to influenza vaccination, and extended lifespan (Chen, C et al. (2009) Sci. Signal. 2:ra75).

To assess the effects of mTOR inhibition on human aging-related phenotypes and whether the mTOR inhibitor RAD001 ameliorates immunosenescence, the response to influenza vaccine in elderly volunteers receiving RAD001 or placebo was evaluated. The findings presented herein suggest that RAD001 enhanced the response to influenza vaccine in elderly volunteers at doses that were well tolerated. RAD001 also reduced the percentage of programmed death (PD)-1 positive CD4 and CD8 T lymphocytes that accumulate with age. These results show that mTOR inhibition has beneficial effects on immunosenescence in elderly volunteers.

As described herein, a 6 week treatment with the mTOR inhibitor RAD001, an analog of rapamycin, improved the response to influenza vaccination in elderly human volunteers.

### Methods

### Study population

Elderly volunteers >= 65 years of age without unstable underlying medical diseases were enrolled at 9 sites in New Zealand and Australia. Exclusion criteria at screening included hemoglobin < 9.0 g/dL, white blood cell count <3,500/mm³, neutrophil count <2,000/mm³, or platelet count <125,000/mm³, uncontrolled diabetes, unstable ischemic heart disease, clinically significant underlying pulmonary disease, history of an immunodeficiency or receiving immunosuppressive therapy, history of coagulopathy or medical condition requiring long-term anticoagulation, estimated glomerular filtration rate < 30 ml/min, presence of severe uncontrolled hypercholesterolemia (>350 mg/dL, 9.1 mmol/L) or hypertriglyceridemia (>500 mg/dL, 5.6 mmol/L).

Baseline demographics between the treatment arms were similar (Table 4). Of the 218 subjects enrolled, 211 completed the study. Seven subjects withdrew from the study. Five subjects withdrew due to adverse events (AEs), one subject withdrew consent, and one subject left the study as a result of a protocol violation.

**Table 4: Demographic and Baseline characteristics of the Study Patients**

| **Population** | | **RAD001 0.5 mg daily N=53** | **RAD001 5 mg weekly N=53** | **RAD001 20 mg weekly N=53** | **Placebo pooled N=59** | **Total N=218** |
|---|---|---|---|---|---|---|
| **Age (Years)** | **Mean (SD)** | 70.8 (5.0) | 72.0 (5.3) | 71.4 (5.2) | 71.1 (5.1) | 71.3 (5.2) |
| **Gender** | **Male- n (%)** | 34 (64%) | 27 (51%) | 32 (60%) | 31 (53%) | 124 (57%) |
| **BMI* (kg/m2)** | **Mean (SD)** | 27.4 (4.2) | 28.8 (5.0) | 28.0 (4.1) | 28.0 (4.2) | 28.0 (4.4) |
| **Race - n (%)** | **Caucasian** | 48 (91%) | 50 (94%) | 46 (87%) | **54** (92%) | 198 (91%) |
| | **Other** | 5(9%) | 3 (6%) | 7 (13%) | 5 (8%) | 20 (9%) |

| | | | | | | |
|---|---|---|---|---|---|---|
| ^{∗}The body-mass index is weight in kilograms divided by the square of the height in meters | | | | | | |

### Study Design and Conduct

From December 2011 to April 2012, 218 elderly volunteers were enrolled in a randomized, observer-blind, placebo-controlled trial. The subjects were randomized to treatment arms using a validated automated randomization system with a ratio of RAD001 to placebo of 5:2 in each treatment arm. The treatment arms were:
RAD001 0.5 mg daily or placebo
RAD001 5 mg weekly or placebo
RAD001 20 mg weekly or placebo

The trial was observer-blind because the placebo in the RAD001 0.5 mg daily and 20 mg weekly cohorts differed slightly from the RAD001 tablets in those cohorts. The study personnel evaluating the subjects did not see the study medication and therefore were fully blinded. The treatment duration for all cohorts was 6 weeks during which time subjects underwent safety evaluations in the clinic every 2 weeks. After subjects had been dosed for 4 weeks, RAD001 steady state levels were measured pre-dose and at one hour post dose. After completing the 6 week course of study drug, subjects were given a 2 week drug free break to reverse any possible RAD001-induced immunosuppression, and then were given a 2012 seasonal influenza vaccination (Agrippal^{®}, Novartis Vaccines and Diagnostics, Siena, Italy) containing the strains H1N1 A/California/ 07/2009, H3N2 A/Victoria/210/2009, B/Brisbane/60/ 2008. Four weeks after influenza vaccination, subjects had serum collected for influenza titer measurements. Antibody titers to the 3 influenza vaccine strains as well as to 2 heterologous strains (A/H1N1 strain A/New Jersy/8/76 and A/H3N2 strain A/Victoria/361/11) were measured by standard hemagglutination inhibition assay (Kendal, AP et al. (1982) Concepts and procedures for laboratory-based influenza surveillance. Atlanta: Centers for Disease Control and Prevention B17-B35). Levels of IgG and IgM specific for the A/H1N1/California/07/2009 were measured in serum samples taken before and 4 weeks after influenza vaccination as described previously (Spensieri, F. et al. (2013) Proc. Natl. Acad. Sci. USA 110:14330-14335). Results were expressed as fluorescence intensity.

All subjects provided written informed consent. The study was conducted in accordance with the principals of Good Clinical Practice and was approved by the appropriate ethics committees and regulatory agencies.

### Safety

Adverse event assessment and blood collection for hematologic and biochemical safety assessments were performed during study visits. Adverse event information was also collected in diaries that subjects filled out at home during the 6 weeks they were on study drug. Data on all adverse events were collected from the time of informed consent until 30 days after the last study visit. Events were classified by the investigators as mild, moderate or severe.

### Statistical Analysis

The primary analysis of geometric mean titer ratios was done using a normal Bayesian regression model with non-informative priors. This model was fitted to each antibody titer on the log scale. The primary outcome in each model was the Day 84 measurement. The Day 63 measurement was included in the outcome vector. The model fitted using SAS 9.2 proc mixed with the prior statement. The covariance structure of the matrix was considered as unstructured (option type=UN). A flat prior was used. For the secondary analysis of seroconversion rates, logistic regression was used.

The intention to treat population was defined as all subjects who received at least one full dose of study drug and who had no major protocol deviations impacting efficacy data. 199 out of the total of 218 subjects enrolled in the study were in the intention to treat population.

### Immunophenotyping

Peripheral blood mononuclear cells were isolated from whole blood collected at 3 time points: baseline; after 6 weeks of study drug treatment; and at the end of study when subjects had been off study drug for 6 weeks and 4 weeks after influenza vaccination. Seventy-six PBMC subsets were analyzed by flow cytometry using 8-color immunophenotyping panels at the Human Immune Monitoring Center at Stanford University, CA, USA as described previously (Maecker, HT et al. (2012) Nat Rev Immunol. 12:191-200). Seventy-six PBMC subsets were analyzed by flow cytometry using 8-color lyophilized immunophenotyping panels (BD Lyoplate, BD Biosciences, San Diego, CA). PBMC samples with viability >80% and yield of 2×10⁶ cells or greater were included in the analysis.

Relative changes of the immunophenotypes from baseline to Week 6 of study drug treatment and from baseline to the end of study (Week 12) were calculated for each of the RAD001 dosing cohorts. Student T test was conducted to examine if the relative change of the immunophenotypes from baseline to the two blood sampling time points was significantly different from zero, respectively, within each dosing group after adjusting for placebo effect. Missing data imputation in treatment effect analysis was not conducted. Therefore if a patient has a missing phenotype data at baseline, this patient was not be included in the analysis for this phenotype. If a patient had a missing phenotype data at 6 or 12 weeks, then this patient did not contribute to the analysis of this phenotype for the affected timepoint.

608 tests in 76 phenotypes under 3 dosing groups were conducted to compare the treatment effect against the placebo effect. Stratified false discovery rate (FDR) control methodology was implemented to control the occurrence of false positives associated with multiple testing yet provide considerably better power. The cell type group was taken as the stratification factor and conducted FDR (q-value) calculation within each stratum respectively. All null-hypotheses were rejected at 0.05 significance level with corresponding q-value ≤ 0.1. The multiple testing adjustment strategy with rejecting at 0.05 significance level and corresponding q<0.1 ensured that less than 10% of the findings are false.

In a second analysis, the immunophenotype changes between pooled treatment and placebo groups, where all three RAD001 dosing groups were combined. To determine which immunophenotype changes differed between the treated and placebo groups, within-patient cell count ratios for each measured phenotype were calculated between baseline and Week 6 of study drug treatment and between baseline and the end of study (Week 12). The ratios were log transformed, and analyzed by analysis of covariance at each time point in order to detect a difference between the pooled treatment and placebo groups. 152 tests in 76 phenotypes were performed to compare the pooled treatment effect against the placebo effect. Stratified false discovery rate (FDR) control methodology was implemented to control the occurrence of false positives associated with multiple testing yet provide considerably better power (Benjamini, Y. et al. (1995) J. Roy. Statist. 57:289-300; and Sun, L. et al. (2006) Genet. Epidemiol. 30:519-530). The cell type group was taken as the stratification factor and FDR (q-value) calculation was conducted within each stratum respectively. All null-hypotheses at 0.05 significance level and q-value less than 20% were rejected. This can be interpreted as rejecting only those hypotheses with P values less than 0.05 and less than 20% probability that the each observed significant result is due to multiple testing.

### Results

In general, RAD001 was well tolerated, particularly the 0.5 mg daily and 5 mg weekly dosing regimens. No deaths occurred during the study. Three subjects experienced four serious adverse events (SAEs) that were assessed as unrelated to RAD001. The 4 SAEs were retinal hemorrhage of the left eye with subsequent blindness in a subject with normal platelet counts who had completed a 6 week course of 5 mg weekly RAD001 6 weeks previously; severe back pain in a subject treated with placebo and severe gastroenteritis in a subject treated with placebo. A list of treatment-related adverse events (AEs) with an incidence >2% in any treatment group is provided in Table 5. The most common RAD001-related AE was mouth ulcer that, in the majority of cases, was of mild severity. Overall, subjects who received RAD001 had a similar incidence of severe AEs as those treated with placebo. Only one severe AE was assessed as related to RAD001 mouth ulcers in a subject treated with 20 mg weekly RAD001.

**Table 5: Incidence of treatment-related AEs >2% in any treatment group by preferred term**

| | **RAD001 0.5 mg daily N=53 n (%)** | **RAD001 5 mg weekly N=53 n (%)** | **RAD001 20 mg weekly N=53 n (%)** | **Placebo, pooled N=59 n (%)** | **Total N=218 n (%)** |
|---|---|---|---|---|---|
| Total AE(s) | 35 | 46 | 109 | 21 | 211 |
| Patients with AE(s) | 22 (41.5%) | 20 (37.7%) | 27 (50.9%) | 12 (20.3%) | 81(37.2%) |
| Mouth ulceration | 6 (11.3%) | 2 (3.8%) | 9 (17.0%) | 3 (5.1%) | 20(9.2%) |
| Headache | 0 | 2 (3.8%) | 9 (17.0%) | 1 (1.7%) | 12 (5.5%) |
| Blood cholesterol increased | 2 (3.8%) | 2 (3.8%) | 2 (3.8%) | 0 | 6 (2.8%) |
| Diarrhea | 1 (1.9%) | 4 (7.5%) | 1 (1.9%) | 0 | 6 (2.8%) |
| Dyspepsia | 0 | 3 (5.7%) | 2 (3.8%) | 1 (1.7%) | 6 (2.8%) |
| Fatigue | 0 | 2 (3.8%) | 4 (7.5%) | 0 | 6 (2.8%) |
| Low density lipoprotein increased | 2 (3.8%) | 1 (1.9%) | 2 (3.8%) | 0 | 5 (2.3%) |
| Tongue ulceration | 3 (5.7%) | 1 (1.9%) | 0 | 1 (1.7%) | 5 (2.3%) |
| Insomnia | 1 (1.9%) | 2 (3.8%) | 1 (1.9%) | 0 | 4 (1.8%) |
| Dry mouth | 0 | 0 | 2 (3.8%) | 1 (1.7%) | 3 (1.4%) |
| Neutropenia | 0 | 0 | 3 (5.7%) | 0 | 3 (1.4%) |
| Oral pain | 0 | 2 (3.8%) | 1 (1.9%) | 0 | 3 (1.4%) |
| Pruritus | 0 | 2 (3.8%) | 1 (1.9%) | 0 | 3 (1.4%) |
| Conjunctivitis | 0 | 2 (3.8%) | 0 | 0 | 2 (0.9%) |
| Erythema | 0 | 2 (3.8%) | 0 | 0 | 2 (0.9%) |
| Limb discomfort | 0 | 2 (3.8%) | 0 | 0 | 2 (0.9%) |
| Mucosal inflammation | 0 | 0 | 2 (3.8%) | 0 | 2 (0.9%) |
| Paresthesia oral | 2 (3.8%) | 0 | 0 | 0 | 2 (0.9%) |
| Stomatitis | 0 | 0 | 2 (3.8%) | 0 | 2 (0.9%) |
| Thrombocytopenia | 0 | 0 | 2 (3.8%) | 0 | 2 (0.9%) |
| Urinary tract infection | 0 | 0 | 2 (3.8%) | 0 | 2 (0.9%) |

The ability of RAD001 to improve immune function in elderly volunteers was evaluated by measuring the serologic response to the 2012 seasonal influenza vaccine. The hemagglutination inhibition (HI) geometric mean titers (GMT) to each of the 3 influenza vaccine strains at baseline and 4 weeks after influenza vaccination are provided in Table 6. The primary analysis variable was the HI GMT ratio (4 weeks post vaccination/baseline). The study was powered to be able to demonstrate that in at least 2 out of 3 influenza vaccine strains there was 1) a ≥ 1.2-fold GMT increase relative to placebo; and 2) a posterior probability no lower than 80% that the placebo-corrected GMT ratio exceeded 1. This endpoint was chosen because a 1.2-fold increase in the influenza GMT ratio induced by the MF-59 vaccine adjuvant was associated with a decrease in influenza illness (lob, A et al. (2005) Epidemiol Infect 133:687-693).

**Table 6. HI GMTs for each influenza vaccine strain at baseline and at 4 weeks after influenza vaccination**

| Influenza Vaccine Strain | | **Time** | **RAD001 0.5mg daily N=50** | **RAD001 5mg weekly N=49** | **RAD001 20mg weekly N=49** | **Placebo N=55** |
|---|---|---|---|---|---|---|
| **A/H1N1** | **GMT (CV%)** | **Baseline** | 102.8 (186.9) | 84.2 (236.4) | 90.1 (188.4) | 103.2 (219.7) |
| | | **Week 4** | 190.2 (236.9) | 198.73(195.6) | 129.7 (175.9) | 169.4 (259.8) |
| | **GMT ratio (CV%)** | | 2.6 (302.5) | 2.5 (214.3) | 1.8 (201.5) | 2.0 (132.7) |
| **A/H3N2** | **GMT (CV%)** | **Baseline** | 106.8 (168.2) | 126.04 (162.6) | 137.1 (211.5) | 131.7 (162.3) |
| | | **Week 4** | 194.4 (129.1) | 223.0 (118.8) | 223.0 (163.6) | 184.3 (153.2) |
| | **GMT ratio (CV%)** | | 2.1 (152.6) | 2.0 (189.2) | 2.1 (277.3) | 1.6 (153.6) |
| **B** | **GMT (CV%)** | **Baseline** | 44.2 (96.6) | 64.8 (87.3) | 58.0 (156.0) | 57.0 (112.6) |
| | | **Week 4** | 98.4 (94.8) | 117.3 (99.9) | 99.2 (124.1) | 114.6 (136.7) |
| | **GMT ratio (CV%)** | | 2.5 (111.2) | 2.2 (112.8) | 2.1 (126.5) | 2.2 (109.2) |

| | | | | | | |
|---|---|---|---|---|---|---|
| Baseline indicates 2 weeks prior to influenza vaccination Week 4 indicates 4 weeks after influenza vaccination N is number of subjects per cohort GMT is geometric mean titer GMT ratio is the GMT at week 4 post vaccination/GMT at baseline CV% indicates coefficient of variation | | | | | | |

In the intent-to-treat (ITT) population, the low, immune enhancing, dose RAD001 (0.5 mg daily or 5 mg weekly) cohorts but not higher dose (20 mg weekly) cohort met the primary endpoint of the study (Figure 41A). This demonstrates that there is a distinct immunomodulatory mechanism of RAD001 at the lower doses, and that at the higher dose the known immunosuppressive effects of mTOR inhibition may come into play. Furthermore, the results suggest a trend toward improved immune function in the elderly after low, immune enhancing, dose RAD001 treatment.

In a subgroup analysis, the subset of subjects with low baseline influenza titers (≤ 1:40) experienced a greater RAD001-associated increase in titers than did the ITT population (Figure 41B). These data show that RAD001 is particularly effective at enhancing the influenza vaccine response of subjects who did not have protective (>1:40) titers at baseline, and therefore were at highest risk of influenza illness.

Scatter plots of RAD001 concentration versus increase in titer to each influenza vaccine strain show an inverse exposure/response relationship (Figure 42). Modeling and simulation based on mTOR mediated phosphorylation of S6 kinase (S6K) predicts that the 20 mg weekly dosing regimen inhibits mTOR-mediated S6K activity almost completely, the 5 mg weekly dosing regimen inhibits S6K activity by over 50%, and the 0.5 mg daily dosing regiment inhibits S6K phosphorylation by approximately 38% during the dosing interval (Tanaka, C et al. (2008) J. Clin. Oncol 26:1596-1602). Thus, partial mTOR inhibition, e.g., mTOR-mediated S6K phosphorylation, with low, immune enhancing, dose RAD001 may be as, if not more effective, than near complete mTOR inhibition with high dose RAD001 at enhancing the immune response of the elderly.

Rates of seroconversion 4 weeks after influenza vaccination were also evaluated. Seroconversion was defined as the change from a negative pre-vaccination titer (i.e., HI titer <1:10) to post-vaccination HI titer ≥ 1:40 or at least 4-fold increase from a non-negative (≥ 1:10) pre-vaccination HI titer. In the intention-to-treat population, seroconversion rates for the H3N2 and B strains were increased in the RAD001 as compared to the placebo cohorts although the increases did not meet statistical significance (Table 7). In the subpopulation of subjects with baseline influenza titers <= 1:40, RAD001 treatment also increased the rates of seroconversion to the H3N2 and B strains, and these results reached statistical significance for the B strain in the 0.5 mg daily dosing cohort. These data further show that RAD001 enhanced the serologic response to influenza vaccination in the elderly.

**Table 7: Percent of subjects with seroconversion to influenza 4 weeks after vaccination**

| | **Placebo N=54** | **0.5 mg N=48** | **5 mg N=49** | **20 mg N=48** |
|---|---|---|---|---|
| Intention to Treat Population | | | | |
| H1N1 | 24 | 27 | 27 | 17 |
| H3N2 | 17 | 27 | 24 | 25 |
| B | 17 | 27 | 22 | 19 |

| Subjects with Baseline Titers <=40 | | | | |
|---|---|---|---|---|
| H1N1 | 40 | 42 | 45 | 36 |
| H3N2 | 42 | 64 | 53 | 71 |
| B | 16 | 40^{∗} | 33 | 28 |

| | | | | |
|---|---|---|---|---|
| ^{∗} Odds ratio for seroconversion between RAD001 and Placebo significantly different than 1 (two-sided p-value < 0.05 obtained by logistic regression with treatment as fixed effect) | | | | |

Current seasonal influenza vaccines often provide inadequate protection against continuously emerging strains of influenza that present as variants of previously circulating viruses. However, mice vaccinated against influenza in the presence of the mTOR inhibitor rapamycin, as compared to placebo, developed a broader serologic response to influenza. The broader serologic response included antibodies to conserved epitopes expressed by multiple subtypes of influenza that provided protection against infection with heterologous strains of influenza not contained in the vaccine (Keating, R et al. (2013) Nat Immunology 14:2166-2178). To determine if RAD001 broadened the serologic response to influenza in the elderly volunteers, HI titers to 2 heterologous strains of influenza not contained in the influenza vaccine (A/H1N1 strain A/New Jersey/8/76 and A/H3N2 strain A/Victoria/361/11) were measured. The increase in the HI GMT ratios for the heterologous strains was higher in the RAD001 as compared to placebo cohorts (Figure 43). In addition, seroconversion rates for the heterologous strains were higher in the RAD001 as compared to placebo cohorts. The increase in seroconversion rates in the 5 and 20 mg weekly RAD001 dosing cohorts was statistically significant for the H3N2 heterologous strain (Table 8). The H3N2 seroconversion rate for the pooled RAD001 cohorts was 39% versus 20% for the placebo cohort (p=0.007). The results presented herein suggest that mTOR inhibition broadens the serologic response of elderly volunteers to influenza vaccination, and increases antibody titers to heterologous strains of influenza not contained in the seasonal influenza vaccine.

Broadened serologic response to heterologous strains of influenza in mice treated with rapamycin has been associated with an inhibition of class switching in B cells and an increase in anti-influenza IgM levels (Keating, R. et al. (2013) Nat Immunol 14:2166-2178). However, inhibition of class switching may not be involved in the broadened serologic response in humans treated with RAD001 because the post-vaccination anti-influenza IgM and IgG levels did not differ between RAD001 and placebo treated cohorts (Figure 44).

**Table 8: Percentage of subjects who seroconvert to heterologous strains of influenza 4 weeks after seasonal influenza vaccination**

| | **Placebo, pooled** | **RAD001 0.5mg daily** | **RAD001 5 mg weekly** | **RAD001 20 mg weekly** |
|---|---|---|---|---|
| A/H1N1 strain: A/NewJersey/8/76 | 7% | 17% | 16% | 8% |
| A/H3N2 strain: A/Victona/361/11 | 20% | 38% | 39%* | 40% * |

| | | | | |
|---|---|---|---|---|
| ^{∗} Odds ratio for seroconversion between RAD001 and Placebo significantly different than 1 (two-sided p-value < 0.05 obtained by logistic regression with treatment as fixed effect) | | | | |

To address the mechanism by which RAD001 enhanced immune function in elderly volunteers, immunophenotyping was performed on PBMC samples obtained from subjects at baseline, after 6 weeks of study drug treatment and 4 weeks after influenza vaccination (6 weeks after study drug discontinuation). Although the percentage of most PBMC subsets did not differ between the RAD001 and placebo cohorts, the percentage of PD-1 positive CD4 and CD8 cells was lower in the RAD001 as compared to placebo cohorts (Figure 45). PD-1 positive CD4 and CD8 cells accumulate with age and have defective responses to antigen stimulation because PD-1 inhibits T cell receptor-induced T cell proliferation, cytokine production and cytolytic function (Lages, CS et al. (2010) Aging Cell 9:785-798). There was an increase in percentage of PD-1 positive T cells over time in the placebo cohort. At week 12 (4 weeks post-vaccination) this increase may have been due to influenza vaccination since influenza virus has been shown to increase PD-1 positive T cells (Erikson, JJ et al. (2012) JCI 122:2967-2982). However the percentage of CD4 PD-1 positive T cells decreased from baseline at week 6 and 12 in all RAD001 cohorts (Figure 45A). The percentage of CD8 PD-1 positive cells also decreased from baseline at both week 6 and 12 in the two lower dose RAD001 cohorts (Figure 45B). The percentage of PD-1 negative CD4 T cells was evaluated and increased in the RAD001 cohorts as compared to the placebo cohorts (Figure 45C).

Under more stringent statistical analysis, where the results from the RAD001 cohorts were pooled and adjusted for differences in baseline PD-1 expression, there was a statistically significant decrease of 30.2% in PD-1 positive CD4 T cells at week 6 in the pooled RAD cohort (n=84) compared to placebo cohort (n=25) with p=0.03 (q=0.13) (Figure 46A). The decrease in PD-1 positive CD4 T cells at week 12 in the pooled RAD as compared to the placebo cohort is 32.7% with p=0.05 (q=0.19). Figure 46B shows a statistically significant decrease of 37.4% in PD-1 positive CD8 T cells at week 6 in the pooled RAD001 cohort (n=84) compared to placebo cohort (n=25) with p=0.008 (q=0.07). The decrease in PD-1 positive CD8 T cells at week 12 in the pooled RAD001 as compared to the placebo cohort is 41.4% with p=0.066 (q=0.21). Thus, the results from Figures 45 and 46 together suggest that the RAD001-associated decrease in the percentage of PD-1 positive CD4 and CD8 T cells may contribute to enhanced immune function.

### Conclusion

In conclusion, the data presented herein show that the mTOR inhibitor RAD001 ameliorates the age-related decline in immunological function of the human elderly as assessed by response to influenza vaccination, and that this amelioration is obtained with an acceptable risk/benefit balance. In a study of elderly mice, 6 weeks treatment with the mTOR inhibitor rapamycin not only enhanced the response to influenza vaccination but also extended lifespan, suggesting that amelioration of immunosenescence may be a marker of a more broad effect on aging-related phenotypes.

Since RAD001 dosing was discontinued 2 weeks prior to vaccination, the immune enhancing effects of RAD001 may be mediated by changes in a relevant cell population that persists after discontinuation of drug treatment. The results presented herein show that RAD001 decreased the percentage of exhausted PD-1 positive CD4 and CD8 T cells as compared to placebo. PD-1 expression is induced by TCR signaling and remains high in the setting of persistent antigen stimulation including chronic viral infection. While not wishing to be bound by theory, is possible that RAD001 reduced chronic immune activation in elderly volunteers and thereby led to a decrease in PD-1 expression. RAD001 may also directly inhibit PD-1 expression as has been reported for the immunophilin cyclosporine A (Oestreich, KJ et al. (2008) J Immunol. 181:4832-4839). A RAD001-induced reduction in the percentage of PD-1 positive T cells is likely to improve the quality of T cell responses. This is consistent with previous studies showing that mTOR inhibition improved the quality of memory CD8 T cell response to vaccination in mice and primates (Araki, K et al. (2009) Nature 460:108-112). In aged mice, mTOR inhibition has also been shown to increase the number of hematopoietic stem cells, leading to increased production of naive lymphocytes (Chen, C et al. (2009) Sci Signal 2:ra75). Although significant differences in the percentages of naive lymphocytes in the RAD001 versus placebo cohorts were not detected in this example, this possible mechanism may be further investigated.

The mechanism by which RAD001 broadened the serologic response to heterologous strains of influenza may be further investigated. Rapamycin has also been shown to inhibit class switching in B cells after influenza vaccination. As a result, a unique repertoire of anti-influenza antibodies was generated that promoted cross-strain protection against lethal infection with influenza virus subtypes not contained in the influenza vaccine (Keating, R et al. (2013) Nat Immunol. 14:2166-2178). The results described herein did not show that RAD001 altered B cell class switching in the elderly subjects who had discontinued RAD001 2 weeks prior to influenza vaccination. Although the underlying mechanism requires further elucidation, the increased serologic response to heterologous influenza strains described herein may confer enhanced protection to influenza illness in years when there is a poor match between the seasonal vaccine and circulating strains of influenza in the community.

The effect of RAD001 on influenza antibody titers was comparable to the effect of the MF59 vaccine adjuvant that is approved to enhance the response of the elderly to influenza vaccination (Podda, A (2001) Vaccine 19:2673-2680). Therefore, RAD001-driven enhancement of the antibody response to influenza vaccination may translate into clinical benefit as demonstrated with MF59-adjuvanted influenza vaccine in the elderly (lob, A et al. (2005) Epidemiol Infect. 133:687-693). However, RAD001 is also used to suppress the immune response of organ transplant patients. These seemingly paradoxical findings raise the possibility that the immunomodulatory effects of mTOR inhibitors may be dose and/or antigen-dependent (Ferrer, IR et al. (2010) J Immunol. 185:2004-2008). A trend toward an inverse RAD001 exposure/vaccination response relationship was seen herein. It is possible that complete mTOR inhibition suppresses immune function through the normal cyclophilin-rapamycin mechanism, whereas partial mTOR inhibition, at least in the elderly, enhances immune function due to a distinct aging-related phenotype inhibition. Of interest, mTOR activity is increased in a variety of tissues including hematopoietic stem cells in aging animal models (Chen C. et al. (2009) Sci Signal 2:ra75 and Barns, M. et al. (2014) Int J Biochem Cell Biol. 53:174-185). Thus, turning down mTOR activity to levels seen in young tissue, as opposed to more complete suppression of mTOR activity, may be of clinical benefit in aging indications.

The safety profile of mTOR inhibitors such as RAD001 in the treatment of aging-related indications has been of concern. The toxicity of RAD001 at doses used in oncology or organ transplant indications includes rates of stomatitis, diarrhea, nausea, cytopenias, hyperlipidemia, and hyperglycemia that would be unacceptable for many aging-related indications. However, these AEs are related to the trough levels of RAD001 in blood. Therefore the RAD001 dosing regimens used in this study were chosen to minimize trough levels. The average RAD001 trough levels of the 0.5 mg daily, 5 mg weekly and 20 mg weekly dosing cohorts were 0.9 ng/ml, below 0.3 ng/ml (the lower limit of quantification), and 0.7 ng/ml, respectively. These trough levels are significantly lower than the trough levels associated with dosing regimens used in organ transplant and cancer patients. In addition, the limited 6 week course of treatment decreased the risk of adverse events. These findings suggest that the dosing regimens used in this study may have an acceptable risk/benefit for some conditions of the elderly. Nonetheless, significant numbers of subjects in the experiments described hereindeveloped mouth ulcers even when dosed as low as 0.5 mg daily. Therefore the safety profile of low, immune enhancing, dose RAD001 warrants further study. Development of mTOR inhibitors with cleaner safety profiles than currently available rapalogs may provide better therapeutic options in the future for aging-associated conditions.

### Example 6: Enhancement of Immune Response to Vaccine in Elderly Subjects

Immune function declines in the elderly, leading to an increase incidence of infection and a decreased response to vaccination. As a first step in determining if mTOR inhibition has anti-aging effects in humans, a randomized placebo-controlled trial was conducted to determine if the mTOR inhibitor RAD001 reverses the aging-related decline in immune function as assessed by response to vaccination in elderly volunteers. In all cases, appropriate patent consents were obtained and the study was approved by national health authorities.

The following 3 dosing regimens of RAD001 were used in the study:
20 mg weekly (trough level: 0.7 ng/ml)
5 mg weekly (trough level was below detection limits)
0.5 mg daily (trough level: 0.9 ng/ml)

These dosing regimens were chosen because they have lower trough levels than the doses of RAD001 approved for transplant and oncology indications. Trough level is the lowest level of a drug in the body. The trough level of RAD001 associated with the 10 mg daily oncology dosing regimen is approximately 20 ng/ml. The trough level associated with the 0.75-1.5 mg bid transplant dosing regimen is approximately 3 ng/ml. In contrast, the trough level associated with the dosing regimens used in our immunization study were 3-20 fold lower.

Since RAD001-related AEs are associated with trough levels, the 3 dosing regimens were predicted to have adequate safety for normal volunteers. In addition, the 3 doses were predicted to give a range of mTOR inhibition. P70 S6 Kinase (P70 S6K) is a downstream target that is phosphorylated by mTOR. Levels of P70 S6K phosphorylation serve as a measure of mTOR activity. Based on modeling and simulation of P70 S6K phosphorylation data obtained in preclinical and clinical studies of RAD001, 20 mg weekly was predicted to almost fully inhibit mTOR activity for a full week, whereas 5 mg weekly and 0.5 mg daily were predicted to partially inhibit mTOR activity.

Elderly volunteers >= 65 years of age were randomized to one of the 3 RAD001 treatment groups (50 subjects per arm) or placebo (20 subjects per arm). Subjects were treated with study drug for 6 weeks, given a 2 week break, and then received influenza (Aggrippal, Novartis) and pneumoccal (Pneumovax 23, Merck), vaccinations. Response to influenza vaccination was assessed by measuring the geometric mean titers (GMTs) by hemagglutination inhibition assay to the 3 influenza strains (H1N1, H3N2 and B influenza subtypes) in the influenza vaccine 4 weeks after vaccination. The primary endpoints of the study were (1) safety and tolerability and (2) a 1.2 fold increase in influenza titers as compared to placebo in 2/3 of the influenza vaccine strains 4 weeks after vaccination. This endpoint was chosen because a 1.2 fold increase in influenza titers is associated with a decrease in influenza illness post vaccination, and therefore is clinically relevant. The 5 mg weekly and 0.5 mg daily doses were well tolerated and unlike the 20 mg weekly dose, met the GMT primary endpoint (Figure 41A). Not only did RAD001 improve the response to influenza vaccination, it also improved the response to pneumococcal vaccination as compared to placebo in elderly volunteers. The pneumococcal vaccine contains antigens from 23 pneumococcal serotypes. Antibody titers to 7 of the serotypes were measured in our subjects. Antibody titers to 6/7 serotypes were increased in all 3 RAD cohorts compared to placebo.

The combined influenza and pneumococcal titer data suggest that partial (less than 80-100%) mTOR inhibition is more effective at reversing the aging-related decline in immune function than more complete mTOR inhibition.

### Example 7: Low dose mTOR inhibition increases energy and exercise

In preclinical models, mTOR inhibition with the rapalog rapamycin increases spontaneous physical activity in old mice (Wilkinson et al. Rapamycin slows aging in mice. (2012) Aging Cell; 11:675-82). Of interest, subjects in the 0.5 mg daily dosing cohort described in Example 6 also reported increased energy and exercise ability as compared to placebo in questionnaires administered one year after dosing (Figure 47). These data suggest that partial mTOR inhibition with rapalogs may have beneficial effects on aging-related morbidity beyond just immune function.

### Example 8: P70 S6 kinase inhibition with RAD001

Modeling and simulation were performed to predict daily and weekly dose ranges of RAD001 that are predicted to partially inhibit mTOR activity. As noted above, P70 S6K is phosphorylated by mTOR and is the downstream target of mTOR that is most closely linked to aging because knockout of P70 S6K increases lifespan. Therefore modeling was done of doses of RAD001 that partially inhibit P70 S6K activity. Weekly dosing in the range of >= 0.1 mg and < 20 mg are predicted to achieve partial inhibition of P70 S6K activity (Figure 48).

For daily dosing, concentrations of RAD001 from 30 pM to 4 nM partially inhibited P70 S6K activity in cell lines (Table 9). These serum concentrations are predicted to be achieved with doses of RAD001 >= 0.005 mg to < 1.5 mg daily.

**Table 9: Percent inhibition of P70 S6K activity in HeLa cells in vitro**

| | | | | | | | |
|---|---|---|---|---|---|---|---|
| RAD001 concentration | 0 | 6 pM | 32 pM | 160 pM | 800 pM | 4 nM | 20 nM |
| % P70 S6K inhibition | 0 | 0 | 18 | 16 | 62 | 90 | 95 |

### Conclusion

Methods of treating aging-related morbidity, or generally enhancing an immune response, with doses of mTOR inhibitors that only partially inhibit P70 S6K. The efficacy of partial mTOR inhibition with low doses of RAD001 in aging indications is an unexpected finding. RAD001 dose ranges between >= 0.1 mg to < 20 mg weekly and >= 0.005 mg to < 1.5 mg daily will achieve partial mTOR inhibition and therefore are expected to have efficacy in aging-related morbidity or in the enhancement of the immune response.

### Example 9: Identification of novel target antigens for CART therapy

The strategy for CART therapy depends upon preferential expression of a target cell surface antigen on tumor cells or when ablation of normal cells expressing the target is clinically tolerable. In B-cell ALL, targeting to CD19 by CART therapy has proven to be effective and feasible clinically. However, some patients with B-cell ALL have no or low expression of CD19, or relapse after CAR19 therapy with CD19-negative disease. Furthermore, T cell ALL and AML are not susceptible to targeting with CART19 cells. Thus, the lack target surface antigens in different cancers have impeded development of CAR-based approaches. In this example, a strategy for target antigen discovery in acute leukemias (AL), e.g., ALL and AML, is described.

QuantiGene assays (Affymetrix) were utilized to measure the RNA level of 53 candidate genes for target antigens in acute leukemias. QuantiGene assays utilize a branched DNA (bDNA) assay, which is a sandwich nucleic acid hybridization method that uses bDNA molecules to amplify signal from captured target RNA. RNA is measured directly from the sample source, without purification or enzymatic manipulation. QuantiGene is thus a robust, reproducible assay with a wide dynamic range.

53 candidate genes were selected to be tested based on one of four criteria:
(i) known expression in AL (as positive controls), e.g., CD19 for B-cell ALL or CD-34 for AML;
(ii) expression during hematopoiesis, on the assumption that AL is a malignancy of the hematopoetic cells (e.g., EMR2);
(iii) expression is likely to be on the surface of target cells;
(iv) where ablation of normal tissues or cells carrying these antigens is expected to be clinically tolerable.

RNA was made from a panel of 33 patient AML samples, 7 ALL samples, 3 healthy bone marrow controls (NBM), as well as one each ALL cell line, AML cell line, and a non-hematopoietic malignancy (A357 melanoma) to serve as a negative control. All samples were run in duplicate in the QuantiGene Assay. Analysis was performed as follows. The average background read and standard deviaition for each gene was calculated. Reads that do not exceed the average + 3xSD for that gene and the duplicate datapoints that fail quality control (e.g., where the CV for the duplicates is > 10%) were excluded. Housekeeping genes (e.g., PP1B or GUSB) were normalized. The median fluorescence intensity (MFI) for each gene relative to the housekeeping gene is expressed. Where more than datapoints were available for normal bone marrow controls, statistical calculation for significance was performed using ANOVA followed by Dunnett's post-test. The candidate genes were ordered by preference for downstream investigation as follows:
1. AML and ALL > NBM and A357
2. AML or ALL > NBM and A357
3. AML and ALL > A357 but not NBM
4. AML or ALL > A357 but not NBM

The normalized MFI values calculated for each candidate gene are shown in Figures 49-57. Figure 58 is a cumulative representation of the average normalized MFI values relative to PP1B housekeeping gene for AML and ALL, normal bone marrow, or the A357 cell line. Based on the analysis described above, the following novel target antigens for acute leukemias were identified: C79a, CD72, LAIR1, FCAR, CD79b, LILRA2, CD300LF, CLEC12A, BST2, EMR2, CLECL1 (CLL-1), LY75, FLT3, CD22, KIT, GPC3, FCRL5, and IGLL1.

Downstream investigation of the target genes include flow cytometry of patient specimens to confirm protein level expression on the cell membrane, immunohistochemistry on healthy tissue microarrays to exclude the presence of the target antigen on important normal tissues.

### Example 10: Optimizing CAR therapy with administration of exogenous cytokines

Cytokines have important functions related to T cell expansion, differentiation, survival and homeostasis. One of the most important cytokine families for clinical use is the common γ-chain (γ_{c}) family cytokines, which includes interleukin (IL)-2, IL-4, IL-7, IL-9, IL-15 and IL-21 (Liao et al., 2013, Immunity, 38:13-25). IL-2 has been widely studied as an immunotherapeutic agent for cancer. The supplement of IL-2 enhanced the antitumor ability of anti-CD19 CAR-T cells in the clinical trials (Xu et al., 2013, Lymphoma, 54:255-60). However, the administration of IL-2 is limited by side effects and a propensity for expansion of regulatory T cells and the effect of activated induced cell death (AICD) (Malek et al., 2010, Immunity, 33:153-65; and Lenardo et al., 1999, Annu Rev Immunol, 17:221-53). IL-7, IL-15, and IL-21 each can enhance the effectiveness of adoptive immunotherapies and seems to be less toxicity compared with IL-2 (Alves et al., 2007, Immunol Lett, 108:113-20). Despite extensive preclinical and clinical studies on the role of the above cytokines, multi-parameter comparative studies on the roles of various exogenous γ_{c} cytokines on CAR-T cell adoptive therapy are lacking.

Besides γ-chain cytokines, IL-18 is another immunostimulatory cytokine regulating immune responses, which enhances the production of IFN-γ by T cells and augments the cytolytic activity of CTLs (Srivastava et al., 2010, Curr Med Chem, 17:3353-7). Administration of IL-18 is safe and well tolerated, even when the dose reaching as high as 1000µg/kg (Robertson et al., 2006, Clin Cancer Res, 12:4265-73). Therefore, IL-18 could be another candidate used to boost the antitumor of CAR-T cells.

To further enhance the efficacy of adoptive therapy with CAR engineered T (CAR-T) cells, optimization of CAR therapy with administration of exogenous cytokines was examined. To compare the roles of different cytokines administrated exogenously during CAR-T cell immunotherapy and find the optimal cytokine for clinical use, the *in vivo* antitumor ability of CAR-T cells was tested using ovarian cancer animal models.

The following materials and methods were used in the experiments described in this example.

### CAR construction and lentivirus preparation

The pELNS-C4-27z CAR vector was constructed as described previously (manuscript under review), Briefly, the pHEN2 plasmid containing the anti-FRa C4/AFRA4 scFv was used as a template for PCR amplification of C4 fragment using the primers of 5'-ataggatcccagctggtggagtctgggggaggc-3' (SEQ ID NO: 52) and 5'-atagctagcacctaggacggtcagcttggtccc-3' (SEQ ID NO: 53) (BamHI and NheI were underlined). The PCR product and the third generation self-inactivating lentiviral expression vectors pELNS were digested with BamHI and NheI. The digested PCR products were then inserted into the pELNS vector containing CD27-CD3z T-cell signaling domain in which transgene expression is driven by the elongation factor-la (EF-1α) promoter.

High-titer replication-defective lentivirus was generated by transfection of human embryonic kidney cell line 293T (293T) cells with four plasmids (pVSV-G, pRSV.REV, pMDLg/p.RRE and pELNS-C4-27z CAR) by using Express In (Open Biosystems) as described previously (manuscript under review). Supernatants were collected and filtered at 24h and 48h after transfection. The media was concentrated by ultracentrifugation. Alternatively, a single collection was done 30 hr after media change. Virus containing media was alternatively used unconcentrated or concentrated by Lenti-X concentrator (Clontech, Cat# 631232). The virus titers were determined based on the transduction efficiency of lentivirus to SupT1 cells by using limiting dilution method.

### T cells and cell lines

Peripheral blood lymphocytes were obtained from healthy donors after informed consent under a protocol approved by University Institutional Review Board at the University of Pennsylvania. The primary T cells were purchased from the Human Immunology Core after purified by negative selection. T cells were cultured in complete media (RPMI 1640 supplemented with 10% FBS, 100U/mL penicillin, 100µg/mL streptomycin sulfate) and stimulated with anti-CD3 and anti-CD28 mAbs-coated beads (Invitrogen) at a ratio of 1:1 following the instruction. Twenty-four hours after activation, cells were transduced with lentivirus at MOI of 5. Indicated cytokines were added to the transduced T cells from the next day with a final concentration of 10ng/mL. The cytokines were replaced every 3 days.

The 293T cell used for lentivirus packaging and the SupT1 cell used for lentiviral titration were obtained from ATCC. The established ovarian cancer cell lines SKOV3 (FRα+) and C30 (FRα-) was used as target cell for cytokine-secreting and cytotoxicity assay. For bioluminescence assays, SKOV3 was transduced with lentivirus to express firefly luciferase (fLuc).

### Flow cytometric analysis and cell sorting

Flow cytometry was performed on a BD FACSCanto. Anti-human CD45 (HI30), CD3 (HIT3a), CD8 (HIT8a), CD45RA (HI100), CD62L (DREG-56), CCR7 (G043H7), IL-7Rα (A019D5), CD27 (M-T271), CD28 (CD28.2), CD95 (DX2), TNF-α (MAb11), IFN-γ (4S.B3), IL-2 (MQ1-17H12), perforin (B-D48), granzym-B (GB11) were obtained from Biolegend. Biotin-SP-conjugated rabbit anti-human IgG (H+L) was purchased from Jackson Immunoresearch and APC conjugated streptavidin was purchased from Biolegand. Anti-human Bcl-xl (7B2.5) was purchased from SouhernBiotech. Apoptosis kit and TruCount tubes were obtained from BD Bioscience. For peripheral blood T cell count, blood was obtained via retro-orbital bleeding and stained for the presence of human CD45, CD3, CD4 and CD8 T cells. Human CD45+-gated, CD3+, CD4+ and CD8+ subsets were quantified with the TruCount tubes following the manufacturer's instructions.

### In vivo study of adoptive cell therapy

Female non-obese diabetic/severe combined immunodeficiency/γ-chain^{-/-}(NSG) mice 8 to 12 weeks of age were obtained from the Stem Cell and Xenograft Core of the Abramson Cancer Center, University of Pennsylvania. The mice were inoculated subcutaneously with 3×10⁶ fLuc⁺ SKOV3 cells on the flank on day 0. Four or Five mice were randomized per group before treatment. After tumors became palpable, human primary T cells were activated and transduced as described previously. T cells were expanded in the presence of IL-2 (5ng/mL) for about 2 weeks. When the tumor burden was ~250-300 mm³, the mice were injected with 5×10⁶ CAR-T cells or 100µl saline intravenously and then received daily intraperitoneal injection of 5µg of IL-2, IL-7, IL-15, IL-18, IL-21 or phosphate buffer solution (PBS) for 7 days. Tumor dimensions were measured with calipers and tumor volumes were calculated with the following formula: tumor volume = (length×width²)/2. The number and phenotype of transferred T cells in recipient mouse blood was determined by flow cytomtry after retro-orbital bleeding. The mice were euthanized when the tumor volumes were more than 2000 mm³ and tumors were resected immediately for further analysis.

### Statistical analysis

Statistical analysis was performed with Prism 5 (GraphPad software) and IBM SPSS Statistics 20.0 software. The data were shown as mean±SEM unless clarified. Paired sample t-tests or nonparametric Wilcoxon rank tests were used for comparison of two groups and repeated measures ANOVA or Friedman test were used to test statistical significance of differences among three or more groups. Findings were considered as statistically significant when P-values were less than 0.05.

### RESULTS

### Construction and expression of anti-FRa C4 CAR

The pELNS-C4-27z CAR comprised of the anti-FRa C4 scFv linked to a CD8α hinge and transmembrane region, followed by a CD3ζ signaling moiety in tandem with the CD27 intracellular signaling motif (Figure 59A). Primary human T cells were efficiently transduced with C4 CAR lentiviral vectors with transduction efficiencies of 43%~65% when detected at 48h after transduction (Figure 59B). The CAR expression levels were comparable between CD4+ and CD8+ T cells (52.6 ± 10.2% vs. 49.5 ± 17.1%, P=0.713).

### Different anti-tumor efficacy of various cytokines in animal models

This study examined whether the in vivo cytokine administration in combination with CAR-T cell injection could enhance the anti-tumor activity of CAR-T cells. Mice bearing subcutaneous SKOV3 tumors received either saline or 5×10⁶ C4-27z CAR-T cell intravenously injection on day 39 (Figure 60). Compared with saline group, mice receiving CAR-T cell therapy underwent short-time tumor regression and the tumor began to rebounded from day 56 (Figure 61). Of the various cytokine groups, mice receiving IL-15 and IL-21 injection presented best tumor suppression, followed by IL-2 and IL-7, whereas IL-18 and PBS treated mice had the heaviest tumor burden. The persistence of transferred T cells in the peripheral blood was determined 15 days after adoptive transfer and when termination. Highest numbers of CD4+ and CD8+ T cells were detected in mice treated with IL-15, followed by IL-21. The day+15 CD4+ and CD8+ T-cell count were consistent with the tumor regression and predicted the final tumor weight. The mice were killed 73 days after tumor challenge and the tumors were analyzed for the presence of human T cells (Figure 63). Similarly with peripheral blood, mice treated with IL-15 presented highest T cell number in the tumor, followed by IL-21, IL-2, IL-7, PBS and IL-18 (Figure 64).The ratios of CD4 to CD8 were comparable among different cytokine groups, with a predomination of CD4+ T cells both in blood and tumor in all cytokine groups. The CAR expression in CD8+ T cells were comparable among the above groups (45.1% ~ 62.4%), while IL-15 and IL-21 groups had higher proportions of CAR+ CD4 T cells than IL-2 and IL-18 groups (Figure 65). As to the phenotype, all the CAR-T cells in the tumor were CD62L⁻ and CCR7⁻, while 35%~60% of them expressed CD45RA+ (Temra) (data not shown). CD8+ T cells were more likely to retained CD27 expression while the CD28 expression was comparable between CD4+ and CD8+ T cells (Figure 66).

### DISCUSSION

In summary, these findings have important implications for administration of exogenous cytokines to enhance the efficacy of CAR-T cell adoptive therapy. All γ_{c} cytokines when administered in combination with CAR-T cell injection enhance antitumor efficacy in the ovarian mouse model. IL-15 and IL-21 were the best cytokine for in vivo supplement, and IL-7 and IL-2 showed evidence of improving antitumor outcome. IL-18 is a proinflammatory cytokine belonging to the IL-1 family, and in these experiments in vivo, showed no enhanced effect on antitumor efficacy.

### Example 11: DGK Inhibition Augments CART Efficacy

Previous studies, for example, the experiments discussed in Example 6, have suggested that CAR T cells lose efficacy over time in vivo (e.g., in the tumor microenvironment). Specifically, mesoCAR T cells that were injected into a tumor mouse model were isolated from tumors after T cell infusion (e.g., 39 days after, hereinafter referred to as tumor infiltrating lymphocytes, TILs) and were assessed for their functional activity in comparison to freshly thawed mesoCAR T cells. The results showed that in ex vivo killing assays and IFNγ release assays, the mesoCAR T cells isolated from the tumor had reduced ability to kill tumor cells (Fig. 67A), reduced IFNg production (Fig. 67B), and reduced ERK signaling (as shown by phosphorylation in western blot analysis, Fig. 67C) in response to antigen or CD3/CD28 stimuli (indicating reduced T cell activation.

Inhibitory mechanisms that possibly explain the decrease in CAR T cell activity in vivo over time include: soluble factors (TGFb, PGE2, adenosine, IL10, RAGE ligands, etc.), cell to cell contact (PD-1, Lag3, CTLA4, TIM3, CD160, etc.), and intrinsic activation-induced intracellular negative feedback systems (diaglycerol kinases: α and ζ isoforms, Egrs (2 and 3), SHP-1, NFAT2, BLIMP-1, Itch, GRAIL, Cbl-b, Ikaros, etc.). T cell activation can induce factors such as DGK. DGK, in turn, inhibits DAG signaling by phosphorylating DAG. This limits DAG-induced activation of the RAS-ERK-AP1 pathway that leads to T cell activation. Previous studies have shown that mice deficient in DGKα or DGKζ results in CD4 T cells that demonstrate enhanced signal transduction and appear more resistant to anergy-inducing stimuli.

### In vitro Cyotoxicity and Cytokine Release Assays

To investigate the effect of DGK inhibition on CART cell efficacy, transgenic mice with deletions in DGK genes DGKα, DGKζ, or both were utilized. Splenic T cells from wild-type and DGK-deficient mice were isolated, and transduced to express mesoCAR (SS1 BBZ) using retrovirus. MIGR1 CAR was used as a control.

A cytotoxicity assay was performed using similar methods to those described in previous Examples. Wild-type and DGK-deficient (KO) mesoCAR expressing cells were incubated at various effector:target ratios and cytotoxicity (% of target cells killed) was quantified (Fig. 68). As shown in Figure 68, deletion of DGKs markedly enhanced effector function of CAR T cells, especially at low effector: target ratios.

Similarly, IFNγ release was examined in response to target cells at varying effector:target ratios after 18 hours. As shown in Figure 69, deletion of DGKs was found to markedly enhance efefctor function of the mesoCAR T cells, especially at low effector:target ratios.

Western blot analysis of DGK-deficient mesoCAR T cells in comparison to wild-type mesoCAR T cells showed increased ERK phosphorylation (Fig. 70), indicating that presence of DGK suppresses ERK signaling, while deletion of DGK results in increased ERK signaling. The increase in ERK signaling in the DGK deleted background suggests that inhibition of DGK results in activation of the Ras-FRK-AP1 pathway, and therefore, T cell activation.

Recent studies have shown that TGFβ modulates the functionality of tumor-infiltrating CD8 T cells though interfering with RAS/ERK signal transduction, the same signaling molecules by which DGK deficiency confers augmented T cell effects. Sensitivity of the DGK-deficient mesoCAR T cells to TGFβ was examined. WT and DGK-deficient mesoCAR T cells was incubated with mesothelin-expressing AE17 tumor cells + or - 10ng/ml of TGFβ for 18 hours. Cytotoxicity and IFNg production by these T cells was measured. As shown in Figure 71, TGFβ inhibited killing by 50% in WT CAR T cells (arrows). However, this TGFβ-induced inhibition was not observed in CAR T cells with DGK deletion, demonstrating that DGK-deficient cells are not sensitive to TGFβ modulation, and are more resistant to inhibitor stimuli such as TGFβ, which may contribute to the increase in T cell activity.

### Therapeutic efficacy of mesoCAR and DGK inhibition in vivo

Next, therapeutic efficacy of mesoCAR T cells was examined in the context of DGK inhibition or deficiency. AE17meso tumor cells (mesothelioma cells) were injected subcutaneously into C57BL/6 mice. When tumors reached 100 mm³ (approximately a week later), 10 million mesoCAR T cells were injected intravenously via tail vein. Tumor volumes were then followed over at least 18 days.

DGK-deficient mesoCAR T cells demonstrated enhanced and prolonged anti-tumor activity compared to wild-type (WT) mesoCAR and untreated cells (Fig. 72A). Specifically, each of the three DGK-deficient mesoCAR T cells was shown to inhibit tumor growth by volume compared to WT and untreated cells up to 18 days after injection. DGKz-deficient cells expressing mesoCAR were also shown to persist and proliferate better than wild-type meso CAR T cells in mice (Fig. 72B).

These results taken together show that DGK inhibition in combination with mesoCAR T cell treatment can improve mesoCAR T cell activation and anti-tumor activity in therapy.

### Example 12: Inhibition of Ikaros augments anti-tumor capacity of CAR-T cells

One of the major hurdles in CAR T cell therapy is up-regulation of intrinsic negative regulators of T cell signaling, such as diacylglycerol kinase (DGK). As described in Example 11, CAR T cells have been shown to lose efficacy *in vivo* over time. Inhibition of negative regulators of T cell function such as DGK was shown to enhance activity and function of CAR-expressing T cells.

Another important negative regulator of T cell function is the transcription factor Ikaros. Unlike DGKs which act mainly in proximal TCR signaling, Ikaros is a zinc finger DNA binding protein that negatively regulates gene expression through the recruitment of chromatin remodeling complexes, such as Sin3A, CtBP, and HDACs. Ikaros plays a role in regulating cytokine production and cytolytic function in CD4+ T cells and CD8+ T cells,

In this example, anti-tumor efficacy of retrovirally-transduced CAR T cells with reduced Ikaros expression was examined *in vitro* and *in vivo.*

### Materials and Methods

***Cell lines.*** Mouse AE17 mesothelioma cells were described in Jackman et al., J Immunol. 2003;171:5051-63). Human mesothelin were introduced into AE17 cells by lentiviral transduction. 3T3Balb/C cells, were purchased from the American Type Culture Collection. Mouse FAP expressing 3T3BALB/C (3T3.FAP) cells were created by lentiviral transduction of the FAP- 3T3 parental line with murine FAP.

***Animals**.* Pathogen-free C57BL/6 mice were purchased from Charles River Laboratories Inc. (Wilmington, MA). Ikaros DN+/- mice contain one wildtype Ikaros allele and one Ikaros allele with a deletion of a DNA binding domain (Winandy et al., Cell. 1995; 83:289-99). Ikzf1+/- mice have one wildtype Ikaros allele and one allele with deletion of exon 7 (Avitahl et al., Immunity. 1999; 10:333-43). Animals used for all experiments were female mice between 6 and 12 weeks old and were housed in pathogen-free animal facilities.

***Isolation, Transduction and Expansion of Primary Mouse T lymphocytes.*** Primary murine splenic T cells were isolated using the "Pan T cell Negative Selection" kit as suggested by the manufacturer (Miltenyi Biotec), and activated in 24-well plates (4×10⁶ cells/well in 2 mL supplemented RPMI-1640 with 100 U/mL IL-2) pre-coated with -CD3 (1 µg/mL) and -CD28 (2 µg/mL). After 48 hours, cells (1×10⁶ cells/well) were mixed with retrovirus (1 mL crude viral supernatant) in a 24-well plate coated withRetronectin (50 µg/mL; Clontech) and centrifuged, without braking, at room temperature for 45 minutes at 1200g. After overnight incubation, cells were expanded with 50 U/mL of IL-2 for additional 48 hours.

***Antigen- or antibody-coated beads.*** Recombinant mesothelin-extracellular domain protein, bovine serum albumin (Fisher Scientific) or anti-CD3/anti-CD28 antibodies (eBioscience) were chemically crosslinked to tosylactivated 4.5 µm Dynabeads (Invitrogen, #140-13) per manufacturers' instructions.

***Immunoblotting.*** Anti-mesothelin-CAR transduced T cells were incubated either with BSA-, mesothelin-, or anti-CD3 antibody-coated beads (at 2:1 bead to T cell ratio) for 5 and 20 min. Total cell lysates were then prepared and immunoblotted for phosphorylated ERK, phosphorylated AKT, phosphorylated IKK, phosphorylated JNK, phosphorylated Lck, phosphorylated PKC, phosphorylated PLC, or phosphorylated ZAP70. All anti-phospho-protein antibodies were purchased from Cell Signaling, with exception of anti-phospho-Lck, which was purchased from Sigma Aldrich. A C-terminus reactive goat anti-mouse antibody to Ikaros (SC-9861) and a goat anti-mouse actin antibody (SC-1615) were purchased from Santa Cruz. β-actin expression levels were determined to normalize the differences in loading.

***Cytotoxicity and IFN ELISA.*** AE17, AE17.meso, 3T3 and 3T3.FAP cells were transduced with luciferase as described (Moon et al., Clinical Cancer Research. 2011; 17:4719-30). T cells and target cells were co-cultured at the indicated ratios, in triplicate, in 96-well round bottom plates. After 18 hours, the culture supernatants were collected for IFN analysis using an ELISA (mouse IFN, BDOpEIA). Cytotoxicity of transduced T cells was determined by detecting the remaining luciferase activity from the cell lysate using a previously described assay (Riese et al., Cancer Res. 2013; 73:3566-77).

***CAR T cell transfer into mice bearing established tumors.*** Mice were injected subcutaneously with 2 × 10⁶ AE17.meso tumor cells into the dorsal-lateral flank of C57BL/6 mice. Mice bearing large established tumors (100-150 mm³) were randomly assigned to receive either wildtype CAR T cells, Ikaros-deficient CAR T cells or remained untreated (minimum, five mice per group, each experiment repeated at least once). 1×10⁷ T cells were administered through the tail vein. Tumor size was measured by electronic scales and calipers, respectively.

For Day 9 T cell activity assessment, spleen and tumors were processed into single cell suspensions as previously described (Moon et al., Clinical Cancer Research. 2014; 20(16):4262-73) . Splenocytes and tumor single cell suspensions were re-stimulated with soluble anti-CD3/CD28 antibodies (1.0 µg/ml) or with phorbol ester/ionomycin (PMA/I: 30ng/ml, 1uM) for 4-6 hours in the presence of Golgi Stop (BD Biosciences, 0.66µl/ml) and then harvested for flow cytometric analysis.

***Flow cytometric assays.*** Fluorochrome conjugated antibodies against anti-mouse IFN-γ (XMG1), anti-mouse CD25 (PC61), anti-mouse IL-2 (JES6-1A12), anti-mouse CD8 (53-6.7), anti-mouse CD44 (IM7), and anti-mouse CD4 (GK1.5) were purchased from Biolegend. Fixable, Live/Dead Aqua stain (L34957) was purchased from Invitrogen. Fluorochrome antibody to anti-mouse Granzyme B (NGZB) and FoxP3 (FJK-16s) was purchased from eBioscience. Fluorochrome antibodies to anti-mouse TNF-α (MP6-XT22) and anti-mouse CD69 (H1.2F3) were purchased from BD Biosciences. For intracellular cytokine staining, cells were treated with Golgi Stop (BD Biosciences, 0.66 µg/ml) for 4-6 hours. Following harvesting, cells were fixed with 1% paraformaldehyde for 30 minutes, spun down and washed once with FACS buffer. Cells were then washed with BD Perm Wash (BD Biosciences) 2 times and then stained with cytokine antibodies for 45 minutes at room temperature. Cells were washed 2 times in BD Perm Wash and then re-suspended in FACS Buffer. For transcription factor staining, cells were surfaced stained with fluorochrome-labeled primary antibodies for 20 minutes on ice. After washing in FACS buffer, cells were fixed with Fix/Perm buffer from eBioscience. Following fixation, cells were permeabilized and stained with APC anti-mouse FoxP3. For Ikaros staining, rabbit anti-mouse Ikaros (Abcam, ab26083, 1:2000) was used following fixation and permeabilization with the eBioscience FoxP3 kit. Following staining with the Ikaros antibody, cells were washed and then stained with a PE-labeled anti-rabbit secondary antibody (1:2000). Following completion of stains, cells were processed on a CyanADP (Beckman Coulter) for flow cytometric analysis.

***Statistical Analysis.*** All statistical tests were done with GraphPad Prism. Two-way ANOVA was conducted with post-hoc testing, with ^{∗} p<0.05, ^{∗∗} p<0.01, ^{∗∗∗} p<0.001, and ^{∗∗∗∗} p<0.0001. Data are presented as mean +/- SEM.

### Results

### Cytokine production and cytolytic mediator release in CAR-expressing T cells with reduced levels of Ikaros are augmented

Given that cytolytic T lymphocytes (CTLs) with reduced Ikaros have enhanced effector function *in vitro* and *in vivo* (O'Brien, et al., J Immunol. 2014; 192:5118-29), experiments were performed to test if depletion of Ikaros could improve the efficacy of CAR T therapy. T cells isolated from wild type C57BL/6 and Ikaros-haplodeficient mice (*Ikzf1*+/-) in the C57BL/6 background were retrovirally-transduced to express an anti-mesothelin CAR. Following *ex vivo* activation, transduction, and expansion in IL2, it was confirmed that, in comparison to wild-type (WT) CAR T cells, *Ikzf1*+/- CAR T cells continued to express less Ikaros protein by flow cytometry and western blot (Fig. 73A).

Since Ikaros is a transcriptional repressor for multi-cytokine gene loci (Thomas et al., J Immunol. 2007; 179:7305-15; Bandyopadhyay et al., Blood. 2006; 109:2878-2886; Thomas et al., J of Biological Chemistry. 2010; 285:2545-53; and O'Brien et al., J Immunol. 2014; 192:5118-29), it was next examined if reduction of Ikaros resulted in autocrine cytokine production by CAR T cells and also whether or not the Ikaros-haplodeficient CAR T cells responded better than their WT counterparts to their target antigen. Both WT and *Ikzf1*+/- CAR T cells were stimulated with beads coated with either BSA- (control) or mesothelin (the CAR antigen) at a 2:1 bead:T cell ratio for 6 hours, and analyzed their ability to produce IFNγ, TNFβ and IL2 by flow cytometry. At baseline (BSA stimulation), there was an ~3-fold increase in IFN-producing *Ikzf1*+/- CAR T cells compared to WT CAR T cells (4.35% vs 1.4%, Fig. 73B), but there was no significant difference in the % IL2 producing cells (Fig. 73D). Following stimulation with mesothelin-coated beads, there was a dramatic increase in the % IFN-γ cytokine-producing *Ikzf1*+/- CAR T cells (25%) while the response was modest in WT CAR T cells (7%). An increase in TNFα production was also seen (Fig. 73C). To investigate if this augmentation in cytokine production was generalized across different stimuli or limited to CAR antigen, both WT and *Ikzf+*/*-* CAR T cells were treated with PMA and ionomycin for 6 hours. In this case, more IFN γ, TNF-β and IL-2 cytokine-producing cells were observed in the *Ikzf1*+/- CAR T cell compared to their WT counterparts (Figs. 73B-73D). These data support the hypothesis that Ikaros is one of the limiting factors that suppresses cytokine production of T cells, or CAR T cells.

An important cytotoxic mediator, granzyme B, was shown to be up-regulated in CD3/CD28-activated Ikaros-deficient OT-I cells, and this increased their cytolytic activity against OVA-expressing EL4 tumor cells (O'Brien et al., J Immunol. 2014; 192:5118-29). It was hypothesized that granzyme B production would also be enhanced in *Ikzf+*/*-* T cells bearing CAR. Both WT and *Ikzf1*+/- CAR T cells were stimulated with either BSA-(baseline) or mesothelin- (CAR antigen) coated beads at 2:1 bead:T cell ratio for 6 hours. PMA/ionomycin was used as the positive control for the assay. Similar to the data above, granzyme B level was higher in *Ikzf*+/*-* CAR T cells than in WT CAR T cells at baseline (BSA stimulation; Fig. 73E). After stimulation with either mesothelin-coated beads or PMA/Ionomycin, granzyme B level increased in both WT and *Ikzf*+/*-* CAR T cells but the production was much higher in the *Ikzf*+/*-* CAR T cells (Fig. 73E). To determine if there was also a difference in degranulation of CAR T cells with reduced Ikaros, CD107a expression after antigen stimulation was assessed. Wild-type transduced T cells had moderate levels of CD107a expression following antigen re-stimulation, however, the T cells with reduced Ikaros demonstrated enhanced CD107a up-regulation (Fig. 73F). Thus, in response to re-stimulation, the CTLs with reduced Ikaros degranulate more and release more cytotoxic mediators in comparison to their wild-type counterparts.

### Depleting Ikaros with a dominant negative allele enhances CAR T cell function

In addition to the cells with lower levels of Ikaros, T cells from mice expressing one dominant-negative allele of Ikaros (IkDN) were studied. Transgenic mice expressing IkDN have normal lymphoid development but have peripheral T cells with 90% reduced Ikaros DNA binding activity (Thomas et al., J Immunol. 2007; 179:7305-15; and Winandy et al., Cell. 1995; 83:289-99). T cells isolated from spleens of WT and IkDN mice were activated with plate-bound anti-CD3/CD28 antibodies, transduced with anti-mesothelin CAR, followed by expansion with IL2. Knockdown of Ikaros in IkDN CAR T cells was confirmed by western. WT and IkDN CAR T cells were re-challenged with either BSA- or mesothelin-coated beads at 2:1 bead:T cell ratio for 6 hours, and analyzed their ability to produce IFN and IL2, as well as to de-granulate in response to CAR antigen. Similar to the *Ikzf1*+/- data above, some autocrine IFNγ production at baseline was observed (Fig. 74A), but not with IL2 (BSA stimulation; Fig. 74B). Upon ligation of the CAR with its target antigen, mesothelin, IkDN T cells made more IFNγ than WT T cells (Mesothelin stimulation; Fig. 74A). De-granulation, as measured by CD107a up-regulation, was also similar in both wild-type and IkDN CAR T cells (Fig. 74D).

### Depletion of Ikaros did not augment activation and signaling of CAR T cells following antigen stimulation

Given that depletion in Ikaros augmented cytokine release and increased the Granzyme B levels and CD107a expression of CAR T cells, possible mechanisms were explored. It is plausible that these changes in effector function could be due to differences in the activation of the wild-type and *Ikzf1*+/- transduced T cells. Thus, the levels of CD69, CD25 and 4-1BB (markers of T cell activation) were measured by flow cytometry after stimulating with mesothelin-coated beads for 6 and 24 hours. CD69, an early activation marker, was up-regulated to the same extent by both the wild-type and *Ikzf1*+/- cells (Fig. 75A). With longer stimulation, the wild-type and *Ikzf1*+/- CAR-expressing T cells continued to express similar levels of CD69, but *Ikzf1*+/- transduced T cells exhibited increased CD25 expression (Fig. 75B). This may not directly indicate a difference in T cell activation, however, as increased IL-2 by *Ikzf1*+/- cells (Fig. 71D) can act in a positive feed-forward loop on CD25, the IL-2Ra (Depper et al., Proc Natl Acad of Sci USA. 1985; 82:4230-4; and Nakajima et al., Immunity. 1997; 7:691-701). 4-1BB, a member of the TNF Receptor superfamily is also expressed on activated T cells (Vinay et al., Seminars in Immunology. 1998; 10:481-9) and was expressed at similar levels by CAR transduced wild-type and *Ikzf1*+/*-* T cells following antigen stimulation (Fig. 75C). Thus, functional differences between WT and *Ikzf1*+/- transduced T cells were not due to differences in T cell activation.

The experiments described in Example 11 and Riese et al., Cancer Research. 2013; 73:3566-77, demonstrate that depletion of the enzyme diacylglycerol kinase (DGK) in CAR T cells resulted in an increase in RAS/ERK signaling, which correlated well with enhanced activation of CAR T cells. Some signaling pathways in WT and Ikaros-deficient T cells after TCR stimulation with CD3/CD28 antibodies were examined. Lysates from stimulated T cells were prepared and immunoblotted for various phospho-proteins implicated in proximal (PLC and Lck) and distal (ERK1/2, JNK, AKT and IKKa) signaling from the TCR. There was a constitutive low-level baseline activation of some TCR signaling proteins in Ikaros-deficient T cells, including Lck, ERK and AKT (Fig. 75D). With TCR/CD28 stimulation, all proteins studied were phosphorylated to the same level when comparing WT and Ikaros-deficient T cells, with the exception of phospho-IKK, which was slightly higher in Ikaros-deficient T cells 20 minutes after stimulation. To determine if the NFB pathway was enhanced in T cells with reduced Ikaros level, the same blot was re-probed for IB, the downstream target for IKK. There was no difference in IB degradation between both WT and Ikaros-depleted T cells. To study CAR signaling, both WT and IkDN anti-mesothelin CAR transduced T cells were re-stimulated with mesothelin-coated beads. Similar to the data with CD3/CD28 stimulation, no difference was found in phosphorylation of PLC and ERK (Fig. 75E). Together, these data indicate that depletion in Ikaros does not alter TCR/CAR-mediated signaling.

### Reduction of Ikaros in CAR T cells augments their response against their target cells.

Given the increased production of effector factors by CAR T cells with reduced Ikaros, their efficacy against their target tumor cells *in vitro* was tested. Wild-type, *Ikzf1*+/- and IkDN T cells expressing mesoCAR were mixed at different ratios with the parental tumor cell line, AE17 or the mesothelin-expressing cell line, AE17meso. When mixed with the parental cell line, both the wild-type, *Ikzf1*+/- and IkDN T cells failed to produce IFN-γ or lyse cells in response to AE17 (Figs. 76A, 76B and 76C). In contrast, when reacted with AE17meso, IFN-γ production and cytolysis by wild-type T cells increased as the E:T ratio increased (Figs. 76B and 76C). However, both the *Ikzf1*+/- and IkDN T cells produced significantly more IFN-γ and had significantly increased tumor lysis than wild-type T cells, even at the lowest E:T ratio 1.3:1 (Figs. 76B, and 76C).

To study the generalizability of this effect, T cells expressing a different CAR construct, which targets fibroblast activation protein (FAP-CAR) and has the same intracellular signaling domain as the anti-mesothelin CAR used above, were examined. The efficacy of comparably transduced FAP-CAR splenic T cells isolated from WT C57BL/6 was compared to those from *Ikzf1*+/- mice. *Ikzf1*+/- FAP-CAR T cells were more efficient in lysing 3T3.FAP cells (Fig. 76D) and in secreting more IFN (Fig. 76E) than WT FAP CAR T cells, with retention of specificity *in vitro.*

### Depletion of Ikaros enhances the efficacy of CAR T cells against established tumors

The capability of mesothelin-specific T cells with reduced Ikaros *(Ikzf1*+/- and IkDN) to control growth of established AE17meso tumors in mice was next examined. Two million of AE17meso tumor cells were injected into the flanks of syngenic C57BL/6 mice and allowed to form large established tumors (~100-150 mm³). Ten million CAR T cells prepared from WT or Ikaros-deficient *(Ikzf1*+/- and IkDN) mice were then adoptively transferred into those tumor-bearing mice, and tumor measurements were followed. Mild tumor growth inhibition was induced by wild-type transduced mesoCAR T cells, while both Ikzf1+/- and IkDN transduced mesoCAR T cells inhibited growth of AE17meso tumors significantly more (Figs. 77A and 77B).

It was also studied if reduction of Ikaros could enhance the therapeutic potential of FAP-CAR T cells. Mice with established AE17meso tumors (100-150 mm³) were adoptively transferred with 10 millions wild-type or *Ikzf1*+/- transduced anti-mouse FAP CAR T cells. Mice receiving wild-type transduced cells provided minimal tumor delay and the AE17meso tumors continued to grow (Fig. 77). In contrast, the *Ikzf1*+/- transduced T cells were able to significantly delay tumor growth.

### Ikzf1+/- CAR T cells persist longer and more resistant to immunosuppressive tumor microenvironment than WT CAR T cells

Given the enhanced efficacy of the Ikaros-inhibited CAR T cells, the possible mechanisms using the Ikzf1+/- mesoCAR T cells were explored. To further interrogate how these mesoCAR T cells operate in an immunosuppressive tumor microenvironment *in vivo,* tumors at 3 and 9 days post-adoptive transfer were harvested and assessed their number and functionality. These two time points allowed characterization of their activity at early and late time points during the anti-tumor immune response.

At Day 3 post-transfer, we observed a similar frequency of wild-type and Ikzf1+/- mesoCAR T cells in both the spleens (Fig. 78A) and tumors (Fig. 78B). These similar levels indicate that both the wild-type and Ikzf1+/- mesoCAR T cells initially traffic equally well to the tumor. In assessing the Day 9 timepoint, the number of both wild-type mesoCAR T cells *Ikzf1*+/- mesoCAR T cells declined in the spleen. However, when the tumors were examined at this time point, there was a significant increase in the number of *Ikzf1*+/- mesoCAR T cells compared with WT mesoCAR T cells (Fig. 78B). These data show that the *Ikzf1*+/- mesoCAR T cells either persist or proliferate better than WT mesoCAR T cells in the immunosuppressive microenvironment.

Tumor infiltrating lymphocytes (TILs) become hypofunctional in response to their cognate antigens within the immunosuppressive tumor microenvironment, and is a key phenomenon associated with tumor progression (Prinz et al., J Immunol. 2012; 188:5990-6000; and Kerkar et al., Cancer Res. 2012; 72:3125-30). Although there were more *Ikzf1*+/- mesoCAR T cells in the tumors at Day 9, they could still be adversely affected by the tumor microenvironment. To evaluate functionality, CD3/CD28 antibodies were used to stimulate TILS isolated from wild-type and *Ikzf1*+/- mesoCAR T cells at Day 9 post-transfer and characterized differences in lytic mediator production. At Day 9 post-transfer, the wild-type mesoCAR T cells in the spleen continued to produce some moderate levels of IFN (Fig. 78C). As expected, isolated wild-type T cells from the tumors produced much less of this cytokine in comparison to wild-type T cells isolated from the spleen. This indicates that the wild-type TILs begin to become hypofunctional at Day 9 post transfer. In contrast, splenic *Ikzf1*+/- mesoCAR T cells continued to produce more IFN- at baseline and upon stimulation (Fig. 78C). Compared to the wild-type TILs, the *Ikzf1*+/- TILs produced higher amounts of IFNγ. These data indicate that Ikzf1+/- TILs could be less sensitive to the immunosuppressive tumor microenvironment.

Bypassing the proximal defect of TCR signaling often seen in TILs can be achieved through use of PMA/Ionomycin (PMA/I) (Prinz et al., J Immunol. 2012; 188:5990-6000). Wild-type and *Ikzf1*+/*-* mesoCAR T cells were re-stimulated with PMA/I to determine if TCR-stimulation insensitive wild-type and *Ikzf1*+/*-* TILs were still capable making cytokines in response to other stimuli. At Day 9 post-transfer, the wild-type TILs demonstrated a noticeable drop in IFN-γ production (Fig. 78C), and this was partially restored through stimulation with PMA/I (Fig. 78D). However, stimulation of splenic and tumor isolated *Ikzf1*+/*-* mesoCAR T cells still resulted in increased levels of IFN-γ in comparison to wild-type transferred cells. Through bypassing any defects in TCR signaling via PMA/I stimulation, these results demonstrate that IFNγ production differs at the chromatin level and is likely due to differential Ikaros function.

In light of the increased anti-tumor activity by the transferred *Ikzf1*+/- T cells, the impacts on the composition of the immunosuppressive tumor microenvironment was also examined and the number of regulatory T cells (Tregs) and Myeloid Derived Suppressor Cells (MDSCs) was evaluated at Day 9. At Day 9 post-transfer, similar levels of Tregs in hosts that received wild-type or *Ikzf1*+/- mesoCAR T cells was observed (Fig. 78E). The presence of Ly6G-/CD11b+/CD206+ macrophages was determined, which are typically characterized as immunosuppressive and pro-tumorigenic M2 macrophages. In the Day 9 treated groups, that CD206 expression was similar in all 3 groups (untreated, wild-type, and Ikzf1+/-) (Fig. 78F).

### T cells with reduced Ikaros are less sensitive to soluble inhibitory factors TGF and adenosine

To further characterize the interaction of the immunosuppressive tumor microenvironment with the mesoCAR T cells, an *in vitro* culture system was utilized. Soluble inhibitory factors such as IDO, IL-10, Adenosine, and TGF-β (Wang et al., Oncoimmunology. 2013; 2:e26492) have been shown to contribute to inhibiting infiltrating tumor lymphocytes. The effects of select inhibitory factors *in vitro* on the Ikaros-deficient CAR T cells was tested to determine if the immunosuppressive environment could impact their lytic function. Wild-type CAR T cells had a 50% reduction in their ability to make IFN-γ and had a reduction in their lytic function in the presence of TGF-β and Adenosine (Fig. 79). CAR T cells with reduced levels of Ikaros *(Ikzf1*+/- and IkDN) continued to produce more IFN γ than their wild-type counterparts in the absence of inhibitors and were only marginally inhibited in the presence of TGF β and Adenosine (Fig. 79A). Increased lytic function in *Ikzf1*+/- and IkDN CAR T cells in comparison to wild-type T cells was observed (Fig. 79B). These data demonstrate that the T cells with reduced Ikaros are less sensitive to TGFβ and adenosine inhibition.

### Discussion

In this example, a new approach for enabling CAR-expressing T cells to survive and enhance their effector functions in the tumor environment has been identified: inactivation of the transcriptional repressor Ikaros, which is known to inhibit a diverse array of genes involved in T cell function, e.g., cytokine genes (IL2 and IFNγ), cytolytic mediators (granzyme B), and the key T-box transcription factors that influence T cell differentiation (R-Bet and Eomes).

An important finding from the experiments described above is that CAR T cells that were deficient in Ikaros function were significantly better than wild type CAR T cells in restricting tumor growth (Fig. 77). These results were observed in multiple tumor models and using two different CAR constructs. Due to the high number of genes that Ikaros regulates, it is plausible that Ikaros may regulate many pathways that are normally sensitive to immunosuppression. Increased IFN-g production by lowering Ikaros level in CAR T cells can result in up-regulation of Class I MHC expression on the tumor, and thereby improving its immunogenicity, improving anti-angiogenic activity, and driving STAT1 mediated fuction of Th1 cells. A possible effect of increased IFNγ could have been an alternation of the macrophage phenotype within the tumors, however, no differences in the total number of macrophages, nor the proportion of M2-like macrophages (as measured by CD206 expression) was observed. The increased IL-2 production could have also increased the formation of CD4 Treg cells, however, no differences were observed when comparing the tumors treated with WT CAR T cells with Ikaros-deficient CAR T cells.

An increased number of tumor infiltrating lymphocytes nine days after injection was observed. This was not likely due to increased trafficking, since the number of WT versus Ikaros-deficient TIL was similar at Day 3. Instead, these results suggest that Ikaros-deficient TIL showed increased proliferation or decreased antigen-induced cell death (AICD). *In vitro* studies suggest that AICD was similar between the two types of T cells, making it more likely that the difference was due to increased proliferation. This would be consistent with the increased IL-2 produced by these cells. In addition to increased persistence, the Ikaros-deficient TIL appeared to be less hypofunctional. When tumor-infiltrating CAR T cells were re-stimulated with anti-CD3 antibody or PMA and ionomycin, CAR TILs with Ikaros deficiency were able to make more IFNγ than their wild-type counterparts (Fig. 78C and 78D).

The *in vitro* studies allowed further studies of the mechanistic underpinnings of the observed increased anti-tumor efficacy *in vivo.* Consistent with the known inhibitor functions of Ikaros, deletion of one Ikaros allele (*Ikzf*+*l-*) or replacing one of its alleles with an Ikaros dominant negative construct (IkDN) resulted in T cells that had some increased baseline autocrine IFNγ and Granzyme B production (Fig. 73), but more importantly, showed markedly augmented cytokine secretion and granule release after TCR or CAR stimulation *in vitro.* This was accompanied by increased tumor cell killing *in vitro.* To test whether or not Ikaros-deficient CAR T cells have lower activation threshold than WT CAR T cells, Dynabeads were coated with 10-fold less mesothelin protein and it was shown that *Ikzf*+/*-* CAR T cells could still respond to the mesothelin antigen at low-density to make IFNγ and TNF-α but the WT CAR T cells could not. The Ikaros-disabled CAR T cells were also more resistant to inhibition by known immunosuppressive factors such as TGF-β and adenosine (Fig. 79). This may be due to the fact that cytokine (i.e. IL2 and IFNγ and T cell effector (i.e. granzyme B) genes are more accessible for transcription in Ikaros-deficient T cells following TCR/CAR activation. This appears to help compensate for suboptimal T cell activation within immunosuppressive tumor microenvironment.

In previous studies, e.g., Example 11, a similar phenotype (i.e. increase in cytolysis and IFN production) has been observed in CAR T cells through depletion of DGKs, enzymes that metabolize the second messenger diacylglycerol and limit RAS/ERK activation. With DGK deletion, however, clear changes were observed in the CAR/TCR signaling pathway. Specifically, RAS/ERK activation was dramatically enhanced after both TCR and CAR activation. This resulted in enhanced activation, as measured by increased CD69 upregulation, however production of effector molecules such as TRAIL, FasL and IFNγ. Perforin and Granzyme B were similar between WT and DGK-deficient CAR T cells. A very different phenotype in this study with Ikaros-deficient CAR T cells. In contrast to the DGK-deficient CAR T cells, the Ikaros-deficient CAR T cells had similar CAR/TCR activation as shown by CD69 and CD25 upregulation (Fig. 75), and similar CAR/TCR signaling as measured by phosphorylation of multiple TCR signaling molecules (Fig. 75) and calcium signaling. Unlike DGK-deficient T cells, Ikaros-depleted CAR T cells had higher granzyme B and IFN levels at baseline (Figs. 73 and 74), as well as constitutive low level activation of some TCR signaling cascades such as ERK and Akt (Fig. 75). This baseline activation may be due to induction of T-bet (Thomas et al., J of Biol Chemistry. 2010; 285:2545-53), which cooperates with other transcription factor like Eomes to transactivate IFN and granzyme B gene expression (Pearce et al., Science. 2003; 302:1041-3; and Intelkofer et al., Nat Immunol. 2005; 6:1236-44).

These findings raise the possibility that therapeutically targeting Ikaros in transduced human T cells (in clinical trials) might be beneficial using either genetic or biochemical approaches. Genetic approaches could mimic these results in mice and include knockdown of Ikaros in CAR T cells using shRNA or use of a dominant negative construct to compete with endogenous Ikaros. Another option would be to use a pharmacological inhibitor to lower Ikaros levels transiently. Recent reports have indicated that the immunomodulatory drug, Lenalidomide, targets Ikaros for ubiquitin-mediated degradation by the E3 ligase complex CRL4CRBN (Gandhi et al., Br J Haematol. 2013; 164:811-21; Kronke et al, Science. 2014; 343:301-5; and Sakamaki et al., Leukemia. 2013; 28:329-37). CD3-stimulated human T cells treated with Lenalidomide produce more IL-2 (Gandhi et al., Br J Haematol. 2013; 164:811-21), a key trait of T cells with reduced Ikaros levels. In preliminary studies, TCR/CD28 stimulated mesothelin-CAR transduced human PBMCs *in vitro* produce more IL-2 and IFNγ after pretreatment with lenalidomide. Thus, the combination of CAR T cell therapy with *in vivo* administration of Lenalidomide may provide a therapeutic strategy for reversal of T cell hypofunction through inhibition of Ikaros.

In conclusion, this example demonstrates for the first time that targeting a transcriptional repressor can enhance CAR-mediated anti-tumor immunity. The mechanisms involved enhanced cytokine and effector function without alterations in signal transduction. Translating this approach into the clinic can be pursued through the use of shRNA, a dominant negative construct, or a pharmacological inhibitor (like Lenalidomide) to target Ikaros in CAR-expressing T cells.

### Example 14: Exogenous IL-7 enhances the function of CAR T cells

After adoptive transfer of CAR T cells, some patients experience limited persistence of the CAR T cells, which can result in suboptimal levels of anti-tumor activity. In this example, the effects of administration of exogenous human IL-7 is assessed in mouse xenograft models where an initial suboptimal response to CAR T cells has been observed.

### Exogenous IL-7 treatment in a lymphoma model

Expression of the IL-7 receptor CD127 was first assessed in different cancer cell lines and in CAR-expressing cells. Two mantle cell lymphoma cell lines (RL and Jeko-1) and one B-ALL cell line (Nalm-6) were analyzed by flow cytometry for CD127 expression. As shown in Figure 80A, out of the three cancer cell lines tested, RL was shown to have the highest expression of CD127, followed by Jeko-1 and Nalm-6. CART19 cells were infused into NSG mice and CD127 expression was assessed on the circulating CART19 cells by flow cytometry. As shown in Figure 80B, CD127 is uniformly expressed on all circulating CART19 cells.

Next, the effect of exogenous IL-7 treatment on anti-tumor activity of CART19 cells was assessed in a lymphoma animal model. NSG mice were engrafted with a luciferase-expressing mantle cell line (RL luc) on Day 0 (D0), followed by treatment of CART19 cells on Day 6. The NSG mice were divided into groups, where one group received no CART19 cells, a second group received 0.5 ×10⁶ CART19 cells, a third group received 1×10⁶ CART19 cells, and a fourth group received 2×10⁶ CART19 cells. Tumor size was monitored by measuring the mean bioluminescence of the engrafted tumors over more than 80 days. Only mice receiving 2×10⁶ CART19 cells demonstrated rejection of the tumor and inhibition of tumor growth (Figure 81A). Mice from the two groups receiving 0.5 ×10⁶ CART19 cells or 1×10⁶ CART19 cells were shown to s a suboptimal anti-tumor response. Mice from these two groups were then randomized, where three mice (mouse #3827 and #3829 which received 0.5×10⁶ CART19 cells, and mouse #3815 which received 1×10⁶ CART19 cells) received exogenous recombinant human IL-7 at a dosage of 200 ng/mouse by intraperitoneal injection three times weekly starting at Day 85, and two mice did not. The tumor burden of mice receiving exogenous IL-7 from Day 85-125, as detected by mean bioluminescence, is shown in Figure 81B. All mice receiving IL-7 showed a dramatic response of 1-3 log reduction in tumor burden. Mice that originally received a higher dose of CART19 cells (mouse #3815 which received 1×10⁶ CART19 cells) showed a more profound response. When comparing the tumor burden of mice that received IL-7 treatment to control, before and after IL-7 treatment, tumor reduction in tumor burden was only seen in the mice that had received IL-7 treatment (Figure 81C).

T cell dynamics following IL-7 treatment in the lymphoma animal model was also examined. Human CART19 cells were not detectable in the blood prior to IL-7 treatment. Upon treatment of IL-7, there was rapid, but variable increase in the numbers of T cells in the treated mice (Figure 82A). The extent of T cell expansion observed in mice receiving the IL-7 also correlated with tumor response. The mouse with the highest number of T cells detected in the blood at peak expansion during IL-7 treatment (mouse #3815) had the most robust reduction in tumor burden (see Figure 81B). Moreover, the time of peak expansion correlated with the T cell dose injected as baseline. The number/level CD3-expressing cells in the blood were also measured before and after IL-7 treatment. In control mice, very few CD3-expressing cells were detected, while IL-7-treated mice showed a significant increase in CD3+ cells after IL-7 treatment (Figure 82B).

### Exogenous IL-7 treatment in a leukemia model

IL-7 receptor (CD127) expression was measured in leukemia cell lines (AML cell line MOLM14 and B-ALL cell line NALM6) and primary AML cells by flow cytometry (Figure 83, top panels). IL-7 receptor expression is expressed on the B-ALL cell line NALM6 cells, but not on the AML cell line MOLM14 or primary AML. Flow cytometry analysis was gated such that IL-7 receptor expression was detected on tumor cells only.

Next, the effect of exogenous IL-7 treatment on anti-tumor activity of CART33 or CART123 cells was assessed in a leukemia animal model of AML relapse after an initial CART treatment (Figure 84). Luciferase-expressing MOLM14 cells were injected into NSG mice, and the mice developed AML. CART33 or CART123 treatment was initiated and tumor burden was monitored by serial bioluminescence imaging. Untransduced T cells were injected as control. Mice that received CART33 or CART123 treatment initially responded to T cell treatment, but relapsed by 14 days after T cell infusion (Figure 85A). IL-7 receptor expression was measured on AML cells by flow cytometry in the mice that exhibited an AML relapse. IL-7 receptor expression was not detected in the relapsed AML cells, whether they were treated by CART33 or CART123 (Figure 83, bottom panels).

At Day 28, the mice that had relapsed were randomized and assigned to receive either no treatment (control) or IL-7 treatment at a dose of 200 ng/mouse by intraperitoneal injection three times a week. Tumor burden after IL-7 treatment or control treatment was monitored by weekly bioluminescence imaging and response, T cells expansion, and overall survival was also assessed. Figure 85B shows the best response after IL-7 treatment was shown for the IL-7 treatment and control groups, as determined by bioluminescence imaging (BLI). Representative bioluminescence images are shown in Figure 85C during the 28 days of IL-7 or control treatment, showing that anti-tumor response was increased in mice receiving IL-7 treatment. T cell expansion (e.g., CART33 and CART123) was quantified from the blood of the mice, and the increase in T cell number in the blood during IL-7 treatment correlated with reduction in tumor burden (Figure 86A). Mice receiving IL-7 treatment also demonstrated enhanced survival (Figure 86B).

Together, the results in this example demonstrate that exogenous IL-7 treatment increases T cell proliferation and anti-tumor activity *in vivo,* indicating that use of IL-7 in patients with suboptimal results after CAR therapy or relapse can improve anti-tumor response in these patients.

Without further description, it is believed that one of ordinary skill in the art can, using the preceding description and the following illustrative examples, make and utilize the compounds of the present disclosure and practice the disclosed methods.

**EQUIVALENTS**

## Claims

1. An immune effector cell (*e.g.* a population of immune effector cells), comprising:
(a) a first chimeric antigen receptor (CAR) molecule or nucleic acid encoding the same, wherein said first CAR molecule is a CAR polypeptide comprising an antigen binding domain, a transmembrane domain, and an intracellular signaling domain comprising a costimulatory domain and/or a primary signaling domain, and wherein said antigen binding domain comprises a VHH domain and binds to a tumor antigen; and
(b) a second CAR molecule or nucleic acid encoding the same, wherein said second CAR molecule is a CAR polypeptide having an antigen binding domain comprising an scFv.

2. The immune effector cell of claim 1, wherein the nucleic acid encoding the first CAR molecule and the nucleic acid encoding the second CAR molecule are each present in a vector.

3. The immune effector cell of claim 1 or 2, wherein the VHH domain is a camelid VHH domain.

4. The immune effector cell of any one of claims 1-3, wherein the tumor antigen is selected from a group consisting of: CD19; mesothelin; epidermal growth factor receptor (EGFR); CD123; CD22; CD30; CD171; CS-1 (also referred to as CD2 subset 1, CRACC, SLAMF7, CD319, and 19A24); C-type lectin-like molecule-1 (CLL-1 or CLECL1); CD33; epidermal growth factor receptor variant III (EGFRvIII); ganglioside G2 (GD2); ganglioside GD3 (aNeu5Ac(2-8)aNeu5Ac(2-3)bDGalp(1-4)bDGlcp(1-1)Cer); TNF receptor family member B cell maturation (BCMA); Tn antigen ((Tn Ag) or (GalNAcα-Ser/Thr)); prostate-specific membrane antigen (PSMA); Receptor tyrosine kinase-like orphan receptor 1 (ROR1); Fms-Like Tyrosine Kinase 3 (FLT3); Tumor-associated glycoprotein 72 (TAG72); CD38; CD44v6; Carcinoembryonic antigen (CEA); Epithelial cell adhesion molecule (EPCAM); B7H3 (CD276); KIT (CD117); Interleukin-13 receptor subunit alpha-2 (IL-13Ra2 or CD213A2); Interleukin 11 receptor alpha (IL-11Ra); prostate stem cell antigen (PSCA); Protease Serine 21 (Testisin or PRSS21); vascular endothelial growth factor receptor 2 (VEGFR2); Lewis(Y) antigen; CD24; Platelet-derived growth factor receptor beta (PDGFR-beta); Stage-specific embryonic antigen-4 (SSEA-4); CD20; Folate receptor alpha; Receptor tyrosine-protein kinase ERBB2 (Her2/neu); Mucin 1, cell surface associated (MUC1); neural cell adhesion molecule (NCAM); Prostase; prostatic acid phosphatase (PAP); elongation factor 2 mutated (ELF2M); Ephrin B2; fibroblast activation protein alpha (FAP); insulin-like growth factor 1 receptor (IGF-I receptor), carbonic anhydrase IX (CAIX); Proteasome (Prosome, Macropain) Subunit, Beta Type, 9 (LMP2); glycoprotein 100 (gp100); oncogene fusion protein consisting of breakpoint cluster region (BCR) and Abelson murine leukemia viral oncogene homolog 1 (Abl) (bcr-abl); tyrosinase; ephrin type-A receptor 2 (EphA2); Fucosyl GM1; sialyl Lewis adhesion molecule (sLe); ganglioside GM3 (aNeu5Ac(2-3)bDGalp(1-4)bDGlcp(1-1)Cer); transglutaminase 5 (TGS5); high molecular weight-melanoma-associated antigen (HMWMAA); o-acetyl-GD2 ganglioside (OAcGD2); Folate receptor beta; tumor endothelial marker 1 (TEM1/CD248); tumor endothelial marker 7-related (TEM7R); claudin 6 (CLDN6); thyroid stimulating hormone receptor (TSHR); G protein-coupled receptor class C group 5, member D (GPRC5D); chromosome X open reading frame 61 (CXORF61); CD97; CD179a; anaplastic lymphoma kinase (ALK); Polysialic acid; placenta-specific 1 (PLAC1); hexasaccharide portion of globoH glycoceramide (GloboH); mammary gland differentiation antigen (NY-BR-1); uroplakin 2 (UPK2); Hepatitis A virus cellular receptor 1 (HAVCR1); adrenoceptor beta 3 (ADRB3); pannexin 3 (PANX3); G protein-coupled receptor 20 (GPR20); lymphocyte antigen 6 complex, locus K 9 (LY6K); Olfactory receptor 51E2 (OR51E2); TCR Gamma Alternate Reading Frame Protein (TARP); Wilms tumor protein (WT1); Cancer/testis antigen 1 (NY-ESO-1); Cancer/testis antigen 2 (LAGE-1a); Melanoma-associated antigen 1 (MAGE-A1); ETS translocation-variant gene 6, located on chromosome 12p (ETV6-AML); sperm protein 17 (SPA17); X Antigen Family, Member 1A (XAGE1); angiopoietin-binding cell surface receptor 2 (Tie 2); melanoma cancer testis antigen-1 (MAD-CT-1); melanoma cancer testis antigen-2 (MAD-CT-2); Fos-related antigen 1; tumor protein p53 (p53); p53 mutant; prostein; surviving; telomerase; prostate carcinoma tumor antigen-1 (PCTA-1 or Galectin 8), melanoma antigen recognized by T cells 1 (MelanA or MART1); Rat sarcoma (Ras) mutant; human Telomerase reverse transcriptase (hTERT); sarcoma translocation breakpoints; melanoma inhibitor of apoptosis (ML-IAP); ERG (transmembrane protease, serine 2 (TMPRSS2) ETS fusion gene); N-Acetyl glucosaminyl-transferase V (NA17); paired box protein Pax-3 (PAX3); Androgen receptor; Cyclin B1; v-myc avian myelocytomatosis viral oncogene neuroblastoma derived homolog (MYCN); Ras Homolog Family Member C (RhoC); Tyrosinase-related protein 2 (TRP-2); Cytochrome P450 1B1 (CYP1B1); CCCTC-Binding Factor (Zinc Finger Protein)-Like (BORIS or Brother of the Regulator of Imprinted Sites), Squamous Cell Carcinoma Antigen Recognized By T Cells 3 (SART3); Paired box protein Pax-5 (PAX5); proacrosin binding protein sp32 (OY-TES1); lymphocyte-specific protein tyrosine kinase (LCK); A kinase anchor protein 4 (AKAP-4); synovial sarcoma, X breakpoint 2 (SSX2); Receptor for Advanced Glycation Endproducts (RAGE-1); renal ubiquitous 1 (RU1); renal ubiquitous 2 (RU2); legumain; human papilloma virus E6 (HPV E6); human papilloma virus E7 (HPV E7); intestinal carboxyl esterase; heat shock protein 70-2 mutated (mut hsp70-2); CD79a; CD79b; CD72; Leukocyte-associated immunoglobulin-like receptor 1 (LAIR1); Fc fragment of IgA receptor (FCAR or CD89); Leukocyte immunoglobulin-like receptor subfamily A member 2 (LILRA2); CD300 molecule-like family member f (CD300LF); C-type lectin domain family 12 member A (CLEC12A); bone marrow stromal cell antigen 2 (BST2); EGF-like module-containing mucin-like hormone receptor-like 2 (EMR2); lymphocyte antigen 75 (LY75); Glypican-3 (GPC3); Fc receptor-like 5 (FCRL5); and immunoglobulin lambda-like polypeptide 1 (IGLL1).

5. The immune effector cell of any one of claims 1-4, wherein:
(i) the transmembrane domain comprises a transmembrane domain of the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137, CD154, KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), CD160, CD19, IL2R beta, IL2R gamma, IL7R α, ITGA1, VLA1, CD49a, ITGA4, IA4, CD49D, ITGA6, VLA-6, CD49f, ITGAD, CD11d, ITGAE, CD103, ITGAL, CD11a, ITGAM, CD11b, ITGAX, CD11c, ITGB1, CD29, ITGB2, CD18, ITGB7, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO-3), BLAME (SLAMF8), SELPLG (CD 162), LTBR, PAG/Cbp, NKp44, NKp30, NKp46, NKG2D, or NKG2C;
(ii) the transmembrane domain comprises:
(a) an amino acid sequence having at least one, two or three modifications but not more than 20, 10 or 5 modifications of the amino acid sequence of SEQ ID NO: 12, or a sequence with 95-99% identity to the amino acid sequence of SEQ ID NO: 12; or
(b) the amino acid sequence of SEQ ID NO: 12;
(iii) the nucleic acid sequence encoding the transmembrane domain comprises the nucleotide sequence of SEQ ID NO: 13, or a sequence with 95-99% identity thereto;
(iv) the antigen binding domain is connected to the transmembrane domain by a hinge region, wherein said hinge region comprises the amino acid sequence of SEQ ID NO: 4 or SEQ ID NO: 6, or a sequence with 95-99% identity thereto; or said hinge region is encoded by the nucleotide sequence of SEQ ID NO: 5 or SEQ ID NO: 7, or a nucleotide sequence with 95-99% identity to SEQ ID NO: 7; and/or
(v) the CAR further comprises a leader sequence comprising SEQ ID NO:2.

6. The immune effector cell of any one of claims 1-5, wherein:
(i) the intracellular signaling domain comprises a primary signaling domain that comprises:
(a) a functional signaling domain of CD3 zeta, CD3 gamma, CD3 delta, CD3 epsilon, common FcR gamma (FCER1G), FcR beta (Fc Epsilon R1b), CD79a, CD79b, Fcgamma RIIa, DAP10, or DAP12; and/or
(b) an amino acid sequence having:
(1) at least one, two or three modifications but not more than 20, 10 or 5 modifications of the amino acid sequence of SEQ ID NO: 18 or SEQ ID NO: 20, or a sequence with 95-99% identity to the amino acid sequence of SEQ ID NO: 18 or SEQ ID NO: 20; or
(2) the amino acid sequence of SEQ ID NO: 18 or SEQ ID NO: 20; and/or
(ii) the intracellular signaling domain comprises a costimulatory signaling domain that comprises a functional signaling domain of CD27, CD28, 4-1BB (CD137), OX40, CD30, CD40, PD-1, ICOS, lymphocyte function-associated antigen-1 (LFA-1), CD2, CD7, LIGHT, NKG2C, B7-H3, a ligand that specifically binds with CD83, CDS, ICAM-1, GITR, BAFFR, HVEM (LIGHTR), SLAMF7, NKp80 (KLRF1), CD160, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, NKp44, NKp30, NKp46, or NKG2D.

7. The immune effector cell of any one of claims 1-6, wherein:
(a) the costimulatory signaling domain comprises:
(i) an amino acid sequence having at least one, two or three modifications but not more than 20, 10 or 5 modifications of the amino acid sequence of SEQ ID NO:14 or SEQ ID NO: 16, or a sequence with 95-99% identity to the amino acid sequence of SEQ ID NO:14 or SEQ ID NO: 16; or
(ii) the sequence of SEQ ID NO: 14 or SEQ ID NO: 16; or
(b) the nucleic acid sequence encoding the costimulatory signaling domain comprises the sequence of SEQ ID NO: 15 or SEQ ID NO: 17, or a sequence with 95-99% identity thereto.

8. The immune effector cell of any one of claims 1-7, wherein:
(i) the intracellular signaling domain comprises the sequence of SEQ ID NO: 14 or SEQ ID NO: 16, and the sequence of SEQ ID NO: 18 or SEQ ID NO: 20, wherein the sequences comprising the intracellular signaling domain are expressed in the same frame and as a single polypeptide chain; or
(ii) the nucleic acid sequence encoding the intracellular signaling domain comprises the sequence of SEQ ID NO: 15 or SEQ ID NO: 17, or a sequence with 95-99% identity thereto, and the sequence of SEQ ID NO:19 or SEQ ID NO:21, or a sequence with 95-99% identity thereto.

9. The immune effector cell of any one of claims 1-8, wherein:
(i) the second CAR molecule targets the same antigen as the first CAR molecule, and wherein the antigen binding domain of the second CAR molecule is different from the antigen binding domain of the first CAR molecule; or
(ii) the antigen binding domain of the second CAR molecule targets a different antigen than the antigen binding domain of the first CAR molecule, optionally wherein the antigen binding domain of the second CAR molecule targets a tumor antigen defined in claim 4.

10. The immune effector cell of any one of claims 1-9, wherein the first CAR molecule comprises an intracellular signaling domain comprising: a costimulatory signaling domain, but not a primary signaling domain; and wherein the second CAR molecule comprises an intracellular signaling domain comprising: a primary signaling domain, but not a costimulatory signaling domain; further wherein said antigen binding domain of the first CAR molecule binds to a tumor antigen selected from a group consisting of: TSHR, CD171, CS-1, CLL-1, GD3, Tn Ag, FLT3, CD38, CD44v6, B7H3, KIT, IL-13Ra2, IL-11Ra, PSCA, PRSS21, VEGFR2, LewisY, CD24, PDGFR-beta, SSEA-4, MUC1, EGFR, NCAM, CAIX, LMP2, EphA2, Fucosyl GM1, sLe, GM3, TGS5, HMWMAA, o-acetyl-GD2, Folate receptor beta, TEM1/CD248, TEM7R, CLDN6, GPRC5D, CXORF61, CD97, CD179a, ALK, Polysialic acid, PLAC1, GloboH, NY-BR-1, UPK2, HAVCR1, ADRB3, PANX3, GPR20, LY6K, OR51E2, TARP, WT1, ETV6-AML, sperm protein 17, XAGE1, Tie 2, MAD-CT-1, MAD-CT-2, Fos-related antigen 1, p53 mutant, hTERT, sarcoma translocation breakpoints, ML-IAP, ERG (TMPRSS2 ETS fusion gene), NA17, PAX3, Androgen receptor, Cyclin Bl, MYCN, RhoC, CYPlBl, BORIS, SART3, PAX5, OY-TES1, LCK, AKAP-4, SSX2, CD79a, CD79b, CD72, LAIR1, FCAR, LILRA2, CD300LF, CLEC12A, BST2, EMR2, LY75, GPC3, FCRL5, and IGLL1.

11. The immune effector cell of any one of claims 1-10, wherein:
(i) the first CAR molecule comprises a costimulatory signaling domain comprising a functional signaling domain of 4-1BB, CD28, CD27 or OX-40; and the second CAR molecule comprises a primary signaling domain comprising a functional signaling domain of CD3 zeta;
(ii) the second CAR molecule is an inhibitory CAR, wherein the inhibitory CAR comprises an antigen binding domain, a transmembrane domain, and an intracellular domain of an inhibitory molecule, wherein the inhibitory molecule is chosen from: PD1, PD-L1, CTLA4, TIM3, LAG3, VISTA, BTLA, TIGIT, LAIR1, CD160, 2B4, TGFR beta, CEACAM-1, CEACAM-3, or CEACAM-5;
(iii) the second CAR molecule further comprises an intracellular signaling domain comprising a primary signaling domain and/or an intracellular signaling domain, wherein the intracellular signaling domain comprises a primary signaling domain comprising the functional domain of CD3 zeta and a costimulatory signaling domain comprising the functional domain of 4-1BB; and/or
(iv) the second CAR molecule comprises the amino acid sequence of SEQ ID NO: 26.

12. The immune effector cell of any one of claims 1-11, wherein the immune effector cell is a human T cell (e.g., CD8+ T cell) or a human NK cell, optionally, wherein the T cell is diaglycerol kinase (DGK) and/or Ikaros deficient.

13. The immune effector cell of any one of claims 1-12, for use as a medicament.

14. The immune effector cell of any one of claims 1-12, for use in the treatment of a disease expressing a tumor antigen.

15. The immune effector cell for use of claim 14, wherein the disease is cancer, *e.g.* a hematologic cancer.

## Patentansprüche

1. Eine Immuneffektorzelle (z. B. eine Population von Immuneffektorzellen), die Folgendes beinhaltet:
(a) ein erstes chimäres Antigenrezeptor(CAR)-Molekül oder eine Nucleinsäure, die dafür kodiert, wobei das genannte erste CAR-Molekül ein CAR-Polypeptid ist, das eine Antigenbindungsdomäne, eine Transmembrandomäne und eine intrazelluläre Signalgebungsdomäne beinhaltet, die eine costimulatorische Domäne und/oder eine primäre Signalgebungsdomäne beinhaltet, und wobei die genannte Antigenbindungsdomäne eine VHH-Domäne beinhaltet und an ein Tumorantigen bindet; und
(b) ein zweites CAR-Molekül oder eine Nucleinsäure, die dafür kodiert, wobei das genannte zweite CAR-Molekül ein CAR-Polypeptid ist, die eine Antigenbindungsdomäne aufweist, die ein scFv beinhaltet.

2. Immuneffektorzelle nach Anspruch 1, wobei die Nucleinsäure, die für das erste CAR-Molekül kodiert, und die Nucleinsäure, die für das zweite CAR-Molekül kodiert, jeweils in einem Vektor vorhanden sind.

3. Immuneffektorzelle nach Anspruch 1 oder 2, wobei die VHH-Domäne eine Camelid-VHH-Domäne beinhaltet.

4. Immuneffektorzelle nach einem der Ansprüche 1-3, wobei das Tumorantigen aus einer Gruppe ausgewählt ist, die aus Folgenden besteht: CD19; Mesothelin; epidermalem Wachstumsfaktorrezeptor (EGFR); CD123; CD22; CD30; CD171; CS-1 (auch bezeichnet als CD2-Subset 1, CRACC, SLAMF7, CD319 und 19A24); C-Typ-lectinähnlichem Molekül-1 (CLL-1 oder CLECL1); CD33; epidermaler Wachstumsfaktorrezeptor-Variante III (EGFRvIII); Gangliosid G2 (GD2); Gangliosid GD3 (aNeu5Ac(2-8)aNeu5Ac(2-3)bDGalp(1-4)bDGlcp(1-1)Cer); TNF-Rezeptor-Familienmitglied B-Zellreifung (BCMA); Tn-Antigen ((Tn Ag) oder (GalNAcα-Ser/Thr)); prostataspezifischem Membranantigen (PSMA); Rezeptortyrosinkinase-ähnlichem Orphan-Rezeptor 1 (ROR1); Fms-ähnlicher Tyrosinkinase 3 (FLT3); tumorassoziiertem Glycoprotein 72 (TAG72); CD38; CD44v6; carcinoembryonischem Antigen (CEA); Epithelzell-Adhäsionsmolekül (EPCAM); B7H3 (CD276); KIT (CD117); Interleukin-13 receptor subunit alpha-2 (IL-13Ra2 oder CD213A2); Interleukin-11-Rezeptor-alpha (IL-11Ra); Prostatastammzellenantigen (PSCA); Proteaseserin 21 (Testisin oder PRSS21); vaskulärem Endothelwachstumsfaktorrezeptor 2 (VEGFR2); Lewis(Y)-Antigen; CD24; plättchenderiviertem Wachstumsfaktorrezeptor-beta (PDGFR-beta); stadienspezifischem embryonischem Antigen-4 (SSEA-4); CD20; Folatrezeptor-alpha; Rezeptortyrosinproteinkinase ERBB2 (Her2/neu); Mucin 1, zelloberflächenassoziiert (MUC1); neuralem Zelladhäsionsmolekül (NCAM); Prostase; Prostatasäurephosphatase (PAP); Elongation-Faktor-2 mutiert (ELF2M); Ephrin B2; Fibroblastenaktivierungsprotein-alpha (FAP); insulinähnlichem Wachstumsfaktor-1-Rezeptor (IGF-I-Rezeptor), Carboanhydrase IX (CAIX); Proteasom (Prosom, Macropain)-Untereinheit, Beta-Typ, 9 (LMP2); Glycoprotein 100 (gp100); Onkogenfusionsprotein, bestehend aus Breakpoint-Clusterregion (BCR) und Abelson-Maus-Leukämievirus-Onkogen-Homolog 1 (Abl) (bcr-abl); Tyrosinase; Ephrin-Typ-A-Rezeptor 2 (EphA2); Fucosyl GM1; Sialyl-Lewis-Adhäsionsmolekül (sLe); Gangliosid GM3 (aNeu5Ac(2-3)bDGalp(1-4)bDGlcp(1-1)Cer); Transglutaminase 5 (TGS5); High Molecular Weight Melanoma-Associated Antigen (HMWMAA); o-Acetyl-GD2-Gangliosid (OAcGD2); Folatrezeptorbeta; Tumorendothel-Marker 1 (TEM1/CD248); Tumorendothel-Marker-7-related (TEM7R); Claudin 6 (CLDN6); schilddrüsenstimulierendem Hormonrezeptor (TSHR); G-Protein-gekoppeltem Rezeptor Klasse C Gruppe 5, Mitglied D (GPRC5D); Chromosom-X-offenes Leseraster 61 (CXORF61); CD97; CD179a; anaplastischer Lymphomkinase (ALK); Polysialsäure; plazentaspezifisch 1 (PLAC1); Hexasaccharidanteil von globoH-Glykoceramid (GloboH); Brustdrüsen-Differenzierungsantigen (NY-BR-1); Uroplakin 2 (UPK2); Hepatitis-A-Virus-zellulärem Rezeptor 1 (HAVCR1); Adrenozeptor-beta 3 (ADRB3); Pannexin 3 (PANX3); G-Proteingekoppeltem Rezeptor 20 (GPR20); Lymphozytenantigen-6-Komplex, Locus K 9 (LY6K); Olfaktorischem Rezeptor 51E2 (OR51E2); TCR-Gamma-Alternate Reading Frame Protein (TARP); Wilmstumor-Protein (WT1); Cancer/Testis-Antigen 1 (NY-ESO-1); Cancer/Testis-Antigen 2 (LAGE-1a); Melanom-assoziiertem Antigen 1 (MAGE-A1); ETS-Translokations-variantem Gen 6, lokalisiert auf Chromosom 12p (ETV6-AML); Sperm-Protein 17 (SPA17); X-Antigen-Familie, Mitglied 1A (XAGE1); Angiopoietinbindungs-Zelloberflächenrezeptor 2 (Tie 2); Melanom-Cancer-Testis-Antigen-1 (MAD-CT-1); Melanom-Cancer-Testis-Antigen-2 (MAD-CT-2); Fos-bezogenem Antigen 1; Tumorprotein p53 (p53); p53-Mutant; Prostein; Survivin; Telomerase; Prostatakarzinom-Tumorantigen-1 (PCTA-1 oder Galectin 8), Melanom-Antigen, erkannt durch T-Zellen 1 (MelanA oder MART1); Rattensarkom(Ras)-Mutant; humaner Telomerase-Reverse-Transkriptase (hTERT); Sarkom-Translokations-Breakpoints; Melanom-Apoptose-Inhibitor (ML-IAP); ERG (Transmembranprotease, Serin 2 (TMPRSS2) ETS-Fusionsgen); N-Acetylglucosaminyltransferase V (NA17); Paired-Box-Protein Pax-3 (PAX3); Androgenrezeptor; Cyclin B1; v-myc avian myelocytomatosis viral oncogene neuroblastoma derived homolog (MYCN); Ras-Homolog-Familienmitglied C (RhoC); Tyrosinase-bezogenem Protein 2 (TRP-2); Cytochrom P450 1B1 (CYP1B1); CCCTC-Bindungsfaktor (Zinkfinger-Protein)-ähnlich (BORIS oder Brother of the Regulator of Imprinted Sites), Plattenepithelkarzinom-Antigen, erkannt durch T-Zellen 3 (SART3); Paired-Box-Protein Pax-5 (PAX5); Proacrosin-Bindungsprotein sp32 (OY-TES1); Lymphozytenspezifischer Protein-Tyrosinkinase (LCK); A-Kinase-Ankerprotein 4 (AKAP-4); Synovialsarkom, X Breakpoint 2 (SSX2); Receptor for Advanced Glycation Endproducts (RAGE-1); Renal Ubiquitous 1 (RU1); Renal Ubiquitous 2 (RU2); Legumain; Humanem Papillomavirus E6 (HPV E6); Humanem Papillomavirus E7 (HPV E7); Intestinaler Carboxylesterase; Heat-Shock-Protein 70-2 mutiert (mut hsp70-2); CD79a; CD79b; CD72; Leukozytenassoziiertem Immunglobulin-ähnlichem Rezeptor 1 (LAIR1); Fc-Fragment von IgA-Rezeptor (FCAR oder CD89); Leukozyten-Immunoglobulin-ähnlichem Rezeptor, Unterfamilie A Mitglied 2 (LILRA2); CD300-Molekül-ähnlicher Familie, Mitglied F (CD300LF); C-Typ-Lectin-Domäne, Familie 12, Mitglied A (CLEC12A); Knochenmark-Stromazell-Antigen 2 (BST2); EGF-like module-containing mucin-like hormone receptor-like 2 (EMR2); Lymphozytenantigen 75 (LY75); Glypican-3 (GPC3); Fc-Rezeptor-ähnlich 5 (FCRL5); und Immunglobulin-Lambda-ähnlichem Polypeptid 1 (IGLL1).

5. Immuneffektorzelle nach einem der Ansprüche 1-4, wobei:
(i) die Transmembrandomäne Folgendes beinhaltet: eine Transmembrandomäne der Alpha-, Beta- oder Zeta-Kette des T-Zell-Rezeptors, CD28, CD3-epsilon, CD45, CD4, CD5, CD8, CD9, CD16, CD22, CD33, CD37, CD64, CD80, CD86, CD134, CD137, CD154, KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), CD160, CD19, IL2R-beta, IL2R-gamma, IL7R α, ITGA1, VLA1, CD49a, ITGA4, IA4, CD49D, ITGA6, VLA-6, CD49f, ITGAD, CD11d, ITGAE, CD103, ITGAL, CD11a, ITGAM, CD11b, ITGAX, CD11c, ITGB1, CD29, ITGB2, CD18, ITGB7, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO-3), BLAME (SLAMF8), SELPLG (CD162), LTBR, PAG/Cbp, NKp44, NKp30, NKp46, NKG2D oder NKG2C;
(ii) die Transmembrandomäne Folgendes beinhaltet:
(a) eine Aminosäuresequenz mit mindestens einer, zwei oder drei Modifikationen, aber nicht mehr als 20, 10 oder 5 Modifikationen der Aminosäuresequenz von SEQ ID NO: 12, oder eine Sequenz mit 95- bis 99%iger Identität mit der Aminosäuresequenz von SEQ ID NO: 12; oder
(b) die Aminosäuresequenz von SEQ ID NO: 12;
(iii) die Nucleinsäuresequenz, die für die Transmembrandomäne kodiert, die Nucleotidsequenz von SEQ ID NO: 13 oder eine Sequenz mit 95- bis 99%iger Identität dazu beinhaltet;
(iv) die Antigenbindungsdomäne durch eine Gelenkregion mit der Transmembrandomäne verbunden ist, wobei die genannte Gelenkregion die Aminosäuresequenz von SEQ ID NO: 4 oder SEQ ID NO: 6 oder eine Sequenz mit 95-bis 99%iger Identität dazu beinhaltet; oder die genannte Gelenkregion durch die Nucleotidsequenz von SEQ ID NO: 5 oder SEQ ID NO: 7 oder eine Nucleotidsequenz mit 95- bis 99%iger Identität mit SEQ ID NO: 7 kodiert wird; und/oder
(v) das CAR ferner eine Leadersequenz beinhaltet, die SEQ ID NO: 2 beinhaltet.

6. Immuneffektorzelle nach einem der Ansprüche 1-5, wobei:
(i) die intrazelluläre Signalgebungsdomäne eine primäre Signalgebungsdomäne beinhaltet, die Folgendes beinhaltet:
(a) eine funktionelle Signalgebungsdomäne von CD3-zeta, CD3-gamma, CD3-delta, CD3-epsilon, gemeinsamem FcR-gamma (FCER1G), FcR-beta (Fc Epsilon R1b), CD79a, CD79b, Fcgamma RIIa, DAP10 oder DAP12; und/oder
(b) eine Aminosäuresequenz, die Folgendes aufweist:
(1) mindestens eine, zwei oder drei Modifikationen, aber nicht mehr als 20, 10 oder 5 Modifikationen der Aminosäuresequenz von SEQ ID NO: 18 oder SEQ ID NO: 20 oder eine Sequenz mit 95- bis 99%iger Identität mit der Aminosäuresequenz von SEQ ID NO: 18 oder SEQ ID NO: 20; oder
(2) die Aminosäuresequenz von SEQ ID NO: 18 oder SEQ ID NO: 20; und/oder
(ii) die intrazelluläre Signalgebungsdomäne eine costimulatorische Signalgebungsdomäne beinhaltet, die eine funktionelle Signalgebungsdomäne von CD27, CD28, 4-1BB (CD137), OX40, CD30, CD40, PD-1, ICOS, lymphozytenfunktionsassoziiertem Antigen-1 (LFA-1), CD2, CD7, LIGHT, NKG2C, B7-H3, einem Liganden, der spezifisch mit CD83 bindet, CDS, ICAM-1, GITR, BAFFR, HVEM (LIGHTR), SLAMF7, NKp80 (KLRF1), CD160, CD19, CD4, CD8alpha, CD8beta, IL2R-beta, IL2R-gamma, IL7R-alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, NKp44, NKp30, NKp46 oder NKG2D beinhaltet.

7. Immuneffektorzelle nach einem der Ansprüche 1-6, wobei:
(a) die costimulatorische Signalgebungsdomäne Folgendes beinhaltet:
(i) eine Aminosäuresequenz mit mindestens einer, zwei oder drei Modifikationen, aber nicht mehr als 20, 10 oder 5 Modifikationen der Aminosäuresequenz von SEQ ID NO: 14 oder SEQ ID NO: 16 oder eine Sequenz mit 95- bis 99%iger Identität mit der Aminosäuresequenz von SEQ ID NO: 14 oder SEQ ID NO: 16; oder
(ii) die Sequenz von SEQ ID NO: 14 oder SEQ ID NO: 16; oder
(b) die Nucleinsäuresequenz, die für die costimulatorische Signalgebungsdomäne kodiert, die Sequenz von SEQ ID NO: 15 oder SEQ ID NO: 17 oder eine Sequenz mit 95- bis 99%iger Identität dazu beinhaltet.

8. Immuneffektorzelle nach einem der Ansprüche 1-7, wobei:
(i) die intrazelluläre Signalgebungsdomäne die Sequenz von SEQ ID NO: 14 oder SEQ ID NO: 16 und die Sequenz von SEQ ID NO: 18 oder SEQ ID NO: 20 beinhaltet, wobei die Sequenzen, die die intrazelluläre Signalgebungsdomäne beinhalten, in dem gleichen Raster und als eine einzelne Polypeptidkette exprimiert werden; oder
(ii) die Nucleinsäuresequenz, die für die intrazelluläre Signalgebungsdomäne kodiert, die Sequenz von SEQ ID NO: 15 oder SEQ ID NO: 17 oder eine Sequenz mit 95-bis 99%iger Identität dazu und die Sequenz von SEQ ID NO: 19 oder SEQ ID NO: 21 oder eine Sequenz mit 95- bis 99%iger Identität dazu beinhaltet.

9. Immuneffektorzelle nach einem der Ansprüche 1-8, wobei:
(i) das zweite CAR-Molekül das gleiche Antigen targetiert wie das erste CAR-Molekül und wobei sich die Antigenbindungsdomäne des zweiten CAR-Moleküls von der Antigenbindungsdomäne des ersten CAR-Moleküls unterscheidet; oder
(ii) die Antigenbindungsdomäne des zweiten CAR-Moleküls ein anderes Antigen targetiert als die Antigenbindungsdomäne des ersten CAR-Moleküls, optional wobei die Antigenbindungsdomäne des zweiten CAR-Moleküls ein Tumorantigen targetiert, das in Anspruch 4 definiert ist.

10. Immuneffektorzelle nach einem der Ansprüche 1-9, wobei das erste CAR-Molekül eine intrazelluläre Signalgebungsdomäne beinhaltet, die Folgendes beinhaltet: eine costimulatorische Signalgebungsdomäne, aber keine primäre Signalgebungsdomäne; und wobei das zweite CAR-Molekül eine intrazelluläre Signalgebungsdomäne beinhaltet, die Folgendes beinhaltet: eine primäre Signalgebungsdomäne, aber keine costimulatorische Signalgebungsdomäne; wobei die genannte Antigenbindungsdomäne des ersten CAR-Moleküls ferner an ein Tumorantigen bindet, das aus einer Gruppe ausgewählt ist, die aus Folgenden besteht: TSHR, CD171, CS-1, CLL-1, GD3, Tn Ag, FLT3, CD38, CD44v6, B7H3, KIT, IL-13Ra2, IL-IIRa, PSCA, PRSS21, VEGFR2, LewisY, CD24, PDGFR-beta, SSEA-4, MUC1, EGFR, NCAM, CAIX, LMP2, EphA2, Fucosyl GM1, sLe, GM3, TGS5, HMWMAA, o-Acetyl-GD2, Folatrezeptorbeta, TEM1/CD248, TEM7R, CLDN6, GPRC5D, CXORF61, CD97, CD179a, ALK, Polysialsäure, PLAC1, GloboH, NY-BR-1, UPK2, HAVCR1, ADRB3, PANX3, GPR20, LY6K, OR51E2, TARP, WT1, ETV6-AML, Sperm-Protein 17, XAGE1, Tie 2, MAD-CT-1, MAD-CT-2, Fos-bezogenem Antigen 1, p53-Mutant, hTERT, Sarkom-Translokations-Breakpoints, ML-IAP, ERG (TMPRSS2 ETS-Fusionsgen), NA17, PAX3, Androgenrezeptor, Cyclin B1, MYCN, RhoC, CYP1B1, BORIS, SART3, PAX5, OY-TES1, LCK, AKAP-4, SSX2, CD79a, CD79b, CD72, LAIR1, FCAR, LILRA2, CD300LF, CLEC12A, BST2, EMR2, LY75, GPC3, FCRL5 und IGLL1.

11. Immuneffektorzelle nach einem der Ansprüche 1-10, wobei:
(i) das erste CAR-Molekül eine costimulatorische Signalgebungsdomäne beinhaltet, die eine funktionelle Signalgebungsdomäne von 4-IBB, CD28, CD27 oder OX-40 beinhaltet; und das zweite CAR-Molekül eine primäre Signalgebungsdomäne beinhaltet, die eine funktionelle Signalgebungsdomäne von CD3-zeta beinhaltet;
(ii) das zweite CAR-Molekül ein inhibitorischer CAR ist, wobei der inhibitorische CAR eine Antigenbindungsdomäne, eine Transmembrandomäne und eine intrazelluläre Domäne eines inhibitorischen Moleküls beinhaltet, wobei das inhibitorische Molekül aus Folgenden ausgewählt ist: PD1, PD-L1, CTLA4, TIM3, LAG3, VISTA, BTLA, TIGIT, LAIR1, CD160, 2B4, TGFR-beta, CEACAM-1, CEACAM-3 oder CEACAM-5;
(iii) das zweite CAR-Molekül ferner Folgendes beinhaltet: eine intrazelluläre Signalgebungsdomäne, die eine primäre Signalgebungsdomäne und/oder eine intrazelluläre Signalgebungsdomäne beinhaltet, wobei die intrazelluläre Signalgebungsdomäne eine primäre Signalgebungsdomäne, die die funktionelle Domäne von CD3-zeta beinhaltet, und eine costimulatorische Signalgebungsdomäne, die die funktionelle Domäne von 4-1BB beinhaltet, beinhaltet; und/oder
(iv) das zweite CAR-Molekül die Aminosäuresequenz von SEQ ID NO: 26 beinhaltet.

12. Immuneffektorzelle nach einem der Ansprüche 1-11, wobei die Immuneffektorzelle eine humane T-Zelle (z. B. CD8+-T-Zelle) oder eine humane NK-Zelle ist, optional wobei die T-Zelle diaglycerolkinase(DGK)- und/oder Ikarosdefizient ist.

13. Immuneffektorzelle nach einem der Ansprüche 1-12 zur Verwendung als ein Medikament.

14. Immuneffektorzelle nach einem der Ansprüche 1-12 zur Verwendung bei der Behandlung einer Erkrankung, die ein Tumorantigen exprimiert.

15. Immuneffektorzelle zur Verwendung nach Anspruch 14, wobei die Erkrankung Krebs, z. B. ein Blutkrebs ist.

## Revendications

1. Cellule effectrice immunitaire (par exemple une population de cellules effectrices immunitaires), comprenant:
(a) une première molécule récepteur antigénique chimérique (CAR) ou un acide nucléique codant celle-ci, ladite première molécule CAR étant un polypeptide CAR comprenant un domaine de liaison à un antigène, un domaine transmembranaire, et un domaine de signalisation intracellulaire comprenant un domaine de costimulation et/ou un domaine de signalisation primaire, et ledit domaine de liaison à un antigène comprenant un domaine VHH et se liant à un antigène tumoral; et
(b) une deuxième molécule CAR ou un acide nucléique codant celle-ci, ladite deuxième molécule CAR étant un polypeptide CAR comportant un domaine de liaison à un antigène comprenant un scFv.

2. Cellule effectrice immunitaire selon la revendication 1, dans laquelle l'acide nucléique codant la première molécule CAR et l'acide nucléique codant la deuxième molécule CAR sont chacun présent dans un vecteur.

3. Cellule effectrice immunitaire selon la revendication 1 ou 2, dans laquelle le domaine VHH est un domaine VHH de camélidé.

4. Cellule effectrice immunitaire selon l'une quelconque des revendications 1 à 3, dans laquelle l'antigène tumoral est sélectionné dans un groupe constitué de: CD19; mésothéline; récepteur du facteur de croissance épidermique (EGFR); CD123; CD22; CD30; CD171; CS-1 (également appelé sous-ensemble CD2 1, CRACC, SLAMF7, CD319 et 19A24); molécule 1 lectine-like de type C (CLL-1 ou CLECL1); CD33; variante III du récepteur du facteur de croissance épidermique (EGFRvIII); ganglioside G2 (GD2); ganglioside GD3 (aNeu5Ac(2-8)aNeu5Ac(2-3)bDGalp(1-4)bDGlcp(1-1)Cer); antigène de maturation des cellules B (BCMA) membre de la famille des récepteurs du TNF; antigène Tn ((Tn Ag) ou (GalNAcα-Ser/Thr)); antigène membranaire spécifique de la prostate (PSMA); récepteur orphelin-1 de type récepteur à tyrosine kinase (ROR1); tyrosine kinase-3 de type fms (FLT3); glycoprotéine 72 associée à une tumeur (TAG72); CD38; CD44v6; antigène carcinoembryonnaire (CEA); molécule d'adhérence des cellules épithéliales (EPCAM); B7H3 (CD276); KIT (CD117); sous-unité alpha-2 du récepteur à l'interleukine-13 (IL-13Ra2 ou CD213A2); sous-unité alpha du récepteur à l'interleukine-11 (IL-IIRa); antigène des cellules souches prostatiques (PSCA); sérine protéase 21 (testisine ou PRSS21); récepteur 2 au facteur de croissance endothéliale vasculaire (VEGFR2); antigène Lewis (Y); CD24; récepteur bêta au facteur de croissance dérivé des plaquettes (PDGFR-bêta); antigène embryonnaire 4 spécifique au stade (SSEA-4); CD20; récepteur alpha des folates; récepteur à activité tyrosine kinase ERBB2 (Her2/neu); mucine 1, associée à la surface cellulaire (MUC1); molécule d'adhérence cellulaire neurale (NCAM); prostase; phosphatase acide prostatique (PAP); facteur d'élongation 2 muté (ELF2M); éphrine B2; protéine alpha d'activation des fibroblastes (FAP); récepteur du facteur de croissance 1 analogue à l'insuline (récepteur IGF-I), anhydrase carbonique IX (CAIX); sous-unité bêta de protéasome (Prosome, Macropain), type 9 (LMP2); Glycoprotéine 100 (gp100); protéine de fusion oncogène constituée du gène breakpoint cluster region (BCR) et de l'homologue 1 de l'oncogène viral de la leucémie murine d'Abelson (Abl) (bcr-abl); tyrosinase; récepteur 2 à l'éphrine de type A (EphA2); fucosyle GM1; molécule d'adhérence des sialyl Lewis (sLe); ganglioside GM3 (aNeu5Ac(2-3)bDGalp(1-4)bDGlcp(1-1)Cer); transglutaminase 5 (TGS5); antigène de haut poids moléculaire associé au mélanome (HMWMAA); ganglioside o-acétyl-GD2 (OAcGD2); récepteur bêta des folates; marqueur 1 de cellules endothéliales de tumeurs (TEM1/CD248); protéine apparentée au marqueur 7 de cellules endothéliales de tumeurs (TEM7R); claudine 6 (CLDN6); récepteur de la thyréostimuline (TSHR); récepteur couplé à une protéine G, classe C, groupe 5, membre D (GPRC5D); cadre de lecture ouvert 61 du chromosome X (CXORF61); CD97; CD179a; kinase du lymphome anaplasique (ALK); acide polysialique; protéine spécifique du placenta 1 (PLAC1); portion hexasaccharide du glycocéramide globoH (GloboH); antigène de différenciation des glandes mammaires (NY-BR-1); uroplakine 2 (UPK2); récepteur cellulaire 1 du virus de l'hépatite A (HAVCR1); récepteur adrénergique bêta 3 (ADRB3); pannexine 3 (PANX3); récepteur 20 couplé à une protéine G (GPR20); complexe 6 d'antigène lymphocytaire, locus K 9 (Y6K); récepteur olfactif 51E2 (OR51E2); protéine du cadre de lecture alternatif de la chaîne gamma du récepteur des cellules T (TARP); protéine tumorale de Wilms (WT1); antigène 1 du cancer du testicule (NY-ESO-1); antigène 2 du cancer du testicule (LAGE-1a); antigène 1 associé au mélanome (MAGE-A1); variant produit par translocation du gène ETV6 de la famille ETS, situé en position p sur le chromosome 12 (ETV6-AML); protéine 17 du sperme (SPA17); famille d'antigènes X, membre 1A (XAGE1); récepteur de surface cellulaire 2 se liant à l'angiopoiétine (Tie 2); antigène du cancer du testicule dérivé du mélanome 1 (MAD-CT-1); antigène du cancer du testicule dérivé du mélanome 2 (MAD-CT-2); antigène 1 lié à Fos; protéine tumorale p53 (p53); mutant p53; prostéine; survivine; télomérase; antigène tumoral 1 du carcinome de la prostate (PCTA-1 ou Galectin 8), antigène du mélanome reconnu par les cellules T 1 (MelanA ou MART1); mutant du sarcome du rat (Ras); télomérase reverse transcriptase humaine (hTERT); points de cassure de translocation du sarcome; inhibiteur de l'apoptose du mélanome (ML-IAP); ERG (protéase transmembranaire, sérine 2 (TMPMRSS2), gène de fusion de la famille ETS); n-acétylglucosaminyltransférase V (NA17); protéine paired box Pax-3 (PAX3); récepteur d'androgènes; cycline B1; v-myc, homologue dérivé de neuroblastome de l'oncogène viral de la myélocytomatose aviaire (MYCN); membre C de la famille Rho (Ras Homologous) (RhoC); protéine 2 apparentée à la tyrosinase (TRP-2); cytochrome P450 1B1 (CYP1B1); facteur de liaison à la séquence CCCTC, de type protéines à doigts de zinc (BORIS ou Brother of the Regulator of Imprinted Sites), antigène 3 de carcinome épidermoïde reconnu par les cellules T (SART3); protéine paired box Pax-5 (PAX5); protéine sp32 se liant à la proacrosine (OY-TES1); protéine spécifique des lymphocytes tyrosine kinase (LCK); protéine d'ancrage kinase 4 (AKAP-4); gène X breakpoint 2 du sarcome synovial (SSX2); récepteur pour les produits terminaux de glycation avancée (RAGE-1); renal ubiquitous 1 (RU1); renal ubiquitous 2 (RU2); legumain; virus du papillome humain E6 (HPV E6); virus du papillome humain E7 (HPV E7); carboxylestérase intestinale; protéine de choc thermique 70-2 mutée (mut hsp70-2); CD79a; CD79b; CD72; récepteur de type immunoglobuline 1 associé aux leucocytes (LAIR1); fragment Fc du récepteur d'lgA (FCAR ou CD89); membre 2 de la sous-famille A de récepteurs de type immunoglobuline associés aux leucocytes (LILRA2); membre f de la famille de gènes de type molécule CD300 (CD300LF); domaine de lectine de type C, famille 12, membre A (CLEC12A); antigène stromal 2 de la moelle osseuse (BST2); récepteur de type récepteur hormonal 2 mucine-like contenant un module de type EGF (EMR2); antigène lymphocytaire 75 (LY75); glypicane-3 (GPC3); protéine 5 de type récepteur fc (FCRL5); et polypeptide 1 de type immunoglobuline lambda (IGLL1).

5. Cellule effectrice immunitaire selon l'une quelconque des revendications 1 à 4, dans laquelle:
(i) le domaine transmembranaire comprend un domaine transmembranaire de la chaîne alpha, bêta ou zêta du récepteur des cellules T, CD28, CD3 epsilon, CD45, CD4, CD5, CD8, CD9, CD16, CD22, CD33, CD37, CD64, CD80, CD86, CD134, CD137, CD154, KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), CD160, CD19, IL2R bêta, IL2R gamma, IL7R α, ITGA1, VLA1, CD49a, ITGA4, IA4, CD49D, ITGA6, VLA-6, CD49f, ITGAD, CD11d, ITGAE, CD103, ITGAL, CD11a, ITGAM, CD11b, ITGAX, CD11c, ITGB1, CD29, ITGB2, CD18, ITGB7, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO-3), BLAME (SLAMF8), SELPLG (CD162), LTBR, PAG/Cbp, NKp44, NKp30, NKp46, NKG2D, ou NKG2C;
(ii) le domaine transmembranaire comprend:
(a) une séquence d'acides aminés comportant au moins une, deux ou trois modifications, mais pas plus de 20, 10 ou 5 modifications de la séquence d'acides aminés de la SÉQ. ID no 12, ou d'une séquence ayant une identité de 95 à 99% avec la séquence d'acides aminés de la SÉQ. ID no 12; ou
(b) la séquence d'acides aminés de la SÉQ. ID n° 12;
(iii) la séquence d'acide nucléique codant le domaine transmembranaire comprend la séquence nucléotidique de la SÉQ. ID n° 13, ou une séquence ayant une identité de 95 à 99% avec celle-ci;
(iv) le domaine de liaison à un antigène est connecté au domaine transmembranaire par une région charnière, ladite région charnière comprenant la séquence d'acides aminés de la SÉQ. ID n° 4 ou de la SÉQ. ID n° 6, ou une séquence ayant une identité de 95 à 99% avec celles-ci; ou ladite région charnière est codée par la séquence nucléotidique de la SÉQ. ID n° 5 ou de la SÉQ. ID n° 7, ou une séquence nucléotidique ayant une identité de 95 à 99% avec la SÉQ. ID n° 7; et/ou
(v) le CAR comprend en outre une séquence de tête comprenant la SÉQ. ID n° 2.

6. Cellule effectrice immunitaire selon l'une quelconque des revendications 1 à 5, dans laquelle:
(i) le domaine de signalisation intracellulaire comprend un domaine de signalisation primaire qui comprend:
(a) un domaine de signalisation fonctionnel de CD3 zêta, CD3 gamma, CD3 delta, CD3 epsilon, FcR gamma commun (FCER1G), FcR bêta (Fc Epsilon R1b), CD79a, CD79b, Fc gamma RIIa, DAP10, ou DAP12; et/ou
(b) une séquence d'acides aminés comportant:
(1) au moins une, deux ou trois modifications, mais pas plus de 20, 10 ou 5 modifications de la séquence d'acides aminés de la SÉQ. ID no 18 ou de la SÉQ. ID no 20, ou d'une séquence ayant une identité de 95 à 99% avec la séquence d'acides aminés de la SÉQ. ID no 18 ou de la SÉQ. ID no 20; ou
(2) la séquence d'acides aminés de la SÉQ. ID no 18 ou de la SÉQ. ID no 20; et/ou
(ii) le domaine de signalisation intracellulaire comprend un domaine de signalisation de costimulation qui comprend un domaine de signalisation fonctionnel de CD27, CD28, 4-1BB (CD137), OX40, CD30, CD40, PD-1, ICOS, antigène-1 associé à la fonction lymphocytaire (LFA-1), CD2, CD7, LIGHT, NKG2C, B7-H3, un ligand qui se lie spécifiquement à CD83, CDS, ICAM-1, GITR, BAFFR, HVEM (LIGHTR), SLAMF7, NKp80 (KLRF1), CD160, CD19, CD4, CD8 alpha, CD8 bêta, IL2R bêta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, NKp44, NKp30, NKp46, ou NKG2D.

7. Cellule effectrice immunitaire selon l'une quelconque des revendications 1 à 6, dans laquelle:
(a) le domaine de signalisation de costimulation comprend:
(i) une séquence d'acides aminés comportant au moins une, deux ou trois modifications, mais pas plus de 20, 10 ou 5 modifications de la séquence d'acides aminés de la SÉQ. ID n° 14 ou de la SÉQ. ID n° 16, ou d'une séquence ayant une identité de 95 à 99% avec la séquence d'acides aminés de la SÉQ. ID n° 14 ou de la SÉQ. ID n° 16; ou
(ii) la séquence de la SÉQ. ID n° 14 ou de la SÉQ. ID n° 16; ou
(b) la séquence d'acide nucléique codant le domaine de signalisation de costimulation comprend la SÉQ. ID n° 15 ou la SÉQ. ID n° 17, ou une séquence ayant une identité de 95 à 99% avec celles-ci.

8. Cellule effectrice immunitaire selon l'une quelconque des revendications 1 à 7, dans laquelle:
(i) le domaine de signalisation intracellulaire comprend la séquence de la SÉQ. ID n° 14 ou de la SÉQ. ID n° 16, et la séquence de la SÉQ. ID n° 18 ou de la SÉQ. ID n° 20, les séquences comprenant le domaine de signalisation intracellulaire étant exprimées dans le même cadre et sous la forme d'une seule chaîne polypeptidique; ou
(ii) la séquence d'acide nucléique codant le domaine de signalisation intracellulaire comprend la séquence de la SÉQ. ID n° 15 ou de la SÉQ. ID n° 17, ou une séquence ayant une identité de 95 à 99% avec celles-ci, et la séquence de la SÉQ. ID n° 19 ou de la SÉQ. ID n° 21, ou une séquence ayant une identité de 95 à 99% avec celles-ci.

9. Cellule effectrice immunitaire selon l'une quelconque des revendications 1 à 8, dans laquelle:
(i) la deuxième molécule CAR cible le même antigène que la première molécule CAR, et dans laquelle le domaine de liaison à un antigène de la deuxième molécule CAR est différent du domaine de liaison à un antigène de la première molécule CAR; ou
(ii) le domaine de liaison à un antigène de la deuxième molécule CAR cible un antigène différent du domaine de liaison à un antigène de la première molécule CAR, optionnellement dans lequel le domaine de liaison à un antigène de la deuxième molécule CAR cible un antigène tumoral défini dans la revendication 4.

10. Cellule effectrice immunitaire selon l'une quelconque des revendications 1 à 9, dans laquelle la première molécule CAR comprend un domaine de signalisation intracellulaire comprenant: un domaine de signalisation de costimulation, mais pas un domaine de signalisation primaire; et dans laquelle la deuxième molécule CAR comprend un domaine de signalisation intracellulaire comprenant: un domaine de signalisation primaire, mais pas un domaine de signalisation de costimulation; en outre dans laquelle ledit domaine de liaison à un antigène de la première molécule CAR se lie à un antigène tumoral sélectionné dans un groupe constitué de: TSHR, CD171, CS-1, CLL-1, GD3, Tn Ag, FLT3, CD38, CD44v6, B7H3, KIT, IL-13Ra2, IL-11Ra, PSCA, PRSS21, VEGFR2, LewisY, CD24, PDGFRbêta, SSEA-4, MUC1, EGFR, NCAM, CAIX, LMP2, EphA2, Fucosyl GM1, sLe, GM3, TGS5, HMWMAA, o-acétyl-GD2, récepteur bêta des folates, TEM1/CD248, TEM7R, CLDN6, GPRC5D, CXORF61, CD97, CD179a, ALK, acide polysialique, PLAC1, GloboH, NY-BR-1, UPK2, HAVCR1, ADRB3, PANX3, GPR20, LY6K, OR51E2, TARP, WT1, ETV6-AML, protéine 17 du sperme, XAGE1, Tie 2, MAD-CT-1, MAD-CT-2, antigène 1 lié à Fos, mutant p53, hTERT, points de cassure de translocation du sarcome, ML-IAP, ERG (TMPMRSS2, gène de fusion de la famille ETS), NA17, PAX3, récepteur d'androgènes, cycline B1, MYCN, RhoC, CYP1B1, BORIS, SART3, PAX5, OY-TES1, LCK, AKAP-4, SSX2, CD79a, CD79b, CD72, LAIR1, FCAR, LILRA2, CD300LF, CLEC12A, BST2, EMR2, LY75, GPC3, FCRL5, et IGLL1.

11. Cellule effectrice immunitaire selon l'une quelconque des revendications 1 à 10, dans laquelle:
(i) la première molécule CAR comprend un domaine de signalisation de costimulation comprenant un domaine de signalisation fonctionnel de 4-1BB, CD28, CD27 ou OX-40; et la deuxième molécule CAR comprend un domaine de signalisation primaire comprenant un domaine de signalisation fonctionnel de CD3 zêta;
(ii) la deuxième molécule CAR est un CAR inhibiteur, le CAR inhibiteur comprenant un domaine de liaison à un antigène, un domaine transmembranaire, et un domaine intracellulaire d'une molécule inhibitrice, la molécule inhibitrice étant sélectionnée parmi: PD1, PD-L1, CTLA4, TIM3, LAG3, VISTA, BTLA, TIGIT, LAIR1, CD160, 2B4, TGFR bêta, CEACAM-1, CEACAM-3, ou CEACAM-5;
(iii) la deuxième molécule CAR comprend en outre un domaine de signalisation intracellulaire comprenant un domaine de signalisation primaire et/ou un domaine de signalisation intracellulaire, le domaine de signalisation intracellulaire comprenant un domaine de signalisation primaire comprenant le domaine fonctionnel de CD3 zêta et un domaine de signalisation de costimulation comprenant le domaine fonctionnel de 4-1BB; et/ou
(iv) la deuxième molécule CAR comprend la séquence d'acides aminés de la SÉQ. ID n° 26.

12. Cellule effectrice immunitaire selon l'une quelconque des revendications 1 à 11, la cellule effectrice immunitaire étant une cellule T humaine (par exemple une cellule T CD8+) ou une cellule NK humaine, la cellule T étant optionnellement déficiente en diacylglycérol kinase (DGK) et/ou en Ikaros.

13. Cellule effectrice immunitaire selon l'une quelconque des revendications 1 à 12, destinée à une utilisation comme médicament.

14. Cellule effectrice immunitaire selon l'une quelconque des revendications 1 à 12, destinée à une utilisation dans le traitement d'une maladie exprimant un antigène tumoral.

15. Cellule effectrice immunitaire destinée à une utilisation selon la revendication 14, la maladie étant un cancer, par exemple un cancer hématologique.
